# EUROPEAN PATENT APPLICATION

(11) **EP 2 128 138 A1**
(43) Date of publication of application: **02.12.2009**
(21) Application number: 08704019.2
(22) Date of filing: 28.01.2008
(51) Int. Cl.: C07D 231/12, A61K 31/415, A61K 31/416, A61K 31/4178, A61K 31/4245, A61K 31/4439, A61K 31/5025, A61P 3/10, A61P 43/00, C07D 231/20, C07D 231/38, C07D 231/56, C07D 401/06, C07D 403/12, C07D 413/12, C07D 487/04

(54) **PYRAZOLE COMPOUND**

(30) Priority: 29.01.2007 JP 2007017656; 08.08.2007 JP 2007207273
(71) Applicant: Takeda Pharmaceutical Company Limited, Osaka 541-0045 (JP)
(72) Inventor: IMOTO, Hiroshi, Palo Alto, CA 94304 (US)
(74) Representative: Weber, Thomas
(86) International application number: PCT/JP2008/051210
(87) International publication number: WO 2008/093639

(57) **Abstract**

The present invention aims to provide an agent for the prophylaxis or treatment of diabetes, which has a superior hypoglycemic action, and is associated with a fewer side effects such as body weight gain and the like.

The present invention relates to an agent for the prophylaxis or treatment of diabetes comprising a compound represented by the formula: 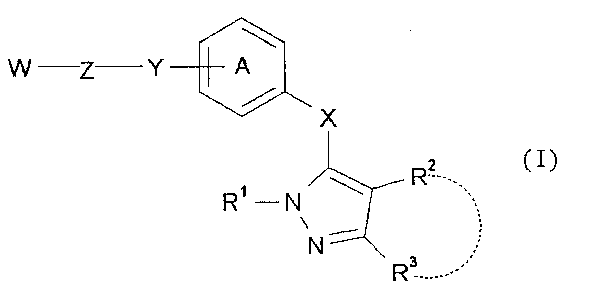 wherein each symbol is as defined in the description, or a salt thereof, or a prodrug thereof.

## Description

### Technical Field

The present invention relates to a pyrazole compound useful as a therapeutic agent for diabetes.

### (Background of the Invention)

As pyrazole compounds, the compounds described in the following documents have been known.
(1) Patent document 1 (W02006/017384) describes a compound represented by the formula:

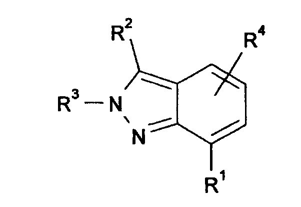

wherein
R¹ is C₁₋₆ alkyl optionally substituted by halogen or hydroxy, cyano or the like;
R² is NR⁷R⁸, arylalkyl optionally substituted by halogen, cyano or OR⁷
   wherein R⁷ and R⁸ are each independently hydrogen or C₁₋₃ alkyl,
   or the like;
R³ is C₁₋₈ alkyl, C₂₋₈ alkenyl, C₂₋₈ alkynyl, C₃₋₈ cycloalkyl, C₃₋₈ cycloalkenyl, phenyl optionally substituted by C₁₋₃ alkyl, or the like; and
R⁴ is hydrogen, halogen, methyl or methoxy,
which is an agent for the prophylaxis or treatment of diseases mediated by LXR (Liver X receptor).

(2) Patent document 2 (WO03/097609) describes a compound represented by the formula:

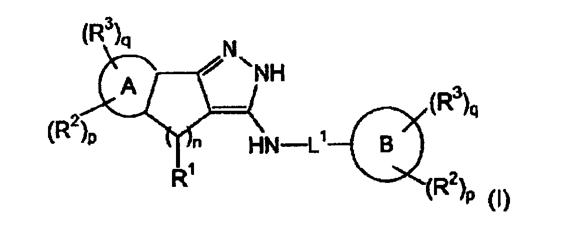

wherein
n is 0 to 4;
R¹ is hydrogen, lower alkyl or the like;
A is aryl, 5- to 6-membered monocyclic heteroaryl or the like;
p is 0 to 2;
R² is -X-A¹-D or -XA¹-YA²;
X and Y are each -O-, -NH-, -N(alkyl)-, -S-, -SO-, -SO₂- or the like;
A¹ is a bond, alkyl or alkenyl;
A² is hydrogen, alkyl or alkenyl;
provided that when A¹ or A² is alkyl or alkenyl, then the alkyl or alkenyl should be optionally substituted by halogen, cyano, hydroxyl and the like;
D is aryl, cycloalkyl, a partially unsaturated carbon ring, heteroaryl or heterocycloalkyl, each of which is optionally substituted by halogen, hydroxy, amino and the like, or the like;
q is 0 to 4;
R³ is halogen, hydroxy, amino, thio, nitro, cyano, alkyl, haloalkyl or the like;
Provided that p+q is 0 to 4;
L¹ is a bond or alkyl; and
B is aryl, cycloalkyl or the like,
which is a therapeutic agent for a cell proliferation disorder or diseases relating to PDGF-R (platelet-derived growth factor receptor).

(3) Patent document 3 (WO01/77084) describes a compound represented by the formula:

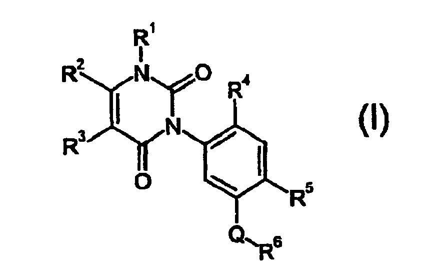

wherein
Q is O, S, SO or SO₂;
R¹ is hydrogen, amino, C₁₋₄ alkyl optionally substituted by cyano, halogen or C₁₋₄ alkoxy, or the like;
R² is cyano, carboxy, carbamoyl, thiocarbamoyl, C₁₋₄ alkyl optionally substituted by cyano, halogen or C₁₋₄ alkoxy, or C₁₋₄ alkoxycarbonyl optionally substituted by cyano, halogen or C₁₋₄ alkoxy;
R³ is hydrogen, halogen or optionally halogenated C₁₋₄ alkyl;
R⁴ is hydrogen, nitro, cyano or halogen;
R⁵ is cyano, thiocarbamoyl, bromine, optionally halogenated C₁₋₄ alkyl or optionally halogenated C₁₋₄ alkoxy;
R⁶ is nitrogen-containing heterocycle such as pyrrolyl, pyrazolyl, imidazolyl, triazolyl and the like, each of which is optionally substituted by nitro, mercapto, amino, cyano, carboxy, carbamoyl, halogen, C₁₋₄ alkyl and the like, which is an insecticide.

(4) Non-patent document 1 (Qatar University Science Journal (1994), 14, pp.114-122) describes compounds represented by the formulas:

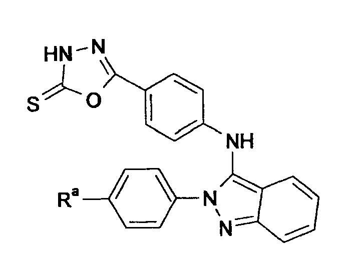

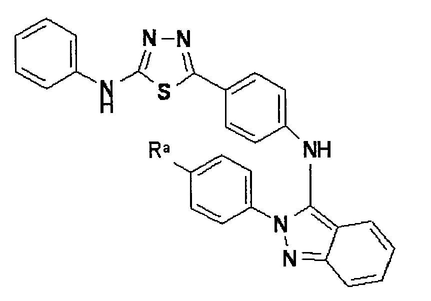

and

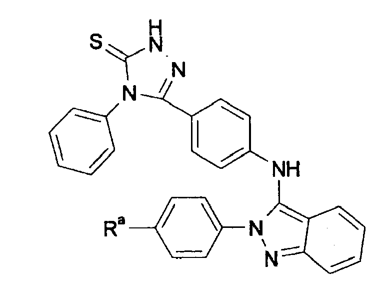

wherein R^{a} is hydrogen, methyl, methoxy, chlorine or nitro, which is an antibacterial agent.

(5) Patent document 4 (WO96/04273) describes a compound represented by the formula:

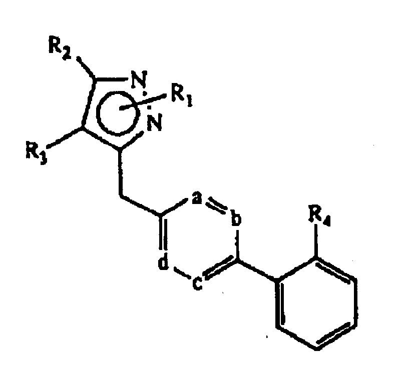

wherein
R₁ is hydrogen, C₁₋₆ alkyl, C₁₋₆ haloalkyl, C₃₋₇ cycloalkyl, aryl or the like;
R₂ is hydrogen, halogen, C₁₋₆ alkyl, C₁₋₆ haloalkyl, C₃₋₇ cycloalkyl or the like;
R₃ is hydrogen, -(CH₂)ₙR₆ wherein n is 1 to 3; and R₆ is hydroxy, C₁₋₆ alkyl etc., or the like;
a, b, c and d are all CR, or the one is N, and the remaining others are CR wherein R is hydrogen, halogen, C₁₋₄ alkyl, hydroxy, C₁₋₄ alkoxy etc.;
R₄ is -CO₂R₈, tetrazol-5-yl, tetrazol-5-ylmethyl, - CONH(tetrazol-5-yl), -CONHSO₂R₉, -CONHSO₂-Het, -CONHOR₈, -CONH₂, -CONR₈R₉, -CONHNHSO₂R₉, -CONHNHCONH₂, -CH₂NHSO₂R₉, -CH₂CO₂R₈, - CH₂CONHSO₂R₉, 3-(trifluoromethyl)-1,2,4-triazol-5-yl or the like, wherein Het is 5- to 6-membered hetero ring optionally substituted by hydroxy, mercapto, C₁₋₄ alkyl, C₁₋₄ alkoxy, halo and the like;
R₈ is hydrogen, C₁₋₄ alkyl, aryl or the like; and
R₉ is C₁₋₄ alkyl, aryl or the like,
which is an angiotensin II antagonist, and a therapeutic agent for hypertension, congestive cardiac failure, increased ocular pressure or diseases associated with an abnormal angiotensin II action.

(6) Non-patent document 2 (Journal of the Indian Chemical Society (1988), 65(1), pp.54-59) describes a compound represented by the formula:

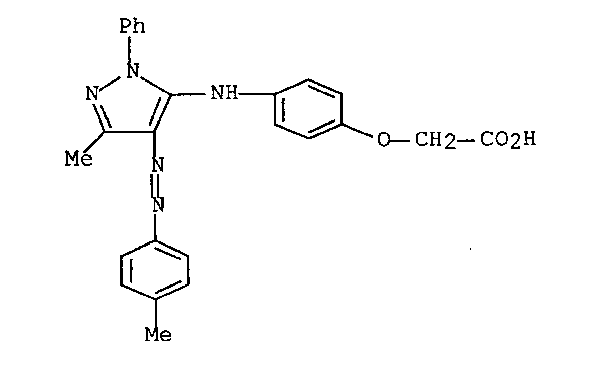

and the like, which is a stain.

(7) Patent document 5 (EP 234045 A) describes a compound represented by the formula:

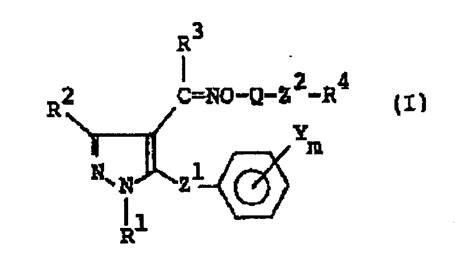

wherein
R¹ is C₁₋₄ alkyl or phenyl:
R² is hydrogen, C₁₋₅ alkyl, C₁₋₃ haloalkyl or phenyl;
R³ is H, C₁₋₄ alkyl or phenyl;
R⁴ is H, C₂₋₄ alkylcarbonyl, benzoyl, naphthoyl or the like;
Y is hydrogen, C₁₋₆ alkyl, C₁₋₄ haloalkyl, halo, hydroxy, C₁₋₄ alkoxy, C₁₋₄ haloalkoxy, C₁₋₃ alkylenedioxy, phenoxy optionally substituted by CF₃, -S(O)_{q}R²⁷ wherein R²⁷ is C₁₋₃ alkyl; and q is 0 to 2, hydroxycarbonyl, C₂₋₅ alkoxycarbonyl or -N(R²⁸)R²⁹
wherein R²⁸ and R²⁹ are each hydrogen, C₁₋₄ alkyl, or benzyl optionally substituted by C₂₋₆ alkoxycarbonyl; m is 1 to 3;
Z¹ is O or S;
Z² is O, S or a single bond; and
Q is C₁₋₈ alkylene optionally substituted by halogen or phenyl, C₃₋₁₂ alkenylene, C₃₋₁₂ haloalkenylene or C₃₋₆ alkynylene,
which is an insecticide.

(8) Non-patent document 3 (Journal of Heterocyclic Chemistry (1983), 20(6), 1501-3) describes a compound represented by the formula:

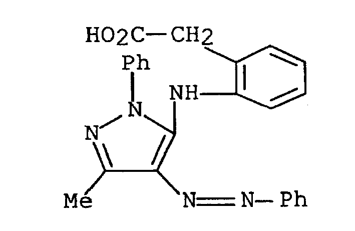

and the like.

(9) Non-patent document 4 (Archives of Pharmacal Research (2002), 25(6), 781-785) describes a compound represented by the formula:

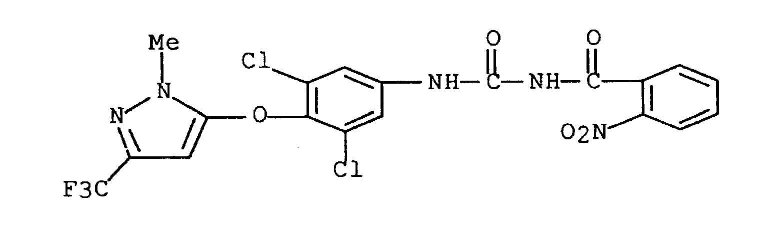

and the like, which is an anti-cancer agent.

(10) Patent document 6 (WO2004/050650) and patent document 7 (W02004/050651) describe compounds represented by the formulas:

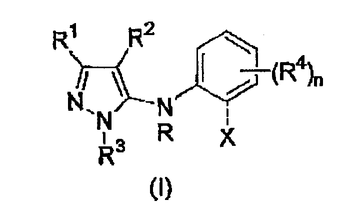

wherein
R is hydrogen or C₁₋₆ alkyl;
R¹ is hydrogen, C₁₋₆ alkyl optionally substituted by C₁₋₄ alkoxy, optionally halogenated phenyl and the like, C₃₋₆ alkenyl or the like;
R² is hydrogen, halogen, C₁₋₆ alkyl optionally substituted by C₁-₄ alkoxy, or the like; or
R¹ and R² optionally form a 5- to 6-membered ring;
R³ is C₁₋₆ alkyl, C₃₋₆ cycloalkyl or the like;
R⁴ is C₁₋₆ alkyl optionally substituted by C₁₋₄ alkoxy, C₁₋₆ alkoxy, halogen, NR⁸R⁸, pyrimidyl, pyridyl, imidazolyl or the like;
n is 0 to 3;
X is CO₂R⁸, CONR⁵R⁶, SO₂NHR⁷ or oxadiazolyl optionally substituted by C₁₋₆ alkyl;
R⁵ is hydrogen, C₁₋₆ alkyl, C₂₋₆ alkyl optionally substituted by OR⁶, benzyl, phenyl, pyridyl or SO₂-phenyl (the benzyl, phenyl, pyridyl and SO₂-phenyl are optionally substituted by halogen, C₁₋₆ alkyl optionally substituted by C₁₋₄ alkoxy, C₁₋₆ alkoxy and the like);
R⁶ is hydrogen or C₁₋₆ alkyl: or
R⁵ and R⁶ optionally form piperidine, morpholine or the like;
R⁷ is hydrogen or methyl; and
R⁸ is hydrogen, C₁₋₆ alkyl, benzyl or phenyl (the benzyl and phenyl are optionally substituted by halogen, C₁₋₆ alkyl optionally substituted by C₁₋₄ alkoxy, C₁₋₆ alkoxy, C₁₋₃ haloalkyl, C₁₋₃ haloalkoxy, cyano, C₁₋₆ alkylthio),
which is a therapeutic agent for diabetes or diabetes-associated diseases.

(11) Patent document 8 (WO2004/069158) describes a compound represented by the formula:

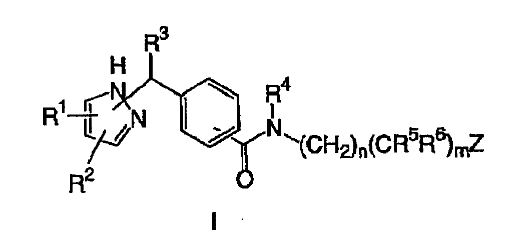

wherein
R¹ is
   (a) C₁₋₁₀ alkyl, C₂₋₁₀ alkenyl or C₂₋₁₀ alkynyl, each of which is optionally substituted by halo, oxo, aryl, heterocyclyl or heteroaryl, each of which is optionally substituted by halogen, hydroxy, CO₂R⁸, CN, S(O)ₚR⁷, NO₂ and the like, or
   (b) aryl, heteroaryl or heterocyclyl, each of which is optionally substituted by C₁₋₁₀ alkyl, C₂₋₁₀ alkenyl, C₂₋₁₀ alkynyl and the like;
R² is hydrogen or as defined in R¹;
R³ and R⁴ are each independently hydrogen or C₁₋₁₀ alkyl;
R⁵ is hydrogen or fluorine;
R⁶ is hydrogen, hydroxy, fluorine or C₁₋₃ alkyl; or
R⁵ and R⁶ optionally form oxo;
R⁷ is C₁₋₁₀ alkyl, aryl or Ar-C₁₋₁₀ alkyl;
R⁸ is hydrogen, C₁₋₁₀ alkyl optionally substituted by phenyl, hydroxy, OC₁₋₆ alkyl, CO₂H and the like;
m is 0 to 2;
n is 0 to 6;
p is 0 to 2;
provided that when at least one of m and n is not 0, then Z should be CO₂R⁸, 5-tetrazolyl or 2-oxo-1,3,4-oxadiazolyl; and when m and n are 0, then Z should be 5-tetrazolyl or 2-oxo-1,3,4-oxadiazolyl,
which is a glucagons receptor antagonist.

Peroxisome proliferator-activated receptor gamma (PPARγ), which is one member of the nuclear hormone receptor superfamily represented by steroid hormone receptors and thyroid gland hormone receptors, shows an induced expression at the beginning of differentiation of adipocytes and plays an important role as a master regulator in the differentiation of adipocytes. PPARγ binds to a ligand to form a dimer with retinoid X receptor (RXR), and the dimer binds to a responsive element of a target gene in the nucleus to directly control (activate) the transcription efficiency.

patent document 1: WO2006/017384
patent document 2: WO03/097609
patent document 3: WO01/77084
patent document 4: WO96/04273
patent document 5: EP 234045 A
patent document 6: WO2004/050650
patent document 7: WO2004/050651
patent document 8: WO2004/069158
non-patent document 1: Qatar University Science Journal (1994), 14, pp.114-122
non-patent document 2: Journal of the Indian Chemical Society (1988), 65(1), pp.54-59
non-patent document 3: Journal of Heterocyclic Chemistry (1983), 20(6), 1501-3
non-patent document 4: Archives of Pharmacal Research (2002), 25(6), 781-785

### Disclosure of the Invention

### Problems to be Solved by the Invention

There is a demand on the development of an agent for the prophylaxis or treatment of diabetes, which has a superior hypoglycemic action, and is associated with a fewer side effects such as body weight gain and the like.

The present inventor has found that a compound represented by the following formula (I) has a superior hypoglycemic action, and is useful for the prophylaxis or treatment of diabetes, and conducted further studies and completed the present invention.

Accordingly, the present invention relates to [1] a compound represented by the formula (I):

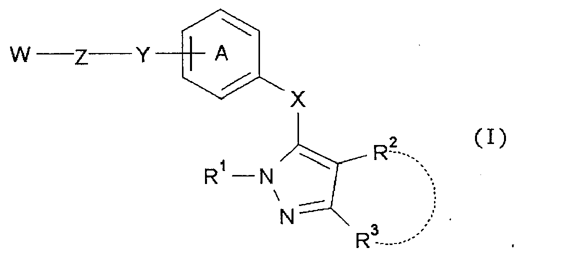

wherein
A is an optionally further substituted benzene ring;
R¹ is a hydrogen atom, an optionally substituted hydrocarbon group or an optionally substituted heterocyclic group;
R² is a hydrogen atom or a substituent (excluding an optionally substituted diazenyl group),
R³ is a hydrogen atom or a substituent, or
R² and R³ are optionally bonded to each other to form an optionally substituted monocyclic ring;
X is O, NR⁴ wherein R⁴ is a hydrogen atom or a C₁₋₆ alkyl group, S, SO, S(O)₂ or CH₂;
Y is a bond, O, NR⁵ wherein R⁵ is a hydrogen atom or a C₁₋₆ alkyl group, S, SO or S(O)₂;
Z is a bond, or a divalent hydrocarbon group having 1 to 9 atoms in the main chain, which is optionally substituted;
W is -C(=O)Q wherein Q is an optionally substituted hydroxy group or an optionally substituted amino group, or a 5- or 6-membered heterocyclic group containing NH and optionally having substituent(s),
provided that
1) when Y is a bond, and Z is a bond, then W should be a 5- or 6-membered heterocyclic group containing NH and optionally having substituent(s) (excluding a 5-phenylimino-4,5-dihydro-1,3,4-thiadiazolyl group), and the heterocyclic group should not be uracil, thioxooxadiazolinyl and thioxotriazolinyl, each of which is optionally substituted;
2) when -Z-Y- is

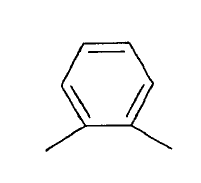

or

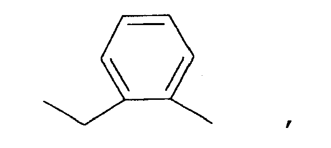

then X should not be CH₂;
3) -Z-Y- is not

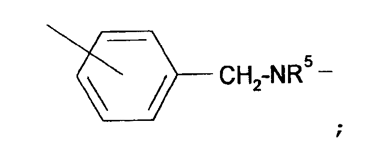

and
4) when Y is NR⁵, and Z is a bond, then W should be a 5- or 6-membered heterocyclic group containing NH and optionally having substituent(s),
or a salt thereof (hereinafter to be abbreviated as compound (I));
[2] compound (I) wherein R¹ is an optionally substituted hydrocarbon group;
[3] compound (I) wherein R² is an optionally substituted hydrocarbon group;
[4] compound (I) wherein R³ is an optionally substituted hydrocarbon group;
[5] compound (I) wherein X is O or S;
[6] compound (I) wherein Y is a bond or O;
[7] compound (I) wherein Z is a C₁₋₆ alkylene group or a C₂₋₆ alkenylene group;
[8] compound (I) wherein W is -C(=O)Q wherein Q is as defined in [1];
[9] (2S)-2-{2,4-dichloro-5-[(1,3,4-trimethyl-1H-pyrazol-5-yl)oxy]phenoxy}propionic acid (Example 1), (2S)-2-{2,4-dichloro-5-[(1,3-dimethyl-4-vinyl-1H-pyrazol-5-yl)oxy]phenoxy}propionic acid (Example 14), (2S)-2-{2,4-dichloro-5-[(2-methyl-2H-indazol-3-yl)oxy]phenoxy}propionic acid (Example 30), (2S)-2-{2,4-dichloro-5-[(2-methyl-4,5,6,7-tetrahydro-2H-indazol-3-yl)oxy]phenoxy}propionic acid (Example 31), (2S)-2-{2,4-dichloro-5-[(4-cyano-1,3-dimethyl-1H-pyrazol-5-yl)thio]phenoxy}propionic acid (Example 210), (2S)-2-{2-bromo-5-[(4-cyano-3-cyclopropyl-1-methyl-1H-pyrazol-5-yl)oxy]-4-methylphenoxy}propionic acid (Example 256), or (2S)-2-(2,4-dichloro-5-{[4-(3-{[(ethylamino)carbonyl]oxy}propyl)-1,3-dimethyl-1H-pyrazol-5-yl]oxy}phenoxy)propionic acid (Example 298), or a salt thereof;
[10] a prodrug of compound (I);
[11] a pharmaceutical agent comprising compound (I) or a prodrug thereof;
[12] the pharmaceutical agent of the above-mentioned [11], which is an insulin sensitizer;
[13] the pharmaceutical agent of the above-mentioned [11], which is an agent for the prophylaxis or treatment of diabetes;
[14] a method of improving insulin resistance in a mammal, which comprises administering compound (I) or a prodrug thereof to the mammal;
[15] a method for the prophylaxis or treatment of diabetes in a mammal, which comprises administering compound (I) or a prodrug thereof to the mammal;
[16] use of compound (I) or a prodrug thereof for the production of an insulin sensitizer;
[17] use of compound (I) or a prodrug thereof for the production of an agent for the prophylaxis or treatment of diabetes;
and the like.

### Effect of the Invention

According to the present invention, an agent for the prophylaxis or treatment of diabetes, which has a superior hypoglycemic action, and is associated with a fewer side effects such as body weight gain and the like, can be provided.

### [Detailed Description of the Invention]

The definition of each symbol in the formula (I) is described in detail in the following.
Unless otherwise specified, the "halogen atom" in the present specification means a fluorine atom, a chlorine atom, a bromine atom or an iodine atom.
Unless otherwise specified, the "C₁₋₃ alkylenedioxy group" in the present specification means methylenedioxy, ethylenedioxy, trimethylenedioxy or the like.
Unless otherwise specified, the "C₁₋₆ alkyl group" in the present specification means methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl or the like.
Unless otherwise specified, the "C₁₋₆ alkoxy group" in the present specification means methoxy, ethoxy, propoxy, isopropoxy, butoxy, isobutoxy, sec-butoxy, tert-butoxy or the like.
Unless otherwise specified, the "C₁₋₆ alkoxy-carbonyl group" in the present specification means methoxycarbonyl, ethoxycarbonyl, propoxycarbonyl, tert-butoxycarbonyl or the like.
Unless otherwise specified, the "C₁₋₆ alkyl-carbonyl group" in the present specification means acetyl, propanoyl, butanoyl, isobutanoyl, pentanoyl, isopentanoyl, hexanoyl or the like.

R² is a hydrogen atom or a substituent (excluding an optionally substituted diazenyl group (-N=NH)), R³ is a hydrogen atom or a substituent, or R² and R³ are optionally bonded to each other to form an optionally substituted monocyclic ring.

Examples of the "substituent" for R² or R³ include an "optionally substituted hydrocarbon group", an "optionally substituted heterocyclic group", an "optionally substituted hydroxy group", an "optionally substituted amino group", an "optionally substituted mercapto group", a "cyano group", a "nitro group", an "acyl group", a "halogen atom" and the like.

Examples of the "hydrocarbon group" of the above-mentioned "optionally substituted hydrocarbon group" include a C₁₋₁₀ alkyl group, a C₂₋₁₀ alkenyl group, a C₂₋₁₀ alkynyl group, a C₃₋₁₀ cycloalkyl group, a C₃₋₁₀ cycloalkenyl group, a C₄₋₁₀ cycloalkadienyl group, a C₆₋₁₄ aryl group, a C₇₋₁₃ aralkyl group, a C₈₋₁₃ arylalkenyl group and the like.

Examples of the C₁₋₁₀ alkyl group include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl and the like. Of these, a C₁₋₆ alkyl group is preferable.

Examples of the C₂₋₁₀ alkenyl group include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octenyl and the like. Of these, a C₂₋₆ alkenyl group is preferable.

Examples of the C₂₋₁₀ alkynyl group include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 1-octynyl and the like. Of these, a C₂₋₆ alkynyl group is preferable.

Examples of the C₃₋₁₀ cycloalkyl group include cyclopropyl, cyclobutyl, cyclopentyl, cyclohexyl, cycloheptyl, cyclooctyl and the like. Of these, a C₃₋₆ cycloalkyl group is preferable.
Examples of the C₃₋₁₀ cycloalkenyl group include 2-cyclopenten-1-yl, 3-cyclopenten-1-yl, 2-cyclohexen-1-yl, 3-cyclohexen-1-yl and the like. Of these, a C₃₋₆ cycloalkenyl group is preferable.

Examples of the C₄₋₁₀ cycloalkadienyl group include 2,4-cyclopentadien-1-yl, 2,4-cyclohexadien-1-yl, 2,5-cyclohexadien-1-yl and the like. Of these, a C₄₋₆ cycloalkadienyl group is preferable.

The above-mentioned C₃₋₁₀ cycloalkyl group, C₃₋₁₀ cycloalkenyl group and C₄₋₁₀ cycloalkadienyl group are each optionally condensed with a benzene ring to form a fused cyclic group. Examples of the fused cyclic group include indanyl, dihydronaphthyl, tetrahydronaphthyl, fluorenyl and the like.

In addition, the above-mentioned C₃₋₁₀ cycloalkyl group, C₃₋₁₀ cycloalkenyl group and C₄₋₁₀ cycloalkadienyl group may be each a C₇₋₁₀ cross-linked hydrocarbon group. Examples of the C₇-₁₀ cross-linked hydrocarbon group include bicyclo[2.2.1]heptyl (norbornyl), bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl, adamantyl and the like.

Moreover, the above-mentioned C₃₋₁₀ cycloalkyl group, C₃₋₁₀ cycloalkenyl group and C₄₋₁₀ cycloalkadienyl group each optionally form, together with a C₃₋₁₀ cycloalkane, a C₃₋₁₀ cycloalkene or a C₄₋₁₀ cycloalkadiene, a spiro ring group. Examples of the C₃₋₁₀ cycloalkane, C₃₋₁₀ cycloalkene and C₄₋₁₀ cycloalkadiene include rings corresponding to the above-mentioned C₃₋₁₀ cycloalkyl group, C₃₋₁₀ cycloalkenyl group and C₄-₁₀ cycloalkadienyl group. Examples of the spiro ring group include spiro[4.5]decan-8-yl and the like.

Examples of the C₆₋₁₄ aryl group include phenyl, naphthyl, anthryl, phenanthryl, acenaphthylenyl, biphenylyl and the like. Of these, a C₆₋₁₂ aryl group is preferable.

Examples of the C₇₋₁₃ aralkyl group include benzyl, phenethyl, naphthylmethyl, biphenylylmethyl and the like.

Examples of the C₈₋₁₃ arylalkenyl group include styryl and the like.

The C₁₋₁₀ alkyl group, C₂₋₁₀ alkenyl group and C₂₋₁₀ alkynyl group exemplified as the above-mentioned "hydrocarbon group" optionally have 1 to 3 substituents at substitutable positions.
Examples of the substituent include
(1) a C₃₋₁₀ cycloalkyl group (e.g., cyclopropyl, cyclohexyl) optionally substituted by 1 to 3 hydroxy groups;
(2) a C₆₋₁₄ aryl group (e.g., phenyl, naphthyl) optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group optionally substituted by 1 to 3 halogen atoms,
   (d) a halogen atom, and
   (e) a cyano group
(3) an aromatic heterocyclic group (e.g., thienyl, furyl, pyridyl, pyrazolyl, imidazolyl, tetrazolyl, oxazolyl, thiazolyl, oxadiazolyl, thiadiazolyl) optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group optionally substituted by 1 to 3 halogen atoms, and
   (d) a halogen atom;
(4) a non-aromatic heterocyclic group (e.g., tetrahydrofuryl, morpholinyl, thiomorpholinyl, piperidinyl, pyrrolidinyl, piperazinyl) optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group optionally substituted by 1 to 3 halogen atoms, and
   (d) a halogen atom;
(5) an amino group optionally mono- or di-substituted by substituent(s) selected from
   (a) a C₁₋₆ alkyl group optionally substituted by 1 to 3 halogen atoms,
   (b) a C₁₋₆ alkyl-carbonyl group optionally substituted by 1 to 3 halogen atoms, and
   (c) a C₁₋₆ alkoxy-carbonyl group optionally substituted by 1 to 3 halogen atoms;
(6) a C₁₋₆ alkyl-carbonyl group optionally substituted by 1 to 3 halogen atoms;
(7) a C₁₋₆ alkoxy-carbonyl group optionally substituted by 1 to 3 substituents selected from
   (a) a halogen atom, and
   (b) a C₁₋₆ alkoxy group;
(8) a C₁₋₆ alkylsulfonyl group (e.g., methylsulfonyl, ethylsulfonyl, isopropylsulfonyl) optionally substituted by 1 to 3 halogen atoms;
(9) a carbamoyl group optionally mono- or di-substituted by C₁₋₆ alkyl group(s) optionally substituted by 1 to 3 halogen atoms;
(10) a thiocarbamoyl group optionally mono- or di-substituted by C₁₋₆ alkyl group(s) optionally substituted by 1 to 3 halogen atoms;
(11) a sulfamoyl group optionally mono- or di-substituted by substituent(s) selected from
   (a) a C₁₋₆ alkyl group optionally substituted by 1 to 3 halogen atoms, and
   (b) a C₁₋₆ alkoxy-carbonyl group;
(12) a carboxy group;
(13) a hydroxy group;
(14) a C₁₋₆ alkoxy group optionally substituted by 1 to 3 substituents selected from
   (a) a halogen atom,
   (b) a carboxy group,
   (c) a C₁₋₆ alkoxy group,
   (d) a C₁₋₆ alkoxy-carbonyl group, and
   (e) an amino group optionally mono- or di-substituted by substituent(s) selected from a C₁₋₆ alkyl group and a C₁₋₆ alkoxy-carbonyl group;
(15) a C₂₋₆ alkenyloxy group (e.g., ethenyloxy) optionally substituted by 1 to 3 halogen atoms;
(16) a C₇₋₁₃ aralkyloxy group (e.g., benzyloxy);
(17) a C₆₋₁₄ aryloxy group (e.g., phenyloxy, naphthyloxy);
(18) a C₁₋₆ alkyl-carbonyloxy group (e.g., acetyloxy, tert-butylcarbonyloxy);
(19) a C₆₋₁₄ aryl-carbonyl group (e.g., benzoyl) optionally substituted by 1 to 3 substituents selected from
   (a) a halogen atom, and
   (b) a C₁₋₆ alkyl group optionally substituted by 1 to 3 halogen atoms;
(20) a non-aromatic heterocyclylcarbonyl group (e.g., pyrrolidinylcarbonyl, morpholinylcarbonyl, 1,1-dioxidothiomorpholinylcarbonyl) optionally substituted by 1 to 3 substituents selected from a C₁₋₆ alkyl group optionally substituted by 1 to 3 halogen atoms;
(21) a mercapto group;
(22) a C₁₋₆ alkylthio group (e.g., methylthio, ethylthio) optionally substituted by 1 to 3 halogen atoms;
(23) a C₇₋₁₃ aralkylthio group (e.g., benzylthio);
(24) a C₆₋₁₄ arylthio group (e.g., phenylthio, naphthylthio);
(25) a cyano group;
(26) a nitro group;
(27) a halogen atom;
(28) a C₁₋₃ alkylenedioxy group;
(29) an aromatic heterocyclylcarbonyl group (e.g., pyrazolylcarbonyl, pyrazinylcarbonyl, isoxazolylcarbonyl, pyridylcarbonyl, thiazolylcarbonyl) optionally substituted by 1 to 3 C₁₋₆ alkyl groups optionally substituted by 1 to 3 halogen atoms;
(30) a hydroxyimino group optionally substituted by a substituent selected from
   (a) a C₁₋₆ alkyl group optionally substituted by 1 to 3 substituents selected from
      (i) a C₆₋₁₄ aryl group (e.g., phenyl),
      (ii) a C₁₋₆ alkyl-carbonyl group, and
      (iii) a cyano group, and
   (b) a C₆₋₁₄ aryl group (e.g., phenyl);
(31) a carbamoyloxy group optionally mono- or di-substituted by C₁₋₆ alkyl group(s);
(32) an imino group optionally substituted by a C₁₋₆ alkyl group optionally substituted by 1 to 3 cyano groups;
(33) a formyl group;
(34) a hydrazono group;
(35) a tri-C₁₋₆ alkylsilyl group;
(36) a dioxaborolanyl group (e.g., 1,3,2-dioxaborolan-2-yl) optionally substituted by 1 to 4 C₁₋₆ alkyl groups;
(37) a 2,4-dioxo-1,3-thiazolidin-5-ylidene group;
(38) a non-aromatic heterocyclyloxy group (e.g., 1,3-dihydro-2H-isoindol-2-yloxy group, dihydropyridyloxy) optionally substituted by 1 to 3 oxo groups;
(39) a tetrahydro-4H-pyran-4-ylidenaminooxy group;
(40) a 1,3-dioxan-5-ylidenaminooxy group optionally substituted by 1 to 3 C₁₋₆ alkyl groups;
(41) an aminooxy group;
(42) a C₁₋₆ alkylidenaminooxy group (e.g., isopropylidenaminooxy) optionally substituted by 1 to 3 C₁₋₆ alkoxy groups;
and the like. When the number of the substituents is not less than 2, the respective substituents may be the same or different.

The C₃₋₁₀ cycloalkyl group, C₃₋₁₀ cycloalkenyl group, C₄₋₁₀ cycloalkadienyl group, C₆₋₁₄ aryl group, C₇₋₁₃ aralkyl group and C₈₋₁₃ arylalkenyl group exemplified as the above-mentioned "hydrocarbon group" optionally have 1 to 3 substituents at substitutable positions.

Examples of the substituent include
(1) the groups exemplified as the substituents for the above-mentioned C₁₋₁₀ alkyl group and the like;
(2) a C₁₋₆ alkyl group optionally substituted by 1 to 3 substituents selected from
   (a) a halogen atom,
   (b) a carboxy group,
   (c) a hydroxy group,
   (d) a C₁₋₆ alkoxy-carbonyl group,
   (e) a C₁₋₆ alkoxy group,
   (f) an amino group optionally mono- or di-substituted by C₁₋₆ alkyl group(s),
   (g) a hydroxyimino group optionally substituted by a substituent selected from a C₁₋₆ alkyl group optionally substituted by 1 to 3 C₆₋₁₄ aryl groups (e.g., phenyl), and C₆₋₁₄ aryl group (e.g., phenyl),
   (h) a cyano group,
   (i) a tri-C₁₋₆ alkylsilyl group,
   (j) a C₆₋₁₄ aryloxy group (e.g., phenyloxy), and
   (k) a C₁₋₆ alkylsulfonyl group (e.g., methylsulfonyl);
(3) a C₂₋₆ alkenyl group (e.g., ethenyl, 1-propenyl) optionally substituted by 1 to 3 substituents selected from
   (a) a halogen atom,
   (b) a carboxy group,
   (c) a hydroxy group,
   (d) a C₁₋₆ alkoxy-carbonyl group,
   (e) a C₁₋₆ alkoxy group, and
   (f) an amino group optionally mono- or di-substituted by C₁₋₆ alkyl group(s);
(4) a C₇₋₁₃ aralkyl group (e.g., benzyl) optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group, and
   (d) a halogen atom;
and the like. When the number of the substituents is not less than 2, the respective substituents may be the same or different.

Examples of the "heterocyclic group" of the above-mentioned "optionally substituted heterocyclic group" include an aromatic heterocyclic group and a non-aromatic heterocyclic group.

Examples of the aromatic heterocyclic group include a 5-to 7-membered monocyclic aromatic heterocyclic group containing, as a ring-constituting atom besides carbon atoms, 1 to 4 hetero atoms selected from an oxygen atom, a sulfur atom and a nitrogen atom, and a fused aromatic heterocyclic group. Examples of the fused aromatic heterocyclic group include a group derived from a fused ring wherein a ring corresponding to the 5- to 7-membered monocyclic aromatic heterocyclic group and 1 or 2 rings selected from a 5- or 6-membered aromatic heterocycle containing 1 or 2 nitrogen atoms (e.g., pyrrole, imidazole, pyrazole, pyrazine, pyridine, pyrimidine), a 5-membered aromatic heterocycle containing one sulfur atom (e.g., thiophene) and a benzene ring are condensed, and the like.

Preferable examples of the aromatic heterocyclic group include monocyclic aromatic heterocyclic groups such as furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl (e.g., 2-pyrimidinyl, 4-pyrimidinyl, 5-pyrimidinyl), pyridazinyl (e.g., 3-pyridazinyl, 4-pyridazinyl), pyrazinyl (e.g., 2-pyrazinyl), pyrrolyl (e.g., 1-pyrrolyl, 2-pyrrolyl, 3-pyrrolyl), imidazolyl (e.g., 1-imidazolyl, 2-imidazolyl, 4-imidazolyl, 5-imidazolyl), pyrazolyl (e.g., 1-pyrazolyl, 3-pyrazolyl, 4-pyrazolyl), thiazolyl (e.g., 2-thiazolyl, 4-thiazolyl, 5-thiazolyl), isothiazolyl (e.g., 4-isothiazolyl), oxazolyl (e.g., 2-oxazolyl, 4-oxazolyl, 5-oxazolyl), isoxazolyl (e.g., 3-isoxazolyl), oxadiazolyl (e.g., 1,2,4-oxadiazol-5-yl, 1,3,4-oxadiazol-2-yl), thiadiazolyl (e.g., 1,3,4-thiadiazol-2-yl), triazolyl (e.g., 1,2,4-triazol-1-yl, 1,2,4-triazol-3-yl, 1,2,3-triazol-1-yl, 1,2,3-triazol-2-yl, 1,2,3-triazol-4-yl), tetrazolyl (e.g., tetrazol-1-yl, tetrazol-5-yl), triazinyl (e.g., 1,2,4-triazin-1-yl, 1,2,4-triazin-3-yl, 1,3,5-triazin-1-yl) and the like; fused aromatic heterocyclic groups such as quinolyl (e.g., 2-quinolyl, 3-quinolyl, 4-quinolyl, 6-quinolyl), isoquinolyl (e.g., 3-isoquinolyl), quinazolyl (e.g., 2-quinazolyl, 4-quinazolyl), quinoxalyl (e.g., 2-quinoxalyl, 6-quinoxalyl), benzofuranyl (e.g., 2-benzofuranyl, 3-benzofuranyl), benzothiophenyl (e.g., 2-benzothiophenyl, 3-benzothiophenyl), benzoxazolyl (e.g., 2-benzoxazolyl), benzisoxazolyl (e.g., 7-benzisoxazolyl), benzothiazolyl (e.g., 2-benzothiazolyl), benzimidazolyl (e.g., benzimidazol-1-yl, benzimidazol-2-yl, benzimidazol-5-yl), benzotriazolyl (e.g., 1H-1,2,3-benzotriazol-5-yl), indolyl (e.g., indol-1-yl, indol-2-yl, indol-3-yl, indol-5-yl), indazolyl (e.g., 1H-indazol-3-yl), pyrrolopyrazinyl (e.g., 1H-pyrrolo[2,3-b]pyrazin-2-yl, 1H-pyrrolo[2,3-b]pyrazin-6-yl), imidazopyridinyl (e.g., 1H-imidazo[4,5-b]pyridin-2-yl, 1H-imidazo[4,5-c]pyridin-2-yl, 2H-imidazo[1,2-a]pyridin-3-yl), imidazopyrazinyl (e.g., 1H-imidazo[4,5-b]pyrazin-2-yl), pyrazolopyridinyl (e.g., 1H-pyrazolo[4,3-c]pyridin-3-yl), pyrazolothienyl (e.g., 2H-pyrazolo[3,4-b]thiophen-2-yl), pyrazolotriazinyl (e.g., pyrazolo[5,1-c][1,2,4]triazin-3-yl) and the like; and the like.

Examples of the non-aromatic heterocyclic group include a 5- to 7-membered monocyclic non-aromatic heterocyclic group containing, as a ring-constituting atom besides carbon atoms, 1 to 4 hetero atoms selected from an oxygen atom, a sulfur atom (the sulfur atom is optionally oxidized) and a nitrogen atom, and a fused non-aromatic heterocyclic group. Examples of the fused non-aromatic heterocyclic group include a group derived from a fused ring wherein a ring corresponding to the 5- to 7-membered monocyclic non-aromatic heterocyclic group and 1 or 2 rings selected from a 5- or 6-membered aromatic or non-aromatic heterocycle containing 1 or 2 nitrogen atoms (e.g., pyrrole, imidazole, pyrazole, pyrazine, pyridine, pyrimidine), a 5-membered aromatic or non-aromatic heterocycle containing one sulfur atom (e.g., thiophene) and a benzene ring are condensed, a group wherein the above-mentioned group is partially saturated, and the like. In addition, Examples of the non-aromatic heterocyclic group also include a group wherein any of ring-constituting carbon atoms on the ring of the above-mentioned non-aromatic heterocyclic group is substituted by 1 to 3 oxo groups and/or thioxo groups.

Preferable examples of the non-aromatic heterocyclic group include monocyclic non-aromatic heterocyclic groups such as tetrahydrofuryl (e.g., 2-tetrahydrofuryl), pyrrolidinyl (e.g., 1-pyrrolidinyl), 1,1-dioxidotetrahydrothienyl (e.g., 1,1-dioxidotetrahydro-3-thienyl), piperidinyl (e.g., piperidino), morpholinyl (e.g., morpholino), thiomorpholinyl (e.g., thiomorpholino), 1,1-dioxidothiomorpholinyl (e.g., 1,1-dioxidothiomorpholino), piperazinyl (e.g., 1-piperazinyl), hexamethyleniminyl (e.g., hexamethylenimin-1-yl), oxazolinyl (e.g., 2,5-dihydrooxazol-3-yl, 3,4-dihydrooxazol-3-yl), thiazolinyl (e.g., 2,5-dihydrothiazol-3-yl, 3,4-dihydrothiazol-3-yl), imidazolinyl (e.g., 2-imidazolin-3-yl), oxazolidinyl (e.g., oxazolidin-3-yl), thiazolidinyl (e.g., thiazolidin-3-yl, thiazolidin-5-yl), imidazolidinyl (e.g., imidazolidin-3-yl), dioxolyl (e.g., 1,3-dioxol-4-yl), dioxolanyl (e.g., 1,3-dioxolan-4-yl), dihydrooxadiazolyl (e.g., 4,5-dihydro-1,2,4-oxadiazol-3-yl), thioxooxazolidinyl (e.g., 2-thioxo-1,3-oxazolidin-5-yl), tetrahydropyranyl (e.g., 4-tetrahydropyranyl), tetrahydrothiopyranyl (e.g., 4-tetrahydrothiopyranyl), 1,1-dioxidotetrahydrothiopyranyl (e.g., 1,1-dioxidotetrahydrothiopyran-4-yl), pyrazolidinyl (e.g., pyrazolidin-1-yl), oxotetrahydropyridazinyl (e.g., 3-oxo-2,3,4,5-tetrahydropyridazin-4-yl) and the like; fused non-aromatic heterocyclic groups such as dihydroisoindolyl (e.g., 1,3-dihydro-2H-isoindol-2-yl), dihydrobenzofuranyl (e.g., 2,3-dihydro-1-benzofuran-5-yl), dihydrobenzodioxinyl (e.g., 2,3-dihydro-1,4-benzodioxin-2-yl), dihydrobenzodioxepinyl (e.g., 3,4-dihydro-2H-1,5-benzodioxepin-2-yl), tetrahydrobenzofuranyl (e.g., 4,5,6,7-tetrahydro-1-benzofuran-3-yl), tetrahydrobenzothiazolyl (e.g., 4,5,6,7-tetrahydro-1-benzothiazol-2-yl), tetrahydrobenzoxazolyl (e.g., 4,5,6,7-tetrahydro-1-benzoxazol-2-yl), chromenyl (e.g., 4H-chromen-2-yl, 2H-chromen-3-yl), dihydroquinolinyl (e.g., 1,2-dihydroquinolin-2-yl), tetrahydroquinolinyl (e.g., 1,2,3,4-tetrahydroquinolin-2-yl), dihydroisoquinolinyl (e.g., 1,2-dihydroisoquinolin-2-yl), tetrahydroisoquinolinyl (e.g., 1,2,3,4-tetrahydroisoquinolin-4-yl, 1,2,3,4-tetrahydroisoquinolin-2-yl), dihydrophthalazinyl (e.g., 1,4-dihydrophthalazin-4-yl), tetrahydroindazolyl (e.g., 4,5,6,7-tetrahydro-2H-indazol-2-yl), tetrahydroquinazolinyl (e.g., 5,6,7,8-tetrahydroquinazolin-6-yl), tetrahydrothiazolopyridinyl (e.g., 4,5,6,7-tetrahydrothiazolo[5.4-c]pyridin-6-yl), tetrahydroimidazopyridinyl (e.g., 1,2,3,4-tetrahydroimidazo[4.5-c]pyridin-2-yl), tetrahydropyrazolopyridinyl (e.g., 1,2,3,4-tetrahydropyrazolo[3.4-c]pyridin-2-yl), tetrahydrotriazolopyrazinyl (e.g., 1,2,3,4-tetrahydrotriazolo[4.3-a]pyrazin-2-yl), tetrahydroimidazopyrazinyl (e.g., 1,2,3,4-tetrahydroimidazo[1.2-a]pyrazin-2-yl, 1,2,3,4-tetrahydroimidazo[3.4-a]pyrazin-2-yl), tetrahydropyridopyrimidinyl (e.g., 5,6,7,8-tetrahydropyrido[5.4-c]pyrimidin-6-yl) and the like.

The "heterocyclic group" of the above-mentioned "optionally substituted heterocyclic group" optionally has 1 to 3 substituents at substitutable positions. Examples of the substituent include those similar to the substituent that the C₃₋₁₀ cycloalkyl group and the like exemplified as the "hydrocarbon group" of the above-mentioned "optionally substituted hydrocarbon group" optionally has. When the number of the substituents is not less than 2, the respective substituents may be the same or different.

Examples of the above-mentioned "optionally substituted hydroxy group" include a hydroxy group optionally substituted by a substituent selected from a C₁₋₁₀ alkyl group, a C₂₋₁₀ alkenyl group, a C₃₋₁₀ cycloalkyl group, a C₃₋₁₀ cycloalkenyl group, a C₆₋₁₄ aryl group, a C₇₋₁₃ aralkyl group, a C₈₋₁₃ arylalkenyl group, a C₁₋₆ alkyl-carbonyl group, a heterocyclic group and the like, each of which is optionally substituted.

Examples of the C₁₋₁₀ alkyl group, C₂₋₁₀ alkenyl group, C₃₋₁₀ cycloalkyl group, C₃₋₁₀ cycloalkenyl group, C₆₋₁₄ aryl group, C₇₋₁₃ aralkyl group and C₈₋₁₃ arylalkenyl group include those exemplified as the "hydrocarbon group" of the above-mentioned "optionally substituted hydrocarbon group".

Examples of the heterocyclic group include those similar to the "aromatic heterocyclic group" and "non-aromatic heterocyclic group" exemplified as the "heterocyclic group" of the above-mentioned "optionally substituted heterocyclic group".

The above-mentioned C₁₋₁₀ alkyl group, C₂₋₁₀ alkenyl group, C₃₋₁₀ cycloalkyl group, C₃₋₁₀ cycloalkenyl group, C₆₋₁₄ aryl group, C₇₋₁₃ aralkyl group, C₈₋₁₃ arylalkenyl group, C₁₋₆ alkyl-carbonyl group and heterocyclic group optionally have 1 to 3 substituents at substitutable positions. When the number of the substituents is not less than 2, the respective substituents may be the same or different.

Examples of the substituent for the C₁₋₁₀ alkyl group, C₂₋₁₀ alkenyl group and C₁₋₆ alkyl-carbonyl group include those similar to the substituent that the C₁₋₁₀ alkyl group and the like exemplified as the "hydrocarbon group" of the above-mentioned "optionally substituted hydrocarbon group" optionally has.

Examples of the substituent for the C₃₋₁₀ cycloalkyl group, C₃₋₁₀ cycloalkenyl group, C₆₋₁₄ aryl group, C₇₋₁₃ aralkyl group, C₈₋₁₃ arylalkenyl group and heterocyclic group include those similar to the substituent that the C₃₋₁₀ cycloalkyl group and the like exemplified as the "hydrocarbon group" of the above-mentioned "optionally substituted hydrocarbon group" optionally has.

Examples of the above-mentioned "optionally substituted mercapto group" include a mercapto group optionally substituted by a substituent selected from a C₁₋₁₀ alkyl group, a C₂₋₁₀ alkenyl group, a C₃₋₁₀ cycloalkyl group, a C₃₋₁₀ cycloalkenyl group, a C₆₋₁₄ aryl group, a C₇₋₁₃ aralkyl group, a C₈₋₁₃ arylalkenyl group, a C₁₋₆ alkyl-carbonyl group, a heterocyclic group and the like, each of which is optionally substituted.

Examples of the substituent include those exemplified as the substituents of the above-mentioned "optionally substituted hydroxy group".

Examples of the above-mentioned "optionally substituted amino group" include an amino group optionally mono- or di-substituted by substituent(s) selected from a C₁₋₁₀ alkyl group, a C₂₋₁₀ alkenyl group, a C₃₋₁₀ cycloalkyl group, a C₃₋₁₀ cycloalkenyl group, a C₆₋₁₄ aryl group, a C₇₋₁₃ aralkyl group, a C₈₋₁₃ arylalkenyl group and a heterocyclic group, each of which is optionally substituted; an acyl group and the like.

Examples of the C₁₋₁₀ alkyl group, C₂₋₁₀ alkenyl group, C₃₋₁₀ cycloalkyl group, C₃₋₁₀ cycloalkenyl group, C₆₋₁₄ aryl group, C₇₋₁₃ aralkyl group and C₈₋₁₃ arylalkenyl group include those exemplified as the "hydrocarbon group" of the above-mentioned "optionally substituted hydrocarbon group".

Examples of the heterocyclic group include those similar to the "aromatic heterocyclic group" and "non-aromatic heterocyclic group" exemplified as the "heterocyclic group" of the above-mentioned "optionally substituted heterocyclic group". Of these, a 5- to 7-membered monocyclic aromatic heterocyclic group is preferable.

The C₁₋₁₀ alkyl group, C₂₋₁₀ alkenyl group, C₃₋₁₀ cycloalkyl group, C₃₋₁₀ cycloalkenyl group, C₆₋₁₄ aryl group, C₇₋₁₃ aralkyl group, C₈₋₁₃ arylalkenyl group and heterocyclic group optionally have 1 to 3 substituents at substitutable positions. When the number of the substituents is not less than 2, the respective substituents may be the same or different.

Examples of the substituent for the C₁₋₁₀ alkyl group and C₂₋₁₀ alkenyl group include those similar to the substituent that the C₁₋₁₀ alkyl group and the like exemplified as the "hydrocarbon group" of the above-mentioned "optionally substituted hydrocarbon group" optionally has.

Examples of the substituent for the C₃₋₁₀ cycloalkyl group, C₃₋₁₀ cycloalkenyl group, C₆₋₁₄ aryl group, C₇₋₁₃ aralkyl group, C₈₋₁₃ arylalkenyl group and heterocyclic group include those similar to the substituent that the C₃₋₁₀ cycloalkyl group and the like exemplified as the "hydrocarbon group" of the above-mentioned "optionally substituted hydrocarbon group" optionally has.

Examples of the "acyl group" exemplified as the substituent for the "optionally substituted amino group" include those similar to the "acyl group" below, which is exemplified as the "substituent" for R² or R³.

Examples of the "acyl group" which is exemplified as the "substituent" for R² or R³ include a group represented by the formula: -COR^{A}, -CO-OR^{A}, -SO₃R^{A}, -S(O)₂R^{A}, -SOR^{A}, -CO-NR^{A},R^{B}, - CS-NR^{A}'R^{B}' or -S(O)₂NR^{A}'R^{B}' wherein R^{A} is a hydrogen atom, an optionally substituted hydrocarbon group or an optionally substituted heterocyclic group, and R^{A}'_{,} and R^{B}', are the same or different and each is a hydrogen atom, an optionally substituted hydrocarbon group or an optionally substituted heterocyclic group, or R^{A}' and R^{B}' optionally form, together with the adjacent nitrogen atom, an optionally substituted nitrogen-containing heterocycle, and the like.

Examples of the "optionally substituted hydrocarbon group" and "optionally substituted heterocyclic group" for R^{A}, R^{A}' or R^{B}' include those similar to the "optionally substituted hydrocarbon group" and "optionally substituted heterocyclic group", which are exemplified as the "substituent" for R² or R³.

Examples of the "nitrogen-containing heterocycle" of the "optionally substituted nitrogen-containing heterocycle" formed by R^{A}' and R^{B}' together with the adjacent nitrogen atom include a 5- to 7-membered nitrogen-containing heterocycle containing, as a ring-constituting atom besides carbon atoms, at least one nitrogen atom and optionally further containing one or two hetero atoms selected from an oxygen atom, a sulfur atom and a nitrogen atom. Preferable examples of the nitrogen-containing heterocycle include pyrrolidine, imidazolidine, pyrazolidine, piperidine, piperazine, morpholine, thiomorpholine, oxopiperazine and the like.

The nitrogen-containing heterocycle optionally has 1 to 3 (preferably 1 or 2) substituents at substitutable positions. Examples of the substituent include those similar to the substituent that the C₃₋₁₀ cycloalkyl group and the like exemplified as the "hydrocarbon group" of the above-mentioned "optionally substituted hydrocarbon group" optionally has. When the number of the substituents is not less than 2, the respective substituents may be the same or different.

Preferable examples of the "acyl group" include
(1) a formyl group;
(2) a carboxy group;
(3) a C₁₋₆ alkyl-carbonyl group (e.g., acetyl) optionally substituted by 1 to 3 halogen atoms;
(4) a C₁₋₆ alkoxy-carbonyl group (e.g., methoxycarbonyl, ethoxycarbonyl, propoxycarbonyl, tert-butoxycarbonyl) optionally substituted by 1 to 3 halogen atoms;
(5) a C₃₋₁₀ cycloalkyl-carbonyl group (e.g., cyclopropylcarbonyl, cyclopentylcarbonyl, cyclohexylcarbonyl);
(6) a C₆₋₁₄ aryl-carbonyl group (e.g., benzoyl, 1-naphthoyl, 2-naphthoyl) optionally substituted by 1 to 3 halogen atoms;
(7) a carbamoyl group optionally mono- or di-substituted by substituent(s) selected from
   (a) a C₁₋₆ alkyl group optionally substituted by 1 to 3 substituents selected from a halogen atom, a C₁₋₆ alkoxy group, a C₁₋₆ alkoxy-carbonyl group and a carboxy group, and
   (b) an amino group optionally mono- or di-substituted by C₁₋₆ alkoxy-carbonyl group(s);
(8) a C₁₋₆ alkylsulfonyl group (e.g., methylsulfonyl, ethylsulfonyl, isopropylsulfonyl) optionally substituted by 1 to 3 halogen atoms;
(9) a C₆₋₁₄ arylsulfonyl group (e.g., benzenesulfonyl);
(10) a sulfamoyl group;
(11) a thiocarbamoyl group;
(12) an aromatic heterocyclylcarbonyl group (e.g., furylcarbonyl, thienylcarbonyl) optionally substituted by 1 to 3 substituents selected from a C₁₋₆ alkyl group optionally substituted by 1 to 3 halogen atoms;
(13) a non-aromatic heterocyclylcarbonyl group (e.g., tetrahydrofurylcarbonyl, pyrrolidinocarbonyl) optionally substituted by 1 to 3 substituents selected from a C₁₋₆ alkyl group optionally substituted by 1 to 3 halogen atoms; and the like.

Examples of the "monocyclic ring" of the "optionally substituted monocyclic ring" formed by R² and R³ bonded to each other include a "monocyclic aromatic ring" and a "monocyclic non-aromatic ring".

Examples of the "monocyclic aromatic ring" include a monocyclic ring, from among a ring (aromatic hydrocarbon) corresponding to the C₆₋₁₄ aryl group exemplified as the "hydrocarbon group" of the above-mentioned "optionally substituted hydrocarbon group", and a monocyclic aromatic heterocycle corresponding to the monocyclic aromatic heterocyclic group exemplified as the "heterocyclic group" of the above-mentioned "optionally substituted heterocyclic group".

Examples of the "monocyclic non-aromatic ring" include a C₃₋₁₀ cycloalkane, C₃₋₁₀ cycloalkene and C₄₋₁₀ cycloalkadiene corresponding to the C₃₋₁₀ cycloalkyl group, C₃₋₁₀ cycloalkenyl group and C₄₋₁₀ cycloalkadienyl group exemplified as the "hydrocarbon group" of the above-mentioned "optionally substituted hydrocarbon group", and a monocyclic non-aromatic heterocycle corresponding to the monocyclic non-aromatic heterocyclic group exemplified as the "heterocyclic group" of the above-mentioned "optionally substituted heterocyclic group". The monocyclic non-aromatic ring is preferably a C₃₋₁₀ cycloalkane (preferably cyclohexane, cycloheptane), a C₃₋₁₀ cycloalkene (preferably cyclohexene), a C₄₋₁₀ cycloalkadiene (preferably cyclohexadiene), a 5- or 6-membered monocyclic non-aromatic heterocycle (preferably oxotetrahydropyridazine (preferably 3-oxo-2,3,4,5-tetrahydropyridazine)) or the like.

The "monocyclic ring" of the "optionally substituted monocyclic ring" formed by R² and R³ bonded to each other optionally has 1 to 3 substituents at substitutable positions. Examples of the substituent include those similar to the substituent that the C₃₋₁₀ cycloalkyl group and the like exemplified as the "substituent" for R² or R³ optionally has. When the number of the substituents is not less than 2, the respective substituents may be the same or different.

R² is preferably a hydrogen atom, an optionally substituted hydrocarbon group, an optionally substituted heterocyclic group, an acyl group or a cyano group, more preferably a hydrogen atom, an optionally substituted C₁₋₁₀ alkyl group, an optionally substituted C₂₋₁₀ alkenyl group, an optionally substituted aromatic heterocyclic group, an acyl group or a cyano group, particularly preferably an optionally substituted hydrocarbon group.
Preferable examples of R² include
(1) a hydrogen atom,
(2) a C₁₋₆ alkyl group (preferably methyl, ethyl, propyl, isobutyl, pentyl) optionally substituted by 1 to 3 substituents selected from
   (a) a hydroxy group,
   (b) a C₃₋₁₀ cycloalkyl group (preferably cyclopropyl, cyclohexyl) optionally substituted by 1 to 3 hydroxy groups,
   (c) a C₁₋₆ alkoxy group (preferably methoxy, ethoxy),
   (d) a C₆₋₁₄ aryl group (preferably phenyl) optionally substituted by 1 to 3 cyano groups,
   (e) a hydroxyimino group optionally substituted by a substituent selected from
      (i) a C₁₋₆ alkyl group (preferably methyl) optionally substituted by 1 to 3 substituents selected from a C₆₋₁₄ aryl group (preferably phenyl), a C₁₋₆ alkyl-carbonyl group (preferably acetyl) and a cyano group, and
      (ii) a C₆₋₁₄ aryl group (preferably phenyl),
   (f) a cyano group,
   (g) an imino group optionally substituted by a C₁₋₆ alkyl group (preferably methyl) optionally substituted by 1 to 3 cyano groups,
   (h) a hydrazono group,
   (i) a halogen atom (preferably a fluorine atom),
   (j) a 2,4-dioxo-1,3-thiazolidin-5-ylidene group,
   (k) a sulfamoyl group,
   (l) a C₆₋₁₄ aryloxy group (preferably phenyloxy), and
   (m) a carbamoyloxy group optionally mono- or di-substituted by C₁₋₆ alkyl group(s) (preferably methyl, ethyl),
(3) a C₂₋₆ alkenyl group (preferably vinyl, 1-propenyl, 2-methyl-1-propenyl, 1-pentenyl, 3-methylbutane-1,3-dienyl) optionally substituted by 1 to 3 substituents selected from
   (a) an aromatic heterocyclic group (preferably pyridyl),
   (b) a hydroxy group,
   (c) a cyano group,
   (d) a C₆₋₁₄ aryl group (preferably phenyl) optionally substituted by 1 to 3 cyano groups,
   (e) a carbamoyloxy group optionally mono- or di-substituted by substituent(s) selected from
      (i) a C₁₋₆ alkyl group (preferably methyl, ethyl) optionally substituted by 1 to 3 C₃₋₁₀ cycloalkyl groups (preferably cyclopropyl), and
      (ii) a C₃₋₁₀ cycloalkyl group (preferably cyclopropyl),
   (f) a sulfamoyl group optionally mono- or di-substituted by C₁₋₆ alkoxy-carbonyl group(s) (preferably t-butoxycarbonyl),
   (g) a C₃₋₁₀ cycloalkyl group (preferably cyclopropyl) optionally substituted by 1 to 3 hydroxy groups,
   (h) a C₁₋₆ alkoxy-carbonyl group (preferably t-butoxycarbonyl), and
   (i) a carboxy group,
(4) a C₁₋₆ alkyl-carbonyl group (preferably acetyl),
(5) a formyl group,
(6) a cyano group,
(7) an aromatic heterocyclic group (preferably isoxazolyl, oxadiazolyl, thiadiazolyl) optionally substituted by 1 to 3 substituents selected from
   (a) a C₁₋₆ alkyl group (preferably methyl, isopropyl) optionally substituted by 1 to 3 substituents selected from a hydroxy group and a tri-C₁₋₆ alkylsilyl group (preferably trimethylsilyl), and
   (b) a tri-C₁₋₆ alkylsilyl group (preferably trimethylsilyl),
(8) a carboxy group,
(9) a carbamoyl group optionally mono- or di-substituted by substituent(s) selected from
   (a) a C₁₋₆ alkyl group (preferably propyl) optionally substituted by 1 to 3 C₁₋₆ alkoxy groups (preferably methoxy), and
   (b) an amino group optionally mono- or di-substituted by C₁₋₆ alkoxy-carbonyl group(s) (preferably t-butoxycarbonyl), and
(10) a non-aromatic heterocyclylcarbonyl group (preferably pyrrolidinocarbonyl).

R³ is preferably a hydrogen atom, an optionally substituted hydrocarbon group, an acyl group or a cyano group, more preferably a hydrogen atom, an optionally substituted C₁₋₁₀ alkyl group, an optionally substituted C₃₋₁₀ cycloalkyl group, an optionally substituted C₆₋₁₄ aryl group, an acyl group or a cyano group. Of these, an optionally substituted hydrocarbon group is preferable.
Preferable examples of R³ include
(1) a hydrogen atom,
(2) a C₁₋₆ alkyl group (preferably methyl, ethyl, propyl, isopropyl) optionally substituted by 1 to 3 substituents selected from a halogen atom (preferably a fluorine atom), a C₁₋₆ alkoxy group (preferably methoxy) and a hydroxyimino group,
(3) a C₆₋₁₄ aryl group (preferably phenyl),
(4) a C₁₋₆ alkoxy-carbonyl group (preferably ethoxycarbonyl),
(5) a carbamoyl group optionally mono- or di-substituted by C₁₋₆ alkyl group(s) (preferably ethyl),
(6) a C₃₋₁₀ cycloalkyl group (preferably cyclopropyl),
(7) a cyano group, and
(8) a formyl group.

Alternatively, preferably, R² and R³ are bonded to each other to form an optionally substituted monocyclic aromatic hydrocarbon, an optionally substituted C₃₋₁₀ cycloalkane, an optionally substituted C₃₋₁₀ cycloalkene, an optionally substituted C₄₋₁₀ cycloalkadiene or an optionally substituted monocyclic non-aromatic heterocycle, more preferably
(1) a C₃₋₁₀ cycloalkane (preferably cyclohexane, cycloheptane),
(2) a C₃₋₁₀ cycloalkene (preferably cyclohexene),
(3) a C₄₋₁₀ cycloalkadiene (preferably cyclohexadiene), or
(4) a 5- or 6-membered monocyclic non-aromatic heterocycle (preferably oxotetrahydropyridazine (preferably 3-oxo-2,3,4,5-tetrahydropyridazine)) optionally substituted by 1 to 3 C₁₋₆ alkyl groups (preferably methyl).

R¹ is a hydrogen atom, an optionally substituted hydrocarbon group or an optionally substituted heterocyclic group.

Examples of the "optionally substituted hydrocarbon group" and "optionally substituted heterocyclic group" for R¹ include those similar to the "optionally substituted hydrocarbon group" and "optionally substituted heterocyclic group", which are exemplified as the "substituent" for R² or R³.

R¹ is preferably an optionally substituted hydrocarbon group, more preferably an optionally substituted C₁₋₁₀ alkyl group or an optionally substituted C₆₋₁₄ aryl group, further more preferably
(1) a C₁₋₆ alkyl group (preferably methyl, ethyl), or
(2) a C₆₋₁₄ aryl group (preferably phenyl) .

A is an optionally further substituted benzene ring.

The "benzene ring" for A optionally further has, besides group: -X- and group: W-Z-Y-, 1 to 4 substituents at substitutable positions. Examples of the substituent include those similar to the substituent that the C₃₋₁₀ cycloalkyl group and the like exemplified as the "substituent" for R² or R³ optionally has. When the number of the substituents is not less than 2, the respective substituents may be the same or different. Specific examples of the substituent include
(1) a halogen atom (preferably a chlorine atom, a bromine atom),
(2) a C₁₋₆ alkyl group (preferably methyl, ethyl, propyl, isopropyl) optionally substituted by 1 to 3 substituents selected from
   (a) a hydroxyimino group optionally substituted by a substituent selected from a C₁₋₆ alkyl group (preferably methyl, ethyl, t-butyl) optionally substituted by 1 to 3 C_{6- 14} aryl groups (preferably phenyl), and a C₆₋₁₄ aryl group (preferably phenyl),
   (b) a hydroxy group,
   (c) a cyano group,
   (d) a C₆₋₁₄ aryloxy group (preferably phenyloxy), and
   (e) a C₁₋₆ alkylsulfonyl group (preferably methylsulfonyl),
(3) a formyl group,
(4) a cyano group,
(5) a C₁₋₆ alkyl-carbonyl group (preferably acetyl),
(6) a carbamoyl group optionally mono- or di-substituted by C₁₋₆ alkyl group(s) (preferably methyl),
(7) a C₁₋₆ alkoxy group (preferably methoxy) optionally substituted by C₁₋₆ alkoxy group(s) (preferably methoxy),
(8) a C₃₋₁₀ cycloalkyl group (preferably cyclopropyl),
(9) a C₆₋₁₄ aryl group (preferably phenyl) optionally substituted by 1 to 3 substituents selected from
   (a) a halogen atom (preferably a fluorine atom), and
   (b) a cyano group,
(10) a dioxaborolanyl group (preferably 1,3,2-dioxaborolan-2-yl) optionally substituted by 1 to 4 C₁₋₆ alkyl groups (preferably methyl), and
(11) an aromatic heterocyclic group (preferably thiazolyl).

A is preferably a benzene ring optionally further substituted by 1 to 3 substituents selected from
(1) a halogen atom (preferably a chlorine atom, a bromine atom),
(2) a C₁₋₆ alkyl group (preferably methyl, ethyl, propyl, isopropyl) optionally substituted by 1 to 3 substituents selected from
   (a) a hydroxyimino group optionally substituted by a substituent selected from a C₁₋₆ alkyl group (preferably methyl, ethyl, t-butyl) optionally substituted by 1 to 3 C₆₋₁₄ aryl groups (preferably phenyl), and a C₆₋₁₄ aryl group (preferably phenyl),
   (b) a hydroxy group,
   (c) a cyano group,
   (d) a C₆₋₁₄ aryloxy group (preferably phenyloxy), and
   (e) a C₁₋₆ alkylsulfonyl group (preferably methylsulfonyl),
(3) a formyl group,
(4) a cyano group,
(5) a C₁₋₆ alkyl-carbonyl group (preferably acetyl),
(6) a carbamoyl group optionally mono- or di-substituted by C₁₋₆ alkyl group(s) (preferably methyl),
(7) a C₁₋₆ alkoxy group (preferably methoxy) optionally substituted by C₁₋₆ alkoxy group(s) (preferably methoxy),
(8) a C₃₋₁₀ cycloalkyl group (preferably cyclopropyl),
(9) a C₆₋₁₄ aryl group (preferably phenyl) optionally substituted by 1 to 3 substituents selected from
   (a) a halogen atom (preferably a fluorine atom), and
   (b) a cyano group,
(10) a dioxaborolanyl group (preferably 1,3,2-dioxaborolan-2-yl) optionally substituted by 1 to 4 C₁₋₆ alkyl groups (preferably methyl), and
(11) an aromatic heterocyclic group (preferably thiazolyl).

X is O, NR⁴ wherein R⁴ is a hydrogen atom or a C₁₋₆ alkyl group, S, SO, S(O)₂ or CH₂.
X is preferably O, NR⁴ wherein R⁴ is a C₁₋₆ alkyl group (preferably methyl), S or CH₂, more preferably O, S or CH₂, particularly preferably O or S.

Y is a bond, O, NR⁵ wherein R⁵ is a hydrogen atom or a C₁₋₆ alkyl group, S, SO or S(O)₂.
Y is preferably a bond, O or NH, more preferably a bond or O.

Z is a bond, or a divalent hydrocarbon group having 1 to 9 atoms in the main chain, which is optionally substituted.

The "main chain" is a divalent straight chain connecting group W and group Y, and the atom number of the main chain is counted such that the number of atoms in the main chain will be minimum. For example, the atom number of the main chain is counted as 2 for 1,2-phenylene, and the atom number of the main chain is counted as 3 for 1,3-phenylene. The total carbon atom number in the divalent hydrocarbon group is not particularly limited as long as the main chain consists of 1 to 9 carbon atoms, and the divalent hydrocarbon group optionally has 9 or more carbon atoms, preferably 1 to 20 carbon atoms, more preferably 1 to 9 carbon atoms, particularly preferably 1 to 6 carbon atoms. The hydrocarbon group may be saturated or unsaturated.

Specific examples of the "divalent hydrocarbon group" of the "divalent hydrocarbon group having 1 to 9 atoms in the main chain, which is optionally substituted" for Z include a "divalent acyclic hydrocarbon group", a "divalent cyclic hydrocarbon group", and a divalent group obtained by a combination of one or more kinds of "divalent acyclic hydrocarbon group" and one or more kinds of "divalent cyclic hydrocarbon group".

Examples of the "divalent acyclic hydrocarbon group" include a C₁₋₂₀ alkylene group (preferably C₁₋₆ alkylene group) having 1 to 9 carbon atoms in the main chain, a C₂₋₂₀ alkenylene group (preferably C₂₋₆ alkenylene group) having 2 to 9 carbon atoms in the main chain, a C₂₋₂₀ alkynylene group (preferably C₂₋₆ alkynylene group) having 2 to 9 carbon atoms in the main chain, and the like.

Examples of the "divalent cyclic hydrocarbon group" include a C₃₋₂₀ cycloalkanylene group (preferably C₃₋₆ cycloalkanylene group) having 1 to 9 carbon atoms in the main chain, a C₃₋₂₀ cycloalkenylene group (preferably C₃₋₆ cycloalkenylene group) having 1 to 9 carbon atoms in the main chain, a C₆₋₂₀ aromatic hydrocarbon group (preferably C₆₋₁₄ arylene group, more preferably phenylene) having 2 to 9 carbon atoms in the main chain, and the like. Specific examples thereof include 1,2-cyclopentylene, 1,3-cyclopentylene, 1,2-cyclohexylene, 1,3-cyclohexylene, 1,4-cyclohexylene, 1,2-cycloheptylene, 1,3-cycloheptylene, 1,4-cycloheptylene, 3-cyclohexen-1,4-ylene, 3-cyclohexen-1,2-ylene, 2,5-cyclohexadien-1,4-ylene, 1,2-phenylene, 1,3-phenylene, 1,4-phenylene, 1,4-naphthylene, 1,6-naphthylene, 2,6-naphthylene, 2,7-naphthylene, 1,5-indenylene, 2,5-indenylene and the like.

Preferable examples of the "divalent hydrocarbon group having 1 to 9 atoms in the main chain" include
(1) a C₁₋₆ alkylene group (e.g., -CH₂-, -(CH₂)₂-, -(CH₂)₃-, - (CH₂)₄-, -(CH₂)₅-, -(CH₂)₆-, -CH(CH3)-, -CH(C₂H₅)-, -CH(C₃H₇)-, - CH(i-C₃H₇)-, -CH(CH₃)CH₂-, -CH₂CH(CH₃)-, -CH(CH₃)(CH₂)₂-, - (CH₂)₂CH(CH₃)-, -CH₂-CH(CH₃)-CH₂-, -C(CH₃)₂-, -(CH(CH₃))₂-, - (CH₂)₂C(CH₃)₂-, -(CH₂)₃C(CH₃)₂-, -CH₂-CH(CH₃)-, -CH₂-C(CH₃)₂-);
(2) a C₂₋₆ alkenylene group (e.g., -CH=CH-, -CH=CH-CH₂-, -CH₂-CH=CH-, -C(CH₃)₂-CH=CH-, -CH₂-CH=CH-CH₂-, -CH₂-CH₂-CH=CH-, - CH=CH-CH=CH-, -CH=CH-CH₂-CH₂-CH₂-, -C(CH₃)=CH-, -CH=C(CH₃)-, - CH=C(C₂H₅)-) ;
(3) a C₂₋₆ alkynylene group (e.g., -CΞC-, -CH₂-CΞC-, -CH₂-CΞC-CH₂-CH₂-)
(4) a C₆₋₁₄ arylene group (e.g., 1,2-phenylene, 1,3-phenylene, 1,4-phenylene, 1,4-naphthylene, 1,6-naphthylene, 2,6-naphthylene, 2,7-naphthylene)
and the like.

The "divalent hydrocarbon group having 1 to 9 atoms in the main chain" of the "divalent hydrocarbon group having 1 to 9 atoms in the main chain, which is optionally substituted" for Z optionally has one or more substituents at substitutable positions. Examples of the substituent include those similar to the substituent that the C₃₋₁₀ cycloalkyl group and the like exemplified as the "substituent" for R² or R³ optionally has, and oxo group. When the number of the substituents is not less than 2, the respective substituents may be the same or different. Examples of the substituent include an oxo group and a halogen atom (preferably a bromine atom, a fluorine atom).

Z is preferably a bond, an optionally substituted C₁₋₆ alkylene group, an optionally substituted C₁₋₆ alkenylene group or an optionally substituted C₆₋₁₄ arylene group, more preferably
(1) a bond,
(2) a C₁₋₆ alkylene group (preferably -CH₂-, -CH(CH₃)-, - CH(C₂H₅)-, -CH(C₃H₇)-, -CH(i-C₃H₇)-, -C(CH₃)₂-, -CH₂CH₂-, - CH₂CH(CH₃)-, -CH(CH₃)CH₂-) optionally substituted by 1 to 3 substituents selected from an oxo group and a halogen atom (preferably a bromine atom, a fluorine atom),
(3) a C₂₋₆ alkenylene group (preferably -CH=CH-, -C(CH₃)=CH-, - CH-C(CH₃)-), or
(4) a C₆₋₁₄ arylene group (preferably phenylene).
Z is more preferably
(1) a C₁₋₆ alkylene group optionally substituted by 1 to 3 halogen atoms (preferably a bromine atom, a fluorine atom), or
(2) a C₂₋₆ alkenylene group,
particularly preferably a C₁₋₆ alkylene group or a C₂₋₆ alkenylene group.

W is -C(=O)Q wherein Q is an optionally substituted hydroxy group or an optionally substituted amino group, or a 5- or 6-membered heterocyclic group containing NH and optionally having substituent(s).

Examples of the "optionally substituted hydroxy group" and "optionally substituted amino group" for Q include those similar to the "optionally substituted hydroxy group" and "optionally substituted amino group", which are exemplified as the "substituent" for R² or R³.
Q is preferably
(1) a hydroxy group optionally substituted by a C₁₋₆ alkyl group (preferably methyl, ethyl, t-butyl), or
(2) an amino group optionally mono- or di-substituted by substituent(s) selected from
   (a) a C₁₋₆ alkyl group (preferably methyl, neopentyl) optionally substituted by 1 to 3 substituents selected from a hydroxy group and a C₃₋₁₀ cycloalkyl group (preferably cyclopropyl),
   (b) a C₃₋₁₀ cycloalkyl group (preferably cyclopropyl),
   (c) a C₁₋₆ alkylsulfonyl group (preferably methylsulfonyl, pentylsulfonyl),
   (d) a C₆₋₁₄ arylsulfonyl group (preferably phenylsulfonyl), and
   (e) a C₁₋₆ alkoxy group (preferably methoxy) .

The "5- or 6-membered heterocyclic group containing NH" in W is a 5- or 6-membered heterocyclic group containing, as a ring-constituting member, at least one unsubstituted NH (-NH-), and further containing, as ring-constituting atoms, 4 to 5 atoms selected from a carbon atom (the carbon atom is optionally substituted by an oxo group or a thioxo group), a oxygen atom, a sulfur atom (the sulfur atom is optionally oxidized) and a nitrogen atom. Examples thereof include a 5-or 6-membered aromatic heterocyclic group and a non-aromatic heterocyclic group, each of which contains NH.
Preferable examples of the "5- or 6-membered aromatic heterocyclic group containing NH" include pyrrolyl, imidazolyl, pyrazolyl, triazolyl, tetrazolyl and the like.
Preferable examples of "5- or 6-membered non-aromatic heterocyclic group containing NH" include pyrrolinyl, 2,5-dioxopyrrolinyl, pyrrolidinyl, 2-oxopyrrolidinyl, 2,5-dioxopyrrolidinyl, pyrazolinyl, pyrazolidinyl, imidazolinyl, imidazolidinyl, 2-oxoimidazolidinyl, 2,5-dioxo-imidazolidinyl, triazolinyl, triazolidinyl, tetrazolinyl, tetrazolidinyl, piperidinyl, 2,6-dioxopiperidinyl, piperazinyl, morpholinyl, thiomorpholinyl, piperazinyl, hexamethyleniminyl, oxazolinyl, oxazolidinyl, 2,4-dioxooxazolidinyl, thiazolinyl, thiazolidinyl, 4-oxothiazolidinyl, 2,4-dioxothiazolidinyl, 2-imino-4-oxothiazolidinyl, isoxazolinyl, isoxazolidinyl, isothiazolinyl, isothiazolidinyl, 1,1-dioxidoisothiazolidinyl, 1,1-dioxido-3-oxoisothiazolidinyl, dihydrooxadiazolyl, tetrahydrooxadiazolyl, oxooxadiazolinyl, dihydrothiadiazolyl, tetrahydrothiadiazolyl, dihydropyridyl, tetrahydropyridyl, dihydropyrimidinyl, tetrahydropyrimidinyl, 2,6-dioxohexahydropyrimidinyl, dihydropyridazinyl, tetrahydropyridazinyl, dihydropyrazinyl, tetrahydropyrazinyl, 1,1-dioxido-1,2-thiazinanyl, 1,1-dioxido-3-oxo-1,2-thiazinanyl and the like.

The "5- or 6-membered heterocyclic group containing NH" for W optionally has 1 to 3 substituents at substitutable positions. Examples of the substituent include those similar to the substituent that the C₃₋₁₀ cycloalkyl group and the like exemplified as the "substituent" for R² or R³ optionally has. When the number of the substituents is not less than 2, the respective substituents may be the same or different.

The "5- or 6-membered heterocyclic group containing NH and optionally having substituent(s)" for W is preferably tetrazolyl, oxooxadiazolinyl (preferably 5(4H)-oxo-1,2,4-oxadiazol-3-yl), 2-oxoimidazolidinyl (preferably 2-oxoimidazolidin-1-yl), 2,4-dioxothiazolidinyl (preferably 2,4-dioxo-1,3-thiazolidin-5-yl) or 2-imino-4-oxothiazolidinyl (preferably 2-imino-4-oxo-1,3-thiazolidin-5-yl).

W is preferably
(A) -C(=O)Q
   wherein Q is
   (1) a hydroxy group optionally substituted by a C₁₋₆ alkyl group (preferably methyl, ethyl, t-butyl), or
   (2) an amino group optionally mono- or di-substituted by substituent(s) selected from
      (a) a C₁₋₆ alkyl group (preferably methyl, neopentyl) optionally substituted by 1 to 3 substituents selected from a hydroxy group and a C₃₋₁₀ cycloalkyl group (preferably cyclopropyl),
      (b) a C₃₋₁₀ cycloalkyl group (preferably cyclopropyl),
      (c) a C₁₋₆ alkylsulfonyl group (preferably methylsulfonyl, pentylsulfonyl),
      (d) a C₆₋₁₄ arylsulfonyl group (preferably phenylsulfonyl), and
      (e) a C₁₋₆ alkoxy group (preferably methoxy), or
(B) a 5- or 6-membered heterocyclic group containing NH [preferably tetrazolyl, oxooxadiazolinyl (preferably 5(4H)-oxo-1,2,4-oxadiazol-3-yl), 2-oxoimidazolidinyl (preferably 2-oxoimidazolidin-1-yl), 2,4-dioxothiazolidinyl (preferably 2,4-dioxo-1,3-thiazolidin-5-yl) or 2-imino-4-oxothiazolidinyl (preferably 2-imino-4-oxo-1,3-thiazolidin-5-yl)].
W is more preferable -C(=O)Q.

In compound (I),
1) when Y is a bond, and Z is a bond, then W should be a 5- or 6-membered heterocyclic group containing NH and optionally having substituent(s) (excluding a 5-phenylimino-4,5-dihydro-1,3,4-thiadiazolyl group), and the heterocyclic group should not be uracil, thioxooxadiazolinyl and thioxotriazolinyl, each of which is optionally substituted;
2) when -Z-Y- is

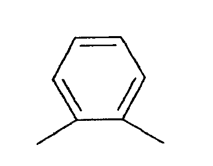

or

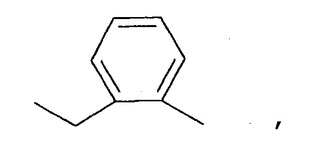

then X should not be CH₂;
3) -Z-Y- is not

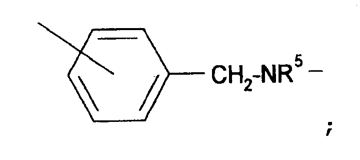

and
4) when Y is NR⁵, and Z is a bond, then W should be a 5- or 6-membered heterocyclic group containing NH and optionally having substituent(s).

Preferable examples of compound (I) include the following compounds and a salt thereof.
[Compound A]
Compound (I) wherein
A is an optionally further substituted benzene ring;
R¹ is an optionally substituted hydrocarbon group;
R² is an optionally substituted hydrocarbon group;
R³ is an optionally substituted hydrocarbon group;
X is O or S;
Y is a bond or O;
Z is a C₁₋₆ alkylene group or a C₂₋₆ alkenylene group; and
W is -C(=O)Q wherein Q is as defined above.

[Compound B]
Compound (I) wherein
A is a benzene ring optionally further substituted by 1 to 3 halogen atoms (preferably a chlorine atom);
R¹ is
   (1) a C₁₋₆ alkyl group (preferably methyl, ethyl), or
   (2) a C₆₋₁₄ aryl group (preferably phenyl);
R² is
   (1) a hydrogen atom,
   (2) a C₁₋₆ alkyl group (preferably methyl, ethyl, propyl, isobutyl, pentyl) optionally substituted by 1 to 3 substituents selected from
      (a) a hydroxy group,
      (b) a C₃₋₁₀ cycloalkyl group (preferably cyclohexyl),
      (c) a C₁₋₆ alkoxy group (preferably methoxy, ethoxy),
      (d) a C₆₋₁₄ aryl group (preferably phenyl),
      (e) a hydroxyimino group optionally substituted by a C₁₋₆ alkyl group (preferably methyl) optionally substituted by 1 to 3 C₆₋₁₄ aryl groups (preferably phenyl), and
      (f) a cyano group,
   (3) a C₂₋₆ alkenyl group (preferably vinyl, 1-propenyl, 2-methyl-1-propenyl, 1-pentenyl) optionally substituted by aromatic heterocyclic group(s) (preferably pyridyl),
   (4) a C₁₋₆ alkyl-carbonyl group (preferably acetyl),
   (5) a formyl group, or
   (6) a cyano group;
R³ is
   (1) a hydrogen atom,
   (2) a C₁₋₆ alkyl group (preferably methyl, ethyl, propyl, isopropyl) optionally substituted by 1 to 3 substituents selected from a halogen atom (preferably a fluorine atom) and a C₁₋₆ alkoxy group (preferably methoxy),
   (3) a C₆₋₁₄ aryl group (preferably phenyl),
   (4) a C₁₋₆ alkoxy-carbonyl group (preferably ethoxycarbonyl), or
   (5) a carbamoyl group optionally mono- or di-substituted by C₁₋₆ alkyl group(s) (preferably ethyl); or
R² and R³ are optionally bonded to each other to form
   (1) a C₃₋₁₀ cycloalkane (preferably cyclohexane, cycloheptane),
   (2) a C₃₋₁₀ cycloalkene (preferably cyclohexene),
   (3) a C₄₋₁₀ cycloalkadiene (preferably cyclohexadiene), or
   (4) a 5- or 6-membered monocyclic non-aromatic heterocycle (preferably oxotetrahydropyridazine (preferably 3-oxo-2,3,4,5-tetrahydropyridazine)) optionally substituted by 1 to 3 C₁₋₆ alkyl groups (preferably methyl);
X is O, NR⁴ wherein R⁴ is a C₁₋₆ alkyl group (preferably methyl), or S;
Y is a bond, O or NH;
Z is
   (1) a bond,
   (2) a C₁₋₆ alkylene group (preferably -CH₂-, -CH(CH₃)-, - CH(C₂H₅)-, -CH(C₃H₇)-, -CH(i-C₃H₇)-, -C(CH₃)₂-, -CH₂CH₂-, - CH₂CH(CH₃)-, -CH(CH₃)CH₂-) optionally substituted by 1 to 3 oxo groups,
   (3) a C₂₋₆ alkenylene group (preferably -CH=CH-, -C(CH₃)=CH-, - CH=C(CH₃)-), or
   (4) a C₆₋₁₄ arylene group (preferably phenylene); and
W is
   (A) -C(=O)Q
wherein Q is
   (1) a hydroxy group optionally substituted by a C₁₋₆ alkyl group, or
   (2) an amino group optionally mono- or di-substituted by substituent(s) selected from
      (a) a C₁₋₆ alkyl group (preferably methyl, neopentyl) optionally substituted by 1 to 3 substituents selected from a hydroxy group and a C₃₋₁₀ cycloalkyl group (preferably cyclopropyl),
      (b) a C₃₋₁₀ cycloalkyl group (preferably cyclopropyl),
      (c) a C₁₋₆ alkylsulfonyl group (preferably methylsulfonyl, pentylsulfonyl), and
      (d) a C₆₋₁₄ arylsulfonyl group (preferably phenylsulfonyl), or

   (B) a 5- or 6-membered non-aromatic heterocyclic group containing NH [preferably oxooxadiazolinyl (preferably 5(4H)-oxo-1,2,4-oxadiazol-3-yl), 2-oxoimidazolidinyl (preferably 2-oxoimidazolidin-1-yl)].

[Compound C]
Compound B wherein
R¹ is
   (1) a C₁₋₆ alkyl group (preferably methyl, ethyl), or
   (2) a C₆₋₁₄ aryl group (preferably phenyl) ;
R² is a C₁₋₆ alkyl group (preferably methyl, ethyl, propyl, isobutyl, pentyl) optionally substituted by 1 to 3 substituents selected from
   (a) a hydroxy group,
   (b) a C₃₋₁₀ cycloalkyl group (preferably cyclohexyl),
   (c) a C₁₋₆ alkoxy group (preferably methoxy, ethoxy),
   (d) a C₆₋₁₄ aryl group (preferably phenyl),
   (e) a hydroxyimino group optionally substituted by a C₁₋₆ alkyl group (preferably methyl) optionally substituted by 1 to 3 C₆₋₁₄ aryl groups (preferably phenyl), and
   (f) a cyano group;
R³ is
   (1) a C₁₋₆ alkyl group (preferably methyl, ethyl, propyl, isopropyl) optionally substituted by 1 to 3 substituents selected from a halogen atom (preferably a fluorine atom) and a C₁₋₆ alkoxy group (preferably methoxy), or
   (2) a C₆₋₁₄ aryl group (preferably phenyl);
X is O or S;
Y is a bond or O;
Z is
   (1) a C₁₋₆ alkylene group (preferably -CH₂-, -CH(CH₃)-, - CH(C₂H₅)-, -CH(C₃H₇)-, -CH(i-C₃H₇)-, -C(CH₃)₂-, -CH₂CH₂-, - CH₂CH(CH₃)-, -CH(CH₃)CH₂-), or
   (2) a C₂₋₆ alkenylene group (preferably -CH=CH-, -C(CH₃)=CH-, - CH=C(CH₃)-); and
W is -C(=O)Q
wherein Q is
   (1) a hydroxy group optionally substituted by a C₁₋₆ alkyl group, or
   (2) an amino group optionally mono- or di-substituted by substituent(s) selected from
      (a) a C₁₋₆ alkyl group (preferably methyl, neopentyl) optionally substituted by 1 to 3 substituents selected from a hydroxy group and a C₃₋₁₀ cycloalkyl group (preferably cyclopropyl),
      (b) a C₃₋₁₀ cycloalkyl group (preferably cyclopropyl),
      (c) a C₁₋₆ alkylsulfonyl group (preferably methylsulfonyl, pentylsulfonyl), and
      (d) a C₆₋₁₄ arylsulfonyl group (preferably phenylsulfonyl) .

[Compound BB]
Compound (I) wherein
A is a benzene ring optionally further substituted by 1 to 3 substituents selected from
   (1) a halogen atom (preferably a chlorine atom),
   (2) a C₁₋₆ alkyl group (preferably methyl, ethyl, isopropyl) optionally substituted by 1 to 3 substituents selected from
      (a) a hydroxyimino group optionally substituted by a substituent selected from a C₁₋₆ alkyl group (preferably methyl, ethyl, t-butyl) optionally substituted by 1 to 3 C₆₋₁₄ aryl groups (preferably phenyl), and a C₆₋₁₄ aryl group (preferably phenyl),
      (b) a hydroxy group, and
      (c) a cyano group,
   (3) a formyl group,
   (4) a cyano group,
   (5) a C₁₋₆ alkyl-carbonyl group (preferably acetyl), and
   (6) a carbamoyl group optionally mono- or di-substituted by C₁₋₆ alkyl group(s) (preferably methyl);
R¹ is
   (1) a C₁₋₆ alkyl group (preferably methyl, ethyl), or
   (2) a C₆₋₁₄ aryl group (preferably phenyl);
R² is
   (1) a hydrogen atom,
   (2) a C₁₋₆ alkyl group (preferably methyl, ethyl, propyl, isobutyl, pentyl) optionally substituted by 1 to 3 substituents selected from
      (a) a hydroxy group,
      (b) a C₃₋₁₀ cycloalkyl group (preferably cyclohexyl),
      (c) a C₁₋₆ alkoxy group (preferably methoxy, ethoxy),
      (d) a C₆₋₁₄ aryl group (preferably phenyl),
      (e) a hydroxyimino group optionally substituted by a C₁₋₆ alkyl group (preferably methyl) optionally substituted by 1 to 3 substituents selected from a C₆₋₁₄ aryl group (preferably phenyl), a C₁₋₆ alkyl-carbonyl group (preferably acetyl) and a cyano group,
      (f) a cyano group,
      (g) an imino group optionally substituted by a C₁₋₆ alkyl group (preferably methyl) optionally substituted by 1 to 3 cyano groups, and
      (h) a hydrazono group,
   (3) a C₂₋₆ alkenyl group (preferably vinyl, 1-propenyl, 2-methyl-1-propenyl, 1-pentenyl) optionally substituted by 1 to 3 substituents selected from
      (a) an aromatic heterocyclic group (preferably pyridyl),
      (b) a hydroxy group,
      (c) a cyano group,
      (d) a C₆₋₁₄ aryl group (preferably phenyl) optionally substituted by 1 to 3 cyano groups, and
      (e) a carbamoyloxy group optionally mono- or di-substituted by C₁₋₆ alkyl group(s) (preferably ethyl),
   (4) a C₁₋₆ alkyl-carbonyl group (preferably acetyl),
   (5) a formyl group,
   (6) a cyano group, or
   (7) an aromatic heterocyclic group (preferably isoxazolyl) optionally substituted by 1 to 3 substituents selected from
      (a) a C₁₋₆ alkyl group (preferably methyl) optionally substituted by 1 to 3 substituents selected from a hydroxy group and a tri-C₁₋₆ alkylsilyl group (preferably trimethylsilyl), and
      (b) a tri-C₁₋₆ alkylsilyl group (preferably trimethylsilyl);
R³ is
   (1) a hydrogen atom,
   (2) a C₁₋₆ alkyl group (preferably methyl, ethyl, propyl, isopropyl) optionally substituted by 1 to 3 substituents selected from a halogen atom (preferably a fluorine atom), a C₁₋₆ alkoxy group (preferably methoxy) and a hydroxyimino group,
   (3) a C₆₋₁₄ aryl group (preferably phenyl),
   (4) a C₁₋₆ alkoxy-carbonyl group (preferably ethoxycarbonyl),
   (5) a carbamoyl group optionally mono- or di-substituted by C₁₋₆ alkyl group(s) (preferably ethyl),
   (6) a C₃₋₁₀ cycloalkyl group (preferably cyclopropyl),
   (7) a cyano group, or
   (8) a formyl group; or
R² and R³ are optionally bonded to each other to form
   (1) a C₃₋₁₀ cycloalkane (preferably cyclohexane, cycloheptane),
   (2) a C₃₋₁₀ cycloalkene (preferably cyclohexene),
   (3) a C₄₋₁₀ cycloalkadiene (preferably cyclohexadiene), or
   (4) a 5- or 6-membered monocyclic non-aromatic heterocycle (preferably oxotetrahydropyridazine (preferably 3-oxo-2,3,4,5-tetrahydropyridazine)) optionally substituted by 1 to 3 C₁₋₆ alkyl groups (preferably methyl);
X is O, NR⁴ wherein R⁴ is a C₁₋₆ alkyl group (preferably methyl), S or CH₂;
Y is a bond, O or NH;
Z is
   (1) a bond,
   (2) a C₁₋₆ alkylene group (preferably -CH₂-, -CH(CH₃)-, - CH(C₂H₅)-, -CH(C₃H₇)-, -CH(i-C₃H₇)-, -C(CH₃)₂-, -CH₂CH₂-, - CH₂CH(CH₃)-, -CH(CH₃)CH₂-) optionally substituted by 1 to 3 substituents selected from an oxo group and a halogen atom (preferably a bromine atom),
   (3) a C₂₋₆ alkenylene group (preferably -CH=CH-, -C(CH₃)=CH-, - CH=C(CH₃)-), or
   (4) a C₆₋₁₄ arylene group (preferably phenylene); and
W is
   (A) -C(=O)Q
wherein Q is
   (1) a hydroxy group optionally substituted by a C₁₋₆ alkyl group (preferably methyl, ethyl), or
   (2) an amino group optionally mono- or di-substituted by substituent(s) selected from
      (a) a C₁₋₆ alkyl group (preferably methyl, neopentyl) optionally substituted by 1 to 3 substituents selected from a hydroxy group and a C₃₋₁₀ cycloalkyl group (preferably cyclopropyl),
      (b) a C₃₋₁₀ cycloalkyl group (preferably cyclopropyl),
      (c) a C₁₋₆ alkylsulfonyl group (preferably methylsulfonyl, pentylsulfonyl), and
      (d) a C₆₋₁₄ arylsulfonyl group (preferably phenylsulfonyl), or

   (B) a 5- or 6-membered heterocyclic group containing NH [preferably tetrazolyl, oxooxadiazolinyl (preferably 5(4H)-oxo-1,2,4-oxadiazol-3-yl), 2-oxoimidazolidinyl (preferably 2-oxoimidazolidin-1-yl), 2,4-dioxothiazolidinyl (preferably 2,4-dioxo-1,3-thiazolidin-5-yl) or 2-imino-4-oxothiazolidinyl (preferably 2-imino-4-oxo-1,3-thiazolidin-5-yl)].

[Compound CC]
Compound BB wherein
R¹ is
   (1) a C₁₋₆ alkyl group (preferably methyl, ethyl), or
   (2) a C₆₋₁₄ aryl group (preferably phenyl);
R²is a C₁₋₆ alkyl group (preferably methyl, ethyl, propyl, isobutyl, pentyl) optionally substituted by 1 to 3 substituents selected from
   (a) a hydroxy group,
   (b) a C₃₋₁₀ cycloalkyl group (preferably cyclohexyl),
   (c) a C₁₋₆ alkoxy group (preferably methoxy, ethoxy),
   (d) a C₆₋₁₄ aryl group (preferably phenyl),
   (e) a hydroxyimino group optionally substituted by a C₁₋₆ alkyl group (preferably methyl) optionally substituted by 1 to 3 substituents selected from a C₆₋₁₄ aryl group (preferably phenyl), a C₁₋₆ alkyl-carbonyl group (preferably acetyl) and a cyano group,
   (f) a cyano group,
   (g) an imino group optionally substituted by a C₁₋₆ alkyl group (preferably methyl) optionally substituted by 1 to 3 cyano groups, and
   (h) a hydrazono group;
R³ is
   (1) a C₁₋₆ alkyl group (preferably methyl, ethyl, propyl, isopropyl) optionally substituted by 1 to 3 substituents selected from a halogen atom (preferably a fluorine atom), a C₁₋₆ alkoxy group (preferably methoxy) and a hydroxyimino group,
   (2) a C₆₋₁₄ aryl group (preferably phenyl), or
   (3) a C₃₋₁₀ cycloalkyl group (preferably cyclopropyl);
X is O, S or CH₂;
Y is a bond or O;
Z is
   (1) a C₁₋₆ alkylene group (preferably -CH₂-, -CH(CH₃)-, - CH(C₂H₅)-, -CH(C₃H₇)-, -CH(i-C₃H₇)-, -C(CH₃)₂-, -CH₂CH₂-, - CH₂CH(CH₃)-, -CH(CH₃)CH₂-) optionally substituted by 1 to 3 halogen atoms (preferably a bromine atom), or
   (2) a C₂₋₆ alkenylene group (preferably -CH=CH-, -C(CH₃)=CH-, - CH=C(CH₃)-); and
W is -C(=O)Q
wherein Q is
   (1) a hydroxy group optionally substituted by a C₁₋₆ alkyl group (preferably methyl, ethyl), or
   (2) an amino group optionally mono- or di-substituted by substituent(s) selected from
      (a) a C₁₋₆ alkyl group (preferably methyl, neopentyl) optionally substituted by 1 to 3 substituents selected from a hydroxy group and a C₃₋₁₀ cycloalkyl group (preferably cyclopropyl),
      (b) a C₃₋₁₀ cycloalkyl group (preferably cyclopropyl),
      (c) a C₁₋₆ alkylsulfonyl group (preferably methylsulfonyl, pentylsulfonyl), and
      (d) a C₆₋₁₄ arylsulfonyl group (preferably phenylsulfonyl).

[Compound BBB]
Compound (I) wherein
A is a benzene ring optionally further substituted by 1 to 3 substituents selected from
   (1) a halogen atom (preferably a chlorine atom, a bromine atom),
   (2) a C₁₋₆ alkyl group (preferably methyl, ethyl, propyl, isopropyl) optionally substituted by 1 to 3 substituents selected from
      (a) a hydroxyimino group optionally substituted by a substituent selected from a C₁₋₆ alkyl group (preferably methyl, ethyl, t-butyl) optionally substituted by 1 to 3 C₆₋₁₄ aryl groups (preferably phenyl), and C₆₋₁₄ aryl group (preferably phenyl),
      (b) a hydroxy group,
      (c) a cyano group,
      (d) a C₆₋₁₄ aryloxy group (preferably phenyloxy), and
      (e) a C₁₋₆ alkylsulfonyl group (preferably methylsulfonyl),
   (3) a formyl group,
   (4) a cyano group,
   (5) a C₁₋₆ alkyl-carbonyl group (preferably acetyl),
   (6) a carbamoyl group optionally mono- or di-substituted by C₁₋₆ alkyl group(s) (preferably methyl),
   (7) a C₁₋₆ alkoxy group (preferably methoxy) optionally substituted by C₁₋₆ alkoxy group(s) (preferably methoxy),
   (8) a C₃₋₁₀ cycloalkyl group (preferably cyclopropyl),
   (9) a C₆₋₁₄ aryl group (preferably phenyl) optionally substituted by 1 to 3 substituents selected from
      (a) a halogen atom (preferably a fluorine atom), and
      (b) a cyano group,
   (10) a dioxaborolanyl group (preferably 1,3,2-dioxaborolan-2-yl) optionally substituted by 1 to 4 C₁₋₆ alkyl groups (preferably methyl), and
   (11) an aromatic heterocyclic group (preferably thiazolyl);
R¹ is
   (1) a C₁₋₆ alkyl group (preferably methyl, ethyl), or
   (2) a C₆₋₁₄ aryl group (preferably phenyl);
R² is
   (1) a hydrogen atom,
   (2) a C₁₋₆ alkyl group (preferably methyl, ethyl, propyl, isobutyl, pentyl) optionally substituted by 1 to 3 substituents selected from
      (a) a hydroxy group,
      (b) a C₃₋₁₀ cycloalkyl group (preferably cyclopropyl, cyclohexyl) optionally substituted by 1 to 3 hydroxy groups,
      (c) a C₁₋₆ alkoxy group (preferably methoxy, ethoxy),
      (d) a C₆₋₁₄ aryl group (preferably phenyl) optionally substituted by 1 to 3 cyano groups,
      (e) a hydroxyimino group optionally substituted by a substituent selected from
         (i) a C₁₋₆ alkyl group (preferably methyl) optionally substituted by 1 to 3 substituents selected from a C₆₋₁₄ aryl group (preferably phenyl), a C₁₋₆ alkyl-carbonyl group (preferably acetyl) and a cyano group, and
         (ii) a C₆₋₁₄ aryl group (preferably phenyl),
      (f) a cyano group,
      (g) an imino group optionally substituted by a C₁₋₆ alkyl group (preferably methyl) optionally substituted by 1 to 3 cyano groups,
      (h) a hydrazono group,
      (i) a halogen atom (preferably a fluorine atom),
      (j) a 2,4-dioxo-1,3-thiazolidin-5-ylidene group,
      (k) a sulfamoyl group,
      (l) a C₆₋₁₄ aryloxy group (preferably phenyloxy), and
      (m) a carbamoyloxy group optionally mono- or di-substituted by C₁₋₆ alkyl group(s) (preferably methyl, ethyl),
   (3) a C₂₋₆ alkenyl group (preferably vinyl, 1-propenyl, 2-methyl-1-propenyl, 1-pentenyl, 3-methylbutane-1,3-dienyl) optionally substituted by 1 to 3 substituents selected from
      (a) an aromatic heterocyclic group (preferably pyridyl),
      (b) a hydroxy group,
      (c) a cyano group,
      (d) a C₆₋₁₄ aryl group (preferably phenyl) optionally substituted by 1 to 3 cyano groups,
      (e) a carbamoyloxy group optionally mono- or di-substituted by substituent(s) selected from
         (i) a C₁₋₆ alkyl group (preferably methyl, ethyl) optionally substituted by 1 to 3 C₃₋₁₀ cycloalkyl groups (preferably cyclopropyl), and
         (ii) a C₃₋₁₀ cycloalkyl group (preferably cyclopropyl),
      (f) a sulfamoyl group optionally mono- or di-substituted by C₁₋₆ alkoxy-carbonyl group(s) (preferably t-butoxycarbonyl),
      (g) a C₃₋₁₀ cycloalkyl group (preferably cyclopropyl) optionally substituted by 1 to 3 hydroxy groups,
      (h) a C₁₋₆ alkoxy-carbonyl group (preferably t-butoxycarbonyl), and
      (i) a carboxy group,
   (4) a C₁₋₆ alkyl-carbonyl group (preferably acetyl),
   (5) a formyl group,
   (6) a cyano group,
   (7) an aromatic heterocyclic group (preferably isoxazolyl, oxadiazolyl, thiadiazolyl) optionally substituted by 1 to 3 substituents selected from
      (a) a C₁₋₆ alkyl group (preferably methyl, isopropyl) optionally substituted by 1 to 3 substituents selected from a hydroxy group and a tri-C₁₋₆ alkylsilyl group (preferably trimethylsilyl), and
      (b) a tri-C₁₋₆ alkylsilyl group (preferably trimethylsilyl),
   (8) a carboxy group,
   (9) a carbamoyl group optionally mono- or di-substituted by substituent(s) selected from
      (a) a C₁₋₆ alkyl group (preferably propyl) optionally substituted by 1 to 3 C₁₋₆ alkoxy groups (preferably methoxy), and
      (b) an amino group optionally mono- or di-substituted by C₁₋₆ alkoxy-carbonyl group(s) (preferably t-butoxycarbonyl), or
   (10) a non-aromatic heterocyclylcarbonyl group (preferably pyrrolidinocarbonyl);
R³ is
   (1) a hydrogen atom,
   (2) a C₁₋₆ alkyl group (preferably methyl, ethyl, propyl, isopropyl) optionally substituted by 1 to 3 substituents selected from a halogen atom (preferably a fluorine atom), a C₁₋₆ alkoxy group (preferably methoxy) and a hydroxyimino group,
   (3) a C₆₋₁₄ aryl group (preferably phenyl),
   (4) a C₁₋₆ alkoxy-carbonyl group (preferably ethoxycarbonyl),
   (5) a carbamoyl group optionally mono- or di-substituted by C₁₋₆ alkyl group(s) (preferably ethyl),
   (6) a C₃₋₁₀ cycloalkyl group (preferably cyclopropyl),
   (7) a cyano group, or
   (8) a formyl group; or
R² and R³ are optionally bonded to each other to form
   (1) a C₃₋₁₀ cycloalkane (preferably cyclohexane, cycloheptane),
   (2) a C₃₋₁₀ cycloalkene (preferably cyclohexene),
   (3) a C₄₋₁₀ cycloalkadiene (preferably cyclohexadiene), or
   (4) a 5- or 6-membered monocyclic non-aromatic heterocycle (preferably oxotetrahydropyridazine (preferably 3-oxo-2,3,4,5-tetrahydropyridazine)) optionally substituted by 1 to 3 C₁₋₆ alkyl groups (preferably methyl);
X is O, NR⁴ wherein R⁴ is a C₁₋₆ alkyl group (preferably methyl), S or CH₂;
Y is a bond, O or NH;
Z is
   (1) a bond,
   (2) a C₁₋₆ alkylene group (preferably -CH₂-, -CH(CH₃)-, - CH(C₂H₅)-, -CH(C₃H₇)-, -CH(i-C₃H₇)-, -C(CH₃)₂-, -CH₂CH₂-, CH₂CH(CH₃)-, -CH(CH₃)CH₂-) optionally substituted by 1 to 3 substituents selected from an oxo group and a halogen atom (preferably a bromine atom, a fluorine atom),
   (3) a C₂₋₆ alkenylene group (preferably -CH=CH-, -C(CH₃)=CH-, - CH=C(CH₃)-), or
   (4) a C₆₋₁₄ arylene group (preferably phenylene); and
W is
   (A) -C(=O)Q
wherein Q is
   (1) a hydroxy group optionally substituted by a C₁₋₆ alkyl group (preferably methyl, ethyl, t-butyl), or
   (2) an amino group optionally mono- or di-substituted by substituent(s) selected from
      (a) a C₁₋₆ alkyl group (preferably methyl, neopentyl) optionally substituted by 1 to 3 substituents selected from a hydroxy group and a C₃₋₁₀ cycloalkyl group (preferably cyclopropyl),
      (b) a C₃₋₁₀ cycloalkyl group (preferably cyclopropyl),
      (c) a C₁₋₆ alkylsulfonyl group (preferably methylsulfonyl, pentylsulfonyl),
      (d) a C₆₋₁₄ arylsulfonyl group (preferably phenylsulfonyl), and
      (e) a C₁₋₆ alkoxy group (preferably methoxy),
   or
   (B) 5- or 6-membered heterocyclic group containing NH [preferably tetrazolyl, oxooxadiazolinyl (preferably 5(4H)-oxo-1,2,4-oxadiazol-3-yl), 2-oxoimidazolidinyl (preferably 2-oxoimidazolidin-1-yl), 2,4-dioxothiazolidinyl (preferably 2,4-dioxo-1,3-thiazolidin-5-yl) or 2-imino-4-oxothiazolidinyl (preferably 2-imino-4-oxo-1,3-thiazolidin-5-yl)].

[Compound CCC]
Compound BBB wherein
R¹ is
   (1) a C₁₋₆ alkyl group (preferably methyl, ethyl), or
   (2) a C₆₋₁₄ aryl group (preferably phenyl);
R² is
   (1) a C₁₋₆ alkyl group (preferably methyl, ethyl, propyl, isobutyl, pentyl) optionally substituted by 1 to 3 substituents selected from
      (a) a hydroxy group,
      (b) a C₃₋₁₀ cycloalkyl group (preferably cyclopropyl, cyclohexyl) optionally substituted by 1 to 3 hydroxy groups,
      (c) a C₁₋₆ alkoxy group (preferably methoxy, ethoxy),
      (d) a C₆₋₁₄ aryl group (preferably phenyl) optionally substituted by 1 to 3 cyano groups,
      (e) a hydroxyimino group optionally substituted by a substituent selected from
         (i) a C₁₋₆ alkyl group (preferably methyl) optionally substituted by 1 to 3 substituents selected from a C₆₋₁₄ aryl group (preferably phenyl), a C₁₋₆ alkyl-carbonyl group (preferably acetyl) and a cyano group, and
         (ii) a C₆₋₁₄ aryl group (preferably phenyl),
      (f) a cyano group,
      (g) an imino group optionally substituted by a C₁₋₆ alkyl group (preferably methyl) optionally substituted by 1 to 3 cyano groups,
      (h) a hydrazono group,
      (i) a halogen atom (preferably a fluorine atom),
      (j) a 2,4-dioxo-1,3-thiazolidin-5-ylidene group,
      (k) a sulfamoyl group,
      (l) a C₆₋₁₄ aryloxy group (preferably phenyloxy), and
      (m) a carbamoyloxy group optionally mono- or di-substituted by C₁₋₆ alkyl group(s) (preferably methyl, ethyl), or
   (2) a cyano group;
R³ is
   (1) a C₁₋₆ alkyl group (preferably methyl, ethyl, propyl, isopropyl) optionally substituted by 1 to 3 substituents selected from a halogen atom (preferably a fluorine atom), a C₁₋₆ alkoxy group (preferably methoxy) and a hydroxyimino group,
   (2) a C₆₋₁₄ aryl group (preferably phenyl), or
   (3) a C₃₋₁₀ cycloalkyl group (preferably cyclopropyl);
X is O, S or CH₂ (preferably O or S);
Y is a bond or O;
Z is
   (1) a C₁₋₆ alkylene group (preferably -CH₂-, -CH(CH₃)-, - CH(C₂H₅)-, -CH(C₃H₇)-, -CH(i-C₃H₇)-, -C(CH₃)₂-, -CH₂CH₂-, - CH₂CH(CH₃)-, -CH(CH₃)CH₂-) optionally substituted by 1 to 3 halogen atoms (preferably a bromine atom, a fluorine atom), or
   (2) a C₂₋₆ alkenylene group (preferably -CH=CH-, -C(CH₃)=CH-, - CH=C(CH₃)-); and
W is -C(=O)Q
wherein Q is
   (1) a hydroxy group optionally substituted by a C₁₋₆ alkyl group (preferably methyl, ethyl, t-butyl), or
   (2) an amino group optionally mono- or di-substituted by substituent(s) selected from
      (a) a C₁₋₆ alkyl group (preferably methyl, neopentyl) optionally substituted by 1 to 3 substituents selected from a hydroxy group and a C₃₋₁₀ cycloalkyl group (preferably cyclopropyl),
      (b) a C₃₋₁₀ cycloalkyl group (preferably cyclopropyl),
      (c) a C₁₋₆ alkylsulfonyl group (preferably methylsulfonyl, pentylsulfonyl),
      (d) a C₆₋₁₄ arylsulfonyl group (preferably phenylsulfonyl), and
      (e) a C₁₋₆ alkoxy group (preferably methoxy) .

[Compound D]
(2S)-2-{2,4-dichloro-5-[(1,3,4-trimethyl-1H-pyrazol-5-yl)oxy]phenoxy}propionic acid (Example 1),
(2S)-2-{2,4-dichloro-5-[(1,3-dimethyl-4-vinyl-1H-pyrazol-5-yl)oxy]phenoxy}propionic acid (Example 14),
(2S)-2-{2,4-dichloro-5-[(2-methyl-2H-indazol-3-yl)oxy]phenoxy}propionic acid (Example 30),
(2S)-2-{2,4-dichloro-5-[(2-methyl-4,5,6,7-tetrahydro-2H-indazol-3-yl)oxy]phenoxy}propionic acid (Example 31),
(2S)-2-{2,4-dichloro-5-[(4-cyano-1,3-dimethyl-1H-pyrazol-5-yl)thio]phenoxylpropionic acid (Example 210),
(2S)-2-{2-bromo-5-[(4-cyano-3-cyclopropyl-1-methyl-1H-pyrazol-5-yl)oxy]-4-methylphenoxy}propionic acid (Example 256), or
(2S)-2-(2,4-dichloro-5-{[4-(3-{[(ethylamino)carbonyl]oxy}propyl)-1,3-dimethyl-1H-pyrazol-5-yl]oxy}phenoxy)propionic acid (Example 298),
or a salt thereof.

The salt of the compound represented by the formula (I) is preferably a pharmacologically acceptable salt. Examples thereof include salts with inorganic base, salts with organic base, salts with inorganic acid, salts with organic acid, salts with basic or acidic amino acid, and the like.

Preferable examples of the salts with inorganic base include alkali metal salts such as sodium salt, potassium salt and the like; alkaline earth metal salts such as calcium salt, magnesium salt and the like; aluminum salt, ammonium salt and the like.

Preferable examples of the salt with organic base include a salt with trimethylamine, triethylamine, pyridine, picoline, ethanolamine, diethanolamine, triethanolamine, tromethamine[tris(hydroxymethyl)methylamine], tert-butylamine, cyclohexylamine, benzylamine, dicyclohexylamine, N,N'-dibenzylethylenediamine and the like.

Preferable examples of the salt with inorganic acid include a salt with hydrochloric acid, hydrobromic acid, nitric acid, sulfuric acid, phosphoric acid and the like.

Preferable examples of the salt with organic acid include a salt with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like.

Preferable examples of the salt with basic amino acid include a salt with arginine, lysine, ornithine and the like.
Preferable examples of the salt with acidic amino acid include a salt with aspartic acid, glutamic acid and the like.

The prodrug of compound (I) is a compound which is converted to compound (I) with a reaction due to an enzyme, gastric acid, etc. under the physiological condition in the living body, that is, a compound which is converted to compound (I) by enzymatic oxidation, reduction, hydrolysis, etc.; a compound which is converted to compound (I) by hydrolysis etc. due to gastric acid, and the like. Examples of the prodrug of compound (I) include a compound obtained by subjecting an amino group in compound (I) to an acylation, alkylation or phosphorylation (e.g., a compound obtained by subjecting an amino group in compound (I) to an eicosanoylation, alanylation, pentylaminocarbonylation, (5-methyl-2-oxo-1,3-dioxolen-4-yl)methoxycarbonylation, tetrahydrofuranylation, tetrahydropyranylation, pyrrolidylmethylation, pivaloyloxymethylation or tert-butylation); a compound obtained by subjecting a hydroxy group in compound (I) to an acylation, alkylation, phosphorylation or boration (e.g., a compound obtained by subjecting an hydroxy group in compound (I) to an acetylation, palmitoylation, propanoylation, pivaloylation, succinylation, fumarylation, alanylation, dimethylaminomethylcarbonylation or tetrahydropyranylation); a compound obtained by subjecting a carboxyl group in compound (I) to an esterification or amidation (e.g., a compound obtained by subjecting a carboxyl group in compound (I) to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification or methylamidation) and the like. Any of these compounds can be produced from compound (I) by a method known *per se.*

A prodrug of compound (I) may be a compound that converts to compound (I) under physiological conditions as described in Development of Pharmaceutical Products, vol. 7, Molecule Design, 163-198, Hirokawa Shoten (1990).

Compound (I) may be in the form of a crystal, and the crystal form of the crystal may be single or plural. The crystal can be produced by a crystallization method known per se. In the present specification, the melting point means that measured using, for example, a micromelting point apparatus (Yanaco, MP-500D or Buchi, B-545), a DSC (differential scanning calorimetry) device (SEIKO, EXSTAR6000) or the like.

In general, the melting points vary depending on the measurement apparatuses, the measurement conditions and the like. The crystal in the present specification may show different values from the melting point described in the present specification, as long as they are within a general error range.

The crystal of compound (I) is superior in physicochemical properties (e.g., melting point, solubility, stability) and biological properties (e.g., pharmacokinetics (absorption, distribution, metabolism, excretion), efficacy expression), and thus it is extremely useful as a medicament.

Compound (I) may be a solvate (e.g., hydrate) or a non-solvate, both of which are encompassed in compound (I).
Compounds labeled with an isotope (e.g., ³H,¹⁴C,³⁵S,¹²⁵I) and the like are encompassed in compound (I).
Deuterium-converted compounds wherein ¹H has been converted to ²H(D) are also encompassed in compound (I).

Compound (I) or a prodrug thereof (hereinafter sometimes to be simply abbreviated as the compound of the present invention) shows low toxicity (e.g., acute toxicity, chronic toxicity, genetic toxicity, reproductive toxicity, cardiotoxicity, drug interaction, carcinogenicity), and can be used as it is or as a pharmaceutical composition in admixture with a pharmaceutically acceptable carrier etc., as an agent for the prophylaxis or treatment of the below-mentioned various disease, an insulin sensitizer and the like, in mammals (e.g., humans, mice, rats, rabbits, dogs, cats, bovines, horses, pigs, monkeys).

Here, as the pharmacologically acceptable carrier, various organic or inorganic carrier substances conventionally used as a preparation material can be used. They are incorporated as excipient, lubricant, binder and disintegrant for solid preparations; solvent, solubilizing agent, suspending agent, isotonicity agent, buffer and soothing agent for liquid preparations and the like. Where necessary, preparation additives such as preservatives, antioxidants, colorants, sweetening agents and the like can be used.

Preferable examples of the excipient include lactose, sucrose, D-mannitol, D-sorbitol, starch, α-starch, dextrin, crystalline cellulose, low-substituted hydroxypropylcellulose, sodium carboxymethylcellulose, gum arabic, pullulan, light anhydrous silicic acid, synthetic aluminum silicate, magnesium alumino metasilicate and the like.

Preferable examples of the lubricant include magnesium stearate, calcium stearate, talc, colloidal silica and the like.
Preferable examples of the binder include α-starch, saccharose, gelatin, gum arabic, methylcellulose, carboxymethylcellulose, carboxymethylcellulose sodium, crystalline cellulose, sucrose, D-mannitol, trehalose, dextrin, pullulan, hydroxypropylcellulose, hydroxypropylmethylcellulose, polyvinylpyrrolidone and the like.

Preferable examples of the disintegrant include lactose, sucrose, starch, carboxymethylcellulose, carboxymethylcellulose calcium, croscarmellose sodium, carboxymethylstarch sodium, light anhydrous silicic acid, low-substituted hydroxypropylcellulose and the like.

Preferable examples of the solvent include water for injection, physiological brine, Ringer solution, alcohol, propylene glycol, polyethylene glycol, sesame oil, corn oil, olive oil, cottonseed oil and the like.
Preferable examples of the solubilizing agents include polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like.

Preferable examples of the suspending agent include surfactants such as stearyltriethanolamine, sodium lauryl sulfate, laurylaminopropionic acid, lecithin, benzalkonium chloride, benzethonium chloride, glycerol monostearate and the like; hydrophilic polymers such as polyvinyl alcohol, polyvinylpyrrolidone, carboxymethylcellulose sodium, methylcellulose, hydroxymethylcellulose, hydroxyethylcellulose, hydroxypropylcellulose and the like; polysorbates, polyoxyethylene hydrogenated castor oil, and the like.

Preferable examples of the isotonicity agent include sodium chloride, glycerin, D-mannitol, D-sorbitol, glucose and the like.
Preferable examples of the buffer include buffers such as phosphate, acetate, carbonate, citrate and the like, and the like.
Preferable examples of the soothing agent include benzyl alcohol and the like.

Preferable examples of the preservative include p-oxybenzoates, chlorobutanol, benzyl alcohol, phenethyl alcohol, dehydroacetic acid, sorbic acid and the like.
Preferable examples of the antioxidant include sulfite, ascorbate and the like.
Preferable examples of the colorant include water-soluble food tar colors (e.g., food colors such as Food Red Nos. 2 and 3, Food Yellow Nos. 4 and 5, Food Blue Nos. 1 and 2 and the like), water insoluble lake dye (e.g., aluminum salts of the above-mentioned water-soluble food tar colors), natural dyes (e.g., β-carotene, chlorophyll, red iron oxide) and the like.
Preferable examples of the sweetening agent include saccharin sodium, dipotassium glycyrrhizinate, aspartame, stevia and the like.

The dosage form of the above-mentioned pharmaceutical composition is, for example, an oral agent such as tablets (inclusive of sugar-coated tablets, film-coated tablets, sublingual tablets and orally disintegrable tablets), capsules (inclusive of soft capsules and microcapsules), granules, powders, troches, syrups, emulsions, suspensions, films (e.g., orally disintegrable film) and the like; a parenteral agent such as injections (e.g., subcutaneous injections, intravenous injections, intramuscular injections, intraperitoneal injections, drip infusions), external agents (e.g., transdermal preparations, ointments), suppositories (e.g., rectal suppositories, vaginal suppositories), pellets, nasal preparations, pulmonary preparations (inhalations), ophthalmic preparations and the like. These may be administered safely via an oral or parenteral (e.g., topical, rectal, intravenous administrations etc.) route.
These preparations may be controlled-release preparations (e.g., sustained-release microcapsule) such as immediate-release preparation, sustained-release preparation and the like.

The pharmaceutical composition can be produced by a method conventionally used in the technical field of formulation of preparations, such as a method described in the Japanese Pharmacopoeia and the like.
While the content of the compound of the present invention in the pharmaceutical composition varies depending on the dosage form, the dose of the compound of the present invention and the like, it is, for example, about 0.1 to 100 wt%.

The compound of the present invention has a hypoglycemic action, a hypolipidemic action, an insulin sensitizing action, an insulin sensitivity enhancing action and a peroxisome proliferator-activated receptor (hereinafter sometimes to be abbreviated as PPAR)γ (GenBank Accession No. L40904) agonist (activation) action. Here, PPARγ may form a heterodimeric receptor with any of retinoid X receptor (hereinafter sometimes to be abbreviated as RXR)α (GenBank Accession No. X52773), RXRβ (GenBank Accession No. M84820) and RXRγ (GenBank Accession No. U38480).
The compound of the present invention particularly has a selective partial agonist action on PPARγ.
A selective partial agonist for PPARγ has been reported to be unaccompanied by side effects such as body weight gain, adipocyte accumulation, cardiac hypertrophy and the like, as compared to a full agonist for PPARγ (e.g., thiazolidinedione compound) (Molecular Endocrinology, vol. 17, NO. 4, page 662, 2003). Therefore, the compound of the present invention is useful as a hypoglycemic agent unaccompanied by side effects such as body weight gain, adipocyte accumulation, cardiac hypertrophy and the like, as compared to a full agonist for PPARγ.
The compound of the present invention can be used, for example, as an agent for the prophylaxis or treatment of diabetes (e.g., type-1 diabetes, type-2 diabetes, gestational diabetes, obesity diabetes); an agent for the prophylaxis or treatment of hyperlipidemia (e.g., hypertriglyceridemia, hypercholesterolemia, hypo-HDL-emia, postprandial hyperlipidemia); insulin sensitizer; an agent for enhancing insulin sensitivity; an agent for the prophylaxis or treatment of impaired glucose tolerance [IGT (Impaired Glucose Tolerance)]; and an agent for preventing progress of impaired glucose tolerance into diabetes.

For diagnostic criteria of diabetes, Japan Diabetes Society reported new diagnostic criteria.
According to this report, diabetes is a condition showing any of a fasting blood glucose level (glucose concentration of intravenous plasma) of not less than 126 mg/dl, a 75 g oral glucose tolerance test (75 g OGTT) 2 hr level (glucose concentration of intravenous plasma) of not less than 200 mg/dl, and a non-fasting blood glucose level (glucose concentration of intravenous plasma) of not less than 200 mg/dl. A condition not falling under the above-mentioned diabetes and different from "a condition showing a fasting blood glucose level (glucose concentration of intravenous plasma) of less than 110 mg/dl or a 75 g oral glucose tolerance test (75 g OGTT) 2 hr level (glucose concentration of intravenous plasma) of less than 140 mg/dl" (normal type) is called a "borderline type".

In addition, ADA (American Diabetes Association) and WHO reported new diagnostic criteria of diabetes.
According to these reports, diabetes is a condition showing a fasting blood glucose level (glucose concentration of intravenous plasma) of not less than 126 mg/dl or a 75 g oral glucose tolerance test 2 hr level (glucose concentration of intravenous plasma) of not less than 200 mg/dl.

According to the above-mentioned reports of ADA and WHO, impaired glucose tolerance is a condition showing a 75 g oral glucose tolerance test 2 hr level (glucose concentration of intravenous plasma) of not less than 140 mg/dl and less than 200 mg/dl. According to the report of ADA, a condition showing a fasting blood glucose level (glucose concentration of intravenous plasma) of not less than 100 mg/dl and less than 126 mg/dl is called IFG (Impaired Fasting Glucose). On the other hand, WHO defines the IFG (Impaired Fasting Glucose) to be a condition showing a fasting blood glucose level (glucose concentration of intravenous plasma) of not less than 110 mg/dl and less than 126 mg/dl, and calls it IFG (Impaired Fasting Glycaemia).

The compound of the present invention can be also used as an agent for the prophylaxis or treatment of diabetes, borderline type, impaired glucose tolerance, IFG (Impaired Fasting Glucose) and IFG (Impaired Fasting Glycaemia), as determined according to the above-mentioned new diagnostic criteria. Moreover, the compound of the present invention can also prevent progress of borderline type, impaired glucose tolerance, IFG (Impaired Fasting Glucose) or IFG (Impaired Fasting Glycaemia) into diabetes.

The compound of the present invention can also be used as an agent for the prophylaxis or treatment of, for example, diabetic complications [e.g., neuropathy, nephropathy, retinopathy, cataract, macroangiopathy, osteopenia, hyperosmolar diabetic coma, infectious disease (e.g., respiratory infection, urinary tract infection, gastrointestinal infection, dermal soft tissue infection, inferior limb infection), diabetic gangrene, xerostomia, hypacusis, cerebrovascular disorder, peripheral blood circulation disorder], obesity, osteoporosis, cachexia (e.g., cancerous cachexia, tuberculous cachexia, diabetic cachexia, blood disease cachexia, endocrine disease cachexia, infectious disease cachexia or cachexia due to acquired immunodeficiency syndrome), fatty liver, hypertension, polycystic ovary syndrome, kidney disease (e.g., diabetic nephropathy, glomerular nephritis, glomerulosclerosis, nephrotic syndrome, hypertensive nephrosclerosis, end stage kidney disease), muscular dystrophy, myocardial infarction, angina pectoris, cerebrovascular accident (e.g., cerebral infarction, cerebral apoplexy), insulin resistance syndrome, Syndrome X, metabolic syndrome (pathology having three or more selected from hypertriglyceridemia (TG), hypoHDL cholesterolemia (HDL-C), hypertension, abdomen overweight and impaired glucose tolerance), hyperinsulinemia, hyperinsulinemia-induced sensory disorder, tumor (e.g., leukemia, breast cancer, prostate cancer, skin cancer), irritable bowel syndrome, acute or chronic diarrhea, inflammatory diseases (e.g., arteriosclerosis (e.g., atherosclerosis), chronic rheumatoid arthritis, spondylitis deformans, osteoarthritis, lumbago, gout, postoperative or traumatic inflammation, swelling, neuralgia, pharyngolaryngitis, cystitis, hepatitis (inclusive of nonalcoholic steatohepatitis), pneumonia, pancreatitis, inflammatory bowel disease, ulcerative colitis, chronic obstructive pulmonary disease (COPD)), visceral obesity syndrome, leg ulcer, sepsis, psoriasis and the like.

In addition, the compound of the present invention can also be used for ameliorating the conditions such as abdominal pain, nausea, vomiting, discomfort in the upper abdomen and the like, which are associated with peptic ulcer, acute or chronic gastritis, biliary dyskinesia, cholecystitis and the like, and the like.

The compound of the present invention can also be used as an agent for the prophylaxis or treatment of inflammatory disease involving TNF-α. Here, the inflammatory disease involving TNF-α is an inflammatory disease developed by the presence of TNF-α, which can be treated via a TNF-α inhibitory effect. Examples of the inflammatory disease include diabetic complications (e.g., retinopathy, nephropathy, neuropathy, macroangiopathy), chronic rheumatoid arthritis, spondylitis deformans, osteoarthritis, lumbago, gout, postoperative or traumatic inflammation, swelling, neuralgia, pharyngolaryngitis, cystitis, hepatitis, pneumonia, stomach mucous membrane injury (including stomach mucous membrane injury caused by aspirin) and the like.

The compound of the present invention has an apoptosis inhibitory action and can also be used as an agent for the prophylaxis or treatment of diseases involving promotion of apoptosis. Examples of the disease involving promotion of apoptosis include viral diseases (e.g., AIDS, fulminant hepatitis), neurodegenerative diseases (e.g., Alzheimer's disease, Parkinson's disease, amyotrophic lateral sclerosis, pigmentosa, cerebellar degeneration), myelodysplasia (e.g., aplastic anemia), ischemic diseases (e.g., myocardial infarction, cerebral apoplexy), hepatic diseases (e.g., alcoholic hepatitis, hepatitis B, hepatitis C), articular diseases (e.g., osteoarthritis), atherosclerosis and the like.

The compound of the present invention can also be used for reduction of visceral fat, inhibition of visceral fat accumulation, glycometabolism improvement, lipometabolism improvement, insulin resistance improvement, oxidized LDL production inhibition, lipoprotein metabolism improvement, coronary metabolism improvement, prophylaxis or treatment of cardiovascular complications, prophylaxis or treatment of heart failure complications, decrease of blood remnant, prophylaxis or treatment of anovulation, prophylaxis or treatment of hirsutism, prophylaxis or treatment of hyperandrogenemia and the like.

While the dose of the compound of the present invention varies depending on the administration subject, administration route, target disease, condition and the like, for example, it is generally about 0.005 to 50 mg/kg body weight, preferably 0.01 to 2 mg/kg body weight, more preferably 0.025 to 0.5 mg/kg body weight, for oral administration to adult diabetic patients, which is desirably administered in one to three portions a day.

The compound of the present invention can be used in combination with pharmaceutical agents (hereinafter to be abbreviated as combination drug) such as therapeutic agents for diabetes, therapeutic agents for diabetic complications, therapeutic agents for hyperlipidemia, antihypertensive agents, antiobesity agents, diuretics, chemotherapeutic agents, immunotherapeutic agents, antithrombotic agents, therapeutic agents for osteoporosis, antidementia agents, erectile dysfunction ameliorating agents, therapeutic agents for urinary incontinence or pollakiuria, therapeutic agents for dysuria and the like. These combination drugs may be low-molecular-weight compounds, high-molecular-weight proteins, polypeptides, antibodies or nucleic acids (including antisense nucleic acid, siRNA, shRNA), vaccines and the like.

The administration time of the compound of the present invention and the combination drug is not restricted, and these can be administered to an administration subject simultaneously, or may be administered at staggered times.
Examples of the administration mode of the compound of the present invention and the combination drug include the following methods: (1) the compound of the present invention and the combination drug are simultaneously formulated to give a single preparation which is administered, (2) the compound of the present invention and the combination drug are separately formulated to give two kinds of preparations which are administered simultaneously by the same administration route, (3) the compound of the present invention and the combination drug are separately formulated to give two kinds of preparations which are administered by the same administration route at staggered times, (4) the compound of the present invention and the combination drug are separately formulated to give two kinds of preparations which are administered simultaneously by the different administration routes, (5) the compound of the present invention and the combination drug are separately formulated to give two kinds of preparations which are administered by the different administration routes at staggered times (e.g., the compound of the present invention and the combination drug are administered in this order, or in the reverse order), and the like.

The dose of the combination drug can be appropriately determined based on the dose employed clinically. The mixing ratio of the compound of the present invention and a combination drug can be appropriately determined depending on the administration subject, administration route, target disease, symptom, combination and the like. When the administration subject is human, for example, a combination drug can be used in 0.01 to 100 parts by weight relative to 1 part by weight of the compound of the present invention.

Examples of the therapeutic agents for diabetes include insulin preparations (e.g., animal insulin preparations extracted from pancreas of bovine or swine; human insulin preparations genetically synthesized using *Escherichia coli* or yeast; zinc insulin; protamine zinc insulin; fragment or derivative of insulin (e.g., INS-1), oral insulin preparation), insulin sensitizers (e.g., pioglitazone or a salt thereof (preferably hydrochloride), rosiglitazone or a salt thereof (preferably maleate), Tesaglitazar, Ragaglitazar, Muraglitazar, Edaglitazone, Metaglidasen, Naveglitazar, AMG-131, THR-0921), α-glucosidase inhibitors (e.g., voglibose, acarbose, miglitol, emiglitate), biguanides (e.g., metformin, buformin or a salt thereof (e.g., hydrochloride, fumarate, succinate)), insulin secretagogues [sulfonylureas (e.g., tolbutamide, glibenclamide, gliclazide, chlorpropamide, tolazamide, acetohexamide, glyclopyramide, glimepiride, glipizide, glybuzole), repaglinide, nateglinide, mitiglinide or a calcium salt hydrate thereof], dipeptidyl peptidase IV inhibitors (e.g., Alogliptin or a salt thereof (preferably benzoate), Vildagliptin, Sitagliptin, Saxagliptin, T-6666, TS-021), β3 agonists (e.g., AJ-9677), GPR40 agonists, GLP-1 receptor agonists [e.g., GLP-1, GLP-1MR agent, NN-2211, AC-2993 (exendin-4), BIM-51077, Aib(8,35)hGLP-1(7,37)NH₂, CJC-1131], amylin agonists (e.g., pramlintide), phosphotyrosine phosphatase inhibitors (e.g., sodium vanadate), gluconeogenesis inhibitors (e.g., glycogen phosphorylase inhibitors, glucose-6-phosphatase inhibitors, glucagon antagonists), SGLUT (sodium-glucose cotransporter) inhibitors (e.g., T-1095), 11β-hydroxysteroid dehydrogenase inhibitors (e.g., BVT-3498), adiponectin or agonist thereof, IKK inhibitors (e.g., AS-2868), leptin resistance improving drugs, somatostatin receptor agonists, glucokinase activators (e.g., Ro-28-1675), GIP (Glucose-dependent insulinotropic peptide) and the like.

Examples of the therapeutic agents for diabetic complications include aldose reductase inhibitors (e.g., tolrestat, epalrestat, zenarestat, zopolrestat, minalrestat, fidarestat, CT-112, ranirestat (AS-3201)), neurotrophic factors and increasing drugs thereof (e.g., NGF, NT-3, BDNF, neurotrophin production/secretion promoting agents described in WO01/14372 (e.g., 4-(4-chlorophenyl)-2-(2-methyl-1-imidazolyl)-5-[3-(2-methylphenoxy)propyl]oxazole)), PKC inhibitors (e.g., ruboxistaurin mesylate), AGE inhibitors (e.g., ALT946, pimagedine, N-phenacylthiazolium bromide (ALT766), EXO-226, Pyridorin, pyridoxamine), active oxygen scavengers (e.g., thioctic acid), cerebral vasodilators (e.g., tiapuride, mexiletine), somatostatin receptor agonists (e.g., BIM23190), apoptosis signal regulating kinase-1 (ASK-1) inhibitors.

Examples of the therapeutic agents for hyperlipidemias include HMG-CoA reductase inhibitors (e.g., cerivastatin, pravastatin, simvastatin, lovastatin, atorvastatin, fluvastatin, itavastatin, rosuvastatin, pitavastatin or a salt thereof (e.g., sodium salt, calcium salt)), squalene synthase inhibitors (e.g., lapaquistat or a salt thereof (preferably acetate)), fibrate compounds (e.g., bezafibrate, clofibrate, simfibrate, clinofibrate), ACAT inhibitors (e.g., Avasimibe, Eflucimibe), anion exchange resins (e.g., colestyramine), probucol, nicotinic acid drugs (e.g., nicomol, niceritrol), ethyl icosapentate, phytosterols (e.g., soysterol, γ-oryzanol) and the like.

Examples of the antihypertensive agents include angiotensin converting enzyme inhibitors (e.g., captopril, enalapril, delapril), angiotensin II antagonists (e.g., candesartan cilexetil, losartan, eprosartan, valsartan, telmisartan, irbesartan, olmesartan medoxomil, tasosartan, 1-[[2'-(2,5-dihydro-5-oxo-4H-1,2,4-oxadiazol-3-yl)biphenyl-4-yl]methyl]-2-ethoxy-1H-benzimidazole-7-carboxylic acid), calcium channel blockers (e.g., manidipine, nifedipine, nicardipine, amlodipine, efonidipine), potassium channel openers (e.g., levcromakalim, L-27152, AL0671, NIP-121), clonidine and the like.

Examples of the antiobesity agents include antiobesity agents acting on the central nervous system (e.g., dexfenfluramine, fenfluramine, phentermine, sibutramine, amfepramone, dexamphetamine, mazindol, phenylpropanolamine, clobenzorex; MCH receptor antagonists (e.g., SB-568849; SNAP-7941; compounds described in WO01/82925 and WO01/87834); neuropeptide Y antagonists (e.g., CP-422935); cannabinoid receptor antagonists (e.g., SR-141716, SR-147778); ghrelin antagonists; 11β-hydroxysteroid dehydrogenase inhibitors (e.g., BVT-3498)), pancreatic lipase inhibitors (e.g., orlistat, cetilistat (ATL-962)), β3 agonists (e.g., AJ-9677), peptide anorexiants (e.g., leptin, CNTF (Ciliary Neurotropic Factor)), cholecystokinin agonists (e.g., lintitript, FPL-15849), feeding deterrents (e.g., P-57) and the like.

Examples of the diuretics include xanthine derivatives (e.g., sodium salicylate and theobromine, calcium salicylate and theobromine), thiazide preparations (e.g., ethiazide, cyclopenthiazide, trichloromethiazide, hydrochlorothiazide, hydroflumethiazide, bentylhydrochlorothiazide, penflutizide, polythiazide, methyclothiazide), antialdosterone preparations (e.g., spironolactone, triamterene), carbonate dehydratase inhibitors (e.g., acetazolamide), chlorobenzenesulfonamide preparations (e.g., chlortalidone, mefruside, indapamide), azosemide, isosorbide, etacrynic acid, piretanide, bumetanide, furosemide and the like.

Examples of the chemotherapeutic agents include alkylating agents (e.g., cyclophosphamide, ifosfamide), metabolic antagonists (e.g., methotrexate, 5-fluorouracil and a derivative thereof), antitumor antibiotics (e.g., mitomycin, adriamycin), plant-derived antitumor agent (e.g., vincristine, vindesine, Taxol), cisplatin, carboplatin, etoposide and the like. Of these, Furtulon or NeoFurtulon, which are 5-fluorouracil derivatives, and the like are preferable.

Examples of the immunotherapeutic agents include microorganism or bacterial components (e.g., muramyl dipeptide derivative, Picibanil), polysaccharides having immunity potentiating activity (e.g., lentinan, schizophyllan, krestin), cytokines obtained by genetic engineering techniques (e.g., interferon, interleukin (IL)), colony stimulating factors (e.g., granulocyte colony stimulating factor, erythropoietin) and the like, with preference given to interleukins such as IL-1, IL-2, IL-12 and the like.

Examples of the antithrombotic agents include heparin (e.g., heparin sodium, heparin calcium, dalteparin sodium), warfarin (e.g., warfarin potassium), anti-thrombin drugs (e.g., aragatroban), thrombolytic agents (e.g., urokinase, tisokinase, alteplase, nateplase, monteplase, pamiteplase), platelet aggregation inhibitors (e.g., ticlopidine hydrochloride, cilostazol, ethyl icosapentate, beraprost sodium, sarpogrelate hydrochloride) and the like.

Examples of the therapeutic agents for osteoporosis include alfacalcidol, calcitriol, elcatonin, calcitonin salmon, estriol, ipriflavone, risedronate disodium, pamidronate disodium, alendronate sodium hydrate, incadronate disodium and the like.

Examples of the antidementia agents include tacrine, donepezil, rivastigmine, galanthamine and the like.
Examples of the erectile dysfunction ameliorating agents include apomorphine, sildenafil citrate and the like.
Examples of the therapeutic agents for urinary incontinence or pollakiuria include flavoxate hydrochloride, oxybutynin hydrochloride, propiverine hydrochloride and the like.
Examples of the therapeutic agents for dysuria include acetylcholine esterase inhibitors (e.g., distigmine) and the like.

Examples of the combination drugs also include drugs having a cachexia-ameliorating action established in animal models and clinical situations, such as cyclooxygenase inhibitors (e.g., indomethacin), progesterone derivatives (e.g., megestrol acetate), glucosteroids (e.g., dexamethasone), metoclopramide agents, tetrahydrocannabinol agents, fat metabolism improving agents (e.g., eicosapentanoic acid), growth hormones, IGF-1, antibodies to a cachexia-inducing factor such as TNF-α, LIF, IL-6 and oncostatin M, and the like.

Examples of the combination drugs also include nerve regeneration promoting drugs (e.g., Y-128, VX853, prosaptide), antidepressants (e.g., desipramine, amitriptyline, imipramine), antiepileptics (e.g., lamotrigine), antiarrhythmic agents (e.g., mexiletine), acetylcholine receptor ligands (e.g., ABT-594), endothelin receptor antagonists (e.g., ABT-627), monoamine uptake inhibitors (e.g., tramadol), narcotic analgesics (e.g., morphine), GABA receptor agonists (e.g., gabapentin), α2 receptor agonists (e.g., clonidine), local analgesics (e.g., capsaicin), antianxiety drugs (e.g., benzothiazepines), dopamine agonists (e.g., apomorphine), midazolam, ketoconazole and the like.

The combination drug is preferably an insulin preparation, an insulin sensitizer, an α-glucosidase inhibitor, biguanide, insulin secretagogues (preferably sulfonylurea) and the like.
The above-mentioned combination drugs may be used in a mixture of two or more kinds thereof at an appropriate ratio.

When the compound of the present invention is used in combination with a combination drug, the dose of each agent can be reduced within a safe range in consideration of the side effects thereof. Particularly, the doses of insulin sensitizers, insulin secretagogues and biguanides can be reduced from generally dose levels. Therefore, the side effects possibly caused by these agents can be safely prevented. In addition, the doses of the therapeutic agents for diabetic complications, the therapeutic agents for hyperlipidemia and the antihypertensive agents can be reduced, and as a result, the side effects possibly caused by these agents can be effectively prevented.

The production method of the compound of the present invention is explained in the following.
Compound (I) can be produced according to a method known per se, for example, the following Method A to Method Z, Method AA to Method AD, Method AE to Method AY, Method C', Method C", Method E', Method E", Method H', Method I', Method K', Method M' or a method analogous thereto. In the following production method, the starting material compounds may be used in the form of a salt, and examples thereof include those exemplified as the above-mentioned salt for compound (I).

Compound (I) can be produced, for example, according to the following Method A.

### [Method A]

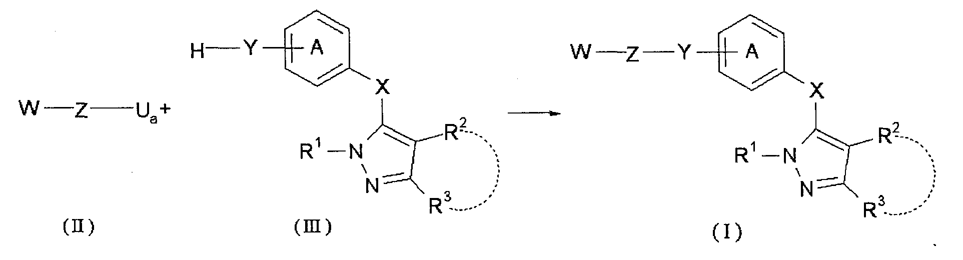

wherein Ua is a leaving group or a hydroxy group, and the other symbols are as defined above.
Examples of the "leaving group" for Ua include a chlorine atom, a bromine atom, an iodine atom, a benzenesulfonyloxy group, a p-toluenesulfonyloxy group, a methanesulfonyloxy group, a trifluoromethanesulfonyloxy group and the like.

In this method, compound (I) can be produced by reacting compound (III) with compound (II).
When Ua is a leaving group (provided that Y is not a bond), this reaction is carried out in the presence of a base, in a solvent that does not adversely influence the reaction.
Examples of the base include amines such as triethylamine, N,N-diisopropylethylamine, N-methylmorpholine, N,N-dimethylaniline, 4-dimethylaminopyridine and the like; alkali metal salts such as sodium hydrogen carbonate, sodium carbonate, potassium carbonate and the like; alkali metal hydroxides such as sodium hydroxide, potassium hydroxide, lithium hydroxide and the like; alkaline earth metal hydroxides such as magnesium hydroxide, calcium hydroxide, barium hydroxide and the like; metal hydrides such as potassium hydride, sodium hydride and the like; alkali metal C₁₋₆ alkoxides such as sodium methoxide, sodium ethoxide, potassium tert-butoxide and the like, and the like. The amount of the base to be used is preferably about 1 to about 5 mol per 1 mol of compound (III).
The amount of compound (II) to be used is preferably about 1 to about 10 mol per 1 mol of compound (III).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; ketones such as acetone, 2-butanone and the like; water and the like. These solvents may be used in a mixture at an appropriate ratio.
The reaction temperature is generally about -80 to about 150°C, preferably about -10 to about 100°C.
The reaction time is generally about 0.5 to about 20 hr.

When Ua is a hydroxy group, and Y is O, this reaction is carried out according to a method known per se, for example, the method described in Synthesis page 1 (1981), or a method analogous thereto. That is, this reaction is generally carried out in the presence of an organic phosphorus compound and an electrophilic agent, in a solvent that does not adversely influence the reaction.
Examples of the organic phosphorus compound include triphenylphosphine, tributylphosphine and the like.
Examples of the electrophilic agent include diethyl azodicarboxylate, diisopropyl azodicarboxylate, 1,1'-azodicarbonyldipiperidine and the like.
The amount of the organic phosphorus compound and electrophilic agent to be used is preferably about 1 to about 5 mol per 1 mol of compound (III), respectively.
The amount of compound (II) to be used is preferably about 1 to about 5 mol per 1 mol of compound (III).
Examples of the solvent that does not adversely influence the reaction include those similar to the above-mentioned solvent.
The reaction temperature is generally about -80 to about 150°C, preferably about -10 to about 100°C.
The reaction time is generally about 0.5 to about 20 hr.

Compound (II) used as a starting material compound for the above-mentioned Method A can be produced according to a method known per se. Compound (III) can be produced, for example, according to the below-mentioned Method C, Method C', Method E, Method E', Method F, Method G, Method H, Method H', Method I, Method I', Method J, Method K, Method K', Method L, Method M, Method M', Method N, Method O, Method P, Method R, Method S, Method T, Method U, Method AD, Method AH, Method AJ, Method AK, Method AT, Method AU or Method AV or a method analogous thereto.

Compound (I-3), which is compound (I) wherein W is -CO₂H, and compound (I-4), which is compound (I) wherein W is - CONR^{5a}R^{6a} wherein R^{5a} and R^{6a} are as defined below, can be produced, for example, according to the following Method B.

### [Method B]

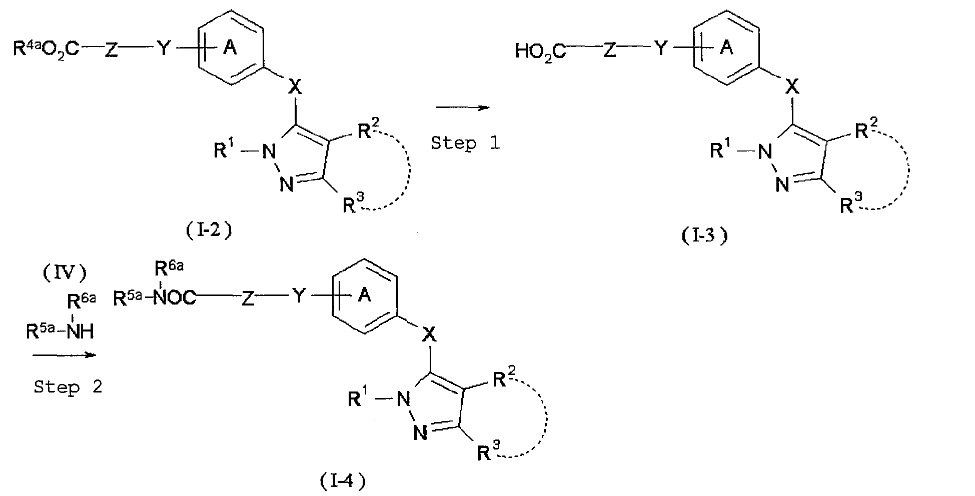

wherein
R^{4a} is a C₁₋₁₀ alkyl group, a benzyl group optionally substituted by C₁₋₆ alkyl group (s) or a C₆₋₁₄ aryl group optionally substituted by C₁₋₆ alkyl group(s),
R^{5a} is a hydrogen atom, a C₁₋₁₀ alkyl group, a benzyl group optionally substituted by C₁₋₆ alkyl group (s) or a C₆₋₁₄ aryl group optionally substituted by C₁₋₆ alkyl group(s),
R^{6a} is a hydrogen atom, a C₁₋₁₀ alkyl group, a benzyl group optionally substituted by C₁₋₆ alkyl group(s), a C₆₋₁₄ aryl group optionally substituted by C₁₋₆ alkyl group (s), a C₁₋₁₀ alkylsulfonyl group, a benzylsulfonyl group optionally substituted by C₁₋₆ alkyl group (s) or a C₆₋₁₄ arylsulfonyl group optionally substituted by C₁₋₆ alkyl group(s), and the other symbols are as defined above.
R^{4a} is preferably methyl, ethyl, tert-butyl, benzyl, phenyl or the like.
R^{5a} is preferably a hydrogen atom, methyl, ethyl, tert-butyl, benzyl, phenyl or the like.
R^{6a} is preferably methanesulfonyl, pentanesulfonyl, benzenesulfonyl, methyl, ethyl, benzyl, phenyl or the like.

### [Step 1]

In this step, compound (I-3) can be produced by subjecting compound (I-2), which is compound (I) wherein W is -CO₂R^{4a} to hydrolysis.
This reaction is carried out in the presence of an acid or a base, in a solvent that does not adversely influence the reaction, according to a conventional method.
Examples of the acid include mineral acids such as hydrochloric acid, sulfuric acid and the like; Lewis acids such as boron trichloride, boron tribromide and the like; organic acids such as trifluoroacetic acid, p-toluenesulfonic acid and the like, and the like. The amount of the acid to be used is preferably about 0.01 to about 5 mol per 1 mol of compound (I-2).
Examples of the base include alkali metal hydroxides such as sodium hydroxide, potassium hydroxide, lithium hydroxide and the like; alkaline earth metal hydroxides such as magnesium hydroxide, calcium hydroxide, barium hydroxide and the like, and the like. The amount of the base to be used is preferably about 1 to about 10 mol per 1 mol of compound (I-2).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; alcohols such as methanol, ethanol and the like; ketones such as acetone and the like, and the like. These solvents may be used in a mixture at an appropriate ratio, or in a mixture with water at an appropriate ratio.
The reaction temperature is generally about -80 to about 150°C, preferably about -10 to about 100°C.
The reaction time is generally about 0.5 to about 20 hr.

### [Step 2]

In this step, compound (I-4) can be produced by subjecting compound (I-3) to an amidation reaction.
This reaction is carried out according to a method known per se, for example, a method of directly condensing compound (I-3) with compound (IV), a method of reacting a reactive derivative of compound (I-3) with compound (IV), or the like.
Examples of the reactive derivative of compound (I-3) include acid anhydrides, acid halides (e.g., acid chlorides, acid bromides), imidazolides, mixed acid anhydrides (e.g., anhydrides with methyl carbonate, ethyl carbonate, isobutyl carbonate) and the like.
The method of directly condensing compound (I-3) with compound (IV) is performed in the presence of a condensation agent, in a solvent that does not adversely influence the reaction.
Examples of the condensation agent include conventional known condensation agents such as carbodiimide condensation reagents (e.g., dicyclohexylcarbodiimide, diisopropylcarbodiimide, 1-ethyl-3-dimethylaminopropylcarbodiimide and hydrochlorides thereof and the like); phosphoric acid condensation reagents (e.g., diethyl cyanophosphate, diphenylphosphoryl azide and the like); N,N'-carbonyldiimidazole, 2-chloro-1,3-dimethylimidazolium tetrafluoroborate and the like, and the like. The amount of the condensation agent to be used is generally 0.1 to 10 mol, preferably 0.3 to 3 mol, per 1 mol of compound (I-3).
The amount of compound (IV) to be used is generally 0.1 to 10 mol, preferably 0.3 to 3 mol, per 1 mol of compound (I-3).
Examples of the solvent that does not adversely influence the reaction include amides such as N,N-dimethylformamide, N,N-dimethylacetamide and the like; halogenated hydrocarbons such as chloroform, dichloromethane and the like; aromatic hydrocarbons such as benzene, toluene and the like; ethers such as tetrahydrofuran, dioxane, diethyl ether and the like; acetonitrile, ethyl acetate, water and the like. These solvents may be used in a mixture at an appropriate ratio.
When a carbodiimide condensation reagent is used as a condensation agent, the reaction efficiency can be improved by the use of a suitable condensation promoter (e.g., 1-hydroxy-7-azabenzotriazole, 1-hydroxybenzotriazole, N-hydroxysuccinimide, N-hydroxyphthalimide), as necessary. When a phosphoric acid condensation reagent is used as a condensation agent, the reaction efficiency can be generally improved by adding an organic amine base such as triethylamine, diisopropylethylamine and the like.
The amount of the condensation promoter or organic amine base to be used is generally 0.1 to 10 mol, preferably 0.3 to 3 mol, per 1 mol of compound (I-3), respectively.
The reaction temperature is generally -30°C to 100°C.
The reaction time is generally 0.5 to 60 hr.

When an acid halide is used as a reactive derivative of compound (I-3) in the method using a reactive derivative of compound (I-3), the reaction is carried out, for example, in the presence of a base, in a solvent that does not adversely influence the reaction.
Examples of the base include amines such as triethylamine, N,N-diisopropylethylamine, N-methylmorpholine, N,N-dimethylaniline, 4-dimethylaminopyridine and the like; alkali metal salts such as sodium hydrogen carbonate, sodium carbonate, potassium carbonate and the like, and the like. The amount of the base to be used is preferably 1 to 10 mol per 1 mol of compound (I-3).
The amount of compound (IV) to be used is generally 0.1 to 10 mol, preferably 0.3 to 3 mol per 1 mol of compound (I-3).
Examples of the solvent that does not adversely influence the reaction include halogenated hydrocarbons such as chloroform, dichloromethane and the like; aromatic hydrocarbons such as benzene, toluene and the like; ethers such as tetrahydrofuran, dioxane, diethyl ether and the like; ethyl acetate, water and the like. These solvents may be used in a mixture at an appropriate ratio.
The reaction temperature is generally -30°C to 100°C.
The reaction time is generally 0.5 to 20 hr.

When a mixed acid anhydride is used as a reactive derivative of compound (I-3), the reaction is carried out by reacting compound (I-3) with a chlorocarbonate (e.g., methyl chlorocarbonate, ethyl chlorocarbonate, isobutyl chlorocarbonate) in the presence of a base (e.g., amines such as triethylamine, N,N-diisopropylethylamine, N-methylmorpholine, N,N-dimethylaniline and the like; alkali metal salts such as sodium hydrogen carbonate, sodium carbonate, potassium carbonate and the like), and reacting the resulting compound with compound (IV).
The amount of the chlorocarbonate to be used is preferably 1 to 5 mol per 1 mol of compound (I-3).
The amount of the base to be used is preferably 1 to 10 mol per 1 mol of compound (I-3).
The amount of compound (IV) to be used is generally 0.1 to 10 mol, preferably 0.3 to 3 mol per 1 mol of compound (I-3).
The reaction temperature is generally -30°C to 100°C.
The reaction time is generally 0.5 to 20 hr.

When an imidazolide is used as a reactive derivative of compound (I-3), the reaction is carried out by reacting compound (I-3) with N,N'-carbonyldiimidazole, and reacting the resulting compound with compound (IV) in the presence of a base (e.g., amines such as triethylamine, N,N-diisopropylethylamine, N-methylmorpholine, N,N-dimethylaniline, 1,8-diazabicyclo[5.4.0]undec-7-ene and the like; alkali metal salts such as sodium hydrogen carbonate, sodium carbonate, potassium carbonate and the like).
The amount of the N,N'-carbonyldiimidazole to be used is preferably 1 to 5 mol per 1 mol of compound (I-3).
The amount of the base to be used is preferably 1 to 10 mol per 1 mol of compound (I-3).
The amount of compound (IV) to be used is generally 0.1 to 10 mol, preferably 0.3 to 3 mol, per 1 mol of compound (I-3).
The reaction temperature is generally -30°C to 100°C.
The reaction time is generally 0.5 to 20 hr.

When an acid anhydride is used as a reactive derivative of compound (I-3), the reaction is carried out by reacting compound (I-3) with a carbodiimide condensation reagent (e.g., dicyclohexylcarbodiimide, diisopropylcarbodiimide, 1-ethyl-3-dimethylaminopropylcarbodiimide), and reacting the resulting compound with compound (IV), preferably in the presence of a base (e.g., amines such as triethylamine, N,N-diisopropylethylamine, N-methylmorpholine, N,N-dimethylaniline and the like; alkali metal salts such as sodium hydrogen carbonate, sodium carbonate, potassium carbonate and the like).
The amount of the carbodiimide condensation reagent to be used is preferably 1 to 5 mol per 1 mol of compound (I-3).
The amount of the base to be used is preferably 0 to 10 mol per 1 mol of compound (I-3).
The amount of compound (IV) to be used is generally 0.1 to 10 mol, preferably 0.3 to 3 mol per 1 mol of compound (I-3).
The reaction temperature is generally -30°C to 150°C.
The reaction time is generally 0.5 to 20 hr.

Compound (I-2) used as a starting material compound for the above-mentioned Method B can be produced, for example, according to the above-mentioned Method A, the below-mentioned Method C", Method E", Method J, Method U, Method W, Method X, Method Y, Method Z, Method AA, Method AB, Method AC, Method AE, Method AF, Method AG, Method AI, Method AM, Method AN, Method AO, Method AP or Method AR or a method analogous thereto. Compound (IV) can be produced according to a method known per se.

Compound (III-2), which is compound (III) wherein Y is a bond, can be produced, for example, according to the following Method C.

### [Method C]

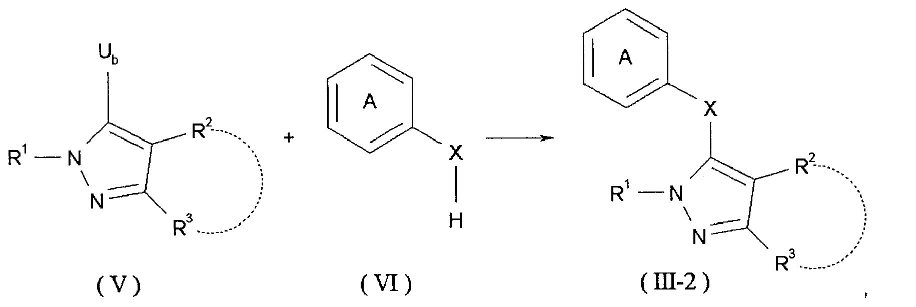

wherein Ub is a leaving group, and the other symbols are as defined above.
Examples of the "leaving group" for Ub include those similar to the above-mentioned "leaving group" for Ua.

In this method, compound (III-2) can be produced by reacting compound (V) with compound (VI).
This reaction is carried out in the presence of a base, in a solvent that does not adversely influence the reaction.
As the base, those exemplified in the above-mentioned Method A can be used. The amount of the base to be used is preferably about 1 to about 5 mol per 1 mol of compound (V).
The amount of compound (VI) to be used is preferably about 1 to about 10 mol per 1 mol of compound (V).
Examples of the solvent that does not adversely influence the reaction include those exemplified in the above-mentioned Method A. These solvents may be used in a mixture at an appropriate ratio.
The reaction temperature is generally about -80 to about 250°C, preferably about -10 to about 150°C.
The reaction time is generally about 0.5 to about 40 hr.
Compound (V) used as a starting material compound for the above-mentioned Method C can be produced, for example, according to a method known per se, or the below-mentioned Method D or Method Q or a method analogous thereto. Compound (VI) can be produced according to a method known per se.

Compound (V-2), which is compound (V) wherein Ub is Uc wherein Uc is as defined below, and R² is -CHO, can be produced, for example, according to the following Method D.

### [Method D]

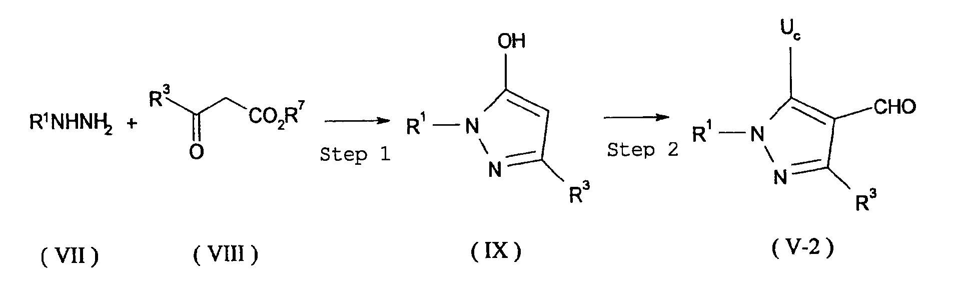

wherein R⁷ is a C₁₋₁₀ alkyl group, a benzyl group optionally substituted by C₁₋₆ alkyl group(s) or a C₆₋₁₄ aryl group optionally substituted by C₁₋₆ alkyl group(s), Uc is a chlorine atom, a bromine atom or an iodine atom, and the other symbols are as defined above.
R⁷ is preferably methyl, ethyl, tert-butyl, benzyl, phenyl or the like.

### [Step 1]

In this step, compound (IX) can be produced by reacting compound (VII) with compound (VIII).
This reaction is carried out in a solvent that does not adversely influence the reaction.
The amount of compound (VII) to be used is preferably about 1 to about 10 mol per 1 mol of compound (VIII).
Examples of the solvent that does not adversely influence the reaction include those exemplified in the above-mentioned Method A. These solvents may be used in a mixture at an appropriate ratio.
The reaction temperature is generally about -80 to about 200°C, preferably about 0 to about 150°C.
The reaction time is generally about 0.5 to about 40 hr.

### [Step 2]

In this step, compound (V-2) can be produced by mixing compound (IX) with N,N-dimethylformamide and a phosphorus oxyhalide compound.
This reaction is carried out without a solvent or in a solvent that does not adversely influence the reaction.
The amount of the N,N-dimethylformamide to be used is preferably about 1 to about 10 mol per 1 mol of compound (IX).
Examples of the phosphorus oxyhalide compound include phosphorus oxychloride, phosphorus oxybromide and the like. The amount of the phosphorus oxyhalide compound to be used is preferably about 1 to about 10 mol per 1 mol of compound (IX).
Examples of the solvent that does not adversely influence the reaction include halogenated hydrocarbons such as chloroform, dichloromethane and the like; aromatic hydrocarbons such as nitrobenzene and the like; amides such as N,N-dimethylformamide and the like, and the like. These solvents may be used in a mixture at an appropriate ratio.
The reaction temperature is generally about -80 to about 200°C, preferably about 0 to about 150°C.
The reaction time is generally about 0.5 to about 40 hr.

Compound (VII) and compound (VIII) used as starting material compounds for the above-mentioned Method D can be produced according to a method known per se.

Compound (III-4), compound (III-5), compound (III-6), compound (III-7), compound (III-8) and compound (III-9) which are compound (III) wherein Y is a bond, R² is -CH=CR⁸R⁹ (compound (III-4)), -CH₂-CR⁸R⁹ (compound (III-5)), -CH(R¹⁰)-OH (compound (III-6)), -CH₂-R¹⁰ (compound (III-7)), -CH=NOR¹¹ (compound (III-8)) or -CN (compound (III-9)) wherein R⁸, R⁹, R¹⁰ and R¹¹ are as defined below can be produced, for example, according to the following Method E.

### [Method E]

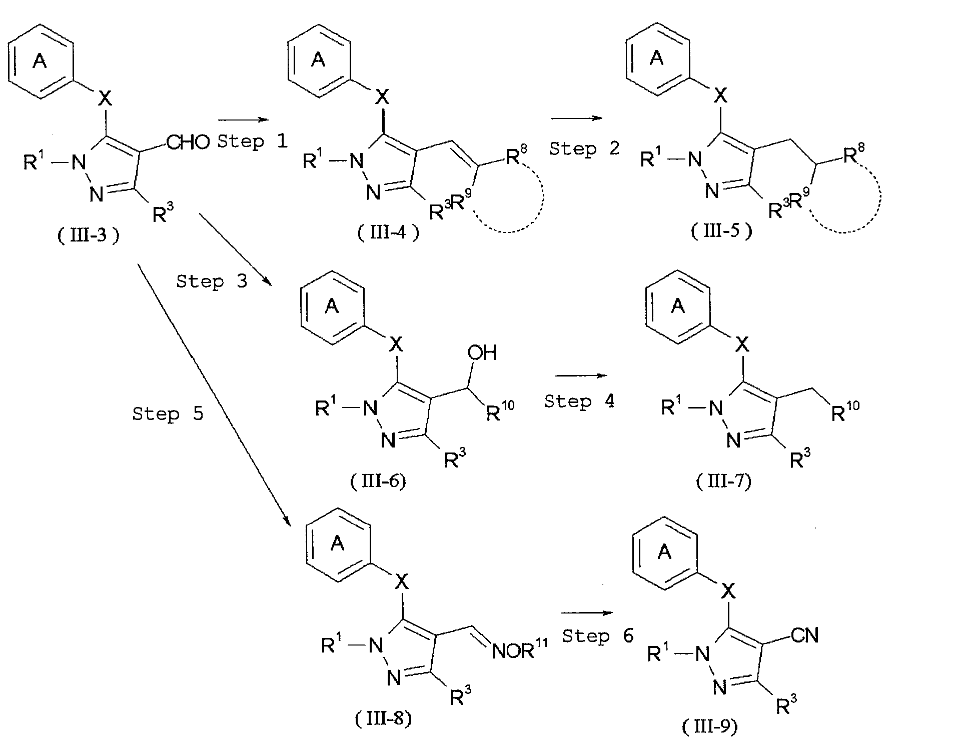

wherein
R⁸ is a hydrogen atom, a C₁₋₆ alkyl group, a benzyl group optionally substituted by C₁₋₆ alkyl group(s), a C₆₋₁₄ aryl group optionally substituted by C₁₋₆ alkyl group(s), a cyano group, a 2-pyridyl group, a 4-cyanophenyl group or an ethoxycarbonyl group,
R⁹, R¹⁰ and R¹¹ are each independently a hydrogen atom, a C₁₋₆ alkyl group, a benzyl group optionally substituted by C₁₋₆ alkyl group (s) or a C₆₋₁₄ aryl group optionally substituted by C₁₋₆ alkyl group(s), or
R⁸ and R⁹ are optionally bonded to each other to form an optionally substituted ring, and
the other symbols are as defined above.
Examples of the "optionally substituted ring" formed by
R⁸ and R⁹ bonded to each other include
   (1) a C₃₋₁₀ cycloalkyl group;
   (2) a C₆₋₁₄ aryl group optionally substituted by 1 to 3 substituents selected from
      (a) a C₁₋₆ alkyl group optionally substituted by 1 to 3 halogen atoms,
      (b) a hydroxy group,
      (c) a C₁₋₆ alkoxy group optionally substituted by 1 to 3 halogen atoms, and
      (d) a halogen atom;
   (3) an aromatic heterocyclic group optionally substituted by 1 to 3 substituents selected from
      (a) a C₁₋₆ alkyl group optionally substituted by 1 to 3 halogen atoms,
      (b) a hydroxy group,
      (c) a C₁₋₆ alkoxy group optionally substituted by 1 to 3 halogen atoms, and
      (d) a halogen atom;
   (4) a monocyclic non-aromatic heterocyclic group optionally substituted by 1 to 3 substituents selected from
      (a) a C₁₋₆ alkyl group optionally substituted by 1 to 3 halogen atoms,
      (b) a hydroxy group,
      (c) a C₁₋₆ alkoxy group optionally substituted by 1 to 3 halogen atoms, and
      (d) a halogen atom,
   and the like.
R⁸ is preferably a hydrogen atom, methyl, ethyl, phenyl, benzyl, cyano, 2-pyridyl, 4-cyanophenyl, ethoxycarbonyl, tert-butoxycarbonyl or the like.
R⁹, R¹⁰ and R¹¹ is preferably each independently a hydrogen atom, methyl, ethyl, phenyl, benzyl or the like.

### [Step 1]

In this step, compound (III-4) can be produced by subjecting compound (III-3) to a carbon-addition reaction.
This reaction is carried out according to a method known per se, for example, a method using an organic phosphorus reagent, or a method of reacting compound (III-3) with an organic metal reagent, and treating the resulting compound with an acid or a base, and the like.

When an organic phosphorus reagent is used for the production of compound (III-4), the reaction is carried out in the presence of a base, in a solvent that does not adversely influence the reaction.
Examples of the base include alkali metal salts such as potassium hydroxide, sodium hydroxide, sodium hydrogen carbonate, potassium carbonate and the like; amines such as pyridine, triethylamine, N,N-diisopropylethylamine, N,N-dimethylaniline, 1,8-diazabicyclo[5.4.0]undec-7-ene and the like; metal hydrides such as potassium hydride, sodium hydride and the like; alkali metal C₁₋₆ alkoxides such as sodium methoxide, sodium ethoxide, potassium tert-butoxide and the like, and the like. The amount of the base to be used is generally 1 to 50 mol, preferably 1 to 10 mol, per 1 mol of compound (III-3).
Examples of the organic phosphorus reagent include methyltriphenylphosphonium bromide, ethyltriphenylphosphonium bromide, triphenyl(2-pyridylmethyl)phosphonium chloride, diethyl cyanomethylphosphonate, (4-cyanobenzyl)(triphenyl)phosphonium chloride, ethyl (diethoxyphosphoryl)acetate and the like. The amount of the organic phosphorus reagent to be used is generally 1 to 50 mol, preferably 1 to 10 mol, per 1 mol of compound (III-3).
Examples of the solvent that does not adversely influence the reaction include aromatic hydrocarbons such as benzene, toluene, xylene and the like; aliphatic hydrocarbons such as hexane, heptane and the like; ethers such as diethyl ether, diisopropyl ether, tert-butyl methyl ether, tetrahydrofuran, dioxane, dimethoxyethane and the like; halogenated hydrocarbons such as chloroform, dichloromethane and the like, and the like. These solvents may be used in a mixture at an appropriate ratio.
The reaction temperature is generally about -100 to about 150°C, preferably about -10 to about 100°C.
The reaction time is generally about 0.5 to about 20 hr.

When the method of reacting compound (III-3) with an organic metal reagent, and treating the resulting compound with an acid or a base are employed for the production of compound (III-4), the reaction is carried out in a solvent that does not adversely influence the reaction.
Examples of the organic metal reagent include methylmagnesium chloride, trimethylsilylmethylmagnesium chloride, 4-cyanophenylmagnesium bromide, 4-cyanophenylmagnesium iodide, n-butyllithium and the like. The amount of the organic metal reagent to be used is generally 1 to 50 mol, preferably 1 to 10 mol, per 1 mol of compound (III-3).
Examples of the acid include mineral acids such as hydrochloric acid, sulfuric acid and the like; Lewis acids such as boron trichloride, boron tribromide and the like; organic acids such as trifluoroacetic acid, p-toluenesulfonic acid and the like, and the like.
Examples of the base include alkali metal salts such as potassium hydroxide, sodium hydroxide, sodium hydrogen carbonate, potassium carbonate and the like; amines such as pyridine, triethylamine, N,N-diisopropylethylamine, N,N-dimethylaniline, 1,8-diazabicyclo[5.4.0]undec-7-ene, piperidine and the like; metal hydrides such as potassium hydride, sodium hydride and the like; alkali metal C₁₋₆ alkoxides such as sodium methoxide, sodium ethoxide, potassium tert-butoxide and the like, and the like.
The amount of the acid or base to be used is generally 1 to 50 mol, preferably 1 to 10 mol, per 1 mol of compound (III-3), respectively.
Examples of the solvent that does not adversely influence the reaction include aromatic hydrocarbons such as benzene, toluene, xylene and the like; aliphatic hydrocarbons such as hexane, heptane and the like; ethers such as diethyl ether, diisopropyl ether, tert-butyl methyl ether, tetrahydrofuran, dioxane, dimethoxyethane and the like; halogenated hydrocarbons such as chloroform, dichloromethane and the like, and the like. These solvents may be used in a mixture at an appropriate ratio.
The reaction temperature is generally about -100 to about 150°C, preferably about -10 to about 100°C.
The reaction time is generally about 0.5 to about 20 hr.

### [Step 2]

In this step, compound (III-5) can be produced by subjecting compound (III-4) to a hydrogenation reaction.
This reaction is carried out, for example, in the presence of a metal catalyst such as palladium-carbon, palladium-carbon ethylenediamine complex, palladium black, palladium chloride, platinum oxide, platinum black, platinum-palladium, Raney-nickel, Raney-cobalt and the like and a hydrogen source, in a solvent that does not adversely influence the reaction.
The amount of the metal catalyst to be used is generally 0.001 to 1000 mol, preferably 0.01 to 100 mol, per 1 mol of compound (III-4).
Examples of the hydrogen source include hydrogen gas, formic acid, amine salts of formic acid, phosphinates, hydrazine and the like.
As the solvent that does not adversely influence the reaction, those exemplified in the above-mentioned Step 1 can be used.
The reaction temperature is generally about -100 to about 150°C, preferably about -10 to about 100°C.
The reaction time is generally about 0.5 to about 20 hr.

### [Step 3]

In this step, compound (III-6) can be produced by reacting compound (III-3) with an organic metal reagent or a reducing agent.

When the method of reacting compound (III-3) with an organic metal reagent is employed for the production of compound (III-6), the reaction is carried out in a solvent that does not adversely influence the reaction.
Examples of the organic metal reagent include methylmagnesium chloride, cyclohexymagnesium bromide, phenyllithium and the like. The amount of the organic metal reagent to be used is generally 1 to 50 mol, preferably 1 to 10 mol, per 1 mol of compound (III-3).
Examples of the solvent that does not adversely influence the reaction include aromatic hydrocarbons such as benzene, toluene, xylene and the like; aliphatic hydrocarbons such as hexane, heptane and the like; ethers such as diethyl ether, diisopropyl ether, tert-butyl methyl ether, tetrahydrofuran, dioxane, dimethoxyethane and the like; halogenated hydrocarbons such as chloroform, dichloromethane and the like, and the like. These solvents may be used in a mixture at an appropriate ratio.
The reaction temperature is generally about -100 to about 150°C, preferably about -10 to about 100°C.
The reaction time is generally about 0.5 to about 20 hr.

When the method of reacting compound (III-3) with a reducing agent is employed for the production of compound (III-6), the reaction is carried out in a solvent that does not adversely influence the reaction.
Examples of the reducing agent include metal hydrogen compounds such as sodium bis(2-methoxyethoxy)aluminum hydride, diisobutylaluminum hydride and the like; metal hydrogen complex compounds such as sodium borohydride, sodium cyanoborohydride, lithium aluminum hydride, sodium aluminum hydride and the like; boron hydride compounds such as borane and the like, and the like. The amount of the reducing agent to be used is generally 1 to 20 mol per 1 mol of compound (III-3).
Examples of the solvent that does not adversely influence the reaction include alcohols such as methanol, ethanol, propanol, 2-propanol, butanol, isobutanol, tert-butanol and the like; aromatic hydrocarbons such as benzene, toluene, xylene and the like; aliphatic hydrocarbons such as hexane, heptane and the like; ethers such as diethyl ether, diisopropyl ether, tert-butyl methyl ether, tetrahydrofuran, dioxane, dimethoxyethane and the like; amides such as N,N-dimethylformamide, N,N-dimethylacetamide, N-methylpyrrolidone and the like; halogenated hydrocarbons such as dichloromethane, chloroform, 1,2-dichloroethane, 1,1,2,2-tetrachloroethane and the like; water and the like. These solvents may be used in a mixture at an appropriate ratio.
The reaction temperature is generally -70 to 150°C, preferably -20 to 100°C.
The reaction time is generally 0.1 to 100 hr, preferably 0.1 to 40 hr.

### [Step 4]

In this step, compound (III-7) can be produced by reducing the hydroxy group of compound (III-6).
This reaction is generally carried out in the presence of a trialkylsilane as a reducing agent, in trifluoroacetic acid.
Examples of the trialkylsilane include triethylsilane, tributylsilane and the like. The amount of the trialkylsilane to be used is generally 1 to 20 mol per 1 mol of compound (III-6).
The amount of the trifluoroacetic acid to be used is generally 1 to 10000 mol per 1 mol of compound (III-6).
The reaction temperature is generally -70 to 150°C, preferably 0 to 100°C.
The reaction time is generally 0.1 to 100 hr, preferably 0.1 to 40 hr.

### [Step 5]

In this step, compound (III-8) can be produced by reacting compound (III-3) with R¹¹ONH₂.
This reaction is carried out in a solvent that does not adversely influence the reaction. The reaction may be carried out in the presence of an acid or a base.
Examples of the acid and base include those exemplified in the above-mentioned Step 1, respectively. The amount of the acid or base to be used is generally 0 to 50 mol, preferably 0 to 10 mol, per 1 mol of compound (III-3), respectively.
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; alcohols such as methanol, ethanol and the like; ketones such as acetone and the like; aliphatic hydrocarbons such as hexane, heptane and the like; water and the like. These solvents may be used in a mixture at an appropriate ratio.
The reaction temperature is generally about -100 to about 150°C, preferably about -10 to about 100°C.
The reaction time is generally about 0.5 to about 20 hr.

### [Step 6]

In this step, compound (III-9) can be produced by converting the hydroxyimino group of compound (III-8) wherein R¹¹ is a hydrogen atom to a nitrile group.
This reaction is carried out in the presence of trichloroacetyl chloride and a base, in a solvent that does not adversely influence the reaction.
The amount of the trichloroacetyl chloride to be used is generally 1 to 20 mol, preferably 1 to 5 mol, per 1 mol of compound (III-8).
Examples of the base include those exemplified in the above-mentioned Step 1. The amount of the base to be used is generally 1 to 50 mol, preferably 1 to 10 mol, per 1 mol of compound (III-8).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like, and the like. These solvents may be used in a mixture at an appropriate ratio.
The reaction temperature is generally about -100 to about 150°C, preferably about -10 to about 100°C.
The reaction time is generally about 0.5 to about 20 hr.

This reaction can also be carried out in the presence of acetic anhydride. In this case, the reaction may be carried out in a solvent that does not adversely influence the reaction.
The amount of the acetic anhydride to be used is generally 1 to 1000 mol, preferably 1 to 100 mol, per 1 mol of compound (III-8).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like, and the like. These solvents may be used in a mixture at an appropriate ratio.
The reaction temperature is generally about 0 to about 200°C, preferably about 20 to about 150°C.
The reaction time is generally about 0.5 to about 20 hr.

Compound (III-3) used as a starting material compound for the above-mentioned Method E can be produced, for example, according to the above-mentioned Method C, the below-mentioned Method H, Method P or Method R or a method analogous thereto.

Compound (III-11), which is compound (III) wherein Y is O can be produced, for example, according to the following Method F.

### [Method F]

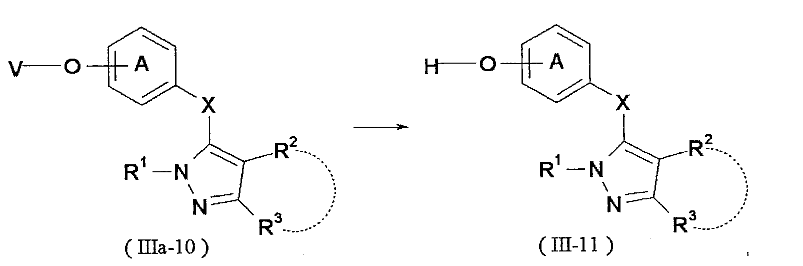

wherein V is a protecting group, and the other symbols are as defined above.
Examples of the "protecting group" for V include methyl, benzyl, p-methoxybenzyl, methoxymethyl, tetrahydropyranyl, trimethylsilyl, triethylsilyl, tert-butyldimethylsilyl, tert-butyldiphenylsilyl and the like.

In this method, compound (III-11) can be produced by eliminating the protecting group V of compound (IIIa-10).
This reaction is carried out according to a method known per se, for example, a method of treating compound (IIIa-10) with an acid, a method of subjecting compound (IIIa-10) to a hydrogenation reaction, a method of eliminating the silyl group with a fluoride, or the like.
When V is methyl, benzyl, p-methoxybenzyl, methoxymethyl, tetrahydropyranyl, trimethylsilyl, triethylsilyl, tert-butyldimethylsilyl or tert-butyldiphenylsilyl, this reaction is carried out, for example, in the presence of an acid, in a solvent that does not adversely influence the reaction, according to a conventional method.
Examples of the acid include mineral acids such as hydrochloric acid, sulfuric acid and the like; Lewis acids such as boron trichloride, boron tribromide and the like; organic acids such as trifluoroacetic acid, p-toluenesulfonic acid and the like; and hydrogen chloride-methanol, hydrogen chloride-ethyl acetate and the like, which are prepared by dissolving hydrogen chloride gas in a solvent such as methanol, ethyl acetate and the like. The amount of the acid to be used is generally 0.01 to 500 mol, preferably 0.01 to 5 mol, per 1 mol of compound (IIIa-10).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; alcohols such as methanol, ethanol and the like; ketones such as acetone and the like; water and the like. These solvents may be used in a mixture at an appropriate ratio.
The reaction temperature is generally about -80 to about 150°C, preferably about -10 to about 100°C.
The reaction time is generally about 0.5 to about 20 hr.
When V is benzyl or p-methoxybenzyl, this reaction is carried out, for example, in the presence of a metal catalyst and a hydrogen source, in a solvent that does not adversely influence the reaction, according to a conventional method.
This reaction is carried out according to the above-mentioned Method E, Step 2.
When V is trimethylsilyl, triethylsilyl, tert-butyldimethylsilyl or tert-butyldiphenylsilyl, this reaction is carried out, for example, in the presence of a fluoride, in a solvent that does not adversely influence the reaction, according to a conventional method.
Examples of the fluoride include potassium fluoride, cesium fluoride, tributylammoniume fluoride and the like. The amount of the fluoride to be used is generally 1 to 10 mol, preferably 1 to 3 mol, per 1 mol of compound (IIIa-10).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; alcohols such as methanol, ethanol and the like; ketones such as acetone and the like; water and the like. These solvents may be used in a mixture at an appropriate ratio.
The reaction temperature is generally about -80 to about 150°C, preferably about -10 to about 100°C.
The reaction time is generally about 0.5 to about 20 hr.

Compound (IIIa-10) used as a starting material compound for the above-mentioned Method F can be produced, for example, using a starting material compound produced according to a method known per se, according to the below-mentioned Method C', Method E', Method H', Method I', Method K', Method M', Method R, Method S, Method T, Method AF, Method AH, Method AJ, Method AK, Method AT or Method AV or a method analogous thereto.

Compound (III-14) and compound (III-17), which are compound (III) wherein Y is O, NR⁵ wherein R⁵ is a hydrogen atom or a C₁₋₆ alkyl group, S, SO or S(O)₂, and R² is methyl (compound (III-14)) or -CH₂-CN (compound (III-17)), can be produced, for example, according to the following Method G.

### [Method G]

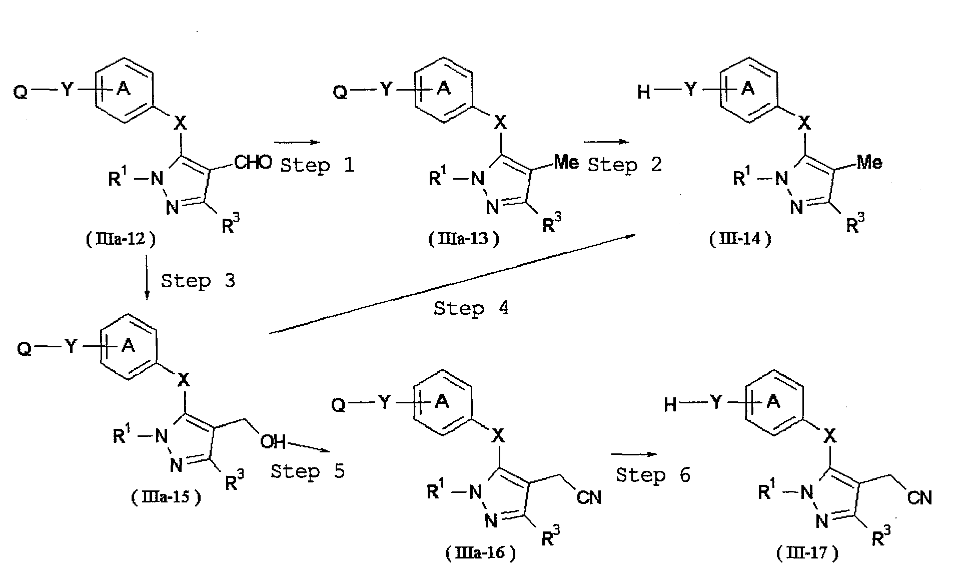

wherein Q is a hydrogen atom or the above-mentioned V, and the other symbols are as defined above.

### [Step 1]

In this step, compound (IIIa-13) can be produced by reducing the formyl group of compound (IIIa-12).
This reaction is carried out according to a method known per se, for example, the method described in Journal of Organic Chemistry page 1327 (1951), or a method analogous thereto. That is, this reaction is generally carried out in the presence of hydrazine and a base, in a solvent that does not adversely influence the reaction.
The amount of the hydrazine to be used is preferably about 1 to about 5 mol per 1 mol of compound (IIIa-12).
Examples of the base include alkali metal salts such as potassium hydroxide, sodium hydroxide, potassium hydroxide, sodium hydrogen carbonate, potassium carbonate and the like; amines such as pyridine, triethylamine, N,N-diisopropylethylamine, N,N-dimethylaniline, 1,8-diazabicyclo[5.4.0]undec-7-ene and the like; metal hydrides such as potassium hydride, sodium hydride and the like; alkali metal C₁₋₆ alkoxides such as sodium methoxide, sodium ethoxide, potassium tert-butoxide and the like, and the like. The amount of the base to be used is generally 1 to 50 mol, preferably 1 to 10 mol, per 1 mol of compound (IIIa-12).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; alcohols such as methanol, ethanol, ethylene glycol, diethylene glycol and the like. These solvents may be used in a mixture at an appropriate ratio, or in a mixture with water at an appropriate ratio.
The reaction temperature is generally about -80 to about 250°C, preferably about -10 to about 150°C.
The reaction time is generally about 0.5 to about 20 hr.

### [Step 2]

In this step, compound (III-14) can be produced by converting Q of compound (IIIa-13) wherein Q is V to a hydrogen atom. This reaction is carried out in the same manner as in the above-mentioned Method F.

### [Step 3]

In this step, compound (IIIa-15) can be produced by reducing the formyl group of compound (IIIa-12).
This reaction is generally carried out in the presence of a reducing agent, in a solvent that does not adversely influence the reaction.
Examples of the reducing agent include metal hydrogen compounds such as sodium bis(2-methoxyethoxy)aluminum hydride, diisobutylaluminum hydride and the like; metal hydrogen complex compounds such as sodium borohydride, sodium cyanoborohydride, lithium aluminum hydride, sodium aluminum hydride and the like, and the like. The amount of the reducing agent to be used is generally 1 to 20 mol per 1 mol of compound (IIIa-12).
Examples of the solvent that does not adversely influence the reaction include alcohols such as methanol, ethanol, propanol, 2-propanol, butanol, isobutanol, tert-butanol and the like; aromatic hydrocarbons such as benzene, toluene, xylene and the like; aliphatic hydrocarbons such as hexane, heptane and the like; ethers such as diethyl ether, diisopropyl ether, tert-butyl methyl ether, tetrahydrofuran, dioxane, dimethoxyethane and the like; amides such as N,N-dimethylformamide, N,N-dimethylacetamide, N-methylpyrrolidone and the like; halogenated hydrocarbons such as dichloromethane, chloroform, 1,2-dichloroethane, 1,1,2,2-tetrachloroethane and the like; water and the like. These solvents may be used in a mixture at an appropriate ratio.
The reaction temperature is generally -70 to 150°C, preferably -20 to 100°C.
The reaction time is generally 0.1 to 100 hr, preferably 0.1 to 40 hr.

### [Step 4]

In this step, compound (III-14) can be produced by simultaneously converting Q of compound (IIIa-15) wherein Q is V to a hydrogen atom, and reducing the hydroxy group of compound (IIIa-15).
This reaction is generally carried out in the presence of a trialkylsilane as a reducing agent, in trifluoroacetic acid.
Examples of the trialkylsilane include triethylsilane, tributylsilane and the like. The amount of the trialkylsilane to be used is generally 1 to 20 mol per 1 mol of compound (IIIa-15).
The amount of the trifluoroacetic acid to be used is generally 1 to 10000 mol per 1 mol of compound (IIIa-15).
The reaction temperature is generally -70 to 150°C, preferably 0 to 100°C.
The reaction time is generally 0.1 to 100 hr, preferably 0.1 to 40 hr.

### [Step 5]

In this step, compound (IIIa-16) can be produced by cyanating the hydroxy group of compound (IIIa-15).
This reaction is generally carried out in the presence of acetone cyanohydrin, an organic phosphorus compound and an electrophilic agent, in a solvent that does not adversely influence the reaction.
The amount of the acetone cyanohydrin to be used is generally 1 to 10 mol per 1 mol of compound (IIIa-15).
Examples of the organic phosphorus compound include triphenylphosphine, tributylphosphine, trimethylphosphine and the like. The amount of the organic phosphorus reagent to be used is generally 1 to 10 mol per 1 mol of compound (IIIa-15).
Examples of the electrophilic agent include diethyl azodicarboxylate, diisopropyl azodicarboxylate, 1,1'-azodicarbonyldipiperidine and the like. The amount of the electrophilic agent to be used is generally 1 to 10 mol per 1 mol of compound (IIIa-15).
Examples of the solvent that does not adversely influence the reaction include aromatic hydrocarbons such as benzene, toluene, xylene and the like; aliphatic hydrocarbons such as hexane, heptane and the like; ethers such as diethyl ether, diisopropyl ether, tert-butyl methyl ether, tetrahydrofuran, dioxane, dimethoxyethane and the like; amides such as N,N-dimethylformamide, N,N-dimethylacetamide, N-methylpyrrolidone and the like; halogenated hydrocarbons such as dichloromethane, chloroform, 1,2-dichloroethane, 1,1,2,2-tetrachloroethane and the like, and the like. These solvents may be used in a mixture at an appropriate ratio.
The reaction temperature is generally -70 to 150°C, preferably -20 to 100°C.
The reaction time is generally 0.1 to 100 hr, preferably 0.1 to 40 hr.

### [Step 6]

In this step, compound (III-17) can be produced by converting Q of compound (IIIa-16) wherein Q is V to a hydrogen atom. This reaction is carried out in the same manner as in the above-mentioned Method F.

Compound (IIIa-12) used as a starting material compound for the above-mentioned Method G can be produced, for example, using a starting material compound produced according to a method known per se, according to the below-mentioned Method C', Method E', Method H', Method R, Method S, Method T or Method AG or a method analogous thereto.

Compound (III-26), which is compound (III) wherein Y is a bond, and R² and R³ are bonded to each other to form

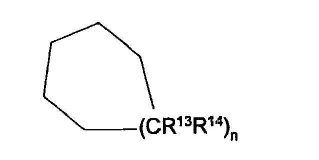

wherein R¹³, R¹⁴ and n are as defined below, can be produced, for example, according to the following Method H.

### [Method H]

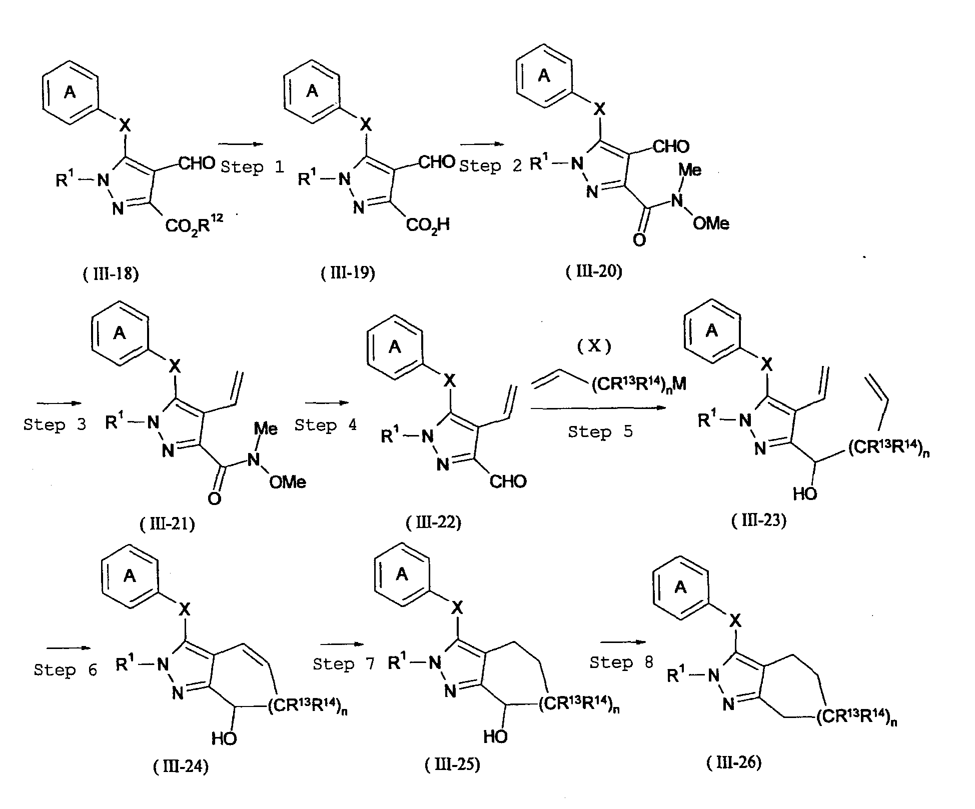

wherein
R¹² is a C₁₋₁₀ alkyl group, a benzyl group optionally substituted by C₁₋₆ alkyl group (s) or a C₆₋₁₄ aryl group optionally substituted by C₁₋₆ alkyl group(s),
R¹³ and R¹⁴ are each independently a hydrogen atom, a C₁₋₆ alkyl group, a benzyl group optionally substituted by C₁₋₆ alkyl group (s) or a C₆₋₁₄ aryl group optionally substituted by C₁₋₆ alkyl group(s),
M is a metal or a halogenated metal, n is an integer of 0 to 5, and the other symbols are as defined above.
R¹² is preferably methyl, ethyl, tert-butyl, benzyl, phenyl or the like.
R¹³ and R¹⁴ are each independently preferably a hydrogen atom, methyl, ethyl or the like.
Examples of the metal or halogenated metal for M include lithium, halogenated magnesium, halogenated silyl and the like.

### [Step 1]

In this step, compound (III-19) can be produced by subjecting compound (III-18) to hydrolysis. This reaction is carried out according to the above-mentioned Method B, Step 1.

### [Step 2]

In this step, compound (III-20) can be produced by condensing compound (III-19) with methoxy(methyl)amine. This reaction is carried out according to the above-mentioned Method B, Step 2.

### [Step 3]

In this step, compound (III-21) can be produced by subjecting compound (III-20) to a one-carbon-addition reaction. This reaction is carried out according to the above-mentioned Method E, Step 1.

### [Step 4]

In this step, compound (III-22) can be produced by reducing compound (III-21). This reaction is carried out according to the above-mentioned Method G, Step 3.

### [Step 5]

In this step, compound (III-23) can be produced by reacting compound (III-22) with compound (X).
This reaction is carried out in a solvent that does not adversely influence the reaction.
Examples of compound (X) include allylmagnesium chloride, homoallylmagnesium chloride, vinylmagnesium chloride and the like. The amount of compound (X) to be used is generally 1 to 50 mol, preferably 1 to 10 mol, per 1 mol of compound (III-22).
Examples of the solvent that does not adversely influence the reaction include aromatic hydrocarbons such as benzene, toluene, xylene and the like; aliphatic hydrocarbons such as hexane, heptane and the like; ethers such as diethyl ether, diisopropyl ether, tert-butyl methyl ether, tetrahydrofuran, dioxane, dimethoxyethane and the like; halogenated hydrocarbons such as chloroform, dichloromethane and the like, and the like. These solvents may be used in a mixture at an appropriate ratio.
The reaction temperature is generally about -100 to about 150°C, preferably about -10 to about 100°C.
The reaction time is generally about 0.5 to about 20 hr.

### [Step 6]

In this step, compound (III-24) can be produced by subjecting compound (III-23) to an intramolecular metathesis. This reaction is generally carried out in the presence of a catalyst, in a solvent that does not adversely influence the reaction.
Examples of the catalyst include ruthenium catalysts such as dichloro(3-phenyl-1H-inden-1-ylidene)bis(tricyclohexylphosphine)ruthenium (IV) and the like. The amount of the catalyst to be used is generally 0.0001 to 50 mol, preferably 0.001 to 0.2 mol, per 1 mol of compound (III-23).
Examples of the solvent that does not adversely influence the reaction include aromatic hydrocarbons such as benzene, toluene, xylene and the like; aliphatic hydrocarbons such as hexane, heptane and the like; ethers such as diethyl ether, diisopropyl ether, tert-butyl methyl ether, tetrahydrofuran, dioxane, dimethoxyethane and the like; halogenated hydrocarbons such as chloroform, dichloromethane and the like, and the like. These solvents may be used in a mixture at an appropriate ratio.
The reaction temperature is generally about -100 to about 150°C, preferably about -10 to about 100°C.
The reaction time is generally about 0.5 to about 20 hr.

### [Step 7]

In this step, compound (III-25) can be produced by reducing the carbon-carbon double bond of compound (III-24). This reaction is carried out according to the above-mentioned Method E, Step 2.

### [Step 8]

In this step, compound (III-26) can be produced by reducing the hydroxy group of compound (III-25). This reaction is carried out according to the above-mentioned Method E, Step 4.

Compound (III-18) used as a starting material compound for the above-mentioned Method H can be produced, for example, according to the above-mentioned Method C or a method analogous thereto. Compound (X) can be produced according to a method known per se.

Compound (III-29) and compound (III-30), which are compound (III) wherein Y is a bond, R² and R³ are bonded to each other to form a cyclohexene (compound (III-29)) or a cyclohexadiene (compound (III-30)) can be produced, for example, according to the following Method I.

### [Method 1]

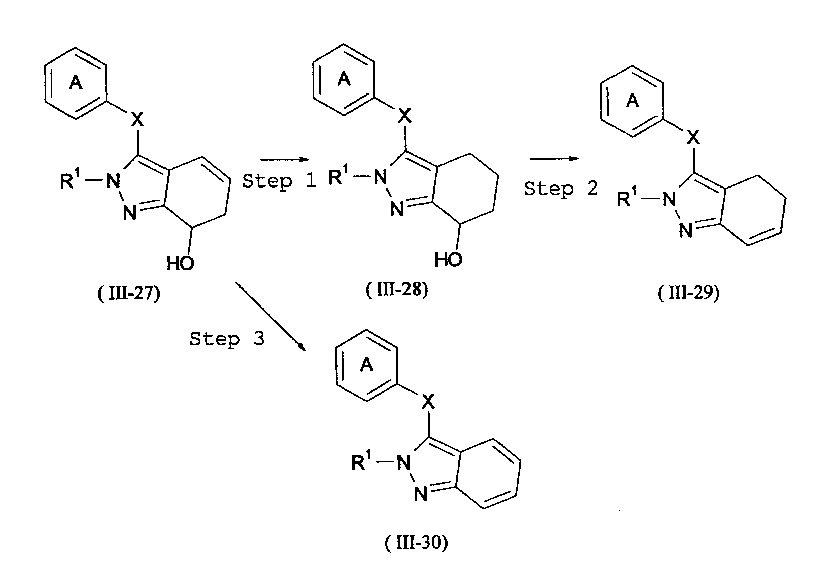

wherein the symbols in the formula are as defined above.

### [Step 1]

In this step, compound (III-28) can be produced by reducing the carbon-carbon double bond of compound (III-27). This reaction is carried out according to the above-mentioned Method E, Step 2.

### [Step 2]

In this step, compound (III-29) can be produced by treating compound (III-28) with an acid or a base. This reaction is carried out in a solvent that does not adversely influence the reaction.
Examples of the acid include mineral acids such as hydrochloric acid, sulfuric acid and the like; Lewis acids such as boron trichloride, boron tribromide and the like; organic acids such as trifluoroacetic acid, p-toluenesulfonic acid and the like, and the like.
Examples of the base include alkali metal salts such as potassium hydroxide, sodium hydroxide, sodium hydrogen carbonate, potassium carbonate and the like; amines such as pyridine, triethylamine, N,N-diisopropylethylamine, N,N-dimethylaniline, 1,8-diazabicyclo[5.4.0]undec-7-ene and the like; metal hydrides such as potassium hydride, sodium hydride and the like; alkali metal C₁₋₆ alkoxides such as sodium methoxide, sodium ethoxide, potassium tert-butoxide and the like, and the like.
The amount of the acid or base to be used is generally 1 to 50 mol, preferably 1 to 10 mol, per 1 mol of compound (III-28).
Examples of the solvent that does not adversely influence the reaction include aromatic hydrocarbons such as benzene, toluene, xylene and the like; aliphatic hydrocarbons such as hexane, heptane and the like; ethers such as diethyl ether, diisopropyl ether, tert-butyl methyl ether, tetrahydrofuran, dioxane, dimethoxyethane and the like; halogenated hydrocarbons such as chloroform, dichloromethane and the like; alcohols such as methanol, ethanol, ethylene glycol, diethylene glycol and the like; water and the like. These solvents may be used in a mixture at an appropriate ratio.
The reaction temperature is generally about -100 to about 150°C, preferably about -10 to about 100°C.
The reaction time is generally about 0.5 to about 100 hr.

### [Step 3]

In this step, compound (III-30) can be produced by treating compound (III-27) with an acid or a base. This reaction is carried out according to the above-mentioned Step 2.

Compound (III-27) used as a starting material compound for the above-mentioned Method I can be produced, for example, according to the above-mentioned Method C or Method H, the below-mentioned Method J, Method O or Method S or a method analogous thereto.

Compound (I-5) and compound (I-6), which are compound (I) wherein Y is a bond, and -Z-W is -CH=C(R¹⁵)CO₂R¹⁶ (compound (I-5)) or -CH₂-CH(R¹⁵)CO₂R¹⁶ (compound (I-6)) wherein R¹⁵ and R¹⁶ are as defined below can be produced, for example, according to the following Method J.

### [Method J]

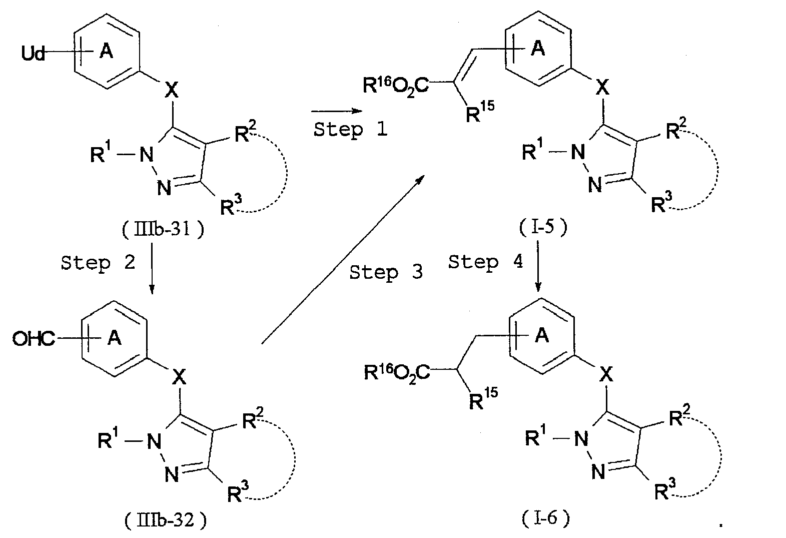

wherein
Ud is a chlorine atom, a bromine atom, an iodine atom or a trifluoromethanesulfonyloxy group,
R¹⁵ is a hydrogen atom, a C₁₋₆ alkyl group, a benzyl group optionally substituted by C₁₋₆ alkyl group (s) or a C₆₋₁₄ aryl group optionally substituted by C₁₋₆ alkyl group(s), R¹⁶ is a hydrogen atom, a C₁₋₁₀ alkyl group, a benzyl group optionally substituted by C₁₋₆ alkyl group (s) or a C₆₋₁₄ aryl group optionally substituted by C₁₋₆ alkyl group(s), and the other symbols are as defined above.
R¹⁵ is preferably a hydrogen atom, methyl, ethyl, tert-butyl, benzyl, phenyl or the like.
R¹⁶ is preferably a hydrogen atom, methyl, ethyl or the like.

### [Step 1]

In this step, compound (I-5) can be produced by reacting compound (IIIb-31) with a α,β-unsaturated alkane acid derivative in the presence of a transition metal catalyst and a base. This reaction is carried out in a solvent that does not adversely influence the reaction.
Examples of the α,β-unsaturated alkane acid derivative include methyl acrylate, ethyl methacrylate and the like. The amount of the α,β-unsaturated alkane acid derivative to be used is generally 1 to 50 mol, preferably 1 to 10 mol, per 1 mol of compound (IIIb-31).
Examples of the transition metal catalyst include palladium(II) acetate, dichlorobistriphenylphosphine palladium(II) and the like. The amount of the transition metal catalyst to be used is generally 0.0001 to 1 mol, preferably 0.01 to 0.2 mol, per 1 mol of compound (IIIb-31).
Examples of the base include alkali metal salts such as potassium hydroxide, sodium hydroxide, sodium hydrogen carbonate, potassium carbonate and the like; amines such as pyridine, triethylamine, N,N-diisopropylethylamine, N,N-dimethylaniline, 1,8-diazabicyclo[5.4.0]undec-7-ene and the like; metal hydrides such as potassium hydride, sodium hydride and the like; alkali metal C₁₋₆ alkoxides such as sodium methoxide, sodium ethoxide, potassium tert-butoxide and the like; alkanoic acids such as sodium acetate and the like. The amount of the base to be used is generally 1 to 50 mol, preferably 1 to 10 mol, per 1 mol of compound (IIIb-31).
Examples of the solvent that does not adversely influence the reaction include aromatic hydrocarbons such as benzene, toluene, xylene and the like; aliphatic hydrocarbons such as hexane, heptane and the like; ethers such as diethyl ether, diisopropyl ether, tert-butyl methyl ether, tetrahydrofuran, dioxane, dimethoxyethane and the like; halogenated hydrocarbons such as chloroform, dichloromethane and the like; amides such as N,N-dimethylformamide, N,N-dimethylacetamide, N-methylpyrrolidone and the like; alcohols such as methanol, ethanol, ethylene glycol, diethylene glycol and the like, and the like. These solvents may be used in a mixture at an appropriate ratio.
The reaction temperature is generally about -100 to about 200°C, preferably about -10 to about 150°C.
The reaction time is generally about 0.5 to about 100 hr.

### [Step 2]

In this step, compound (IIIb-32) can be produced by reacting compound (IIIb-31) wherein Ud is a bromine atom or an iodine atom with an alkyllithium reagent, and reacting the resulting compound with N,N-dimethylformamide.
This reaction is carried out in a solvent that does not adversely influence the reaction.
Examples of the alkyllithium reagent include n-butyllithium, tert-butyllithium, methyllithium and the like. The amount of the alkyllithium reagent to be used is generally 1 to 10 mol, preferably 1 to 3 mol, per 1 mol of compound (IIIb-31).
The amount of the N,N-dimethylformamide to be used is generally 1 to 50 mol, preferably 1 to 10 mol, per 1 mol of compound (IIIb-31).
Examples of the solvent that does not adversely influence the reaction include aromatic hydrocarbons such as benzene, toluene, xylene and the like; aliphatic hydrocarbons such as hexane, heptane and the like; ethers such as diethyl ether, diisopropyl ether, tert-butyl methyl ether, tetrahydrofuran, dioxane, dimethoxyethane and the like, and the like. These solvents may be used in a mixture at an appropriate ratio.
The reaction temperature is generally about -100 to about 100°C, preferably about -80 to about 50°C.
The reaction time is generally about 0.5 to about 100 hr.

### [Step 3]

In this step, compound (I-5) can be produced by subjecting compound (IIIb-32) to a carbon-addition reaction.
This reaction is generally carried out using an organic phosphorus reagent in the presence of a base, in a solvent that does not adversely influence the reaction.
Examples of the base include alkali metal salts such as potassium hydroxide, sodium hydroxide, sodium hydrogen carbonate, potassium carbonate and the like; amines such as pyridine, triethylamine, N,N-diisopropylethylamine, N,N-dimethylaniline, 1,8-diazabicyclo[5.4.0]undec-7-ene and the like; metal hydrides such as potassium hydride, sodium hydride and the like; alkali metal C₁₋₆ alkoxides such as sodium methoxide, sodium ethoxide, potassium tert-butoxide and the like, and the like. The amount of the base to be used is generally 1 to 50 mol, preferably 1 to 10 mol, per 1 mol of compound (IIIb-32).
Examples of the organic phosphorus reagent include trimethyl phosphonoacetate, methyl diethylphosphonoacetate, triethyl phosphonoacetate, tert-butyl diethylphosphonoacetate, ethyl 2-(diethoxyphosphoryl)propionate and the like. The amount of the organic phosphorus reagent to be used is generally 1 to 50 mol, preferably 1 to 10 mol, per 1 mol of compound (IIIb-32).
Examples of the solvent that does not adversely influence the reaction include aromatic hydrocarbons such as benzene, toluene, xylene and the like; aliphatic hydrocarbons such as hexane, heptane and the like; ethers such as diethyl ether, diisopropyl ether, tert-butyl methyl ether, tetrahydrofuran, dioxane, dimethoxyethane and the like; halogenated hydrocarbons such as chloroform, dichloromethane and the like; amides such as N,N-dimethylformamide, N,N-dimethylacetamide, N-methylpyrrolidone and the like; sulfoxides such as dimethylsulfoxide and the like; alcohols such as methanol, ethanol, isopropanol, tert-butanol and the like, and the like. These solvents may be used in a mixture at an appropriate ratio.
The reaction temperature is generally about -100 to about 150°C, preferably about -10 to about 100°C.
The reaction time is generally about 0.5 to about 20 hr.

### [Step 4]

In this step, compound (I-6) can be produced by subjecting compound (I-5) to a hydrogenation reaction. This reaction is carried out according to the above-mentioned Method E, Step 2.

Compound (IIIb-31) used as a starting material compound for the above-mentioned Method J can be produced, for example, using a starting material compound produced according to a method known per se, according to the below-mentioned Method C', Method E', Method H', Method I', Method K', Method M' or Method T or a method analogous thereto.

Compound (III-34), which is compound (III) wherein Y is a bond, and R² is -CH₂-OR¹⁷ wherein R¹⁷ is as defined below, can be produced, for example, according to the following Method K.

### [Method K]

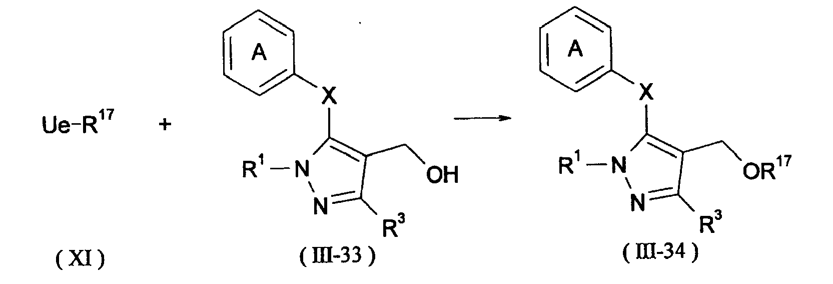

wherein Ue is a leaving group, R¹⁷ is a C₁₋₆ alkyl group, a benzyl group or a C₆₋₁₄ aryl group, and the other symbols are as defined above.
Examples of the "leaving group" for Ue include a chlorine atom, a bromine atom, an iodine atom, a benzenesulfonyloxy group, a p-toluenesulfonyloxy group, a methanesulfonyloxy group, a trifluoromethanesulfonyloxy group and the like.
R¹⁷ is preferably methyl, ethyl, benzyl, phenyl or the like.

In this method, compound (III-34) can be produced by reacting compound (III-33) with compound (XI).
This reaction is carried out in the presence of a base, in a solvent that does not adversely influence the reaction.
Examples of the base include alkali metal hydrides such as sodium hydride, potassium hydride and the like; alkali metal hydroxides such as sodium hydroxide, potassium hydroxide, lithium hydroxide and the like; alkaline earth metal hydroxides such as magnesium hydroxide, calcium hydroxide, barium hydroxide and the like, and the like. The amount of the base to be used is preferably about 1 to about 5 mol per 1 mol of compound (III-33).
The amount of compound (XI) to be used is preferably about 1 to about 10 mol per 1 mol of compound (III-33).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; ketones such as acetone and the like; and water. These solvents may be used in a mixture at an appropriate ratio.
The reaction temperature is generally about -80 to about 150°C, preferably about -10 to about 100°C.
The reaction time is generally about 0.5 to about 20 hr.

Compound (III-33) used as a starting material compound for the above-mentioned Method K can be produced, for example, according to the above-mentioned Method C or Method E, the below-mentioned Method O, Method P, Method R or Method S or a method analogous thereto. Compound (XI) can be produced according to a method known per se.

Compound (III-36), which is compound (III) wherein H-Y-ring A is 2,4-dichlorophenol, can be produced, for example, according to the following Method L.

### [Method L]

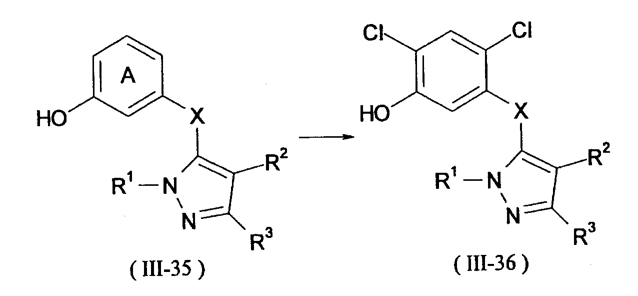

wherein the symbols in the formula are as defined above.
In this method, compound (III-36) can be produced by reacting compound (III-35) with a chlorinating agent. This reaction is carried out in a solvent that does not adversely influence the reaction.
Examples of the chlorinating agent include sulfuryl chloride, N-chlorosuccinimide and the like. The amount of the chlorinating agent to be used is preferably about 1 to about 5 mol per 1 mol of compound (III-35).
Examples of the solvent that does not adversely influence the reaction include ethers such as diethyl ether, tetrahydrofuran, dioxane and the like; halogenated hydrocarbons such as chloroform, dichloromethane, 1,2-dichloroethane and the like; aromatic hydrocarbons such as nitrobenzene and the like; amides such as N,N-dimethylformamide and the like; sulfoxides such as dimethylsulfoxide and the like; and water. These solvents may be used in a mixture at an appropriate ratio.
The reaction temperature is generally about -80 to about 150°C, preferably about -10 to about 100°C.
The reaction time is generally about 0.5 to about 20 hr.
Compound (III-35) used as a starting material compound for the above-mentioned Method L can be produced, for example, according to the above-mentioned Method F or Method G, the below-mentioned Method C', Method E', Method H', Method I', Method K', Method M', Method R, Method S, Method T, Method U, Method AF, Method AG, Method AH, Method AJ, Method AK, Method AT, Method AU or Method AV or a method analogous thereto.

Compound (III-38), which is compound (III) wherein Y is a bond, and R² and R³ are bonded to each other to form a ring represented by the formula

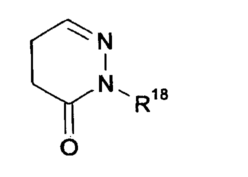

wherein R¹⁸ is as defined below, can be produced, for example, according to the following Method M.

### [Method M]

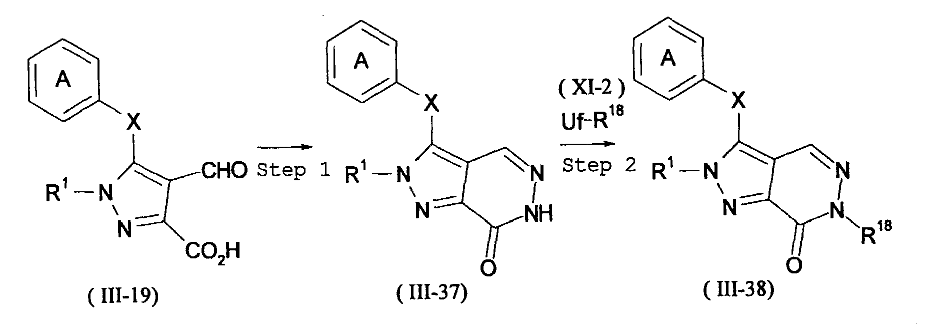

wherein Uf is a leaving group, R¹⁸ is a C₁₋₆ alkyl group, a benzyl group optionally substituted by C₁₋₆ alkyl group(s) or a C₆₋₁₄ aryl group optionally substituted by C₁₋₆ alkyl group(s), and the other symbols are as defined above.
Examples of the "leaving group" for Uf include those similar to the above-mentioned "leaving group" for Ue.
R¹⁸ is preferably methyl, ethyl, benzyl or the like.

### [Step 1]

In this step, compound (III-37) can be produced by reacting compound (III-19) with hydrazine.
This reaction is carried out in a solvent that does not adversely influence the reaction.
The amount of the hydrazine to be used is preferably about 1 to about 5 mol per 1 mol of compound (III-19).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; alcohols such as methanol, ethanol and the like; ketones such as acetone and the like; and water. These solvents may be used in a mixture at an appropriate ratio.
The reaction temperature is generally about -80 to about 150°C, preferably about -10 to about 100°C.
The reaction time is generally about 0.5 to about 20 hr.

### [Step 2]

In this step, compound (III-38) can be produced by reacting compound (III-37) with compound (XI-2). This reaction is carried out according to the above-mentioned Method K.

Compound (III-19) used as a starting material compound for the above-mentioned Method M can be produced, for example, according to the below-mentioned Method C' or a method analogous thereto.

Compound (I-7), which is compound (I) wherein Z-Y is T wherein T is as defined below, and W is 5-oxo-1,2,4-oxadiazol-3-yl, can be produced, for example, according to the following Method N.

### [Method N]

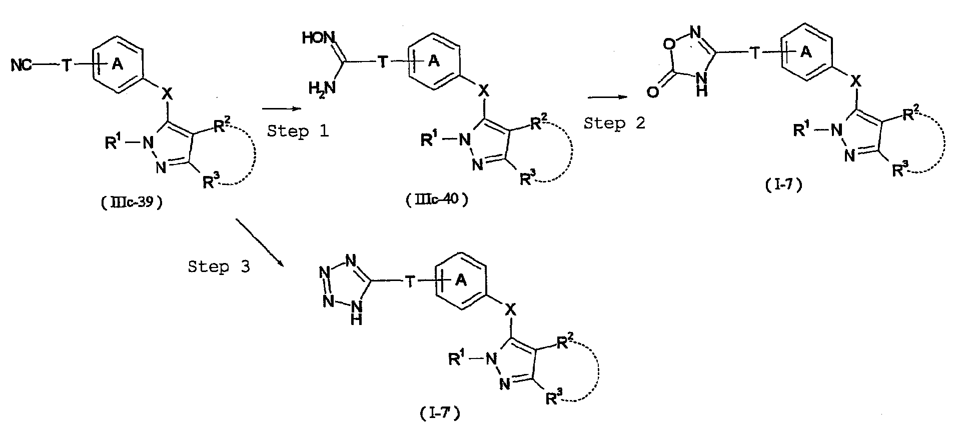

wherein T is a bond, CH₂, CH₂O CH(CH₃)O, CH=CH or CH₂CH₂O, and the other symbols are as defined above.

### [Step 1]

In this step, compound (IIIc-40) can be produced by reacting compound (IIIc-39) with hydroxylamine.
This reaction is carried out in a solvent that does not adversely influence the reaction.
The amount of the hydroxylamine to be used is preferably about 1 to about 10 mol per 1 mol of compound (IIIc-39).
Examples of the solvent that does not adversely influence the reaction include ethers such as diethyl ether, tetrahydrofuran, dioxane and the like; halogenated hydrocarbons such as chloroform, dichloromethane, 1,2-dichloroethane and the like; aromatic hydrocarbons such as nitrobenzene and the like; amides such as N,N-dimethylformamide and the like; sulfoxides such as dimethylsulfoxide and the like; ketones such as acetone and the like; and water. These solvents may be used in a mixture at an appropriate ratio.
The reaction temperature is generally about -80 to about 180°C, preferably about -10 to about 120°C.
The reaction time is generally about 0.5 to about 20 hr.

### [Step 2]

In this step, compound (I-7) can be produced by reacting compound (IIIc-40) with a carbonylating agent such as 1,1'-carbonylbis-1H-imidazole, bis(trichloromethyl) carbonate, diphosgene, triphosgene and the like.
This reaction is carried out in the presence of a base, in a solvent that does not adversely influence the reaction.
The amount of the carbonylating agent such as 1,1'-carbonylbis-1H-imidazole and the like to be used is generally 1 to 50 mol, preferably 1 to 5 mol, per 1 mol of compound (IIIc-40).
Examples of the base include alkali metal salts such as potassium hydroxide, sodium hydroxide, sodium hydrogen carbonate, potassium carbonate and the like; amines such as pyridine, triethylamine, N,N-diisopropylethylamine, N,N-dimethylaniline, 1,8-diazabicyclo[5.4.0]undec-7-ene and the like; metal hydrides such as potassium hydride, sodium hydride and the like; alkali metal C₁₋₆ alkoxides such as sodium methoxide, sodium ethoxide, potassium tert-butoxide and the like, and the like. The amount of the base to be used is generally 1 to 50 mol, preferably 1 to 5 mol, per 1 mol of compound (IIIc-40).
Examples of the solvent that does not adversely influence the reaction include aromatic hydrocarbons such as benzene, toluene, xylene and the like; aliphatic hydrocarbons such as hexane, heptane and the like; ethers such as diethyl ether, diisopropyl ether, tert-butyl methyl ether, tetrahydrofuran, dioxane, dimethoxyethane and the like; halogenated hydrocarbons such as chloroform, dichloromethane and the like; amides such as N,N-dimethylformamide, N,N-dimethylacetamide, N-methylpyrrolidone and the like; sulfoxides such as dimethylsulfoxide and the like, and the like. These solvents may be used in a mixture at an appropriate ratio.
The reaction temperature is generally about -100 to about 150°C, preferably about -10 to about 100°C.
The reaction time is generally about 0.5 to about 20 hr.

### [Step 3]

In this step, compound (I-7') can be produced by reacting compound (IIIc-39) with sodium azide.
This reaction is carried out in a solvent that does not adversely influence the reaction.
The amount of the sodium azide to be used is preferably about 1 to about 10 mol per 1 mol of compound (IIIc-39).
Examples of the solvent that does not adversely influence the reaction include aromatic hydrocarbons such as nitrobenzene and the like; amides such as N,N-dimethylformamide and the like; sulfoxides such as dimethylsulfoxide and the like; and water. These solvents may be used in a mixture at an appropriate ratio.
The reaction temperature is generally about -80 to about 180°C, preferably about 20 to about 150°C.
The reaction time is generally about 0.5 to about 20 hr.

Compound (IIIc-39) used as a starting material compound for the above-mentioned Method N can be produced, for example, using a starting material compound produced according to a method known per se, according to the above-mentioned Method C, Method E, Method H, Method I, Method K or Method M, the below-mentioned Method C', Method E', Method H', Method I', Method K', Method M', Method O or Method P or a method analogous thereto.

Compound (III-2'), which is compound (III) wherein Y is O, NR⁵ wherein R⁵ is a hydrogen atom or a C₁₋₆ alkyl group, S, SO or S(O)₂, can be produced, for example, according to the following Method C'.

### [Method C']

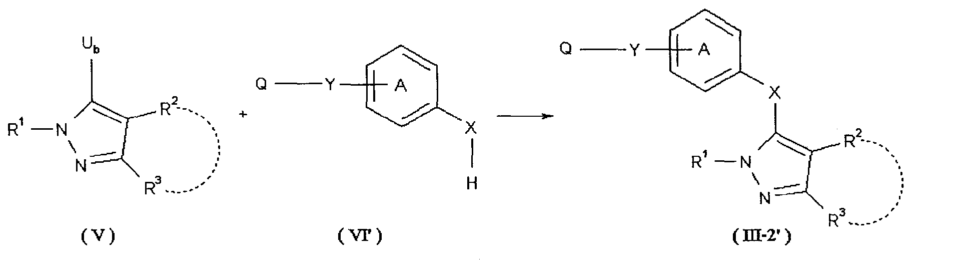

wherein the symbols in the formula are as defined above.
In this method, compound (III-2') can be produced by reacting compound (V) with compound (VI').
This reaction is carried out according to the above-mentioned Method C.
Compound (VI') used as a starting material compound for the above-mentioned Method C' can be produced according to a method known per se, or the method shown in the below-mentioned Reference Examples 1, 80, 107, 108, 109, 110, 114, 115, 156, 157, 182, 185, 186, 187 or 191.

Compound (I) can also be produced, for example, according to the following Method C''.

### [Method C'']

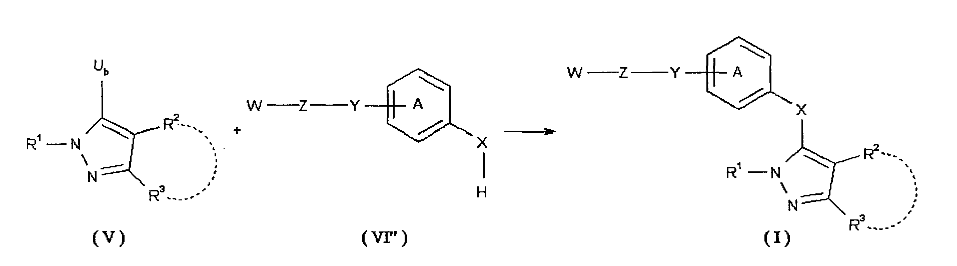

wherein the symbols in the formula are as defined above.
In this method, compound (I) can be produced by reacting compound (V) with compound (VI").
This reaction is carried out according to the above-mentioned Method C.
Compound (VI") used as a starting material compound for the above-mentioned Method C" can be produced according to a method known per se, or the method shown in the below-mentioned Reference Example 4.

Compound (III-4'), compound (III-5'), compound (III-6'), compound (III-7'), compound (III-8') and compound (III-9'), which are compound (III) wherein Y is O, NR⁵ wherein R⁵ is a hydrogen atom or a C₁₋₆ alkyl group, S, SO or S(O)₂, and R² is - CH=CR⁸R⁹ (compound (III-4')), -CH₂-CR⁸R⁹ (compound (III-5')), - CH(R¹⁰)-OH (compound (III-6')), -CH₂-R¹⁰ (compound (III-7')), - CH=NOR¹¹ (compound (III-8')) or -CN (compound (III-9')) wherein R⁸, R⁹, R¹⁰ and R¹¹ are as defined above, can be produced, for example, according to the following Method E'.

### [Method E']

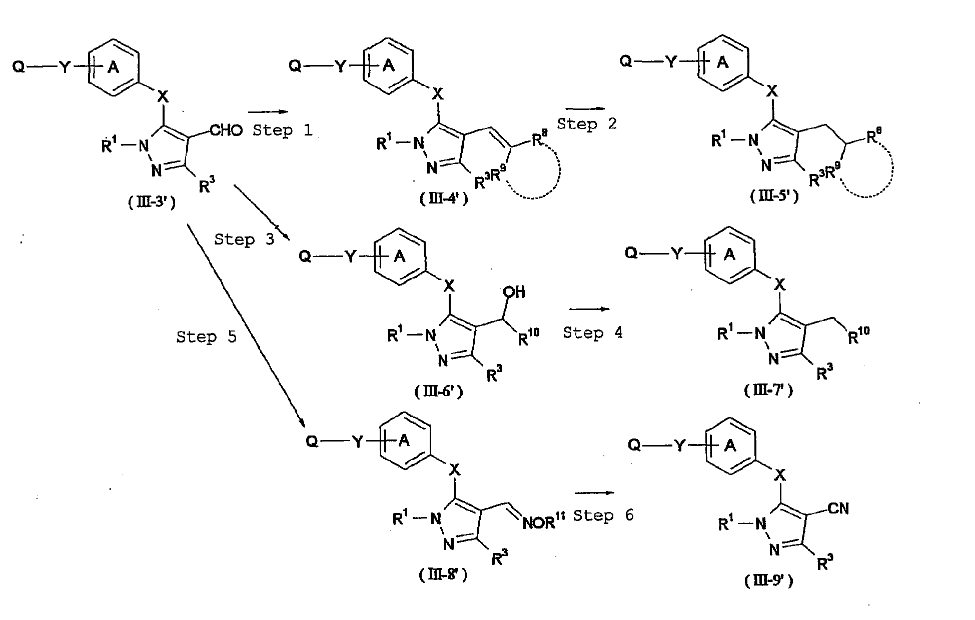

wherein the symbols in the formula are as defined above.

### [Step 1]

In this step, compound (III-4') can be produced by subjecting compound (III-3') to a carbon-addition reaction.
This reaction is carried out according to the above-mentioned Method E, Step 1.
In this reaction, when Q is V, the elimination reaction of Q may simultaneously proceed to convert Q to a hydrogen atom.

### [Step 2]

In this step, compound (III-5') can be produced by subjecting compound (III-4') to a hydrogenation reaction.
This reaction is carried out according to the above-mentioned Method E, Step 2.
In this reaction, when Q is V, the elimination reaction of Q may simultaneously proceed to convert Q to a hydrogen atom.

### [Step 3]

In this step, compound (III-6') can be produced by reacting compound (III-3') with an organic metal reagent or a reducing agent.
This reaction is carried out according to the above-mentioned Method E, Step 3.

### [Step 4]

In this step, compound (III-7') can be produced by reducing the hydroxy group of compound (III-6').
This reaction is carried out according to the above-mentioned Method E, Step 4.
In this reaction, when Q is V, the elimination reaction of Q may simultaneously proceed to convert Q to a hydrogen atom.

### [Step 5]

In this step, compound (III-8') can be produced by reacting compound (III-3') with R¹¹ONH₂.
This reaction is carried out according to the above-mentioned Method E, Step 5.

### [Step 6]

In this step, compound (III-9') can be produced by converting the hydroxyimino group of compound (III-8') wherein R¹¹ is a hydrogen atom to a nitrile group.
This reaction is carried out according to the above-mentioned Method E, Step 6.

Compound (III-3') used as a starting material compound for the above-mentioned Method E' can be produced, for example, according to the above-mentioned Method C' or Method L, the below-mentioned Method H', Method P, Method R, Method AF or Method AG or a method analogous thereto, or the method shown in the below-mentioned Reference Example 204 or 208.

Compound (I-9), compound (I-10), compound (I-11), compound (I-12), compound (I-13) and compound (I-14), which are compound (I) wherein R² is -CH=CR⁸R⁹ (compound (I-9)), -CH₂-CR⁸R⁹ (compound (I-10)), -CH(R¹⁰)-OH (compound (I-11)), -CH₂-R¹⁰ (compound (I-12)), -CH=NOR¹¹ (compound (I-13)) or -CN (compound (I-14)) wherein R⁸, R⁹, R¹⁰ and R¹¹ are as defined above can be produced, for example, according to the following Method E''.

### [Method E'']

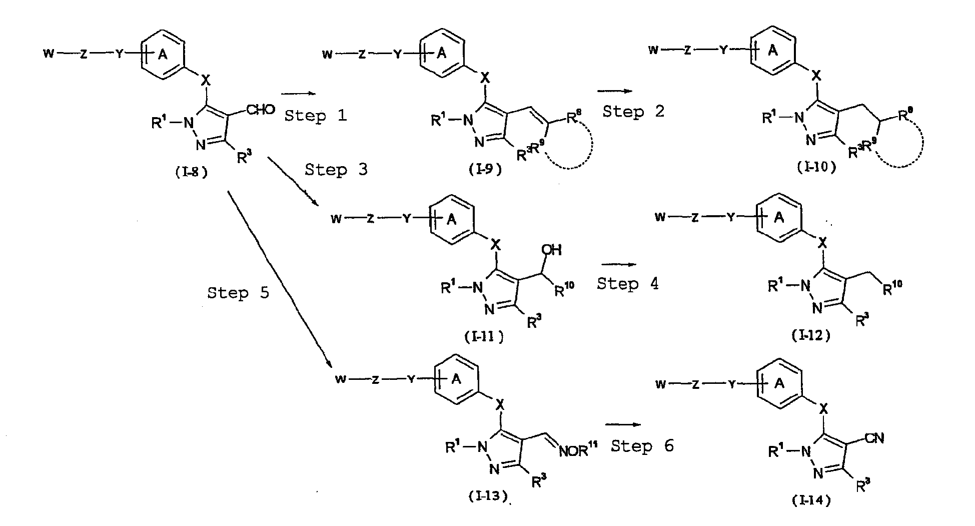

wherein the symbols in the formula are as defined above.

### [Step 1]

In this step, compound (I-9) can be produced by subjecting compound (I-8) to a carbon-addition reaction.
This reaction is carried out according to the above-mentioned Method E, Step 1.

### [Step 2]

In this step, compound (I-10) can be produced by subjecting compound (I-9) to a hydrogenation reaction.
This reaction is carried out according to the above-mentioned Method E, Step 2.

### [Step 3]

In this step, compound (I-11) can be produced by reacting compound (I-8) with an organic metal reagent or a reducing agent.
This reaction is carried out according to the above-mentioned Method E, Step 3.

### [Step 4]

In this step, compound (I-12) can be produced by reducing the hydroxy group of compound (I-11).
This reaction is carried out according to the above-mentioned Method E, Step 4.

### [Step 5]

In this step, compound (I-13) can be produced by reacting compound (I-8) with R¹¹ONH₂.
This reaction is carried out according to the above-mentioned Method E, Step 5.

### [Step 6]

In this step, compound (I-14) can be produced by converting the hydroxyimino group of compound (I-13) wherein R¹¹ is a hydrogen atom to a nitrile group.
This reaction is carried out according to the above-mentioned Method E, Step 6.

Compound (I-8) used as a starting material compound for the above-mentioned Method E'' can be produced, for example, according to the above-mentioned Method A, Method B, or Method C'', the below-mentioned Method V, Method W, Method Y, Method AD, Method AG or Method AI or a method analogous thereto.

Compound (III-26'), which is compound (III) wherein Y is O, NR⁵ wherein R⁵ is a hydrogen atom or a C₁₋₆ alkyl group, S, SO or S(O)₂, and R² and R³ are bonded to each other to form

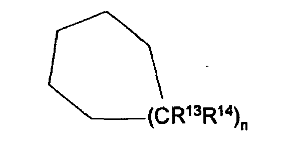

wherein R¹³, R¹⁴ and n are as defined above, can be produced, for example, according to the following Method H'.

### [Method H']

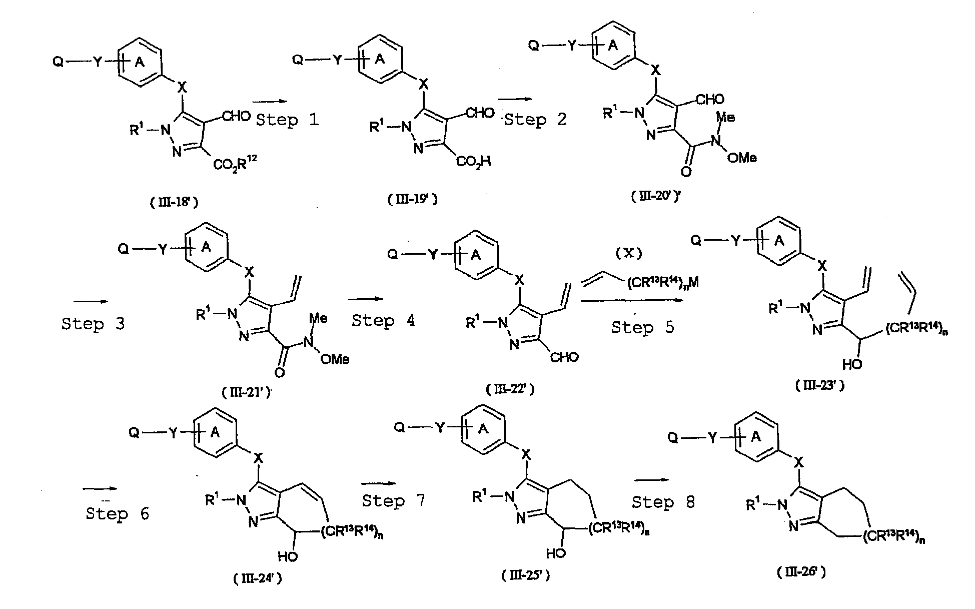

wherein the symbols in the formula are as defined above.

### [Step 1]

In this step, compound (III-19') can be produced by subjecting compound (III-18') to hydrolysis. This reaction is carried out according to the above-mentioned Method B, Step 1.

### [Step 2]

In this step, compound (III-20') can be produced by condensing compound (III-19') with methoxy(methyl)amine. This reaction is carried out according to the above-mentioned Method B, Step 2.

### [Step 3]

In this step, compound (III-21') can be produced by subjecting compound (III-20') to a one-carbon-addition reaction. This reaction is carried out according to the above-mentioned Method E, Step 1.

### [Step 4]

In this step, compound (III-22') can be produced by reducing compound (III-21'). This reaction is carried out according to the above-mentioned Method G, Step 3.

### [Step 5]

In this step, compound (III-23') can be produced by reacting compound (III-22') with compound (X). This reaction is carried out according to the above-mentioned Method H, Step 5.

### [Step 6]

In this step, compound (III-24') can be produced by subjecting compound (III-23') to an intramolecular metathesis. This reaction is carried out according to the above-mentioned Method H, Step 6.

### [Step 7]

In this step, compound (III-25') can be produced by reducing the carbon-carbon double bond of compound (III-24'). This reaction is carried out according to the above-mentioned Method E, Step 2.
In this reaction, when Q is V, the elimination reaction of Q may simultaneously proceed to convert Q to a hydrogen atom.

### [Step 8]

In this step, compound (III-26') can be produced by reducing the hydroxy group of compound (III-25'). This reaction is carried out according to the above-mentioned Method E, Step 4.
In this reaction, when Q is V, the elimination reaction of Q may simultaneously proceed to convert Q to a hydrogen atom.

Compound (III-18') used as a starting material compound for the above-mentioned Method H' can be produced, for example, according to the above-mentioned Method C', Method F or Method L, the below-mentioned Method P or Method AG or a method analogous thereto. Compound (X) can be produced according to a method known per se.

Compound (III-29') and compound (III-30'), which are compound (III) wherein Y is O, NR⁵ wherein R⁵ is a hydrogen atom or a C₁₋₆ alkyl group, S, SO or S(O)₂, and R² and R³ are bonded to each other to form cyclohexene (compound (III-29')) or cyclohexadiene (compound (III-30')) can be produced, for example, according to the following Method I'.

### [Method I']

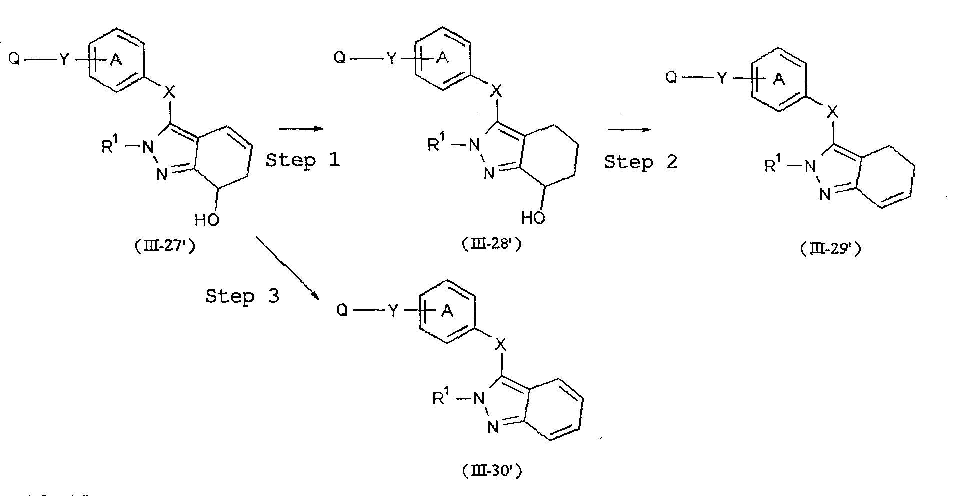

wherein the symbols in the formula are as defined above.

### [Step 1]

In this step, compound (III-28') can be produced by reducing the carbon-carbon double bond of compound (III-27'). This reaction is carried out according to the above-mentioned Method E, Step 2.
In this reaction, when Q is V, the elimination reaction of Q may simultaneously proceed to convert Q to a hydrogen atom.

### [Step 2]

In this step, compound (III-29') can be produced by treating compound (III-28') with an acid or a base. This reaction is carried out according to the above-mentioned Method I, Step 2.
In this reaction, when Q is V, the elimination reaction of Q may simultaneously proceed to convert Q to a hydrogen atom.

### [Step 3]

In this step, compound (III-30') can be produced by treating compound (III-27') with an acid or a base. This reaction is carried out according to the above-mentioned Method I, Step 2.
In this reaction, when Q is V, the elimination reaction of Q may simultaneously proceed to convert Q to a hydrogen atom.

Compound (III-27') used as a starting material compound for the above-mentioned Method I' can be produced, for example, according to the above-mentioned Method C', Method F, Method H' or Method L, the below-mentioned Method P, Method S, Method AF, Method AG or Method AH or a method analogous thereto.

Compound (III-34'), which is compound (III) wherein Y is O, NR⁵ wherein R⁵ is a hydrogen atom or a C₁₋₆ alkyl group, S, SO or S(O)₂, and R² is -CH₂-OR¹⁷ wherein R¹⁷ is as defined above, can be produced, for example, according to the following Method K'.

### [Method K']

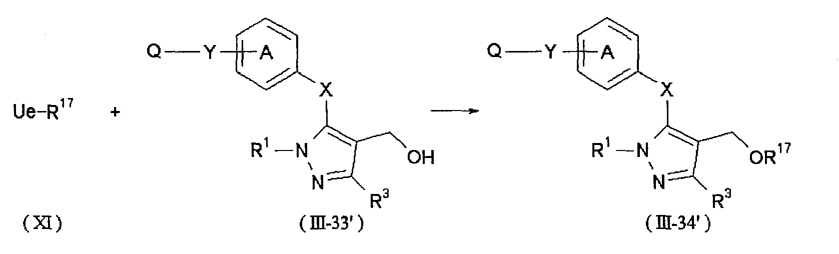

wherein the symbols in the formula are as defined above.
In this method, compound (III-34') can be produced by reacting compound (III-33') with compound (XI). This reaction is carried out according to the above-mentioned Method K.

Compound (III-33') used as a starting material compound for the above-mentioned Method K' can be produced, for example, according to the above-mentioned Method C' or Method E', the below-mentioned Method P, Method R, Method S, Method AF, Method AG or Method AH or a method analogous thereto. Compound (XI) can be produced according to a method known per se.

Compound (III-38'), which is compound (III) wherein Y is O, NR⁵ wherein R⁵ is a hydrogen atom or a C₁₋₆ alkyl group, S, SO or S(O)₂, and R² and R³ are bonded to each other to form a ring represented by the formula

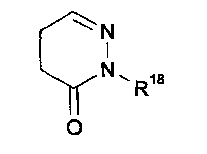

wherein R¹⁸ is as defined above, can be produced, for example, according to the following Method M'.

### [Method M']

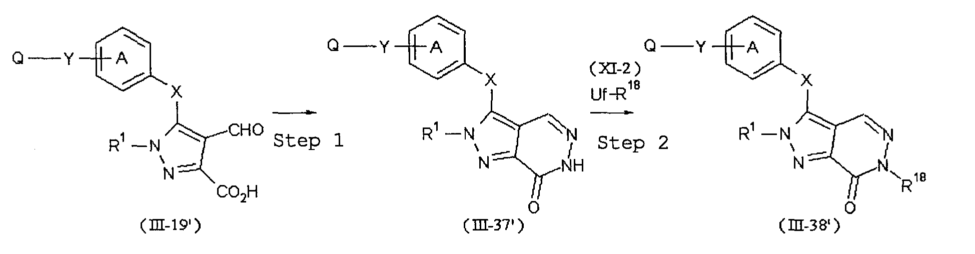

wherein the symbols in the formula are as defined above.

### [Step 1]

In this step, compound (III-37') can be produced by reacting compound (III-19') with hydrazine. This reaction is carried out according to the above-mentioned Method M, Step 1.

### [Step 2]

In this step, compound (III-38') can be produced by reacting compound (III-37') with compound (XI-2). This reaction is carried out according to the above-mentioned Method K.

Compound (III-19') used as a starting material compound for the above-mentioned Method M' can be produced, for example, according to the above-mentioned Method C', Method F or Method L, the below-mentioned Method P, Method R or Method AG or a method analogous thereto.

Compound (IIIc-39'), compound (IIIc-39'') and compound (IIIc-39'''), which are compound (IIIc-39) wherein T is a bond (compound (IIIc-39')), -CH=CH- (compound (IIIc-39'')) or -CH₂-(compound (IIIc-39''')), and compound (I-15), which is compound (I) wherein W is 1,3-thiazolidine-2,4-dione, and Z is -CH₂-, can be produced, for example, according to the following Method O.

### [Method O]

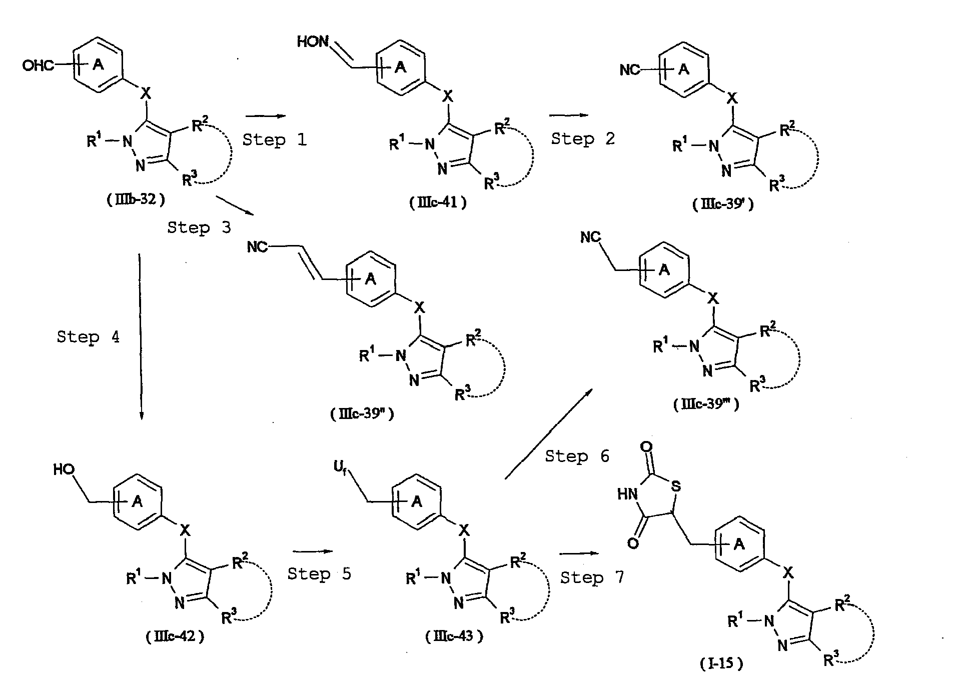

wherein U_{f} is a chlorine atom, a bromine atom or an iodine atom, and the other symbols are as defined above.

### [Step 1]

In this step, compound (IIIc-41) can be produced by reacting compound (IIIb-32) with hydroxylamine. This reaction is carried out according to the above-mentioned Method E, Step 5.

### [Step 2]

In this step, compound (IIIc-39') can be produced by converting the hydroxyimino group of compound (IIIc-41) to a nitrile. This reaction is carried out according to the above-mentioned Method E, Step 6.

### [Step 3]

In this step, compound (IIIc-39'') can be produced by reacting compound (IIIb-32) with diethyl cyanomethylphosphonate. This reaction is carried out according to the above-mentioned Method E, Step 1.

### [Step 4]

In this step, compound (IIIc-42) can be produced by reducing the formyl group of compound (IIIb-32). This reaction is carried out according to the above-mentioned Method G, Step 3.

### [Step 5]

In this step, compound (IIIc-43) can be produced by converting the hydroxy group of compound (IIIc-42) to U_{f}.
This reaction is carried out in the presence of a halogenating agent, in a solvent that does not adversely influence the reaction.
Examples of the halogenating agent include hydrochloric acid, hydrobromic acid, hydroiodic acid, thionyl chloride, phosphorus pentachloride, phosphorus tribromide, phosphorus triiodide, bromine, iodine and the like. The amount of the halogenating agent to be used is preferably about 1 to about 5 mol per 1 mol of compound (IIIc-42).
In addition, this reaction may be carried out in the co-presence of an organic phosphorus compound such as triphenylphosphine and the like as a reaction activator. The amount of the reaction activator to be used is preferably about 1 to about 10 mol per 1 mol of compound (IIIc-42).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; and water. These solvents may be used in a mixture at an appropriate ratio.
The reaction temperature is generally about -80 to about 150°C, preferably about -10 to about 100°C.
The reaction time is generally about 0.5 to about 20 hr.

### [Step 6]

In this step, compound (IIIc-39''') can be produced by converting U_{f} of compound (IIIc-43) to a cyano group.
This reaction is carried out in the presence of a cyanating agent, in a solvent that does not adversely influence the reaction.
Examples of the cyanating agent include sodium cyanide, potassium cyanide, trimethylsilyl cyanide and the like. The amount of the cyanating agent to be used is preferably about 1 to about 5 mol per 1 mol of compound (IIIc-43).
Examples of the solvent that does not adversely influence the reaction include aromatic hydrocarbons such as benzene, toluene, xylene and the like; aliphatic hydrocarbons such as hexane, heptane and the like; ethers such as diethyl ether, diisopropyl ether, tert-butyl methyl ether, tetrahydrofuran, dioxane, dimethoxyethane and the like; halogenated hydrocarbons such as chloroform, dichloromethane and the like; amides such as N,N-dimethylformamide, N,N-dimethylacetamide, N-methylpyrrolidone and the like; sulfoxides such as dimethylsulfoxide and the like; alcohols such as methanol, ethanol, isopropanol, tert-butanol and the like; nitriles such as acetonitrile and the like; and water. These solvents may be used in a mixture at an appropriate ratio.
The reaction temperature is generally about -80 to about 150°C, preferably about -10 to about 100°C.
The reaction time is generally about 0.5 to about 20 hr.

### [Step 7]

In this step, compound (I-15) can be produced by converting U_{f} of compound (IIIc-43) to a 2,4-dioxo-1,3-thiazolidin-5-yl group.
This reaction is carried out in the presence of 1,3-thiazolidine-2,4-dione and a base, in a solvent that does not adversely influence the reaction.
The amount of the 1,3-thiazolidine-2,4-dione to be used is preferably about 1 to about 5 mol per 1 mol of compound (IIIc-43).
Examples of the base include alkali metal hydrides such as sodium hydride, potassium hydride and the like; and alkali metal amides such as lithium diisopropylamide and the like. The amount of the base to be used is preferably about 2 to about 10 mol per 1 mol of compound (IIIc-43).
Examples of the solvent that does not adversely influence the reaction include ethers such as diethyl ether, tetrahydrofuran, dioxane and the like; and aromatic hydrocarbons such as benzene, toluene, xylene and the like. These solvents may be used in a mixture at an appropriate ratio.
The reaction temperature is generally about -80 to about 150°C, preferably about -40 to about 30°C.
The reaction time is generally about 0.5 to about 20 hr.

Compound (IIIb-32) used as a starting material compound for the above-mentioned Method O can be produced, for example, using a starting material compound produced according to a method known per se, according to the above-mentioned Method C, Method E, Method J or Method K, the below-mentioned Method S or Method AF or a method analogous thereto.

Compound (IIIc-39''''), which is compound (IIIc-39) wherein T is -CH₂O-, -CH(CH₃)O- or -CH₂CH₂O- can be produced, for example, according to the following Method P.

### [Method P]

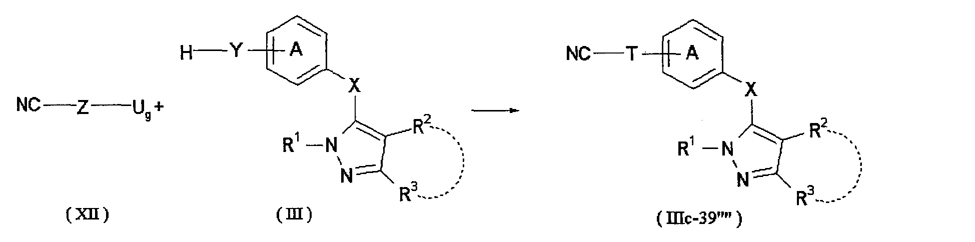

wherein Ug is a leaving group or a hydroxy group, and the other symbols are as defined above.
Examples of the "leaving group" for Ug include a chlorine atom, a bromine atom, an iodine atom, a benzenesulfonyloxy group, a p-toluenesulfonyloxy group, a methanesulfonyloxy group, a trifluoromethanesulfonyloxy group and the like.

In this method, compound (IIIc-39'''') can be produced by reacting compound (III) with compound (XII). This reaction is carried out according to the above-mentioned Method A.

Compound (XII) used as a starting material compound for the above-mentioned Method P can be produced according to a method known per se.

Compound (V-2), which is compound (V) wherein R² is CHO, can be produced, for example, according to the following Method Q.

### [Method Q]

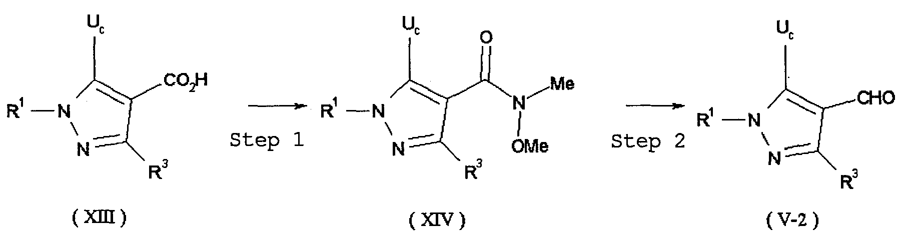

wherein the symbols in the formula are as defined above.

### [Step 1]

In this step, compound (XIV) can be produced by condensing compound (XIII) with methoxy(methyl)amine. This reaction is carried out according to the above-mentioned Method B, Step 2.

### [Step 2]

In this step, compound (V-2) can be produced by reducing compound (XIV). This reaction is carried out according to the above-mentioned Method G, Step 3.

Compound (XIII) used as a starting material compound for the above-mentioned Method Q can be produced according to a method known per se.

Compound (III-46), which is compound (III-2') wherein R³ is -CONR²⁰R²¹ wherein R²⁰ and R²¹ are as defined below, and compound (III-48), which is compound (III-2') wherein R³ is - CHO, can be produced, for example, according to the following Method R.

### [Method R]

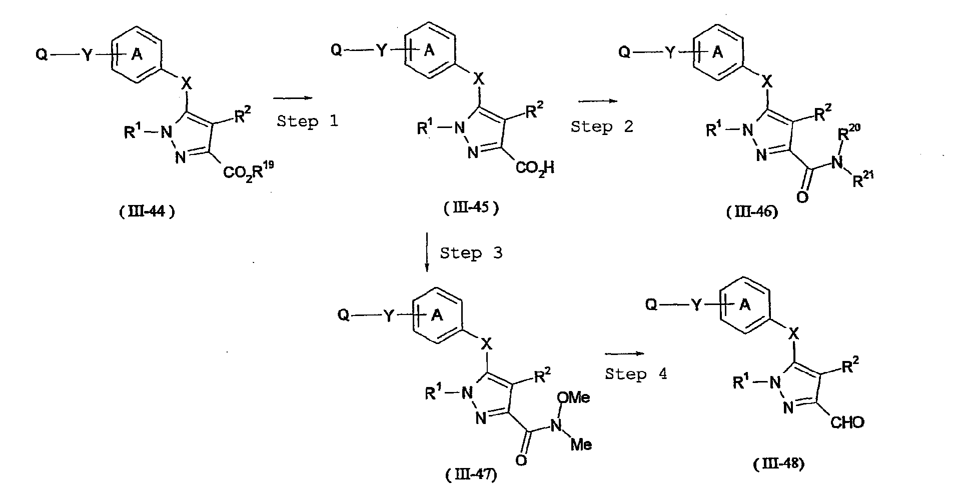

wherein
R¹⁹ is a C₁₋₆ alkyl group, a benzyl group optionally substituted by C₁₋₆ alkyl group (s) or a C₆₋₁₄ aryl group optionally substituted by C₁₋₆ alkyl group(s),
R²⁰ is a hydrogen atom, a C₁₋₁₀ alkyl group, a benzyl group optionally substituted by C₁₋₆ alkyl group (s) or a C₆₋₁₄ aryl group optionally substituted by C₁₋₆ alkyl group(s), R²¹ is a hydrogen atom, a C₁₋₁₀ alkyl group, a benzyl group optionally substituted by C₁₋₆ alkyl group(s), a C₆₋₁₄ aryl group optionally substituted by C₁₋₆ alkyl group(s), a C₁₋₁₀ alkylsulfonyl group, a benzylsulfonyl group optionally substituted by C₁₋₆ alkyl group(s) or a C₆₋₁₄ arylsulfonyl group optionally substituted by C₁₋₆ alkyl group(s), and the other symbols are as defined above.
R¹⁹ is preferably methyl, ethyl, tert-butyl, benzyl, phenyl or the like.
R²⁰ is preferably a hydrogen atom, methyl, ethyl, tert-butyl, benzyl, phenyl or the like.
R²¹ is preferably a hydrogen atom, methanesulfonyl, pentanesulfonyl, benzenesulfonyl, methyl, ethyl, benzyl, phenyl or the like.

### [Step 1]

In this step, compound (III-45) can be produced by subjecting compound (III-44) to hydrolysis. This reaction is carried out according to the above-mentioned Method B, Step 1.

### [Step 2]

In this step, compound (III-46) can be produced by subjecting compound (III-45) to an amidation reaction. This reaction is carried out according to the above-mentioned Method B, Step 2.

### [Step 3]

In this step, compound (III-47) can be produced by condensing compound (III-45) with methoxy(methyl)amine. This reaction is carried out according to the above-mentioned Method B, Step 2.

### [Step 4]

In this step, compound (III-48) can be produced by reducing compound (III-47). This reaction is carried out according to the above-mentioned Method G, Step 3.

Compound (III-44) used as a starting material compound for the above-mentioned Method R can be produced, for example, using a starting material compound produced according to a method known per se, according to the above-mentioned Method C', Method E', Method F, Method G, Method K', Method L, Method O or Method P, the below-mentioned Method S, Method AF, Method AG, Method AH or Method AT or a method analogous thereto.

Compound (III-52), which is compound (III-2') having - CONR²²R²³ wherein R²² and R²³ are as defined below on ring A, compound (III-53), which is compound (III-2') having -C(OH)R²⁴₂ wherein R²⁴ is as defined below on ring A, and compound (III-55), which is compound (III-2') having -C(O)R²⁵ wherein R²⁵ is as defined below on ring A, can be produced, for example, according to the following Method S.

### [Method S]

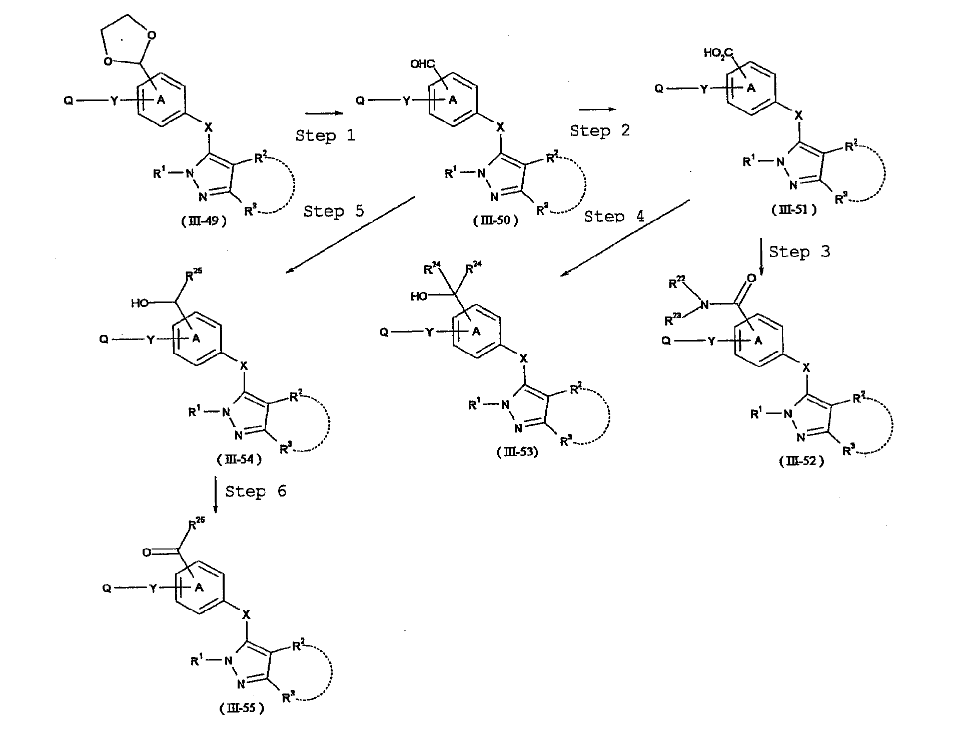

wherein
R²² is a hydrogen atom, a C₁₋₁₀ alkyl group, a benzyl group optionally substituted by C₁₋₆ alkyl group (s) or a C₆₋₁₄ aryl group optionally substituted by C₁₋₆ alkyl group(s),
R²³ is a hydrogen atom, a C₁₋₁₀ alkyl group, a benzyl group optionally substituted by C₁₋₆ alkyl group(s), a C₆₋₁₄ aryl group optionally substituted by C₁₋₆ alkyl group(s), a C₁₋₁₀ alkylsulfonyl group, a benzylsulfonyl group optionally substituted by C₁₋₆ alkyl group (s) or a C₆₋₁₄ arylsulfonyl group optionally substituted by C₁₋₆ alkyl group(s), R²⁴ is a C₁₋₆ alkyl group, a benzyl group optionally substituted by C₁₋₆ alkyl group (s) or a C₆₋₁₄ aryl group optionally substituted by C₁₋₆ alkyl group(s),
R²⁵ is a hydrogen atom, a C₁₋₁₀ alkyl group, a benzyl group optionally substituted by C₁₋₆ alkyl group (s) or a C₆₋₁₄ aryl group optionally substituted by C₁₋₆ alkyl group(s), and the other symbols are as defined above.
R²² is preferably a hydrogen atom, methyl, ethyl, benzyl, phenyl or the like.
R²³ is preferably a hydrogen atom, methanesulfonyl, pentanesulfonyl, benzenesulfonyl, methyl, ethyl, benzyl, phenyl or the like.
R²⁴ is preferably methyl, ethyl, benzyl, phenyl or the like.
R²⁵ is preferably a hydrogen atom, methyl, ethyl, tert-butyl, benzyl, phenyl or the like.

### [Step 1]

In this step, compound (III-50) can be produced by subjecting compound (III-49) to hydrolysis.
This reaction is carried out in the presence of an acid, in a solvent that does not adversely influence the reaction, according to a conventional method.
Examples of the acid include mineral acids such as hydrochloric acid, sulfuric acid and the like; organic acids such as trifluoroacetic acid, p-toluenesulfonic acid and the like; and hydrogen chloride-methanol, hydrogen chloride-ethyl acetate and the like, which are prepared by dissolving hydrogen chloride gas in a solvent such as methanol, ethyl acetate and the like. The amount of the acid to be used is generally 0.01 to 500 mol, preferably 0.01 to 5 mol, per 1 mol of compound (III-49).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; alcohols such as methanol, ethanol and the like; ketones such as acetone and the like; water and the like. These solvents may be used in a mixture at an appropriate ratio.
The reaction temperature is generally about -80 to about 150°C, preferably about -10 to about 100°C.
The reaction time is generally about 0.5 to about 20 hr.

### [Step 2]

In this step, compound (III-51) can be produced by subjecting compound (III-50) to an oxidization reaction.
This reaction is carried out in the presence of an oxidant, in a solvent that does not adversely influence the reaction, according to a conventional method.
Examples of the oxidant include potassium permanganate, potassium dichromate and the like. The amount of the oxidant to be used is generally 1 to 50 mol, preferably 1 to 10 mol, per 1 mol of compound (III-50).
Examples of the solvent that does not adversely influence the reaction include halogenated hydrocarbons such as chloroform, dichloromethane and the like; aromatic hydrocarbons such as benzene, toluene, xylene and the like; water and the like. These solvents may be used in a mixture at an appropriate ratio.
The reaction temperature is generally about -80 to about 150°C, preferably about -10 to about 100°C.
The reaction time is generally about 0.5 to about 20 hr.

### [Step 3]

In this step, compound (III-52) can be produced by subjecting compound (III-51) to an amidation reaction. This reaction is carried out according to the above-mentioned Method B, Step 2.

### [Step 4]

In this step, compound (III-53) can be produced by reacting compound (III-51) with an organic metal reagent. This reaction is carried out according to the above-mentioned Method E, Step 3.

### [Step 5]

In this step, compound (III-54) can be produced by reacting compound (III-50) with an organic metal reagent or a reducing agent. This reaction is carried out according to the above-mentioned Method E, Step 3.

### [Step 6]

In this step, compound (III-55) can be produced by subjecting compound (III-54) to an oxidization reaction.
This reaction is carried out in the presence of an oxidant, in a solvent that does not adversely influence the reaction, according to a conventional method.
Examples of the oxidant include manganese dioxide, potassium permanganate, potassium dichromate and the like. The amount of the oxidant to be used is generally 1 to 50 mol, preferably 1 to 10 mol, per 1 mol of compound (III-54).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; alcohols such as methanol, ethanol and the like; ketones such as acetone and the like; water and the like. These solvents may be used in a mixture at an appropriate ratio.
The reaction temperature is generally about -80 to about 150°C, preferably about -10 to about 100°C.
The reaction time is generally about 0.5 to about 20 hr.

Compound (III-49) used as a starting material compound for the above-mentioned Method S can be produced, for example, using a starting material compound produced according to a method known per se, according to the above-mentioned Method C', Method E', Method F, Method G, Method H', Method I',
Method K', Method L, Method M', Method N, Method P or Method R, the below-mentioned Method T, Method U, Method AF, Method AG, Method AH, Method AJ, Method AK, Method AT, Method AU or Method AV or a method analogous thereto.

Compound (III-57), which is compound (III-2') wherein X is -CH₂-, can be produced, for example, according to the following Method T.

### [Method T]

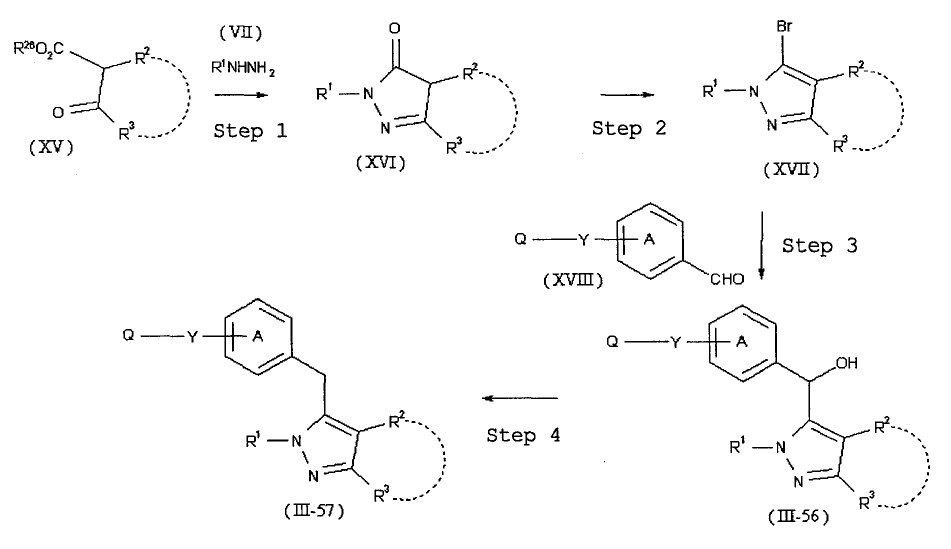

wherein R²⁶ is a C₁₋₁₀ alkyl group, a benzyl group optionally substituted by C₁₋₆ alkyl group (s) or a C₆₋₁₄ aryl group optionally substituted by C₁₋₆ alkyl group(s), and the other symbols are as defined above.
R²⁶ is preferably methyl, ethyl, benzyl, phenyl or the like.

### [Step 1]

In this step, compound (XVI) can be produced by reacting compound (XV) with compound (VII). This reaction is carried out according to the above-mentioned Method D, Step 1.

### [Step 2]

In this step, compound (XVII) can be produced by reacting compound (XVI) with phosphorus oxybromide.
This reaction is carried out without a solvent or in a solvent that does not adversely influence the reaction, according to a conventional method.
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like, and the like. These solvents may be used in a mixture at an appropriate ratio.
The reaction temperature is generally about -80 to about 150°C, preferably about 0 to about 100°C.
The reaction time is generally about 0.5 to about 20 hr.

### [Step 3]

In this step, compound (III-56) can be produced by reacting compound (XVII) with an organic lithium reagent, and then reacting the resulting compound with compound (XVIII).
This reaction is carried out in a solvent that does not adversely influence the reaction, according to a conventional method.
Examples of the organic lithium reagent include methyllithium, n-butyllithium, sec-butyllithium, tert-butyllithium, phenyllithium and the like. The amount of the organic lithium reagent to be used is generally 1 to 5 mol, preferably 1 to 2 mol, per 1 mol of compound (XVII).
The amount of compound (XVIII) to be used is preferably 1 to 3 mol per 1 mol of compound (XVII).
Examples of the solvent that does not adversely influence the reaction include ethers such as diethyl ether, tetrahydrofuran, dioxane and the like; aliphatic hydrocarbons such as hexane, heptane and the like; aromatic hydrocarbons such as benzene, toluene, xylene and the like, and the like. These solvents may be used in a mixture at an appropriate ratio.
The reaction temperature is generally about -100 to about 150°C, preferably about -80 to about 30°C.
The reaction time is generally about 0.5 to about 20 hr.

### [Step 4]

In this step, compound (III-57) can be produced by reducing the hydroxy group of compound (III-56). This reaction is carried out according to the above-mentioned Method E, Step 4.
In this reaction, when Q is V, the elimination reaction of Q may simultaneously proceed to convert Q to a hydrogen atom.

Compound (XV) used as a starting material compound for the above-mentioned Method T can be produced according to a method known per se. Compound (XVIII) can be produced according to a method known per se, or the method shown in the below-mentioned Reference Example 125, 126 or 127.

Compound (1-17), which is compound (I) wherein R² is R²⁸NHCOOCH₂CH=CH- wherein R²⁸ is as defined below, can be produced, for example, according to the following Method U.

### [Method U]

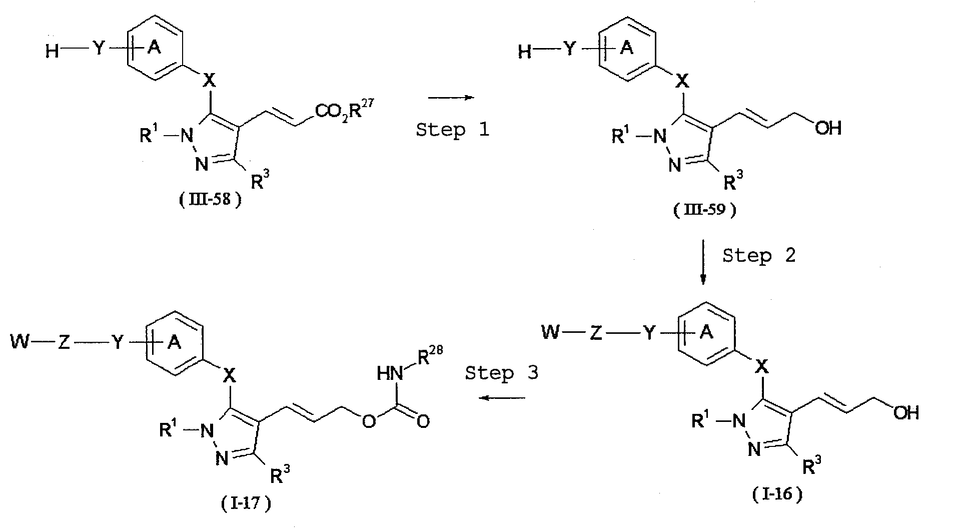

wherein
R²⁷ is a hydrogen atom, a C₁₋₁₀ alkyl group, a benzyl group optionally substituted by C₁₋₆ alkyl group(s) or a C₆₋₁₄ aryl group optionally substituted by C₁₋₆ alkyl group(s),
R²⁸ is a C₁₋₁₀ alkyl group, a benzyl group optionally substituted by C₁₋₆ alkyl group(s), a C₆₋₁₄ aryl group optionally substituted by C₁₋₆ alkyl group(s), a C₃₋₁₀ cycloalkyl group, a C₃₋₁₀ cycloalkyl-C₁₋₆ alkyl group, a C₁₋₁₀ alkoxy group, a benzyloxy group optionally substituted by C₁₋₆ alkyl group(s), a C₆₋₁₄ aryloxy group optionally substituted by C₁₋₆ alkyl group(s) or a C₁₋₁₀ alkyl group optionally substituted by aromatic heterocyclic group(s) optionally substituted by C₁₋₆ alkyl group(s), and
   the other symbols are as defined above.
R²⁷ is preferably a hydrogen atom, methyl, ethyl, tert-butyl, benzyl, phenyl or the like.
R²⁸ is preferably methyl, ethyl, n-butyl, tert-butyl, cyclopropyl, cyclopropylmethyl, benzyl, phenyl, methoxy, ethoxy, phenoxy or the like.

### [Step 1]

In this step, compound (III-59) can be produced by reducing compound (III-58). This reaction is carried out according to the above-mentioned Method G, Step 3.

### [Step 2]

In this step, compound (I-16) can be produced by introducing the substituent W-Z to compound (III-59). This reaction is carried out according to the above-mentioned Method A.

### [Step 3]

In this step, compound (I-17) can be produced by reacting compound (I-16) with R²⁸-NCO.
This reaction is carried out in a solvent that does not adversely influence the reaction, according to a conventional method.
Examples of R²⁸-NCO include methyl isocyanate, ethyl isocyanate, n-butyl isocyanate, tert-butyl isocyanate, cyclopropyl isocyanate, cyclopropylmethyl isocyanate, phenyl isocyanate, benzyl isocyanate and the like. The amount of R²⁸-NCO to be used is generally 1 to 10 mol, preferably 1 to 3 mol, per 1 mol of compound (I-16).
Examples of the solvent that does not adversely influence the reaction include pyridines such as pyridine, lutidine, collidine and the like; ethers such as diethyl ether, tetrahydrofuran, dioxane and the like; aliphatic hydrocarbons such as hexane, heptane and the like; aromatic hydrocarbons such as benzene, toluene, xylene and the like; amides such as N,N-dimethylformamide, N,N-dimethylacetamide, N-methylpyrrolidone and the like; halogenated hydrocarbons such as dichloromethane, chloroform, 1,2-dichloroethane, 1,1,2,2-tetrachloroethane and the like, and the like. These solvents may be used in a mixture at an appropriate ratio.
The reaction temperature is generally about -80 to about 150°C, preferably about 0 to about 100°C.
The reaction time is generally about 0.5 to about 20 hr.

Compound (III-58) used as a starting material compound for the above-mentioned Method U can be produced, for example, using a starting material compound produced according to a method known per se, according to the above-mentioned Method C', Method E', Method F, Method L, Method R, Method S, Method AG or Method AH or a method analogous thereto.

Compound (I-18), which is compound (I) wherein W-Z- is HO₂CCH₂CR²⁹R³⁰- wherein R²⁹ and R³⁰ are as defined below, can be produced, for example, according to the following Method V.

### [Method V]

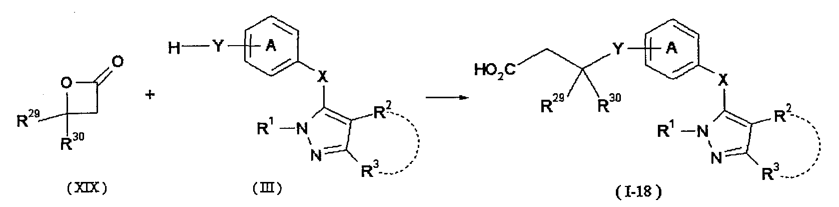

wherein R²⁹ and R³⁰ are each independently a hydrogen atom, a C₁₋₁₀ alkyl group, a benzyl group optionally substituted by C₁₋₆ alkyl group(s) or a C₆₋₁₄ aryl group optionally substituted by C₁₋₆ alkyl group(s), and the other symbols are as defined above.
R²⁹ and R³⁰ is preferably each independently a hydrogen atom, methyl, ethyl, benzyl, phenyl or the like.

In this method, compound (I-18) can be produced by reacting compound (III) with compound (XIX).
This reaction is carried out in the presence of a base, in a solvent that does not adversely influence the reaction.
Examples of the base include alkali metal hydrides such as sodium hydride, potassium hydride and the like; alkali metal hydroxides such as sodium hydroxide, potassium hydroxide, lithium hydroxide and the like; alkaline earth metal hydroxides such as magnesium hydroxide, calcium hydroxide, barium hydroxide and the like; alkali metal carbonate such as sodium carbonate, potassium carbonate, cesium carbonate and the like; alkali metal hydrogen carbonates such as sodium hydrogen carbonate and the like; alkali metal C₁₋₆ alkoxides such as sodium methoxide, sodium ethoxide, sodium tert-butoxide, potassium tert-butoxide and the like; organic lithiums such as methyllithium, n-butyllithium, sec-butyllithium, tert-butyllithium and the like; aromatic amines such as pyridine, lutidine and the like; tertiary amines such as trimethylamine, triethylamine, tripropylamine, tributylamine, N-ethyldiisopropylamine, cyclohexyldimethylamine, 4-dimethylaminopyridine, N,N-dimethylaniline, N-methylpiperidine, N-methylpyrrolidine, N-methylmorpholine and the like; 1,5-diazabicyclo[4.3.0]-5-nonene, 1,4-diazabicyclo[2.2.2]octane, 1,8-diazabicyclo[5.4.0]-7-undecene; metal amides such as sodium amide, lithium diisopropylamide, lithium hexamethyldisilazide and the like; and the like. The amount of the base to be used is preferably about 1 to about 5 mol per 1 mol of compound (III).
The amount of compound (XIX) to be used is preferably about 1 to about 5 mol per 1 mol of compound (III).
Examples of the solvent that does not adversely influence the reaction include ethers such as diethyl ether, tetrahydrofuran, dioxane and the like; aromatic hydrocarbons such as benzene, toluene, xylene and the like; amides such as N,N-dimethylformamide and the like; sulfoxides such as dimethylsulfoxide and the like, and the like. These solvents may be used in a mixture at an appropriate ratio.
The reaction temperature is generally about -80 to about 150°C, preferably about -10 to about 100°C.
The reaction time is generally about 0.5 to about 20 hr.
Compound (XIX) used as a starting material compound for the above-mentioned Method V can be produced according to a known method.

Compound (I-19), which is compound (I) wherein Y is a bond, and Z is

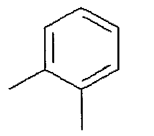

or

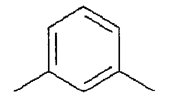

or

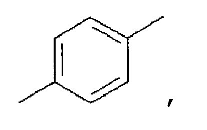

can be produced, for example, according to the following Method W.

### [Method W]

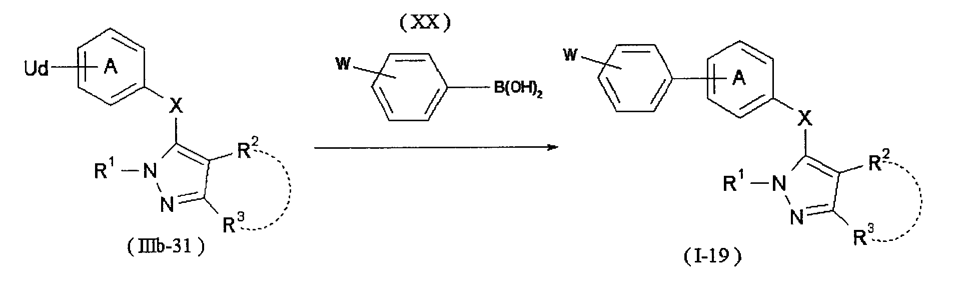

wherein the symbols in the formula are as defined above.
In this method, compound (I-19) can be produced by coupling compound (IIIb-31) with compound (XX) in the presence of a transition metal catalyst and a base.
This reaction is carried out in a solvent that does not adversely influence the reaction, under an inert gas atmosphere, according to a conventional method.
Examples of the inert gas include argon, nitrogen and the like.
Examples of the transition metal catalyst include tetrakis(triphenylphosphine)palladium(0), [1,1'-bis(diphenylphosphino)ferrocene]palladium(2) dichloride, a catalyst prepared by mixing tris(dibenzylideneacetone)dipalladium(0) with 4 equivalents of 2-(dicyclohexylphosphino)-2',6'-dimethoxybiphenyl, and the like. The amount of the transition metal catalyst to be used is generally 0.001 to 2 mol, preferably 0.01 to 0.1 mol, per 1 mol of compound (IIIb-31).
Examples of the base include alkali metal salts such as potassium hydroxide, sodium hydroxide, sodium hydrogen carbonate, potassium carbonate and the like; amines such as pyridine, triethylamine, N,N-diisopropylethylamine, N,N-dimethylaniline, 1,8-diazabicyclo[5.4.0]undec-7-ene and the like; metal hydrides such as potassium hydride, sodium hydride and the like; alkali metal C₁₋₆ alkoxides such as sodium methoxide, sodium ethoxide, potassium tert-butoxide and the like; carboxylates such as potassium acetate, sodium acetate, sodium formate and the like; alkali metal fluorides such as potassium fluoride, sodium fluoride and the like, and the like. The amount of the base to be used is generally 1 to 10 mol, preferably 1 to 3 mol, per 1 mol of compound (IIIb-31).
Examples of compound (XX) include 2-carboxyphenylboric acid, 3-carboxyphenylboric acid, 4-carboxyphenylboric acid and the like. The amount of compound (XX) to be used is generally 1 to 10 mol, preferably 1 to 3 mol, per 1 mol of compound (IIIb-31).
Examples of the solvent that does not adversely influence the reaction include ethers such as diethyl ether, tetrahydrofuran, dioxane and the like; aliphatic hydrocarbons such as hexane, heptane and the like; aromatic hydrocarbons such as benzene, toluene, xylene and the like; amides such as N,N-dimethylformamide, N,N-dimethylacetamide, N-methylpyrrolidone and the like; halogenated hydrocarbons such as dichloromethane, chloroform, 1,2-dichloroethane, 1,1,2,2-tetrachloroethane and the like; nitriles such as acetonitrile and the like; sulfoxides such as dimethylsulfoxide and the like; water and the like. These solvents may be used in a mixture at an appropriate ratio.
The reaction temperature is generally about -80 to about 150°C, preferably about 0 to about 100°C.
The reaction time is generally about 0.5 to about 20 hr.
Compound (XX) used as a starting material compound for the above-mentioned Method W can be produced according to a method known per se.

Compound (I-22), which is compound (I) having -CN on ring A, and compound (I-24),which is compound (I) having -CH(CN)R³¹ wherein R³¹ is as defined below on ring A, can be produced for example, according to the following Method X.

### [Method X]

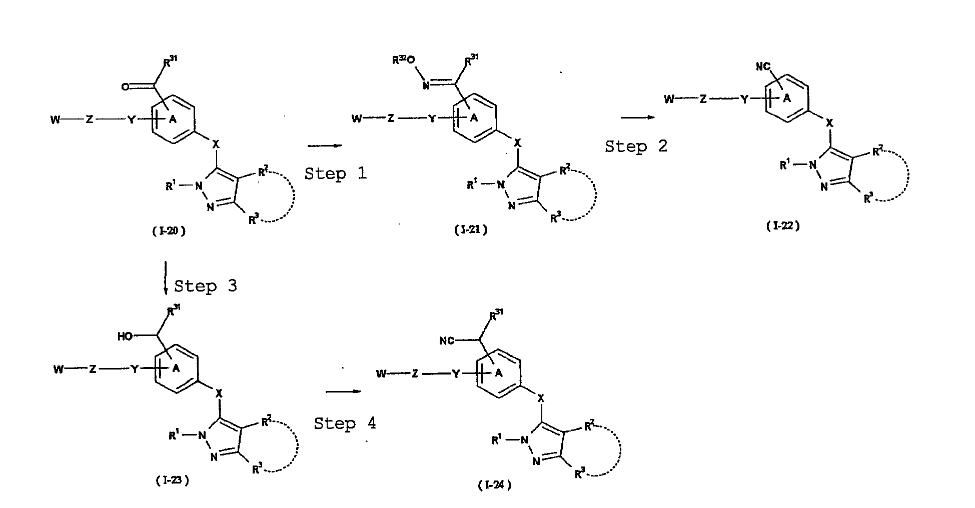

wherein R³¹ and R³² are each independently a hydrogen atom, a C₁₋₁₀ alkyl group, a benzyl group optionally substituted by C₁₋₆ alkyl group(s) or a C₆₋₁₄ aryl group optionally substituted by C₁₋₆ alkyl group(s), and the other symbols are as defined above.
_{R}³¹ and _{R}³² is preferably each independently a hydrogen atom, methyl, ethyl, benzyl, phenyl or the like.

### [Step 1]

In this step, compound (I-21) can be produced by reacting compound (I-20) with R³²ONH₂. This reaction is carried out according to the above-mentioned Method E, Step 5.

### [Step 2]

In this step, compound (I-22) can be produced by converting the hydroxyimino group of compound (I-21) wherein _{R}³¹ and _{R}³² are each a hydrogen atom to a nitrile group. This reaction is carried out according to the above-mentioned Method E, Step 6.

### [Step 3]

In this step, compound (I-23) can be produced by reducing compound (I-20). This reaction is carried out according to the above-mentioned Method G, Step 3.

### [Step 4]

In this step, compound (I-24) can be produced by cyanating the hydroxy group of compound (I-23). This reaction is carried out according to the above-mentioned Method G, Step 5.

Compound (I-20) used as a starting material compound for the above-mentioned Method X can be produced, for example, using a starting material compound produced according to a method known per se, according to the above-mentioned Method A, Method B, Method C'', Method E'', Method V or Method W, the below-mentioned Method Y, Method AB, Method AD, Method AG, Method AI, Method AM, Method AN, Method AO, Method AP, Method AR, Method AX or Method AY or a method analogous thereto.

Compound (I-27), which is compound (I) wherein Y is a bond, Z is -CH₂-, and W is 2,4-dioxo-1,3-thiazolidin-5-yl group, can be produced, for example, according to the following Method Y.

### [Method Y]

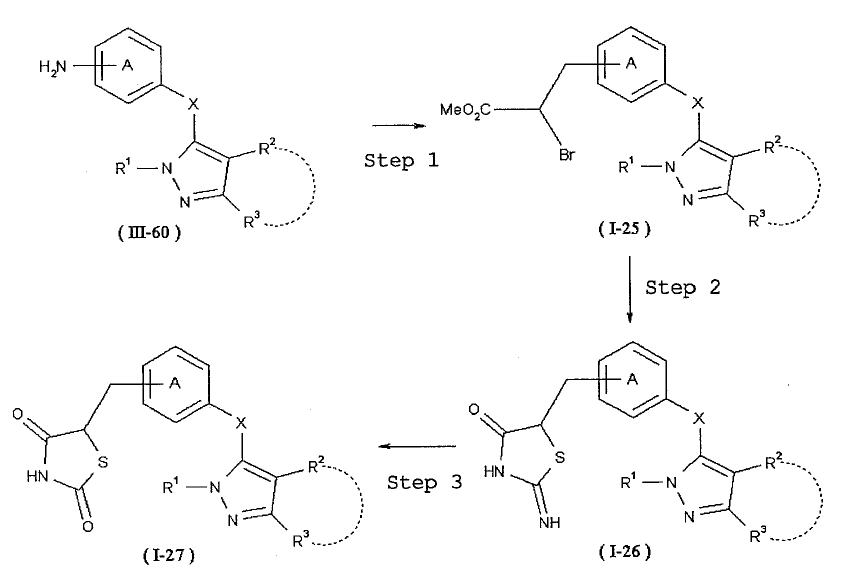

wherein the symbols in the formula are as defined above.

### [Step 1]

In this step, compound (I-25) can be produced by reacting compound (III-60) with methyl acrylate, in the presence of sodium nitrite and hydrogen bromide.
This reaction is carried out in a solvent that does not adversely influence the reaction.
The amount of the sodium nitrite to be used is preferably 1 to 5 mol per 1 mol of compound (III-60).
The amount of the hydrogen bromide to be used is preferably 1 to 10 mol per 1 mol of compound (III-60).
The amount of the methyl acrylate to be used is preferably 1 to 5 mol per 1 mol of compound (III-60).
Examples of the solvent that does not adversely influence the reaction include ethers such as diethyl ether, tetrahydrofuran, dioxane and the like; aliphatic hydrocarbons such as hexane, heptane and the like; aromatic hydrocarbons such as benzene, toluene, xylene and the like; amides such as N,N-dimethylformamide, N,N-dimethylacetamide, N-methylpyrrolidone and the like; halogenated hydrocarbons such as dichloromethane, chloroform, 1,2-dichloroethane, 1,1,2,2-tetrachloroethane and the like; alcohols such as methanol, ethanol and the like; nitriles such as acetonitrile and the like; acetones such as acetone, methyl ethyl ketone and the like; water and the like. These solvents may be used in a mixture at an appropriate ratio.
The reaction temperature is generally about -80 to about 150°C, preferably about 0 to about 100°C.
The reaction time is generally about 0.5 to about 20 hr.

### [Step 2]

In this step, compound (I-26) can be produced by reacting compound (I-25) with thiourea in the presence of sodium acetate,.
This reaction is carried out in a solvent that does not adversely influence the reaction.
The amount of the sodium acetate to be used is preferably 1 to 5 mol per 1 mol of compound (I-25).
The amount of the thiourea to be used is preferably 1 to 5 mol per 1 mol of compound (I-25).
Examples of the solvent that does not adversely influence the reaction include ethers such as diethyl ether, tetrahydrofuran, dioxane and the like; aliphatic hydrocarbons such as hexane, heptane and the like; aromatic hydrocarbons such as benzene, toluene, xylene and the like; amides such as N,N-dimethylformamide, N,N-dimethylacetamide, N-methylpyrrolidone and the like; halogenated hydrocarbons such as dichloromethane, chloroform, 1,2-dichloroethane, 1,1,2,2-tetrachloroethane and the like; alcohols such as methanol, ethanol and the like: nitriles such as acetonitrile and the like; water and the like. These solvents may be used in a mixture at an appropriate ratio.
The reaction temperature is generally about -80 to about 150°C, preferably about 0 to about 100°C.
The reaction time is generally about 0.5 to about 20 hr.

### [Step 3]

In this step, compound (I-27) can be produced by treating compound (I-26) with an acid.
This reaction is carried out in a solvent that does not adversely influence the reaction.
Examples of the acid include mineral acids such as hydrochloric acid, sulfuric acid and the like; organic acids such as trifluoroacetic acid, p-toluenesulfonic acid and the like; and hydrogen chloride-methanol, hydrogen chloride-ethyl acetate and the like, which are prepared by dissolving hydrogen chloride gas in a solvent such as methanol, ethyl acetate and the like. The amount of the acid to be used is generally 0.01 to 500 mol, preferably 0.01 to 5 mol, per 1 mol of compound (I-26).
Examples of the solvent that does not adversely influence the reaction include ethers such as diethyl ether, tetrahydrofuran, dioxane and the like; aliphatic hydrocarbons such as hexane, heptane and the like; aromatic hydrocarbons such as benzene, toluene, xylene and the like; amides such as N,N-dimethylformamide, N,N-dimethylacetamide, N-methylpyrrolidone and the like; halogenated hydrocarbons such as dichloromethane, chloroform, 1,2-dichloroethane, 1,1,2,2-tetrachloroethane and the like; alcohols such as methanol, ethanol and the like: nitriles such as acetonitrile and the like; water and the like. These solvents may be used in a mixture at an appropriate ratio.
The reaction temperature is generally about -80 to about 150°C, preferably about 0 to about 100°C.
The reaction time is generally about 0.5 to about 20 hr.

Compound (III-60) used as a starting material compound for the above-mentioned Method Y can be produced, for example, using a starting material compound produced according to a method known per se, according to the above-mentioned Method C', Method E', Method H', Method I', Method K', Method M', Method R, Method S or Method T or a method analogous thereto.

Compound (I-29), which is compound (I) wherein R³ is a hydroxyimino group, and compound (I-30), which is compound (I) wherein R³ is a nitrile group, can be produced, for example, according to the following Method Z.

### [Method Z]

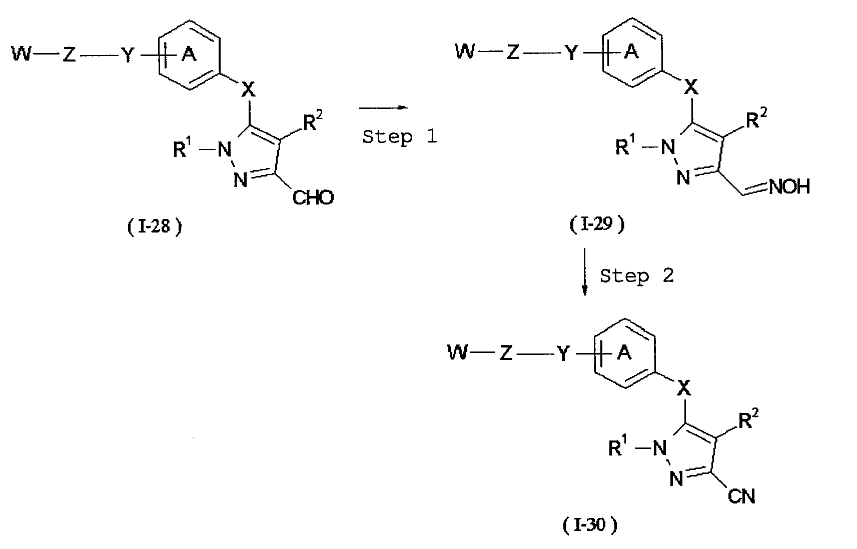

wherein the symbols in the formula are as defined above.

### [Step 1]

In this step, compound (I-29) can be produced by reacting compound (I-28) with hydroxylamine. This reaction is carried out according to the above-mentioned Method E, Step 5.

### [Step 2]

In this step, compound (I-30) can be produced by converting the hydroxyimino group of compound (I-29) to a nitrile. This reaction is carried out according to the above-mentioned Method E, Step 6.

Compound (I-28) used as a starting material compound for the above-mentioned Method Z can be produced, for example, using a starting material compound produced according to a method known per se, according to the above-mentioned Method A, Method B, Method C'', Method E'', Method P, Method V, Method W or Method Y, the below-mentioned Method AD, Method AI, Method AO, Method AP or Method AX or a method analogous thereto.

Compound (I-32), which is compound (I) wherein R² is R³³N=CH- wherein R³³ is as defined below, can be produced, for example, according to the following Method AA.

### [Method AA]

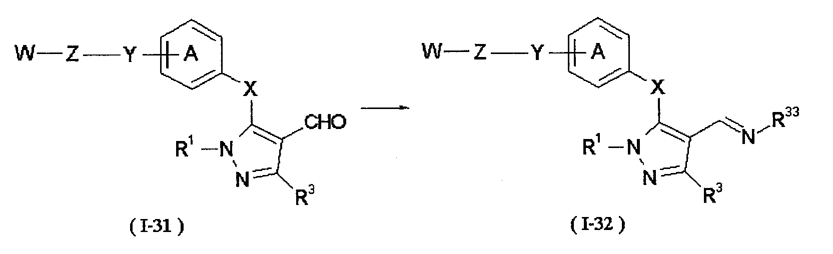

wherein R³³ is an unsubstituted amino group, an amino group optionally mono- or di-substituted by C₁₋₆ alkyl group(s), or a cyanomethyl group, and the other symbols are as defined above.
R³³ is preferably unsubstituted amino, cyanomethyl or the like.

In this method, compound (I-32) can be produced by reacting compound (I-31) with a hydrazine or aminoacetonitrile.
This reaction is carried out in a solvent that does not adversely influence the reaction. The reaction may be carried out in the presence of an acid or a base.
Examples of the hydrazine include hydrazine, methylhydrazine and the like.
The amount of the hydrazine and aminoacetonitrile to be used is generally 1 to 10 mol, preferably 1 to 5 mol, per 1 mol of compound (I-31), respectively.
Examples of the acid and base include those exemplified in the above-mentioned Method E, Step 1. The amount of the acid or base to be used is generally 0 to 50 mol, preferably 0 to 10 mol, per 1 mol of compound (I-31), respectively.
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; alcohols such as methanol, ethanol and the like; ketones such as acetone and the like; aliphatic hydrocarbons such as hexane, heptane and the like; water and the like. These solvents may be used in a mixture at an appropriate ratio.
The reaction temperature is generally about -100 to about 150°C, preferably about -10 to about 100°C.
The reaction time is generally about 0.5 to about 20 hr.

Compound (I-31) used as a starting material compound for the above-mentioned Method AA can be produced, for example, using a starting material compound produced according to a method known per se, according to the above-mentioned Method A, Method B, Method C'', Method E'', Method P, Method V, Method W or Method Y, the below-mentioned Method AD, Method AG or Method AI or a method analogous thereto.

Compound (I-34), which is compound (I) wherein R² is R³⁴ON=CH- wherein R³⁴ is as defined below, can be produced, for example, according to the following Method AB.

### [Method AB]

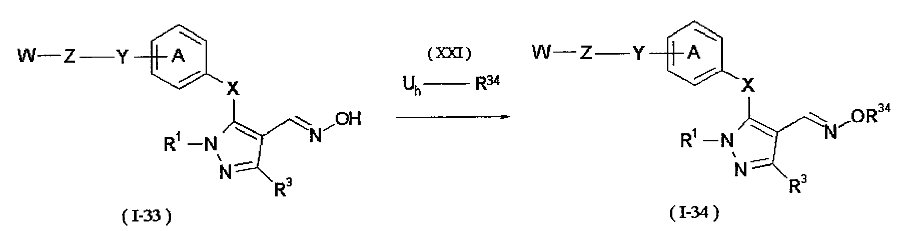

wherein Uh is a leaving group, R³⁴ is a C₁₋₁₀ alkyl group, a benzyl group optionally substituted by C₁₋₆ alkyl group(s), a C₆₋₁₄ aryl group optionally substituted by C₁₋₆ alkyl group(s), a cyanomethyl group or a 2-oxopropyl group, and the other symbols are as defined above.
Examples of the "leaving group" for Uh include a chlorine atom, a bromine atom, an iodine atom, benzenesulfonyloxy group, p-toluenesulfonyloxy group, methanesulfonyloxy group, trifluoromethanesulfonyloxy group and the like.
R³⁴ is preferably cyanomethyl, 2-oxopropyl or the like.

In this method, compound (I-34) can be produced by reacting compound (I-33) with compound (XXI) in the presence of a base.
This reaction is carried out in a solvent that does not adversely influence the reaction.
The amount of compound (XXI) to be used is generally 1 to 10 mol, preferably 1 to 3 mol, per 1 mol of compound (I-33).
Examples of the base include those exemplified in the above-mentioned Method E, Step 1. The amount of the base to be used is generally 1 to 10 mol, preferably 1 to 3 mol, per 1 mol of compound (I-33).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; alcohols such as methanol, ethanol and the like; ketones such as acetone and the like; aliphatic hydrocarbons such as hexane, heptane and the like; water and the like. These solvents may be used in a mixture at an appropriate ratio.
The reaction temperature is generally about -100 to about 150°C, preferably about -10 to about 100°C.
The reaction time is generally about 0.5 to about 20 hr.

Compound (I-33) used as a starting material compound for the above-mentioned Method AB can be produced, for example, using a starting material compound produced according to a method known per se, according to the above-mentioned Method A, Method B, Method C", Method E'', Method W or Method Y, the below-mentioned Method AD or Method AI or a method analogous thereto.

Compound (I-36), wherein compound (I) wherein R² is

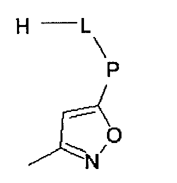

wherein L and P are as defined below, can be produced, for example, according to the following Method AC.

### [Method AC]

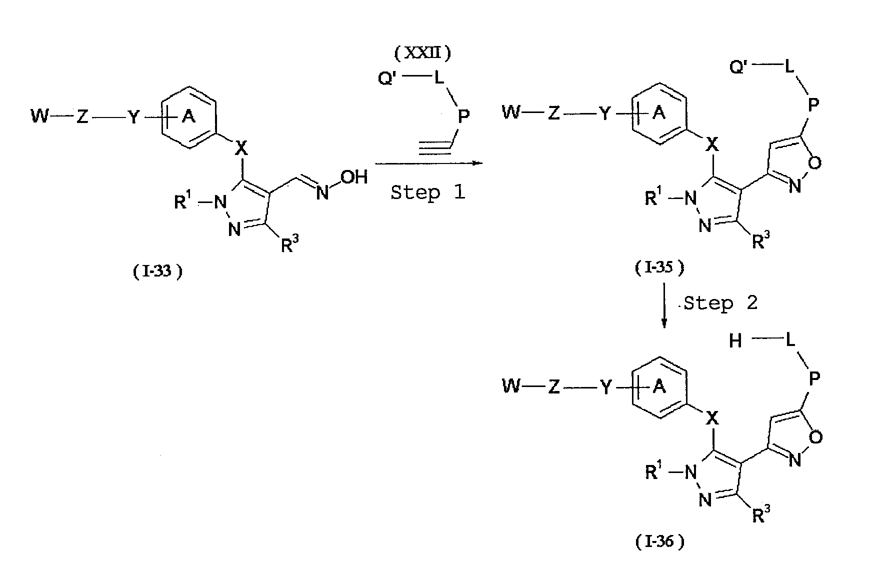

wherein Q' is a hydrogen atom or the above-mentioned V, L is a bond, an oxygen atom or an amino group optionally substituted by C₁₋₆ alkyl group(s), P is a bond, or a divalent hydrocarbon group having 1 to 6 atoms in the main chain, which is optionally substituted, and the other symbols are as defined above.
Q' is preferably a hydrogen atom, trimethylsilyl or the like.
L is preferably a bond, an oxygen atom or the like.
P is preferably a bond, -CH₂-, -(CH₂)₂-, -CH(CH₃)-, - C(CH₃)₂- or the like.

### [Step 1]

In this step, compound (I-35) can be produced by reacting compound (I-33) with compound (XXII) in the presence of sodium hypochlorite, according to a method known per se, for example, the method described in EP399645B1, or a method analogous thereto.
This reaction is carried out in a solvent that does not adversely influence the reaction.
The amount of the sodium hypochlorite to be used is generally 1 to 10 mol, preferably 1 to 3 mol, per 1 mol of compound (I-33).
The amount of compound (XXII) to be used is generally 1 to 10 mol, preferably 1 to 3 mol, per 1 mol of compound (I-33).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; alcohols such as methanol, ethanol and the like; ketones such as acetone and the like; aliphatic hydrocarbons such as hexane, heptane and the like; water and the like. These solvents may be used in a mixture at an appropriate ratio.
The reaction temperature is generally about -100 to about 150°C, preferably about -10 to about 100°C.
The reaction time is generally about 0.5 to about 20 hr.
In this reaction, when Q' is V, the elimination reaction of Q may simultaneously proceed to convert Q' to a hydrogen atom.

### [Step 2]

In this step, compound (I-36) can be produced by converting Q' of compound (I-35) wherein Q' is V to a hydrogen atom. This reaction is carried out according to the above-mentioned Method F.

Compound (XXII) used as a starting material compound for the above-mentioned Method AC can be produced according to a method known per se.

Compound (I-37) which is compound (I) wherein Y and Z are each a bond, and W is 2-oxoimidazolidin-1-yl group, can be produced, for example, according to the following Method AD.

### [Method AD]

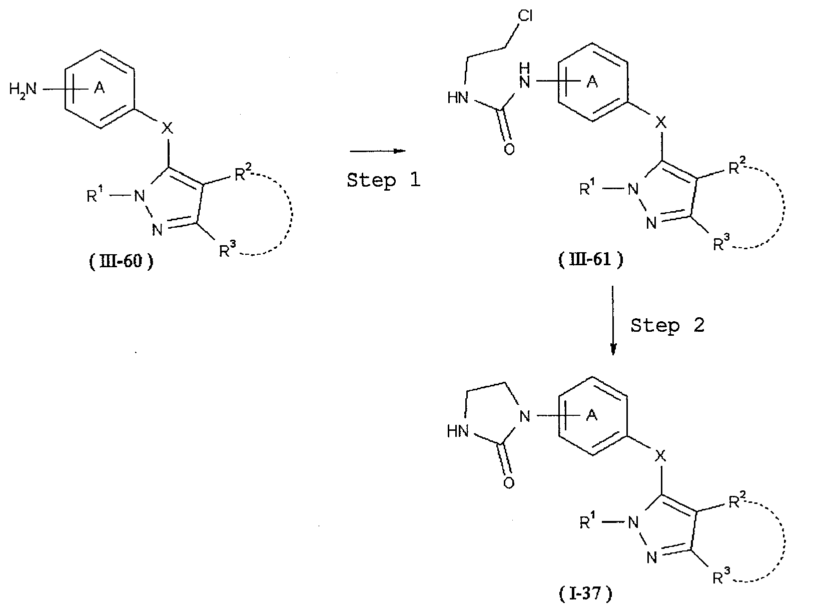

wherein the symbols in the formula are as defined above.

### [Step 1]

In this step, compound (III-61) can be produced by reacting compound (III-60) with 2-chloroethyl isocyanate.
This reaction is carried out in a pyridine solvent.
The amount of the 2-chloroethyl isocyanate to be used is generally 1 to 10 mol, preferably 1 to 3 mol, per 1 mol of compound (III-60).
The reaction temperature is generally about -100 to about 150°C, preferably about -10 to about 100°C.
The reaction time is generally about 0.5 to about 20 hr.

### [Step 2]

In this step, compound (I-37) can be produced by treating compound (III-61) with a base.
This reaction is carried out in a solvent that does not adversely influence the reaction.
Examples of the base include those exemplified in the above-mentioned Method E, Step 1. The amount of the base to be used is generally 1 to 10 mol, preferably 1 to 3 mol, per 1 mol of compound (III-61).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; alcohols such as methanol, ethanol and the like; ketones such as acetone and the like; aliphatic hydrocarbons such as hexane, heptane and the like; water and the like. These solvents may be used in a mixture at an appropriate ratio.
The reaction temperature is generally about -100 to about 150°C, preferably about -10 to about 100°C.
The reaction time is generally about 0.5 to about 20 hr.

Compound (I-39), which is compound (I) wherein R² is a difluoromethyl group, can be produced, for example, according to the following Method AE.

### [Method AE]

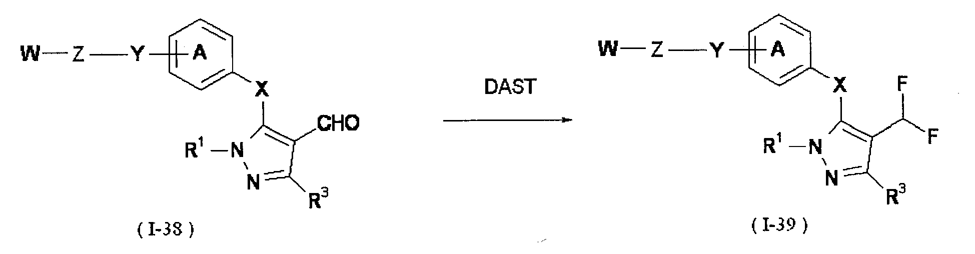

wherein the symbols in the formula are as defined above.

In this method, compound (I-39) can be produced by reacting compound (I-38) with diethylaminosulfur difluoride (DAST) .
This reaction is carried out in a solvent that does not adversely influence the reaction.
The amount of the DAST to be used is generally 1 to 50 mol, preferably 1 to 10 mol, per 1 mol of compound (I-38).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; aliphatic hydrocarbons such as hexane, heptane and the like, and the like. These solvents may be used in a mixture at an appropriate ratio.
The reaction temperature is generally about -100 to about 150°C, preferably about 0 to about 100°C.
The reaction time is generally about 0.5 to about 100 hr.
Compound (I-38) used as a starting material compound for the above-mentioned Method AE can be produced, for example, using a starting material compound produced according to a method known per se, according to the above-mentioned Method A, Method B, Method J, Method C'', Method V, Method W, Method Y or Method AD, the below-mentioned Method AG or Method AI or a method analogous thereto.

Compound (I-42), which is compound (I) wherein Y is O, having a 3-phenoxypropyl group on ring A, for example, can be produced according to the following Method AF.

### [Method AF]

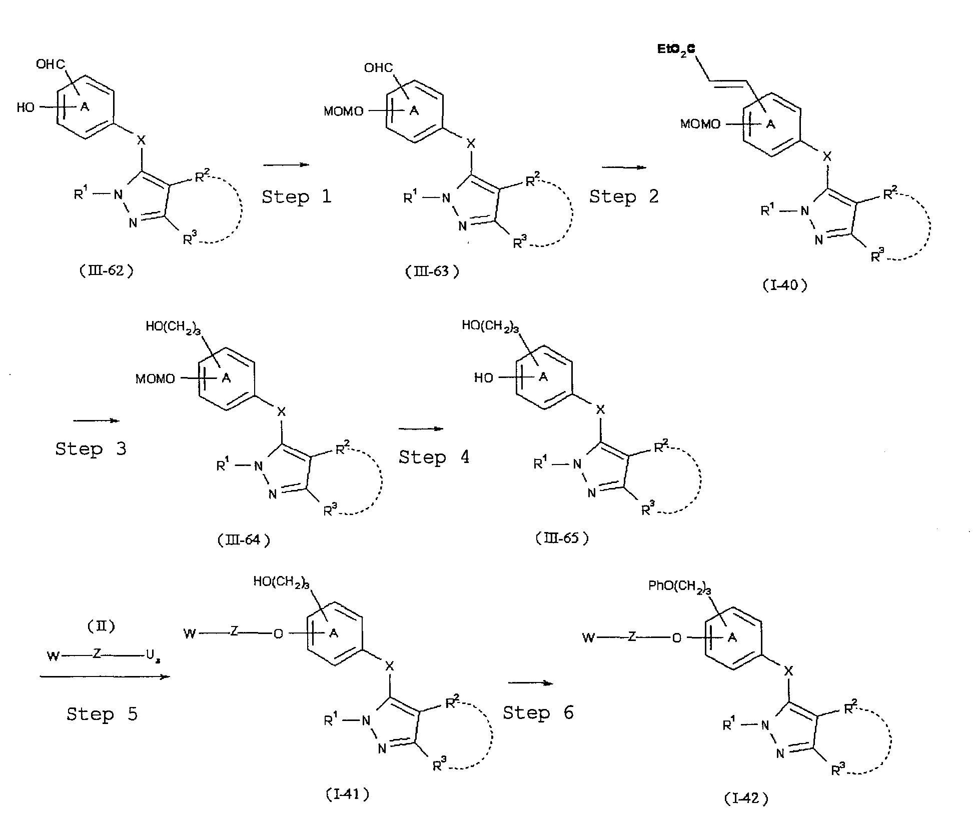

wherein the symbols in the formula are as defined above.

### [Step 1]

In this step, compound (III-63) can be produced by introducing a methoxymethyl (MOM) group to the hydroxy group of compound (III-62).
This reaction is carried out in the presence of chloromethyl methyl ether and a base, in a solvent that does not adversely influence the reaction.
The amount of the chloromethyl methyl ether to be used is preferably about 1 to about 5 mol per 1 mol of compound (III-62).
Examples of the base include amines such as triethylamine, N,N-diisopropylethylamine, N-methylmorpholine, N,N-dimethylaniline, 4-dimethylaminopyridine and the like; alkali metal salts such as sodium hydrogen carbonate, sodium carbonate, potassium carbonate and the like; alkali metal hydroxides such as sodium hydroxide, potassium hydroxide, lithium hydroxide and the like; alkaline earth metal hydroxides such as magnesium hydroxide, calcium hydroxide, barium hydroxide and the like; metal hydrides such as potassium hydride, sodium hydride and the like; alkali metal C₁₋₆ alkoxides such as sodium methoxide, sodium ethoxide, potassium tert-butoxide and the like, and the like. The amount of the base to be used is preferably about 1 to about 5 mol per 1 mol of compound (III-62).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; ketones such as acetone and the like, and the like. These solvents may be used in a mixture at an appropriate ratio.
The reaction temperature is generally about -80 to about 150°C, preferably about -10 to about 100°C.
The reaction time is generally about 0.5 to about 20 hr.

### [Step 2]

In this step, compound (I-40) can be produced by converting the formyl group of compound (III-63) to a 2-(ethoxycarbonyl)vinyl group.
This reaction is carried out in the presence of ethyl diethylphosphonoacetate and a base, in a solvent that does not adversely influence the reaction.
The amount of the ethyl diethylphosphonoacetate to be used is generally 1 to 10 mol, preferably 1 to 3 mol, per 1 mol of compound (III-63).
Examples of the base include alkali metal salts such as potassium hydroxide, sodium hydroxide, sodium hydrogen carbonate, potassium carbonate and the like; amines such as pyridine, triethylamine, N,N-diisopropylethylamine, N,N-dimethylaniline, 1,8-diazabicyclo[5.4.0]undec-7-ene and the like; metal hydrides such as potassium hydride, sodium hydride and the like; alkali metal C₁₋₆ alkoxides such as sodium methoxide, sodium ethoxide, potassium tert-butoxide and the like, and the like. The amount of the base to be used is generally 1 to 10 mol, preferably 1 to 3 mol, per 1 mol of compound (III-63).
Examples of the solvent that does not adversely influence the reaction include aromatic hydrocarbons such as benzene, toluene, xylene and the like; aliphatic hydrocarbons such as hexane, heptane and the like; ethers such as diethyl ether, diisopropyl ether, tert-butyl methyl ether, tetrahydrofuran, dioxane, dimethoxyethane and the like; halogenated hydrocarbons such as chloroform, dichloromethane and the like, and the like. These solvents may be used in a mixture at an appropriate ratio.
The reaction temperature is generally about -100 to about 150°C, preferably about -10 to about 100°C.
The reaction time is generally about 0.5 to about 20 hr.

### [Step 3]

In this step, compound (III-64) can be produced by converting the 2-(ethoxycarbonyl)vinyl group of compound (I-40) to a 3-hydroxypropyl group.
This reaction is carried out in the presence of a reducing agent, in a solvent that does not adversely influence the reaction.
Examples of the reducing agent include metal hydrogen compounds such as sodium bis(2-methoxyethoxy)aluminum hydride, diisobutylaluminum hydride and the like; metal hydrogen complex compounds such as sodium borohydride, sodium cyanoborohydride, lithium aluminum hydride, sodium aluminum hydride and the like, and the like. The amount of the reducing agent to be used is generally 1 to 20 mol per 1 mol of compound (I-40).
Examples of the solvent that does not adversely influence the reaction include alcohols such as methanol, ethanol, propanol, 2-propanol, butanol, isobutanol, tert-butanol and the like; aromatic hydrocarbons such as benzene, toluene, xylene and the like; aliphatic hydrocarbons such as hexane, heptane and the like; ethers such as diethyl ether, diisopropyl ether, tert-butyl methyl ether, tetrahydrofuran, dioxane, dimethoxyethane and the like; halogenated hydrocarbons such as dichloromethane, chloroform, 1,2-dichloroethane, 1,1,2,2-tetrachloroethane and the like, and the like. These solvents may be used in a mixture at an appropriate ratio.
The reaction temperature is generally -70 to 150°C, preferably -20 to 100°C.
The reaction time is generally 0.1 to 100 hr, preferably 0.1 to 40 hr.

### [Step 4]

In this step, compound (III-65) can be produced by removing the methoxymethyl group of compound (III-64). This reaction is carried out according to the above-mentioned Method F.

### [Step 5]

In this step, compound (I-41) can be produced by reacting compound (III-65) with compound (II). This reaction is carried out according to the above-mentioned Method A.

### [Step 6]

In this step, compound (I-42) can be produced by condensing the hydroxy group of compound (I-41) with phenol.
This reaction is carried out according to a method known per se, for example, the method described in Synthesis page 1 (1981), or a method analogous thereto. That is, this reaction is generally carried out in the presence of an organic phosphorus compound and an electrophilic agent, in a solvent that does not adversely influence the reaction.
Examples of the organic phosphorus compound include triphenylphosphine, tributylphosphine and the like.
Examples of the electrophilic agent include diethyl azodicarboxylate, diisopropyl azodicarboxylate, 1,1'-azodicarbonyldipiperidine and the like.
The amount of the organic phosphorus compound and electrophilic agent to be used is preferably about 1 to about 5 mol per 1 mol of compound (I-41), respectively.
The amount of the phenol to be used is preferably about 1 to about 5 mol per 1 mol of compound (I-41).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; ketones such as acetone and the like, and the like. These solvents may be used in a mixture at an appropriate ratio.
The reaction temperature is generally about -80 to about 150°C, preferably about -10 to about 100°C.
The reaction time is generally about 0.5 to about 20 hr.

Compound (III-62) used as a starting material compound for the above-mentioned Method AF can be produced, for example, using a starting material compound produced according to a method known per se, according to the above-mentioned Method F, Method C', Method I', Method K' or Method S, the below-mentioned Method AJ or a method analogous thereto.

Compound (I-43), which is compound (I) wherein Y is O, having a 3-(methanesulfonyl)propyl group on ring A, can be produced, for example, according to the following Method AG.

### [Method AG]

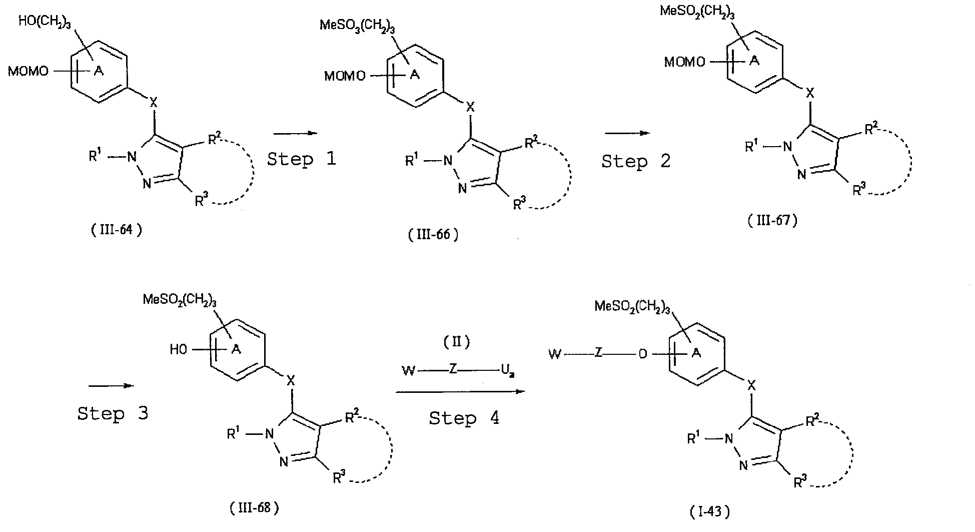

wherein the symbols in the formula are as defined above.

### [Step 1]

In this step, compound (III-66) can be produced by converting the 3-hydroxypropyl group on ring A of compound (III-64) to a 3-(methanesulfonyloxy)propyl group.
This reaction is carried out in the presence of methanesulfonyl chloride and a base, in a solvent that does not adversely influence the reaction.
The amount of the methanesulfonyl chloride to be used is preferably about 1 to about 5 mol per 1 mol of compound (III-64).
Examples of the base include amines such as triethylamine, N,N-diisopropylethylamine, N-methylmorpholine, N,N-dimethylaniline, 4-dimethylaminopyridine and the like; alkali metal salts such as sodium hydrogen carbonate, sodium carbonate, potassium carbonate and the like; alkali metal hydroxides such as sodium hydroxide, potassium hydroxide, lithium hydroxide and the like; alkaline earth metal hydroxides such as magnesium hydroxide, calcium hydroxide, barium hydroxide and the like; metal hydrides such as potassium hydride, sodium hydride and the like; alkali metal C₁₋₆ alkoxides such as sodium methoxide, sodium ethoxide, potassium tert-butoxide and the like, and the like. The amount of the base to be used is preferably about 1 to about 5 mol per 1 mol of compound (III-64).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; ketones such as acetone and the like: esters such as ethyl acetate and the like, and the like. These solvents may be used in a mixture at an appropriate ratio.
The reaction temperature is generally about -80 to about 150°C, preferably about -10 to about 100°C.
The reaction time is generally about 0.5 to about 20 hr.

### [Step 2]

In this step, compound (III-67) can be produced by converting the 3-(methanesulfonyloxy)propyl group on ring A of compound (III-66) to a 3-(methanesulfonyl)propyl group.
This reaction is carried out in the presence of sodium methanesulfinate, in a solvent that does not adversely influence the reaction.
The amount of the sodium methanesulfinate to be used is preferably about 1 to about 10 mol per 1 mol of compound (III-66).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; ketones such as acetone and the like; esters such as ethyl acetate and the like, and the like. These solvents may be used in a mixture at an appropriate ratio.
The reaction temperature is generally about -80 to about 180°C, preferably about -10 to about 120°C.
The reaction time is generally about 0.5 to about 20 hr.

### [Step 3]

In this step, compound (III-68) can be produced by removing the methoxymethyl group of compound (III-67). This reaction is carried out according to the above-mentioned Method F.

### [Step 4]

In this step, compound (I-43) can be produced by reacting compound (III-68) with compound (II). This reaction is carried out according to the above-mentioned Method A.

Compound (III-71) can be produced, for example, according to the following Method AH.

### [Method AH]

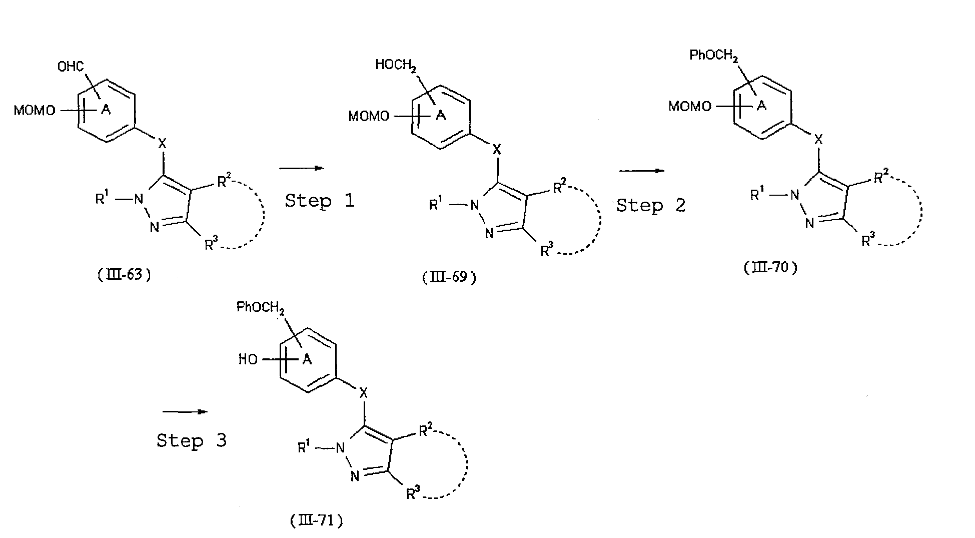

wherein the symbols in the formula are as defined above.

### [Step 1]

In this step, compound (III-69) can be produced by converting the formyl group on ring A of compound (III-63) to a hydroxymethyl group. This reaction is carried out according to the above-mentioned Method G, Step 3.

### [Step 2]

In this step, compound (III-70) can be produced by converting the hydroxymethyl group on ring A of compound (III-69) to a phenoxymethyl group. This reaction is carried out according to the above-mentioned Method AF, Step 6.

### [Step 3]

In this step, compound (III-71) can be produced by removing the methoxymethyl group on ring A of compound (III-70). This reaction is carried out according to the above-mentioned Method F.

Compound (I-45), which is compound (I) having R³⁵ on ring A, and compound (I-47), which is compound (I) having R³⁶ on ring A, wherein R³⁵ and R³⁶ are as defined below, can be produced, for example, according to the following Method AI.

### [Method AI]

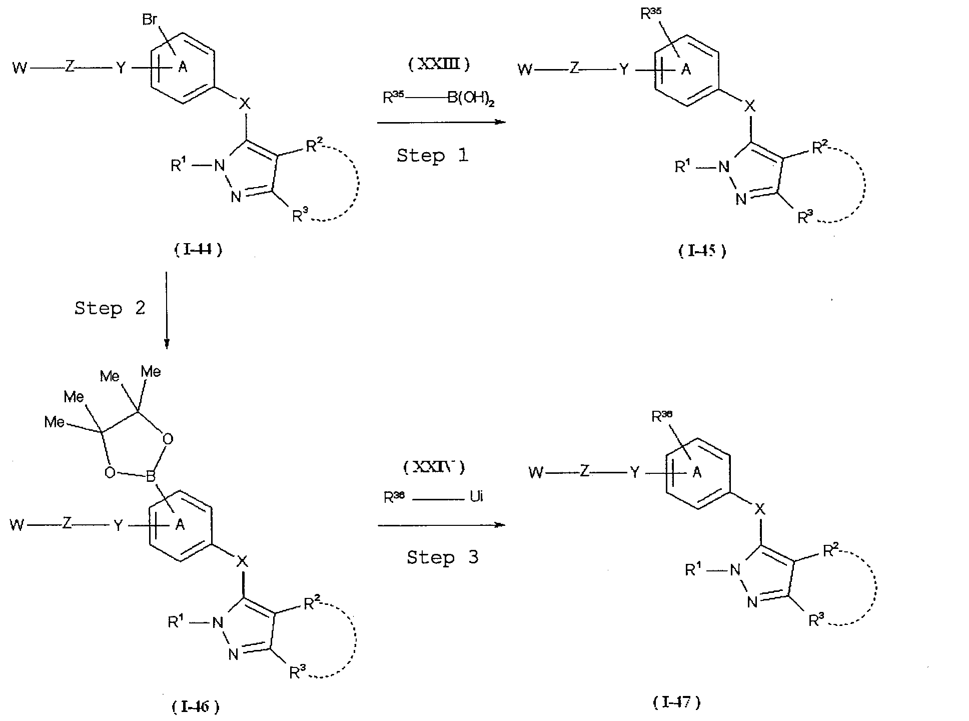

wherein R³⁵ is a C₁₋₁₀ alkyl group, a C₃₋₁₀ cycloalkyl group or an optionally substituted C₆₋₁₄ aryl group, R³⁶ is an optionally substituted C₆₋₁₄ aromatic heterocyclic group, Uᵢ is a leaving group, and the other symbols are as defined above.
Examples of the "leaving group" for Uᵢ include a chlorine atom, a bromine atom, an iodine atom, a trifluoromethanesulfonyloxy group and the like.
R³⁵ is preferably methyl, cyclopropyl, phenyl, 4-fluorophenyl, 4-cyanophenyl or the like.
R³⁶ is preferably thiazolyl, oxazolyl, furanyl, thienyl, pyridyl or the like.

### [Step 1]

In this step, compound (I-45) can be produced by coupling compound (I-44) with compound (XXIII) in the presence of a transition metal catalyst and a base. This reaction is carried out according to the above-mentioned Method W.
Compound (XXIII) used as a starting material compound for this step can be produced according to a method known per se.

### [Step 2]

In this step, compound (I-46) can be produced by coupling compound (I-44) with bis(pinacolato)diboron in the presence of a transition metal catalyst and a base.
This reaction is carried out in a solvent that does not adversely influence the reaction, under an inert gas atmosphere, according to a conventional method.
Examples of the inert gas include argon, nitrogen and the like.
Examples of the transition metal catalyst include tetrakis(triphenylphosphine)palladium(0), [1,1'-bis(diphenylphosphino)ferrocene]palladium(2) dichloride, a catalyst prepared by mixing tris(dibenzylideneacetone)dipalladium(0) with 4 equivalents of 2-(dicyclohexylphosphino)-2',6'-dimethoxybiphenyl, and the like. The amount of the transition metal catalyst to be used is generally 0.001 to 2 mol, preferably 0.01 to 0.1 mol, per 1 mol of compound (I-44).
Examples of the base include alkali metal salts such as potassium hydroxide, sodium hydroxide, sodium hydrogen carbonate, potassium carbonate and the like; amines such as pyridine, triethylamine, N,N-diisopropylethylamine, N,N-dimethylaniline, 1,8-diazabicyclo[5.4.0]undec-7-ene and the like; metal hydrides such as potassium hydride, sodium hydride and the like; alkali metal C₁₋₆ alkoxides such as sodium methoxide, sodium ethoxide, potassium tert-butoxide and the like; carboxylates such as potassium acetate, sodium acetate, sodium formate and the like; alkali metal fluorides such as potassium fluoride, sodium fluoride and the like, and the like. The amount of the base to be used is generally 1 to 10 mol, preferably 1 to 3 mol, per 1 mol of compound (I-44).
The amount of the bis(pinacolato)diboron to be used is generally 1 to 10 mol, preferably 1 to 3 mol, per 1 mol of compound (I-44).
Examples of the solvent that does not adversely influence the reaction include ethers such as diethyl ether, tetrahydrofuran, dioxane and the like; aliphatic hydrocarbons such as hexane, heptane and the like; aromatic hydrocarbons such as benzene, toluene, xylene and the like; amides such as N,N-dimethylformamide, N,N-dimethylacetamide, N-methylpyrrolidone and the like; halogenated hydrocarbons such as dichloromethane, chloroform, 1,2-dichloroethane, 1,1,2,2-tetrachloroethane and the like; nitriles such as acetonitrile and the like; sulfoxides such as dimethylsulfoxide and the like; water and the like. These solvents may be used in a mixture at an appropriate ratio.
The reaction temperature is generally about -80 to about 150°C, preferably about 0 to about 100°C.
The reaction time is generally about 0.5 to about 20 hr.
The bis(pinacolato)diboron used as a starting material compound for this step can be produced according to a method known per se.

### [Step 3]

In this step, compound (I-47) can be produced by coupling compound (I-46) with compound (XXIV) in the presence of a transition metal catalyst and a base. This reaction is carried out according to the above-mentioned Method W.
The amount of compound (XXIV) to be used is generally 1 to 10 mol, preferably 1 to 3 mol, per 1 mol of compound (I-46).
Compound (XXIV) used as a starting material compound for this step can be produced according to a method known per se.

Compound (I-44) used as a starting material compound for the above-mentioned Method AI can be produced, for example, using a starting material compound produced according to a method known per se, according to the above-mentioned Method A, Method B, Method J, Method N, Method C", Method E", Method O, Method U, Method V, Method W, Method X, Method Y, Method Z, Method AA, Method AB, Method AC, Method AD, Method AE, Method AF or Method AG, the below-mentioned Method AM, Method AN, Method AO, Method AP, Method AR, Method AX or Method AY or a method analogous thereto.

Compound (III-75) can be produced, for example, according to the following Method AJ.

### [Method AJ]

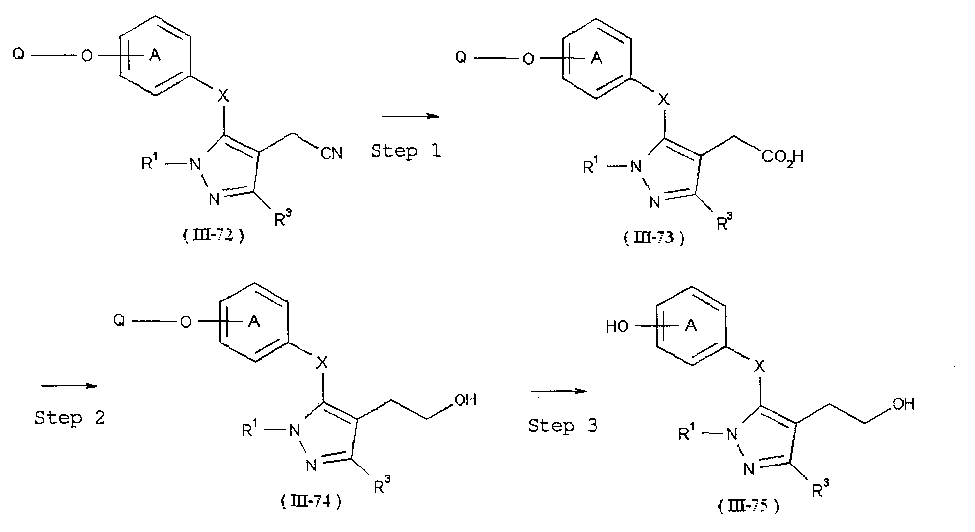

wherein the symbols in the formula are as defined above.

### [Step 1]

In this step, compound (III-73) can be produced by subjecting the cyanomethyl group at 4-position on the pyrazole of compound (III-72) to hydrolysis.
This reaction is carried out in the presence of an acid or a base, in a solvent that does not adversely influence the reaction, according to a conventional method.
Examples of the acid include mineral acids such as hydrochloric acid, sulfuric acid and the like; Lewis acids such as boron trichloride, boron tribromide and the like; organic acids such as trifluoroacetic acid, p-toluenesulfonic acid and the like, and the like. The amount of the acid to be used is preferably about 0.01 to about 5 mol per 1 mol of compound (III-72).
Examples of the base include alkali metal hydroxides such as sodium hydroxide, potassium hydroxide, lithium hydroxide and the like; alkaline earth metal hydroxides such as magnesium hydroxide, calcium hydroxide, barium hydroxide and the like, and the like. The amount of the base to be used is preferably about 1 to about 50 mol per 1 mol of compound (III-72).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; alcohols such as methanol, ethanol and the like; ketones such as acetone and the like, and the like. These solvents may be used in a mixture at an appropriate ratio, or in a mixture with water at an appropriate ratio.
The reaction temperature is generally about -80 to about 150°C, preferably about -10 to about 100°C.
The reaction time is generally about 0.5 to about 20 hr.

### [Step 2]

In this step, compound (III-74) can be produced by reducing compound (III-73).
This reaction is carried out in a solvent that does not adversely influence the reaction, according to a conventional method.
Examples of the reducing agent include metal hydrogen compounds such as sodium bis(2-methoxyethoxy)aluminum hydride, diisobutylaluminum hydride and the like; metal hydrogen complex compounds such as sodium borohydride, sodium cyanoborohydride, lithium aluminum hydride, sodium aluminum hydride and the like; boron hydride compounds such as borane and the like, and the like. The amount of the reducing agent to be used is generally 1 to 20 mol per 1 mol of compound (III-73).
Examples of the solvent that does not adversely influence the reaction include alcohols such as methanol, ethanol, propanol, 2-propanol, butanol, isobutanol, tert-butanol and the like; aromatic hydrocarbons such as benzene, toluene, xylene and the like; aliphatic hydrocarbons such as hexane, heptane and the like; ethers such as diethyl ether, diisopropyl ether, tert-butyl methyl ether, tetrahydrofuran, dioxane, dimethoxyethane and the like; halogenated hydrocarbons such as dichloromethane, chloroform, 1,2-dichloroethane, 1,1,2,2-tetrachloroethane and the like; water and the like. These solvents may be used in a mixture at an appropriate ratio.
The reaction temperature is generally -70 to 150°C, preferably -20 to 100°C.
The reaction time is generally 0.1 to 100 hr, preferably 0.1 to 40 hr.

### [Step 3]

In this step, compound (III-75) can be produced by converting Q of compound (III-74) wherein Q is V to a hydrogen atom. This reaction is carried out in the same manner as in the above-mentioned Method F.

Compound (III-72) used as a starting material compound for the above-mentioned Method AJ can be produced, for example, using a starting material compound produced according to a method known per se, according to the above-mentioned Method F, Method G, Method L, Method C', Method R, Method S, Method AF, Method AG or Method AH or a method analogous thereto.

Compound (III-78) can be produced, for example, according to the following Method AK.

### [Method AK]

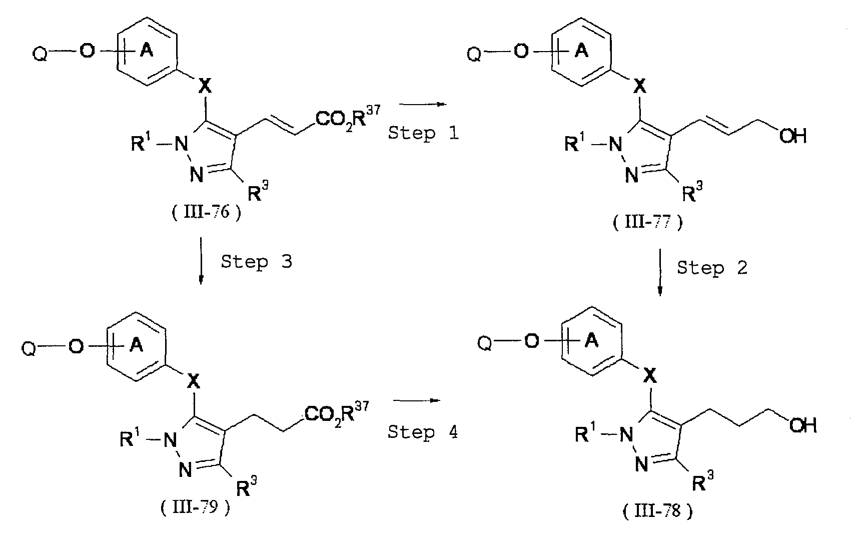

wherein R³⁷ is a hydrogen atom, a C₁₋₁₀ alkyl group, a benzyl group optionally substituted by C₁₋₆ alkyl group(s) or a C₆₋₁₄ aryl group optionally substituted by C₁₋₆ alkyl group(s), and the other symbols are as defined above.
R³⁷ is preferably a hydrogen atom, methyl, ethyl, tert-butyl, benzyl, phenyl or the like.

### [Step 1]

In this step, compound (III-77) can be produced by reducing compound (III-76). This reaction is carried out according to the above-mentioned Method G, Step 3.

### [Step 2]

In this step, compound (III-78) can be produced by subjecting compound (III-77) to a hydrogenation reaction. This reaction is carried out according to the above-mentioned Method E, Step 2.

### [Step 3]

In this step, compound (III-79) can be produced by subjecting compound (III-76) to a hydrogenation reaction. This reaction is carried out according to the above-mentioned Method E, Step 2.

### [Step 4]

In this step, compound (III-78) can be produced by reducing compound (III-79). This reaction is carried out according to the above-mentioned Method G, Step 3.

Compound (III-76) used as a starting material compound for the above-mentioned Method AK can be produced, for example, using a starting material compound produced according to a method known per se, according to the above-mentioned Method F, Method L, Method C', Method E', Method S, Method AG or Method AH or a method analogous thereto.

Compound (I-48), which is compound (I) wherein R² is a (2,4-dioxo-1,3-thiazolidin-5-ylidene)methyl group, can be produced, for example, according to the following Method AM.

### [Method AM]

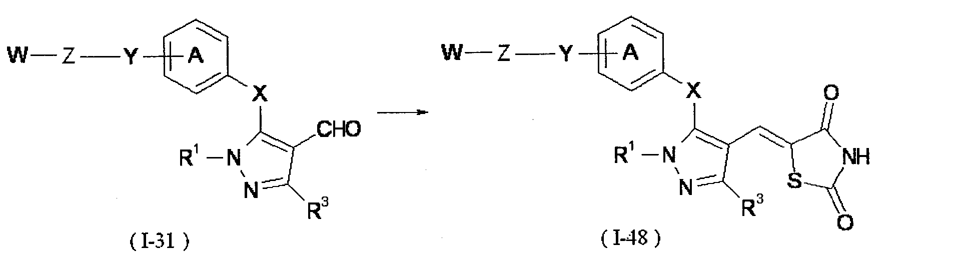

wherein the symbols in the formula are as defined above.

In this method, compound (I-48) can be produced by reacting compound (I-31) with 1,3-thiazolidine-2,4-dione in the presence of a base.
This reaction is carried out in a solvent that does not adversely influence the reaction.
Examples of the base include those exemplified in the above-mentioned Method E, Step 1. The amount of the base to be used is generally 1 to 10 mol, preferably 1 to 5 mol, per 1 mol of compound (I-31).
The amount of the 1,3-thiazolidine-2,4-dione to be used is generally 1 to 10 mol, preferably 1 to 5 mol, per 1 mol of compound (I-31).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; alcohols such as methanol, ethanol and the like; ketones such as acetone and the like; aliphatic hydrocarbons such as hexane, heptane and the like; water and the like. These solvents may be used in a mixture at an appropriate ratio.
The reaction temperature is generally about -100 to about 150°C, preferably about -10 to about 100°C.
The reaction time is generally about 0.5 to about 20 hr.

Compound (I-49), which is compound (I) wherein R² is a 2-{[(tert-butoxycarbonyl)amino]sulfonyl}vinyl group, compound (I-50), which is compound (I) wherein R² is a 2-(aminosulfonyl)vinyl group, and compound (I-51), which is compound (I) wherein R² is a 2-(aminosulfonyl)ethyl, can be produced, for example, according to the following Method AN.

### [Method AN]

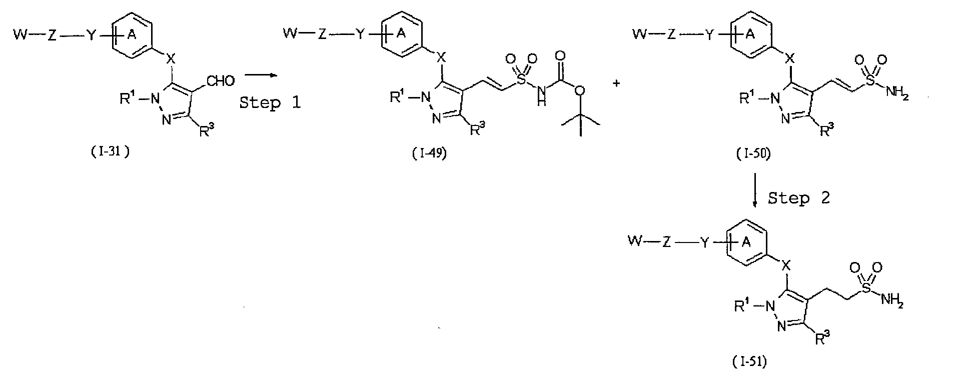

wherein the symbols in the formula are as defined above.

### [Step 1]

In this step, compound (I-49) and compound (I-50) can be produced by reacting compound (I-31) with tert-butyl {[(diphenylphosphoryl)methyl]sulfonyl}carbamate in the presence of a base.
This reaction is carried out in a solvent that does not adversely influence the reaction, according to a conventional method.
Examples of the base include those exemplified in the above-mentioned Method E, Step 1. The amount of the base to be used is preferably about 1 to about 5 mol per 1 mol of compound (I-31).
The amount of the tert-butyl {[(diphenylphosphoryl)methyl]sulfonyl}carbamate to be used is preferably about 1 to about 5 mol per 1 mol of compound (I-31).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like, and the like. These solvents may be used in a mixture at an appropriate ratio.
The reaction temperature is generally about -80 to about 150°C, preferably about -10 to about 100°C.
The reaction time is generally about 0.5 to about 20 hr.

### [Step 2]

In this step, compound (I-51) can be produced by subjecting compound (I-50) to a hydrogenation reaction. This reaction is carried out according to the above-mentioned Method E, Step 2.

Compound (I-53), which is compound (I) wherein R² is R³⁸OCH₂- wherein R³⁸ is as defined below, can be produced, for example, according to the following Method AO.

### [Method AO]

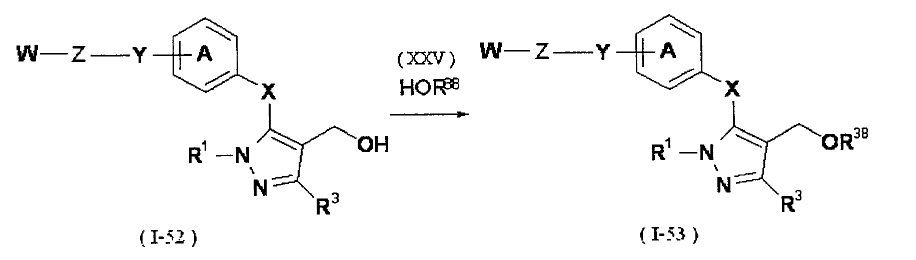

wherein R³⁸ is a C₆₋₁₄ aryl group optionally substituted by C₁₋₆ alkyl group(s), and the other symbols are as defined above.
R³⁸ is preferably phenyl or the like.

In this method, compound (I-53) can be produced by condensing compound (I-52) with compound (XXV).
This reaction is carried out according to a method known per se, for example, the method described in Synthesis page 1 (1981), or a method analogous thereto. That is, this reaction is generally carried out in the presence of an organic phosphorus compound and an electrophilic agent, in a solvent that does not adversely influence the reaction.
Examples of the organic phosphorus compound include triphenylphosphine, tributylphosphine and the like.
Examples of the electrophilic agent include diethyl azodicarboxylate, diisopropyl azodicarboxylate, 1,1'-azodicarbonyldipiperidine and the like.
The amount of the organic phosphorus compound and electrophilic agent to be used is preferably about 1 to about 5 mol per 1 mol of compound (I-52), respectively.
Examples of compound (XXV) include phenol, 4-methylphenol, 4-methoxyphenol and the like. The amount of compound (XXV) to be used is preferably about 1 to about 5 mol per 1 mol of compound (I-52).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; ketones such as acetone, 2-butanone and the like, and the like. These solvents may be used in a mixture at an appropriate ratio.
The reaction temperature is generally about -80 to about 150°C, preferably about -10 to about 100°C.
The reaction time is generally about 0.5 to about 20 hr.

Compound (I-52) used as a starting material compound for the above-mentioned Method AO can be produced, for example, using a starting material compound produced according to a method known per se, according to the above-mentioned Method A, Method B, Method J, Method N, Method C'', Method E'', Method O, Method V, Method W, Method X, Method Y, Method Z, Method AF, Method AG or Method AI or a method analogous thereto. Compound (XXV) can be produced according to a method known per se.

Compound (I-55), which is compound (I) wherein R² is R³⁹R⁴⁰NCO₂T'- wherein R³⁹, R⁴⁰ and T' are as defined below, can be produced, for example, according to the following Method AP.

### [Method AP]

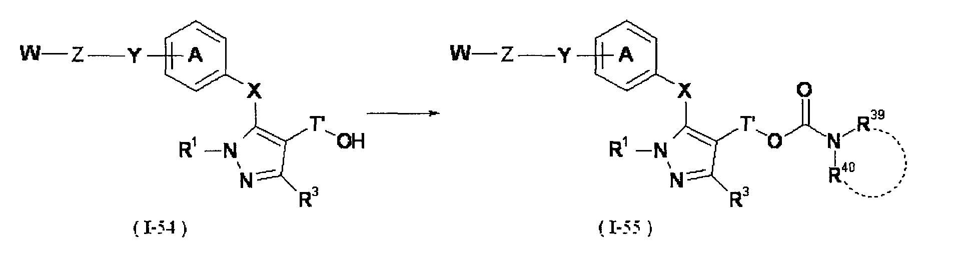

wherein R³⁹ and R⁴⁰ are each independently a hydrogen atom, a C₁₋₁₀ alkyl group, a benzyl group optionally substituted by C₁₋₆ alkyl group(s), a C₆₋₁₄ aryl group optionally substituted by C₁₋₆ alkyl group(s), a C₃₋₁₀ cycloalkyl group, a C₃₋₁₀ cycloalkyl-C₁₋₆ alkyl group, a C₁₋₆ alkoxy-C₁₋₁₀ alkyl group, a C₁₋₁₀ alkoxy group, a benzyloxy group optionally substituted by C₁₋₆ alkyl group(s), a C₆₋₁₄ aryloxy group optionally substituted by C₁₋₆ alkyl group(s) or a C₁₋₁₀ alkyl group optionally substituted by aromatic heterocyclic group(s) optionally substituted by C₁₋₆ alkyl group(s), or R³⁹ and R⁴⁰ are optionally bonded to each other to form an optionally substituted ring, T' is CH₂, CH₂CH₂, CH₂CH₂CH₂, CH₂CH₂CH₂CH₂ or CH=CHCH₂, and the other symbols are as defined above.
Examples of the "optionally substituted ring" formed by R³⁹ and R⁴⁰ bonded to each other include
(1) a C₃₋₁₀ cycloalkyl group;
(2) a C₆₋₁₄ aryl group optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group optionally substituted by 1 to 3 halogen atoms, and
   (d) a halogen atom;
(3) an aromatic heterocyclic group optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group optionally substituted by 1 to 3 halogen atoms, and
   (d) a halogen atom;
(4) a monocyclic non-aromatic heterocyclic group optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group optionally substituted by 1 to 3 halogen atoms, and
   (d) a halogen atom
and the like.
R³⁹ and R⁴⁰ is preferably each independently a hydrogen atom, methyl, ethyl, propyl, cyclopropyl, cyclopropylmethyl, 2-methoxyethyl, phenyl, methoxy, ethoxy, phenoxy or the like, or R³⁹ and R⁴⁰ are bonded to each other to form pyrrolidine, piperidine, hexamethylenimine, morpholine or the like.

In this method, compound (I-55) can be produced by reacting compound (I-54) with R³⁹R⁴⁰NCO. This reaction is carried out according to the above-mentioned Method U, Step 3.
Alternatively, in this step, compound (I-55) can be produced by reacting compound (I-54) with 1,1'-carbonylbis-1H-imidazole, and then reacting the resulting compound with R³⁹R⁴⁰NH₂.
This reaction is carried out in a solvent that does not adversely influence the reaction, according to a conventional method.
The amount of the 1,1'-carbonylbis-1H-imidazole to be used is preferably about 1 to about 3 mol per 1 mol of compound (I-54).
Examples of R³⁹R ⁴⁰NH₂ include amines such as ammonia, methylamine, ethylamine, dimethylamine, diethylamine, ethylmethylamine, propylamine, methylpropylamine; anilines such as aniline, N-methylaniline and the like, and the like. The amount of R³⁹R⁴⁰NH₂ to be used is preferably about 1 to about 5 mol per 1 mol of compound (I-54).
Examples of the solvent that does not adversely influence the reaction include ethers such as diethyl ether, tetrahydrofuran, dioxane, 1,2-dimethoxyethane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; ketones such as acetone, 2-butanone and the like, and the like. These solvents may be used in a mixture at an appropriate ratio.
In the case of the reaction with 1,1'-carbonylbis-1H-imidazole, the reaction temperature is generally about -100 to about 150°C, preferably about -20 to about 50°C. In the case of the reaction with R³⁹R⁴⁰NH₂, the reaction temperature is generally about -80 to about 150°C, preferably about 0 to about 100°C.
In the case of the reaction with 1,1'-carbonylbis-1H-imidazole, the reaction time is generally about 0.5 to about 20 hr. In the case of the reaction with R³⁹R⁴⁰NH₂, the reaction time is generally about 0.5 to about 20 hr.

Compound (I-54) used as a starting material compound for the above-mentioned Method AP can be produced, for example, using a starting material compound produced according to a method known per se, according to the above-mentioned Method A, Method B, Method J, Method N, Method C'', Method E'', Method O, Method V, Method W, Method X, Method Y, Method Z, Method AD, Method AF, Method AG, Method AI, Method AU or Method AV or a method analogous thereto.

Compound (I-59), which is compound (I) wherein R² is R⁴¹R⁴²NCO- wherein R⁴¹ and R⁴² are as defined below, and compound (I-60), which is compound (I) wherein R² is a 1,3,4-oxadiazol-2-yl group, can be produced, for example, according to the following Method AR.

### [Method AR]

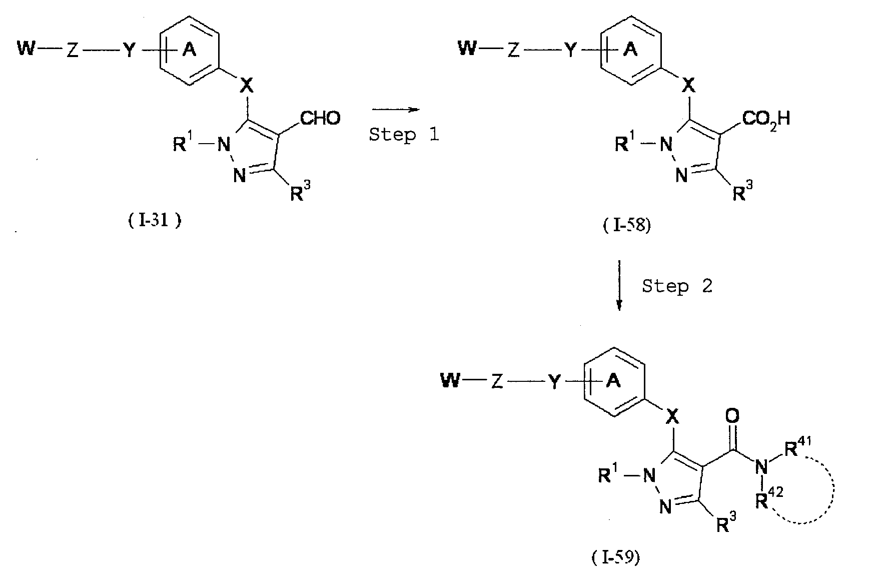

wherein R⁴¹ and R⁴² are each independently a hydrogen atom, a C₁₋₁₀ alkyl group, a benzyl group optionally substituted by C₁₋₆ alkyl group(s), a C₆₋₁₄ aryl group optionally substituted by C₁₋₆ alkyl group(s), a C₃₋₁₀ cycloalkyl group, a C₃₋₁₀ cycloalkyl-C₁₋₆ alkyl group or a C₁₋₆ alkoxy-C₁₋₁₀ alkyl group, or R⁴¹ and R⁴² are optionally bonded to each other to form an optionally substituted ring, and the other symbols are as defined above.
Examples of the "optionally substituted ring" formed by R⁴¹ and R⁴² bonded to each other include
(1) a C₃₋₁₀ cycloalkyl group;
(2) a C₆₋₁₄ aryl group optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group optionally substituted by 1 to 3 halogen atoms, and
   (d) a halogen atom;
(3) an aromatic heterocyclic group optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group optionally substituted by 1 to 3 halogen atoms, and
   (d) a halogen atom;
(4) a monocyclic non-aromatic heterocyclic group optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group optionally substituted by 1 to 3 halogen atoms, and
   (d) a halogen atom
and the like.
R⁴¹ and R⁴² is preferably each independently a hydrogen atom, methyl, ethyl, propyl, cyclopropyl, cyclopropylmethyl, 3-methoxypropyl, phenyl or the like, or R⁴¹ and R⁴² are bonded to each other to form pyrrolidine, piperidine, hexamethylenimine, morpholine or the like.

### [Step 1]

In this step, compound (I-58) can be produced by oxidizing compound (I-31).
This reaction can be carried out in the presence of 2-methyl-2-butene, sodium dihydrogen phosphate and sodium chlorite, in a solvent that does not adversely influence the reaction, according to a conventional method.
The amount of the 2-methyl-2-butene to be used is preferably about 1 to about 20 mol per 1 mol of compound (I-31).
The amount of the sodium dihydrogen phosphate to be used is preferably about 1 to about 20 mol per 1 mol of compound (I-31) .
The amount of the sodium chlorite to be used is preferably about 1 to about 10 mol per 1 mol of compound (I-31) .
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; alcohols such as methanol, ethanol, tert-butanol and the like; water and the like. These solvents may be used in a mixture at an appropriate ratio.
The reaction temperature is generally about -80 to about 150°C, preferably about -10 to about 100°C.
The reaction time is generally about 0.5 to about 20 hr.

Alternatively, this reaction can be carried out in the presence of the Jones' reagent, in a solvent that does not adversely influence the reaction according to a conventional method.
The amount of the Jones' reagent to be used is preferably about 1 to about 10 mol per 1 mol of compound (I-31).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; alcohols such as methanol, ethanol, tert-butanol and the like; ketones such as acetone and the like; water and the like. These solvents may be used in a mixture at an appropriate ratio.
The reaction temperature is generally about -80 to about 150°C, preferably about -10 to about 100°C.
The reaction time is generally about 0.5 to about 20 hr.

### [Step 2]

In this step, compound (I-59) can be produced by subjecting compound (I-58) to an amidation reaction. This reaction is carried out according to the above-mentioned Method B, Step 2.

Compound (III-89), compound (III-91) and compound (III-93) wherein R⁴⁵ and R⁴⁶ are as defined below can be produced, for example, according to the following Method AT.

### [Method AT]

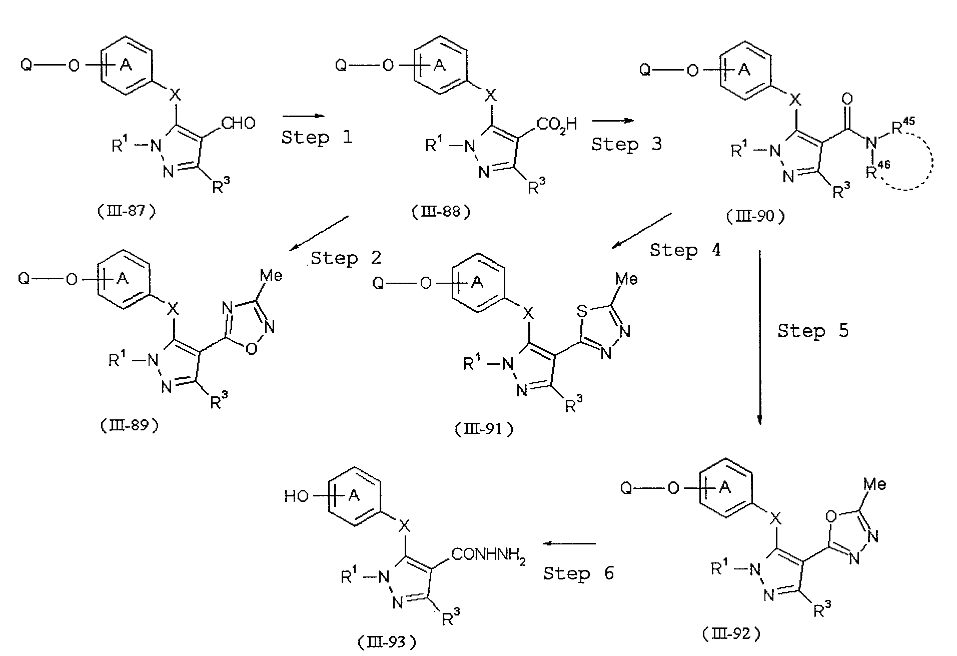

wherein R⁴⁵ and R⁴⁶ are each independently a hydrogen atom, a C₁₋₁₀ alkyl group, a benzyl group optionally substituted by C₁₋₆ alkyl group(s), a C₆₋₁₄ aryl group optionally substituted by C₁₋₆ alkyl group(s), a C₃₋₁₀ cycloalkyl group, a C₃₋₁₀ cycloalkyl-C₁₋₆ alkyl group, a C₁₋₆ alkoxy-C₁₋₁₀ alkyl group or a C₁₋₆ alkylcarbonylamino group, or R⁴⁴ and R⁴⁵ are optionally bonded to each other to form an optionally substituted ring, and the other symbols are as defined above.
Examples of the "optionally substituted ring" formed by R⁴⁵ and R⁴⁶ bonded to each other include
(1) a C₃₋₁₀ cycloalkyl group;
(2) a C₆₋₁₄ aryl group optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group optionally substituted by 1 to 3 halogen atoms, and
   (d) a halogen atom;
(3) an aromatic heterocyclic group optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group optionally substituted by 1 to 3 halogen atoms, and
   (d) a halogen atom;
(4) a monocyclic non-aromatic heterocyclic group optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group optionally substituted by 1 to 3 halogen atoms, and
   (d) a halogen atom
and the like.
R⁴⁵ and R⁴⁶ is preferably each independently a hydrogen atom, methyl, ethyl, propyl, cyclopropyl, cyclopropylmethyl, 3-methoxypropyl, phenyl, acetylamino or the like, or R⁴⁴ and R⁴⁵ are bonded to each other to form pyrrolidine, piperidine, hexamethylenimine, morpholine or the like.

### [Step 1]

In this step, compound (III-88) can be produced by oxidizing compound (III-87). This reaction is carried out according to the above-mentioned Method AR, Step 1.

### [Step 2]

In this step, compound (III-89) can be produced from compound (III-88).
This reaction is carried out according to a method known per se, for example, the method described in Bioorganic & Medicinal Chemistry Letters, page 209 (1999), or a method analogous thereto. That is, this reaction is generally carried out in the presence of N,N'-carbonyldiimidazole and N-hydroxyacetamidine, in a solvent that does not adversely influence the reaction.
The amount of the N,N'-carbonyldiimidazole to be used is preferably about 1 to about 10 mol per 1 mol of compound (III-88).
The amount of the N-hydroxyacetamidine to be used is preferably about 1 to about 10 mol per 1 mol of compound (III-88).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; nitriles such as acetonitrile and the like, and the like. These solvents may be used in a mixture at an appropriate ratio.
The reaction temperature is generally about -80 to about 150°C, preferably about -10 to about 100°C.
The reaction time is generally about 0.5 to about 20 hr.

### [Step 3]

In this step, compound (III-90) can be produced by subjecting compound (III-88) to an amidation reaction. This reaction is carried out according to the above-mentioned Method B, Step 2.

### [Step 4]

In this step, compound (III-91) can be produced from compound (III-90) wherein R⁴⁵ is a hydrogen atom, and R⁴⁶ is acetylamino.
This reaction is carried out according to a method known per se, for example, the method described in Journal of Medicinal Chemistry, page 4409 (2006), or a method analogous thereto. That is, this reaction is generally carried out in the presence of the Lawesson's reagent, in a solvent that does not adversely influence the reaction.
The amount of the Lawesson's reagent to be used is preferably about 1 to about 10 mol per 1 mol of compound (III-90).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; nitriles such as acetonitrile and the like, and the like. These solvents may be used in a mixture at an appropriate ratio.
The reaction temperature is generally about -80 to about 150°C, preferably about -10 to about 100°C.
The reaction time is generally about 0.5 to about 20 hr.

### [Step 5]

In this step, compound (III-92) can be produced from compound (III-90) wherein R⁴⁵ is a hydrogen atom, and R⁴⁶ is acetylamino.
This reaction is carried out according to a method known per se, for example, the method described in Synlett, page 382, or a method analogous thereto. That is, this reaction is generally carried out in the presence of (methoxycarbonylsulfamoyl)triethylammonium hydroxide intramolecular salt, in a solvent that does not adversely influence the reaction.
The amount of the (methoxycarbonylsulfamoyl)triethylammonium hydroxide intramolecular salt to be used is preferably about 1 to about 10 mol per 1 mol of compound (III-90).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; nitriles such as acetonitrile and the like, and the like. These solvents may be used in a mixture at an appropriate ratio.
The reaction temperature is generally about -80 to about 150°C, preferably about -10 to about 100°C.
The reaction time is generally about 0.5 to about 20 hr.

### [Step 6]

In this step, compound (III-93) can be produced by reacting compound (III-92) with an acid.
This reaction is carried out in a solvent that does not adversely influence the reaction, according to a conventional method.
Examples of the acid include mineral acids such as hydrochloric acid, sulfuric acid and the like; Lewis acids such as boron trichloride, boron tribromide and the like; organic acids such as trifluoroacetic acid, p-toluenesulfonic acid and the like; and hydrogen chloride-methanol, hydrogen chloride-ethyl acetate and the like, which are prepared by dissolving hydrogen chloride gas in a solvent such as methanol, ethyl acetate and the like. The amount of the acid to be used is generally 0.01 to 500 mol, preferably 0.01 to 20 mol, per 1 mol of compound (III-92).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; nitriles such as acetonitrile and the like; esters such as ethyl acetate and the like; alcohols such as methanol, ethanol and the like; water and the like. These solvents may be used in a mixture at an appropriate ratio.
The reaction temperature is generally about -80 to about 150°C, preferably about -10 to about 100°C.
The reaction time is generally about 0.5 to about 20 hr.

Compound (III-87) used as a starting material compound for the above-mentioned Method AT can be produced, for example, using a starting material compound produced according to a method known per se, according to the above-mentioned Method F, Method L, Method C' or Method AG or a method analogous thereto.

Compound (III-96) can be produced, for example, according to the following Method AU.

### [Method AU]

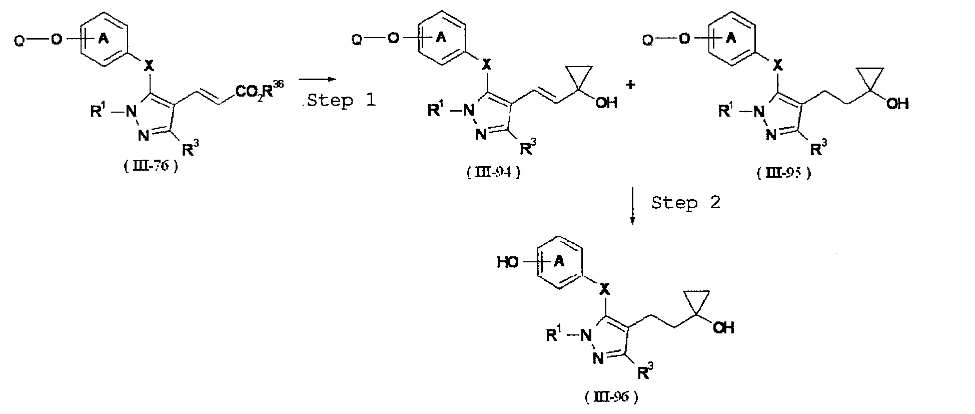

wherein the symbols in the formula are as defined above.

### [Step 1]

In this step, compound (III-94) or a mixture of compound (III-94) and compound (III-95) can be produced, form compound (III-76).
This reaction is carried out according to a method known per se, for example, the method described in Synthesis page 234 (1991), or a method analogous thereto. That is, this reaction is generally carried out in the presence of titanium tetraisopropoxide and ethylmagnesium bromide, in a solvent that does not adversely influence the reaction.
The amount of the titanium tetraisopropoxide to be used is preferably about 1 to about 5 mol per 1 mol of compound (III-76).
The amount of the ethylmagnesium bromide to be used is preferably about 1 to about 5 mol per 1 mol of compound (III-76).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like, and the like. These solvents may be used in a mixture at an appropriate ratio.
The reaction temperature is generally about -100 to about 150°C, preferably about -80 to about 50°C.
The reaction time is generally about 0.5 to about 20 hr.

### [Step 2]

In this step, when Q is benzyl or p-methoxybenzyl, compound (III-96) can be produced by subjecting compound (III-94) or a mixture of compound (III-94) and compound (III-95) to an hydrogenation reaction. This reaction is carried out according to the above-mentioned Method E, Step 2.

Compound (III-97) wherein R⁴⁷ is as defined below can be produced, for example, according to the following Method AV.

### [Method AV]

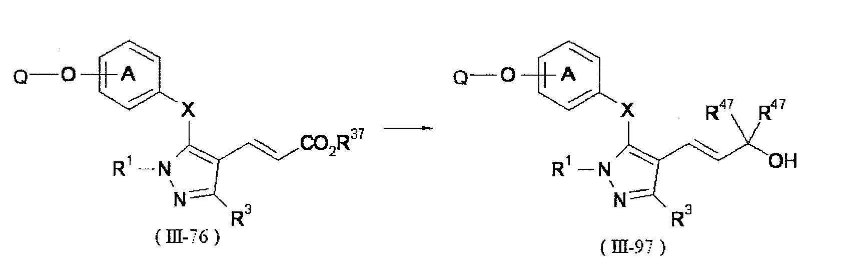

wherein R⁴⁷ is a C₁₋₁₀ alkyl group, a benzyl group optionally substituted by C₁₋₆ alkyl group(s), a C₆₋₁₄ aryl group optionally substituted by C₁₋₆ alkyl group (s) or a C₃₋₁₀ cycloalkyl group, and the other symbols are as defined above.
R⁴⁷ is preferably methyl, ethyl, propyl, cyclopropyl, phenyl or the like.

In this method, compound (III-97) can be produced by reacting compound (III-76) with an organic metal reagent. This reaction is carried out according to the above-mentioned Method E, Step 3.

Compound (1-66), which is compound (I) wherein R² is R⁵¹R⁵²NCOCH=CH- wherein R⁵¹ and R⁵² are as defined below, and W is -CO₂Me, compound (I-68), which is compound (I) wherein R² is HO₂CC(R⁵³) (R⁵⁴)NHCOCH=CH- wherein R⁵³ and R⁵⁴ are as defined below, and W is -CO₂Me, and compound (I-70), which is compound (I) wherein R² is HO₂CC(R⁵³) (R⁵⁴)NHCOCH=CH-, and W is -CO₂H, can be produced, for example, according to the following Method AX.

### [Method AX]

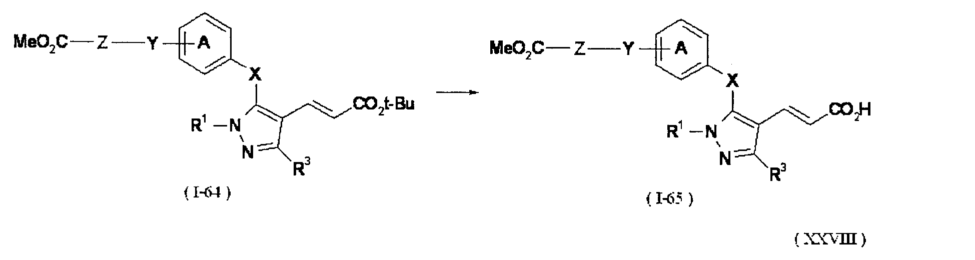

wherein the symbols in the formula are as defined above.

In this method, compound (I-65) can be produced by reacting compound (I-64) with trifluoroacetic acid.
This reaction may be carried out without solvent or in a solvent that does not adversely influence the reaction.
The amount of the trifluoroacetic acid to be used is preferably about 0.01 to about 500 mol per 1 mol of compound (I-64).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; nitriles such as acetonitrile and the like, and the like. These solvents may be used in a mixture at an appropriate ratio.
The reaction temperature is generally about -100 to about 150°C, preferably about -20 to about 80°C.
The reaction time is generally about 0.5 to about 20 hr.

Compound (I-64) used as a starting material compound for the above-mentioned Method AX can be produced, for example, using a starting material compound produced according to a method known per se, according to the above-mentioned Method A, Method J, Method C", Method E", Method V, Method X, Method Y, Method Z, Method AF, Method AG or Method AI or a method analogous thereto.

Compound (I-74), which is compound (I) wherein R² is - CONHNH₂, and W is CO₂H, can be produced, for example, according to the following Method AY.

### [Method AY]

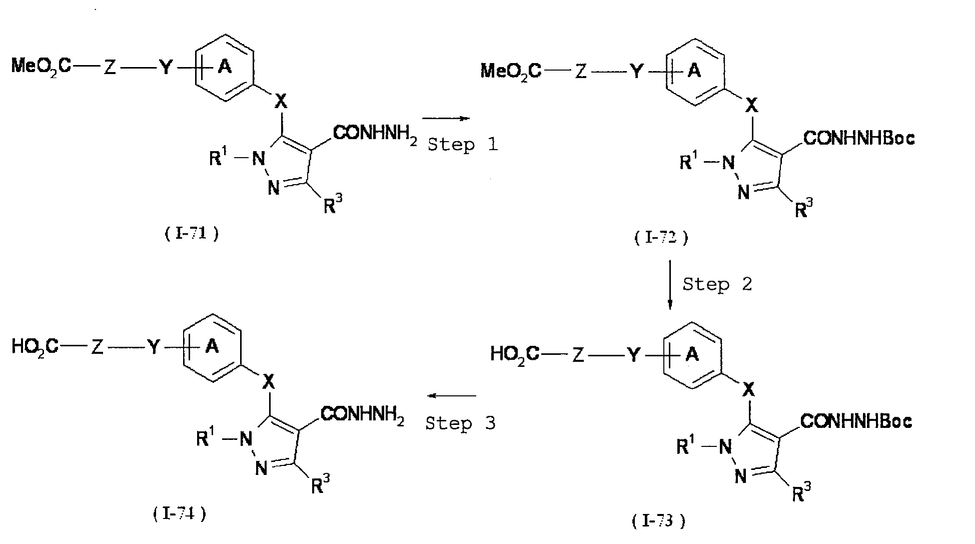

wherein the symbols in the formula are as defined above.

### [Step 1]

In this step, compound (I-72) can be produced by reacting compound (I-71) with di-tert-butyl dicarbonate in the presence of a base.
This reaction is carried out in a solvent that does not adversely influence the reaction.
Examples of the base include amines such as triethylamine, N,N-diisopropylethylamine, N-methylmorpholine, N,N-dimethylaniline, 4-dimethylaminopyridine and the like; alkali metal salts such as sodium hydrogen carbonate, sodium carbonate, potassium carbonate and the like; alkali metal hydroxides such as sodium hydroxide, potassium hydroxide, lithium hydroxide and the like; alkaline earth metal hydroxides such as magnesium hydroxide, calcium hydroxide, barium hydroxide and the like; metal hydrides such as potassium hydride, sodium hydride and the like; alkali metal C₁₋₆ alkoxides such as sodium methoxide, sodium ethoxide, potassium tert-butoxide and the like, and the like. The amount of the base to be used is preferably about 1 to about 5 mol per 1 mol of compound (I-71).
The amount of the di-tert-butyl dicarbonate to be used is preferably about 1 to about 5 mol per 1 mol of compound (I-71).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; nitriles such as acetonitrile and the like, and the like. These solvents may be used in a mixture at an appropriate ratio.
The reaction temperature is generally about -100 to about 100°C, preferably about -20 to about 80°C.
The reaction time is generally about 0.5 to about 20 hr.

### [Step 2]

In this step, compound (I-73) can be produced by subjecting compound (I-72) to hydrolysis. This reaction is carried out according to the above-mentioned Method B, Step 1.

### [Step 3]

In this step, compound (I-74) can be produced by reacting compound (I-73) with an acid.
This reaction is carried out in a solvent that does not adversely influence the reaction, according to a conventional method.
Examples of the acid include mineral acids such as hydrochloric acid, sulfuric acid and the like; Lewis acids such as boron trichloride, boron tribromide and the like; organic acids such as trifluoroacetic acid, p-toluenesulfonic acid and the like; and hydrogen chloride-methanol, hydrogen chloride-ethyl acetate and the like, which are prepared by dissolving hydrogen chloride gas in a solvent such as methanol, ethyl acetate and the like. The amount of the acid to be used is generally 0.01 to 500 mol, preferably 0.01 to 5 mol, per 1 mol of compound (I-73).
Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like; alcohols such as methanol, ethanol and the like; ketones such as acetone and the like; esters such as ethyl acetate and the like; water and the like. These solvents may be used in a mixture at an appropriate ratio.
The reaction temperature is generally about -80 to about 150°C, preferably about -10 to about 100°C.
The reaction time is generally about 0.5 to about 20 hr.

Compound (I-71) used as a starting material compound for the above-mentioned Method AY can be produced, for example, using a starting material compound produced according to a method known per se, according to the above-mentioned Method A, Method J, Method C", Method V, Method X, Method Y, Method Z, Method AF, Method AG, Method AI or Method AT or a method analogous thereto.

In each of the above-mentioned reactions, when the starting compound has an amino group, a carboxyl group, a hydroxy group or a carbonyl group as a substituent, a protecting group generally used in the peptide chemistry and the like may be introduced into these groups, and the object compound can be obtained by eliminating the protecting group as necessary after the reaction.

Examples of the amino-protecting group include a formyl group, a C₁₋₆ alkyl-carbonyl group, a C₁₋₆ alkoxy-carbonyl group, a benzoyl group, a C₇₋₁₀ aralkyl-carbonyl group (e.g., benzylcarbonyl), a C₇₋₁₄ aralkyloxy-carbonyl group (e.g., benzyloxycarbonyl, 9-fluorenylmethoxycarbonyl), a trityl group, a phthaloyl group, a N,N-dimethylaminomethylene group, a substituted silyl group (e.g., trimethylsilyl, triethylsilyl, dimethylphenylsilyl, tert-butyldimethylsilyl, tert-butyldiethylsilyl), C₂₋₆ alkenyl group (e.g., 1-allyl) and the like. These groups are optionally substituted by 1 to 3 substituents selected from a halogen atom, a C₁₋₆ alkoxy group and a nitro group.

Examples of the carboxyl-protecting group include a C₁₋₆ alkyl group, a C₇₋₁₁ aralkyl group (e.g., benzyl), a phenyl group, a trityl group, a substituted silyl group (e.g., trimethylsilyl, triethylsilyl, dimethylphenylsilyl, tert-butyldimethylsilyl, tert-butyldiethylsilyl), a C₂₋₆ alkenyl group (e.g., 1-allyl) and the like. These groups are optionally substituted by 1 to 3 substituents selected from a halogen atom, a C₁₋₆ alkoxy group and a nitro group.

Examples of the hydroxy-protecting group include a C₁₋₆ alkyl group, a phenyl group, a trityl group, a C₇₋₁₀ aralkyl group (e.g., benzyl), a formyl group, a C₁₋₆ alkyl-carbonyl group, a benzoyl group, a C₇₋₁₀ aralkyl-carbonyl group (e.g., benzylcarbonyl), a 2-tetrahydropyranyl group, a 2-tetrahydrofuranyl group, a substituted silyl group (e.g., trimethylsilyl, triethylsilyl, dimethylphenylsilyl, tert-butyldimethylsilyl, tert-butyldiethylsilyl), a C₂₋₆ alkenyl group (e.g., 1-allyl) and the like. These groups are optionally substituted by 1 to 3 substituents selected from a halogen atom, a C₁₋₆ alkyl group, a C₁₋₆ alkoxy group and a nitro group.

Examples of the carbonyl-protecting group include a cyclic acetals (e.g., 1,3-dioxane), a non-cyclic acetals (e.g., di-C₁₋₆ alkylacetal) and the like.

Elimination of the above-mentioned protecting group can be carried out according to a method known per se, for example, a method described in Protective Groups in Organic Synthesis, John Wiley and Sons (1980) and the like. For example, employed is a method using an acid, a base, UV light, hydrazine, phenyl hydrazine, sodium N-methyldithiocarbamate, tetrabutylammonium fluoride, palladium acetate, trialkylsilyl halides (e.g., trimethylsilyl iodide, trimethylsilyl bromide) or the like, reduction or the like.

The compound of the present invention obtained by each production method mentioned above can be isolated and purified by a known means such as concentration, concentration under reduced pressure, solvent extraction, crystallization, recrystallization, phase transfer, chromatography and the like. Each starting compound used in each of the above-mentioned production methods can be isolated and purified by a known means similar to those mentioned above. It is also possible to use such starting compound as it is in a reaction mixture without isolation, as a starting material for the next step.

When compound (I) contains an optical isomer, a stereoisomer, a regioisomer or a rotational isomer, they are also encompassed in compound (I) and can be obtained as single products by synthesis techniques and separation techniques known *per se.* For example, when compound (I) contains an optical isomer, an optical isomer separated from the compound is also encompassed in compound (I).

The present invention is explained in detail in the following by referring to Experimental Example, Reference Examples, Examples and Formulation Examples, which are not to be construed as limitative.

### Example

In the following Reference Examples and Examples, "%" means wt% unless otherwise specified, and "room temperature" means a temperature of 1°C to 30°C unless otherwise specified.

### Reference Example 1 2,4-dichloro-5-[(4-methoxybenzyl)oxy]phenol

While stirring a solution of 4,6-dichlororesorcinol (103.37 g) cooled to 0°C in an ice bath in N,N-dimethylformamide (1000 mL), 60% sodium hydride (oil, 46.20 g) was added by small portions. The ice bath was removed, and the reaction mixture was stirred at room temperature for 1 hr, after which cooled to 0°C again in an ice bath. While stirring the reaction mixture, 4-methoxybenzyl chloride (90.36 g) was added dropwise over 30 min. The reaction mixture was stirred at 0°C for 4 hr. The ice bath was removed and the mixture was stirred at room temperature for 70 hr, and concentrated under reduced pressure. Water (750 mL) and ethyl acetate (750 mL) were added to the residue, and the mixture was stirred while heating to 60°C until it was completely dissolved. Two layers were separated, and the organic layer was washed with saturated brine, and dried over anhydrous magnesium sulfate. After filtration, the filtrate was concentrated. Toluene (600 mL) and 0.5N aqueous sodium hydroxide solution (1300 mL) were added to the residue, and the mixture was stirred while heating to 80°C until it was completely dissolved. Two layers were separated, and the aqueous layer was washed with toluene, neutralized with 1N hydrochloric acid (650 mL), and extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 80:20, v/v) and crystallized from hexane-diisopropyl ether to give the title compound (53.40 g, yield 31%) as colorless crystals.
¹H-NMR (300 MHz, CDCl₃)δ:3.82 (s, 3 H), 5.04 (s, 2 H), 5.45 (s, 1 H), 6.68 (s, 1 H), 6.92 (d, J=8.7Hz, 2 H), 7.32 (s, 1 H), 7.36 (d, J = 8.9 Hz, 2 H).

### Reference Example 2 5-{2,4-dichloro-5-[(4-methoxybenzyl)oxy]phenoxy}-1,3-dimethyl-1H-pyrazole-4-carbaldehyde

Potassium carbonate (66.32 g) was added to a solution of 2,4-dichloro-5-[(4-methoxybenzyl)oxy]phenol (115.92 g) obtained in Reference Example 1 and 5-chloro-1,3-dimethyl-1H-pyrazole-4-carbaldehyde (58.68 g) in N,N-dimethylformamide (360 mL) with stirring. Under a nitrogen atmosphere, the mixture was stirred at 120°C for 24 hr. The reaction mixture was cooled to room temperature, water (1500 mL) was slowly added with vigorous stirring, and the mixture was further stirred for 1 hr. The resulting brown crystals were collected by filtration, washed with water, and dried. The crystals were dissolved in a small amount of toluene, and the solution was subjected to silica gel column chromatography (hexane-ethyl acetate 75:25 - 67:33, v/v) and crystallized from hexane-ethyl acetate to give the title compound (111.45 g, yield 68%) as colorless crystals.
¹H-NMR (300 MHz, CDCl₃)δ:2.47 (s, 3 H), 3.59 (s, 3 H), 3.82 (s, 3 H), 4.97 (s, 2 H), 6.45 (s, 1 H), 6.88 (d, J=8.7 Hz, 2 H), 7.21 (d, J = 8.7 Hz, 2 H), 7.47 (s, 1 H), 9.39 (s, 1 H).

### Reference Example 3 2,4-dichloro-5-[(1,3,4-trimethyl-1H-pyrazol-5-yl)oxy]phenol

To a mixture of 5-{2,4-dichloro-5-[(4-methoxybenzyl)oxy]phenoxy}-1,3-dimethyl-1H-pyrazole-4-carbaldehyde (25.00 g) obtained in Reference Example 2 and diethylene glycol (100 mL) was added hydrazine monohydrate (5.05 mL), and the mixture was stirred with heating at 125°C for 15 min. Potassium hydroxide (4.00 g) was added, and the mixture was stirred with heating at 125°C for 7 hr. The reaction mixture was cooled to room temperature, 30% sulfuric acid (200 mL) and tetrahydrofuran (200 mL) were added, and the mixture was stirred with heating at 50°C for 2 hr. While cooling the reaction mixture in an ice water bath, 8M sodium hydroxide solution (125 mL) and water were added. The mixture was neutralized by adding sodium hydrogen carbonate by small portions until the gas was not generated, and the mixture was extracted with ethyl acetate. The organic layer was washed with water and saturated brine, dried over anhydrous magnesium sulfate and filtered, and the filtrate was concentrated. Toluene was added to the obtained residue. The resulting crystals were collected by filtration, and washed with toluene to give the title compound as pale-yellow crystals (11.11 g, yield 65%).
¹H-NMR (300 MHz, DMSO-d₆)δ:1.70 (s, 3 H), 2.08 (s, 3 H), 3.48 (s, 3 H), 6.39 (s, 1 H), 7.60 (s, 1 H), 10.61 (br s, 1 H).

### Reference Example 4 ethyl 2-(2,4-dichloro-5-hydroxyphenoxy)propionate

While stirring a solution of 4,6-dichlororesorcinol (10.00 g, 55.9 mmol) cooled to 0°C in an ice bath in N,N-dimethylformamide (40 mL), sodium methoxide (3.02 g, 55.9 mmol) was added by small portions. The ice bath was removed, and the mixture was stirred at room temperature for 1 hr, after which cooled to 0°C again in an ice bath. While stirring the reaction mixture, ethyl 2-bromopropionate (7.26 mL, 55.9 mmol) was added dropwise. The reaction mixture was stirred at 0°C for 1 hr. The ice bath was removed and the mixture was stirred at room temperature for 18 hr. Dil. hydrochloric acid was added to the reaction mixture, and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 85:15 - 67:33, v/v) to give the title compound as a colorless oil (3.19 g, yield 20%).
¹H-NMR (300 MHz, CDCl₃)δ:1.27 (t, J = 7.2 Hz, 3 H), 1.67 (d, J = 6.8 Hz, 3 H), 4.23 (q, J = 7.0 Hz, 2 H), 4.69 (q, J = 6.8Hz, 1 H), 5.53 (s, 1 H), 6.54 (s, 1 H), 7.33 (s, 1 H).

### Reference Example 5 5-{2,4-dichloro-5-[(4-methoxybenzyl)oxy]phenoxy}-1,3-dimethyl-4-vinyl-1H-pyrazole

To a mixture of methyltriphenylphosphonium bromide (5.14 g) and tetrahydrofuran (30 mL) was added potassium tert-butoxide (1.62 g), and the mixture was stirred at room temperature for 2 hr. To the reaction mixture was added 5-{2,4-dichloro-5-[(4-methoxybenzyl)oxy]phenoxy}-1,3-dimethyl-1H-pyrazole-4-carbaldehyde (4.04 g) obtained in Reference Example 2, and the mixture was stirred at room temperature for 15 hr. To the reaction mixture was added dil. hydrochloric acid, and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 85:15 - 67:33, v/v) to give the title compound as a colorless oil (1.75 g, yield 44%).
¹H-NMR (300 MHz, CDCl₃)δ:2.34 (s, 3 H), 3.49 (s, 3 H), 3.81 (s, 3 H), 4.89 (s, 2 H), 5.01 (dd, J=11.7, 1.3Hz, 1 H), 5.23 (dd, J = 18.0, 1.4 Hz, 1 H), 6.18 - 6.30 (m, 2 H), 6.83 - 6.89 (m, 2 H), 7.15 - 7.21 (m, 2 H), 7.44 (s, 1 H).

### Reference Example 6 2,4-dichloro-5-[(1,3-dimethyl-4-vinyl-1H-pyrazol-5-yl)oxy]phenol

5-{2,4-Dichloro-5-[(4-methoxybenzyl)oxy]phenoxy}-1,3-dimethyl-4-vinyl-1H-pyrazole (1.74 g) obtained in Reference Example 5 was dissolved in 4N hydrochloric acid-ethyl acetate solution (20 mL), and the solution was stirred at room temperature for 2.5 hr. The reaction mixture was washed successively with aqueous sodium hydrogen carbonate solution and saturated brine. The organic layer was dried over anhydrous magnesium sulfate and filtered. The filtrate was concentrated, and the residue was subjected to silica gel column chromatography (hexane-ethyl acetate 85:15 - 60:40, v/v), and crystallized from hexane-ethyl acetate to give the title compound (424 mg, yield 34%) as colorless crystals.
¹H-NMR (300 MHz, CDCl₃)δ:2.21 (s, 3 H), 3.52 (s, 3 H), 5.12 (d, J = 11.9 Hz, 1 H), 5.30 (dd, J = 17.9, 0.9 Hz, 1 H), 6.22 (s, 1 H), 6.28 (dd, J=17.9, 11.7Hz, 1 H), 7.44 (s, 1 H), 9.30 (br s, 1 H).

### Reference Example 7 5-{2,4-dichloro-5-[(4-methoxybenzyl)oxy]phenoxy}-3-ethyl-1-methyl-1H-pyrazole-4-carbaldehyde

The title compound was obtained in the same manner as in Reference Example 2 and from 2,4-dichloro-5-[(4-methoxybenzyl)oxy]phenol obtained in Reference Example 1 and 5-chloro-3-ethyl-1-methyl-1H-pyrazole-4-carbaldehyde. ¹H-NMR (300 MHz, CDCl₃)δ:1.29 (t, J = 7.5 Hz, 3 H), 2.88 (q, J = 7.5 Hz, 2 H), 3.58 (s, 3 H), 3.82 (s, 3 H), 4.96 (s, 2 H), 6.44 (s, 1 H), 6.84 - 6.91 (m, 2 H), 7.17 - 7.24 (m, 2 H), 7.47 (s, 1 H), 9.43 (s, 1 H).

### Reference Example 8 5-{2,4-dichloro-5-[(4-methoxybenzyl)oxy]phenoxy}-1-methyl-3-(trifluoromethyl)-1H-pyrazole-4-carbaldehyde

The title compound was obtained in the same manner as in Reference Example 2 and from 2,4-dichloro-5-[(4-methoxybenzyl)oxy]phenol obtained in Reference Example 1 and 5-chloro-1-methyl-3-(trifluoromethyl)-1H-pyrazole-4-carbaldehyde.
¹H-NMR (300 MHz, CDCl₃)δ:3.73 (s, 3 H), 3.81 (s, 3 H), 5.00 (s, 2 H), 6.41 (s, 1 H), 6.84 - 6.90 (m, 2 H), 7.17 - 7.22 (m, 2 H), 7.47 (s, 1 H), 9.56 (s, 1 H).

### Reference Example 9 5-{2,4-dichloro-5-[(4-methoxybenzyl)oxy]phenoxy}-3-methyl-1-phenyl-1H-pyrazole-4-carbaldehyde

The title compound was obtained in the same manner as in Reference Example 2 and from 2,4-dichloro-5-[(4-methoxybenzyl)oxy]phenol obtained in Reference Example 1 and 5-chloro-3-methyl-1-phenyl-1H-pyrazole-4-carbaldehyde. ¹H-NMR (300 MHz, CDCl₃)δ:2.56 (s, 3 H), 3.81 (s, 3 H), 4.91 (s, 2 H), 6.43 (s, 1 H), 6.85 (d, J=8.7Hz, 2 H), 7.18 (d, J = 8.7 Hz, 2 H), 7.30 - 7.45 (m, 4 H), 7.57 (d, J = 7.2 Hz, 2 H), 9.49 (s, 1 H).

### Reference Example 10 2,4-dichloro-5-[(3-ethyl-1-methyl-4-vinyl-1H-pyrazol-5-yl)oxy]phenol

Trimethylsilylmethylmagnesium chloride (1M diethyl ether solution, 4.7 mL) was added dropwise to a solution of 5-{2,4-dichloro-5-[(4-methoxybenzyl)oxy]phenoxy}-3-ethyl-1-methyl-1H-pyrazole-4-carbaldehyde (1.57 g) obtained in Reference Example 7 in tetrahydrofuran (5 mL) with stirring at room temperature. The reaction mixture was stirred at room temperature for 15 hr, 30% sulfuric acid (10 mL) was added, and the mixture was stirred at 40°C for 3 hr. Water was added to the reaction mixture, and the mixture was extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 85:15 - 67:33, v/v) and crystallized from hexane-ethyl acetate to give the title compound (490 mg, yield 44%) as colorless crystals.
¹H-NMR (300 MHz, CDCl₃)δ:1.16 (t, J = 7.5 Hz, 3 H), 2.58 (q, J =7.3Hz, 2 H), 3.52 (s, 3 H), 5.11 (d, J = 11.7 Hz, 1 H), 5.29 (dd, J = 17.9,0.9 Hz, 1 H), 6.17 - 6.36 (m, 2 H), 7.45 (s, 1 H), 9.91 (s, 1 H).

### Reference Example 11 2,4-dichloro-5-{[1-methyl-3-(trifluoromethyl)-4-vinyl-1H-pyrazol-5-yl]oxy}phenol

The title compound was obtained in the same manner as in Reference Example 10 and from 5-{2,4-dichloro-5-[(4-methoxybenzyl)oxy]phenoxy}-1-methyl-3-(trifluoromethyl)-1H-pyrazole-4-carbaldehyde obtained in Reference Example 8. ¹H-NMR (300 MHz, CDCl₃)δ:3.71 (s, 3 H), 5.23 (dd, J = 11.7, 0.9 Hz, 1 H), 5.45 (d, J = 17.9 Hz, 1 H), 5.64 (s, 1 H), 6.33 (s, 1 H), 6.36-6.50 (m, 1 H), 7.46 (s, 1 H).

### Reference Example 12 2,4-dichloro-5-[(3-methyl-1-phenyl-4-vinyl-1H-pyrazol-5-yl)oxy]phenol

The title compound was obtained in the same manner as in Reference Example 10 and from 5-{2,4-dichloro-5-[(4-methoxybenzyl)oxy]phenoxy}-3-methyl-1-phenyl-1H-pyrazole-4-carbaldehyde obtained in Reference Example 9. ¹H-NMR (300 MHz, CDCl₃)δ:2.29 (s, 3 H), 5.22 (dd, J = 11.7, 0.9 Hz, 1 H), 5.42 (dd, J = 18.0, 1.0 Hz, 1 H), 6.29 - 6.42 (m, 2 H), 7.21 - 7.44 (m, 6 H), 8.76 (br s, 1 H).

### Reference Example 13 2-ethyl-5-methyl-2,4-dihydro-3H-pyrazol-3-one

A mixture of ethylhydrazine (20.00 g), methyl acetoacetate (38.67 g), and toluene (200 mL) was stirred at 100°C for 3 hr. After cooling to room temperature, the reaction mixture was concentrated. The residue was crystallized from hexane-ethyl acetate to give the title compound (38.44 g, yield 91%) as yellow crystals. ¹H-NMR (300 MHz, CDCl₃)δ:1.26 (t, J = 7.2 Hz, 3 H), 2.10 (s, 3 H), 3.19 (s, 2 H), 3.69 (q, J = 7.2 Hz, 2 H).

### Reference Example 14 5-chloro-1-ethyl-3-methyl-1H-pyrazole-4-carbaldehyde

Phosphorus oxychloride (109.5 g) was added dropwise to N,N-dimethylformamide (17.40 g) cooled to 0°C. To the reaction mixture was added 1-ethyl-5-hydroxy-3-methyl-1H-pyrazole (15.00 g) obtained in Reference Example 13, and the mixture was stirred at 80°C for 1 hr. The reaction mixture was cooled to room temperature, and poured into ice water (700 g), and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 98:2 - 75:25, v/v) to give the title compound as a colorless oil (9.15 g, yield 45%). ¹H-NMR (300 MHz, CDCl₃)δ:1.45 (t, J = 7.3 Hz, 3 H), 2.46 (s, 3 H), 4.16 (q, J=7.2Hz, 2 H), 9.87 (s, 1H).

### Reference Example 15 5-{2,4-dichloro-5-[(4-methoxybenzyl)oxy]phenoxy}-1-ethyl-3-methyl-1H-pyrazole-4-carbaldehyde

The title compound was obtained in the same manner as in Reference Example 2 and from 2,4-dichloro-5-[(4-methoxybenzyl)oxy]phenol obtained in Reference Example 1, and 5-chloro-1-ethyl-3-methyl-1H-pyrazole-4-carbaldehyde obtained in Reference Example 14.
¹H-NMR (300 MHz, CDCl₃)δ:1.35 (t, J = 7.3 Hz, 3 H), 2.48 (s, 3 H), 3.82 (s, 3 H), 3.93 (q, J = 7.2 Hz, 2 H), 4.96 (s, 2 H), 6.44 (s, 1 H), 6.84 - 6.89 (m, 2 H), 7.18 - 7.22 (m, 2 H), 7.47 (s, 1 H), 9.39 (s, 1H).

### Reference Example 16 2,4-dichloro-5-[(1-ethyl-3-methyl-4-vinyl-1H-pyrazol-5-yl)oxy]phenol

The title compound was obtained in the same manner as in Reference Example 10 and from 5-{2,4-dichloro-5-[(4-methoxybenzyl)oxy]phenoxy}-1-ethyl-3-methyl-1H-pyrazole-4-carbaldehyde obtained in Reference Example 15.
¹H-NMR (300 MHz, CDCl₃)δ:1.23 (t, J = 7.3 Hz, 3 H), 2.18 (s, 3 H), 3.82 (q, J = 7.2 Hz, 2 H), 5.13 (dd, J = 11.7, 0.9 Hz, 1 H), 5.29 (dd, J = 17.9, 0.9 Hz, 1 H), 6.18 (s, 1 H), 6.26 (dd, J=18.0, 11.6Hz, 1 H), 7.44 (s, 1 H), 10.76 (s, 1 H).

### Reference Example 17 5-{2,4-dichloro-5-[(4-methoxybenzyl)oxy]phenoxy}-1-methyl-3-phenyl-1H-pyrazole-4-carbaldehyde

The title compound was obtained in the same manner as in Reference Example 2 and from 2,4-dichloro-5-[(4-methoxybenzyl)oxy]phenol obtained in Reference Example 1 and 5-chloro-1-methyl-3-phenyl-1H-pyrazole-4-carbaldehyde. ¹H-NMR (300 MHz, DMSO-d₆)δ:3.64 (s, 3 H), 3.75 (s, 3 H), 5.06 (s, 2 H), 6.76 - 6.83 (m, 3 H), 7.16 - 7.24 (m, 2 H), 7.47 - 7.56 (m, 3 H), 7.75-7.83 (m, 3 H), 9.48 (s, 1 H).

### Reference Example 18 2,4-dichloro-5-[(1-methyl-3-phenyl-4-vinyl-1H-pyrazol-5-yl)oxy]phenol

The title compound was obtained in the same manner as in Reference Example 10 and from 5-{2,4-dichloro-5-[(4-methoxybenzyl)oxy]phenoxy}₋1-methyl-3-phenyl-1H-pyrazole-4-carbaldehyde obtained in Reference Example 17. ¹H-NMR (300 MHz, CDCl₃)δ:3.69 (s, 3 H), 5.09 (dd, J = 11.5, 1.3 Hz, 1 H), 5.33 (dd, J = 17.9, 1.3 Hz, 1 H), 6.34 - 6.47 (m, 2 H), 7.34 - 7.46 (m, 4 H), 7.48 - 7.56 (m, 2 H).

### Reference Example 19 ethyl 5-chloro-4-formyl-1-methyl-1H-pyrazole-3-carboxylate

Phosphorus oxychloride (98.8 mL) was added dropwise to N,N-dimethylformamide (14.18 g) cooled to 0°C. To the reaction mixture was added ethyl 5-hydroxy-1-methyl-1H-pyrazole-3-carboxylate (30.00 g), and the mixture was stirred at 100°C for 18 hr. The reaction mixture was cooled to room temperature, and poured into a mixture of sodium hydrogen carbonate (360 g) and water (1000 mL) by small portions. The resulting crystals were collected by filtration, and dissolved in ethyl acetate, and the solution was washed with saturated brine. The organic layer was dried over anhydrous magnesium sulfate and filtered. The filtrate was concentrated, and the residue was crystallized from hexane-ethyl acetate to give the title compound (33.78 g, yield 88%) as pale-brown crystals.
¹H-NMR (300 MHz, CDCl₃)δ:1.44 (t, J = 7.2 Hz, 3 H), 3.96 (s, 3 H), 4.48 (q, J = 7.2 Hz, 2 H), 10.43 (s, 1 H).

### Reference Example 20 ethyl 5-{2,4-dichloro-5-[(4-methoxybenzyl)oxy]phenoxy}-4-formyl-1-methyl-1H-pyrazole-3-carboxylate

Potassium carbonate (5.81 g) was added to a solution of 2,4-dichloro-5-[(4-methoxybenzyl)oxy]phenol (9.67 g) obtained in Reference Example 1 and ethyl 5-chloro-4-formyl-1-methyl-1H-pyrazole-3-carboxylate (7.00 g) obtained in Reference Example 19 in N,N-dimethylformamide (40 mL) with stirring. The reaction mixture was stirred under a nitrogen atmosphere at 100°C for 15 hr. The reaction mixture was cooled to room temperature, water was added, and the mixture was extracted with ethyl acetate. The organic layer was washed with saturated brine, dried over anhydrous magnesium sulfate and filtered. The filtrate was concentrated, and the residue was crystallized from hexane-ethyl acetate to give the title compound (10.70 g, yield 69%) as pale-brown crystals.
¹H-NMR (300 MHz, CDCl₃)δ:1.47 (t, J = 7.2 Hz, 3 H), 3.80 (s, 3 H), 3.81 (s, 3 H), 4.52 (q, J=7.0Hz, 2 H), 4.94 (s, 2 H), 6.35 (s, 1 H), 6.83 - 6.90 (m, 2 H), 7.17 - 7.25 (m, 2 H), 7.45 (s, 1 H), 10.15 (s, 1 H).

### Reference Example 21 5-{2,4-dichloro-5-[(4-methoxybenzyl)oxy]phenoxy}-4-formyl-1-methyl-1H-pyrazole-3-carboxylic acid

Ethyl 5-{2,4-dichloro-5-[(4-methoxybenzyl)oxy]phenoxy}-4-formyl-1-methyl-1H-pyrazole-3-carboxylate (9.70 g) obtained in Reference Example 20 was dissolved in a mixed solvent of tetrahydrofuran (300 mL) and ethanol (100 mL), 1N aqueous sodium hydroxide solution (100 mL) was added, and the mixture was stirred at room temperature for 12 hr. The reaction mixture was concentrated to a half liquid amount, and neutralized with 0.1N hydrochloric acid (1000 mL). The resulting crystals were collected by filtration to give the title compound as pale-brown crystals (8.36 g, yield 92%).
¹H-NMR (300 MHz, DMSO-d₆)δ:3.75 (s, 3 H), 3.78 (s, 3 H), 5.02 (s, 2 H), 6.75 (s, 1 H), 6.85 - 6.92 (m, 2 H), 7.17 - 7.25 (m, 2 H), 7.73 (s, 1 H), 9.96 (s, 1 H), 13.70 (s, 1 H).

### Reference Example 22 5-{2,4-dichloro-5-[(4-methoxybenzyl)oxy]phenoxy}-4-formyl-N-methoxy-1,N-dimethyl-1H-pyrazole-3-carboxamide

To a mixture of N,O-dimethylhydroxylamine hydrochloride (2.01 g) and N,N-dimethylformamide (35 mL) was added triethylamine (2.87 mL), and the mixture was stirred at room temperature for 10 min. To the reaction mixture were added 5-{2,4-dichloro-5-[(4-methoxybenzyl)oxy]phenoxy}-4-formyl-1-methyl-1H-pyrazole-3-carboxylic acid (8.08 g) obtained in Reference Example 21, N-ethyl-N'-(3-dimethylaminopropyl)carbodiimide hydrochloride (3.95 g), and 1-hydroxybenzotriazole monohydrate (3.15 g), and the mixture was stirred at room temperature for 15 hr. Water was added to the reaction mixture, and the mixture was extracted with ethyl acetate. The organic layer was washed successively with aqueous potassium carbonate solution and saturated brine, and dried over anhydrous magnesium sulfate. After filtration, the filtrate was concentrated and the residue was subjected to silica gel column chromatography (ethyl acetate), and crystallized from hexane-ethyl acetate to give the title compound (7.22 g, yield 82%) as colorless crystals.
¹H-NMR (300 MHz, CDCl₃)δ:3.43 (s, 3 H), 3.67 - 3.77 (m, 6 H), 3.81 (s, 3 H), 4.97 (s, 2 H), 6.50 (s, 1 H), 6.83 - 6.96 (m, 2 H), 7.18 - 7.29 (m, 2 H), 7.46 (s, 1 H), 9.61 (s, 1 H).

### Reference Example 23 5-{2,4-dichloro-5-[(4-methoxybenzyl)oxy]phenoxy}-N-methoxy-1,N-dimethyl-4-vinyl-1H-pyrazole-3-carboxamide

Trimethylsilylmethylmagnesium chloride (1M diethyl ether solution, 1.37 mL) was added dropwise to a solution of 5-{2,4-dichloro-5-[(4-methoxybenzyl)oxy]phenoxy}-4-formyl-N-methoxy-1,N-dimethyl-1H-pyrazole-3-carboxamide (503 mg) obtained in Reference Example 22 in tetrahydrofuran (10 mL) with stirring at -78°C. Cooling of the reaction mixture was stopped, and the mixture was stirred at room temperature for 1 hr. 1N Sulfuric acid (10 mL) was added, and the mixture was stirred at room temperature for 3 hr. Water was added to the reaction mixture, and the mixture was extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 75:25 - 40:60, v/v) to give the title compound as a colorless oil (380 mg, yield 76%).
¹H-NMR (300 MHz, CDCl₃)δ:3.42 (s, 3 H), 3.56 (s, 3 H), 3.78 (s, 3 H), 3.80 (s, 3 H), 4.91 (s, 2 H), 5.09 (dd, J = 11.5, 1.3 Hz, 1 H), 5.34 (dd, J = 18.0, 1.2 Hz, 1 H), 6.28 (s, 1 H), 6.55 (dd, J = 18.0, 11.6 Hz, 1 H), 6.82 - 6.91 (m, 2 H), 7.14 - 7.23 (m, 2 H), 7.45 (s, 1 H).

### Reference Example 24 5-{2,4-dichloro-5-[(4-methoxybenzyl)oxy]phenoxy}-1-methyl-4-vinyl-1H-pyrazole-3-carbaldehyde

Diisobutylaluminum hydride (1.5M toluene solution, 2.44 mL) was added dropwise to a solution of 5-{2,4-dichloro-5-[(4-methoxybenzyl)oxy]phenoxy}-N-methoxy-1,N-dimethyl-4-vinyl-1H-pyrazole-3-carboxamide (1.50 g) obtained in Reference Example 23 in tetrahydrofuran (10 mL) with stirring at 0°C. The reaction mixture was stirred at 0°C for 1 hr, magnesium sulfate decahydrate was gradually added, and the mixture was stirred at room temperature for 2 hr. The precipitate was removed by filtration, and the filtrate was concentrated. The residue was subjected to silica gel column chromatography (hexane-ethyl acetate 91:9 - 75:25, v/v) to give the title compound as a colorless oil (1.20 g, yield 91%).
¹H-NMR (300 MHz, CDCl₃)δ:3.62 (s, 3 H), 3.81 (s, 3 H), 4.91 (s, 2 H), 5.22 (dd, J = 11.7, 1.3 Hz, 1 H), 5.55 (dd, J = 18.1, 1.3 Hz, 1 H), 6.15 (s, 1 H), 6.78 - 6.92 (m, 3 H), 7.09 - 7.16 (m, 2 H), 7.47 (s, 1 H), 9.97 (s, 1 H).

### Reference Example 25 1-(5-{2,4-dichloro-5-[(4-methoxybenzyl)oxy]phenoxy}-1-methyl-4-vinyl-1H-pyrazol-3-yl)-3-buten-1-ol

Allylmagnesium chloride (1M tetrahydrofuran solution, 5.49 mL) was added dropwise to a solution of 5-{2,4-dichloro-5-[(4-methoxybenzyl)oxy]phenoxy}-1-methyl-4-vinyl-1H-pyrazole-3-carbaldehyde (1.19 g) obtained in Reference Example 24 in tetrahydrofuran (10 mL) while stirring at 0°C. The reaction mixture was stirred at 0°C for 3 hr, aqueous ammonium chloride solution was added, and the mixture was extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 67:33 - 33:67, v/v) and crystallized from hexane-diisopropyl ether to give the title compound (847 mg, yield 65%) as colorless crystals.
¹H-NMR (300 MHz, CDCl₃)δ:2.50 - 2.77 (m, 3 H), 3.51 (s, 3 H), 3.81 (s, 3 H), 4.82 - 4.96 (m, 3 H), 5.06 (dd, J = 11.7, 1.3 Hz, 1 H), 5.12 - 5.35 (m, 3 H), 5.81 - 6.01 (m, 1 H), 6.23 (s, 1 H), 6.37 (dd, J = 17.9, 11.5 Hz, 1 H), 6.81 - 6.91 (m, 2 H), 7. 13 - 7.21 (m, 2 H), 7.45 (s, 1 H).

### Reference Example 26 3-{2,4-dichloro-5-[(4-methoxybenzyl)oxy]phenoxy}-2-methyl-6,7-dihydro-2H-indazol-7-ol

To a solution of 1-(5-{2,4-dichloro-5-[(4-methoxybenzyl)oxy]phenoxy}-1-methyl-4-vinyl-1H-pyrazol-3-yl)-3-buten-1-ol (771 mg) obtained in Reference Example 25 in toluene (32 mL) was added Hoveyda-Grubbs catalyst 2^{nd} generation ([301224-40-8], 51 mg). The mixture was stirred under a nitrogen atmosphere at 60°C for 6 hr, and concentrated. The residue was subjected to silica gel column chromatography (hexane-ethyl acetate 60:40 - 1:99, v/v) to give the title compound as a pale-brown oil (690 mg, yield 95%).
¹H-NMR (300 MHz, CDCl₃)δ:2.07 - 2.13 (m, 1 H), 2.58 - 2.66 (m, 2 H), 3.67 (s, 3 H), 3.81 (s, 3 H), 4.89 (q, J = 5.3 Hz, 1 H), 4.96 (s, 2 H), 5.51 - 5.65 (m, 1 H), 5.72 - 5.82 (m, 1 H), 6.60 (s, 1 H), 6.84 - 6.93 (m, 2 H), 7.21 - 7.28 (m, 2 H), 7.46 (s, 1 H).

### Reference Example 27 2,4-dichloro-5-[(2-methyl-2H-indazol-3-yl)oxy]phenol

To a solution of 3-{2,4-dichloro-5-[(4-methoxybenzyl)oxy]phenoxy}-2-methyl-6,7-dihydro-2H-indazol-7-ol (284 mg) obtained in Reference Example 26 in tetrahydrofuran (15 mL) was added 30% sulfuric acid (5 mL), and the mixture was stirred at room temperature for 1 hr and at 50°C for 6 hr. The reaction mixture was cooled to room temperature, neutralized with aqueous sodium hydrogen carbonate solution, and extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 75:25 - 40:60, v/v) and crystallized from hexane-ethyl acetate to give the title compound (117 mg, yield 60%) as colorless crystals.
¹H-NMR (300 MHz, CDCl₃)δ:3.89 (s, 3 H), 6.36 (s, 1 H), 6.90 - 6.98 (m, 1 H), 7.11 - 7.24 (m, 2 H), 7.39 (d, J = 8.9 Hz, 1 H), 7.50 (s, 1 H), 8.19 (s, 1 H).

### Reference Example 28 3-{2,4-dichloro-5-[(4-methoxybenzyl)oxy]phenoxy}-2-methyl-4,5,6,7-tetrahydro-2H-indazol-7-ol

3-{2,4-Dichloro-5-[(4-methoxybenzyl)oxy]phenoxy}-2-methyl-6,7-dihydro-2H-indazol-7-ol (470 mg) obtained in Reference Example 26 was dissolved in a mixed solvent of ethyl acetate (5 mL) and ethanol (5 mL), platinum oxide (4.8 mg) was added, and the mixture was stirred at room temperature under a hydrogen atmosphere (1 atm) for 2 hr. The catalyst was removed by filtration, and the filtrate was concentrated. The residue was subjected to silica gel column chromatography (hexane-ethyl acetate 50:50 - 1:99, v/v) to give the title compound as a colorless oil (380 mg, yield 81%).
¹H-NMR (300 MHz, CDCl₃)δ:1.71 - 2.04 (m, 5 H), 2.25 - 2.37 (m, 1 H), 3.64 (s, 3 H), 3.81 (s, 3 H), 4.75-4.84 (m, 1 H), 4.97 (s, 2 H), 6.45 (s, 1 H), 6.84 - 6.92 (m, 2 H), 7.20 - 7.27 (m, 2 H), 7.44 (s, 1H).

### Reference Example 29 2,4-dichloro-5-[(2-methyl-4,5,6,7-tetrahydro-2H-indazol-3-yl)oxy]phenol

3-{2,4-Dichloro-5-[(4-methoxybenzyl)oxy]phenoxy}-2-methyl-4,5,6,7-tetrahydro-2H-indazol-7-ol (100 mg) obtained in Reference Example 28 was dissolved in trifluoroacetic acid (2 mL), triethylsilane (0.292 mL) was added and the mixture was stirred at 60°C for 14 hr. The reaction mixture was cooled to room temperature, neutralized with aqueous sodium hydrogen carbonate solution, and extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 75:25 - 33:67, v/v), and crystallized from diisopropyl ether to give the title compound (46 mg, yield 48%) as colorless crystals.
¹H-NMR (300 MHz, CDCl₃)δ:1.59 - 1.82 (m, 4 H), 2.19 (t, J = 6.1 Hz, 2 H), 2.54 (t, J = 6.2 Hz, 2 H), 3.55 (s, 3 H), 6.36 (s, 1 H), 7.41 (s, 1 H), 9.40 (br s, 1H).

### Reference Example 30 2,4-dichloro-5-[(2-methyl-4,5-dihydro-2H-indazol-3-yl)oxy]phenol

To a solution of 3-{2,4-dichloro-5-[(4-methoxybenzyl)oxy]phenoxy}-2-methyl-4,5,6,7-tetrahydro-2H-indazol-7-ol (170 mg) obtained in Reference Example 28 in tetrahydrofuran (5 mL) was added 30% sulfuric acid (5 mL), and the mixture was stirred under a nitrogen atmosphere at 80°C for 5 days. The reaction mixture was cooled to room temperature, neutralized with aqueous sodium hydrogen carbonate solution, and extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 75:25 - 25:75, v/v) to give the title compound as a colorless oil (33 mg, yield 28%).
¹H-NMR (300 MHz, CDCl₃)δ:2.23 - 2.44 (m, 4 H), 3.53 (s, 3 H), 5.96 - 6.05 (m, 1 H), 6.29 - 6.37 (m, 2 H), 7.42 (s, 1 H), 10.46 (br s, 1 H).

### Reference Example 31 1-(5-{2,4-dichloro-5-[(4-methoxybenzyl)oxy]phenoxy}-1-methyl-4-vinyl-1H-pyrazol-3-yl)-4-penten-1-ol

The title compound was obtained in the same manner as in Reference Example 25 and from 5-{2,4-dichloro-5-[(4-methoxybenzyl)oxy]phenoxy}-1-methyl-4-vinyl-1H-pyrazole-3-carbaldehyde obtained in Reference Example 24 and 3-butenylmagnesium bromide.
¹H-NMR (300 MHz, CDCl₃)δ:1.88 - 2.01 (m, 2 H), 2.16 - 2.31 (m, 2 H), 2.47 (d, J=6.0Hz, 1 H), 3.50 (s, 3 H), 3.81 (s, 3 H), 4.82 - 4.91 (m, 3 H), 4.96 - 5.13 (m, 3 H), 5.25 (dd, J = 17.9, 1.3 Hz, 1 H), 5.79 - 5.96 (m, 1 H), 6.22 (s, 1 H), 6.34 (dd, J = 18.0, 11.6 Hz, 1 H), 6.82 - 6.90 (m, 2 H), 7.13 - 7.19 (m, 2 H), 7.45 (s, 1H).

### Reference Example 32 3-{2,4-dichloro-5-[(4-methoxybenzyl)oxy]phenoxy}-2-methyl-2,6,7,8-tetrahydrocyclohepta[c]pyrazol-8-ol

The title compound was obtained in the same manner as in Reference Example 26 and from 1-(5-{2,4-dichloro-5-[(4-methoxybenzyl)oxy]phenoxy}-1-methyl-4-vinyl-1H-pyrazol-3-yl)-4-penten-1-ol obtained in Reference Example 31.
¹H-NMR (300 MHz, CDCl₃)δ:2.09 (q, J = 6.0 Hz, 2 H), 2.35 - 2.49 (m, 1 H), 2.56 - 2.75 (m, 2 H), 3.52 (s, 3 H), 3.81 (s, 3 H), 4.90 - 4.98 (m, 3 H), 5.55 - 5.64 (m, 1 H), 5.76 - 5.83 (m, 1 H), 6.31 (s, 1 H), 6.83 - 6.90 (m, 2 H), 7.16 - 7.22 (m, 2 H), 7.44 (s, 1 H).

### Reference Example 33 2,4-dichloro-5-[(2-methyl-2,4,5,6,7,8-hexahydrocyclohepta[c]pyrazol-3-yl)oxy]phenol

3-{2,4-Dichloro-5-[(4-methoxybenzyl)oxy]phenoxy}-2-methyl-2,6,7,8-tetrahydrocyclohepta[c]pyrazol-8-ol (350 mg) obtained in Reference Example 32 was dissolved in trifluoroacetic acid (2 mL), triethylsilane (0.727 mL) was added and the mixture was stirred at 50°C for 8 hr. The reaction mixture was cooled to room temperature, neutralized with aqueous sodium hydrogen carbonate solution, and extracted with ethyl acetate. The organic layer was washed with saturated brine, and dried over anhydrous magnesium sulfate. After filtration, the filtrate was concentrated and the residue was dissolved in a mixed solvent of ethyl acetate (10 mL) and ethanol (10 mL), platinum oxide (2.6 mg) was added, and the mixture was stirred under a hydrogen atmosphere (1 atm) at room temperature for 10 hr. The catalyst was removed by filtration, and the filtrate was concentrated. The residue was crystallized from hexane-ethyl acetate to give the title compound (114 mg, yield 46%) as colorless crystals.
¹H-NMR (300 MHz, CDCl₃)δ:1.48 - 1.66 (m, 4 H), 1.76 - 1.86 (m, 2 H), 2.23 - 2.30 (m, 2 H), 2.60 - 2.67 (m, 2 H), 3.47 (s, 3 H), 6.24 (s, 1 H), 7.42 (s, 1 H), 10.54 (br s, 1 H)

### Reference Example 34 5-chloro-N-methoxy-N,1-dimethyl-1H-pyrazole-4-carboxamide

The title compound was obtained in the same manner as in Reference Example 22 and from 5-chloro-1-methyl-1H-pyrazole-4-carboxylic acid.
¹H-NMR (300 MHz, CDCl₃)δ:3.33 (s, 3 H), 3.66 (s, 3 H), 3.88 (s, 3 H), 7.92 (s, 1 H).

### Reference Example 35 5-chloro-1-methyl-1H-pyrazole-4-carbaldehyde

The title compound was obtained in the same manner as in Reference Example 24 and from 5-chloro-N-methoxy-N,1-dimethyl-1H-pyrazole-4-carboxamide obtained in Reference Example 34.
¹H-NMR (300 MHz, CDCl₃)δ:3.90 (s, 3 H), 7.96 (s, 1 H), 9.83 (s, 1 H).

### Reference Example 36 5-{2,4-dichloro-5-[(4-methoxybenzyl)oxy]phenoxy}-1-methyl-1H-pyrazole-4-carbaldehyde

The title compound was obtained in the same manner as in Reference Example 2 and from 2,4-dichloro-5-[(4-methoxybenzyl)oxy]phenol obtained in Reference Example 1, and 5-chloro-1-methyl-1H-pyrazole-4-carbaldehyde obtained in Reference Example 35.
¹H-NMR (300 MHz, CDCl₃)δ:3.68 (s, 3 H), 3.82 (s, 3 H), 4.98 (s, 2 H), 6.50 (s, 1 H), 6.88 (d, J = 8.7 Hz, 2 H), 7.22 (d, J = 8.7 Hz, 2 H), 7.48 (s, 1 H), 7.89 (s, 1 H), 9.28 (s, 1H).

### Reference Example 37 5-{2,4-dichloro-5-[(4-methoxybenzyl)oxy]phenoxy}-1-methyl-4-vinyl-1H-pyrazole

Trimethylsilylmethylmagnesium chloride (1M diethyl ether solution, 3.4 mL) was added dropwise to a solution of 5-{2,4-dichloro-5-[(4-methoxybenzyl)oxy]phenoxy}-1-methyl-1H-pyrazole-4-carbaldehyde (1.05 g) obtained in Reference Example 36 in tetrahydrofuran (10 mL) with stirring at room temperature. The reaction mixture was stirred at room temperature for 15 hr and cooled to 0°C. 1N Sulfuric acid (10 mL) was added to the reaction mixture, and the mixture was stirred at 0°C for 1 hr. Water was added to the reaction mixture, and the mixture was extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 85:15 - 67:33, v/v) and crystallized from hexane-ethyl acetate to give the title compound (428 mg, yield 41%) as colorless crystals.
¹H-NMR (300 MHz, CDCl₃)δ:3.54 (s, 3 H), 3.81 (s, 3 H), 4.89 (s, 2 H), 5.01 (dd, J = 11.3, 1.3 Hz, 1 H), 5.34 (dd, J = 17.8, 1.2 Hz, 1 H), 6.18 (dd, J=17.8, 11.2 Hz, 1 H), 6.27 (s, 1 H), 6.86 (d, J = 8.7 Hz, 2 H), 7.18 (d, J=8.7Hz, 2 H), 7.44 (s, 1 H), 7.60 (s, 1 H).

### Reference Example 38 2,4-dichloro-5-[(4-ethyl-1-methyl-1H-pyrazol-5-yl)oxy]phenol

5-{2,4-Dichloro-5-[(4-methoxybenzyl)oxy]phenoxy}-1-methyl-4-vinyl-1H-pyrazole (920 mg) obtained in Reference Example 37 was dissolved in a mixed solvent of ethyl acetate (5 mL) and ethanol (5 mL), platinum oxide (5.2 mg) was added, and the mixture was stirred under a hydrogen atmosphere (1 atm) at room temperature for 15 hr. The catalyst was removed by filtration, and the filtrate was concentrated. The residue was dissolved in tetrahydrofuran (10 mL), 30% sulfuric acid (10 mL) was added, and the mixture was stirred at 50°C for 2 hr. The reaction mixture was cooled to room temperature, neutralized with aqueous sodium hydrogen carbonate solution, and extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 85:15 - 60:40, v/v), and crystallized from hexane-ethyl acetate to give the title compound (171 mg, yield 26%) as colorless crystals.
¹H-NMR (300 MHz, CDCl₃)δ:1.10 (t, J = 7.5 Hz, 3 H), 2.26 (q, J = 7.6 Hz, 2 H), 3.56 (s, 3 H), 6.21 (s, 1 H), 7.28 (s, 1 H), 7.43 (s, 1 H), 9.68 (br s, 1 H).

### Reference Example 39 5-(methoxymethyl)-2-methyl-2,4-dihydro-3H-pyrazol-3-one

To a solution (110 mL) of methyl 4-methoxyacetoacetate (34.8 g) in toluene was added dropwise a solution of methylhydrazine (11.0 g) in toluene (35 mL) at 0°C over 20 min, and the mixture was stirred at 100°C for 1.5 hr. After cooling, the reaction mixture was concentrated. The obtained residue was crystallized from diisopropyl ether-hexane to give the title compound (32.0 g, yield 95%) as orange crystals. The obtained crystals were recrystallized from hexane-ethyl acetate to give the title compound as colorless crystals.
¹H-NMR (300 MHz, CDCl₃)δ:3.29 (s, 2 H), 3.31 (s, 3 H), 3.39 (s, 3 H), 4.17 (s, 2 H).

### Reference Example 40 5-chloro-3-(methoxymethyl)-1-methyl-1H-pyrazole-4-carbaldehyde

Phosphorus oxychloride (201.1 g) was added dropwise over 30 min to N,N-dimethylformamide (31.9 g) cooled to 0°C. To the reaction mixture was added 5-(methoxymethyl)-2-methyl-2,4-dihydro-3H-pyrazol-3-one (31.0 g) obtained in Reference Example 39, and the mixture was stirred at 80°C for 3 hr. The reaction mixture was cooled to room temperature, and poured into ice water, and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 10:1 - 1:1, v/v) to give the title compound as a colorless oil (23.5 g, yield 57%).
¹H-NMR (300 MHz, CDCl₃)δ:3.48 (s, 3 H), 3.88 (s, 3 H), 4.69 (s, 2 H), 9.90 (s, 1 H).

### Reference Example 41 5-{2,4-dichloro-5-[(4-methoxybenzyl)oxy]phenoxy}-3-(methoxymethyl)-1-methyl-1H-pyrazole-4-carbaldehyde

Potassium carbonate (1.14 g) was added to a solution of 2,4-dichloro-5-[(4-methoxybenzyl)oxy]phenol (2.0 g) obtained in Reference Example 1 and 5-chloro-3-(methoxymethyl)-1-methyl-1H-pyrazole-4-carbaldehyde (1.2 g) obtained in Reference Example 40 in N,N-dimethylformamide (15 mL) with stirring. The mixture was stirred under a nitrogen atmosphere at 120°C for 4.5 hr. The reaction mixture was cooled to room temperature, and poured into ice water, and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 10:1 - 1:2, v/v) to give the title compound (2.18 g, yield 76%) as pale-pink crystals.
¹H-NMR (300 MHz, CDCl₃)δ:3.50 (s, 3 H), 3.67 (s, 3 H), 3.82 (s, 3 H), 4.68 (s, 2 H), 4.96 (s, 2 H), 6.49 (s, 1 H), 6.88 (d, J = 8.7 Hz, 2 H), 7.22 (d, J = 8.7 Hz, 2 H), 7.47 (s, 1 H), 9.42 (s, 1 H).

### Reference Example 42 2,4-dichloro-5-{[3-(methoxymethyl)-1-methyl-4-vinyl-1H-pyrazol-5-yl]oxy}phenol

Trimethylsilylmethylmagnesium chloride (1M diethyl ether solution, 5.5 mL) was added dropwise to a solution of 5-{2,4-dichloro-5-[(4-methoxybenzyl)oxy]phenoxy}-3-(methoxymethyl)-1-methyl-1H-pyrazole-4-carbaldehyde (1.57 g) obtained in Reference Example 41 in tetrahydrofuran (15 mL) with stirring at 0°C, and the mixture was stirred for 1.5 hr. Trimethylsilylmethylmagnesium chloride (1M diethyl ether solution, 5.5 mL) was added dropwise to the reaction mixture at 0°C, and the mixture was stirred for 2 hr. 30% Sulfuric acid (15 mL) was added to the reaction mixture, and the mixture was stirred at 50°C for 5 hr. Water was added to the reaction mixture, and the mixture was extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 10:1 - 1:2, v/v) and crystallized from hexane-diethyl ether to give the title compound (398 mg, yield 25%) as pale-orange crystals.
¹H-NMR (300 MHz, CDCl₃)δ:3.33 (s, 3 H), 3.58 (s, 3 H), 4.39 (s, 2 H), 5.14 (dd, J = 11.5, 1.1 Hz, 1 H), 5.37 (dd, J = 17.9, 1.1 Hz, 1 H), 6.27 (s, 1 H), 6.35 (dd, J = 17.9, 11.5 Hz, 1 H), 7.44 (s, 1 H), 8.32 (s, 1 H).

### Reference Example 43 2-methyl-5-propyl-2,4-dihydro-3H-pyrazol-3-one

The title compound was obtained in the same manner as in Reference Example 13 and from ethyl 3-oxohexanoate and methylhydrazine.
¹H-NMR (300 MHz, CDCl₃)δ:0.98 (t, J = 7.4 Hz, 3 H), 1.55 - 1.68 (m, 2 H), 2.37 (t, J = 7.5 Hz, 2 H), 3.16 (s, 2 H), 3.28 (s, 3 H).

### Reference Example 44 5-chloro-1-methyl-3-propyl-1H-pyrazole-4-carbaldehyde

The title compound was obtained in the same manner as in Reference Example 14 and from 2-methyl-5-propyl-2,4-dihydro-3H-pyrazol-3-one obtained in Reference Example 43.
¹H-NMR (300 MHz, CDCl₃)δ:0.97 (t, J = 7.5 Hz, 3 H (m, 2 H), 2.78-2.84 (m, 2 H), 3.83 (s, 3 H), 9.84 (s, 1 H).

### Reference Example 45 5-{2,4-dichloro-5-[(4-methoxybenzyl)oxy]phenoxy}-1-methyl-3-propyl-1H-pyrazole-4-carbaldehyde

The title compound was obtained in the same manner as in Reference Example 2 and from 2,4-dichloro-5-[(4-methoxybenzyl)oxy]phenol obtained in Reference Example 1 and 5-chloro-1-methyl-3-propyl-1H-pyrazole-4-carbaldehyde obtained in Reference Example 44.
¹H-NMR (300 MHz, CDCl₃)δ:1.01 (d, J = 7.4 Hz, 3 H), 1.66 - 1.79 (m, 2 H), 2.79 - 2.85 (m, 2 H), 3.57 (s, 3 H), 3.81 (s, 3 H), 4.95 (s, 2 H), 6.42 (s, 1 H), 6.87 (d, J = 9.0 Hz, 2 H), 7.20 (d, J=9.0Hz, 2 H), 7.46 (s, 1 H), 9.43 (s, 1 H).

### Reference Example 46 2,4-dichloro-5-[(1-methyl-3-propyl-4-vinyl-1H-pyrazol-5-yl)oxy]phenol

The title compound was obtained in the same manner as in Reference Example 10 and from 5-{2,4-dichloro-5-[(4-methoxybenzyl)oxy]phenoxy}-1-methyl-3-propyl-1H-pyrazole-4-carbaldehyde obtained in Reference Example 45.
¹H-NMR (300 MHz, CDCl₃)δ:0.94 (t, J = 7.3 Hz, 3 H), 1.41 - 1.76 (m, 2 H), 2.54 (t, J = 7.3 Hz, 2 H), 3.54 (s, 3 H), 5.08 (d, J = 11.7 Hz, 1 H), 5.27 (dd, J = 17.9, 1.1 Hz, 1 H), 6.28 (dd, J = 17.9, 11.7 Hz, 1 H), 6.24 (s, 1 H), 7.44 (s, 1 H), 8.74 (br s, 1 H).

### Reference Example 47 5-isopropyl-2-methyl-2,4-dihydro-3H-pyrazol-3-one

The title compound was obtained in the same manner as in Reference Example 13 and from ethyl 4-methyl-3-oxopentanoate and methylhydrazine.
¹H-NMR (300 MHz, CDCl₃)δ:1.18 (d, J = 7.0 Hz, 6 H), 2.43 - 2.83 (m, 1 H), 3.17 (s, 2 H), 3.28 (s, 3 H).

### Reference Example 48 5-chloro-3-isopropyl-1-methyl-1H-pyrazole-4-carbaldehyde

The title compound was obtained in the same manner as in Reference Example 14 and from 5-isopropyl-2-methyl-2,4-dihydro-3H-pyrazol-3-one obtained in Reference Example 47.
¹H-NMR (300 MHz, CDCl₃)δ:1.28 (d, J = 6.8 Hz, 6 H), 3.00 - 3.50 (m, 1 H), 3.83 (s, 3 H), 9.87 (s, 1 H).

### Reference Example 49 5-{2,4-dichloro-5-[(4-methoxybenzyl)oxy]phenoxy}-3-isopropyl-1-methyl-1H-pyrazole-4-carbaldehyde

The title compound was obtained in the same manner as in Reference Example 2 and from 2,4-dichloro-5-[(4-methoxybenzyl)oxy]phenol obtained in Reference Example 1, and 5-chloro-3-isopropyl-1-methyl-1H-pyrazole-4-carbaldehyde obtained in Reference Example 48.
¹H-NMR (300 MHz, CDCl₃)δ:1.33 (d, J = 7.0 Hz, 6 H), 3.29 - 3.47 (m, 1 H), 3.55 (s, 3 H), 3.82 (s, 3 H), 4.96 (s, 2 H), 6.40 (s, 1 H), 6.87 (d, J = 8.9 Hz, 2 H), 7.19 (d, J = 8.7 Hz, 2 H), 7.47 (s, 1 H), 9.48 (s, 1 H).

### Reference Example 50 5-(2,4-dichloro-5-hydroxyphenoxy)-3-isopropyl-1-methyl-1H-pyrazole-4-carbaldehyde

To a solution of 5-{2,4-dichloro-5-[(4-methoxybenzyl)oxy]phenoxy}-3-isopropyl-1-methyl-1H-pyrazole-4-carbaldehyde obtained in Reference Example 49 in ethyl acetate (125 mL) was added 4N hydrochloric acid-ethyl acetate solution (125 mL), and the mixture was stirred at 50°C for 12 hr. After cooling to room temperature, water was added to the reaction mixture, and the mixture was diluted with ethyl acetate. The organic layer was washed with water and saturated brine, dried over anhydrous magnesium sulfate and filtered, and the filtrate was concentrated. The obtained residue was subjected to silica gel column chromatography (hexane-ethyl acetate 3:1 - 2:1, v/v) and crystallized from hexane-ethyl acetate to give the title compound (4.75 g, yield 60%) as white crystals.
¹H-NMR (300 MHz, CDCl₃)δ:1.30 (d, J = 7.0 Hz, 6 H), 3.33 - 3.44 (m, 1 H), 3.66 (s, 3 H), 6.22 (s, 1 H), 6.49 (s, 1 H), 7.46 (s, 1 H), 9.60 (s, 1 H).

### Reference Example 51 2,4-dichloro-5-[(3-isopropyl-1-methyl-4-vinyl-1H-pyrazol-5-yl)oxy]phenol

Trimethylsilylmethylmagnesium chloride (1M diethyl ether solution, 45 mL) was added dropwise to a solution of 5-(2,4-dichloro-5-hydroxyphenoxy)-3-isopropyl-1-methyl-1H-pyrazole-4-carbaldehyde (4.70 g) obtained in Reference Example 50 in tetrahydrofuran (30 mL) with stirring for 15 min under ice-cooling. The reaction mixture was stirred at room temperature for 1 hr and ice-cooled. 1N Hydrochloric acid (45 mL) was added, and the mixture was stirred at room temperature for 1 hr. The reaction mixture was diluted with ethyl acetate. The organic layer was washed with 10% aqueous citric acid solution and saturated brine, and dried over anhydrous magnesium sulfate. The residue was filtered, and the filtrate was concentrated. The residue was crystallized from hexane-ethyl acetate to give the title compound (4.67 g, yield 100%) as white crystals.
¹H-NMR (300 MHz, CDCl₃)δ:1.23 (d, J = 7.2 Hz, 6 H), 2.93 - 3.10 (m, 1 H), 3.53 (s, 3 H), 5.07 (d, J=11.7 Hz, 1 H), 5.27 (d, J = 18.2 Hz, 1 H), 6.25 (s, 1 H), 6.31 (dd, J = 17.8, 11.7 Hz, 1 H), 7.44 (s, 1 H), 8.64 (br s, 1 H).

### Reference Example 52 5-(2,4-dichloro-5-hydroxyphenoxy)-1,3-dimethyl-1H-pyrazole-4-carbaldehyde

30% Sulfuric acid (3.0 mL) was added to a solution (10 mL) of 5-{2,4-dichloro-5-[(4-methoxybenzyl)oxy]phenoxy}-1,3-dimethyl-1H-pyrazole-4-carbaldehyde (3.5 g) obtained in Reference Example 2 in tetrahydrofuran, and the mixture was stirred at 50°C for 2.5 hr. Water was added to the reaction mixture, and the mixture was extracted with ethyl acetate. The organic layer was washed with water and saturated brine, dried over anhydrous magnesium sulfate and filtered, and the filtrate was concentrated. The resulting crystals were recrystallized from hexane-ethyl acetate to give the title compound as colorless crystals (1.9 g, yield 76%).
¹H-NMR (300 MHz, CDCl₃)δ:2.44 (s, 3 H), 3.67 (s, 3 H), 6.54 (s, 1 H), 7.46 (s, 1 H), 9.53 (s, 1 H).

### Reference Example 53 2,4-dichloro-5-[(4-formyl-1,3-dimethyl-1H-pyrazol-5-yl)oxy]phenyl trifluoromethanesulfonate

To a solution of 5-(2,4-dichloro-5-hydroxyphenoxy)-1,3-dimethyl-1H-pyrazole-4-carbaldehyde (1.20 g) obtained in Reference Example 52 in tetrahydrofuran (40 mL) was added 60% sodium hydride (oil, 192 mg) at 0°C, and the mixture was stirred for 20 min. N-Phenylbis(trifluoromethanesulfonimide) (1.86 g) was added to the reaction mixture, and the mixture was stirred for 2 hr. Saturated aqueous ammonium chloride solution was added, and the mixture was extracted with ethyl acetate. The organic layer was washed with water and saturated brine, dried over anhydrous magnesium sulfate and filtered. The filtrate was concentrated, and the residue was subjected to silica gel column chromatography (hexane-ethyl acetate 5:1 - 3:1, v/v) to give the title compound as a colorless oil (1.57 g, yield 91%). Recrystallization from hexane-ethyl acetate gave colorless crystals.
¹H-NMR (300 MHz, CDCl₃)δ:2.48 (s, 3 H), 3.72 (s, 3 H), 6.89 (s, 1 H), 7.67 (s, 1 H), 9.59 (s, 1 H).

### Reference Example 54 5-(5-bromo-2-chlorophenoxy)-1,3-dimethyl-1H-pyrazole-4-carbaldehyde

To a solution of 5-bromo-2-chlorophenol (5.00 g) and 5-chloro-1,3-dimethyl-1H-pyrazole-4-carbaldehyde (3.19 g) in N-methylpyrrolidone (15 mL) was added potassium carbonate (4.15 g), and the mixture was stirred under an argon stream at 120°C for 36 hr. Water was added to the reaction mixture, and the mixture was extracted with ethyl acetate. The organic layer was washed with water and saturated brine, dried over anhydrous magnesium sulfate and filtered. The filtrate was concentrated, and the residue was recrystallized from hexane-ethyl acetate to give the title compound as colorless crystals (4.83 g, yield 73%).
¹H-NMR (300 MHz, CDCl₃)δ:2.47 (s, 3 H), 3.69 (s, 3 H), 6.99 (d, J = 2.1 Hz, 1 H), 7.24 - 7.31 (m, 1 H), 7.33 - 7.39 (m, 1 H), 9.54 (s, 1 H).

### Reference Example 55 5-(4-bromo-2-chlorophenoxy)-1,3-dimethyl-1H-pyrazole-4-carbaldehyde

The title compound was obtained in the same manner as in Reference Example 54 and from 4-bromo-2-chlorophenol and 5-chloro-1,3-dimethyl-1H-pyrazole-4-carbaldehyde.
¹H-NMR (300 MHz, CDCl₃)δ:2.45 (s, 3 H), 3.68 (s, 3 H), 6.78 (d, J = 8.7 Hz, 1 H), 7.34 (dd, J = 8.7, 2.3 Hz, 1 H), 7.64 (d, J = 2.3 Hz, 1 H), 9.51 (s, 1 H).

### Reference Example 56 5-(5-amino-2,4-dichlorophenoxy)-1,3-dimethyl-1H-pyrazole-4-carbaldehyde

The title compound was obtained in the same manner as in Reference Example 54 and from 5-amino-2,4-dichlorophenol and 5-chloro-1,3-dimethyl-1H-pyrazole-4-carbaldehyde.
¹H-NMR (300 MHz, CDCl₃)δ:2.45 (s, 3 H), 3.66 (s, 3 H), 4.15 (br s, 2 H), 6.28 (s, 1 H), 7.35 (s, 1 H), 9.52 (s, 1 H).

### Reference Example 57 2-{2,4-dichloro-5-[(1,3-dimethyl-4-vinyl-1H-pyrazol-5-yl)oxy]phenoxy}propanenitrile

To a solution of 2,4-dichloro-5-[(1,3-dimethyl-4-vinyl-1H-pyrazol-5-yl)oxy]phenol (450 mg) obtained in Reference Example 6 and 2-bromopropanenitrile (0.156 mL) in N,N-dimethylformamide (5 mL) was added potassium carbonate (311 mg), and the mixture was stirred at room temperature for 15 hr. Water was added to the reaction mixture, and the mixture was extracted with ethyl acetate. The organic layer was washed with water and saturated brine, dried over anhydrous magnesium sulfate and filtered. The filtrate was concentrated, and the residue was subjected to silica gel column chromatography (hexane-ethyl acetate 9:1 - 4:1, v/v) to give the title compound as a colorless oil (501 mg, yield 95%).
¹H-NMR (300 MHz, CDCl₃)δ:1.77 (d, J = 6.8 Hz, 3 H), 2.32 (s, 3 H), 3.63 (s, 3 H), 4.73 (q, J = 6.8 Hz, 1 H), 5.07 (dd, J = 11.7, 1.3 Hz, 1 H), 5.26 (dd, J = 17.9, 1.3 Hz, 1 H), 6.31 (dd, J = 17.9, 11.7 Hz, 1 H), 6.48 (s, 1 H), 7.53 (s, 1 H).

### Reference Example 58 5-(2,4-dichloro-5-hydroxyphenoxy)-1-methyl-3-(trifluoromethyl)-1H-pyrazole-4-carbaldehyde

To a solution of 5-{2,4-dichloro-5-[(4-methoxybenzyl)oxy]phenoxy}-1-methyl-3-(trifluoromethyl)-1H-pyrazole-4-carbaldehyde (1.43 g) obtained in Reference Example 8 in tetrahydrofuran (20 mL) was added 4N hydrochloric acid-ethyl acetate solution (20 mL), and the mixture was stirred at 50°C for 3.5 hr. The reaction mixture was concentrated, and the residue was recrystallized from hexane-ethyl acetate to give the title compound as colorless crystals (0.95 g, yield 89%).
¹H-NMR (300 MHz, CDCl₃)δ:3.85 (s, 3 H), 5.72 (br s, 1 H), 6.53 (s, 1 H), 7.47 (s, 1 H), 9.70 (d, J=0.75Hz, 1 H).

### Reference Example 59 2,4-dichloro-5-{[4-(hydroxymethyl)-1-methyl-3-(trifluoromethyl)-1H-pyrazol-5-yl]oxy}phenol

To a solution of 5-(2,4-dichloro-5-hydroxyphenoxy)-1-methyl-3-(trifluoromethyl)-1H-pyrazole-4-carbaldehyde (800 mg) obtained in Reference Example 58 in a mixed solvent of tetrahydrofuran (10 mL) and methanol (1 mL) was added sodium borohydride (85 mg), and the mixture was stirred at room temperature for 1 hr. Water was added to the reaction mixture, and the mixture was extracted with ethyl acetate. The organic layer was washed with saturated brine, dried over anhydrous magnesium sulfate and filtered. The filtrate was concentrated, and the residue was recrystallized from hexane-ethyl acetate to give the title compound as colorless crystals (754 mg, yield 94%).
¹H-NMR (300 MHz, DMSO-d₆)δ:3.68 (s, 3 H), 4.21 (s, 2 H), 5.06 (br s, 1 H), 6.47 (s, 1 H), 7.64 (s, 1 H), 10.75 (br s, 1 H).

### Reference Example 60 2,4-dichloro-5-{[1,4-dimethyl-3-(trifluoromethyl)-1H-pyrazol-5-yl]oxy}phenol

To a solution of 2,4-dichloro-5-{[4-(hydroxymethyl)-1-methyl-3-(trifluoromethyl)-1H-pyrazol-5-yl]oxy}phenol (677 mg) obtained in Reference Example 59 in trifluoroacetic acid (10 mL) was added triethylsilane (1.0 mL), and the mixture was stirred at room temperature for 2 hr. The reaction mixture was concentrated, and the residue was washed with diisopropyl ether to give the title compound as colorless crystals (144 mg, yield 22%).
¹H-NMR (300 MHz, CDCl₃)δ:1.93 (d, J = 0.9 Hz, 3 H), 3.70 (s, 3 H), 5.98 (br s, 1 H), 6.35 (s, 1 H), 7.45 (s, 1 H).

### Reference Example 61 2,4-dichloro-5-{[4-ethyl-1-methyl-3-(trifluoromethyl)-1H-pyrazol-5-yl]oxy}phenol

To a solution of 2,4-dichloro-5-{[1-methyl-3-(trifluoromethyl)-4-vinyl-1H-pyrazol-5-yl]oxy}phenol (1.5 g) obtained in Reference Example 11 in a mixed solvent of tetrahydrofuran (20 mL) and ethanol (20 mL) was added platinum oxide (200 mg), and the mixture was stirred under a hydrogen stream for 2 hr. The reaction mixture was filtered, and the filtrate was concentrated. The residue was recrystallized from hexane-ethyl acetate to give the title compound as colorless crystals (1.2 g, yield 79%).
¹H-NMR (300 MHz, CDCl₃)δ:1.06 (t, J = 7.5 Hz, 3 H), 2.32 - 2.45 (m, 2 H), 3.68 (s, 3 H), 5.99 (s, 1 H), 6.33 (s, 1 H), 7.45 (s, 1 H).

### Reference Example 62 [5-(5-bromo-2-chlorophenoxy)-1,3-dimethyl-1H-pyrazol-4-yl]methanol

The title compound was obtained in the same manner as in Reference Example 59 and from 5-(5-bromo-2-chlorophenoxy)-1,3-dimethyl-1H-pyrazole-4-carbaldehyde obtained in Reference Example 54.
¹H-NMR (300 MHz, CDCl₃)δ:1.40 (t, J = 5.5 Hz, 1 H), 2.30 (s, 3 H), 3.60 (s, 3 H), 4.33 (d, J=5.5Hz, 2 H), 6.94 (d, J=2.1 Hz, 1 H), 7.17 - 7.23 (m, 1 H), 7.29- 7.36 (m, 1 H).

### Reference Example 63 5-(5-bromo-2-chlorophenoxy)-1,3,4-trimethyl-1H-pyrazole

The title compound was obtained in the same manner as in Reference Example 60 and from [5-(5-bromo-2-chlorophenoxy)-1,3-dimethyl-1H-pyrazol-4-yl]methanol obtained in Reference Example 62.
¹H-NMR (300 MHz, CDCl₃)δ:1.70 (s, 3 H), 2.32 (s, 3 H), 3.83 (s, 3 H), 7.01 (d, J = 2.1 Hz, 1 H), 7.28 - 7.40 (m, 2 H).

### Reference Example 64 4-chloro-3-[(1,3,4-trimethyl-1H-pyrazol-5-yl)oxy]benzaldehyde

To a solution of 5-(5-bromo-2-chlorophenoxy)-1,3,4-trimethyl-1H-pyrazole (1.89 g) obtained in Reference Example 63 in tetrahydrofuran (30 mL) was added dropwise n-butyllithium (1.6M hexane solution, 3.9 mL) at -78°C under an argon stream, and the mixture was stirred for 40 min. N,N-Dimethylformamide (0.56 mL) was added to the reaction solution, and the mixture was stirred at -78°C for 1 hr. Water was added to the reaction mixture, and the mixture was extracted with ethyl acetate. The organic layer was washed with water and saturated brine, dried over anhydrous magnesium sulfate and filtered. The filtrate was concentrated, and the residue was subjected to silica gel column chromatography (hexane-ethyl acetate 9:1 - 2:1, v/v) to give the title compound as a colorless oil (986 mg, yield 62%).
¹H-NMR (300 MHz, CDCl₃)δ:1.76 (s, 3 H), 2.21 (s, 3 H), 3.60 (s, 3 H), 7.19 (d, J = 1.5 Hz, 1 H), 7.52 - 7.60 (m, 1 H), 7.61 - 7.68 (m, 1 H), 9.88 (s, 1 H).

### Reference Example 65 5-{2,4-dichloro-5-[(4-methoxybenzyl)oxy]phenoxy}-4-ethynyl-1,3-dimethyl-1H-pyrazole

To a solution cooled to -78°C of lithium diisopropylamide (1.8 M heptane-tetrahydrofuran solution, 1.66 mL) in tetrahydrofuran (30 mL) was added trimethylsilyldiazomethane (2.0 M diethyl ether solution, 1.90 mL) with stirring, and the mixture was stirred at the same temperature for 30 min. A solution of 5-{2,4-dichloro-5-[(4-methoxybenzyl)oxy]phenoxy}-1,3-dimethyl-1H-pyrazole-4-carbaldehyde (1.00 g) obtained in Reference Example 2 in tetrahydrofuran (16 mL) was added dropwise. After the completion of the dropwise addition, the mixture was stirred at the same temperature for 10 min. Cooling was stopped, the reaction mixture was gradually allowed to warm to room temperature, and ethyl acetate and water were added. Respective layers were separated, and the aqueous layer was extracted again with ethyl acetate. The organic layers were combined, and the mixture was washed with saturated brine, dried over anhydrous magnesium sulfate and filtered. The filtrate was concentrated, and the residue was subjected to silica gel column chromatography (hexane-ethyl acetate 1:0 - 3:7, v/v) to give the title compound (0.52 g, yield 52%) as an orange oil.
¹H-NMR (300 MHz, CDCl₃)δ:2.28 (s, 3 H), 2.94 (s, 1 H), 3.63 (s, 3 H), 3.81 (s, 3 H), 5.00 (s, 2 H), 6.72 (s, 1 H), 6.86 - 6.92 (m, 2 H), 7.27 - 7.33 (m, 2 H), 7.43 (s, 1 H).

### Reference Example 66 1-[5-(2,4-dichloro-5-hydroxyphenoxy)-1,3-dimethyl-1H-pyrazol-4-yl]ethanone

30% Sulfuric acid (8 mL) was added to a solution of 5-{2,4-dichloro-5-[(4-methoxybenzyl)oxy]phenoxy}-4-ethynyl-1,3-dimethyl-1H-pyrazole (0.52 g) obtained in Reference Example 65 in tetrahydrofuran (8 mL) with stirring, and the mixture was stirred with heating at 60°C for 8 hr. The reaction mixture was poured into ice, and the mixture was extracted with ethyl acetate. The organic layer was dried over anhydrous magnesium sulfate and filtered. The filtrate was concentrated, and the residue was subjected to silica gel column chromatography (hexane-ethyl acetate 1:0 - 4:6, v/v) and crystallized from hexane-ethyl acetate to give the title compound (0.25 g, yield 64%) as colorless crystals.
¹H-NMR (300 MHz, CDCl₃)δ:2.24 (s, 3 H), 2.43 (s, 3 H), 3.58 (s, 3 H), 6.27 (s, 1 H), 6.64 (br s, 1 H), 7.48 (s, 1 H).

### Reference Example 67 2,4-dichloro-5-{[1,3-dimethyl-4-(2-methyl-1-propen-1-yl)-1H-pyrazol-5-yl]oxy}phenol

The title compound was obtained in the same manner as in Reference Example 10 and from 5-{2,4-dichloro-5-[(4-methoxybenzyl)oxy]phenoxy}-1,3-dimethyl-1H-pyrazole-4-carbaldehyde obtained in Reference Example 2 and isopropylmagnesium bromide.
¹H-NMR (300 MHz, CDCl₃)δ:1.64 (s, 3 H), 1.76 (s, 3 H), 2.11 (s, 3 H), 3.55 (s, 3 H), 5.57 (s, 1 H), 6.24 (s, 1 H), 7.39 (s, 1 H), 8.44 (br s, 1 H).

### Reference Example 68 2,4-dichloro-5-({1,3-dimethyl-4-[(lE)-1-penten-1-yl]-1H-pyrazol-5-yl}oxy)phenol

The title compound was obtained in the same manner as in Reference Example 10 and from 5-{2,4-dichloro-5-[(4-methoxybenzyl)oxy]phenoxy}-1,3-dimethyl-1H-pyrazole-4-carbaldehyde obtained in Reference Example 2 and n-butylmagnesium bromide.
¹H-NMR (300 MHz, CDCl₃)δ:0.83 (t, J = 7.3 Hz, 3 H), 1.29 - 1.42 (m, 2 H), 1.99 - 2.10 (m, 2 H), 2.12 (s, 3 H), 3.48 (s, 3 H), 5.78 (dt, J = 16.2, 6.6 Hz, 1 H), 5.91 (d, J = 16.2 Hz, 1 H), 6.16 (s, 1H), 7.44 (s, 1 H), 11.08 (br s, 1 H).

### Reference Example 69 1-(5-{2,4-dichloro-5-[(4-methoxybenzyl)oxy]phenoxy}-1,3-dimethyl-1H-pyrazol-4-yl)-2-methylpropan-1-ol

Isopropylmagnesium bromide (1M tetrahydrofuran solution, 7.9 mL) was added dropwise to a solution of 5-{2,4-dichloro-5-[(4-methoxybenzyl)oxy]phenoxy}-1,3-dimethyl-1H-pyrazole-4-carbaldehyde (2.00 g) obtained in Reference Example 2 in tetrahydrofuran (20 mL) with stirring at room temperature. The reaction mixture was stirred at room temperature for 4 hr, saturated aqueous ammonium chloride solution was added, and the mixture was extracted with ethyl acetate. The organic layer was dried over anhydrous magnesium sulfate and filtered. The filtrate was concentrated, and the residue was subjected to silica gel column chromatography (hexane-ethyl acetate 1:0 - 4:6, v/v) to give the title compound (2.11 g, yield 95%) as a colorless oil.
¹H-NMR (300 MHz, CDCl₃)δ:0.74 (d, J = 6.8 Hz, 3 H), 0.99 (d, J = 6.4 Hz, 3 H), 1.32 (d, J = 4.3 Hz, 1 H), 1.85 - 2.03 (m, 1 H), 2.31 (s, 3 H), 3.44 (s, 3 H), 3.81 (s, 3 H), 4.01 (dd, J = 8.8, 3.5 Hz, 1 H), 4.92 (s, 2 H), 6.32 (s, 1 H), 6. 83 - 6.92 (m, 2 H), 7.15 - 7.23 (m, 2 H), 7.43 (s, 1 H).

### Reference Example 70 2,4-dichloro-5-[(4-isobutyl-1,3-dimethyl-1H-pyrazol-5-yl)oxy]phenol

Triethylsilane (0.86 mL) was added to a solution of 1-(5-{2,4-dichloro-5-[(4-methoxybenzyl)oxy]phenoxy}-1,3-dimethyl-1H-pyrazol-4-yl)-2-methylpropan-1-ol (1.00 g) obtained in Reference Example 69 in trifluoroacetic acid (20 mL) with stirring. The reaction mixture was stirred at room temperature for 6 hr and concentrated. Saturated aqueous sodium hydrogen carbonate solution was added to the residue, and the mixture was extracted with ethyl acetate. The organic layer was dried over anhydrous magnesium sulfate and filtered. The filtrate was concentrated, and the residue was subjected to silica gel column chromatography (hexane-ethyl acetate 1:0 - 1:1, v/v) and crystallized from hexane-ethyl acetate to give the title compound (0.63 g, yield 90%) as colorless crystals.
¹H-NMR (300 MHz, CDCl₃)δ:0.84 (d, J = 6.6 Hz, 6 H), 1.61 - 1.79 (m, 1 H), 2.02 (d, J = 7.0 Hz, 2 H), 2.08 (s, 3 H), 3.49 (s, 3 H), 6.21 (s, 1 H), 7.43 (s, 1 H), 10.49 (br s, 1 H).

### Reference Example 71 cyclohexyl(5-{2,4-dichloro-5-[(4-methoxybenzyl)oxy]phenoxy}-1,3-dimethyl-1H-pyrazol-4-yl)methanol

The title compound was obtained in the same manner as in Reference Example 69 and from 5-{2,4-dichloro-5-[(4-methoxybenzyl)oxy]phenoxy}-1,3-dimethyl-1H-pyrazole-4-carbaldehyde obtained in Reference Example 2 and cyclohexylmagnesium bromide.
¹H-NMR (300 MHz, CDCl₃)δ:0.64 - 1.81 (m, 11 H), 1.94 - 2.07 (m, 1 H), 2.30 (s, 3 H), 3.44 (s, 3 H), 3.81 (s, 3 H), 4.05 (d, J = 9.0 Hz, 1 H), 4.91 (s, 2 H), 6.33 (s, 1 H), 6.82 - 6.91 (m, 2 H), 7.15 - 7.23 (m, 2 H), 7.43 (s, 1 H).

### Reference Example 72 2,4-dichloro-5-{[4-(cyclohexylmethyl)-1,3-dimethyl-1H-pyrazol-5-yl]oxy}phenol

The title compound was obtained in the same manner as in Reference Example 70 and from cyclohexyl(5-{2,4-dichloro-5-[(4-methoxybenzyl)oxy]phenoxy}-1,3-dimethyl-1H-pyrazol-4-yl)methanol obtained in Reference Example 71.
¹H-NMR (300 MHz, CDCl₃)δ:0.74 - 0.96 (m, 2 H), 1.00 - 1.42 (m, 4 H), 1.50 - 1.73 (m, 5 H), 2.01 (d, J = 7.0 Hz, 2 H), 2.09 (s, 3 H), 3.50 (s, 3 H), 6.23 (s, 1 H), 7.42 (s, 1 H), 9.49 (br s, 1 H).

### Reference Example 73 1-(5-{2,4-dichloro-5-[(4-methoxybenzyl)oxy]phenoxy}-1,3-dimethyl-1H-pyrazol-4-yl)pentan-1-ol

The title compound was obtained in the same manner as in Reference Example 69 and from 5-{2,4-dichloro-5-[(4-methoxybenzyl)oxy]phenoxy}-1,3-dimethyl-1H-pyrazole-4-carbaldehyde obtained in Reference Example 2 and n-butylmagnesium bromide.
¹H-NMR (300 MHz, CDCl₃)δ:0.84 (t, J = 7.1 Hz, 3 H), 1.06 - 1.32 (m, 5 H), 1.51 - 1.81 (m, 2 H), 2.32 (s, 3 H), 3.46 (s, 3 H), 3.81 (s, 3 H), 4. 35 - 4.46 (m, 1 H), 4.93 (s, 2 H), 6.33 (s, 1 H), 6.82 - 6.90 (m, 2 H), 7.15 - 7.23 (m, 2 H), 7.43 (s, 1 H).

### Reference Example 74 2,4-dichloro-5-[(1,3-dimethyl-4-pentyl-1H-pyrazol-5-yl)oxy]phenol

The title compound was obtained in the same manner as in Reference Example 70 and from 1-(5-{2,4-dichloro-5-[(4-methoxybenzyl)oxy]phenoxy}-1,3-dimethyl-1H-pyrazol-4-yl)pentan-1-ol obtained in Reference Example 73.
¹H-NMR (300 MHz, CDCl₃)δ:0.84 (t, J = 6.9 Hz, 3 H), 1.09 - 1.49 (m, 6 H), 2.01 - 2.23 (m, 5 H), 3.49 (s, 3 H), 6.25 (s, 1 H), 7.42 (s, 1 H), 9.69 (br s, 1 H).

### Reference Example 75 (5-{2,4-dichloro-5-[(4-methoxybenzyl)oxy]phenoxy}-1,3-dimethyl-1H-pyrazol-4-yl)methanol

A solution of 5-{2,4-dichloro-5-[(4-methoxybenzyl)oxy]phenoxy}-1,3-dimethyl-1H-pyrazole-4-carbaldehyde (5.00 g) obtained in Reference Example 2 in methanol (30 mL)-tetrahydrofuran (10 mL) was cooled to 0°C with stirring, sodium borohydride (0.68 g) was added, and the mixture was stirred for 4 hr while allowing the mixture to warm to room temperature. Ice was added to the reaction mixture, methanol and tetrahydrofuran were evaporated, and the residual suspension was extracted with ethyl acetate. The organic layer was dried over anhydrous magnesium sulfate and filtered. The filtrate was concentrated, and the residue was subjected to silica gel column chromatography (hexane-ethyl acetate 1:0 - 3:7, v/v) to give the title compound (3.99 g, yield 79%) as a colorless oil.
¹H-NMR (300 MHz, CDCl₃)δ:1.09 (t, J = 5.2 Hz, 1 H), 2.31 (s, 3 H), 3.52 (s, 3 H), 3.81 (s, 3 H), 4.21 (d, J = 5.3 Hz, 2 H), 4.95 (s, 2 H), 6.46 (s, 1 H), 6.83 - 6.90 (m, 2 H), 7.17 - 7.23 (m, 2 H), 7.43 (s, 1 H).

### Reference Example 76 5-{2,4-dichloro-5-[(4-methoxybenzyl)oxy]phenoxy}-4-(ethoxymethyl)-1,3-dimethyl-1H-pyrazole

A solution of (5-{2,4-dichloro-5-[(4-methoxybenzyl)oxy]phenoxy}-1,3-dimethyl-1H-pyrazol-4-yl)methanol (1.00 g) obtained in Reference Example 75 in N,N-dimethylformamide (10 mL) was cooled to 0°C with stirring, 60% sodium hydride (oil, 0.24 g) was added by small portions, and the mixture was stirred at the same temperature for 30 min. To the reaction solution was added ethyl iodide (0.76 mL), and the mixture was stirred for 4 hr while allowing the mixture to warm to room temperature. The reaction mixture was cooled to 0°C, ice was added, and the mixture was stirred for 10 min and extracted with ethyl acetate. The organic layer was dried over anhydrous magnesium sulfate and filtered. The filtrate was concentrated, and the residue was subjected to silica gel column chromatography (hexane-ethyl acetate 1:0 - 1:1, v/v) to give the title compound (0.95 g, yield 89%) as a colorless solid.
¹H-NMR (300 MHz, CDCl₃)δ:1.10 (t, J = 7.1 Hz, 3 H), 2.28 (s, 3 H), 3.34 (q, J = 7.0 Hz, 2 H), 3.51 (s, 3 H), 3.81 (s, 3 H), 4.00 (s, 2 H), 4.93 (s, 2 H), 6.53 (s, 1 H), 6.83 - 6.90 (m, 2 H), 7.17 - 7.25 (m, 2 H), 7.42 (s, 1 H).

### Reference Example 77 2,4-dichloro-5-{[4-(ethoxymethyl)-1,3-dimethyl-1H-pyrazol-5-yl]oxy}phenol

Platinum oxide (0.039 g) was added to a solution of 5-{2,4-dichloro-5-[(4-methoxybenzyl)oxy]phenoxy}-4-(ethoxymethyl)-1,3-dimethyl-1H-pyrazole (0.76 g) obtained in Reference Example 76 in ethanol (4 mL)-ethyl acetate (1 mL) with stirring. The reaction vessel was deaerated and purged with hydrogen. The reaction mixture was stirred for 12 hr, platinum oxide was filtered off, and the filtrate was concentrated. The residue was subjected to silica gel column chromatography (hexane-ethyl acetate 8:2 - 3:7, v/v) to give the title compound (0.43 g, yield 76%) as a colorless solid.
¹H-NMR (300 MHz, CDCl₃)δ:1.12 (t, J = 7.1 Hz, 3 H), 2.22 (s, 3 H), 3.37 (q, J = 7.0 Hz, 2 H), 3.55 (s, 3 H), 4.11 (s, 2 H), 6.40 - 6.46 (m, 1 H), 7.41 (s, 1 H).

### Reference Example 78 5-{2,4-dichloro-5-[(4-methoxybenzyl)oxy]phenoxy}-4-(methoxymethyl)-1,3-dimethyl-1H-pyrazole

The title compound was obtained in the same manner as in Reference Example 76 and from (5-{2,4-dichloro-5-[(4-methoxybenzyl)oxy]phenoxy}-1,3-dimethyl-1H-pyrazol-4-yl)methanol obtained in Reference Example 75 and methyl iodide.
¹H-NMR (300 MHz, CDCl₃)δ:2.28 (s, 3 H), 3.20 (s, 3 H), 3.52 (s, 3 H), 3.81 (s, 3 H), 3.97 (s, 2 H), 4.93 (s, 2 H), 6.57 (s, 1 H), 6.84 - 6.90 (m, 2 H), 7.18 - 7.25 (m, 2 H), 7.43 (s, 1 H).

### Reference Example 79 2,4-dichloro-5-{[4-(methoxymethyl)-1,3-dimethyl-1H-pyrazol-5-yl]oxy}phenol

The title compound was obtained in the same manner as in Reference Example 77 and from 5-{2,4-dichloro-5-[(4-methoxybenzyl)oxy]phenoxy}-4-(methoxymethyl)-1,3-dimethyl-1H-pyrazole obtained in Reference Example 78.
¹H-NMR (300 MHz, CDCl₃)δ:2.21 (s, 3 H), 3.23 (s, 3 H), 3.55 (s, 3 H), 4.07 (s, 2 H), 6.40 (s, 1 H), 7.42 (s, 1 H), 8.07 (br s, 1 H).

### Reference Example 80 2,4-dichloro-5-[(4-methoxybenzyl)oxy]aniline

A solution of 5-amino-2,4-dichlorophenol (5.00 g) in N,N-dimethylformamide (10 mL) was cooled to 0°C with stirring, 4-methoxybenzyl chloride (4.00 mL) was added dropwise, and the mixture was stirred for 12 hr while allowing the mixture to warm to room temperature. The reaction mixture was filtered, water was added to the mother liquor, and the mixture was extracted with ethyl acetate. The organic layer was dried over anhydrous magnesium sulfate and filtered, and the filtrate was concentrated. The obtained brown solid was crystallized from hexane-ethyl acetate to give the title compound (3.82 g, yield 46%) as colorless crystals. The mother liquor was concentrated, and the residue was subjected to silica gel column chromatography (hexane-ethyl acetate 1:0 - 1:1, v/v) and crystallized from hexane-ethyl acetate to give the title compound (1.86 g, yield 22%) as colorless crystals.
¹H-NMR (300 MHz, CDCl₃)δ:3.82 (s, 3 H), 4.00 (br s, 2 H), 5.01 (s, 2 H), 6.38 (s, 1 H), 6.91 (d, J = 8. 9 Hz, 2 H), 7.24 (s, 1 H), 7.35 (d, J = 8.7 Hz, 2 H).

### Reference Example 81 5-({2,4-dichloro-5-[(4-methoxybenzyl)oxy]phenyl}amino)-1,3-dimethyl-1H-pyrazole-4-carbaldehyde

A solution of 2,4-dichloro-5-[(4-methoxybenzyl)oxy]aniline (10.35 g) obtained in Reference Example 80 in N,N-dimethylformamide (100 mL) was cooled to 0°C with stirring, 60% sodium hydride (oil, 2.52 g) was added by small portions, and the mixture was stirred at the same temperature for 30 min. A solution of 5-chloro-1,3-dimethyl-1H-pyrazole-4-carbaldehyde (5.00 g) in N,N-dimethylformamide (25 mL) was added dropwise to the reaction mixture, and the mixture was stirred at 60°C for 4 hr and cooled to room temperature. Saturated aqueous ammonium chloride solution was added to the reaction mixture, and the mixture was extracted with ethyl acetate. The organic layer was dried over anhydrous magnesium sulfate and filtered. The filtrate was concentrated, and the residue was subjected to silica gel column chromatography (hexane-ethyl acetate 1:0 - 4:6, v/v) and crystallized from hexane-ethyl acetate to give the title compound (9.97 g, yield 75%) as pale-yellow crystals.
¹H-NMR (300 MHz, CDCl₃)δ:2.45 (s, 3 H), 3.24 (s, 3 H), 3.80 - 3.84 (m, 3 H), 4. 97 (s, 2 H), 6.26 (s, 1 H), 6.84 - 6.93 (m, 2 H), 7.21 - 7.29 (m, 2 H), 7.39 - 7.45 (m, 2 H), 9.75 (s, 1 H).

### Reference Example 82 5-[{2,4-dichloro-5-[(4-methoxybenzyl)oxy]phenyl}(methyl)amino]-1,3-dimethyl-1H-pyrazole-4-carbaldehyde

A solution of 5-({2,4-dichloro-5-[(4-methoxybenzyl)oxy]phenyl}amino)-1,3-dimethyl-1H-pyrazole-4-carbaldehyde (0.50 g) obtained in Reference Example 81 in N,N-dimethylformamide (5 mL) was cooled to 0°C with stirring, 60% sodium hydride (oil, 0.095 g) was added by small portions, and the mixture was stirred at the same temperature for 30 min. Methyl iodide (0.090 mL) was added to the reaction mixture, and the mixture was stirred for 5 hr while allowing the mixture to warm to room temperature. Ice was added to the reaction mixture, and the mixture was extracted with ethyl acetate. The organic layer was dried over anhydrous magnesium sulfate and filtered. The filtrate was concentrated, and the residue was subjected to silica gel column chromatography (hexane-ethyl acetate 1:0 - 4:6, v/v) to give the title compound (0.53 g, yield 100%) as a colorless solid.
¹H-NMR (300 MHz, CDCl₃)δ:2.44 (s, 3 H), 3.31 (s, 3 H), 3.45 (s, 3 H), 3.82 (s, 3 H), 5.03 (s, 2 H), 6.64 (s, 1 H), 6.86 - 6.92 (m, 2 H), 7.24 - 7.31 (m, 2 H), 7.33 (s, 1 H), 9.68 (s, 1 H).

### Reference Example 83 2,4-dichloro-5-[(1,3-dimethyl-1H-pyrazol-5-yl)(methyl)amino]phenol

A solution of 5-[{2,4-dichloro-5-[(4-methoxybenzyl)oxy]phenyl}(methyl)amino]-1,3-dimethyl-1H-pyrazole-4-carbaldehyde (0.52 g) obtained in Reference Example 82 in tetrahydrofuran (5 mL) was cooled to 0°C with stirring, trimethylsilylmethylmagnesium chloride (1M diethyl ether solution, 2.40 mL) was added dropwise, and the mixture was stirred for 12 hr while allowing the mixture to warm to room temperature. 0.5N Hydrochloric acid was added to the reaction mixture, and the mixture was extracted with ethyl acetate. The organic layer was dried over anhydrous magnesium sulfate and filtered, and the filtrate was concentrated. 30% Sulfuric acid (5 mL) and tetrahydrofuran (5 mL) were added to the obtained residue, and the mixture was stirred with heating at 80°C for 5 hr. The reaction mixture was cooled to room temperature, ice was added, and the mixture was extracted with ethyl acetate. The organic layer was dried over anhydrous magnesium sulfate and filtered. The filtrate was concentrated, and the residue was subjected to silica gel column chromatography (hexane-ethyl acetate 9:1 - 1:9, v/v) to give the title compound (0.069 g, yield 20%) as a colorless solid.
¹H-NMR (300 MHz, CDCl₃)δ:2.20 (s, 3 H), 3.09 (s, 3 H), 3.20 (s, 3 H), 5.76 (s, 1 H), 6.35 (s, 1 H), 7.37 (s, 1 H), 9.82 (br s, 1 H).

### Reference Example 84 (5-{2,4-dichloro-5-[(4-methoxybenzyl)oxy]phenoxy}-1,3-dimethyl-1H-pyrazol-4-yl)(phenyl)methanol

The title compound was obtained in the same manner as in Reference Example 69 and from 5-{2,4-dichloro-5-[(4-methoxybenzyl)oxy]phenoxy}-1,3-dimethyl-1H-pyrazole-4-carbaldehyde obtained in Reference Example 2 and phenylmagnesium bromide.
¹H-NMR (300 MHz, CDCl₃)δ:1.84 (d, J = 4.2 Hz, 1 H), 2.23 (s, 3 H), 3.44 (s, 3 H), 3.81 (s, 3 H), 4.78 (s, 2 H),5.65 (d, J = 4.2 Hz, 1 H), 6.14 (s, 1 H), 6.84 - 6.91 (m, 2 H), 7.11 - 7.28 (m, 7 H), 7.36 (s, 1 H).

### Reference Example 85 5-[(4-benzyl-1,3-dimethyl-1H-pyrazol-5-yl)oxy]-2,4-dichlorophenol

The title compound was obtained in the same manner as in Reference Example 70 and from (5-{2,4-dichloro-5-[(4-methoxybenzyl)oxy]phenoxy}-1,3-dimethyl-1H-pyrazol-4-yl)(phenyl)methanol obtained in Reference Example 84. ¹H-NMR (300 MHz, CDCl₃)δ:2. 10 (s, 3 H), 3.52 (s, 3 H), 3.53 (s, 2 H), 6.19 (s, 1 H), 6.97 - 7.06 (m, 2 H), 7.08 - 7.23 (m, 3 H), 7.36 (s, 1 H), 8.28 (s, 1 H).

### Reference Example 86 2-[(E)-2-(5-{2,4-dichloro-5-[(4-methoxybenzyl)oxy]phenoxy}-1,3-dimethyl-1H-pyrazol-4-yl)vinyl]pyridine hydrochloride

To a solution of 5-12,4-dichloro-5-[(4-methoxybenzyl)oxy]phenoxy}-1,3-dimethyl-1H-pyrazole-4-carbaldehyde (5.00 g) obtained in Reference Example 2 and triphenyl(2-pyridylmethyl)phosphonium chloride hydrochloride (10.2 g) in tetrahydrofuran (50 mL) was added potassium tert-butoxide (6.02 g), and the mixture was stirred at room temperature for 6 hr. Water was added to the reaction mixture, and the mixture was extracted with ethyl acetate. The organic layer was dried over anhydrous magnesium sulfate and filtered. The filtrate was concentrated, and the residue was subjected to silica gel column chromatography (hexane-ethyl acetate 1:0 - 3:7, v/v) to give a mixture containing a free form of the title compound. 4N Hydrochloric acid-ethyl acetate solution was cooled to 0°C with stirring, the above-mentioned mixture was added, and the mixture was stirred at the same temperature for 15 min. The precipitate was collected by filtration and washed with ethyl acetate to give the title compound (1.54 g, yield 24%) as a pale-yellow solid.
¹H-NMR (300 MHz, DMSO-d₆)δ:2.44 (s, 3 H), 3.48 (s, 3 H), 3.66 (s, 3 H), 4.97 - 5.05 (m, 3 H), 6.49 (s, 1 H), 6.74 - 6.85 (m, 3 H), 7.10 - 7.18 (m, 2 H), 7.64 (d, J = 16.6 Hz, 1 H), 7.68 - 7.76 (m, 1 H), 7.83 (s, 1 H), 8.12 (d, J = 8.5 Hz, 1 H), 8.30 - 8.44 (m, 1 H), 8.65 (d, J = 4.7 Hz, 1 H).

### Reference Example 87 2,4-dichloro-5-({1,3-dimethyl-4-[(E)-2-pyridin-2-ylvinyl]-1H-pyrazol-5-yl}oxy)phenol

To a suspension of 2-[(E)-2-(5-{2,4-dichloro-5-[(4-methoxybenzyl)oxy]phenoxy}-1,3-dimethyl-1H-pyrazol-4-yl)vinyl]pyridine hydrochloride (1.54 g) obtained in Reference Example 86 in ethyl acetate (100 mL) was added 1N sodium hydroxide (5 mL), and the mixture was extracted with ethyl acetate. The organic layer was dried over anhydrous magnesium sulfate and filtered, and the filtrate was concentrated to give 2-[(E)-2-(5-{2,4-dichloro-5-[(4-methoxybenzyl)oxy]phenoxy}-1,3-dimethyl-1H-pyrazol-4-yl)vinyl]pyridine as a brown solid.
The compound was dissolved in tetrahydrofuran (30 mL), 30% sulfuric acid (5 mL) was added to the solution with stirring, and the mixture was stirred at 60°C for 5 hr. The reaction mixture was neutralized with 1N aqueous sodium hydroxide solution while cooling with ice water, and further adjusted with saturated aqueous sodium hydrogen carbonate solution to pH 8 - 9. The mixture was extracted with ethyl acetate. The organic layer was dried over anhydrous magnesium sulfate and filtered, and the filtrate was concentrated to give the title compound (0.78 g, yield 72%) as a pale-yellow solid.
¹H-NMR (300 MHz, DMSO-d₆)δ:2.35 (s, 3 H), 3.56 (s, 3 H), 6.36 (s, 1 H), 6.68 (d, J = 16.4 Hz, 1 H), 7. 12 - 7.21 (m, 1 H), 7.24 - 7.35 (m, 2 H), 7.60 - 7.76 (m, 2 H), 8.47 (dd, J = 4.9, 0.9 Hz, 1 H), 10.70 (br s, 1 H,).

### Reference Example 88 (5-{2,4-dichloro-5-[(4-methoxybenzyl)oxy]phenoxy}-1,3-dimethyl-1H-pyrazol-4-yl)acetonitrile

A solution of (5-{2,4-dichloro-5-[(4-methoxybenzyl)oxy]phenoxy}-1,3-dimethyl-1H-pyrazol-4-yl)methanol (0.97 g) obtained in Reference Example 75 and tributylphosphine (1.85 g) in tetrahydrofuran (10 mL) was cooled to 0°C with stirring, and 1,1'-(azodicarbonyl)dipiperidine (2.30 g) was added by small portions. The reaction mixture was stirred for 10 min, a solution of acetone cyanohydrin (0.78 g) in tetrahydrofuran (5 mL) was added, and the mixture was stirred for 12 hr while allowing the mixture to warm to room temperature. The reaction mixture was filtered, the mother liquor was concentrated, and the residue was subjected to silica gel column chromatography (hexane-ethyl acetate 8:2 - 3:7, v/v) to give the title compound (0.85 g, 86%) as a yellow oil.
¹H-NMR (300 MHz, CDCl₃)δ:2.32 (s, 3 H), 3.14 (s, 2 H), 3.49 (s, 3 H), 3.82 (s, 3 H), 4.99 (s, 2 H), 6.31 (s, 1 H), 6. 81 - 6.92 (m, 2 H), 7.17 - 7.25 (m, 2 H), 7.46 (s, 1 H).

### Reference Example 89 [5-(2,4-dichloro-5-hydroxyphenoxy)-1,3-dimethyl-1H-pyrazol-4-yl]acetonitrile

(5-{2,4-Dichloro-5-[(4-methoxybenzyl)oxy]phenoxy}-1,3-dimethyl-1H-pyrazol-4-yl)acetonitrile (0.79 g) obtained in Reference Example 88 and 4N hydrochloric acid-ethyl acetate solution (5 mL) were mixed, and the mixture was stirred at 60°C for 2 hr. Water was added to the reaction mixture, and the mixture was extracted with ethyl acetate. The organic layer was dried over anhydrous magnesium sulfate and filtered. The filtrate was concentrated, and the residue was subjected to silica gel column chromatography (hexane-ethyl acetate 1:0 - 1:1, v/v) to give the title compound (0.48 g, yield 84%) as a pale-yellow solid.
¹H-NMR (300 MHz, CDCl₃)δ:2.28 (s, 3 H), 3.30 (s, 2 H), 3.58 (s, 3 H), 6.36 (s, 1 H), 6.82 (br s, 1 H), 7.46 (s, 1 H).

### Reference Example 90 5-[(3-hydroxyphenyl)thio]-1,3-dimethyl-1H-pyrazole-4-carbaldehyde

A mixture of 3-hydroxythiophenol (5.00 g), 5-chloro-1,3-dimethyl-1H-pyrazole-4-carbaldehyde (6.28 g), potassium carbonate (5.53 g) and N,N-dimethylformamide (100 mL) was stirred under a nitrogen atmosphere at 70°C overnight. The reaction mixture was cooled to room temperature, water was added, and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 90:10 - 65:35, v/v) to give the title compound (3.5 g, yield 36%) as crystals.
¹H-NMR (300 MHz, CDCl3)δ:2. 43 (s, 3 H), 3.77 (s, 3 H), 6.41 - 6.42 (m, 1 H), 6.71 - 6.77 (m, 2 H), 7.18 (t, J = 7.8 Hz, 1 H), 7.43 (s, 1 H), 9.94 (s, 1 H).

### Reference Example 91 3-[(1,3-dimethyl-4-vinyl-1H-pyrazol-5-yl)thio]phenol

The title compound was obtained in the same manner as in Reference Example 5 and using 5-[(3-hydroxyphenyl)thio]-1,3-dimethyl-1H-pyrazole-4-carbaldehyde obtained in Reference Example 90.
¹H-NMR (300 MHz, CDCl₃)δ:2.22 (s, 3 H), 3.69 (s, 3 H), 5.23 - 5.28 (m, 1 H), 5.54 - 5.61 (m, 1 H), 6.14 - 6.16 (m, 1 H), 6.57 - 6.77 (m, 3 H), 7.13 - 7.19 (m, 1 H), 9.83 (s, 1 H).

### Reference Example 92 3-{[4-(hydroxymethyl)-1,3-dimethyl-1H-pyrazol-5-yl]thio}phenol

To a solution of 5-[(3-hydroxyphenyl)thio]-1,3-dimethyl-1H-pyrazole-4-carbaldehyde (7.03 g) obtained in Reference Example 90 in a mixed solvent of tetrahydrofuran (200 mL) and methanol (20 mL) was added sodium borohydride (2.16 g), and the mixture was stirred at room temperature for 3 hr. Water was added to the reaction mixture, and the mixture was extracted with ethyl acetate. The organic layer was washed with saturated brine, dried over anhydrous magnesium sulfate and filtered, and the filtrate was concentrated to give the title compound as a colorless oil (5.53 g, yield 78%).
¹H-NMR (300 MHz, CDCl₃)δ:2.21 (s, 3 H), 3.68 (s, 3 H), 4.54 (s, 2 H), 6.25 - 6.27 (m, 1 H), 6.62 - 6.71 (m, 2 H), 7.09 - 7.14 (m, 1 H), 8.82 (s, 1 H).

### Reference Example 93 3-[(1,3,4-trimethyl-1H-pyrazol-5-yl)thio]phenol

To a solution of 3-{[4-(hydroxymethyl)-1,3-dimethyl-1H-pyrazol-5-yl]thio}phenol (5.53 g) obtained in Reference Example 92 in trifluoroacetic acid (150 mL) was added triethylsilane (16 mL), and the mixture was stirred at room temperature for 3 hr. The reaction mixture was concentrated, and the residue was subjected to silica gel column chromatography (hexane-ethyl acetate 80:20 - 50:50, v/v) to give the title compound as a colorless oil (2.1 g, yield 41%).
¹H-NMR (300 MHz, CDCl₃)δ:2.02 (s, 3 H), 2.16 (s, 3 H), 3.75 (s, 3 H), 6.21 - 6.23 (m, 1 H), 6.63 - 6.71 (m, 2 H), 7.11 - 7.17 (m, 1 H), 7.59 (br, 1 H).

### Reference Example 94 2,4-dichloro-5-[(1,3,4-trimethyl-1H-pyrazol-5-yl)thio]phenol

To a solution, which was cooled to 0°C in an ice bath, of 3-[(1,3,4-trimethyl-1H-pyrazol-5-yl)thio]phenol (2.0 g) obtained in Reference Example 93 in 1,2-dichloroethane (12 mL) was added dropwise sulfuryl chloride (2.3 g), and the mixture was stirred at room temperature for 30 min. The solvent was evaporated and the residue was purified by preparative HPLC using "apparatus: High Throughput purification system manufactured by Gilson Inc.
column: YMC Combiprep ODS-A, S-5 µm, 50 x 20 mm solvent: SOLUTION A; 0.1% trifluoroacetic acid-containing water, SOLUTION B; 0.1% trifluoroacetic acid-containing acetonitrile, gradient cycle: 0.00 min (SOLUTION A/SOLUTION B = 90/10), 1.00 min (SOLUTION A/SOLUTION B = 90/10), 4.20 min (SOLUTION A/SOLUTION B = 10/90), 5.40 min (SOLUTION A/SOLUTION B = 10/90), 5.50 min (SOLUTION A/SOLUTION B = 90/10), 5.60 min (SOLUTION A/SOLUTION B = 90/10), flow rate: 25 mL/min, detection method: UV 220 nm" to give the title compound (0.42 g, yield 16%).
¹H-NMR (300 MHz, DMSO-d₆)δ:1.93 (s, 3 H), 2.19 (s, 3 H), 3.70 (s, 3 H), 6.10 (s, 1 H), 7.53 (s, 1 H), 10.51 (s, 1 H).

### Reference Example 95 5-[(2,4-dichloro-5-hydroxyphenyl)(methyl)amino]-1,3-dimethyl-1H-pyrazole-4-carbaldehyde

The title compound was obtained in the same manner as in Reference Example 6 and from 5-[{2,4-dichloro-5-[(4-methoxybenzyl)oxy]phenyl}(methyl)amino]-1,3-dimethyl-1H-pyrazole-4-carbaldehyde obtained in Reference Example 82.
¹H-NMR (300 MHz, CDCl₃)δ:2.40 (s, 3 H), 3.34 (s, 3 H), 3.53 (s, 3 H), 6.71 (s, 1 H), 6.92 (br, 1 H), 7.30 (s, 1 H), 9.68 (s, 1 H).

### Reference Example 96 methyl (2R)-2-{[(4-methylphenyl)sulfonyl]oxy}propionate

Methyl (R)-(+)-lactate (5.00 g, 48.0 mmol) and ethyl acetate (25 mL) were added, this solution was cooled to 0°C, and tosyl chloride (10.1 g, 52.8 mmol) was added. The mixture was stirred at 0°C for 10 min, and a solution of 1,4-diazabicyclo[2,2,2]octane (808 mg, 7.20 mmol) in ethyl acetate (25 mL) and a solution of triethylamine (10.0 mL, 72.0 mmol) in ethyl acetate (25 mL) were successively added. The mixture was stirred at room temperature for 2 hr, water (12 mL) and 2N hydrochloric acid (26 mL) were added, and the mixture was left standing for 10 min. The aqueous layer was removed, and the organic layer was washed successively with water (26 mL) and 4 wt% aqueous sodium hydrogen carbonate solution (32 mL), dried over anhydrous magnesium sulfate and filtered. The filtrate was concentrated to give the title compound (12.4 g, yield 100%).
¹H-NMR (300 MHz, CDCl₃)δ:1.51 (d, J = 6.8 Hz, 3 H), 2.45 (s, 3 H), 3.67 (s, 3 H), 4.95 (q, J = 6. 8 Hz, 1 H), 7.35 (d, J = 8. 3 Hz, 2 H), 7.82 (d, J = 8.3 Hz, 2 H).

### Reference Example 97 3-{2,4-dichloro-5-[(4-methoxybenzyl)oxy]phenoxy}-2-methyl-2,6-dihydro-7H-pyrazolo[3,4-d]pyridazin-7-one

A solution (24 mL) of 5-{2,4-dichloro-5-[(4-methoxybenzyl)oxy]phenoxy)-4-formyl-1-methyl-1H-pyrazole-3-carboxylic acid (900 mg) obtained in Reference Example 21 and hydrazine monohydrate (104 mg) in 95% aqueous ethanol was stirred at room temperature for 2.5 hr and at 80°C for 16 hr. The reaction mixture was concentrated under reduced pressure, water was added, and the mixture was extracted with ethyl acetate. The organic layer was washed with saturated brine, dried over anhydrous magnesium sulfate and filtered. The filtrate was concentrated, and the residue was crystallized from hexane-ethyl acetate to give the title compound as yellow crystals (517 mg, yield 58%).
¹H-NMR (300 MHz, DMSO-d₆)δ:3.74 (s, 3 H), 4.01 (s, 3 H), 5.10 (s, 2 H), 6.89 (d, J = 8.7 Hz, 2 H), 7.08 (s, 1 H), 7.30 (d, J = 8.7 Hz, 2 H), 7.46 (s, 1 H), 7.89 (s, 1 H), 12.26 (s, 1 H).

### Reference Example 98 3-{2,4-dichloro-5-[(4-methoxybenzyl)oxy]phenoxy}-2,6-dimethyl-2,6-dihydro-7H-pyrazolo[3,4-d]pyridazin-7-one

A solution of 3-{2,4-dichloro-5-[(4-methoxybenzyl)oxy]phenoxy}-2-methyl-2,6-dihydro-7H-pyrazolo[3,4-d]pyridazin-7-one (300 mg) obtained in Reference Example 97 in N,N-dimethylformamide (8 mL) was cooled to 0°C in an ice bath. While stirring and cooling the reaction mixture to 0°C in an ice bath, 60% sodium hydride (oil, 32 mg) and methyl iodide (114 mg) were added. The reaction mixture was removed from the ice bath, stirred at room temperature for 1.5 hr and concentrated under reduced pressure. Water was added to the reaction mixture, and the mixture was extracted with ethyl acetate. The organic layer was washed with saturated brine, dried over anhydrous magnesium sulfate and filtered. The filtrate was concentrated, and the residue was crystallized from hexane-ethyl acetate to give the title compound as yellow crystals (192 mg, yield 62%).
¹H-NMR (300 MHz, DMSO-d₆)δ:3.60 (s, 3 H), 3.74 (s, 3 H), 4.01 (s, 3 H), 5.11 (s, 2 H), 6.88 (d, J = 8.7 Hz, 2 H), 7.05 (s, 1 H), 7.29 (d, J = 8.7 Hz, 2 H), 7.44 (s, 1 H), 7.89 (s, 1 H).

### Reference Example 99 3-(2,4-dichloro-5-hydroxyphenoxy)-2,6-dimethyl-2,6-dihydro-7H-pyrazolo[3,4-d]pyridazin-7-one

The title compound was obtained in the same manner as in Reference Example 52 and from 3-{2,4-dichloro-5-[(4-methoxybenzyl)oxy]phenoxy}-2,6-dimethyl-2,6-dihydro-7H-pyrazolo[3,4-d]pyridazin-7-one obtained in Reference Example 98.
¹H-NMR (300 MHz, DMSO-d₆)δ:3.61 (s, 3 H), 3.99 (s, 3 H), 6.92 (s, 1 H), 7.40 (s, 1 H), 7.77 (s, 1 H), 10.90 (s, 1 H).

### Reference Example 100 [5-(4-bromo-2-chlorophenoxy)-1,3-dimethyl-1H-pyrazol-4-yl]methanol

To a solution of 5-(4-bromo-2-chlorophenoxy)-1,3-dimethyl-1H-pyrazole-4-carbaldehyde (1.50 g) obtained in Reference Example 55 in a mixed solvent of tetrahydrofuran (28 mL) and methanol (4 mL) was added sodium borohydride (189 mg), and the mixture was stirred at room temperature for 2 hr. Water was added to the reaction mixture, and the mixture was extracted with ethyl acetate. The organic layer was washed with saturated brine, dried over anhydrous magnesium sulfate and filtered. The filtrate was concentrated, and the residue was crystallized from hexane-ethyl acetate to give the title compound as white crystals (1.18 g, yield 78%).
¹H-NMR (300 MHz, CDCl₃)δ:2.27 (s, 3 H), 3.58 (s, 3 H), 4.30 (d, J = 5.3 Hz, 2 H),6.70 (d, J = 8.9 Hz, 1 H), 7.28 (m, 1 H), 7.60 (d, J = 2.5 Hz, 1 H).

### Reference Example 101 5-(4-bromo-2-chlorophenoxy)-1,3,4-trimethyl-1H-pyrazole

To a solution of [5-(4-bromo-2-chlorophenoxy)-1,3-dimethyl-1H-pyrazol-4-yl]methanol (1.0 g) obtained in Reference Example 100 in trifluoroacetic acid (10 mL) was added triethylsilane (700 mg), and the mixture was stirred at room temperature for 5 hr. The reaction mixture was concentrated, and the residue was subjected to silica gel column chromatography (hexane-ethyl acetate 9:1 - 8:2, v/v) to give the title compound as white crystals (886 mg, yield 94%).
¹H-NMR (300 MHz, CDCl₃)δ:1.69 (s, 3 H), 2.29 (s, 3 H), 3.76 (s, 3 H), 6.70 (d, J = 8.7 Hz, 1 H), 7.35 (dd, J = 8.7, 2.4 Hz, 1 H), 7.64 (d, J = 2.4 Hz, 1 H).

### Reference Example 102 ethyl 5-(2,4-dichloro-5-hydroxyphenoxy)-4-formyl-1-methyl-1H-pyrazole-3-carboxylate

The title compound was obtained in the same manner as in Reference Example 52 and from ethyl 5-{2,4-dichloro-5-[(4-methoxybenzyl)oxy]phenoxy}-4-formyl-1-methyl-1H-pyrazole-3-carboxylate obtained in Reference Example 20.
¹H-NMR (300 MHz, CDCl₃)δ:1.45 (t, J = 7.2 Hz, 3 H), 3.83 (s, 3 H), 4.49 (q, J = 7.2 Hz, 2 H), 5.67 (br s, 1 H), 6.43 (s, 1 H), 7.44 (s, 1 H), 10.25 (s, 1 H).

### Reference Example 103 ethyl 5-(2,4-dichloro-5-hydroxyphenoxy)-4-(hydroxymethyl)-1-methyl-1H-pyrazole-3-carboxylate

The title compound was obtained in the same manner as in Reference Example 100 and from ethyl 5-(2,4-dichloro-5-hydroxyphenoxy)-4-formyl-1-methyl-1H-pyrazole-3-carboxylate obtained in Reference Example 102.
¹H-NMR (300 MHz, DMSO-d₆)δ:1.30 (t, J = 7.2 Hz, 3 H), 3.65 (s, 3 H), 4.29 (q, J = 7.2 Hz, 2 H), 4.41 (d, J = 4.9 Hz, 2 H), 4.74 (t, J = 5.1 Hz, 1 H), 6.44 (s, 1 H), 7.62 (s, 1 H), 10.62 (br s, 1 H).

### Reference Example 104 ethyl 5-(2,4-dichloro-5-hydroxyphenoxy)-1,4-dimethyl-1H-pyrazole-3-carboxylate

The title compound was obtained in the same manner as in Reference Example 101 and from ethyl 5-(2,4-dichloro-5-hydroxyphenoxy)-4-(hydroxymethyl)-1-methyl-1H-pyrazole-3-carboxylate obtained in Reference Example 103.
¹H-NMR (300 MHz, CDC1₃)δ:1.40 (t, J = 7.2 Hz, 3 H), 2.07 (s, 3 H), 3.70 (s, 3 H), 4.40 (q, J = 7.2 Hz, 2 H), 6.32 (s, 1 H), 6.34 (br s, 1 H), 7.44 (s, 1 H).

### Reference Example 105 5-(2,4-dichloro-5-hydroxyphenoxy)-1,4-dimethyl-1H-pyrazole-3-carboxylic acid

To a solution of ethyl 5-(2,4-dichloro-5-hydroxyphenoxy)-1,4-dimethyl-1H-pyrazole-3-carboxylate (1.04 g) obtained in Reference Example 104 in a mixed solvent of tetrahydrofuran (20 mL) and methanol (10 mL) was added 2N aqueous sodium hydroxide solution (6 mL), and the mixture was stirred at room temperature for 1 hr. Dil. hydrochloric acid was added to the reaction mixture, and the mixture was extracted with ethyl acetate. The organic layer was washed with saturated brine, dried over anhydrous magnesium sulfate and filtered, and the filtrate was concentrated. The resulting crystals were recrystallized from hexane-ethyl acetate to give the title compound as colorless crystals (850 mg, yield 89%).
¹H-NMR (300 MHz, DMSO-dₛ)δ:1.96 (s, 3 H), 3.67 (s, 3 H), 6.40 (s, 1 H), 7.64 (s, 1 H), 10.60 (br s, 1 H), 12.77 (br s, 1 H).

### Reference Example 106 5-(2,4-dichloro-5-hydroxyphenoxy)-N-ethyl-1,4-dimethyl-1H-pyrazole-3-carboxamide

To a solution of 5-(2,4-dichloro-5-hydroxyphenoxy)-1,4-dimethyl-1H-pyrazole-3-carboxylic acid (400 mg) obtained in Reference Example 105 and ethylamine hydrochloride (123 mg) in N,N-dimethylformamide (5 mL) were added 1-ethyl-3-(3-dimethylaminopropyl)carbodiimide hydrochloride (338 mg), 1-hydroxybenzotriazole (238 mg) and triethylamine (0.263 mL), and the mixture was stirred at room temperature for 3 hr. Water was added to the reaction mixture, and the mixture was extracted with ethyl acetate. The organic layer was washed with water and saturated brine, dried over anhydrous magnesium sulfate and filtered. The filtrate was concentrated, and the residue was subjected to silica gel column chromatography (hexane-ethyl acetate 7:3 - 1:1, v/v) to give the title compound as white crystals (228 mg, yield 52%).
¹H-NMR (300 MHz, DMSO-d₆)δ:1.08 (t, J = 7.2 Hz, 3 H), 1.96 (s, 3 H), 3.19 - 3.30 (m, 2 H), 3.65 (s, 3 H), 6.40 (s, 1 H), 7.60 (s, 1 H), 8.13 (t, J = 8.4 Hz, 1 H), 10.65 (br s, 1 H).

### Reference Example 107 5-chloro-2-hydroxy-4-(methoxymethoxy)benzaldehyde

To a mixture of 2,4-dihydroxybenzaldehyde (2.00 g), N-chlorosuccinimide (1.97 g) and 2-propanol (20 mL) was added concentrated hydrochloric acid (0.5 mL), and the mixture was stirred at room temperature for 4 hr and concentrated under reduced pressure. Water and ethyl acetate were added to the residue, and the mixture was stirred until complete dissolution. Two layers were separated, and the organic layer was washed with saturated brine, and dried over anhydrous magnesium sulfate. After filtration, the filtrate was concentrated, the residue was dissolved in acetone (20 mL), and the solution was cooled to 0°C. Potassium carbonate (2.81 g) was added to the mixture, and chloromethyl methyl ether (1.54 mL) was added dropwise with stirring at 0°C. The mixture was stirred at 0°C for 1 hr and at room temperature for 2 hr, and the insoluble material was removed by filtration. The filtrate was concentrated, and the residue was subjected to silica gel column chromatography (hexane-ethyl acetate 89:11 - 80:20, v/v) and crystallized from hexane-ethyl acetate to give the title compound (698 mg, yield 22%) as colorless crystals.
¹H-NMR (300 MHz, CDCl₃)δ: 3. 52 (s, 3 H), 5.31 (s, 2 H), 6.77 (s, 1 H), 7.53 (s, 1 H), 9.71 (s, 1 H), 11.28 (s, 1 H).

### Reference Example 108 2-(benzyloxy)-5-chloro-4-(methoxymethoxy)benzaldehyde

To a mixture of 5-chloro-2-hydroxy-4-(methoxymethoxy)benzaldehyde (40.00 g) obtained in Reference Example 107, potassium carbonate (34.52 g) and N,N-dimethylformamide (200 mL) was added dropwise benzyl bromide (26.4 mL) with stirring. The reaction mixture was stirred at room temperature for 3 hr and concentrated under reduced pressure. Water and ethyl acetate were added to the residue, and the mixture was stirred until complete dissolution. Two layers were separated, and the organic layer was washed with saturated brine and dried over anhydrous magnesium sulfate. After filtration, the filtrate was concentrated and the residue was crystallized from hexane-ethyl acetate to give the title compound (50.31 g, yield 89%) as colorless crystals.
¹H-NMR (300 MHz, CDCl₃)δ:3.51 (s, 3 H), 5.18 (s, 2 H), 5.28 (s, 2 H), 6.90 (s, 1 H), 7.31 - 7.47 (m, 5 H), 7.86 (s, 1 H), 10.36 (s, 1 H).

### Reference Example 109 1-(benzyloxy)-4-chloro-5-(methoxymethoxy)-2-methylbenzene

A mixture of 2-(benzyloxy)-5-chloro-4-(methoxymethoxy)benzaldehyde (10.00 g) obtained in Reference Example 108, hydrazine monohydrate (2.86 g) and diethylene glycol (40 mL) was stirred under a nitrogen atmosphere at 125°C for 30 min. Potassium hydroxide (2.20 g) was added to the reaction mixture, and the mixture was stirred at 125°C for 90 min. The reaction mixture was cooled to room temperature and poured into water, and the mixture was extracted with ethyl acetate. The organic layer was washed with saturated brine and dried over anhydrous magnesium sulfate. After filtration, the filtrate was concentrated and the residue was subjected to silica gel column chromatography (hexane-ethyl acetate 99:1 - 90:10, v/v) and crystallized from hexane to give the title compound (4.40 g, yield 46%) as colorless crystals.
¹H-NMR (300 MHz, CDCl₃)δ:2.19 (s, 3 H), 3.51 (s, 3 H), 5.05 (s, 2 H), 5.18 (s, 2 H), 6.80 (s, 1 H), 7.13 (s, 1 H), 7.28 - 7.47 (m, 5 H).

### Reference Example 110 5-(benzyloxy)-2-chloro-4-methylphenol

A mixture of 1-(benzyloxy)-4-chloro-5-(methoxymethoxy)-2-methylbenzene (3.46 g) obtained in Reference Example 109, concentrated hydrochloric acid (0.2 mL), and methanol (20 mL) was stirred at 60°C for 2 hr. The reaction mixture was cooled to room temperature and poured into water, and the mixture was extracted with ethyl acetate. The organic layer was washed with saturated brine, and dried over anhydrous magnesium sulfate. After filtration, the filtrate was concentrated and the residue was crystallized from hexane to give the title compound (2.73 g, yield 93%) as colorless crystals. ¹H-NMR (300 MHz, CDCl₃)δ:2.18 (s, 3 H), 5.03 (s, 2 H), 5.34 (s, 1 H), 6.59 (s, 1 H), 7.06 (s, 1 H), 7.28 - 7.47 (m, 5 H).

### Reference Example 111 5-[5-(benzyloxy)-2-chloro-4-methylphenoxy]-1,3-dimethyl-1H-pyrazole-4-carbaldehyde

The title compound was obtained in the same manner as in Reference Example 2 and from 5-(benzyloxy)-2-chloro-4-methylphenol obtained in Reference Example 110 and 5-chloro-1,3-dimethyl-1H-pyrazole-4-carbaldehyde.
¹H-NMR (300 MHz, CDCl₃)δ:2.23 (s, 3 H), 2.45 (s, 3 H), 3.60 (s, 3 H), 4.96 (s, 2 H), 6.44 (s, 1 H), 7.22 (d, J = 0.8 Hz, 1 H), 7.27 - 7.40 (m, 5 H), 9.29 (s, 1 H).

### Reference Example 112 5-[5-(benzyloxy)-2-chloro-4-methylphenoxy]-1,3,4-trimethyl-1H-pyrazole

The title compound was obtained in the same manner as in Reference Example 100 and then Reference Example 101, and from 5-[5-(benzyloxy)-2-chloro-4-methylphenoxy]-1,3-dimethyl-1H-pyrazole-4-carbaldehyde obtained in Reference Example 111. ¹H-NMR (300 MHz, CDCl₃)δ:1.58 (s, 3 H), 2.18 (s, 3 H), 2.21 (s, 3 H), 3.52 (s, 3 H), 4.90 (s, 2 H), 6.17 (s, 1 H), 7.18 (d, J = 0.8 Hz, 1 H), 7.25 - 7.39 (m, 5 H).

### Reference Example 113 4-chloro-2-methyl-5-[(1,3,4-trimethyl-1H-pyrazol-5-yl)oxy]phenol

5-[5-(Benzyloxy)-2-chloro-4-methylphenoxy]-1,3,4-trimethyl-1H-pyrazole (1.41 g) obtained in Reference Example 112 was dissolved in ethanol (100 mL), palladium carbon ethylenediamine complex (100 mg) was added, and the mixture was stirred under a hydrogen atmosphere (1 atm) at room temperature for 3 hr. The catalyst was removed by filtration, and the filtrate was concentrated. The residue was crystallized from toluene to give the title compound (727 mg, yield 69%) as colorless crystals.
¹H-NMR (300 MHz, CDCl₃)δ:1.77 (s, 3 H), 2.06 (s, 3 H), 2.20 (s, 3 H), 3.45 (s, 3 H), 6.08 (s, 1 H), 7.16 (s, 1 H), 10.74 (s, 1 H).

### Reference Example 114 2-(benzyloxy)-5-chloro-4-hydroxybenzaldehyde

To a solution of 2-(benzyloxy)-5-chloro-4-(methoxymethoxy)benzaldehyde (15.00 g) obtained in Reference Example 108 in methanol (100 mL) was added concentrated hydrochloric acid (1 mL), and the mixture was stirred at 60°C for 15 hr and concentrated under reduced pressure. Water and ethyl acetate were added to the residue, and the mixture was stirred until complete dissolution. Two layers were separated, and the organic layer was washed with saturated brine and dried over anhydrous magnesium sulfate. After filtration, the filtrate was concentrated and the residue was crystallized from hexane-ethyl acetate to give the title compound (12.32 g, yield 96%) as pale-orange crystals.
¹H-NMR (300 MHz, CDCl₃)δ:5.15 (s, 2 H), 6.09 (s, 1 H), 6.71 (s, 1 H), 7.29 - 7.50 (m, 5 H), 7.86 (s, 1 H), 10.33 (s, 1 H).

### Reference Example 115 5-(benzyloxy)-2-chloro-4-(1,3-dioxolan-2-yl)phenol

A mixture of 2-(benzyloxy)-5-chloro-4-hydroxybenzaldehyde (5.00 g) obtained in Reference Example 114, ethylene glycol (2.37 g), p-toluenesulfonic acid monohydrate (18 mg) and toluene (50 mL) was heated under reflux for 15 hr while removing generated water. The reaction mixture was cooled to room temperature and poured into aqueous sodium hydrogen carbonate solution, and the mixture was extracted with ethyl acetate. The organic layer was washed with saturated brine and dried over anhydrous magnesium sulfate. After filtration, the filtrate was concentrated and the residue was crystallized from hexane-toluene to give the title compound (5.04 g, yield 86%) as pale-yellow crystals.
¹H-NMR (300 MHz, CDCl₃)δ:3.96 - 4.15 (m, 4 H), 5.09 (s, 2 H), 5.56 (s, 1 H), 6.12 (s, 1 H), 6.63 (s, 1 H), 7.27 - 7.45 (m, 5 H), 7.48 (s, 1 H).

### Reference Example 116 5-[5-(benzyloxy)-2-chloro-4-(1,3-dioxolan-2-yl)phenoxy]-1,3-dimethyl-1H-pyrazole-4-carbaldehyde

The title compound was obtained in the same manner as in Reference Example 2 and from 5-(benzyloxy)-2-chloro-4-(1,3-dioxolan-2-yl)phenol obtained in Reference Example 115 and 5-chloro-1,3-dimethyl-1H-pyrazole-4-carbaldehyde.
¹H-NMR (300 MHz, CDCl₃)δ:2.46 (s, 3 H), 3.52 (s, 3 H), 3.98 - 4.19 (m, 4 H), 5.01 (s, 2 H), 6.13 (s, 1 H), 6.35 (s, 1 H), 7.22 - 7.38 (m, 5 H), 7.63 (s, 1 H), 9.40 (s, 1 H).

### Reference Example 117 5-[5-(benzyloxy)-2-chloro-4-(1,3-dioxolan-2-yl)phenoxy]-1,3,4-trimethyl-1H-pyrazole

To a mixture of 5-[5-(benzyloxy)-2-chloro-4-(1,3-dioxolan-2-yl)phenoxy]-1,3-dimethyl-1H-pyrazole-4-carbaldehyde (3.67 g) obtained in Reference Example 116 and diethylene glycol (15 mL) was added hydrazine monohydrate (750 mg), and the mixture was stirred with heating at 125°C for 15 min. Potassium hydroxide (577 mg) was further added, and the mixture was stirred with heating at 125°C for 1.5 hr. The reaction mixture was cooled to room temperature and poured into water, and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 82:18 - 50:50, v/v) and crystallized from hexane-diisopropyl ether to give the title compound (1.78 g, yield 50%) as pale-yellow crystals.
¹H-NMR (300 MHz, CDCl₃)δ:1.55 (s, 3 H), 2.19 (s, 3 H), 3.48 (s, 3 H), 4.00 - 4.18 (m, 4 H), 4.96 (s, 2 H), 6.13 (s, 1 H), 6.16 (s, 1 H), 7.21 - 7.43 (m, 5 H), 7.59 (s, 1 H).

### Reference Example 118 4-chloro-2-(1,3-dioxolan-2-yl)-5-[(1,3,4-trimethyl-1H-pyrazol-5-yl)oxy]phenol

The title compound was obtained in the same manner as in Reference Example 113 and from 5-[5-(benzyloxy)-2-chloro-4-(1,3-dioxolan-2-yl)phenoxy]-1,3,4-trimethyl-1H-pyrazole obtained in Reference Example 117.
¹H-NMR (300 MHz, CDCl₃)δ:1.77 (s, 3 H), 2.13 (s, 3 H), 3.52 (s, 3 H), 4.02 - 4.20 (m, 4 H), 5.95 (s, 1 H), 6.18 (s, 1 H), 7.39 (s, 1 H), 9.06 (s, 1 H).

### Reference Example 119 5-chloro-2-hydroxy-4-[(1,3,4-trimethyl-1H-pyrazol-5-yl)oxy]benzaldehyde

To a solution of 4-chloro-2-(1,3-dioxolan-2-yl)-5-[(1,3,4-trimethyl-1H-pyrazol-5-yl)oxy]phenol (971 mg) obtained in Reference Example 118 in acetone (50 mL) was added 1N hydrochloric acid (10 mL), and the mixture was stirred at 60°C for 3 hr and concentrated under reduced pressure. Water and ethyl acetate were added to the residue, and the mixture was stirred until complete dissolution. Two layers were separated, and the organic layer was washed with saturated brine and dried over anhydrous magnesium sulfate. After filtration, the filtrate was concentrated and the residue was subjected to silica gel column chromatography (hexane-ethyl acetate 80:20 - 50:50, v/v), and crystallized from hexane-ethyl acetate to give the title compound (581 mg, yield 69%) as colorless crystals.
¹H-NMR (300 MHz, CDCl₃)δ:1.79 (s, 3 H), 2.20 (s, 3 H), 3.58 (s, 3 H), 6.27 (s, 1 H), 7.64 (s, 1 H), 9.77 (s, 1 H), 11.25 (s, 1 H).

### Reference Example 120 5-chloro-2-hydroxy-4-[(1,3,4-trimethyl-1H-pyrazol-5-yl)oxy]benzoic acid

Potassium permanganate (2.12 g) was added to a solution of 5-chloro-2-hydroxy-4-[(1,3,4-trimethyl-1H-pyrazol-5-yl)oxy]benzaldehyde (3.41 g) obtained in Reference Example 119 and potassium hydroxide (1.65 g) in water (100 mL) with stirring. The mixture was stirred at room temperature for 2 hr, and the resulting precipitate was removed by filtration. 1N Hydrochloric acid (30 mL) was added to the filtrate, and the mixture was extracted with ethyl acetate. The organic layer was washed with saturated brine, and dried over anhydrous magnesium sulfate. After filtration, the filtrate was concentrated and the residue was crystallized from hexane-ethyl acetate to give the title compound as colorless crystals (962 mg, yield 27%).
¹H-NMR (300 MHz, CDCl₃)δ:1.80 (s, 3 H), 2.24 (s, 3 H), 3.64 (s, 3 H), 6.31 (s, 1 H), 8.02 (s, 1 H), 11.10 (br s, 1 H).

### Reference Example 121 5-chloro-2-hydroxy-4-[(1,3,4-trimethyl-1H-pyrazol-5-yl)oxy]benzamide

To a solution of 5-chloro-2-hydroxy-4-[(1,3,4-trimethyl-1H-pyrazol-5-yl)oxy]benzoic acid (250 mg) obtained in Reference Example 120 in N,N-dimethylformamide (3 mL) were added N-ethyl-N'-(3-dimethylaminopropyl)carbodiimide hydrochloride (178 mg) and 1-hydroxybenzotriazole ammonia complex (141 mg), and the mixture was stirred at room temperature for 15 hr. Dil. hydrochloric acid was added to the reaction mixture, and the mixture was extracted with ethyl acetate. The organic layer was washed successively with aqueous sodium hydrogen carbonate solution and saturated brine, and dried over anhydrous magnesium sulfate. After filtration, the filtrate was concentrated and the residue was crystallized from hexane-ethyl acetate to give the title compound (115 mg, yield 46%) as colorless crystals.
¹H-NMR (300 MHz, DMSO-d₆)δ:1.71 (s, 3 H), 2.09 (s, 3 H), 3.49 (s, 3 H), 6.11 (s, 1 H), 8.01 (s, 1 H), 8.15 (s, 1 H), 8.38 (s, 1 H), 13.30 (s, 1 H).

### Reference Example 122 5-chloro-2-hydroxy-N,N-dimethyl-4-[(1,3,4-trimethyl-1H-pyrazol-5-yl)oxy]benzamide

To a solution of 5-chloro-2-hydroxy-4-[(1,3,4-trimethyl-1H-pyrazol-5-yl)oxy]benzoic acid (200 mg) obtained in Reference Example 120 in N,N-dimethylformamide (2 mL) were added dimethylamine (2N tetrahydrofuran solution, 0.404 mL), N-ethyl-N'-(3-dimethylaminopropyl)carbodiimide hydrochloride (142 mg) and 1-hydroxybenzotriazole (113 mg), and the mixture was stirred at room temperature for 15 hr. Dil. hydrochloric acid was added to the reaction mixture, and the mixture was extracted with ethyl acetate. The organic layer was washed successively with aqueous sodium hydrogen carbonate solution and saturated brine, and dried over anhydrous magnesium sulfate. After filtration, the filtrate was concentrated and the residue was crystallized from hexane-ethyl acetate to give the title compound (116 mg, yield 53%) as colorless crystals.
¹H-NMR (300 MHz, CDCl₃)δ:1.79 (s, 3 H), 2.18 (s, 3 H), 3.18 (s, 6 H), 3.57 (s, 3 H), 6.30 (s, 1 H), 7.43 (s, 1 H), 10.61 (s, 1 H).

### Reference Example 123 2-methyl-2,3a,4,5,6,7-hexahydro-3H-indazol-3-one

To a solution of ethyl 2-cyclohexanone carboxylate (54.16 g) in toluene (500 mL) was added methylhydrazine (14.66 g), and the mixture was stirred at 100°C for 3 hr. After cooling to room temperature, the reaction mixture was concentrated. The residue was crystallized from hexane-ethyl acetate to give the title compound (41.62 g, yield 86%) as pale-brown crystals.
¹H-NMR (300 MHz, CDCl₃)δ:1.22 - 1.69 (m, 3 H), 1.85 - 1.97 (m, 1 H), 2.02 - 2.18 (m, 1 H), 2.19 - 2.37 (m, 1 H), 2.40 - 2.54 (m, 1 H), 2.57 - 2.70 (m, 1 H), 2.84 - 2.96 (m, 1 H), 3.29 (s, 3 H).

### Reference Example 124 3-bromo-2-methyl-4,5,6,7-tetrahydro-2H-indazole

To a solution of 2-methyl-2,3a,4,5,6,7-hexahydro-3H-indazol-3-one (6.64 g) obtained in Reference Example 123 in toluene (100 mL) was added phosphorus oxybromide (25.00 g), and the mixture was stirred at 100°C for 14 hr. The reaction mixture was cooled to room temperature, and ice water was added. Furthermore, sodium hydrogen carbonate was gradually added until the development of gas stopped, and the mixture was extracted with ethyl acetate. The organic layer was washed with saturated brine, and dried over anhydrous magnesium sulfate. After filtration, the filtrate was concentrated, the residue was subjected to silica gel column chromatography (hexane-ethyl acetate 82:18 - 33:67, v/v), and the solvent was evaporated to give the title compound (1.60 g, yield 17%) as a pale-brown oil.
¹H-NMR (300 MHz, CDCl₃)δ:1.68 - 1.87 (m, 4 H), 2.38 (t, J = 5.8 8 Hz, 2 H), 2.62 (t, J = 5.9 Hz, 2 H), 3.80 (s, 3 H).

### Reference Example 125 2,4-dichloro-5-(hydroxymethyl)phenol

2-Hydroxybenzyl alcohol (100 g) was added to a flask (1 L), and ethyl acetate (500 mL) was added. The flask was cooled with ice water, and the mixture was stirred for 20 min. Sulfuryl chloride (130 mL) was added dropwise at 0°C over 1.5 hr. After the completion of the dropwise addition, the mixture was stirred at room temperature for 2 hr. Water (300 mL) was added to the reaction mixture, and the mixture was extracted with ethyl acetate (250 mL x 3). The organic layer was washed with saturated brine, and dried over magnesium sulfate. The solvent was evaporated, and the residue was stood overnight as it was. The precipitated crystals were collected by filtration, and washed with cooled diisopropyl ether to give the title compound (40 g, yield 26%) as milk-white crystals.
¹H-NMR (300 MHz, DMSO-d₆)δ:4.44 (s, 2 H), 5.44 (br, 1 H), 7.19 (s, 1 H), 7.34 (s, 1 H), 10.40 (s, 1 H).

### Reference Example 126 [5-(benzyloxy)-2,4-dichlorophenyl]methanol

To a mixture of 2,4-dichloro-5-(hydroxymethyl)phenol (20.00 g) obtained in Reference Example 125, potassium carbonate (18.69 g) and N,N-dimethylformamide (100 mL) was added dropwise benzyl bromide (14.8 mL) with stirring. The reaction mixture was stirred at room temperature for 15 hr, water was added, and the mixture was extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 90:10 - 75:25, v/v), and crystallized from hexane-diisopropyl ether to give the title compound (4.97 g, yield 17%) as colorless crystals.
¹H-NMR (300 MHz, CDCl₃)δ:1-90 (t, J=6.1 Hz, 1 H), 4.72 (d, J=5.8 Hz, 2 H), 5.17 (s, 2 H), 7.17 (s, 1 H), 7.27 - 7.50 (m, 6 H) .

### Reference Example 127 5-(benzyloxy)-2,4-dichlorobenzaldehyde

A mixture of [5-(benzyloxy)-2,4-dichlorophenyl]methanol (19.24 g) obtained in Reference Example 126, manganese dioxide (59.07 g) and tetrahydrofuran (300 mL) was stirred at room temperature for 15 hr. The insoluble material was removed by filtration, and the filtrate was concentrated. The residue was crystallized from hexane-diisopropyl ether to give the title compound (14.35 g, yield 75%) as colorless crystals. ¹H-NMR (300 MHz, CDCl₃)δ:5.20 (s, 2 H), 7.30 - 7.50 (m, 6 H), 7.51 (s, 1 H), 10.37 (s, 1 H).

### Reference Example 128 [5-(benzyloxy)-2,4-dichlorophenyl] (2-methyl-4,5,6,7-tetrahydro-2H-indazol-3-yl)methanol

To a solution of 3-bromo-2-methyl-4,5,6,7-tetrahydro-2H-indazole (1.58 g) obtained in Reference Example 124 in tetrahydrofuran (15 mL) was added dropwise n-butyllithium (1.6M hexane solution, 5.1 mL) under an argon stream at -78°C, and the mixture was stirred for 90 min. To the reaction solution was added 5-(benzyloxy)-2,4-dichlorobenzaldehyde (2.07 g) obtained in Reference Example 127, and the mixture was gradually allowed to warm to room temperature and stirred at room temperature for 90 min. Water was added to the reaction mixture, and the mixture was extracted with ethyl acetate. The organic layer was washed with water and saturated brine, dried over anhydrous magnesium sulfate and filtered. The filtrate was concentrated, and the residue was crystallized from hexane-ethyl acetate to give the title compound (2.58 g, yield 84%) as colorless crystals.
¹H-NMR (300 MHz, CDCl₃)δ:1.43 - 1.77 (m, 5 H), 1.90 - 2.03 (m, 1 H), 2.51 - 2.63 (m, 3 H), 3.72 (s, 3 H), 5.14 - 5.31 (m, 2 H), 5.99 (s, 1 H), 7.27 - 7.46 (m, 7 H).

### Reference Example 129 2,4-dichloro-5-[(2-methyl-4,5,6,7-tetrahydro-2H-indazol-3-yl)methyl]phenol

A mixture of [5-(benzyloxy)-2,4-dichlorophenyl] (2-methyl-4,5,6,7-tetrahydro-2H-indazol-3-yl)methanol (2.45 g) obtained in Reference Example 128, triethylsilane (11.3 mL) and trifluoroacetic acid (6 mL) was stirred at 70°C for 5 days, and cooled to room temperature. Aqueous sodium hydrogen carbonate solution was gradually added to the reaction mixture until the development of gas stopped, diisopropyl ether was further added, and the mixture was stirred at room temperature for 15 min. The resulting crystals were collected by filtration and washed with water and diisopropyl ether to give the title compound (1.19 g, yield 65%) as colorless crystals. ¹H-NMR (300 MHz, DMSO-d₆)δ:1.54 - 1.75 (m, 4 H), 2.20 (t, J = 5.9 Hz, 2 H), 2.45 - 2.53 (m, 2 H), 3.58 (s, 3 H), 3.90 (s, 2 H), 6.57 (s, 1 H), 7.48 (s, 1 H), 10.38 (s, 1 H).

### Reference Example 130 5-[(2,4-dichloro-5-hydroxyphenyl)thio]-1,3-dimethyl-1H-pyrazole-4-carbaldehyde

To a solution, which was cooled to 0°C in an ice bath, of 5-[(3-hydroxyphenyl)thio]-1,3-dimethyl-1H-pyrazole-4-carbaldehyde (17.23 g) obtained in Reference Example 90 in dichloromethane (200 mL) was added dropwise sulfuryl chloride (18.76 g), and the mixture was stirred at 0°C for 30 min. The solvent was evaporated to give crude crystals containing the title compound (22 g). A part (6.2 g) of the obtained crude crystals were purified by preparative HPLC using "apparatus: High Throughput purification system manufactured by Gilson Inc. column: YMC Combiprep ODS-A, S-5 µm, 50 x 20 mm solvent: SOLUTION A; 0.1% trifluoroacetic acid containing water, SOLUTION B; 0.1% trifluoroacetic acid containing acetonitrile, gradient cycle: 0.00 min (SOLUTION A/SOLUTION B = 90/10), 1.00 min (SOLUTION A/SOLUTION B = 90/10), 4.20 min (SOLUTION A/SOLUTION B = 10/90), 5.40 min (SOLUTION A/SOLUTION B = 10/90), 5.50 min (SOLUTION A/SOLUTION B = 90/10), 5.60 min (SOLUTION A/SOLUTION B = 90/10), flow rate: 25 mL/min, detection method: UV 220 nm" to give the title compound (3.1 g).
¹H-NMR (300 MHz, CDCl₃)δ:2. 47 (s, 3 H), 3.81 (s, 3 H), 6.19 (s, 1 H), 7.11 (br, 1 H), 7.39 (s, 1 H), 9.92 (s, 1 H).

### Reference Example 131 5-(5-bromo-2,4-dichlorophenoxy)-1,3-dimethyl-1H-pyrazole-4-carbaldehyde

To a solution (50 mL) of tert-butyl nitrite (1.8 mL) and copper bromide (2.68 g) in acetonitrile was added 5-(5-amino-2,4-dichlorophenoxy)-1,3-dimethyl-1H-pyrazole-4-carbaldehyde (3.0 g) obtained in Reference Example 56 at 0°C, and the mixture was stirred for 1 hr and concentrated under reduced pressure. The residue was subjected to silica gel column chromatography (hexane-ethyl acetate 4:1 - 3:1, v/v), and the obtained oil was crystallized from hexane-ethyl acetate to give the title compound (2.81 g, yield 77%) as colorless crystals.
¹H-NMR (300 MHz, CDCl₃)δ:2.48 (s, 3 H), 3.70 (s, 3 H), 7.09 (s, 1 H), 7.60 (s, 1 H), 9.57 (s, 1 H).

### Reference Example 132 4-chloro-3-[(1,3,4-trimethyl-1H-pyrazol-5-yl)oxy]benzaldehyde oxime

To a solution (20 mL) of 4-chloro-3-[(1,3,4-trimethyl-1H-pyrazol-5-yl)oxy]benzaldehyde (234 mg) obtained in Reference Example 64 in tetrahydrofuran were added a solution (1 mL) of hydroxylamine hydrochloride (77 mg) in water, and sodium carbonate (118 mg), and the mixture was stirred at room temperature for 1 hr. Water was added to the reaction mixture, and the mixture was extracted with ethyl acetate. The organic layer was washed with saturated brine, and dried over anhydrous magnesium sulfate. After filtration, the filtrate was concentrated and the residue was crystallized from hexane-diethyl ether to give the title compound (245 mg, yield 99%) as colorless crystals.
¹H-NMR (300 MHz, CDCl₃)δ:1.75 (s, 3 H), 2.19 (s, 3 H), 3.61 (s, 3 H), 7.04 (d, J = 1. 7 Hz, 1 H), 7.14 (dd, J = 8 .1, 1. 7 Hz, 1 H), 7.45 (d, J = 8.1 Hz, 1 H), 8.02 (s, 1 H), 9.42 (s, 1 H).

### Reference Example 133 4-chloro-3-[(1,3,4-trimethyl-1H-pyrazol-5-yl)oxy]benzonitrile

To a suspension (5 mL) of 4-chloro-3-[(1,3,4-trimethyl-1H-pyrazol-5-yl)oxy]benzaldehyde oxime (207 mg) obtained in Reference Example 132 and triethylamine (0.21 mL) in tetrahydrofuran was added trichloroacetyl chloride (0.091 mL), and the mixture was stirred at 60°C for 3 hr. Water was added to the reaction mixture, and the mixture was extracted with ethyl acetate. The organic layer was dried over anhydrous magnesium sulfate and filtered. The filtrate was concentrated, and the residue was subjected to silica gel column chromatography (hexane-ethyl acetate 9:1 - 2:1, v/v), and crystallized from hexane-ethyl acetate to give the title compound (180 mg, yield 93%) as a pale-yellow oil. Recrystallization from hexane-ethyl acetate gave colorless crystals.
¹H-NMR (300 MHz, CDCl₃)δ:1.76 (s, 3 H), 2.21 (s, 3 H), 3.59 (s, 3 H), 6.93 (d, J=1.9Hz, 1 H), 7.34 (dd, J=8.3, 1.9Hz, 1 H), 7.58 (d, J = 8.3 Hz, 1 H).

### Reference Example 134 [5-(5-bromo-2,4-dichlorophenoxy)-1,3-dimethyl-1H-pyrazol-4-yl]methanol

The title compound was obtained in the same manner as in Reference Example 100 and from 5-(5-bromo-2,4-dichlorophenoxy)-1,3-dimethyl-1H-pyrazole-4-carbaldehyde obtained in Reference Example 131.
¹H-NMR (300 MHz, CDCl₃)δ:1.31 (t, J = 4.7 Hz, 1 H), 2.30 (s, 3 H), 3.61 (s, 3 H), 4.33 (d, J = 4.7 Hz, 2 H), 7.07 (s, 1 H), 7.56 (s, 1 H).

### Reference Example 135 3-chloro-4-[(1,3,4-trimethyl-1H-pyrazol-5-yl)oxy]benzaldehyde

The title compound was obtained in the same manner as in Reference Example 64 and from 5-(4-bromo-2-chlorophenoxy)-1,3,4-trimethyl-1H-pyrazole obtained in Reference Example 101.
¹H-NMR (300 MHz, CDCl₃)δ:1.77 (s, 3 H), 2.21 (s, 3 H), 3.59 (s, 3 H), 6.82 (d, J = 8.5 Hz, 1 H), 7.70 (dd, J = 8.5, 2.1 Hz, 1 H), 8.01 (d, J = 2.1 Hz, 1 H), 9.91 (s, 1 H).

### Reference Example 136 5-(5-bromo-2,4-dichlorophenoxy)-1,3,4-trimethyl-1H-pyrazole

The title compound was obtained in the same manner as in Reference Example 60 and from [5-(5-bromo-2,4-dichlorophenoxy)-1,3-dimethyl-1H-pyrazol-4-yl]methanol obtained in Reference Example 134.
¹H-NMR (300 MHz, CDCl₃)δ:1.31 (t, J = 4.7 Hz, 1 H), 2.30 (s, 3 H), 3.61 (s, 3 H), 4.33 (d, J = 4.7 Hz, 2 H), 7.07 (s, 1 H), 7.56 (s, 1 H).

### Reference Example 137 5-[(5-bromo-2,4-dichlorophenyl)thio]-1,3-dimethyl-1H-pyrazole-4-carbaldehyde

1-Bromo-2,4-dichlorobenzene (10 g) was added to a mixture of chlorosulfonic acid (30 mL) and thionyl chloride (3 mL) at 0°C, and the mixture was stirred at room temperature for 3 hr. The reaction solution was carefully added to ice water and ethyl acetate. Two layers were separated, and the organic layer was washed with saturated brine, and dried over anhydrous magnesium sulfate. After filtration, the filtrate was concentrated. The obtained oil was dissolved in acetic acid (100 mL), zinc (4.0 g) was added, and the mixture was stirred with heating at 120°C for 30 min. The reaction solution was filtered, and the filtrate was concentrated to give pale-yellow crystals. To a solution of the obtained crystals and 5-chloro-1,3-dimethyl-1H-pyrazole-4-carbaldehyde (7.0 g) in N,N-dimethylformamide (100 mL) was added potassium carbonate (13.8 g) with stirring. The reaction mixture was stirred under a nitrogen atmosphere at 100°C for 2 hr. The reaction mixture was cooled to room temperature, and the reaction solution was added to water and ethyl acetate. Two layers were separated, and the organic layer was washed with saturated brine, and dried over anhydrous magnesium sulfate. After filtration, the filtrate was concentrated and the residue was dissolved in a small amount of toluene. The solution was subjected to silica gel column chromatography (hexane-ethyl acetate 90:10 - 75:25, v/v), and crystallized from hexane-ethyl acetate to give the title compound (2.92 g, yield 17%) as colorless crystals.
¹H-NMR (300 MHz, CDCl₃)δ:2.56 (s, 3 H), 3.85 (s, 3 H), 6.81 (s, 1 H), 7.51 (s, 1 H), 9.96 (s, 1 H).

### Reference Example 138 {5-[(5-bromo-2,4-dichlorophenyl)thio]-1,3-dimethyl-1H-pyrazol-4-yl}methanol

The title compound was obtained in the same manner as in Reference Example 100 and from 5-[(5-bromo-2,4-dichlorophenyl)thio]-1,3-dimethyl-1H-pyrazole-4-carbaldehyde obtained in Reference Example 137.
¹H-NMR (300 MHz, CDCl₃)δ:1.51 (br s, 1 H), 2.39 (s, 3 H), 3.81 (s, 3 H), 4.51 - 4.60 (m, 2 H), 6.67 (s, 1 H), 7.47 (s, 1 H).

### Reference Example 139 3-chloro-4-[(1,3,4-trimethyl-1H-pyrazol-5-yl)oxy]benzonitrile

The title compound was obtained in the same manner as in Reference Examples 132 and 133 and from 3-chloro-4-[(1,3,4-trimethyl-1H-pyrazol-5-yl)oxy]benzaldehyde obtained in Reference Example 135.
¹H-NMR (300 MHz, CDCl₃)δ:1.76 (s, 3 H), 2.20 (s, 3 H), 3.59 (s, 3 H), 6.77 (d, J = 8.5 Hz, 1 H), 7.49 (dd, J = 8.5, 2.0 Hz, 1 H), 7.78 (d, J = 2.0 Hz, 1 H).

### Reference Example 140 5-[(5-bromo-2,4-dichlorophenyl)thio]-1,3,4-trimethyl-1H-pyrazole

The title compound was obtained in the same manner as in Reference Example 60 and from {5-[(5-bromo-2,4-dichlorophenyl)thio]-1,3-dimethyl-1H-pyrazol-4-yl}methanol obtained in Reference Example 138.
¹H-NMR (300 MHz, CDCl₃)δ:2.00 (s, 3 H), 2.28 (s, 3 H), 3.79 (s, 3 H), 6.58 (s, 1 H), 7.46 (s, 1 H).

### Reference Example 141 2,4-dichloro-5-[(1,3,4-trimethyl-1H-pyrazol-5-yl)thio]benzaldehyde

The title compound was obtained in the same manner as in Reference Example 64 and from 5-[(5-bromo-2,4-dichlorophenyl)thio]-1,3,4-trimethyl-1H-pyrazole obtained in Reference Example 140.
¹H-NMR (300 MHz, CDCl₃)δ:1.99 (s, 3 H), 2.28 (s, 3 H), 3.78 (s, 3 H), 6.99 (s, 1 H), 7.51 (s, 1 H), 10.28 (s, 1 H).

### Reference Example 142 2,4-dichloro-5-[(1,3,4-trimethyl-1H-pyrazol-5-yl)oxy]benzonitrile

A solution (10 mL) of 5-(5-bromo-2,4-dichlorophenoxy)-1,3,4-trimethyl-1H-pyrazole (700 mg) obtained in Reference Example 136, zinc cyanide (129 mg) and tetrakistriphenylphosphine palladium (116 mg) in N,N-dimethylformamide was stirred under an argon stream at 80°C for 15 hr. Water was added to the reaction mixture, and the mixture was extracted with ethyl acetate. The organic layer was washed with water and saturated brine, dried over anhydrous magnesium sulfate and filtered. The filtrate was concentrated, and the residue was recrystallized from hexane-ethyl acetate to give the title compound (250 mg, yield 42%).
¹H-NMR (300 MHz, CDCl₃)δ:1.76 (s, 3 H), 2.21 (s, 3 H), 3.59 (s, 3 H), 6.94 (s, 1 H), 7.64 (s, 1 H).

### Reference Example 143 2,4-dichloro-5-[(1,3,4-trimethyl-1H-pyrazol-5-yl)oxy]aniline

The title compound was obtained in the same manner as in Reference Example 117 and from 5-(5-amino-2,4-dichlorophenoxy)-1,3-dimethyl-1H-pyrazole-4-carbaldehyde obtained in Reference Example 56.
¹H-NMR (300 MHz, CDCl₃)δ:1.77 (s, 3 H), 2.18 (s, 3 H), 3.57 (s, 3 H), 4.07 (br s, 2H), 6.06 (s, 1 H), 7.31 (s, 1 H).

### Reference Example 144 {4-chloro-3-[(1,3,4-trimethyl-1H-pyrazol-5-yl)oxy]phenyl}methanol

The title compound was obtained in the same manner as in Reference Example 100 and from 4-chloro-3-[(1,3,4-trimethyl-1H-pyrazol-5-yl)oxy]benzaldehyde obtained in Reference Example 64.
¹H-NMR (300 MHz, CDCl₃)δ:1.75 (s, 3 H), 2.12 (t, J = 5.3 Hz, 1 H), 2.17 (s, 3 H), 3.57 (s, 3 H), 4.59 (d, J = 5.3 Hz, 2 H), 6.71 (d, J = 1.9 Hz, 1 H), 7.03 (dd, J = 8.0, 1.9 Hz, 1 H), 7.42 (d, J = 8.0 Hz, 1 H).

### Reference Example 145 5-[5-(bromomethyl)-2-chlorophenoxy]-1,3,4-trimethyl-1H-pyrazole

A solution (15 mL) of {4-chloro-3-[(1,3,4-trimethyl-1H-pyrazol-5-yl)oxy]phenyl}methanol (880 mg) obtained in Reference Example 144, carbon tetrabromide (1.42 g) and triphenylphosphine (1.13 g) in dichloromethane was stirred at room temperature for 1 hr. The reaction mixture was concentrated, and the residue was subjected to silica gel column chromatography (hexane-ethyl acetate 9:1 - 3:1, v/v) to give the title compound as a colorless oil (913 mg, yield 84%). Recrystallization from hexane-ethyl acetate gave colorless crystals.
¹H-NMR (300 MHz, CDCl₃)δ:1.75 (s, 3 H), 2.21 (s, 3 H), 3.60 (s, 3 H), 4.34 (s, 2 H), 6.70 (d, J = 1.9 Hz, 1H), 7.06 (dd, J = 8.0, 1.9 Hz), 7.41 (d, J = 8.0 Hz, 1 H).

### Reference Example 146 {4-chloro-3-[(1,3,4-trimethyl-1H-pyrazol-5-yl)oxy]phenyl}acetonitrile

To a solution (10 mL) of 5-[5-(bromomethyl)-2-chlorophenoxy]-1,3,4-trimethyl-1H-pyrazole (330 mg) obtained in Reference Example 145 and trimethylsilyl cyanide (0.20 mL) in acetonitrile was added tetrabutylammonium fluoride (1.0M tetrahydrofuran solution, 1.5 mL) at room temperature, and the mixture was stirred for 30 min. The reaction mixture was concentrated, and the residue was subjected to silica gel column chromatography (hexane-ethyl acetate 1:1, v/v) to give the title compound as a colorless oil (248 mg, yield 90%). Recrystallization from hexane-ethyl acetate gave colorless crystals.
¹H-NMR (300 MHz, CDCl₃)δ:1.76 (s, 3 H), 2.20 (s, 3 H), 3.59 (s, 3 H), 3.64 (s, 2 H), 6.61 (d, J = 1.9 Hz, 1 H), 7.05 (dd, J = 8.3, 1.9 Hz, 1 H), 7.47 (d, J = 8.3 Hz, 1 H).

### Reference Example 147 2-{4-chloro-3-[(1,3,4-trimethyl-1H-pyrazol-5-yl)oxy]phenyl}-N'-hydroxyacetimidamide

Hydroxylamine hydrochloride (417 mg) and sodium hydrogen carbonate (615 mg) were stirred in dimethyl sulfoxide (4 mL) at 40°C for 1 hr. {4-Chloro-3-[(1,3,4-trimethyl-1H-pyrazol-5-yl)oxy]phenyl}acetonitrile (414 mg) obtained in Reference Example 146 was added to the reaction mixture, and the mixture was stirred at 80°C for 4 hr. Water was added to the reaction mixture, and the mixture was extracted with ethyl acetate. The organic layer was washed with water and saturated brine, dried over anhydrous magnesium sulfate and filtered. The filtrate was concentrated, and the residue was recrystallized from hexane-ethyl acetate to give the title compound as colorless crystals (449 mg, yield 97%).
¹H-NMR (300 MHz, CDCl₃)δ:1.73 (s, 3 H), 2.18 (s, 3 H), 3.33 (s, 2 H), 3.58 (s, 3 H), 4.44 (br s, 2 H), 6.62 (d, J = 1.9 Hz, 1H), 6.96 (dd, J = 8.1, 1.9 Hz), 7.39 (d, J = 8.1 Hz, 1 H).

### Reference Example 148 (2E)-3-{4-chloro-3-[(1,3,4-trimethyl-1H-pyrazol-5-yl)oxy]phenyl}acrylonitrile

To a solution (20 mL) of diethyl (cyanomethyl)phosphonate (0.402 mL) in tetrahydrofuran was added sodium hydride (60% in oil, 110 mg) at room temperature, and the mixture was stirred for 30 min. 4-Chloro-3-[(1,3,4-trimethyl-1H-pyrazol-5-yl)oxy]benzaldehyde (560 mg) obtained in Reference Example 64 was added to the reaction mixture, and the mixture was stirred for 30 min. Water was added to the reaction mixture, and the mixture was extracted with ethyl acetate. The organic layer was washed with water and saturated brine, dried over anhydrous magnesium sulfate and filtered. The filtrate was concentrated, and the residue was subjected to silica gel column chromatography (hexane-ethyl acetate 4:1, v/v) to give the title compound as a colorless oil (426 mg, yield 70%).
¹H-NMR (300 MHz, CDCl₃)δ:1.75 (s, 3 H), 2.21 (s, 3 H), 3.60 (s, 3 H), 5.77 (d, J = 16.7 Hz, 1 H), 6.71 (d, J = 1.9 Hz, 1H), 7.15 (dd, J = 8.3, 1.9 Hz, 1 H), 7.24 (d, J = 16.7 Hz, 1 H), 7.50 (d, J = 8.3 Hz, 1 H).

### Reference Example 149 4-chloro-2-(1-hydroxyethyl)-5-[(1,3,4-trimethyl-1H-pyrazol-5-yl)oxy]phenol

To a solution of 5-chloro-2-hydroxy-4-[(1,3,4-trimethyl-1H-pyrazol-5-yl)oxy]benzaldehyde (560 mg) obtained in Reference Example 119 in tetrahydrofuran (15 mL) was added methylmagnesium bromide (1.0M tetrahydrofuran solution, 6.0 mL) under an argon stream at -40°C, and the mixture was stirred for 2 hr and further at -20°C for 1 hr. Saturated aqueous ammonium chloride solution was added to the reaction mixture, and the mixture was extracted with ethyl acetate. The organic layer was washed with water and saturated brine, dried over anhydrous magnesium sulfate and filtered. The filtrate was concentrated, and the residue was recrystallized from hexane-ethyl acetate to give the title compound as colorless crystals (561 mg, yield 94%).
¹H-NMR (300 MHz, CDCl₃)δ:1.57 (d, J = 6.6 Hz, 3 H), 1.77 (s, 3 H), 2.10 (s, 3 H), 2.88 (d, J = 4.9 Hz, 1 H), 3.49 (s, 3 H), 4.97 - 5.13 (m, 1 H), 6.13 (s, 1 H), 7.22 (s, 1 H), 10.19 (br s, 1 H).

### Reference Example 150 1-{5-chloro-2-hydroxy-4-[(1,3,4-trimethyl-1H-pyrazol-5-yl)oxy]phenyl}ethanone

To a solution (25 mL) of 4-chloro-2-(1-hydroxyethyl)-5-[(1,3,4-trimethyl-1H-pyrazol-5-yl)oxy]phenol (890 mg) obtained in Reference Example 149 in tetrahydrofuran was added manganese dioxide (4.0 g), and the mixture was stirred at room temperature for 18 hr and further at 50°C for 8 hr. The reaction mixture was filtered through celite, and the filtrate was concentrated. The residue was subjected to silica gel column chromatography (hexane-ethyl acetate 9:1 - 2:1, v/v) to give the title compound as a pale-yellow oil (579 mg, yield 66%). Recrystallization from hexane-ethyl acetate gave colorless crystals.
¹H-NMR (300 MHz, CDCl₃)δ:1.78 (s, 3 H), 2.19 (s, 3 H), 2.61 (s, 3 H), 3.57 (s, 3 H), 6.26 (s, 1 H), 7.81 (s, 1 H), 12.46 (s, 1 H).

### Reference Example 151 ethyl (2E)-3-[5-(2,4-dichloro-5-hydroxyphenoxy)-1,3-dimethyl-1H-pyrazol-4-yl]acrylate

While stirring a solution of ethyl (diethoxyphosphoryl)acetate (1.494 g) cooled to 0°C in an ice bath in tetrahydrofuran (8 mL), sodium hydride (291 mg) was added, and the mixture was stirred at 0°C for 30 min. To the reaction mixture was added a solution of 5-(2,4-dichloro-5-hydroxyphenoxy)-1,3-dimethyl-1H-pyrazole-4-carbaldehyde (1.825 g) obtained in Reference Example 52 in tetrahydrofuran (4 mL) at 0°C, and the reaction mixture was stirred at 0°C for 4 hr. Sodium hydride (291 mg) and ethyl (diethoxyphosphoryl)acetate (1.494 g) were further added, and the mixture was stirred at room temperature for 12 hr. Sodium hydride (145 mg) and ethyl (diethoxyphosphoryl)acetate (0.70 g) were further added, and the mixture was stirred at room temperature for 2 hr. Aqueous ammonium chloride solution was added to the reaction mixture, and the mixture was extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 4:1 - 3:1, v/v) to give the title compound as colorless crystals (2.23 g, yield 99%) .
¹H-NMR (300 MHz, CDCl₃)δ:1.12 - 1.37 (m, 3 H), 2.32 (s, 3 H), 3.56 (s, 3 H), 4.19 (q, J = 7.2 Hz, 2 H), 6.01 (d, J = 16.2 Hz, 1 H), 6.25 (s, 1 H), 6.95 (br s, 1 H), 7.36 (d, J= 16.2 Hz, 1 H), 7.46 (s, 1 H).

### Reference Example 152 2,4-dichloro-5-({4-[(1E)-3-hydroxyprop-1-en-1-yl]-1,3-dimethyl-1H-pyrazol-5-yl}oxy)phenol

A solution (4 mL) of ethyl (2E)-3-[5-(2,4-dichloro-5-hydroxyphenoxy)-1,3-dimethyl-1H-pyrazol-4-yl]acrylate (300 mg) obtained in Reference Example 151 in THF was cooled to 0°C in an ice bath. Lithium aluminum hydride (34 mg) was further added with stirring, and the mixture was stirred at 0°C for 30 min. Lithium aluminum hydride (17 mg) was added, and the mixture was stirred at 0°C for 30 min. Sodium sulfate decahydrate (573 mg) was added to the reaction mixture, and the mixture was stirred at room temperature for 10 min. The reaction mixture was filtered through celite, and the filtrate was concentrated to give the title compound as colorless crystals (162 mg, yield 61%).
¹H-NMR (300 MHz, DMSO-d₆)δ:2.18 (s, 3 H), 3.43 (s, 3 H), 3.86 - 3.97 (m, 2 H), 5.60 (s, 1 H), 5.71 - 5.90 (m, 1 H), 6.04 - 6.25 (m, 1 H), 7.09 (s, 1 H).

### Reference Example 153 5-(2,4-dichloro-5-hydroxyphenoxy)-N-methoxy-N,1,4-trimethyl-1H-pyrazole-3-carboxamide

The title compound was obtained in the same manner as in Reference Example 22 and from 5-(2,4-dichloro-5-hydroxyphenoxy)-1,4-dimethyl-1H-pyrazole-3-carboxylic acid obtained in Reference Example 105.
¹H-NMR (300 MHz, CDCl₃)δ:1.94 (s, 3 H), 3.45 (s, 3 H), 3.67 (s, 3 H), 3.74 (s, 3 H), 6.59 (s, 1 H), 7.41 (s, 1 H).

### Reference Example 154 5-(2,4-dichloro-5-hydroxyphenoxy)-1,4-dimethyl-1H-pyrazole-3-carbaldehyde

The title compound was obtained in the same manner as in Reference Example 24 and from 5-(2,4-dichloro-5-hydroxyphenoxy)-N-methoxy-N,1,4-trimethyl-1H-pyrazole-3-carboxamide obtained in Reference Example 153.
¹H-NMR (300 MHz, CDCl₃)δ:2.07 (s, 3 H), 3.77 (s, 3 H), 5.76 (s, 1 H), 6.37 (s, 1 H), 7.45 (s, 1 H), 9.90 (s, 1 H).

### Reference Example 155 (2E)-3-[5-(2,4-dichloro-5-hydroxyphenoxy)-1-methyl-3-(trifluoromethyl)-1H-pyrazol-4-yl]acrylonitrile

While stirring a solution of diethyl cyanomethylphosphonate (450 mg) cooled to 0°C in an ice bath in tetrahydrofuran (4 mL), sodium hydride (149 mg) was added, and the mixture was stirred at 0°C for 20 min. To the reaction mixture was added a solution of 5-(2,4-dichloro-5-hydroxyphenoxy)-1-methyl-3-(trifluoromethyl)-1H-pyrazole-4-carbaldehyde (600 mg) obtained in Reference Example 58 in tetrahydrofuran (2 mL) at 0°C, and the reaction mixture was stirred at 0°C for 1.5 hr. Aqueous ammonium chloride solution was added to the reaction mixture, and the mixture was extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 9:1 - 4:1, v/v), and crystallized from hexane-ethyl acetate to give the title compound as colorless crystals (271 mg, yield 42%).
¹H-NMR (300 MHz, CDCl₃)δ:3.74 (s, 3 H), 5.51 - 5.74 (m, 2 H), 6.27 (s, 1 H), 7.14 (d, J = 16.7 Hz, 1 H), 7.52 (s, 1 H).

### Reference Example 156 4-(benzyloxy)-5-chloro-2-hydroxybenzaldehyde

To a mixture of 2,4-dihydroxybenzaldehyde (100.0 g) cooled to 0°C in an ice bath, N-chlorosuccinimide (99.58 g), and acetonitrile (1000 mL) was added concentrated hydrochloric acid (5.0 mL), and the mixture was stirred at 0°C for 1 hr and at room temperature for 22 hr, and concentrated under reduced pressure. Water and ethyl acetate were added to the residue, and the mixture was stirred until complete dissolution. Two layers were separated, and the organic layer was washed with saturated brine and dried over anhydrous magnesium sulfate. After filtration, the filtrate was concentrated and the residue was dissolved in acetone (1000 mL), and the solution was cooled to 0°C. Potassium carbonate (140.1 g) was added to the mixture, and benzylbromide (174.0 g) was added dropwise at 0°C over 90 min with stirring. The mixture was stirred at 0°C for 2 hr and at room temperature for 17 hr, and the insoluble material was removed by filtration. The filtrate was concentrated, 2N aqueous sodium hydroxide solution (1000 mL) was added to the residue, and the mixture was extracted with ethyl acetate (800 mL). The aqueous layer was acidified with 6N hydrochloric acid (500 mL), and the mixture was extracted with ethyl acetate (1000 mL). The organic layer was washed with saturated brine, and dried over anhydrous magnesium sulfate. After filtration, the filtrate was concentrated and the residue was subjected to silica gel column chromatography (hexane-ethyl acetate 95:5 - 90:10, v/v) to give the title compound (15.08 g, yield 8%) as colorless crystals.
¹H-NMR (300 MHz, CDCl₃)δ:5.20 (s, 2 H), 6.55 (s, 1 H), 7.32 - 7.49 (m, 5 H), 7.53 (s, 1 H), 9.69 (s, 1 H), 11.39 (s, 1 H).

### Reference Example 157 5-(benzyloxy)-4-chloro-2-(1,3-dioxolan-2-yl)phenol

The title compound was obtained in the same manner as in Reference Example 115 and from 4-(benzyloxy)-5-chloro-2-hydroxybenzaldehyde obtained in Reference Example 156.
¹H-NMR (300 MHz, CDCl₃)δ:3.94 - 4.26 (m, 4 H), 5.12 (s, 2 H), 5.84 (s, 1 H), 6.53 (s, 1 H), 7.22 (s, 1 H), 7.30 - 7.51 (m, 5 H), 7.84 (s, 1 H).

### Reference Example 158 5-[5-(benzyloxy)-4-chloro-2-(1,3-dioxolan-2-yl)phenoxy]-1,3-dimethyl-1H-pyrazole-4-carbaldehyde

To a solution of 5-(benzyloxy)-4-chloro-2-(1,3-dioxolan-2-yl)phenol (4.48 g) obtained in Reference Example 157 and 5-chloro-1,3-dimethyl-1H-pyrazole-4-carbaldehyde (2.32 g) in 1-methyl-2-pyrrolidone (15 mL) was added potassium carbonate (3.03 g). The reaction mixture was stirred under an argon atmosphere at 130°C for 16 hr. The reaction mixture was cooled to room temperature, water was added, and the mixture was extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 4:1, v/v), and crystallized from ethyl acetate-hexane to give the title compound (3.02 g, yield 48%) as yellow crystals.
¹H-NMR (300 MHz, CDCl₃)δ:2.47 (s, 3 H), 3.49 (s, 3 H), 3.91 - 4.24 (m, 4 H), 5.03 (s, 2 H), 6.13 (s, 1 H), 6.22 (s, 1 H), 7.09 - 7.40 (m, 5 H), 7.64 (s, 1 H), 9.44 (s, 1 H).

### Reference Example 159 5-[5-(benzyloxy)-4-chloro-2-(1,3-dioxolan-2-yl)phenoxy]-1,3,4-trimethyl-1H-pyrazole

The title compound was obtained in the same manner as in Reference Example 117 and from 5-[5-(benzyloxy)-4-chloro-2-(1,3-dioxolan-2-yl)phenoxy]-1,3-dimethyl-1H-pyrazole-4-carbaldehyde obtained in Reference Example 158.
¹H-NMR (300 MHz, CDCl₃)δ:1.56 (s, 3 H), 2.19 (s, 3 H), 3.47 (s, 3 H), 3.94 - 4.24 (m, 4 H), 5.00 (s, 2 H), 6.13 (s, 1 H), 6.17 (s, 1 H), 7.26 - 7.40 (m, 5 H), 7.60 (s, 1 H).

### Reference Example 160 5-chloro-4-hydroxy-2-[(1,3,4-trimethyl-1H-pyrazol-5-yl)oxy]benzaldehyde

5-[5-(Benzyloxy)-4-chloro-2-(1,3-dioxolan-2-yl)phenoxy]-1,3,4-trimethyl-1H-pyrazole (644 mg) obtained in Reference Example 159 was dissolved in ethanol (40 mL), palladium carbon ethylenediamine complex (40 mg) was added, and the mixture was stirred under a hydrogen atmosphere (1 atm) at room temperature for 6 hr. The catalyst was removed by filtration, and the filtrate was concentrated. To a solution of the residue in tetrahydrofuran (5 mL) was added 1N hydrochloric acid (2 mL), and the mixture was stirred at room temperature for 1.5 hr. Water and ethyl acetate were added to the reaction mixture, and the mixture was stirred until complete dissolution. Two layers were separated, and the organic layer was washed with saturated brine, and dried over anhydrous magnesium sulfate. After filtration, the filtrate was concentrated and the residue was crystallized from hexane-ethyl acetate-diisopropyl ether to give the title compound (191 mg, yield 44%) as colorless crystals.
¹H-NMR (300 MHz, CDCl₃)δ:1.82 (s, 3 H), 2.08 (s, 3 H), 3.51 (s, 3 H), 6.14 (s, 1 H), 7.97 (s, 1 H), 10.37 (s, 1 H), 12.14 (br s, 1 H).

### Reference Example 161 4-chloro-3-[(4-ethyl-1,3-dimethyl-1H-pyrazol-5-yl)thio]phenol

A solution of 5-[(3-hydroxyphenyl)thio]-1,3-dimethyl-1H-pyrazole-4-carbaldehyde (20.0 g) obtained in Reference Example 90 in tetrahydrofuran (200 mL) was cooled to 0°C, methylmagnesium bromide (1.0 M tetrahydrofuran solution, 170 mL) was added dropwise thereto, and the mixture was stirred at room temperature for 3 hr. Saturated aqueous ammonium chloride solution was added to the reaction mixture, and the mixture was extracted with ethyl acetate. The organic layer was washed with water and saturated brine, dried over anhydrous magnesium sulfate and filtered. The filtrate was concentrated, and the residue was dissolved in trifluoroacetic acid (150 mL). Triethylsilane (20 mL) was added, and the mixture was stirred at 60°C for 3 hr. The solvent of the reaction mixture was evaporated, and the residue was dissolved in ethyl acetate. The organic layer was washed with water and saturated aqueous sodium hydrogen carbonate, dried over anhydrous magnesium sulfate and filtered. The filtrate was concentrated to give colorless crystals (15.0 g). A part (1.0 g) of the colorless crystals was dissolved in dichloromethane (50 mL), sulfuryl chloride (540 mg) was added dropwise at 0°C, and the mixture was stirred at room temperature for 30 min. The solvent was evaporated, and the residue was subjected to silica gel column chromatography (hexane-ethyl acetate 4:1, v/v) to give the title compound as colorless crystals (172.3 mg, yield 11%).
¹H-NMR (300 MHz, CDCl₃)δ:1.08 (t, J = 7.5 Hz, 3 H), 2.11 (s, 3 H), 2.46 (q, J = 7.5 Hz, 2 H), 3.67 (s, 3 H), 6.11 (t, J = 2.1 Hz, 1 H), 6.62 (dd, J = 8.7, 2.1 Hz, 1 H), 7.20 (d, J = 8.7 Hz, 1 H), 10.43 (s, 1 H).

### Reference Example 162 2,4-dichloro-3-[(1,3,4-trimethyl-1H-pyrazol-5-yl)thio]phenol

The title compound was obtained in the same manner as in Reference Example 94 from 3-[(1,3,4-trimethyl-1H-pyrazol-5-yl)thio]phenol obtained in Reference Example 93, and from a fraction having shorter retention time than that in Reference Example 94 by preparative HPLC.
¹H-NMR (300 MHz, DMSO-d₆)δ:1.74 (s, 3 H), 2.03 (s, 3 H), 3.70 (s, 3 H), 6.99 (d, J = 8.7 Hz, 1 H), 7.34 (d, J = 8.7 Hz, 1 H), 10.70 (s, 1 H).

### Reference Example 163 2,4-dichloro-5-[(1,3-dimethyl-4-vinyl-1H-pyrazol-5-yl)thio]phenol

The title compound was obtained in the same manner as in Reference Example 51 and from 5-[(2,4-dichloro-5-hydroxyphenyl)thio]-1,3-dimethyl-1H-pyrazole-4-carbaldehyde obtained in Reference Example 130.
¹H-NMR (300 MHz, DMSO-d₆)δ:2.35 (s, 3 H), 3.74 (s, 3 H), 5.23 (dd, J = 11.7, 1.5 Hz, 1 H), 5.54 (dd, J = 18.0, 1.5 Hz, 1 H), 6.11 (s, 1 H), 6.48 - 6.58 (m, 1 H), 7.55 (s, 1 H), 10.55 (s, 1 H).

### Reference Example 164 2,4-dichloro-5-[(4-ethyl-1,3-dimethyl-1H-pyrazol-5-yl)thio]phenol

The title compound was obtained in the same manner as in Reference Example 161 and from 5-[(3-hydroxyphenyl)thio]-1,3-dimethyl-1H-pyrazole-4-carbaldehyde obtained in Reference Example 90.
¹H-NMR (300 MHz, DMSO-d₆)δ: 0.97 (t, J = 7.6 Hz, 3 H), 2.22 (s, 3 H), 2.38 (d, J = 7.6 Hz, 2 H), 3.69 (s, 3 H), 6.10 (s, 1 H), 7.53 (s, 1 H), 10.55 (s, 1 H).

### Reference Example 165 5-cyclopropyl-2-methyl-2,4-dihydro-3H-pyrazol-3-one

The title compound was obtained in the same manner as in Reference Example 13 and from methyl 3-cyclopropyl-3-oxopropionate and methylhydrazine.
¹H-NMR (300 MHz, CDCl₃)δ:0.74 - 0.82 (m, 2 H), 0.92 - 1.01 (m, 2 H), 1.71 - 1.83 (m, 1 H), 3.05 (s, 2 H), 3.26 (s, 3 H).

### Reference Example 166 5-chloro-3-cyclopropyl-1-methyl-1H-pyrazole-4-carbaldehyde

The title compound was obtained in the same manner as in Reference Example 14 and from 5-cyclopropyl-2-methyl-2,4-dihydro-3H-pyrazol-3-one obtained in Reference Example 165.
¹H-NMR (300 MHz, CDCl₃)δ:0.89 - 1.03 (m, 4 H), 2.39 - 2.51 (m, 1 H), 3.77 (s, 3 H), 9.91 (s, 1 H).

### Reference Example 167 3-cyclopropyl-5-{2,4-dichloro-5-[(4-methoxybenzyl)oxy]phenoxy}-1-methyl-1H-pyrazole-4-carbaldehyde

The title compound was obtained in the same manner as in Reference Example 2 and from 2,4-dichloro-5-[(4-methoxybenzyl)oxy]phenol obtained in Reference Example 1, and 5-chloro-3-cyclopropyl-1-methyl-1H-pyrazole-4-carbaldehyde obtained in Reference Example 166.
¹H-NMR (300 MHz, CDCl₃)δ:0-99 - 1.03 (m, 4 H), 2.32 - 2.41 (m, 1 H), 3.52 (s, 3 H), 3.81 (s, 3 H), 4.96 (s, 2 H), 6.41 (s, 1 H), 6.87 (d, J = 8.7 Hz, 2 H), 7.20 (d, J = 8.7 Hz, 2 H), 7.45 (s, 1 H), 9.50 (s, 1 H).

### Reference Example 168 3-cyclopropyl-5-(2,4-dichloro-5-hydroxyphenoxy)-1-methyl-1H-pyrazole-4-carbaldehyde

The title compound was obtained in the same manner as in Reference Example 50 and from 3-cyclopropyl-5-{2,4-dichloro-5-[(4-methoxybenzyl)oxy]phenoxy}-1-methyl-1H-pyrazole-4-carbaldehyde obtained in Reference Example 167.
¹H-NMR (300 MHz, CDCl₃)δ:0.95 - 0.99 (m, 4 H), 2.32 - 2.41 (m, 1 H), 3.61 (s, 3 H), 6.49 (s, 1 H), 6.58 (br, 1 H), 7.44 (s, 1 H), 9.63 (s, 1 H).

### Reference Example 169 2,4-dichloro-5-[(3-cyclopropyl-4-ethyl-1-methyl-1H-pyrazol-5-yl)oxy]phenol

A solution of 3-cyclopropyl-5-(2,4-dichloro-5-hydroxyphenoxy)-1-methyl-1H-pyrazole-4-carbaldehyde (3.0 g) obtained in Reference Example 168 in tetrahydrofuran (70 mL) was cooled to 0°C, methylmagnesium bromide (1.0 M tetrahydrofuran solution, 19 mL) was added dropwise thereto, and the mixture was stirred at room temperature for 3 hr. Saturated aqueous ammonium chloride solution was added to the reaction mixture, and the mixture was extracted with ethyl acetate. The organic layer was washed with water and saturated brine, dried over anhydrous magnesium sulfate and filtered. The filtrate was concentrated, and the residue was dissolved in trifluoroacetic acid (50 mL), triethylsilane (10 mL) was added, and the mixture was stirred at 60°C for 3 hr. The solvent of the reaction mixture was evaporated, and the residue was dissolved in ethyl acetate. The organic layer was washed with water and saturated aqueous sodium hydrogen carbonate. The organic layer was dried over anhydrous magnesium sulfate, and filtered, and the filtrate was concentrated to give the title compound as colorless crystals (2.45 g, yield 82%).
¹H-NMR (300 MHz, CDCl₃)δ:0.70 - 0.75 (m, 2 H), 0.78 - 0.88 (m, 2 H), 1.10 (t, J = 7.2 Hz, 3 H), 1.66 - 1.75 (m, 1 H), 2.35 (q, J = 7.2 Hz, 2 H), 3.41 (s, 3 H), 6.15 (s, 1 H), 7.42 (s, 1 H), 10.99 (br, 1 H).

### Reference Example 170 2,4-dichloro-5-[(3-cyclopropyl-1,4-dimethyl-1H-pyrazol-5-yl)oxy]phenol

The title compound was obtained in the same manner as in Reference Example 92 and then Reference Example 93, and from 3-cyclopropyl-5-(2,4-dichloro-5-hydroxyphenoxy)-1-methyl-1H-pyrazole-4-carbaldehyde obtained in Reference Example 168. ¹H-NMR (300 MHz, CDCl₃)δ:0.70 - 0.79 (m, 2 H), 0.83 - 0.89 (m, 2 H), 1.65-1.74 (m, 1 H), 1.87 (s, 3 H), 3.41 (s, 3 H), 6.14 (s, 1 H), 7.41 (s, 1 H), 11.09 (br, 1 H).

### Reference Example 171 2,4-dichloro-5-[(3-cyclopropyl-1-methyl-4-vinyl-1H-pyrazol-5-yl)oxy]phenol

The title compound was obtained in the same manner as in Reference Example 51 and from 3-cyclopropyl-5-(2,4-dichloro-5-hydroxyphenoxy)-1-methyl-1H-pyrazole-4-carbaldehyde obtained in Reference Example 168.
¹H-NMR (300 MHz, CDCl₃)δ:0.76 - 0.79 (m, 2 H), 0.83 - 0.89 (m, 2 H), 1.76 - 1.82 (m, 1 H), 3.45 (s, 3 H), 5.14 (dd, J = 11.4, 1.2 Hz, 1 H), 5.43 (dd, J = 18.0, 1.5 Hz, 1 H), 6.17 (s, 1 H), 6.35 - 6.45 (m, 1 H), 7.44 (s, 1 H), 10.35 (br, 1 H).

### Reference Example 172 4-{(E)-2-[5-(2,4-dichloro-5-hydroxyphenoxy)-1,3-dimethyl-1H-pyrazol-4-yl]vinyl}benzonitrile

To a solution of 5-{2,4-dichloro-5-[(4-methoxybenzyl)oxy]phenoxy}-1,3-dimethyl-1H-pyrazole-4-carbaldehyde (1.00 g) obtained in Reference Example 2 and (4-cyanobenzyl)(triphenyl)phosphonium chloride (4.93 g) in toluene (50 mL) was added 1,8-diazabicyclo[5.4.0]undec-7-ene (1.78 mL), and the mixture was heated under reflux for 14 hr. Water was added to the reaction mixture, and the mixture was extracted with ethyl acetate. The organic layer was washed with water and saturated brine, dried over anhydrous magnesium sulfate and filtered. The filtrate was concentrated, and the residue was dissolved in ethyl acetate (100 mL), 4N hydrochloric acid-ethyl acetate solution (10 mL) was added, and the mixture was stirred at 60°C for 3 hr. A solvent of the reaction mixture was evaporated, and the residue was dissolved in ethyl acetate. The organic layer was washed with water and saturated aqueous sodium hydrogen carbonate. The organic layer was dried over anhydrous magnesium sulfate, and filtered, and the filtrate was concentrated to give the title compound as colorless crystals (600 mg, yield 63%).
¹H-NMR (300 MHz, CDCl₃)δ:2.28 (s, 3 H), 3.58 (s, 3 H), 6.26 (s, 1 H), 6. 65 - 6.80 (m, 2 H), 7.38 (d, J = 8.4 Hz, 2 H), 7.48 (s, 1 H), 7.57 (d, J = 8.4 Hz, 2 H), 8.44 (br, 1 H).

### Reference Example 173 3-(difluoromethyl)-1-methyl-1H-pyrazol-5-ol

The title compound was obtained in the same manner as in Reference Example 13 and from ethyl 4,4-difluoro-3-oxobutanoate and methylhydrazine.
¹H-NMR (300 MHz, DMSO-d₆)δ:3.54 (s, 3 H), 5.55 (s, 1 H), 6.70 (t, J =54.8 Hz, 1 H), 11.35 (s, 1 H).

### Reference Example 174 5-chloro-3-(difluoromethyl)-1-methyl-1H-pyrazole-4-carbaldehyde

The title compound was obtained in the same manner as in Reference Example 14 and from 3-(difluoromethyl)-1-methyl-1H-pyrazol-5-ol obtained in Reference Example 173.
¹H-NMR (300 MHz, CDCl₃)δ:3.93 (s, 3 H), 6.90 (t, J=53.6 Hz, 1 H), 9.96 (s, 1 H).

### Reference Example 175 5-{2,4-dichloro-5-[(4-methoxybenzyl)oxy]phenoxy}-3-(difluoromethyl)-1-methyl-1H-pyrazole-4-carbaldehyde

The title compound was obtained in the same manner as in Reference Example 2 and from 2,4-dichloro-5-[(4-methoxybenzyl)oxy]phenol obtained in Reference Example 1, and 5-chloro-3-(difluoromethyl)-1-methyl-1H-pyrazole-4-carbaldehyde obtained in Reference Example 174.
¹H-NMR (300 MHz, CDCl₃)δ:3.70 (s, 3 H), 3.81 (s, 3 H), 5.00 (s, 2 H), 6.46 (s, 1 H), 6.85 - 6.88 (m, 3 H), 7.19 (d, J = 8.7 Hz, 2 H), 7.47 (s, 1 H), 9.44 (s, 1 H).

### Reference Example 176 5-(2,4-dichloro-5-hydroxyphenoxy)-3-(difluoromethyl)-1-methyl-1H-pyrazole-4-carbaldehyde

The title compound was obtained in the same manner as in Reference Example 50 and from 5-{2,4-dichloro-5-[(4-methoxybenzyl)oxy]phenoxy}-3-(difluoromethyl)-1-methyl-1H-pyrazole-4-carbaldehyde obtained in Reference Example 175.
¹H-NMR (300 MHz, CDCl3)δ:3.81 (s, 3 H), 5.86 (s, 1 H), 6.59 (s, 1 H), 6.82 (s, 1 H), 7.46 (s, 1 H), 9.63 (s, 1 H).

### Reference Example 177 2,4-dichloro-5-{[3-(difluoromethyl)-1-methyl-4-vinyl-1H-pyrazol-5-yl]oxy}phenol

The title compound was obtained in the same manner as in Reference Example 51 and from 5-(2,4-dichloro-5-hydroxyphenoxy)-3-(difluoromethyl)-1-methyl-1H-pyrazole-4-carbaldehyde obtained in Reference Example 176.
¹H-NMR (300 MHz, DMSO-d₆)δ:3.83 (s, 3 H), 5.32 (dd, J = 12.9, 2.1 Hz, 1 H), 5.83 (dd, J = 17.7, 2.1 Hz, 1 H), 6.34 (s, 1 H), 6.47 - 6.57 (m, 1 H), 6.94 (s, 1 H), 7.12 (s, 1 H), 7.56 (s, 1 H).

### Reference Example 178 2,4-dichloro-5-{[3-(difluoromethyl)-1,4-dimethyl-1H-pyrazol-5-yl]oxy}phenol

The title compound was obtained in the same manner as in Reference Example 92 and then Reference Example 93, and from 5-(2,4-dichloro-5-hydroxyphenoxy)-3-(difluoromethyl)-1-methyl-1H-pyrazole-4-carbaldehyde obtained in Reference Example 176.
¹H-NMR (300 MHz, DMSO-d₆)δ:1.86 (s, 3 H), 3.63 (s, 3 H), 6.42 (s, 1 H), 6.95 (s, 1 H), 7.65 (s, 1 H), 10.69 (s, 1 H).

### Reference Example 179 2,4-dichloro-5-{[3-(difluoromethyl)-4-ethyl-1-methyl-1H-pyrazol-5-yl]oxy}phenol

The title compound was obtained in the same manner as in Reference Example 169 and from 5-(2,4-dichloro-5-hydroxyphenoxy)-3-(difluoromethyl)-1-methyl-1H-pyrazole-4-carbaldehyde obtained in Reference Example 176.
¹H-NMR (300 MHz, CDCl₃)δ:1.07 (t, J = 7.6 Hz, 3 H), 2.41 (q, J = 7.6 Hz, 2 H), 3.63 (s, 3 H), 6.18 (s, 1 H), 6.34 (s, 1 H), 6.57 (t, J = 54.2 Hz, 1 H), 7.44 (s, 1 H).

### Reference Example 180 3-cyclopropyl-5-[(3-hydroxyphenyl)thio]-1-methyl-1H-pyrazole-4-carbaldehyde

The title compound was obtained in the same manner as in Reference Example 2 and from 3-hydroxythiophenol, and 5-chloro-3-cyclopropyl-1-methyl-1H-pyrazole-4-carbaldehyde obtained in Reference Example 166.
¹H-NMR (300 MHz, DMSO-d₆)δ:0.87 0.90 (m, 2 H), 0. 95 - 0.99 (m, 2 H), 2.52 - 2.56 (m, 1 H), 3.74 (s, 3 H), 6.49 (s, 1 H), 6.62 - 6.68 (m, 2 H), 7.16 (t, J = 8.1 Hz, 1 H), 9.74 (s, 1 H), 9.91 (s, 1 H).

### Reference Example 181 3-cyclopropyl-5-[(2,4-dichloro-5-hydroxyphenyl)thio]-1-methyl-1H-pyrazole-4-carbaldehyde .

The title compound was obtained in the same manner as in Reference Example 94 and from 3-cyclopropyl-5-[(3-hydroxyphenyl)thio]-1-methyl-1H-pyrazole-4-carbaldehyde obtained in Reference Example 180.
¹H-NMR (300 MHz, DMSO-d₆)δ:0.88 - 0.95 (m, 2 H), 0.97 - 1.05 (m, 2 H), 2.55 - 2.59 (m, 1 H), 3.75 (s, 3 H), 6.27 (s, 1 H), 7.59 (s, 1 H), 9.87 (s, 1 H), 10.63 (s, 1 H).

### Reference Example 182 4-chloro-5-(methoxymethoxy)-2-methylphenol

The title compound was obtained in the same manner as in Reference Example 113 and from 1-(benzyloxy)-4-chloro-5-(methoxymethoxy)-2-methylbenzene obtained in Reference Example 109.
¹H-NMR (300 MHz, CDCl₃)δ:2.16 (s, 3 H), 3.52 (s, 3 H), 4.78 (s, 1 H), 5.18 (s, 2 H), 6.69 (s, 1 H), 7.10 (s, 1 H).

### Reference Example 183 5-[4-chloro-5-(methoxymethoxy)-2-methylphenoxy]-1,3-dimethyl-1H-pyrazole-4-carbaldehyde

The title compound was obtained in the same manner as in Reference Example 2 and from 4-chloro-5-(methoxymethoxy)-2-methylphenol obtained in Reference Example 182 and 5-chloro-1,3-dimethyl-1H-pyrazole-4-carbaldehyde.
¹H-NMR (300 MHz, CDCl₃)δ:2.29 (s, 3 H), 2.45 (s, 3 H), 3.44 (s, 3 H), 3.67 (s, 3 H), 5.09 (s, 2 H), 6.65 (s, 1 H), 7.27 (s, 1 H), 9.35 (s, 1 H).

### Reference Example 184 2-chloro-4-methyl-5-[(1,3,4-trimethyl-1H-pyrazol-5-yl)oxy]phenol

To a mixture of 5-[4-chloro-5-(methoxymethoxy)-2-methylphenoxy]-1,3-dimethyl-1H-pyrazole-4-carbaldehyde (2.43 g) obtained in Reference Example 183 and diethylene glycol (15 mL) was added hydrazine monohydrate (655 mg), and the mixture was stirred with heating at 125°C for 15 min. Potassium hydroxide (504 mg) was added, and the mixture was stirred with heating at 125°C for 5 hr. 30% Aqueous sulfuric acid solution (12 mL) was added, and the mixture was stirred at 50°C for 5 hr. The reaction mixture was cooled to room temperature and poured into water, and the mixture was extracted with ethyl acetate. The organic layer was washed with saturated brine, dried over anhydrous magnesium sulfate and filtered, and the filtrate was concentrated. The obtained residue was washed with toluene to give the title compound (1.19 g, yield 60%) as yellow crystals.
¹H-NMR (300 MHz, CDCl₃)δ:1.74 (s, 3 H), 2.10 (s, 3 H), 2.28 (s, 3 H), 3.48 (s, 3 H), 6.15 (s, 1 H), 7.17 (s, 1 H), 9.05 (br s, 1 H).

### Reference Example 185 1-(benzyloxy)-4-chloro-5-(methoxymethoxy)-2-vinylbenzene

While stirring a mixture of methyltriphenylphosphonium bromide (17.47 g) and tetrahydrofuran (200 mL), potassium tert-butoxide (5.49 g) was added. This mixture was stirred at room temperature for 1.5 hr, and 2-(benzyloxy)-5-chloro-4-(methoxymethoxy)benzaldehyde (10.00 g) obtained in Reference Example 108 was added. The reaction mixture was stirred at room temperature for 15 hr and poured into water, and the mixture was extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 67:33, v/v) to give the title compound (9.86 g, yield 99%) as pale-yellow crystals.
¹H-NMR (300 MHz, CDCl₃)δ:3.50 (s, 3 H), 5.07 (s, 2 H), 5.17 - 5.24 (m, 3 H), 5.65 (dd, J = 17.7, 1.3 Hz, 1 H), 6.83 (s, 1 H), 6.97 (dd, J = 17.7, 11.1 Hz, 1 H), 7.29 - 7.48 (m, 6 H).

### Reference Example 186 1-(benzyloxy)-4-chloro-2-ethyl-5-(methoxymethoxy)benzene

The title compound was obtained in the same manner as in Example 8 and from 1-(benzyloxy)-4-chloro-5-(methoxymethoxy)-2-vinylbenzene obtained in Reference Example 185.
¹H-NMR (300 MHz, CDCl₃)δ:1.18 (t, J= 7.5 Hz, 3 H), 2.61 (q, J = 7.5 Hz, 2 H), 3.51 (s, 3 H), 5.05 (s, 2 H), 5.18 (s, 2 H), 6.81 (s, 1 H), 7.13 (s, 1 H), 7.28 - 7.50 (m, 5 H).

### Reference Example 187 5-(benzyloxy)-2-chloro-4-ethylphenol

A mixture of 1-(benzyloxy)-4-chloro-2-ethyl-5-(methoxymethoxy)benzene (5.17 g) obtained in Reference Example 186 and 10% hydrochloric acid-methanol solution (30 mL) was stirred at 60°C for 1.5 hr. The reaction mixture was cooled to room temperature and poured into water, and the mixture was extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 100:0 - 75:25, v/v). The solvent was evaporated to give the title compound (4.20 g, yield 100%) as a colorless oil.
¹H-NMR (300 MHz, CDCl₃)δ:1.18 (t, J = 7.6 Hz, 3 H), 2.60 (q, J - 7.3 Hz, 2 H), 5.03 (s, 2 H), 5.36 (s, 1 H), 6.61 (s, 1 H), 7.07 (s, 1 H), 7.28 - 7.47 (m, 5 H).

### Reference Example 188 5-[5-(benzyloxy)-2-chloro-4-ethylphenoxy]-1,3-dimethyl-1H-pyrazole-4-carbaldehyde

The title compound was obtained in the same manner as in Reference Example 2 and from 5-(benzyloxy)-2-chloro-4-ethylphenol obtained in Reference Example 187 and 5-chloro-1,3-dimethyl-1H-pyrazole-4-carbaldehyde.
¹H-NMR (300 MHz, CDCl₃)δ:1.21 (t, J = 7.6 Hz, 3 H), 2.45 (s, 3 H), 2.65 (q, J = 7.4 Hz, 2 H), 3.60 (s, 3 H), 4.96 (s, 2 H), 6.44 (s, 1 H), 7.17 - 7.46 (m, 6 H), 9.31 (s, 1 H).

### Reference Example 189 {5-[5-(benzyloxy)-2-chloro-4-ethylphenoxy]-1,3-dimethyl-1H-pyrazol-4-yl}methanol

The title compound was obtained in the same manner as in Reference Example 100 and from 5-[5-(benzyloxy)-2-chloro-4-ethylphenoxy]-1,3-dimethyl-1H-pyrazole-4-carbaldehyde obtained in Reference Example 188.
¹H-NMR (300 MHz, CDCl₃)δ:0.96 (t, J = 5.4 Hz, 1 H), 1.21 (t, J = 7.5 Hz, 3 H), 2.29 (s, 3 H), 2.64 (q, J = 7.5 Hz, 2 H), 3.54 (s, 3 H), 4.16 (d, J = 5.3 Hz, 2 H), 4.93 (s, 2 H), 6.34 (s, 1 H), 7.19 (s, 1 H), 7.21 - 7.41 (m, 5 H).

### Reference Example 190 4-chloro-2-ethyl-5-[(1,3,4-trimethyl-1H-pyrazol-5-yl)oxy]phenol

The title compound was obtained in the same manner as in Reference Example 101 and from {5-[5-(benzyloxy)-2-chloro-4-ethylphenoxy]-1,3-dimethyl-1H-pyrazol-4-yl}methanol obtained in Reference Example 189.
¹H-NMR (300 MHz, CDCl₃)δ:1.23 (t, J = 7.6 Hz, 3 H), 1.76 (s, 3 H), 2.10 (s, 3 H), 2.61 (q, J = 7.6 Hz, 2 H), 3.51 (s, 3 H), 6.12 (s, 1 H), 7.17 (s, 1 H).

### Reference Example 191 4-chloro-2-ethyl-5-(methoxymethoxy)phenol

The title compound was obtained in the same manner as in Reference Example 113 and from 1-(benzyloxy)-4-chloro-2-ethyl-5-(methoxymethoxy)benzene obtained in Reference Example 186.
¹H-NMR (300 MHz, CDCl₃)δ:1.20 (t, J = 7.6 Hz, 3 H), 2.54 (q, J = 7.6 Hz, 2 H), 3.52 (s, 3 H), 4.84 (br s, 1 H), 5.19 (s, 2 H), 6.68 (s, 1 H).

### Reference Example 192 5-[4-chloro-2-ethyl-5-(methoxymethoxy)phenoxy]-1,3-dimethyl-1H-pyrazole-4-carbaldehyde

The title compound was obtained in the same manner as in Reference Example 2 and from 4-chloro-2-ethyl-5-(methoxymethoxy)phenol obtained in Reference Example 191, and 5-chloro-1,3-dimethyl-1H-pyrazole-4-carbaldehyde.
¹H-NMR (300 MHz, CDCl₃)δ:1.25 (t, J = 7.5 Hz, 3 H), 2.45 (s, 3 H), 2.70 (q, J = 7.5 Hz, 2 H), 3.43 (s, 3 H), 3.67 (s, 3 H), 5.09 (s, 2 H), 6.63 (s, 1 H), 7.29 (s, 1 H), 9.36 (s, 1 H).

### Reference Example 193 2-chloro-4-ethyl-5-[(1,3,4-trimethyl-1H-pyrazol-5-yl)oxy]phenol

The title compound was obtained in the same manner as in Reference Example 100 and then Reference Example 101, and from 5-[4-chloro-2-ethyl-5-(methoxymethoxy)phenoxy]-1,3-dimethyl-1H-pyrazole-4-carbaldehyde obtained in Reference Example 192.
¹H-NMR (300 MHz, CDCl₃)δ:1.26 (t, J = 7.5 Hz, 3 H), 1.73 (s, 3 H), 2.20 (s, 3 H), 2.70 (q, J = 7.7 Hz, 2 H), 3.60 (s, 3 H), 6.22 (s, 1 H), 7.18 (s, 1 H).

### Reference Example 194 5-[5-(benzyloxy)-2-chloro-4-ethylphenoxy]-1,3-dimethyl-1H-pyrazole-4-carbaldehyde oxime

To a solution (5 mL) of 5-[5-(benzyloxy)-2-chloro-4-ethylphenoxy]-1,3-dimethyl-1H-pyrazole-4-carbaldehyde (700 mg) obtained in Reference Example 188 in methanol was added hydroxylamine hydrochloride (455 mg), and the mixture was stirred at 50°C for 30 min. The solvent was evaporated, water was added to the residue, and the mixture was extracted with a mixed solvent of ethyl acetate-tetrahydrofuran. The organic layer was dried over anhydrous magnesium sulfate. After filtration, the filtrate was concentrated to give the title compound (731 mg, yield 100%) as colorless crystals.
¹H-NMR (300 MHz, CDCl₃)δ:1.20 (t, J = 7.5 Hz, 3 H), 2.39 (s, 3 H), 2.63 (q, J = 7.4 Hz, 2 H), 3.50 (s, 3 H), 4.90 (s, 2 H), 6.20 (s, 1 H), 6.85 (s, 1 H), 7.19 (s, 1 H), 7.21 - 7.42 (m, 5 H), 7.75 (s, 1 H).

### Reference Example 195 5-(2-chloro-4-ethyl-5-hydroxyphenoxy)-1,3-dimethyl-1H-pyrazole-4-carbonitrile

The title compound was obtained in the same manner as in Reference Example 133 and then Reference Example 28, and from 5-[5-(benzyloxy)-2-chloro-4-ethylphenoxy]-1,3-dimethyl-1H-pyrazole-4-carbaldehyde oxime obtained in Reference Example 194.
¹H-NMR (300 MHz, CDCl₃)δ:1.24 (t, J = 7.5 Hz, 3 H), 2.27 (s, 3 H), 2.62 (q, J = 7.5 Hz, 2 H), 3.73 (s, 3 H), 6.65 (s, 1 H), 7.21 (s, 1 H).

### Reference Example 196 2-methyl-3a,4,5,6-tetrahydrocyclopenta[c]pyrazol-3(2H)-one

The title compound was obtained in the same manner as in Reference Example 123 and from ethyl 2-cyclopentanonecarboxylate and methylhydrazine.
¹H-NMR (300 MHz, DMSO-d₆)δ:2.12 - 2.45 (m, 6 H), 3.41 (s, 3 H), 10.38 (s, 1 H).

### Reference Example 197 3-bromo-2-methyl-2,4,5,6-tetrahydrocyclopenta[c]pyrazole

The title compound was obtained in the same manner as in Reference Example 124 and from 2-methyl-3a,4,5,6-tetrahydrocyclopenta[c]pyrazol-3(2H)-one obtained in Reference Example 196.
¹H-NMR (300 MHz, CDCl₃)δ:2.31 - 2.43 (m, 2 H), 2.54 - 2.62 (m, 2 H), 2.68 - 2.75 (m, 2 H), 3.80 (s, 3 H).

### Reference Example 198 [5-(benzyloxy)-2,4-dichlorophenyl] (2-methyl-2,4,5,6-tetrahydrocyclopenta[c]pyrazol-3-yl)methanol

The title compound was obtained in the same manner as in Reference Example 128 and from 3-bromo-2-methyl-2,4,5,6-tetrahydrocyclopenta[c]pyrazole obtained in Reference Example 197 and 5-(benzyloxy)-2,4-dichlorobenzaldehyde obtained in Reference Example 127.
¹H-NMR (300 MHz, CDCl₃)δ:1.62 - 1.74 (m, 1 H), 1.79 - 1.91 (m, 1 H), 2.09 - 2.21 (m, 2 H), 2.57 (t, J = 7.4 Hz, 2 H), 3.87 (s, 3 H), 5.14 - 5.26 (m, 2 H), 6.04 (s, 1 H), 7.27 - 7.47 (m, 7 H) .

### Reference Example 199 2,4-dichloro-5-[(2-methyl-2,4,5,6-tetrahydrocyclopenta[c]pyrazol-3-yl)methyl]phenol

The title compound was obtained in the same manner as in Reference Example 129 and from [5-(benzyloxy)-2,4-dichlorophenyl] (2-methyl-2,4,5,6-tetrahydrocyclopenta[c]pyrazol-3-yl)methanol obtained in Reference Example 198.
¹H-NMR (300 MHz, DMSO-d₆)δ:1.99 - 2.22 (m, 4 H), 2.41 - 2.53 (m, 2 H), 3.65 (s, 3 H), 3.87 (s, 2 H), 6.75 (s, 1 H), 7.39 (s, 1 H) .

### Reference Example 200 4-(benzyloxy)-5-bromo-2-hydroxybenzaldehyde

To a solution of 4-(benzyloxy)-2-hydroxybenzaldehyde (500 mg) in acetonitrile (5 mL) was added N-bromosuccinimide (390 mg) at 0°C, and the mixture was stirred for 3 hr and concentrated under reduced pressure. Heated diisopropyl ether (15 mL) was added to the residue, and the insoluble material was removed by filtration. The filtrate was concentrated, and the residue was subjected to silica gel column chromatography (hexane-ethyl acetate 91:9, v/v), and crystallized from hexane-diisopropyl ether to give the title compound (274 mg, yield 41%) as colorless crystals.
¹H-NMR (300 MHz, CDC1₃)δ:5.20 (s, 2 H), 6.53 (s, 1 H), 7.31 - 7.49 (m, 5 H), 7.71 (s, 1 H), 9.69 (s, 1 H), 11.40 (s, 1 H).

### Reference Example 201 4-(benzyloxy)-5-bromo-2-(methoxymethoxy)benzaldehyde

While stirring a mixture of 4-(benzyloxy)-5-bromo-2-hydroxybenzaldehyde (5.00 g) obtained in Reference Example 200, potassium carbonate (3.37 g), and N,N-dimethylformamide (20 mL), chloromethyl methyl ether (1.85 mL) was added dropwise at 0°C. The reaction mixture was stirred at room temperature for 15 hr, and poured into water, and the mixture was extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 89:11 - 75:25, v/v), and crystallized from hexane-ethyl acetate to give the title compound (4.17 g, yield 73%) as colorless crystals.
¹H-NMR (300 MHz, CDCl₃)δ:3.50 (s, 3 H), 5.22 (s, 2 H), 5.24 (s, 2 H), 6.84 (s, 1H), 7.31 - 7.50 (m, 5 H), 8.04 (s, 1 H), 10.25 (s, 1 H).

### Reference Example 202 1-(benzyloxy)-2-bromo-5-(methoxymethoxy)-4-methylbenzene

To a mixture of 4-(benzyloxy)-5-bromo-2-(methoxymethoxy)benzaldehyde (1.00 g) obtained in Reference Example 201 and diethylene glycol (5 mL) was added hydrazine monohydrate (157 mg), and the mixture was stirred with heating at 125°C for 15 min. Potassium hydroxide (240 mg) was added, and the mixture was stirred with heating at 125°C for 15 hr. The reaction mixture was cooled to room temperature, and poured into water, and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 99:1 - 91:9, v/v), and crystallized from hexane to give the title compound (754 mg, yield 78%) as colorless crystals.
¹H-NMR (300 MHz, CDC13))S:2.14 (s, 3 H), 3.45 (s, 3 H), 5.12 (s, 4 H), 6.77 (s, 1 H), 7.27 - 7.51 (m, 6 H).

### Reference Example 203 5-(benzyloxy)-4-bromo-2-methylphenol

A mixture of 1-(benzyloxy)-2-bromo-5-(methoxymethoxy)-4-methylbenzene (19.18 g) obtained in Reference Example 202, concentrated hydrochloric acid (1.5 mL), and methanol (150 mL) was stirred at 60°C for 5 hr. The reaction mixture was cooled to room temperature and concentrated. The residue was diluted with ethyl acetate, washed with aqueous sodium hydrogen carbonate solution and saturated brine, and dried over anhydrous magnesium sulfate. After filtration, the filtrate was concentrated and the residue was crystallized from hexane-diisopropyl ether to give the title compound (9.77 g, yield 59%) as colorless crystals.
¹H-NMR (300 MHz, CDCl₃)δ:2.15 (s, 3 H), 4.75 (s, 1 H), 5.09 (s, 2 H), 6.44 (s, 1 H), 7.27 - 7.48 (m, 6 H).

### Reference Example 204 5-[5-(benzyloxy)-4-bromo-2-methylphenoxy]-1,3-dimethyl-1H-pyrazole-4-carbaldehyde

The title compound was obtained in the same manner as in Reference Example 2 and from 5-(benzyloxy)-4-bromo-2-methylphenol obtained in Reference Example 203 and 5-chloro-1,3-dimethyl-1H-pyrazole-4-carbaldehyde.
¹H-NMR (300 MHz, CDCl₃)δ:2.27 (s, 3 H), 2.46 (s, 3 H), 3.51 (s, 3 H), 5.00 (s, 2 H), 6.22 (s, 1 H), 7.27 - 7.45 (m, 6 H), 9.33 (s, 1 H).

### Reference Example 205 5-[5-(benzyloxy)-4-bromo-2-methylphenoxy]-1,3-dimethyl-1H-pyrazole-4-carbonitrile

A mixture of 5-[5-(benzyloxy)-4-bromo-2-methylphenoxy]-1,3-dimethyl-1H-pyrazole-4-carbaldehyde (1.00 g) obtained in Reference Example 204, hydroxylamine hydrochloride (201 mg), sodium acetate (297 mg) and methanol (10 mL) was stirred at room temperature for 15 hr, and poured into water, and the mixture was extracted with ethyl acetate. The organic layer was washed with saturated brine, and dried over anhydrous magnesium sulfate. After filtration, the filtrate was concentrated. Acetic anhydride (5 mL) was added to the residue, and the mixture was heated under reflux for 3 hr. After cooling to room temperature, the reaction mixture was concentrated. The residue was crystallized from hexane-ethyl acetate to give the title compound (785 mg, yield 79%) as colorless crystals.
¹H-NMR (300 MHz, CDCl₃)δ:2.21 (s, 3 H), 2.29 (s, 3 H), 3.64 (s, 3 H), 5.08 (s, 2 H), 6.52 (s, 1 H), 7.28 - 7.43 (m, 5 H), 7.47 (s, 1 H).

### Reference Example 206 5-(4-bromo-5-hydroxy-2-methylphenoxy)-1,3-dimethyl-1H-pyrazole-4-carbonitrile

The title compound was obtained in the same manner as in Reference Example 113 and from 5-[5-(benzyloxy)-4-bromo-2-methylphenoxy]-1,3-dimethyl-1H-pyrazole-4-carbonitrile obtained in Reference Example 205.
¹H-NMR (300 MHz, CDCl₃)δ:2.24 (s, 3 H), 2.29 (s, 3 H), 3.70 (s, 3 H), 5.55 (s, 1 H), 6.60 (s, 1 H), 7.37 (s, 1 H).

### Reference Example 207 2-bromo-5-[(4-ethyl-1,3-dimethyl-1H-pyrazol-5-yl)oxy]-4-methylphenol

The title compound was obtained in the same manner as in Reference Example 169 and from 5-[5-(benzyloxy)-4-bromo-2-methylphenoxy]-1,3-dimethyl-1H-pyrazole-4-carbaldehyde obtained in Reference Example 204.
¹H-NMR (300 MHz, DMSO-d₆)δ:0.93 (t, J = 7.5 Hz, 3 H), 2.07 - 2.20 (m, 5 H), 2.22 (s, 3 H), 3.44 (s, 3 H), 6.20 (s, 1 H), 7.38 (s, 1 H), 10.08 (s, 1 H).

### Reference Example 208 5-[5-(benzyloxy)-4-bromo-2-methylphenoxy]-3-cyclopropyl-1-methyl-1H-pyrazole-4-carbaldehyde

While stirring a solution of 5-(benzyloxy)-4-bromo-2-methylphenol (14.00 g) obtained in Reference Example 203, and 5-chloro-3-cyclopropyl-1-methyl-1H-pyrazole-4-carbaldehyde (8.82 g) obtained in Reference Example 166 in N,N-dimethylformamide (60 mL), potassium carbonate (8.58 g) was added. The reaction mixture was stirred under a nitrogen atmosphere at 120°C for 15 hr. The reaction mixture was cooled to room temperature, and diluted with ethyl acetate, washed with water and saturated brine, and dried over anhydrous magnesium sulfate. After filtration, the filtrate was concentrated and the residue was subjected to silica gel column chromatography (hexane-ethyl acetate 85:15 - 75:25, v/v), and crystallized from hexane-ethyl acetate to give the title compound (16.51 g, yield 78%) as colorless crystals.
¹H-NMR (300 MHz, CDCl₃)δ:0.97 - 1.03 (m, 4 H), 2.27 (s, 3 H), 2.39 - 2.50 (m, 1 H), 3.44 (s, 3 H), 5.00 (s, 2 H), 6.18 (s, 1 H), 7.25 - 7.39 (m, 5 H), 7.43 (s, 1 H), 9.44 (s, 1 H).

### Reference Example 209 2-bromo-5-[(3-cyclopropyl-4-ethyl-1-methyl-1H-pyrazol-5-yl)oxy]-4-methylphenol

The title compound was obtained in the same manner as in Reference Example 169 and from 5-[5-(benzyloxy)-4-bromo-2-methylphenoxy]-3-cyclopropyl-1-methyl-1H-pyrazole-4-carbaldehyde obtained in Reference Example 208.
¹H-NMR (300 MHz, DMSO-d₆)δ:0.69 - 0.85 (m, 4 H), 0.99 (t, J = 7.5 Hz, 3 H), 1.72 - 1.84 (m, 1 H), 2.18 - 2.30 (m, 5 H), 3.41 (s, 3 H), 6.20 (s, 1 H), 7.37 (s, 1 H), 10.09 (s, 1 H).

### Reference Example 210 {5-[5-(benzyloxy)-4-bromo-2-methylphenoxy]-3-cyclopropyl-1-methyl-1H-pyrazol-4-yl}methanol

The title compound was obtained in the same manner as in Reference Example 100 and from 5-[5-(benzyloxy)-4-bromo-2-methylphenoxy]-3-cyclopropyl-1-methyl-1H-pyrazole-4-carbaldehyde obtained in Reference Example 208.
¹H-NMR (300 MHz, CDCl₃)δ:0.93 (d, J = 6.6 Hz, 4 H), 0.99 (t, J = 5.5 Hz, 1 H), 1.84 - 1.95 (m, 1 H), 2.26 (s, 3 H), 3.43 (s, 3 H), 4.20 (d, J = 5.5 Hz, 2 H), 5.00 (s, 2 H), 6.19 (s, 1 H), 7.24 - 7.39 (m, 6 H).

### Reference Example 211 2-bromo-5-[(3-cyclopropyl-1,4-dimethyl-1H-pyrazol-5-yl)oxy]-4-methylphenol

The title compound was obtained in the same manner as in Reference Example 60 and from {5-[5-(benzyloxy)-4-bromo-2-methylphenoxy]-3-cyclopropyl-1-methyl-1H-pyrazol-4-yl}methanol obtained in Reference Example 210.
¹H-NMR (300 MHz, DMSO-d₆)δ:0.68 - 0.86 (m, 4 H), 1.70 - 1.82 (m, 4 H), 2.21 (s, 3 H), 3.42 (s, 3 H), 6.17 (s, 1 H), 7.35 (s, 1 H), 10.35 (br s, 1 H).

### Reference Example 212 5-[5-(benzyloxy)-4-bromo-2-methylphenoxy]-3-cyclopropyl-1-methyl-1H-pyrazole-4-carbonitrile

A mixture of 5-[5-(benzyloxy)-4-bromo-2-methylphenoxy]-3-cyclopropyl-1-methyl-1H-pyrazole-4-carbaldehyde (5.00 g) obtained in Reference Example 208, hydroxylamine hydrochloride (945 mg), sodium acetate (1394 mg), and methanol (50 mL) was stirred at 60°C for 3 hr, and concentrated. Ethyl acetate was added to the residue, and the mixture was washed with water and saturated brine, and dried over anhydrous magnesium sulfate. After filtration, the filtrate was concentrated. Acetic anhydride (10 mL) was added to the residue, and the mixture was heated under reflux for 5 hr. After cooling to room temperature, the reaction mixture was concentrated. The residue was subjected to silica gel column chromatography (hexane-ethyl acetate 85:15 - 75:25, v/v), and crystallized from hexane-ethyl acetate to give the title compound (4337 mg, yield 87%) as colorless crystals.
¹H-NMR (300 MHz, CDCl₃)δ:0.94 - 1.01 (m, 4 H), 1.84 - 1.95 (m, J = 6.7, 6.7 Hz, 1 H), 2.21 (s, 3 H), 3.59 (s, 3 H), 5.09 (s, 2 H), 6.50 (s, 1 H), 7.29 - 7.44 (m, 5 H), 7.46 (s, 1 H).

### Reference Example 213 5-(4-bromo-5-hydroxy-2-methylphenoxy)-3-cyclopropyl-1-methyl-1H-pyrazole-4-carbonitrile

5-[5-(Benzyloxy)-4-bromo-2-methylphenoxy]-3-cyclopropyl-1-methyl-1H-pyrazole-4-carbonitrile (4.27 g) obtained in Reference Example 212 was dissolved in a mixed solvent of ethanol (60 mL) and tetrahydrofuran (60 mL), palladium carbon ethylenediamine complex (400 mg) was added, and the mixture was stirred under a hydrogen atmosphere (1 atm) at room temperature for 40 hr. The catalyst was removed by filtration, and the filtrate was concentrated. The residue was crystallized from hexane-ethyl acetate to give the title compound (2086 mg, yield 62%) as colorless crystals.
¹H-NMR (300 MHz, CDCl₃)δ:0.94 - 1.00 (m, 4 H), 1.85 - 1.96 (m, 1 H), 2.24 (s, 3 H), 3.66 (s, 3 H), 5.51 (s, 1 H), 6.59 (s, 1 H), 7.37 (s, 1 H).

### Reference Example 214 5-chloro-2-(methoxymethoxy)-4-[(1,3,4-trimethyl-1H-pyrazol-5-yl)oxy]benzaldehyde

The title compound was obtained in the same manner as in Reference Example 201 and from 5-chloro-2-hydroxy-4-[(1,3,4-trimethyl-1H-pyrazol-5-yl)oxy]benzaldehyde obtained in Reference Example 119.
¹H-NMR (300 MHz, CDCl₃)δ:1.78 (s, 3 H), 2.21 (s, 3 H), 3.44 (s, 3 H), 3.61 (s, 3 H), 5.13 (s, 2 H), 6.58 (s, 1 H), 7.94 (s, 1 H), 10.32 (s, 1 H).

### Reference Example 215 3-{5-chloro-2-(methoxymethoxy)-4-[(1,3,4-trimethyl-1H-pyrazol-5-yl)oxy]phenyl}propan-1-ol

To a solution of ethyl (2E)-3-{5-chloro-2-(methoxymethoxy)-4-[(1,3,4-trimethyl-1H-pyrazol-5-yl)oxy]phenyl}acrylate (2.30 g) obtained in the below-mentioned Example 258, which was cooled to 0°C in an ice bath, in tetrahydrofuran (20 mL) was added lithium aluminum hydride (244 mg), and the mixture was stirred at 0°C for 1 hr. To the reaction mixture was carefully added 1N aqueous sodium hydroxide solution (1.2 mL), and the mixture was stirred at room temperature for 10 hr. The insoluble material was removed by filtration. The filtrate was concentrated, and the residue was subjected to silica gel column chromatography (hexane-ethyl acetate 75:25 - 25:75, v/v) to give the title compound (490 mg, yield 24%) as a colorless oil.
¹H-NMR (300 MHz, CDCl₃)δ:1.74 (s, 3 H), 1.79 - 1.89 (m, 2 H), 2.18 (s, 3 H), 2.62 - 2.71 (m, 2 H), 3.40 (s, 3 H), 3.58 - 3.69 (m, 5 H), 5.03 (s, 2 H), 6.52 (s, 1 H), 7.21 (s, 1 H).

### Reference Example 216 4-chloro-2-(3-hydroxypropyl)-5-[(1,3,4-trimethyl-1H-pyrazol-5-yl)oxy]phenol

The title compound was obtained in the same manner as in Reference Example 110 and from 3-{5-chloro-2-(methoxymethoxy)-4-[(1,3,4-trimethyl-1H-pyrazol-5-yl)oxy]phenyl}propan-1-ol obtained in Reference Example 215.
¹H-NMR (300 MHz, CDC1₃)δ:1.77 (s, 3 H), 1.81 - 1.91 (m, 2 H), 2.10 (s, 3 H), 2.72 (t, J= 6.8 Hz, 2 H), 3.50 (s, 3 H), 3.60 - 3. 67 (m, 2 H), 6.15 (s, 1 H), 7. 16 (s, 1 H), 9.72 (s, 1 H).

### Reference Example 217 {5-chloro-2-(methoxymethoxy)-4-[(1,3,4-trimethyl-1H-pyrazol-5-yl)oxy]phenyl}methanol

The title compound was obtained in the same manner as in Reference Example 100 and from 5-chloro-2-(methoxymethoxy)-4-[(1,3,4-trimethyl-1H-pyrazol-5-yl)oxy]benzaldehyde obtained in Reference Example 214.
¹H-NMR (300 MHz, CDCl₃)δ:1.75 (s, 3 H), 2.11 (t, J = 6.2 Hz, 1 H), 2.18 (s, 3 H), 3.41 (s, 3 H), 3.60 (s, 3 H), 4.64 (d, J = 6.1 Hz, 2 H), 5.05 (s, 2 H), 6.52 (s, 1 H), 7.41 (s, 1 H).

### Reference Example 218 5-[2-chloro-5-(methoxymethoxy)-4-(phenoxymethyl)phenoxy]-1,3,4-trimethyl-1H-pyrazole

To a solution, which was cooled to 0°C in an ice bath, of {5-chloro-2-(methoxymethoxy)-4-[(1,3,4-trimethyl-1H-pyrazol-5-yl)oxy]phenyl}methanol (350 mg) obtained in Reference Example 217 phenol (121 mg) and tributylphosphine (0.773 mL) in tetrahydrofuran (15 mL) was added 1,1'-(azodicarbonyl)dipiperidine (810 mg). The mixture was stirred at room temperature for 40 hr. To the reaction mixture was added hexane, and the insoluble material was removed by filtration. The filtrate was concentrated, and the residue was subjected to silica gel column chromatography (hexane-ethyl acetate 80:20 - 50:50, v/v) to give the title compound as a colorless oil (0.430 g, yield 100%).
¹H-NMR (300 MHz, CDCl₃)δ:1.76 (s, 3 H), 2.19 (s, 3 H), 3.40 (s, 3 H), 3.61 (s, 3 H), 5.03 (s, 2 H), 5.05 (s, 2 H), 6.55 (s, 1 H), 6.94 - 7.04 (m, 3 H), 7.25 - 7.36 (m, 2 H), 7.55 (s, 1 H).

### Reference Example 219 4-chloro-2-(phenoxymethyl)-5-[(1,3,4-trimethyl-1H-pyrazol-5-yl)oxy]phenol

The title compound was obtained in the same manner as in Reference Example 110 and from 5-[2-chloro-5-(methoxymethoxy)-4-(phenoxymethyl)phenoxy]-1,3,4-trimethyl-1H-pyrazole obtained in Reference Example 218.
¹H-NMR (300 MHz, CDCl₃)δ:1.79 (s, 3 H), 2.12 (s, 3 H), 3.51 (s, 3 H), 5.10 (s, 2 H), 6.18 (s, 1 H), 6.95 - 7.06 (m, 3 H), 7.27 - 7.36 (m, 2 H), 7 . 43 (s, 1 H), 9.46 (br s, 1 H).

### Reference Example 220 5-[5-(benzyloxy)-4-bromo-2-methylphenoxy]-1,3,4-trimethyl-1H-pyrazole

The title compound was obtained in the same manner as in Reference Example 202 and from 5-[5-(benzyloxy)-4-bromo-2-methylphenoxy]-1,3-dimethyl-1H-pyrazole-4-carbaldehyde obtained in Reference Example 204.
¹H-NMR (300 MHz, CDCl₃)δ:1.53 (s, 3 H), 2.19 (s, 3 H), 2.27 (s, 3 H), 3.47 (s, 3 H), 4.97 (s, 2 H), 6.10 (s, 1 H), 7.27 - 7.39 (m, 6 H).

### Reference Example 221 2-bromo-4-methyl-5-[(1,3,4-trimethyl-1H-pyrazol-5-yl)oxy]phenol

The title compound was obtained in the same manner as in Reference Example 113 and from 5-[5-(benzyloxy)-4-bromo-2-methylphenoxy]-1,3,4-trimethyl-1H-pyrazole obtained in Reference Example 220.
¹H-NMR (300 MHz, DMSO-d₆)δ:1.69 (s, 3 H), 2.08 (s, 3 H), 2.22 (s, 3 H), 3.46 (s, 3 H), 6.21 (s, 1 H); 7.38 (s, 1 H); 10.06 (s, 1 H).

### Reference Example 223 (5-{2,4-dichloro-5-[(4-methoxybenzyl)oxy]phenoxy}-1,3-dimethyl-1H-pyrazol-4-yl)acetic acid

To a solution of (5-{2,4-dichloro-5-[(4-methoxybenzyl)oxy]phenoxy}-1,3-dimethyl-1H-pyrazol-4-yl)acetonitrile (8.13 g) obtained in Reference Example 88 in ethanol (50 mL) was added 8N aqueous sodium hydroxide solution (19 mL), and the mixture was heated under reflux with stirring for 3 hr. The reaction mixture was cooled to room temperature, neutralized with 6N hydrochloric acid, and extracted with ethyl acetate. The organic layer was washed with saturated brine, and dried over anhydrous magnesium sulfate. After filtration, the filtrate was concentrated to give the title compound as a white solid (7.63 g, yield 90%). Recrystallization from hexane-ethyl acetate gave white crystals.
¹H-NMR (300 MHz, DMSO-d₆)δ:2.10 (s, 3 H), 3.07 (s, 2 H), 3.38 (s, 3 H), 3.76 (s, 3 H), 4.99 (s, 2 H), 6.75 (br s, 1 H), 6.91 (d, J = 8. 5 Hz, 2 H), 7.24 (d, J = 8 .7 Hz, 2 H), 7.71 (s, 1 H).

### Reference Example 224 2,4-dichloro-5-{[4-(2-hydroxyethyl)-1,3-dimethyl-1H-pyrazol-5-yl]oxy}phenol

Borane (1.0M tetrahydrofuran solution, 40 mL) was added at 0°C to (5-{2,4-dichloro-5-[(4-methoxybenzyl)oxy]phenoxy}-1,3-dimethyl-1H-pyrazol-4-yl)acetic acid (5.63 g) obtained in Reference Example 223, and the mixture was stirred for 1 hr. Aqueous citric acid solution (10%, w/w) was added to the reaction mixture, and the mixture was extracted with ethyl acetate. The organic layer was washed with aqueous citric acid solution (10%, w/w) and saturated brine, and dried over anhydrous magnesium sulfate. After filtration, the filtrate was concentrated and the residue was dissolved in a mixed solvent of tetrahydrofuran (10 mL) and methanol (10 mL). 4N Hydrochloric acid-ethyl acetate solution (4 mL) was added, and the mixture was stirred at 50°C for 3 hr. The reaction mixture was cooled to room temperature, and diluted with ethyl acetate, and the organic layer was washed with water, saturated aqueous sodium hydrogen carbonate solution and saturated brine, and dried over anhydrous magnesium sulfate. After filtration, the filtrate was concentrated and the residue was subjected to silica gel column chromatography (hexane-ethyl acetate 9:1 - ethyl acetate alone, v/v) to give the title compound as a white solid (0.78 g, yield 20%).
¹H-NMR (300 MHz, CDCl₃)δ:1.75 (br s, 1 H), 2.15 (s, 3 H), 2.47 (t, J = 6.8 Hz, 2 H), 3.50 (s, 3 H), 3.65 (t, J = 6.7 Hz, 2 H), 6.25 (s, 1 H), 7.43 (s, 1 H), 9.91 (br s, 1 H).

### Reference Example 225 ethyl 3-[5-(2,4-dichloro-5-hydroxyphenoxy)-1,3-dimethyl-1H-pyrazol-4-yl]propionate

To a solution of ethyl (2E)-3-[5-(2,4-dichloro-5-hydroxyphenoxy)-1,3-dimethyl-1H-pyrazol-4-yl]acrylate (9.86 g) obtained in Reference Example 151 in tetrahydrofuran (50 mL) and ethanol (50 mL) was added palladium carbon ethylenediamine complex (4.04 g), and the mixture was stirred under a hydrogen atmosphere (1 atm) at room temperature for 80 hr. The catalyst was removed by filtration, and the filtrate was concentrated. The residue was crystallized from hexane-ethyl acetate to give the title compound as white crystals (7.47 g, 75%).
¹H-NMR (300 MHz, CDCl₃)δ:1.22 (t, J = 7.2 Hz, 3 H), 2.17 (s, 3 H), 2.41 (t, J = 7.2 Hz, 2 H), 2.54 (t, J = 7.5 Hz, 2 H), 3.50 (s, 3 H), 4.09 (q, J = 7.2 Hz, 2 H), 6.26 (s, 1 H), 7.43 (s, 1 H), 8.46 (br s, 1 H).

### Reference Example 226 2,4-dichloro-5-{[4-(3-hydroxypropyl)-1,3-dimethyl-1H-pyrazol-5-yl]oxy}phenol

To a solution of ethyl 3-[5-(2,4-dichloro-5-hydroxyphenoxy)-1,3-dimethyl-1H-pyrazol-4-yl]propionate (7.09 g) obtained in Reference Example 225 in tetrahydrofuran (60 mL) was added diisobutylaluminum hydride (1.5M toluene solution, 44 mL) at 0°C, and the mixture was stirred for 1 hr. Saturated aqueous ammonium chloride solution (13 mL) was added to the reaction mixture at 0°C, and the mixture was stirred at room temperature for 2 hr. The resulting solid was removed by filtration, and the filtrate was concentrated. The residue was crystallized from hexane-ethyl acetate to give the title compound as white crystals (4.22 g, 67%).
¹H-NMR (300 MHz, CDCl₃)δ:1.61 - 1.73 (m, 2 H), 2.15 (s, 3 H), 2.30 (t, J = 7.4 Hz, 2 H), 3.51 (s, 3 H), 3.59 (t, J = 6.2 Hz, 2 H), 6.26 (s, 1 H), 7.43 (s, 1 H).

### Reference Example 236 5-[(2,4-dichloro-5-hydroxyphenyl)thio]-1,3-dimethyl-1H-pyrazole-4-carbaldehyde oxime

The title compound was obtained in the same manner as in Reference Example 132 and from 5-[(2,4-dichloro-5-hydroxyphenyl)thio]-1,3-dimethyl-1H-pyrazole-4-carbaldehyde obtained in Reference Example 130.
¹H-NMR (300 MHz, CDCl₃)δ:2.43 (s, 3 H), 3.80 (s, 3 H), 6.08 (s, 1 H), 6.46 - 6.61 (m, 1 H), 7.20 (s, 1 H), 7.34 - 7.39 (m, 1 H), 8.12 (s, 1 H).

### Reference Example 237 5-[(2,4-dichloro-5-hydroxyphenyl)thio]-1,3-dimethyl-1H-pyrazole-4-carbonitrile

A mixture of 5-[(2,4-dichloro-5-hydroxyphenyl)thio]-1,3-dimethyl-1H-pyrazole-4-carbaldehyde oxime (5.00 g) obtained in Reference Example 236, and acetic anhydride (3 mL) was heated under reflux for 5 hr. After cooling to room temperature, the reaction mixture was concentrated. The residue was dissolved in a mixed solvent of tetrahydrofuran (14 mL) and water (6 mL). Lithium hydroxide monohydrate (380 mg) was added, and the mixture was stirred at room temperature for 1 hr. The reaction mixture was neutralized with 0.1N hydrochloric acid (91 mL), and extracted with ethyl acetate. The organic layer was washed with saturated brine, dried over anhydrous magnesium sulfate and filtered. The filtrate was concentrated, and the residue was crystallized from toluene to give the title compound as brown crystals (358 mg, yield 76%).
¹H-NMR (300 MHz, DMSO-d₆)δ:2.36 (s, 3 H), 3.83 (s, 3 H), 6.40 (s, 1 H), 7.64 (s, 1 H), 10.69 (s, 1 H).

### Reference Example 238 5-{2,4-dichloro-5-[(4-methoxybenzyl)oxy]phenoxy}-1,3-dimethyl-1H-pyrazole-4-carboxylic acid

While stirring a solution of 5-{2,4-dichloro-5-[(4-methoxybenzyl)oxy]phenoxy}-1,3-dimethyl-1H-pyrazole-4-carbaldehyde (6.00 g) obtained in Reference Example 2 in acetone (120 mL) at 0°C, Jones reagent (12.2 mL) (mixture of chrome trioxide (3.66 g), conc. sulfuric acid (3.20 mL) and water (13.7 mL)) was added. The mixture was stirred at the same temperature for 15 min, and for 4 hr while heating the mixture to room temperature. The reaction mixture was cooled again to 0°C, Jones reagent (4.00 mL) was added, and the mixture was stirred for 2 hr while heating the mixture to room temperature. Water (400 mL) was added to the reaction mixture and the mixture was stirred for 1 hr. The resulting solid was filtered, and washed with water and diisopropyl ether. The obtained solid was dried to give the title compound (5.63 g, yield 91%) as a pale-yellow solid.
¹H-NMR (300 MHz, CDCl₃)δ:2.48 (s, 3 H), 3.55 (s, 3 H), 3.79 (s, 3 H), 4.92 (s, 2 H), 6.22 (s, 1 H), 6.78 - 6.88 (m, 2 H), 7.16 (d, J = 8.9 Hz, 2 H), 7.44 (s, 1 H).

### Reference Example 239 5-(2,4-dichloro-5-hydroxyphenoxy)-1,3-dimethyl-1H-pyrazole-4-carboxamide

A mixture of 5-{2,4-dichloro-5-[(4-methoxybenzyl)oxy]phenoxy}-1,3-dimethyl-1H-pyrazole-4-carboxylic acid (383 mg) obtained in Reference Example 238, N-ethyl-N'-(3-dimethylaminopropyl)carbodiimide hydrochloride (203 mg), 1-hydroxybenzotriazole ammonium salt (161 mg) and N,N-dimethylformamide (20 mL) was stirred at room temperature for 6 hr. Water was added to the reaction mixture, and the mixture was extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 100:0 - 30:70, v/v) to give a mixture containing 5-{2,4-dichloro-5-[(4-methoxybenzyl)oxy]phenoxy}-1,3-dimethyl-1H-pyrazole-4-carboxamide as a main component.
10% Hydrochloric acid-methanol solution (25 mL) was added to the obtained mixture, and the mixture was stirred at 50°C for 5 hr. The solvent was evaporated, water was added to the residue, and the mixture was extracted with ethyl acetate. The organic layer was dried over anhydrous magnesium sulfate. After filtration, the filtrate was concentrated. The resulting solid was washed with diisopropyl ether, filtered, and dried to give the title compound (162 mg, yield 58%) as a white solid.
¹H-NMR (300 MHz, CDCl₃)δ:2.45 (s, 3 H), 3.58 (s, 3 H), 5.24 (br s, 1 H), 5.89 (br s, 1 H), 6.38 (s, 1 H), 7.46 (s, 1 H).

### Reference Example 240 5-{2,4-dichloro-5-[(4-methoxybenzyl)oxy]phenoxy}-1,3-dimethyl-4-(pyrrolidin-1-ylcarbonyl)-1H-pyrazole

A mixture of 5-{2,4-dichloro-5-[(4-methoxybenzyl)oxy]phenoxy}-1,3-dimethyl-1H-pyrazole-4-carboxylic acid (383 mg) obtained in Reference Example 238, N-ethyl-N'-(3-dimethylaminopropyl)carbodiimide hydrochloride (203 mg), 1-hydroxybenzotriazole (144 mg), pyrrolidine (94 mg) and N,N-dimethylformamide (20 mL) was stirred at room temperature for 12 hr. Water was added to the reaction mixture, and the mixture was extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 80:20 - 0:100, v/v) to give the title compound (420 mg, yield 97%) as a white solid.
¹H-NMR (300 MHz, CDCl₃)δ:1.74 - 1.85 (m, 4 H), 2.34 (s, 3 H), 3.29 - 3.41 (m,4 H), 3.55 (s, 3 H), 3.81 (s, 3 H), 5.00 (s, 2 H), 6.66 (s, 1 H), 6.82 - 6.89 (m, 2 H), 7.30 (d, J = 8.9 Hz, 2 H), 7.40 (s, 1 H).

### Reference Example 241 2,4-dichloro-5-{[1,3-dimethyl-4-(pyrrolidin-1-ylcarbonyl)-1H-pyrazol-5-yl]oxy}phenol

10% Hydrochloric acid-methanol solution (25 mL) was added to 5-{2,4-dichloro-5-[(4-methoxybenzyl)oxy]phenoxy}-1,3-dimethyl-4-(pyrrolidin-1-ylcarbonyl)-1H-pyrazole (420 mg) obtained in Reference Example 240, and the mixture was stirred at 50°C for 5 hr. The solvent was evaporated, water was added to the residue, and the mixture was extracted with ethyl acetate. The organic layer was dried over anhydrous magnesium sulfate. After filtration, the filtrate was concentrated and the residue was subjected to silica gel column chromatography (ethyl acetate-methanol 100:0 - 90:10, v/v) to give the title compound (231 mg, yield 73%) as a pale-yellow solid.
¹H-NMR (300 MHz, CDCl₃)δ:1.43 - 2.01 (m, 4 H), 2.19 (br s, 3 H), 2.77 - 4.03 (m, 7 H), 6.75 (br s, 1 H), 7.36 (s, 1 H), 9.54 (br s, 1 H).

### Reference Example 242 ethyl (2E)-3-(5-{2,4-dichloro-5-[(4-methoxybenzyl)oxy]phenoxy}-1,3-dimethyl-1H-pyrazol-4-yl)acrylate

The title compound was obtained in the same manner as in Reference Example 151 and from 5-{2,4-dichloro-5-[(4-methoxybenzyl)oxy]phenoxy}-1,3-dimethyl-1H-pyrazole-4-carbaldehyde obtained in Reference Example 2.
¹H-NMR (300 MHz, CDCl₃)δ:1.28 (t, J = 7.0 Hz, 3 H), 2.40 (s, 3 H), 3.46 (s, 3 H), 3.81 (s, 3 H), 4.19 (q, J = 7.2 Hz, 2 H), 4.89 (s, 2 H), 5.96 (d, J = 16.2 Hz, 1 H), 6.17 (s, 1 H), 6.78 - 6.89 (m, 2 H), 7.15 (d, J = 8.7 Hz, 2 H), 7.33 (d, J = 16.2 Hz, 1 H), 7.47 (s, 1 H).

### Reference Example 243 2,4-dichloro-5-({4-[2-(1-hydroxycyclopropyl)ethyl]-1,3-dimethyl-1H-pyrazol-5-yl}oxy)phenol

While stirring a solution of ethyl (2E)-3-(5-{2,4-dichloro-5-[(4-methoxybenzyl)oxy]phenoxy}-1,3-dimethyl-1H-pyrazol-4-yl)acrylate (500 mg) obtained in Reference Example 242 and titanium tetraisopropoxide (1.08 mL) in tetrahydrofuran (10 mL) at 0°C, ethylmagnesium bromide (3 mol/L diethyl ether solution, 2.1 mL) was added, and the mixture was stirred at the same temperature for 30 min and for 4 hr while heating to room temperature. The reaction mixture was acidified with water (8 mL) and 1N hydrochloric acid (10 mL), and the organic solvent was evaporated, and the residue was extracted with ethyl acetate, and dried over anhydrous magnesium sulfate. After filtration, the filtrate was concentrated and the residue was subjected to silica gel column chromatography (hexane-ethyl acetate 100:0 - 50:50, v/v) to give a mixture of 1-[(E)-2-(5-12,4-dichloro-5-[(4-methoxybenzyl)oxy]phenoxy}-1,3-dimethyl-1H-pyrazol-4-yl)vinyl]cyclopropanol and 1-[2-(5-{2,4-dichloro-5-[(4-methoxybenzyl)oxy]phenoxy}-1,3-dimethyl-1H-pyrazol-4-yl)ethyl]cyclopropanol.
The mixture was dissolved in ethanol (30 mL), palladium carbon ethylenediamine complex (30 mg) was added, and the mixture was stirred under a hydrogen atmosphere (1 atm) at room temperature for 20 hr. The catalyst was removed by filtration, and the filtrate was concentrated. The residue was subjected to silica gel column chromatography (hexane-ethyl acetate 90:10 - 20:80, v/v) to give the title compound (137 mg, yield 38%) as a white solid.
¹H-NMR (300 MHz, DMSO-d₆)δ:0.84 (t, J = 7.4 Hz, 3 H), 2.10 (s, 3 H), 2.22 - 2.39 (m, 4 H), 3.32 (s, 1 H), 3.46 (s, 3 H), 6.34 (s, 1 H), 7.60 (s, 1 H), 10.67 (br s, 1 H).

### Reference Example 244 2,4-dichloro-5-({4-[(1E)-3-hydroxy-3-methylbut-1-en-1-yl]-1,3-dimethyl-1H-pyrazol-5-yl}oxy)phenol

To a solution of ethyl (2E)-3-[5-(2,4-dichloro-5-hydroxyphenoxy)-1,3-dimethyl-1H-pyrazol-4-yl]acrylate (500 mg) obtained in Reference Example 151 in tetrahydrofuran (10 mL) was added methylmagnesium bromide (1 mol/L tetrahydrofuran solution, 5.4 mL) at -78°C, and the mixture was stirred for 1 hr while heating to 0°C. The reaction mixture was cooled to - 78°C again, methylmagnesium bromide (1 mol/L tetrahydrofuran solution, 10.8 mL) was added, and the mixture was stirred at the same temperature for 30 min. Water was added to the reaction mixture, and the mixture was stirred while heating to room temperature for 2 hr, and carefully neutralized with 1N hydrochloric acid. The mixture was extracted with ethyl acetate, and dried over anhydrous magnesium sulfate. After filtration, the filtrate was concentrated and the residue was subjected to silica gel column chromatography (hexane-ethyl acetate 90:10 - 10:90, v/v) to give the title compound (389 mg, yield 81%) as a colorless amorphous solid.
¹H-NMR (300 MHz, CDCl₃)δ:1.26 (s, 6 H), 1.39 (s, 1 H), 2.21 (s, 3H), 3.54 (s, 3 H), 5.92 (d, J = 16.5 Hz, 1 H), 6.17 (d, J = 16.5 Hz, 1H), 6.23 (s, 1 H), 7.44 (s, 1 H), 8.66 (br s, 1 H).

### Reference Example 245 2,4-dichloro-5-({4-[(E)-2-(1-hydroxycyclopropyl)vinyl]-1,3-dimethyl-1H-pyrazol-5-yl}oxy)phenol

Under an argon atmosphere, while stirring a solution of ethyl (2E)-3-[5-(2,4-dichloro-5-hydroxyphenoxy)-1,3-dimethyl-1H-pyrazol-4-yl]acrylate (600 mg) obtained in Reference Example 151 and titanium tetraisopropoxide (1.92 mL) in tetrahydrofuran (10 mL) at -78°C, ethylmagnesium bromide (3 mol/L diethyl ether solution, 4.3 mL) was added, and the mixture was stirred at the same temperature for 1 hr and while heating to 0°C for 2 hr. The reaction mixture was acidified with water and 1N hydrochloric acid, and extracted with ethyl acetate. The organic layer was dried over anhydrous magnesium sulfate. After filtration, the filtrate was concentrated and the residue was subjected to silica gel column chromatography (hexane-ethyl acetate 100:0 - 80:20, v/v) to give the title compound (170 mg, yield 30%) as a white solid.
¹H-NMR (300 MHz, CDCl₃)δ:0.62 - 0.71 (m, 2 H), 1.02 - 1.11 (m, 2 H), 2.01 (s, 1 H), 2.21 (s, 3 H), 3.54 (s, 3 H), 5.56 (d, J = 16.2 Hz,1 H), 6.22 (d, J = 16.2 Hz, 1 H), 6.24 (s, 1 H), 7.43 (s, 1 H), 8.51 (br s, 1 H).

### Reference Example 246 5-(5-{2,4-dichloro-5-[(4-methoxybenzyl)oxy]phenoxy}-1,3-dimethyl-1H-pyrazol-4-yl)-3-methyl-1,2,4-oxadiazole

While stirring a solution of 5-{2,4-dichloro-5-[(4-methoxybenzyl)oxy]phenoxy}-1,3-dimethyl-1H-pyrazole-4-carboxylic acid (800 mg) obtained in Reference Example 238 in N,N-dimethylformamide (3.7 mL), a solution of N,N'-carbonyldiimidazole (326 mg) in N,N-dimethylformamide (2.0 mL) was added, and the mixture was stirred at room temperature for 30 min. A solution of N-hydroxyacetoamidine (149 mg) in N,N-dimethylformamide (3.7 mL) was added, and the mixture was stirred at room temperature for 3 hr. A solution of N,N'-carbonyldiimidazole (326 mg) in N,N-dimethylformamide (3.7 mL) was added again, and the mixture was stirred at 115°C for 6 hr. The reaction mixture was gradually cooled to room temperature, water was added, and the mixture was extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 100:0 - 50:50, v/v) to give the title compound (405 mg, yield 47%) as a white solid.
¹H-NMR (300 MHz, CDCl₃)δ:2.32 (s, 3 H), 2.59 (s, 3 H), 3.59 (s, 3 H), 3.81 (s, 3 H), 4.87 (s, 2 H), 6.21 (s, 1 H), 6.74 - 6.83 (m, 2 H), 7.11 (d, J = 8.9 Hz, 2 H), 7.46 (s, 1 H).

### Reference Example 247 2,4-dichloro-5-{[1,3-dimethyl-4-(3-methyl-1,2,4-oxadiazol-5-yl)-1H-pyrazol-5-yl]oxy}phenol

The title compound was obtained in the same manner as in Reference Example 50 and from 5-(5-{2,4-dichloro-5-[(4-methoxybenzyl)oxy]phenoxy}-1,3-dimethyl-1H-pyrazol-4-yl)-3-methyl-1,2,4-oxadiazole obtained in Reference Example 246.
¹H-NMR (300 MHz, CDCl₃)δ:2.34 (s, 3 H), 2.56 (s, 3 H), 3.70 (s, 3 H), 5.69 (s, 1 H), 6.35 (s, 1 H), 7.45 (s, 1 H).

### Reference Example 248 tert-butyl (2E)-3-[5-(2,4-dichloro-5-hydroxyphenoxy)-1,3-dimethyl-1H-pyrazol-4-yl]acrylate

The title compound was obtained in the same manner as in Reference Example 151 and from 5-(2,4-dichloro-5-hydroxyphenoxy)-1,3-dimethyl-1H-pyrazole-4-carbaldehyde obtained in Reference Example 52 and tert-butyl diethoxyphosphorylacetate.
¹H-NMR (300 MHz, CDCl₃)δ:1.48 (s, 9 H), 2.34 (s, 3 H), 3.57 (s, 3 H), 5.95 (d, J = 16.2 Hz, 1 H), 6.15 (s, 1 H), 6.29 (s, 1 H), 7.27 (d, J = 16.2 Hz, 1 H), 7.45 (s, 1 H).

### Reference Example 249 4-[(5-{2,4-dichloro-5-[(4-methoxybenzyl)oxy]phenoxy}-1,3-dimethyl-1H-pyrazol-4-yl)(hydroxy)methyl]benzonitrile

To a solution of 4-iodobenzonitrile (546 mg) in tetrahydrofuran (24 mL) was added isopropylmagnesium bromide (1.0 mol/L tetrahydrofuran solution, 2.48 mL) at -40°C, and the mixture was stirred at the same temperature for 1 hr. Then, 5-{2,4-dichloro-5-[(4-methoxybenzyl)oxy]phenoxy}-1,3-dimethyl-1H-pyrazole-4-carbaldehyde (1.00 g) obtained in Reference Example 2 was added, and the mixture was stirred while heating to room temperature for 6 hr. Water was added to the reaction mixture, and the mixture was extracted with ethyl acetate. The organic layer was dried over anhydrous magnesium sulfate. After filtration, the filtrate was concentrated and the residue was subjected to silica gel column chromatography (hexane-ethyl acetate 90:10 - 20:80, v/v) to give the title compound (525 mg, yield 42%) as a yellow oil.
¹H-NMR (300 MHz, CDCl₃)δ:1.75 (d, J = 4.0 Hz, 1 H), 2.20 (s, 3 H), 3.47 (s, 3 H), 3.81 (s, 3 H), 4.90 (dd, J = 12.3, 16.2 Hz, 2 H), 5.61 (d, J = 4.0 Hz, 1 H), 6.12 (s, 1 H), 6. 84 - 6.93 (m, 2 H), 7.14 - 7.23 (m, 2 H), 7.24 - 7.31 (m, 2 H), 7.37 (s, 1 H), 7.44 - 7.51 (m, 2 H).

### Reference Example 250 4-{[5-(2,4-dichloro-5-hydroxyphenoxy)-1,3-dimethyl-1H-pyrazol-4-yl]methyl}benzonitrile

The title compound was obtained in the same manner as in Reference Example 70 and from 4-[(5-{2,4-dichloro-5-[(4-methoxybenzyl)oxy]phenoxy}-1,3-dimethyl-1H-pyrazol-4-yl)(hydroxy)methyl]benzonitrile obtained in Reference Example 249.
¹H-NMR (300 MHz, CDCl₃)δ:2.16 (s, 3 H), 3.59 (s, 3 H), 3.59 (s, 2 H), 6.17 (s, 1 H), 7.12 (d, J = 8.3 Hz, 2 H), 7.34 (s, 1 H), 7.47 (d, J = 8.3 Hz, 2 H).

### Reference Example 256 N'-acetyl-5-{2,4-dichloro-5-[(4-methoxybenzyl)oxy]phenoxy}-1,3-dimethyl-1H-pyrazole-4-carbohydrazide

The title compound was obtained in the same manner as in Reference Example 240 and from 5-{2,4-dichloro-5-[(4-methoxybenzyl)oxy]phenoxy}-1,3-dimethyl-1H-pyrazole-4-carboxylic acid obtained in Reference Example 238 and acetohydrazide.
¹H-NMR (300 MHz, CDCl₃)δ:2.04 (s, 3 H), 2.53 (s, 3 H), 3.40 (s, 3 H), 3.82 (s, 3 H), 4.99 (s, 2 H), 6.29 (s, 1 H), 6.81 - 6.92 (m, 2 H), 7.18 (d, J = 8.7 Hz, 2 H), 7.47 (s, 1 H), 7.66 (d, J = 4.7 Hz, 1 H), 8.08 (d, J = 4.0 Hz, 1 H).

### Reference Example 257 2-(5-{2,4-dichloro-5-[(4-methoxybenzyl)oxy]phenoxy}-1,3-dimethyl-1H-pyrazol-4-yl)-5-methyl-1,3,4-thiadiazole

To a solution of N'-acetyl-5-{2,4-dichloro-5-[(4-methoxybenzyl)oxy]phenoxy}-1,3-dimethyl-1H-pyrazole-4-carbohydrazide (1.00 g) obtained in Reference Example 256 in tetrahydrofuran (20 mL) was added Lawesson's reagent (902 mg), and the mixture was heated under reflux for 3 hr with stirring. The reaction mixture was added to saturated aqueous sodium hydrogen carbonate solution, and the mixture was extracted with ethyl acetate. The organic layer was dried over anhydrous magnesium sulfate. After filtration, the filtrate was concentrated and the residue was subjected to silica gel column chromatography (hexane-ethyl acetate 90:10 - 30:70, v/v) to give the title compound (903 mg, yield 91%) as a white solid.
¹H-NMR (300 MHz, CDCl₃)δ:2.68 (s, 3 H), 2.69 (s, 3 H), 3.51 (s, 3 H), 3.79 (s, 3 H), 4.87 (s, 2 H), 6.14 (s, 1 H), 6.72 - 6.80 (m, 2 H), 7.04 - 7.12 (m, 2 H), 7.48 (s, 1 H).

### Reference Example 258 2,4-dichloro-5-{[1,3-dimethyl-4-(5-methyl-1,3,4-thiadiazol-2-yl)-1H-pyrazol-5-yl]oxy}phenol

The title compound was obtained in the same manner as in Reference Example 50 and from 2-(5-{2,4-dichloro-5-[(4-methoxybenzyl)oxy]phenoxy}-1,3-dimethyl-1H-pyrazol-4-yl)-5-methyl-1,3,4-thiadiazole obtained in Reference Example 257.
¹H-NMR (300 MHz, CDCl₃)δ:2.46 (s, 3 H), 2.71 (s, 3 H), 3.62 (s, 3 H), 6.37 (s, 1 H), 7.48 (s, 1 H), 8.20 (br s, 1 H).

### Reference Example 259 2-(5-{2,4-dichloro-5-[(4-methoxybenzyl)oxy]phenoxy}-1,3-dimethyl-1H-pyrazol-4-yl)-5-methyl-1,3,4-oxadiazole

To a solution of N'-acetyl-5-{2,4-dichloro-5-[(4-methoxybenzyl)oxy]phenoxy}-1,3-dimethyl-1H-pyrazole-4-carbohydrazide (1.10 g) obtained in Reference Example 256 in tetrahydrofuran (20 mL) was added (methoxycarbonylsulfamoyl)triethylammonium hydroxide intramolecular salt (3.19 g), and the mixture was heated under reflux for 3 hr. The solvent was evaporated, water was added to the residue, and the mixture was extracted with ethyl acetate. The aqueous layer was extracted with ethyl acetate again, the organic layers were combined and the mixture was washed with water. The organic layer was dried over anhydrous magnesium sulfate, and filtered, and the filtrate was concentrated. The residue was subjected to silica gel column chromatography (hexane-ethyl acetate 100:0 - 40:60, v/v) to give the title compound (1.06 g, yield 100%) as a colorless amorphous solid.
¹H-NMR (300 MHz, CDCl₃)δ:2.35 (s, 3 H), 2.59 (s, 3 H), 3.60 (s, 3 H), 3.81 (s, 3 H), 4.89 (s, 2 H), 6.23 (s, 1 H), 6.74 - 6.83 (m, 2 H), 7.06 - 7.16 (m, 2 H), 7.46 (s, 1 H).

### Reference Example 260 5-(2,4-dichloro-5-hydroxyphenoxy)-1,3-dimethyl-1H-pyrazole-4-carbohydrazide

The title compound was obtained in the same manner as in Reference Example 50 and from 2-(5-{2,4-dichloro-5-[(4-methoxybenzyl)oxy]phenoxy}-1,3-dimethyl-1H-pyrazol-4-yl)-5-methyl-1,3,4-oxadiazole obtained in Reference Example 259.
¹H-NMR (300 MHz, DMSO-d₆)δ:2.36 (s, 3 H), 3.56 (s, 3 H), 6.42 (br s, 1 H), 7.63 (s, 1 H), 10.02 - 10.54 (m, 3 H), 10.69 (br s, 1 H).

### Reference Example 261 tert-butyl (2R)-2-{[(4-methylphenyl)sulfonyl]oxy}propionate

The title compound was obtained in the same manner as in Reference Example 96 and from tert-butyl (R)-(+)-lactate and tosyl chloride.
¹H-NMR (300 MHz, CDCl₃)δ:1.39 (s, 9 H), 1.48 (d, J = 6.6 Hz, 3 H), 2.44 (s, 3 H), 4.81 (q, J = 6.9 Hz, 1 H), 7.34 (d, J = 8.1 Hz, 2 H), 7.76 - 7.87 (m, 2 H).

### Reference Example 262 ethyl (2E)-3-{5-[5-(benzyloxy)-4-bromo-2-methylphenoxy]-3-cyclopropyl-1-methyl-1H-pyrazol-4-yl}acrylate

The title compound was obtained in the same manner as in Reference Example 151 and from 5-[5-(benzyloxy)-4-bromo-2-methylphenoxy]-3-cyclopropyl-1-methyl-1H-pyrazole-4-carbaldehyde obtained in Reference Example 208 and ethyl (diethoxyphosphoryl)acetate.
¹H-NMR (300 MHz, CDCl₃)δ:0.93 - 1.03 (m, 4 H), 1.28 (t, J = 7.2 Hz, 3 H), 1.90 - 2.02 (m, 1 H), 2.31 (s, 3 H), 3.33 (s, 3 H), 4.18 (q, J = 7.2 Hz, 2 H), 4.94 (s, 2 H), 5.99 (s, 1 H), 6.04 (d, J = 16.2 Hz, 1 H), 7.21 - 7.35 (m, 5 H), 7.43 (d, J = 16.2 Hz, 1 H), 7.42 (s, 1 H).

### Reference Example 263 ethyl (2E)-3-[5-(4-bromo-5-hydroxy-2-methylphenoxy)-3-cyclopropyl-1-methyl-1H-pyrazol-4-yl]acrylate

Trifluoroacetic acid (150 mL) was added to ethyl (2E)-3-{5-[5-(benzyloxy)-4-bromo-2-methylphenoxy]-3-cyclopropyl-1-methyl-1H-pyrazol-4-yl}acrylate (18.30 g) obtained in Reference Example 262, and the mixture was stirred at 80°C for 4 hr. After cooling to room temperature, the reaction mixture was concentrated. Aqueous sodium hydrogen carbonate solution was added to the residue, and the mixture was extracted with ethyl acetate. The organic layer was washed with saturated brine, dried over anhydrous magnesium sulfate and filtered. The filtrate was concentrated, and the residue was crystallized from hexane-ethyl acetate to give the title compound as dark-blue crystals (2.56 g, yield 17%).
¹H-NMR (300 MHz, CDCl₃)δ:0.86 - 1.00 (m, 4 H), 1.28 (t, J = 7.1 Hz, 3 H), 1.83 - 1.99 (m, 1 H), 2.34 (s, 3 H), 3.51 (s, 3 H), 4.18 (q, J = 7.2 Hz, 2 H), 5.75 (s, 1 H), 6.11 (d, J = 16.2 Hz, 1 H), 6.15 (s, 1 H), 7.34 (s, 1 H), 7.49 (d, J = 16.2 Hz, 1 H).

### Reference Example 264 2-bromo-5-({3-cyclopropyl-4-[(1E)-3 hydroxyprop-1-en-1-yl]-1-methyl-1H-pyrazol-5-yl}oxy)-4-methylphenol

To a solution of ethyl (2E)-3-[5-(4-bromo-5-hydroxy-2-methylphenoxy)-3-cyclopropyl-1-methyl-1H-pyrazol-4-yl]acrylate (16.5 g) obtained in Reference Example 263 in tetrahydrofuran (150 mL) was added dropwise diisobutylaluminum hydride (1.5M toluene solution, 90 mL) at 0°C over 15 min, and the mixture was stirred for 20 min. Saturated aqueous ammonium chloride solution (25 mL) was added to the reaction mixture at 0°C, and the mixture was stirred at room temperature for 1 hr. The resulting solid was removed by filtration, and the filtrate was concentrated to give the title compound as a pink solid (13.6 g, quant.), which was subjected to silica gel column chromatography (hexane-ethyl acetate 85:15 - 75:25, v/v) and the residue was crystallized from hexane-ethyl acetate to give white crystals.
¹H-NMR (300 MHz, DMSO-d₆)δ:0.74 - 0.81 (m, 2 H), 0.81 - 0.91 (m, 2 H), 1.85 - 1.96 (m, 1 H), 2.25 (s, 3 H), 3.43 (s, 3 H), 3.96 (t, J = 4.5 Hz, 2 H), 4.70 (t, J = 5.3 Hz, 1 H), 5.88 (td, J = 16.0, 5.3 Hz, 1 H), 6.12 (s, 1 H), 6.27 (d, J = 16.3 Hz, 1 H), 7.40 (s, 1 H), 10.09 (br. s., 1 H).

### Reference Example 265 2-bromo-5-{[3-cyclopropyl-4-(3-hydroxypropyl)-1-methyl-1H-pyrazol-5-yl]oxy}-4-methylphenol

To a solution of 2-bromo-5-({3-cyclopropyl-4-[(1E)-3-hydroxyprop-1-en-1-yl]-1-methyl-1H-pyrazol-5-yl}oxy)-4-methylphenol (13.5 g) obtained in Reference Example 264 in tetrahydrofuran (150 mL) and ethanol (150 mL) was added platinum oxide (3.0 g), and the mixture was stirred under a hydrogen atmosphere (1 atm) at room temperature for 5 hr. The catalyst was removed by filtration, and the filtrate was concentrated. The residue was subjected to silica gel column chromatography (hexane-ethyl acetate 9:1 - 1:4, v/v) to give the title compound as a pale-brown solid (8.64 g, 63%). Crystallization from hexane-diisopropyl ether-ethyl acetate gave pale-brown crystals.
¹H-NMR (300 MHz, DMSO-d₆)δ:0.69 - 0.87 (m, 4 H), 1.45 - 1.60 (m, 2 H), 1.68 - 1.86 (m, 1 H), 2.18 - 2.31 (m, 2 H), 2.22 (s, 3 H), 3.24 - 3.37 (m, 2 H), 3.41 (s, 3 H), 4.38 (t, J = 4.9 Hz, 1 H), 6.18 (s, 1 H), 7.37 (s, 1 H), 10.08 (s, 1 H).

### Example 1 (2S)-2-{2,4-dichloro-5-[(1,3,4-trimethyl-1H-pyrazol-5-yl)oxy]phenoxy}propionic acid

A solution of 2,4-dichloro-5-[(1,3,4-trimethyl-1H-pyrazol-5-yl)oxy]phenol (6.00 g) obtained in Reference Example 3, methyl (R)-(+)-lactate (3.26 g), and triphenylphosphine (9.59 g) in tetrahydrofuran (50 mL) was purged with nitrogen, and cooled to 0°C. Diethyl azodicarboxylate (40% toluene solution, 16.7 mL) was added dropwise to the reaction mixture, and the mixture was stirred at 0°C for 15 min, and at room temperature for 15 hr. The reaction mixture was concentrated, and the residue was subjected to silica gel column chromatography (hexane-ethyl acetate 80:20 - 60:40, v/v). The obtained colorless oil was dissolved in a mixed solvent of tetrahydrofuran (70 mL) and water (30 mL), and the solution was cooled to 0°C. Lithium hydroxide monohydrate (1.75 g) was added, and the mixture was stirred at 0°C for 2 hr. To the reaction mixture were added water (300 mL) and ethyl acetate (400 mL), and the mixture was vigorously stirred for 5 min. Two layers were separated, the aqueous layer was neutralized with 1N hydrochloric acid (42 mL), and the mixture was extracted with ethyl acetate. The organic layer was washed with saturated brine, dried over anhydrous magnesium sulfate and filtered. The filtrate was concentrated, and the residue was crystallized from hexane-ethyl acetate to give the title compound as colorless crystals (5.64 g, yield 75%).
¹H-NMR (300 MHz, CDCl₃)δ:1.67 (d, J = 7.0 Hz, 3 H), 1.83 (s, 3 H), 2.17 (s, 3 H), 3.53 (s, 3 H), 4.41 (q, J = 6.8 Hz, 1 H), 6.14 (s, 1 H), 7.46 (s, 1 H).

### Example 2 ethyl 2-12,4-dichloro-5-[(4-formyl-1,3-dimethyl-1H-pyrazol-5-yl)oxy]phenoxy}propionate

The title compound was obtained in the same manner as in Reference Example 2 and from ethyl 2-(2,4-dichloro-5-hydroxyphenoxy)propionate obtained in Reference Example 4 and 5-chloro-1,3-dimethyl-1H-pyrazole-4-carbaldehyde.
¹H-NMR (300 MHz, CDCl₃)δ:1.22 (t, J = 7.2 Hz, 3 H), 1.64 (d, J = 6.8 Hz, 3 H), 2.45 (s, 3 H), 3.66 (s, 3 H), 4.06 - 4.23 (m, 2 H), 4.57 (q, J = 6.7 Hz, 1 H), 6.44 (s, 1 H), 7.50 (s, 1 H), 9.48 (s, 1 H).

### Example 3 2-{2,4-dichloro-5-[(4-formyl-1,3-dimethyl-1H-pyrazol-5-yl)oxy]phenoxy}propionic acid

Ethyl 2-{2,4-dichloro-5-[(4-formyl-1,3-dimethyl-1H-pyrazol-5-yl)oxy]phenoxy}propionate (210 mg) obtained in Example 2 was dissolved in a mixed solvent of tetrahydrofuran (6 mL) and ethanol (4 mL), 1N aqueous sodium hydroxide solution (4 mL) was added, and the mixture was stirred at room temperature for 30 min. The reaction mixture was neutralized with 0.1N hydrochloric acid (42 mL), and extracted with ethyl acetate. The organic layer was washed with saturated brine, dried over anhydrous magnesium sulfate and filtered. The filtrate was concentrated, and the residue was crystallized from hexane-ethyl acetate to give the title compound as colorless crystals (153 mg, yield 79%).
¹H-NMR (300 MHz, CDCl₃)δ:1.68 (d, J = 7.0 Hz, 3 H), 2.43 (s, 3 H), 3.64 (s, 3 H), 4.61 (q, J = 6.8 Hz, 1 H), 6.48 (s, 1 H), 7.50 (s, 1 H), 9.47 (s, 1 H).

### Example 4 ethyl 2-(2,4-dichloro-5-{[4-(hydroxymethyl)-1,3-dimethyl-1H-pyrazol-5-yl]oxy}phenoxy)propionate

Ethyl 2-{2,4-dichloro-5-[(4-formyl-1,3-dimethyl-1H-pyrazol-5-yl)oxy]phenoxy}propionate obtained in Example 2 was dissolved in a mixed solvent of tetrahydrofuran (10 mL) and water (1 mL), sodium borohydride (65 mg) was added, and the mixture was stirred at room temperature for 30 min. Water was added to the reaction mixture, and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 75:25 - 33:67, v/v), and crystallized from hexane-diethyl ether to give the title compound (487 mg, yield 70%) as colorless crystals.
¹H-NMR (300 MHz, CDCl₃)δ:1.20 (t, J = 7.2 Hz, 3 H), 1.62 (d, J = 6.8 Hz, 3 H), 1.66 (t, J = 5.5 Hz, 1 H), 2.29 (s, 3 H), 3.58 (s, 3 H), 4.02 - 4.22 (m, 2 H), 4.29 (d, J = 5.7 Hz, 2 H), 4.59 (q, J = 6.9 Hz, 1 H), 6.48 (s, 1 H), 7.46 (s, 1 H).

### Example 5 2-(2,4-dichloro-5-{[4-(hydroxymethyl)-1,3-dimethyl-1H-pyrazol-5-yl]oxy}phenoxy)propionic acid

The title compound was obtained in the same manner as in Example 3 and from ethyl 2-(2,4-dichloro-5-{[4-(hydroxymethyl)-1,3-dimethyl-1H-pyrazol-5-yl]oxy}phenoxy)propionate obtained in Example 4.
¹H-NMR (300 MHz, CDCl₃)δ:1.66 (d, J = 6.8 Hz, 3 H), 2.24 (s, 3 H), 3.54 (s, 3 H), 4.27 - 4.39 (m, 2 H), 4.55 (q, J = 6.8 Hz, 1 H), 6.46 (s, 1 H), 7.47 (s, 1 H).

### Example 6 ethyl 2-{2,4-dichloro-5-[(1,3-dimethyl-4-vinyl-1H-pyrazol-5-yl)oxy]phenoxy}propionate

To a mixture of methyltriphenylphosphonium bromide (2.20 g) and tetrahydrofuran (20 mL) was added potassium tert-butoxide (671 mg), and the mixture was stirred at room temperature for 2 hr. To the reaction mixture was added ethyl 2-{2,4-dichloro-5-[(4-formyl-1,3-dimethyl-1H-pyrazol-5-yl)oxy]phenoxy}propionate (800 mg) obtained in Example 2, and the mixture was stirred at room temperature for 15 hr. To the reaction mixture was added dil. hydrochloric acid, and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 85:15 - 75:25, v/v) to give the title compound as a colorless oil (340 mg, yield 43%).
¹H-NMR (300 MHz, CDCl₃)δ:1.20 (t, J = 7.2 Hz, 3 H), 1.59 (d, J = 6.8 Hz, 3 H), 2.31 (s, 3 H), 3.56 (s, 3 H), 4.00 - 4.21 (m, 2 H), 4.48 (q, J = 6.8 Hz, 1 H), 5.05 (dd, J = 11.7, 1.3 Hz, 1 H), 5.25 (dd, J = 17. 9, 1. 3 Hz, 1 H), 6.17 (s, 1 H), 6.28 (dd, J = 18. 0, 11.6 Hz, 1 H), 7.47 (s, 1 H).

### Example 7 2-{2,4-dichloro-5-[(1,3-dimethyl-4-vinyl-1H-pyrazol-5-yl)oxy]phenoxy}propionic acid

The title compound was obtained in the same manner as in Example 3 and from ethyl 2-{2,4-dichloro-5-[(1,3-dimethyl-4-vinyl-1H-pyrazol-5-yl)oxy]phenoxy}propionate obtained in Example 6.
¹H-NMR (300 MHz, CDCl₃)δ:1.65 (d, J = 6.8 Hz, 3 H), 2.30 (s, 3 H), 3.54 (s, 3 H), 4.44 (q, J = 6.8 Hz, 1 H), 5.13 (dd, J = 11.6, 1.2 Hz, 1 H), 5.35 (dd, J = 18.0, 1.2 Hz, 1 H), 6.15 (s, 1 H), 6.33 (dd, J = 17.9, 11.5 Hz, 1 H), 7.48 (s, 1 H).

### Example 8 ethyl 2-{2,4-dichloro-5-[(4-ethyl-1,3-dimethyl-1H-pyrazol-5-yl)oxy]phenoxy}propionate

Ethyl 2-{2,4-dichloro-5-[(1,3-dimethyl-4-vinyl-1H-pyrazol-5-yl)oxy]phenoxy}propionate (330 mg) obtained in Example 6 was dissolved in a mixed solvent of tetrahydrofuran (10 mL) and ethanol (10 mL), Lindlar's catalyst (30 mg) was added, and the mixture was stirred under a hydrogen atmosphere (1 atm) at room temperature for 18 hr. The catalyst was removed by filtration, and the filtrate was concentrated. The residue was subjected to silica gel column chromatography (hexane-ethyl acetate 80:20 - 60:40, v/v) to give the title compound as a colorless oil (296 mg, yield 89%).
¹H-NMR (300 MHz, CDCl₃)δ:0.99 (t, J = 7.6 Hz, 3 H), 1.21 (t, J = 7.2 Hz, 3 H), 1.60 (d, J = 6.8 Hz, 3 H), 2.16 - 2.27 (m, 5 H), 3.53 (s, 3 H), 4.00 - 4.22 (m, 2 H), 4.49 (q, J = 6.8 Hz, 1 H), 6.17 (s, 1 H), 7.46 (s, 1 H).

### Example 9 2-{2,4-dichloro-5-[(4-ethyl-1,3-dimethyl-1H-pyrazol-5-yl)oxy]phenoxy}propionic acid

The title compound was obtained in the same manner as in Example 3 and from ethyl 2-12,4-dichloro-5-[(1,3-dimethyl-4-vinyl-1H-pyrazol-5-yl)oxy]phenoxy}propionate obtained in Example 8.
¹H-NMR (300 MHz, CDCl₃)δ:1.05 (t, J = 7.5 Hz, 3 H), 1.67 (d, J = 6.8 Hz, 3 H), 2.20 (s, 3 H), 2.22 - 2.43 (m, 2 H), 3.51 (s, 3 H), 4.39 (q, J = 6.8 Hz, 1 H), 6.15 (s, 1 H), 7.46 (s, 1 H).

### Example 10 ethyl 2-(2,4-dichloro-5-{[1,3-dimethyl-4-(1-propenyl)-1H-pyrazol-5-yl]oxy}phenoxy)propionate

The title compound was obtained in the same manner as in Example 6 and from ethyltriphenylphosphonium bromide and ethyl 2-{2,4-dichloro-5-[(4-formyl-1,3-dimethyl-1H-pyrazol-5-yl)oxy]phenoxy}propionate obtained in Example 2.
LC-MS; m/z 413 [M+H⁺].

### Example 11 ethyl 2-{2,4-dichloro-5-[(1,3-dimethyl-4-propyl-1H-pyrazol-5-yl)oxy]phenoxy}propionate

Ethyl 2-(2,4-dichloro-5-{[1,3-dimethyl-4-(1-propenyl)-1H-pyrazol-5-yl]oxy}phenoxy)propionate (315 mg) obtained in Example 10 was dissolved in a mixed solvent of tetrahydrofuran (10 mL) and ethanol (10 mL), palladium carbon ethylenediamine complex (200 mg) was added, and the mixture was stirred under a hydrogen atmosphere (1 atm) at room temperature for 10 hr. The catalyst was removed by filtration, and the filtrate was concentrated. The residue was subjected to silica gel column chromatography (hexane-ethyl acetate 90:10 - 67:33, v/v) to give the title compound as a colorless oil (294 mg, yield 93%).
¹H-NMR (300 MHz, CDCl₃)δ:0.84 (t, J = 7.3 Hz, 3 H), 1.21 (t, J = 7.2 Hz, 3 H), 1.32 - 1.47 (m, 2 H), 1.60 (d, J = 7.0 Hz, 3 H), 2.11 - 2.18 (m, 2 H), 2.20 (s, 3 H), 3.53 (s, 3 H), 4.01 - 4.22 (m, 2 H), 4.48 (q, J = 6.8 Hz, 1 H), 6.16 (s, 1 H), 7.46 (s, 1 H).

### Example 12 2-{2,4-dichloro-5-[(1,3-dimethyl-4-propyl-1H-pyrazol-5-yl)oxy]phenoxy}propionic acid

The title compound was obtained in the same manner as in Example 3 and from ethyl 2-{2,4-dichloro-5-[(1,3-dimethyl-4-propyl-1H-pyrazol-5-yl)oxy]phenoxy}propionate obtained in Example 11.
¹H-NMR (300 MHz, CDCl₃)δ:0.88 (t, J = 7.4 Hz, 3 H), 1.37 - 1.53 (m, 2 H), 1.66 (d, J = 6.8 Hz, 3 H), 2.13 - 2.34 (m, 5 H), 3.51 (s, 3 H), 4.39 (q, J = 6.7 Hz, 1 H), 6.14 (s, 1 H), 7.46 (s, 1 H).

### Example 13 (2R)-2-{2,4-dichloro-5-[(1,3-dimethyl-4-vinyl-1H-pyrazol-5-yl)oxy]phenoxy}propionic acid

The title compound was obtained in the same manner as in Example 1 and from 2,4-dichloro-5-[(1,3-dimethyl-4-vinyl-1H-pyrazol-5-yl)oxy]phenol obtained in Reference Example 6 and methyl (S)-(-)-lactate.
¹H-NMR (300 MHz, CDCl₃)δ:1.66 (d, J = 6.8 Hz, 3 H), 2.30 (s, 3 H), 3.55 (s, 3 H), 4.43 (q, J = 6.7 Hz, 1 H), 5.13 (dd, J = 11.5, 1.1 Hz, 1 H), 5.35 (dd, J = 17.9, 1.1 Hz, 1 H), 6.12 (s, 1 H), 6.33 (dd, J = 18.0, 11.6 Hz, 1 H), 7.48 (s, 1 H).

### Example 14 (2S)-2-{2,4-dichloro-5-[(1,3-dimethyl-4-vinyl-1H-pyrazol-5-yl)oxy]phenoxy}propionic acid

A solution of 2,4-dichloro-5-[(1,3-dimethyl-4-vinyl-1H-pyrazol-5-yl)oxy]phenol (190 mg) obtained in Reference Example 6, methyl (R)-(+)-lactate (165 mg), and triphenylphosphine (502 mg) in dichloromethane (2 mL) was purged with nitrogen, and cooled to 0°C. Diethyl azodicarboxylate (40% toluene solution, 0.87 mL) was added dropwise to the reaction mixture and the mixture was stirred at 0°C for 15 min and at room temperature for 2 hr. The reaction mixture was concentrated, and the residue was subjected to silica gel column chromatography (hexane-ethyl acetate 85:15 - 67:33, v/v). The obtained colorless oil was dissolved in a mixed solvent of tetrahydrofuran (3.5 mL) and water (1.5 mL), and the solution was cooled to 0°C. Lithium hydroxide monohydrate (53 mg) was added, and the mixture was stirred at 0°C for 2 hr. The mixture was neutralized with dil. hydrochloric acid, and extracted with ethyl acetate. The organic layer was washed with saturated brine, dried over anhydrous magnesium sulfate and filtered. The filtrate was concentrated, and the residue was crystallized from hexane-ethyl acetate to give the title compound as colorless crystals (152 mg, yield 65%).
¹H-NMR (300 MHz, CDCl₃)δ:1.66 (d, J = 6.8 Hz, 3 H), 2.30 (s, 3 H), 3.55 (s, 3 H), 4.43 (q, J = 6.8 Hz, 1 H), 5.13 (dd, J = 11.6, 1.0 Hz, 1 H), 5.35 (dd, J = 17.9, 1.1 Hz, 1 H), 6.12 (s, 1 H), 6.33 (dd, J = 18.0, 11.6 Hz, 1 H), 7.48 (s, 1 H).

### Example 15 2-{2,4-dichloro-5-[(1,3-dimethyl-4-vinyl-1H-pyrazol-5-yl)oxy]phenoxy}-2-methylpropionic acid

A mixture of 2,4-dichloro-5-[(1,3-dimethyl-4-vinyl-1H-pyrazol-5-yl)oxy]phenol (364 mg) obtained in Reference Example 6, tert-butyl 2-bromo-2-methylpropionate (1.63 g), potassium carbonate (1.01 g), anhydrous magnesium sulfate (441 mg), and N,N-dimethylformamide (5 mL) was stirred at 90°C for 18 hr. The reaction mixture was cooled to room temperature, water was added, and the mixture was extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 91:9 - 75:25, v/v) to give a colorless oil. This was dissolved in trifluoroacetic acid (1 mL), and the solution was stirred at room temperature for 2 hr. The reaction mixture was concentrated, and the residue was dissolved in 1N aqueous sodium hydroxide solution (5 mL). Then, aqueous citric acid solution was added and the mixture was extracted with ethyl acetate. The organic layer was washed with saturated brine, and dried over anhydrous magnesium sulfate. After filtration, the filtrate was concentrated and the residue was crystallized from hexane-ethyl acetate to give the title compound (155 mg, yield 33%) as colorless crystals.
¹H-NMR (300 MHz, CDCl₃)δ:1.55 (s, 6 H), 2.27 (s, 3 H), 3.63 (s, 3 H), 5.09 (dd, J = 11.6, 1.2 Hz, 1 H), 5.30 (dd, J = 18.0, 1.2 Hz, 1 H), 6.28 (dd, J = 17.9, 11.7 Hz, 1 H), 6.37 (s, 1 H), 7.48 (s, 1 H).

### Example 16 ethyl {2,4-dichloro-5-[(1,3-dimethyl-4-vinyl-1H-pyrazol-5-yl)oxy]phenoxy}acetate

A mixture of 2,4-dichloro-5-[(1,3-dimethyl-4-vinyl-1H-pyrazol-5-yl)oxy]phenol (300 mg) obtained in Reference Example 6, ethyl bromoacetate (0.144 mL,), potassium carbonate (207 mg), and N,N-dimethylformamide (5 mL) was stirred at room temperature for 15 hr. Water was added to the reaction mixture, and the mixture was extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 85:15 - 67:33, v/v), and crystallized from hexane-ethyl acetate to give the title compound (143 mg, yield 37%) as colorless crystals.
¹H-NMR (300 MHz, CDCl₃)δ:1.25 (t, J = 7.2 Hz, 3 H), 2.32 (s, 3 H), 3.58 (s, 3 H), 4.19 (q, J = 7.2 Hz, 2 H), 4.50 (s, 2 H), 5.06 (dd, J = 11.6, 1.4 Hz, 1 H), 5.25 (dd, J = 18.0, 1.4 Hz, 1 H), 6.17 (s, 1 H), 6.28 (dd, J = 17.9, 11.7 Hz, 1 H), 7.49 (s, 1 H).

### Example 17 {2,4-dichloro-5-[(1,3-dimethyl-4-vinyl-1H-pyrazol-5-yl)oxy]phenoxy}acetic acid

The title compound was obtained in the same manner as in Example 3 and from ethyl {2,4-dichloro-5-[(1,3-dimethyl-4-vinyl-1H-pyrazol-5-yl)oxy]phenoxy}acetate obtained in Example 16.
¹H-NMR (300 MHz, CDCl₃)δ:2.30 (s, 3 H), 3.56 (s, 3 H), 4.56 (s, 2 H), 5.14 (dd, J = 11.6, 1.2 Hz, 1 H), 5.36 (dd, J = 18.0, 1.2 Hz, 1 H), 6.07 (s, 1 H), 6.32 (dd, J = 17.9, 11.5 Hz, 1 H), 7.49 (s, 1 H).

### Example 18 (2S)-2-{2,4-dichloro-5-[(1,3-dimethyl-4-vinyl-1H-pyrazol-5-yl)oxy]phenoxy}propanamide

To a solution of (2S)-2-{2,4-dichloro-5-[(1,3-dimethyl-4-vinyl-1H-pyrazol-5-yl)oxy]phenoxy}propionic acid (96 mg) obtained in Example 14 in N,N-dimethylformamide (2 mL) were added N-ethyl-N'-(3-dimethylaminopropyl)carbodiimide hydrochloride (60 mg) and 1-hydroxybenzotriazole ammonia complex (47 mg), and the mixture was stirred at room temperature for 15 hr. Water was added to the reaction mixture, and the mixture was extracted with ethyl acetate. The organic layer was washed successively with aqueous potassium carbonate solution and saturated brine, and dried over anhydrous magnesium sulfate. After filtration, the filtrate was concentrated and the residue was crystallized from ethyl acetate to give the title compound (66 mg, yield 69%) as colorless crystals.
¹H-NMR (300 MHz, DMSO-d₆)δ:1.38 (d, J = 6.6 Hz, 3 H), 2.21 (s, 3 H), 3.52 (s, 3 H), 4.50 (q, J = 6.7 Hz, 1 H), 4.99 (dd, J = 11.6, 1.6 Hz, 1 H), 5.12 (dd, J = 17.9, 1.7 Hz, 1 H), 6.20 - 6.34 (m, 2 H), 7.25 (s, 1 H), 7.40 (s, 1 H), 7.78 (s, 1 H).

### Example 19 (2S)-2-{2,4-dichloro-5-[(3-ethyl-1-methyl-4-vinyl-1H-pyrazol-5-yl)oxy]phenoxy}propionic acid

The title compound was obtained in the same manner as in Example 1 and from 2,4-dichloro-5-[(3-ethyl-1-methyl-4-vinyl-1H-pyrazol-5-yl)oxy]phenol obtained in Reference Example 10 and methyl (R)-(+)-lactate.
¹H-NMR (300 MHz, CDCl₃)δ:1.22 (t, J = 7.5 Hz, 3 H), 1.65 (d, J = 7.0 Hz, 3 H), 2.60 - 2.72 (m, 2 H), 3.56 (s, 3 H), 4.43 (q, J = 6.8 Hz, 1 H), 5.12 (dd, J = 11.6, 1.2 Hz, 1 H), 5.34 (dd, J = 17.9, 1.1 Hz, 1 H), 6.07 (s, 1 H), 6.34 (dd, J = 18. 0, 11.6 Hz, 1 H), 7.48 (s, 1 H).

### Example 20 (2S)-2-(2,4-dichloro-5-{[1-methyl-3-(trifluoromethyl)-4-vinyl-1H-pyrazol-5-yl] oxy}phenoxy)propionic acid

The title compound was obtained in the same manner as in Example 1 and from 2,4-dichloro-5-{[1-methyl-3-(trifluoromethyl)-4-vinyl-1H-pyrazol-5-yl]oxy}phenol obtained in Reference Example 11 and methyl (R)-(+)-lactate.
¹H-NMR (300 MHz, CDCl₃)δ:1.65 (d, J = 7.0 Hz, 3 H), 3.67 (s, 3 H), 4.54 (q, J = 6.8 Hz, 1 H), 5.22 (d, J = 11.7 Hz, 1 H), 5.44 (d, J = 17.9 Hz, 1 H), 6.17 (s, 1 H), 6.35 - 6.48 (m, 1 H), 7.51 (s, 1 H).

### Example 21 (2S)-2-{2,4-dichloro-5-[(3-methyl-1-phenyl-4-vinyl-1H-pyrazol-5-yl)oxy]phenoxy}propionic acid

The title compound was obtained in the same manner as in Example 1 and from 2,4-dichloro-5-[(3-methyl-1-phenyl-4-vinyl-1H-pyrazol-5-yl)oxy]phenol obtained in Reference Example 12 and methyl (R)-(+)-lactate.
¹H-NMR (300 MHz, CDCl₃)δ:1.54 (d, J = 6.8 Hz, 3 H), 2.40 (s, 3 H), 4.46 (q, J = 6.8 Hz, 1 H), 5.14 (dd, J = 11.7, 1.3 Hz, 1 H), 5.38 (dd, J = 18.0, 1.2 Hz, 1 H), 6.23 (s, 1 H), 6.34 (dd, J = 18.0, 11.6 Hz, 1 H), 7.21 - 7.29 (m, 1 H), 7.31 - 7.40 (m, 3 H), 7.53 - 7.58 (m, 2 H).

### Example 22 ethyl 2-{2,4-dichloro-5-[(1,3-dimethyl-4-vinyl-1H-pyrazol-5-yl)oxy]phenoxy}butanoate

The title compound was obtained in the same manner as in Example 16 and from 2,4-dichloro-5-[(1,3-dimethyl-4-vinyl-1H-pyrazol-5-yl)oxy]phenol obtained in Reference Example 6, and ethyl 2-bromobutanoate.
¹H-NMR (300 MHz, CDCl₃)δ:1.05 (t, J = 7.4 Hz, 3 H), 1.19 (t, J - 7.2 Hz, 3 H), 1.91 - 2.03 (m, 2 H), 2.31 (s, 3 H), 3.56 (s, 3 H), 3.99 - 4.21 (m, 2 H), 4.30 (t, J = 6.0 Hz, 1 H), 5.05 (dd, J = 11.7, 1.3 Hz, 1 H), 5.26 (dd, J = 18.1, 1.3 Hz, 1 H), 6. 12 (s, 1 H), 6.29 (dd, J = 18.0, 11.6 Hz, 1 H), 7.47 (s, 1 H).

### Example 23 2-{2,4-dichloro-5-[(1,3-dimethyl-4-vinyl-1H-pyrazol-5-yl)oxy]phenoxy}butanoic acid

The title compound was obtained in the same manner as in Example 3 and from ethyl 2-{2,4-dichloro-5-[(1,3-dimethyl-4-vinyl-1H-pyrazol-5-yl)oxy]phenoxy}butanoate obtained in Example 22.
¹H-NMR (300 MHz, CDCl₃)δ:1.11 (t, J = 7.3 Hz, 3 H), 1.96 - 2.08 (m, 2 H), 2.30 (s, 3 H), 3.54 (s, 3 H), 4.25 (t, J = 6.1 Hz, 1 H), 5.14 (dd, J = 11.5, 1.3 Hz, 1 H), 5.37 (dd, J = 18.0, 1.2 Hz, 1 H), 6.11 (s, 1 H), 6.33 (dd, J = 18.0, 11.6 Hz, 1 H), 7.48 (s, 1 H).

### Example 24 ethyl 2-{2,4-dichloro-5-[(1,3-dimethyl-4-vinyl-1H-pyrazol-5-yl)oxy]phenoxy}pentanoate

The title compound was obtained in the same manner as in Example 16 and from 2,4-dichloro-5-[(1,3-dimethyl-4-vinyl-1H-pyrazol-5-yl)oxy]phenol obtained in Reference Example 6, and ethyl 2-bromopentanoate.
¹H-NMR (300 MHz, CDCl₃)δ:0.94 (t, J = 7.4 Hz, 3 H), 1.19 (t, J = 7.2 Hz, 3 H), 1.43 - 1.57 (m, 2 H), 1.77 - 2.00 (m, 2 H), 2.31 (s, 3 H), 3.56 (s, 3 H), 3.98 - 4.20 (m, 2 H), 4.34 (dd, J = 7.6, 4.8 Hz, 1 H), 5.05 (dd, J = 11.5, 1.3 Hz, 1 H), 5.25 (dd, J = 18.0, 1.4 Hz, 1 H), 6.12 (s, 1 H), 6.28 (dd, J = 17.9, 11.7 Hz, 1 H), 7.46 (s, 1 H).

### Example 25 2-{2,4-dichloro-5-[(1,3-dimethyl-4-vinyl-1H-pyrazol-5-yl)oxy]phenoxy}pentanoic acid

The title compound was obtained in the same manner as in Example 3 and from ethyl 2-{2,4-dichloro-5-[(1,3-dimethyl-4-vinyl-1H-pyrazol-5-yl)oxy]phenoxy}pentanoate obtained in Example 24.
¹H-NMR (300 MHz, CDCl₃)δ:0.97 (t, J = 7.3 Hz, 3 H), 1.46 - 1.71 (m, 2 H), 1.82 - 2.11 (m, 2 H), 2.30 (s, 3 H), 3.54 (s, 3 H), 4.29 (dd, J = 7.7, 4.7 Hz, 1 H), 5.14 (d, J = 11.5 Hz, 1 H), 5.32 - 5.41 (m, 1 H), 6.11 (s, 1 H), 6.33 (dd, J = 18.0, 11.6 Hz, 1 H), 7.47 (s, 1 H).

### Example 26 ethyl 2-{2,4-dichloro-5-[(1,3-dimethyl-4-vinyl-1H-pyrazol-5-yl)oxy]phenoxy}-3-methylbutanoate

The title compound was obtained in the same manner as in Example 16 and from 2,4-dichloro-5-[(1,3-dimethyl-4-vinyl-1H-pyrazol-5-yl)oxy]phenol obtained in Reference Example 6, and ethyl 2-bromo-3-methylbutanoate.
¹H-NMR (300 MHz, CDCl₃)δ:1.00 - 1.10 (m, 6 H), 1.18 (t, J = 7.2 Hz, 3 H), 2.19 - 2.35 (m, 4 H), 3.55 (s, 3 H), 3.97 - 4.19 (m, 3 H), 5.05 (d, J = 11.7 Hz, 1 H), 5.26 (d, J = 17.8 Hz, 1 H), 6.08 (s, 1 H), 6.21 - 6.35 (m, 1 H), 7.45 (s, 1 H).

### Example 27 2-{2,4-dichloro-5-[(1,3-dimethyl-4-vinyl-1H-pyrazol-5-yl)oxy]phenoxy}-3-methylbutanoic acid

The title compound was obtained in the same manner as in Example 3 and from ethyl 2-{2,4-dichloro-5-[(1,3-dimethyl-4-vinyl-1H-pyrazol-5-yl)oxy]phenoxy}-3-methylbutanoate obtained in Example 26.
¹H-NMR (300 MHz, CDCl₃)δ:1.05 - 1.15 (m, 6 H), 2.24 - 2.37 (m, 4 H), 3.54 (s, 3 H), 4.09 (d, J = 4.7 Hz, 1 H), 5.14 (dd, J = 11.5, 1.3 Hz, 1 H), 5.37 (dd, J = 18.0, 1.2 Hz, 1 H), 6.10 (s, 1 H), 6.34 (dd, J = 18.0, 11.6 Hz, 1 H), 7.47 (s, 1 H).

### Example 28 (2S)-2-{2,4-dichloro-5-[(1-ethyl-3-methyl-4-vinyl-1H-pyrazol-5-yl)oxy]phenoxy}propionic acid

The title compound was obtained in the same manner as in Example 1 and from 2,4-dichloro-5-[(1-ethyl-3-methyl-4-vinyl-1H-pyrazol-5-yl)oxy]phenol obtained in Reference Example 16 and methyl (R)-(+)-lactate.
¹H-NMR (300 MHz, CDCl₃)δ:1.19 (t, J = 7.2 Hz, 3 H), 1.66 (d, J = 6.8 Hz, 3 H), 2.30 (s, 3 H), 3.82 - 4.01 (m, 2 H), 4.43 (q, J = 6.9 Hz, 1 H), 5.12 (dd, J = 11.6, 1.2 Hz, 1 H), 5.29 (dd, J = 18.0, 1.2 Hz, 1 H), 6.22 (s, 1 H), 6.32 (dd, J = 17.9, 11.5 Hz, 1 H), 7.48 (s, 1 H).

### Example 29 (2S)-2-{2,4-dichloro-5-[(1-methyl-3-phenyl-4-vinyl-1H-pyrazol-5-yl)oxy]phenoxy}propionic acid

The title compound was obtained in the same manner as in Example 1 and from 2,4-dichloro-5-[(1-methyl-3-phenyl-4-vinyl-1H-pyrazol-5-yl)oxy]phenol obtained in Reference Example 18 and methyl (R)-(+)-lactate.
¹H-NMR (300 MHz, CDCl₃)δ:1.59 (d, J = 6.8 Hz, 3 H), 3.64 (s, 3 H), 4.47 (q, J = 6.8 Hz, 1 H), 5.08 - 5.13 (m, 1 H), 5.36 (dd, J = 17.8, 1.5 Hz, 1 H), 6.20 (s, 1 H), 6.45 (dd, J = 17.8, 11.4 Hz, 1 H), 7.37 - 7.44 (m, 3 H), 7.50 (s, 1 H), 7.52 - 7.58 (m, 2 H).

### Example 30 (2S)-2-{2,4-dichloro-5-[(2-methyl-2H-indazol-3-yl)oxy]phenoxy}propionic acid

A solution of 2,4-dichloro-5-[(2-methyl-2H-indazol-3-yl)oxy]phenol (95 mg) obtained in Reference Example 27, methyl (R)-(+)-lactate (48 mg), and triphenylphosphine (141 mg) in tetrahydrofuran (2 mL) was purged with nitrogen, and cooled to 0°C. Diethyl azodicarboxylate (40% toluene solution, 0.25 mL) was added dropwise to the reaction mixture, and the mixture was stirred at 0°C for 15 min, and at room temperature for 15 hr. The reaction mixture was concentrated, and the residue was subjected to silica gel column chromatography (hexane-ethyl acetate 82:18 - 60:40, v/v). The obtained colorless oil was dissolved in a mixed solvent of tetrahydrofuran (3.5 mL) and water (1.5 mL), and the solution was cooled to 0°C. Lithium hydroxide monohydrate (39 mg) was added, and the mixture was stirred at 0°C for 1.5 hr. To the reaction mixture were added water (50 mL) and ethyl acetate (50 mL), and the mixture was vigorously stirred for 5 min. Two layers were separated, the aqueous layer was neutralized with dil. hydrochloric acid, and the mixture was extracted with ethyl acetate. The organic layer was washed with saturated brine, dried over anhydrous magnesium sulfate and filtered. The filtrate was concentrated, and the residue was crystallized from hexane-ethyl acetate to give the title compound as colorless crystals (98 mg, yield 84%).
¹H-NMR (300 MHz, DMSO-d₆)δ:1.45 (d, J = 6.8 Hz, 3 H), 4.01 (s, 3 H), 4.90 (q, J = 6.8 Hz, 1 H), 6. 80 - 6.90 (m, 2 H), 7.02 (s, 1 H), 7.14 - 7.23 (m, 1 H), 7.47 (d, J = 8.9 Hz, 1 H), 7.85 (s, 1 H), 13.14 (br s, 1 H).

### Example 31 (2S)-2-{2,4-dichloro-5-[(2-methyl-4,5,6,7-tetrahydro-2H-indazol-3-yl)oxy]phenoxy}propionic acid

A solution of 2,4-dichloro-5-[(2-methyl-4,5,6,7-tetrahydro-2H-indazol-3-yl)oxy]phenol (48 mg) obtained in Reference Example 29, methyl (R)-(+)-lactate (24 mg), and triphenylphosphine (70 mg) in tetrahydrofuran (2 mL) was purged with nitrogen, and cooled to 0°C. Diethyl azodicarboxylate (40% toluene solution, 0.12 mL) was added dropwise to the reaction mixture, and the mixture was stirred at 0°C for 15 min, and at room temperature for 48 hr. The reaction mixture was concentrated, and the residue was subjected to silica gel column chromatography (hexane-ethyl acetate 80:20 - 60:40, v/v). The obtained colorless oil was dissolved in a mixed solvent of tetrahydrofuran (3.5 mL) and water (1.5 mL), and the solution was cooled to 0°C. Lithium hydroxide monohydrate (26 mg) was added, and the mixture was stirred at 0°C for 2 hr. To the reaction mixture were added water (30 mL) and ethyl acetate (40 mL), and the mixture was vigorously stirred for 5 min. Two layers were separated, the aqueous layer was neutralized with dil. hydrochloric acid, and the mixture was extracted with ethyl acetate. The organic layer was washed with saturated brine, dried over anhydrous magnesium sulfate and filtered. The filtrate was concentrated, and the residue was crystallized from hexane-ethyl acetate to give the title compound as colorless crystals (38 mg, yield 64%).
¹H-NMR (300 MHz, CDCl₃)δ:1.65 - 1.88 (m, 7 H), 2.17 - 2.34 (m, 2 H), 2.55 - 2.62 (m, 2 H), 3.56 (s, 3 H), 4.43 (q, J = 6.8 Hz, 1 H), 6.26 (s, 1 H), 7.45 (s, 1 H).

### Example 32 (2S)-2-{2,4-dichloro-5-[(2-methyl-4,5-dihydro-2H-indazol-3-yl)oxy]phenoxy}propionic acid

The title compound was obtained in the same manner as in Example 1 and from 2,4-dichloro-5-[(2-methyl-4,5-dihydro-2H-indazol-3-yl)oxy]phenol obtained in Reference Example 30 and methyl (R)-(+)-lactate.
¹H-NMR (300 MHz, CDCl₃)δ:1.68 (d, J = 6.8 Hz, 3 H), 2.25 - 2.48 (m, 4 H), 3.58 (s, 3 H), 4.48 (q, J = 6.5 Hz, 1 H), 5.99 - 6.09 (m, 1 H), 6.36 (s, 1 H), 6.38 - 6.47 (m, 1 H), 7.47 (s, 1 H).

### Example 33 (2S)-2-{2,4-dichloro-5-[(2-methyl-2,4,5,6,7,8-hexahydrocyclohepta[c]pyrazol-3-yl)oxy]phenoxy}propionic acid

The title compound was obtained in the same manner as in Example 1 and from 2,4-dichloro-5-[(2-methyl-2,4,5,6,7,8-hexahydrocyclohepta[c]pyrazol-3-yl)oxy]phenol obtained in Reference Example 33 and methyl (R)-(+)-lactate.
¹H-NMR (300 MHz, CDCl₃)δ:1.47 - 1.96 (m, 5 H), 2.35 (t, J = 5.7 Hz, 2 H), 2.54 - 2.86 (m, 6 H), 3.50 (s, 3 H), 4.38 (q, J = 6.9 Hz, 1 H), 6.10 (s, 1 H), 7.45 (s, 1 H).

### Example 34 (2R)-2-{2,4-dichloro-5-[(1,3,4-trimethyl-1H-pyrazol-5-yl)oxy]phenoxy}propionic acid

The title compound was obtained in the same manner as in Example 1 and from 2,4-dichloro-5-[(1,3,4-trimethyl-1H-pyrazol-5-yl)oxy]phenol obtained in Reference Example 3 and methyl (S)-(-)-lactate.
¹H-NMR (300 MHz, CDCl₃)δ:1.67 (d, J = 7.0 Hz, 3 H), 1.83 (s, 3 H), 2.17 (s, 3 H), 3.53 (s, 3 H), 4.42 (q, J = 6.8 Hz, 1 H), 6.14 (s, 1 H), 7.46 (s, 1 H).

### Example 35 (2S)-2-{2,4-dichloro-5-[(4-ethyl-1-methyl-1H-pyrazol-5-yl)oxy]phenoxy}propionic acid

The title compound was obtained in the same manner as in Example 1 and from 2,4-dichloro-5-[(4-ethyl-1-methyl-1H-pyrazol-5-yl)oxy]phenol obtained in Reference Example 38 and methyl (R)-(+)-lactate.
¹H-NMR (300 MHz, CDCl₃)δ:1.13 (t, J = 7.6 Hz, 3 H), 1.67 (d, J = 6.8 Hz, 3 H), 2.32 (q, J = 7.7 Hz, 2 H), 3.58 (s, 3 H), 4.40 (q, J = 6.8 Hz, 1 H), 6.13 (s, 1 H), 7.41 (s, 1 H), 7.47 (s, 1 H).

### Example 36 methyl (2S)-2-{2,4-dichloro-5-[(4-ethyl-1,3-dimethyl-1H-pyrazol-5-yl)oxy]phenoxy}propionate

A solution of 2,4-dichloro-5-[(1,3-dimethyl-4-vinyl-1H-pyrazol-5-yl)oxy]phenol (1.00 g) obtained in Reference Example 6, methyl (R)-(+)-lactate (870 mg), and triphenylphosphine (2.63 g) in tetrahydrofuran (10 mL) was purged with nitrogen, and cooled to 0°C. Diethyl azodicarboxylate (40% toluene solution, 4.6 mL) was added dropwise to the reaction mixture, and the mixture was stirred at 0°C for 15 min, and at room temperature for 15 hr. The reaction mixture was concentrated, and the residue was subjected to silica gel column chromatography (hexane-ethyl acetate 85:15 - 75:25, v/v). The obtained colorless oil was dissolved in ethanol (20 mL), Lindlar's catalyst (200 mg) was added, and the mixture was stirred under a hydrogen atmosphere (1 atm) at room temperature for 5 hr. The catalyst was removed by filtration, and the filtrate was concentrated. The residue was subjected to silica gel column chromatography (hexane-ethyl acetate 85:15 - 67:33, v/v) to give the title compound as a colorless oil (510 mg, yield 55%).
¹H-NMR (300 MHz, CDCl₃)δ:1.00 (t, J = 7.5 Hz, 3 H), 1.60 (d, J = 7.0 Hz, 3 H), 2.15 - 2.29 (m, 5 H), 3.53 (s, 3 H), 3.66 (s, 3 H), 4.51 (q, J = 6.8 Hz, 1 H), 6.15 (s, 1 H), 7.46 (s, 1 H).

### Example 37 (2S)-2-{2,4-dichloro-5-[(4-ethyl-1,3-dimethyl-1H-pyrazol-5-yl)oxy]phenoxy}propionic acid

Methyl (2S)-2-{2,4-dichloro-5-[(4-ethyl-1,3-dimethyl-1H-pyrazol-5-yl)oxy]phenoxy}propionate (500 mg) obtained in Example 36 was dissolved in a mixed solvent of tetrahydrofuran (14 mL) and water (6 mL), and the solution was cooled to 0°C. Lithium hydroxide monohydrate (108 mg) was added, and the mixture was stirred at 0°C for 2 hr. The reaction mixture was neutralized with 0.1N hydrochloric acid (26 mL), and extracted with ethyl acetate. The organic layer was washed with saturated brine, dried over anhydrous magnesium sulfate and filtered. The filtrate was concentrated, and the residue was crystallized from hexane-ethyl acetate to give the title compound as colorless crystals (421 mg, yield 87%).
¹H-NMR (300 MHz, CDCl₃)δ:1.06 (t, J = 7.5 Hz, 3 H), 1.67 (d, J = 6.8 Hz, 3 H), 2.20 (s, 3 H), 2.22 - 2.42 (m, 2 H), 3.52 (s, 3 H), 4.39 (q, J = 6.9 Hz, 1 H), 6.12 (s, 1 H), 7.46 (s, 1 H).

### Example 38 methyl (2S)-2-(2,4-dichloro-5-{[3-(methoxymethyl)-1-methyl-4-vinyl-1H-pyrazol-5-yl]oxy}phenoxy)propionate

A solution of 2,4-dichloro-5-{[3-(methoxymethyl)-1-methyl-4-vinyl-1H-pyrazol-5-yl]oxy}phenol (340 mg) obtained in Reference Example 42, methyl (R)-(+)-lactate (0.189 mL), and triphenylphosphine (525 mg) in dichloromethane (12 mL) was purged with nitrogen, and cooled to 0°C. Diethyl azodicarboxylate (40% toluene solution, 0.91 mL) was added dropwise to the reaction mixture, and the mixture was stirred at room temperature for 1.5 hr. Water was added to the reaction mixture, and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 20:1 - 2:1, v/v) to give the title compound as a colorless oil (0.59 g, since impurity was contained somewhat, the oil was directly used for the next reaction).
¹H-NMR (300 MHz, CDCl₃)δ:1.59 (d, J = 6.8 Hz, 3 H), 3.41 (s, 3 H), 3.62 (s, 3 H), 3.62 (s, 3 H), 4.39 - 4.56 (m, 3 H), 5.11 (dd, J = 11.6, 1.3 Hz, 1H), 5.36 (dd, J = 17.8, 1.5 Hz, 1 H), 6.18 (s, 1 H), 6.37 (dd, J = 18.0, 11.6 Hz, 1 H), 7.48 (s, 1 H).

### Example 39 (2S)-2-(2,4-dichloro-5-{[3-(methoxymethyl)-1-methyl-4-vinyl-1H-pyrazol-5-yl]oxy}phenoxy)propionic acid

Methyl (2S)-2-(2,4-dichloro-5-{[3-(methoxymethyl)-1-methyl-4-vinyl-1H-pyrazol-5-yl]oxy}phenoxy)propionate (0.59 g) obtained in Example 38 was dissolved in a mixed solvent of tetrahydrofuran (9 mL) and water (3 mL), lithium hydroxide monohydrate (84 mg) was added, and the mixture was stirred at room temperature for 1.5 hr. The reaction mixture was neutralized with 1N hydrochloric acid (2 mL), and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 10:1 - 1:1, v/v) and recrystallized from hexane-ethyl acetate to give the title compound as colorless crystals [263 mg, yield 64% (yield from Example 38)].
¹H-NMR (300 MHz, CDCl₃)δ:1.62 (d, J = 6.8 Hz, 3 H), 3.45 (s, 3 H), 3.65 (s, 3 H), 4.41 (q, J = 6.9 Hz, 1 H), 4.43 - 4.54 (m, 2 H), 5.12 (dd, J = 11.6, 1.2 Hz, 1H), 5.34 (dd, J = 17.9, 1.3 Hz, 1 H), 5.94 (br.s, 1 H), 6.11 (s, 1 H), 6.35 (dd, J = 18.0, 11.6 Hz, 1 H), 7.46 (s, 1 H).

### Example 40 methyl (2S)-2-{2,4-dichloro-5-[(1-methyl-3-propyl-4-vinyl-1H-pyrazol-5-yl)oxy]phenoxy}propionate

To a solution of 2,4-dichloro-5-[(1-methyl-3-propyl-4-vinyl-1H-pyrazol-5-yl)oxy]phenol (0.87 g) obtained in Reference Example 46, methyl (R)-(+)-lactate (0.631 mL), and triphenylphosphine (1.74 g) in methylene chloride (15 mL) was added dropwise diethyl azodicarboxylate (40% toluene solution, 3.0 mL) under ice-cooling, and the mixture was stirred at 0°C for 2 hr. The reaction mixture was diluted with hexane (15 mL), and subjected to silica gel column chromatography (hexane-ethyl acetate 19:1 - 3:2, v/v). The obtained oil was subjected to silica gel column chromatography (hexane-ethyl acetate 9:1 - 3:7, v/v) to give the title compound (424 mg, yield 39%).
¹H-NMR (300 MHz, CDCl₃)δ:1.05 (t, J = 7.3 Hz, 3 H), 1.64 (d, J = 6.8 Hz, 3 H), 1.70 - 1.84 (m, 2 H), 2.68 (t, J = 7.7 Hz, 2 H), 3.62 (s, 3 H), 3.70 (s, 3 H), 4.53 (q, J = 6.8 Hz, 1 H), 5.09 (dd, J = 11.5, 1.3 Hz, 1 H), 5.28 (dd, J = 18.1, 1.3 Hz, 1 H), 6.18 (s, 1 H), 6.37 (dd, J = 18.0, 11.6 Hz, 1 H), 7.52 (s, 1 H).

### Example 41 (2S)-2-{2,4-dichloro-5-[(1-methyl-3-propyl-4-vinyl-1H-pyrazol-5-yl)oxy]phenoxy}propionic acid

To a solution of methyl (2S)-2-{2,4-dichloro-5-[(1-methyl-3-propyl-4-vinyl-1H-pyrazol-5-yl)oxy]phenoxy}propionate (424 mg) obtained in Example 40 in tetrahydrofuran (7.4 mL) and water (3.1 mL) was added lithium hydroxide monohydrate (108 mg) under ice-cooling, and the mixture was stirred at 0°C for 2 hr. 1N Hydrochloric acid (2.56 mL) was added to the reaction mixture, and the mixture was extracted with ethyl acetate. The organic layer was washed with water and saturated brine, dried over anhydrous magnesium sulfate and filtered, and the filtrate was concentrated. The obtained residue was subjected to reversed-phase high performance liquid chromatography (0.1% trifluoroacetic acid acetonitrile solution -0.1% aqueous trifluoroacetic acid solution 40:60 - 100:0, v/v), and the eluate was concentrated. The obtained oil was dissolved in ethyl acetate, the organic layer was washed with saturated brine, dried over anhydrous magnesium sulfate and filtered, and the filtrate was concentrated. The residue was crystallized from hexane-ethyl acetate to give the title compound as white crystals (140 mg, yield 34%). ¹H-NMR (300 MHz, CDCl₃)δ:0.95 (t, J = 7.3 Hz, 3 H), 1.53 - 1.71 (m, 5 H), 2.42 - 2.75 (m, 2 H), 3.57 (s, 3 H), 4.42 (q, J = 6.8 Hz, 1 H), 5.11 (dd, J = 11.5, 1.3 Hz, 1 H), 5.33 (dd, J = 17.9, 1.3 Hz, 1 H), 6.05 (s, 1 H), 6.34 (dd, J = 18.0, 11.6 Hz, 1 H), 7.48 (s, 1 H).

### Example 42 methyl (2S)-2-{2,4-dichloro-5-[(3-isopropyl-1-methyl-4-vinyl-1H-pyrazol-5-yl)oxy]phenoxy}propionate

The title compound was obtained in the same manner as in Example 40 and from 2,4-dichloro-5-[(3-isopropyl-1-methyl-4-vinyl-1H-pyrazol-5-yl)oxy]phenol obtained in Reference Example 51 and methyl (R)-(+)-lactate.
¹H-NMR (300 MHz, CDCl₃)δ:1.32 (d, J = 7.0 Hz, 6 H), 1.59 (d, J = 6.8 Hz, 3 H), 2.97 - 3.15 (m, 1 H), 3.57 (s, 3 H), 3.65 (s, 3 H), 4.47 (q, J = 6.8 Hz, 1 H), 5.04 (dd, J = 11.6, 1.4 Hz, 1 H), 5. 23 (dd, J = 17. 9, 1. 5 Hz, 1 H), 6.11 (s, 1 H), 6.37 (dd, J = 17.9, 11.7 Hz, 1 H), 7.47 (s, 1 H).

### Example 43 (2S)-2-{2,4-dichloro-5-[(3-isopropyl-1-methyl-4-vinyl-1H-pyrazol-5-yl)oxy]phenoxy}propionic acid

To a solution of methyl (2S)-2-{2,4-dichloro-5-[(3-isopropyl-1-methyl-4-vinyl-1H-pyrazol-5-yl)oxy]phenoxy}propionate (0.96 g) obtained in Example 42 in tetrahydrofuran (16 mL) and water (7 mL) was added lithium hydroxide monohydrate (244 mg) under ice-cooling, and the mixture was stirred at 0°C for 30 min. 1N Hydrochloric acid (6 mL) was added to the reaction mixture, and the mixture was extracted with ethyl acetate. The organic layer was washed with saturated brine, dried over anhydrous magnesium sulfate and filtered, and the filtrate was concentrated. The residue was crystallized from hexane-ethyl acetate to give the title compound as white crystals (704 mg, yield 76%).
¹H-NMR (300 MHz, CDCl₃)δ:1.28 (dd, J = 7.1, 3.1 Hz, 6 H), 1.64 (d, J = 7.0 Hz, 3 H), 2.96 - 3.15 (m, 1 H), 3.53 (s, 3 H), 4.44 (q, J = 6.8 Hz, 1 H), 5.08 (dd, J = 11.6, 1.4 Hz, 1 H), 5.31 (dd, J = 17.9, 1.3 Hz, 1 H), 6.07 (s, 1 H), 6.38 (dd, J = 17.9, 11.5 Hz, 1 H), 7. 47 (s, 1 H).

### Example 44 (2S)-2-{2,4-dichloro-5-[(4-ethyl-1-methyl-3-propyl-1H-pyrazol-5-yl)oxy]phenoxy}propionic acid

To a solution of (2S)-2-{2,4-dichloro-5-[(1-methyl-3-propyl-4-vinyl-1H-pyrazol-5-yl)oxy]phenoxy}propionic acid (95 mg) obtained in Example 41 in tetrahydrofuran (2.5 mL) and methanol (2.5 mL) was added platinum oxide (11 mg), and the mixture was stirred under a hydrogen atmosphere (1 atm) at room temperature for 3 hr. The catalyst was removed by filtration, and the filtrate was concentrated. The residue was crystallized from hexane-ethyl acetate to give the title compound as white crystals (47 mg, yield 49%). ¹H-NMR (300 MHz, CDCl₃)δ:0.94 (t, J = 7.3 Hz, 3 H), 1.04 (t, J = 7.5 Hz, 3 H), 1.55 - 1.70 (m, 5 H), 2.19 - 2.40 (m, 2 H), 2.46 - 2.57 (m, 2 H), 3.53 (s, 3 H), 4.38 (q, J = 6.7 Hz, 1 H), 6.04 (s, 1 H), 7.46 (s, 1 H).

### Example 45 (2S)-2-{2,4-dichloro-5-[(4-ethyl-3-isopropyl-1-methyl-1H-pyrazol-5-yl)oxy]phenoxy}propionic acid

The title compound was obtained in the same manner as in Example 44 and from (2S)-2-{2,4-dichloro-5-[(3-isopropyl-1-methyl-4-vinyl-1H-pyrazol-5-yl)oxy]phenoxy}propionic acid obtained in Example 43.
¹H-NMR (300 MHz, CDCl₃)δ:1.04 (t, J = 7.6 Hz, 3 H), 1.26 (dd, J = 7.0, 4.4 Hz, 6 H), 1. 65 (d, J = 6.8 Hz, 3 H), 2.14 - 2.48 (m, 2 H), 2.83 - 3.09 (m, 1 H), 3.51 (s, 3 H), 4.41 (q, J = 6.8 Hz, 1 H), 6.06 (s, 1 H), 7.46 (s, 1 H).

### Example 46 methyl {2,4-dichloro-5-[(1,3-dimethyl-4-vinyl-1H-pyrazol-5-yl)oxy]phenoxy}acetate

The title compound was obtained in the same manner as in Example 16 and from 2,4-dichloro-5-[(1,3-dimethyl-4-vinyl-1H-pyrazol-5-yl)oxy]phenol obtained in Reference Example 6 and methyl bromoacetate.
¹H-NMR (300 MHz, CDCl₃)δ:2.32 (s, 3 H), 3.58 (s, 3 H), 3.73 (s, 3 H), 4.51 (s, 2 H), 5.06 (dd, J = 11.6, 1.4 Hz, 1 H), 5.25 (dd, J = 17.9, 1.4 Hz, 1 H), 6.17 (s, 1 H), 6.29 (dd, J = 17.9, 11.6 Hz, 1 H), 7.49 (s, 1 H).

### Example 47 3-{2,4-dichloro-5-[(1,3-dimethyl-4-vinyl-1H-pyrazol-5-yl)oxy]phenoxy}butanoic acid

To a solution of 2,4-dichloro-5-[(1,3-dimethyl-4-vinyl-1H-pyrazol-5-yl)oxy]phenol (300 mg) obtained in Reference Example 6 and 4-methyloxetan-2-one (0.196 mL) in a mixed solvent of tetrahydrofuran (4 mL) and N,N-dimethylformamide (2 mL) was added potassium tert-butoxide (246 mg), and the mixture was stirred at 60°C for 15 hr. 1N Hydrochloric acid (2.2 mL) was added to the reaction mixture, and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 2:1 - 1:1, v/v) to give the title compound as a colorless oil (81 mg, yield 21%).
¹H-NMR (300 MHz, CDCl₃)δ:1.33 (d, J = 6.0 Hz, 3 H), 2.29 (s, 3 H), 2.52 (dd, J = 16.0, 5.8 Hz, 1 H), 2.74 (dd, J = 16.0, 6.2 Hz, 1 H), 3.61 (s, 3 H), 4.48 - 4.51 (m, 1 H), 5.06 (dd, J = 11.6, 1.3 Hz, 1 H), 5.25 (dd, J = 17.9, 1.3 Hz, 1 H), 6.28 (dd, J = 17.9, 11.6 Hz, 1 H), 6.40 (s, 1 H), 7.45 (s, 1 H).

### Example 48 methyl (2E)-3-{2,4-dichloro-5-[(4-formyl-1,3-dimethyl-1H-pyrazol-5-yl)oxy]phenyl}acrylate

To a solution of 2,4-dichloro-5-[(4-formyl-1,3-dimethyl-1H-pyrazol-5-yl)oxy]phenyl trifluoromethanesulfonate (866 mg) obtained in Reference Example 53 in N,N-dimethylformamide (4 mL) were added methyl acrylate (0.215 mL), dichlorobistriphenylphosphine palladium (140 mg) and triethylamine (0.335 mL), and the mixture was stirred under an argon stream at 80°C for 15 hr, and at 90°C for 3 hr. To the reaction solution was added water, and the mixture was extracted with ethyl acetate. The organic layer was washed with water and saturated brine, dried over anhydrous magnesium sulfate and filtered, and the filtrate was concentrated. The resulting crystals were recrystallized from diethyl ether to give the title compound as colorless crystals (256 mg, yield 35%).
¹H-NMR (300 MHz, CDCl₃)δ:2.48 (s, 3 H), 3.71 (s, 3 H), 3,81 (s, 3 H), 6.23 (d, J = 16.0 Hz, 1 H), 7.07 (s, 1 H), 7.57 (s, 1 H), 7.90 (d, J = 16.0 Hz, 1 H), 9.55 (s, 1 H).

### Example 49 (2E)-3-{2,4-dichloro-5-[(4-formyl-1,3-dimethyl-1H-pyrazol-5-yl)oxy]phenyl}acrylic acid

To a solution of methyl (2E)-3-{2,4-dichloro-5-[(4-formyl-1,3-dimethyl-1H-pyrazol-5-yl)oxy]phenyl}acrylate (150 mg) obtained in Example 48 in a mixed solvent of tetrahydrofuran (5 mL) and methanol (1 mL) was added 2N aqueous sodium hydroxide solution (1 mL), and the mixture was stirred at room temperature for 1 hr. 2N Hydrochloric acid (1 mL) was added to the reaction mixture, and the mixture was extracted with ethyl acetate. The organic layer was washed with saturated brine, dried over anhydrous magnesium sulfate and filtered, and the filtrate was concentrated. The resulting crystals were recrystallized from hexane-ethyl acetate to give the title compound as colorless crystals (94 mg, yield 65%).
¹H-NMR (300 MHz, DMSO-d₆)δ:2.36 (s, 3 H), 3.67 (s, 3 H), 6.62 (d, J = 15.8 Hz, 1 H), 7.65 (s, 1 H), 7.69 (d, J = 15.8 Hz, 1 H), 7.94 (s, 1 H), 9.43 (s, 1 H).

### Example 50 ethyl (2E)-3-{4-chloro-3-[(4-formyl-1,3-dimethyl-1H-pyrazol-5-yl)oxy]phenyl}acrylate

To a solution of 5-(5-bromo-2-chlorophenoxy)-1,3-dimethyl-1H-pyrazole-4-carbaldehyde (1.98 g) obtained in Reference Example 54 in N-methylpyrrolidone (15 mL) were added ethyl acrylate (0.8 mL), palladium acetate (202 mg), triphenylphosphine (472 mg), sodium acetate (984 mg) and benzyltriethylammonium chloride (1.37 g), and the mixture was stirred under an argon stream at 100°C for 1 hr, and at 120°C for 2 hr. The reaction mixture was filtered, water was added to the filtrate and the mixture was extracted with ethyl acetate. The organic layer was washed with water and saturated brine, dried over anhydrous magnesium sulfate and filtered. The filtrate was concentrated, and the residue was subjected to silica gel column chromatography (hexane-ethyl acetate 9:1 - 4:1, v/v) to give the title compound as a yellow oil (1.56 g, yield 75%).
¹H-NMR (300 MHz, CDCl₃)δ:1.32 (t, J = 7.2 Hz, 3 H), 2.48 (s, 3 H), 3.70 (s, 3 H), 4.25 (q, J = 7.2 Hz, 2 H), 6.32 (d, J = 16.0 Hz, 1 H), 6.98 (d, J = 1.9 Hz, 1 H), 7.30 (dd, J = 8.3, 1.9 Hz, 1 H), 7.46 - 7.56 (m, 2 H), 9.51 (s, 1 H).

### Example 51 (2E)-3-{4-chloro-3-[(4-formyl-1,3-dimethyl-1H-pyrazol-5-yl)oxy]phenyl}acrylic acid

The title compound was obtained in the same manner as in Example 49 and from ethyl (2E)-3-{4-chloro-3-[(4-formyl-1,3-dimethyl-1H-pyrazol-5-yl)oxy]phenyl}acrylate obtained in Example 50.
¹H-NMR (300 MHz, DMSO-d₆)δ:2.36 (s, 3 H), 3.65 (s, 3 H), 6.56 (d, J = 16.0 Hz, 1 H), 7.41 - 7.73 (m, 4 H), 9.42 (s, 1 H).

### Example 52 ethyl 3-{4-chloro-3-[(4-formyl-1,3-dimethyl-1H-pyrazol-5-yl)oxy]phenyl}propionate

To a solution of ethyl (2E)-3-{4-chloro-3-[(4-formyl-1,3-dimethyl-1H-pyrazol-5-yl)oxy]phenyl}acrylate (862 mg) obtained in Example 50 in a mixed solvent of tetrahydrofuran (10 mL) and ethanol (10 mL) was added platinum oxide (112 mg), and the mixture was stirred under a hydrogen stream for 10 hr. The reaction mixture was filtered, and the filtrate was concentrated. The residue was subjected to silica gel column chromatography (hexane-ethyl acetate 9:1 - 2:1, v/v) to give the title compound as a colorless oil (440 mg, yield 51%).
¹H-NMR (300 MHz, CDCl₃)δ:1.22 (t, J = 7.1 Hz, 3 H), 2.46 (s, 3 H), 2.54 (t, J = 7.5 Hz, 2 H), 2.87 (t, J = 7.5 Hz, 2 H), 3.68 (s, 3 H), 4.09 (q, J = 7.1 Hz, 2 H), 6.77 (d, J = 1.9 Hz, 1 H), 6.99 (dd, J = 8.2, 1.9 Hz, 1 H), 7.39 (d, J = 8.2 Hz, 1 H), 9.42 (s, 1 H).

### Example 53 3-{4-chloro-3-[(4-formyl-1,3-dimethyl-1H-pyrazol-5-yl)oxy]phenyl}propionic acid

The title compound was obtained in the same manner as in Example 49 and from ethyl 3-{4-chloro-3-[(4-formyl-1,3-dimethyl-1H-pyrazol-5-yl)oxy]phenyl}propionate obtained in Example 52.
¹H-NMR (300 MHz, DMSO-d₆)δ:2.34 (s, 3 H), 2.41 - 2.55 (m, 2 H), 2.76 (t, J = 7.5 Hz, 2 H), 3.62 (s, 3 H), 7.00 (d, J = 1.9 Hz, 1 H), 7.12 (dd, J = 8.1, 1.9 Hz, 1 H), 7.52 (d, J = 8.1 Hz, 1 H), 9.35 (s, 1 H), 12.14 (br s, 1 H).

### Example 54 2-{2,4-dichloro-5-[(1,3-dimethyl-4-vinyl-1H-pyrazol-5-yl)oxy]phenoxy}-N-(phenylsulfonyl)acetamide

To a solution of {2,4-dichloro-5-[(1,3-dimethyl-4-vinyl-1H-pyrazol-5-yl)oxy]phenoxy}acetic acid (179 mg) obtained in Example 17 in acetonitrile (3 mL) were added benzenesulfonamide (86 mg), 1-ethyl-3-(3-dimethylaminopropyl)carbodiimide hydrochloride (144 mg) and N,N-dimethylaminopyridine (92 mg), and the mixture was stirred at room temperature for 15 hr. Water was added to the reaction mixture, and the mixture was extracted with ethyl acetate. The organic layer was washed with saturated brine, dried over anhydrous magnesium sulfate and filtered. The filtrate was concentrated, and the residue was recrystallized from hexane-diethyl ether to give the title compound (138 mg, yield 56%).
LC-MS; m/z 496 [M+H⁺].

### Example 55 2-{2,4-dichloro-5-[(1,3-dimethyl-4-vinyl-1H-pyrazol-5-yl)oxy]phenoxy}-N-(methylsulfonyl)acetamide

The title compound was obtained in the same manner as in Example 54 and from {2,4-dichloro-5-[(1,3-dimethyl-4-vinyl-1H-pyrazol-5-yl)oxy]phenoxy}acetic acid obtained in Example 17, and methanesulfonamide.
LC-MS; m/z 434 [M+H⁺].

### Example 56 ethyl {3-chloro-4-[(4-formyl-1,3-dimethyl-1H-pyrazol-5-yl)oxy]phenyl}acetate

The title compound was obtained in the same manner as in Reference Example 54 and from ethyl (3-chloro-4-hydroxyphenyl)acetate and 5-chloro-1,3-dimethyl-1H-pyrazole-4-carbaldehyde.
¹H-NMR (300 MHz, CDCl₃) δ: 1.27 (t, J = 7.2 Hz, 3 H), 2.45 (s, 3 H), 3.58 (s, 2 H), 3.68 (s, 3 H), 4.17 (q, J = 7.2 Hz, 2 H), 6.86 (d, J = 8.4 Hz, 1 H), 7.14 (dd, J = 8.4, 2.0 Hz, 1 H), 7.43 (d, J = 2.0 Hz, 1 H), 9.46 (s, 1 H).

### Example 57 ethyl (2E)-3-{3-chloro-4-[(4-formyl-1,3-dimethyl-1H-pyrazol-5-yl)oxy]phenyl}acrylate

The title compound was obtained in the same manner as in Example 48 and from 5-(4-bromo-2-chlorophenoxy)-1,3-dimethyl-1H-pyrazole-4-carbaldehyde obtained in Reference Example 55 and ethyl acrylate.
¹H-NMR (300 MHz, CDCl₃)δ:1.34 (t, J = 7.2 Hz, 3 H), 2.47 (s, 3 H), 3.69 (s, 3 H), 4.27 (q, J = 7.2 Hz, 2 H), 6.38 (d, J = 16.0 Hz, 1 H), 6.88 (d, J = 8.7 Hz, 1 H), 7.36 (dd, J = 8.7, 2.1 Hz, 1 H), 7.57 (d, J = 16.0 Hz, 1 H), 7.66 (d, J = 2.1 Hz, 1 H), 9.54 (s, 1 H).

### Example 58 {3-chloro-4-[(4-formyl-1,3-dimethyl-1H-pyrazol-5-yl)oxy]phenyl}acetic acid

The title compound was obtained in the same manner as in Example 49 and from ethyl {3-chloro-4-[(4-formyl-1,3-dimethyl-1H-pyrazol-5-yl)oxy]phenyl}acetate obtained in Example 56.
¹H-NMR (300 MHz, CDCl₃)δ:2.45 (s, 3 H), 3.63 (s, 2 H), 3.68 (s, 3 H), 6.87 (d, J = 8.5 Hz, 1 H), 7.15 (dd, J = 8.5, 2.1 Hz, 1 H), 7.44 (d, J = 2.1 Hz, 1 H), 9.46 (s, 1 H).

### Example 59 (2E)-3-{3-chloro-4-[(4-formyl-1,3-dimethyl-1H-pyrazol-5-yl)oxy]phenyl}acrylic acid

The title compound was obtained in the same manner as in Example 49 and from ethyl (2E)-3-{3-chloro-4-[(4-formyl-1,3-dimethyl-1H-pyrazol-5-yl)oxy]phenyl}acrylate obtained in Example 57.
¹H-NMR (300 MHz, DMSO-d₆)δ:2.36 (s, 3 H), 3.64 (s, 3 H), 6.57 (d, J = 16.0 Hz, 1 H), 7.03 (d, J = 8.7 Hz, 1 H), 7.56 (d, J = 16.0 Hz, 1 H), 7.64 (dd, J = 8.7, 1.9 Hz, 1 H), 8.03 (d, J = 1.9 Hz, 1 H), 9.47 (s, 1 H).

### Example 60 methyl (3-chloro-4-{[4-(hydroxymethyl)-1,3-dimethyl-1H-pyrazol-5-yl]oxy}phenyl)acetate

To a solution of ethyl 13-chloro-4-[(4-formyl-1,3-dimethyl-1H-pyrazol-5-yl)oxy]phenyl}acetate obtained in Example 56 in a mixed solvent of tetrahydrofuran (20 mL) and methanol (2 mL) was added sodium borohydride (153 mg), and the mixture was stirred at room temperature for 3 hr. Water was added to the reaction mixture, and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 3:1 - 1:2, v/v) to give the title compound as a colorless oil (887 mg, yield 68%).
¹H-NMR (300 MHz, CDCl₃)δ:1.42 - 1.52 (m, 1 H), 2.28 (s, 3 H), 3.57 (s, 2 H), 3.59 (s, 3 H), 3.71 (s, 3 H), 4.30 (d, J = 4.0 Hz, 2 H), 6.74 (d, J = 8.4 Hz, 1 H), 7.08 (dd, J = 8.4, 2.1 Hz, 1 H), 7.39 (d, J = 2.1 Hz, 1 H).

### Example 61 methyl {3-chloro-4-[(1,3,4-trimethyl-1H-pyrazol-5-yl)oxy]phenyl}acetate

To a solution of methyl (3-chloro-4-{[4-(hydroxymethyl)-1,3-dimethyl-1H-pyrazol-5-yl]oxy}phenyl)acetate (887 mg) obtained in Example 60 in trifluoroacetic acid (10 mL) was added triethylsilane (1.8 mL), and the mixture was stirred at room temperature for 3 hr. The reaction mixture was concentrated, and the residue was subjected to silica gel column chromatography (hexane-ethyl acetate 5:1 - 2:1, v/v) to give the title compound as a colorless oil (716 mg, yield 85%).
¹H-NMR (300 MHz, CDCl₃)δ:1.74 (s, 3 H), 2.19 (s, 3 H), 3.57 (s, 2 H), 3.59 (s, 3 H), 3.71 (s, 3 H), 6.63 (d, J = 8.5 Hz, 1 H), 7.06 (dd, J = 8.5, 2.3 Hz, 1 H), 7.39 (d, J = 2.3 Hz, 1 H).

### Example 62 {3-chloro-4-[(1,3,4-trimethyl-1H-pyrazol-5-yl)oxy]phenyl}acetic acid

The title compound was obtained in the same manner as in Example 49 and from methyl {3-chloro-4-[(1,3,4-trimethyl-1H-pyrazol-5-yl)oxylphenyllacetate obtained in Example 61.
¹H-NMR (300 MHz, CDCl₃)δ:1.73 (s, 3 H), 2.18 (s, 3 H), 3.57 - 3.62 (m, 5 H), 6.63 (d, J = 8.5 Hz, 1 H), 7.08 (dd, J = 8.5, 2.3 Hz, 1 H), 7.41 (d, J = 2.3 Hz, 1 H).

### Example 63 2-{3-chloro-4-[(1,3,4-trimethyl-1H-pyrazol-5-yl)oxy]phenyl}-N-(pentylsulfonyl)acetamide

To a solution of {3-chloro-4-[(1,3,4-trimethyl-1H-pyrazol-5-yl)oxylphenyllacetic acid (294 mg) obtained in Example 62 in acetonitrile (5 mL) were added pentanesulfonamide (159 mg), 2-methyl-6-nitrobenzoic anhydride (413 mg), N,N-dimethylaminopyridine (122 mg) and triethylamine (0.418 mL), and the mixture was stirred at room temperature for 3 hr. After evaporation of the solvent, saturated aqueous ammonium chloride solution was added to the residue and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 2:1 - 1:1, v/v), and the obtained oil was recrystallized from hexane-ethyl acetate to give the title compound as colorless crystals (131 mg, yield 31%).
¹H-NMR (300 MHz, CDCl₃)δ:0.83 - 0.95 (m, 3 H), 1.24 - 1.46 (m, 4 H), 1.69 - 1.85 (m, 5 H), 2.19 (s, 3 H), 3.38 - 3.48 (m, 2 H), 3.58 (s, 3 H), 3.62 (s, 2 H), 6.66 (d, J = 8.5 Hz, 1 H), 7.04 (dd, J = 8.5, 2.3 Hz, 1 H), 7.38 (d, J = 2.3 Hz, 1 H), 8.60 (br s, 1 H).

### Example 64 2-{2,4-dichloro-5-[(1,3-dimethyl-4-vinyl-1H-pyrazol-5-yl)oxy]phenoxy}-N-(methylsulfonyl)propanamide

The title compound was obtained in the same manner as in Example 63 and from (2S)-2-{2,4-dichloro-5-[(1,3-dimethyl-4-vinyl-1H-pyrazol-5-yl)oxy]phenoxy}propionic acid obtained in Example 14 and methanesulfonamide.
LC-MS; m/z 448 [M+H⁺].

### Example 65 N-(cyclopropylmethyl)-2-{2,4-dichloro-5-[(1,3-dimethyl-4-vinyl-1H-pyrazol-5-yl)oxy]phenoxy}acetamide

To a solution of {2,4-dichloro-5-[(1,3-dimethyl-4-vinyl-1H-pyrazol-5-yl)oxy]phenoxy}acetic acid (214 mg) obtained in Example 17 and cyclopropylmethylamine (0.062 mL) in N,N-dimethylformamide (3 mL) were added 1-ethyl-3-(3-dimethylaminopropyl)carbodiimide hydrochloride (138 mg) and 1-hydroxybenzotriazole (110 mg), and the mixture was stirred at room temperature for 15 hr. Water was added to the reaction mixture and the mixture was extracted with ethyl acetate. The organic layer was washed with water and saturated brine, dried over anhydrous magnesium sulfate and filtered. The filtrate was concentrated, and the residue was subjected to silica gel column chromatography (hexane-ethyl acetate 9:1 - 4:1, v/v) to give the title compound as a colorless oil (146 mg, yield 59%). Recrystallization from hexane-diethyl ether gave colorless crystals.
¹H-NMR (300 MHz, CDCl₃)δ:0-19 - 0.29 (m, 2 H), 0.49 - 0.59 (m, 2 H), 0.92 - 1.06 (m, 1 H), 2.33 (s, 3 H), 3.21 (dd, J = 7.2, 5.7 Hz, 2 H), 3.60 (s, 3 H), 4.31 (s, 2 H), 5.06 (dd, J = 11.7, 1.3 Hz, 1 H), 5.24 (dd, J = 17.9, 1.3 Hz, 1 H), 6.21 - 6.36 (m, 2 H), 6.80 - 6.89 (m, 1 H), 7.52 (s, 1 H).

### Example 66 (2S)-2-{2,4-dichloro-5-[(1,3-dimethyl-4-vinyl-1H-pyrazol-5-yl)oxy]phenoxy}-N,N-dimethylpropanamide

To a solution of (2S)-2-{2,4-dichloro-5-[(1,3-dimethyl-4-vinyl-1H-pyrazol-5-yl)oxy]phenoxy}propionic acid (223 mg) obtained in Example 14 and dimethylamine hydrochloride (98 mg) in N,N-dimethylformamide (3 mL) were added 1-ethyl-3-(3-dimethylaminopropyl)carbodiimide hydrochloride (138 mg), 1-hydroxybenzotriazole (110 mg) and triethylamine (0.167 mL), and the mixture was stirred at room temperature for 15 hr. Water was added to the reaction mixture and the mixture was extracted with ethyl acetate. The organic layer was washed with water and saturated brine, dried over anhydrous magnesium sulfate and filtered. The filtrate was concentrated, and the residue was subjected to silica gel column chromatography (hexane-ethyl acetate 9:1 - 0:100, v/v) to give the title compound as a colorless oil (153 mg, yield 64%).
¹H-NMR (300 MHz, CDCl₃)δ:1.55 (d, J = 6.7 Hz, 3 H), 2.33 (s, 3 H), 2.87 (s, 3 H), 2.93 (s, 3 H), 3.55 (s, 3 H), 4.71 (q, J = 6.7 Hz, 1 H), 5.05 (dd, J = 11.5, 1.3 Hz, 1 H), 5.26 (dd, J = 18.0, 1.3 Hz, 1 H), 6.01 (s, 1 H), 6.29 (dd, J = 18.0, 11.5 Hz, 1 H), 7.46 (s, 1 H).

### Example 67 (2S)-N-cyclopropyl-2-{2,4-dichloro-5-[(1,3-dimethyl-4-vinyl-1H-pyrazol-5-yl)oxy]phenoxy}propanamide

The title compound was obtained in the same manner as in Example 65 and from (2S)-2-12,4-dichloro-5-[(1,3-dimethyl-4-vinyl-1H-pyrazol-5-yl)oxy]phenoxy}propionic acid obtained in Example 14 and cyclopropylamine.
¹H-NMR (300 MHz, CDCl₃)δ:0.44 - 0.53 (m, 2 H), 0.75 - 0.86 (m, 2 H), 1.45 (d, J = 6.7 Hz, 3 H), 2.33 (s, 3 H), 2.67 - 2.79 (m, 1 H), 3.60 (s, 3 H), 4.45 (q, J = 6.7 Hz, 1 H), 5.06 (dd, J = 11.6, 1.3 Hz, 1 H), 5.23 (dd, J = 17.9, 1.3 Hz, 1 H), 6.21 - 6.36 (m, 2 H), 6.71 (br s, 1 H), 7.49 (s, 1 H).

### Example 68 (2S)-2-{2,4-dichloro-5-[(1,3-dimethyl-4-vinyl-1H-pyrazol-5-yl)oxy]phenoxy}-N-(3-hydroxy-2,2-dimethylpropyl)propanamide

The title compound was obtained in the same manner as in Example 65 and from (2S)-2-{2,4-dichloro-5-[(1,3-dimethyl-4-vinyl-1H-pyrazol-5-yl)oxy]phenoxy}propionic acid obtained in Example 14 and 3-amino-2,2-dimethylpropan-1-ol.
¹H-NMR (300 MHz, CDCl₃)δ:0.85 (s, 3 H), 0.88 (s, 3 H), 1.49 (d, J = 6.7 Hz, 3 H), 2.32 (s, 3 H), 3.06 - 3.20 (m, 4 H), 3.60 (s, 3 H), 4.52 (q, J = 6.7 Hz, 1 H), 5.07 (dd, J = 11.5, 1.3 Hz, 1 H), 5.24 (dd, J = 17.9, 1.3 Hz, 1 H), 6.20 - 6.37 (m, 2 H), 7.04 (br s, 1 H), 7.51 (s, 1 H).

### Example 69 5-[2,4-dichloro-5-(2-oxoimidazolidin-1-yl)phenoxy]-1,3-dimethyl-1H-pyrazole-4-carbaldehyde

5-(5-Amino-2,4-dichlorophenoxy)-1,3-dimethyl-1H-pyrazole-4-carbaldehyde (700 mg) obtained in Reference Example 56 and 2-chloroethyl isocyanate (0.22 mL) were stirred in pyridine (10 mL) at room temperature for 15 hr. Pyridine was evaporated, to a solution of the residue in N,N-dimethylformamide (5 mL) was added 60% sodium hydride (oil, 103 mg), and the mixture was stirred at room temperature for 6 hr. Saturated aqueous ammonium chloride solution was added to the reaction mixture, and the mixture was extracted with ethyl acetate. The organic layer was washed with water and saturated brine, dried over anhydrous magnesium sulfate and filtered. The filtrate was concentrated, and the residue was subjected to silica gel column chromatography (hexane-ethyl acetate 3:1 - ethyl acetate-methanol 95:5, v/v), and the obtained oil was recrystallized from hexane-ethyl acetate to give the title compound as colorless crystals (339 mg, yield 39%).
¹H-NMR (300 MHz, CDCl₃)δ:2.46 (s, 3 H), 3.59 (t, J = 7.8 Hz, 2 H), 3.70 (s, 3 H), 3.87 (t, J = 7.8 Hz, 2 H), 5.07 (br s, 1 H), 6.96 (s, 1 H), 7.57 (s, 1 H), 9.55 (s, 1 H).

### Example 70 methyl ({2,4-dichloro-5-[(4-formyl-1,3-dimethyl-1H-pyrazol-5-yl)oxy]phenyl}amino)(oxo)acetate

To a solution of 5-(5-amino-2,4-dichlorophenoxy)-1,3-dimethyl-1H-pyrazole-4-carbaldehyde (300 mg) obtained in Reference Example 56 in tetrahydrofuran (5 mL) were added methyl chloroglyoxylate (0.111 mL) and triethylamine (0.209 mL), and the mixture was stirred at room temperature for 15 hr. Water was added to the reaction mixture, and the mixture was extracted with ethyl acetate. The organic layer was washed with water and saturated brine, dried over anhydrous magnesium sulfate and filtered. The filtrate was concentrated, and the residue was recrystallized from hexane-ethyl acetate to give the title compound as colorless crystals (285 mg, yield 74%).
¹H-NMR (300 MHz, CDCl₃)δ:2.45 (s, 3 H), 3.72 (s, 3 H), 3.98 (s, 3 H), 7.57 (s, 1 H), 8.17 (s, 1 H), 9.41 (br s, 1 H), 9.54 (s, 1 H).

### Example 71 ({2,4-dichloro-5-[(4-formyl-1,3-dimethyl-1H-pyrazol-5-yl)oxy]phenyl}amino)(oxo)acetic acid

The title compound was obtained in the same manner as in Example 49 and from methyl ({2,4-dichloro-5-[(4-formyl-1,3-dimethyl-1H-pyrazol-5-yl)oxy]phenyl}amino)(oxo)acetate obtained in Example 70.
¹H-NMR (300 MHz, DMSO-d₆)δ:2.37 (s, 3 H), 3.64 (s, 3 H), 7.55 (s, 1 H), 7.97 (s, 1 H), 9.51 (s, 1 H), 10.17 (br s, 1 H).

### Example 72 3-(1-{2,4-dichloro-5-[(1,3-dimethyl-4-vinyl-1H-pyrazol-5-yl)oxy]phenoxy}ethyl)-1,2,4-oxadiazol-5(2H)-one

Hydroxylamine hydrochloride (306 mg) and sodium hydrogen carbonate (496 mg) were stirred in dimethyl sulfoxide (3 mL) at 40°C for 1 hr. 2-{2,4-Dichloro-5-[(1,3-dimethyl-4-vinyl-1H-pyrazol-5-yl)oxy]phenoxy}propanenitrile (400 mg) obtained in Reference Example 57 was added to the reaction mixture, and the mixture was stirred at 80°C for 3 hr. Water was added to the reaction mixture, and the mixture was extracted with ethyl acetate. The organic layer was washed with water and saturated brine, dried over anhydrous magnesium sulfate and filtered, and the filtrate was concentrated. To a solution of the obtained residue in tetrahydrofuran (5 mL) were added 1,1'-carbonylbis-1H-imidazole (357 mg) and 1,8-diazabicyclo[5.4.0]undec-7-ene (0.41 mL), and the mixture was stirred at room temperature for 1 hr. 1N Hydrochloric acid (2 mL) was added to the reaction mixture, and the mixture was extracted with ethyl acetate. The organic layer was washed with water and saturated brine, dried over anhydrous magnesium sulfate and filtered. The filtrate was concentrated, and the residue was subjected to silica gel column chromatography (hexane-ethyl acetate 9:1 - 1:1, v/v), and the obtained oil was recrystallized from hexane-diethyl ether to give the title compound as colorless crystals (57 mg, yield 12%).
¹H-NMR (300 MHz, CDCl₃)δ:1.69 (d, J = 6.8 Hz, 3 H), 2.29 (s, 3 H), 3.59 (s, 3 H), 4.98 - 5.13 (m, 2 H), 5.18 (dd, J = 17.9, 1.3 Hz, 1 H), 6.25 (dd, J = 17. 9, 11.7 Hz, 1 H), 6.35 (s, 1 H), 7.51 (s, 1 H).

### Example 73 methyl (2S)-2-(2,4-dichloro-5-{[1,4-dimethyl-3-(trifluoromethyl)-1H-pyrazol-5-yl]oxy}phenoxy)propionate

The title compound was obtained in the same manner as in Example 40 and from 2,4-dichloro-5-{[1,4-dimethyl-3-(trifluoromethyl)-1H-pyrazol-5-yl]oxy}phenol obtained in Reference Example 60 and methyl (R)-(+)-lactate.
¹H-NMR (300 MHz, CDCl₃)δ:1.63 (d, J = 6.8 Hz, 3 H), 1.90 (d, J = 0.9 Hz, 3 H), 3.66 (s, 3 H), 3.69 (s, 3 H), 4.52 (q, J = 6.8 Hz, 1 H), 6.15 (s, 1 H), 7.49 (s, 1 H).

### Example 74 (2S)-2-(2,4-dichloro-5-{[1,4-dimethyl-3-(trifluoromethyl)-1H-pyrazol-5-yl]oxy}phenoxy)propionic acid

The title compound was obtained in the same manner as in Example 43 and from methyl (2S)-2-(2,4-dichloro-5-{[1,4-dimethyl-3-(trifluoromethyl)-1H-pyrazol-5-yl]oxy}phenoxy)propionate obtained in Example 73.
¹H-NMR (300 MHz, CDCl₃)δ:1.68 (d, J = 6.9 Hz, 3 H), 1.90 (d, J = 0.9 Hz, 3 H), 3.67 (s, 3 H), 4.58 (q, J = 6.9 Hz, 1 H), 6.23 (s, 1 H), 7.50 (s, 1 H).

### Example 75 methyl (2S)-2-(2,4-dichloro-5-{[4-ethyl-1-methyl-3-(trifluoromethyl)-1H-pyrazol-5-yl]oxy}phenoxy)propionate

The title compound was obtained in the same manner as in Example 40 and from 2,4-dichloro-5-{[4-ethyl-1-methyl-3-(trifluoromethyl)-1H-pyrazol-5-yl]oxy}phenol obtained in Reference Example 61 and methyl (R)-(+)-lactate.
¹H-NMR (300 MHz, CDCl₃)δ:1.04 (t, J = 7.5 Hz, 3 H), 1.62 (d, J = 6.8 Hz, 3 H), 2.37 (q, J = 7.5 Hz, 2 H), 3.65 (s, 3 H), 3.66 (s, 3 H), 4.49 (q, J = 6.8 Hz, 1 H), 6.11 (s, 1 H), 7.49 (s, 1 H).

### Example 76 (2S)-2-(2,4-dichloro-5-{[4-ethyl-1-methyl-3-(trifluoromethyl)-1H-pyrazol-5-yl]oxy}phenoxy)propionic acid

The title compound was obtained in the same manner as in Example 43 and from methyl (2S)-2-(2,4-dichloro-5-{[4-ethyl-1-methyl-3-(trifluoromethyl)-1H-pyrazol-5-yl]oxy}phenoxy)propionate obtained in Example 75.
¹H-NMR (300 MHz, CDCl₃)δ:1.03 (t, J = 7.6 Hz, 3 H), 1.67 (d, J = 6.9 Hz, 3 H), 2.37 (q, J = 7.6 Hz, 2 H), 3.64 (s, 3 H), 4.55 (q, J = 6.9 Hz, 1 H), 6.19 (s, 1 H), 7.50 (s, 1 H), 8.57 (br s, 1 H).

### Example 77 ethyl (2E)-3-{4-chloro-3-[(1,3,4-trimethyl-1H-pyrazol-5-yl)oxy]phenyl}acrylate

The title compound was obtained in the same manner as in Example 50 and from 5-(5-bromo-2-chlorophenoxy)-1,3,4-trimethyl-1H-pyrazole obtained in Reference Example 63 and ethyl acrylate.
¹H-NMR (300 MHz, CDCl₃)δ:1.33 (t, J = 7.2 Hz, 3 H), 1.76 (s, 3 H), 2.21 (s, 3 H), 3.60 (s, 3 H), 4.25 (q, J = 7.2 Hz, 2 H), 6.29 (d, J = 16.0 Hz, 1 H), 6.79 (d, J = 2.1 Hz, 1 H), 7.20 (dd, J = 8.2, 1.8 Hz, 1 H), 7.40 - 7.56 (m, 2 H).

### Example 78 ethyl 3-{4-chloro-3-[(1,3,4-trimethyl-1H-pyrazol-5-yl)oxy]phenyl}propionate

The title compound was obtained in the same manner as in Example 52 and from ethyl (2E)-3-{4-chloro-3-[(1,3,4-trimethyl-1H-pyrazol-5-yl)oxy]phenyl}acrylate obtained in Example 77.
¹H-NMR (300 MHz, CDCl₃)δ:1.22 (t, J = 7.1 Hz, 3 H), 1.74 (s, 3 H), 2.20 (s, 3 H), 2.51 (t, J = 7.6 Hz, 2 H), 2.83 (t, J = 7.6 Hz, 2 H), 3.58 (s, 3 H), 4.09 (q, J = 7.1 Hz, 2 H), 6.51 (d, J = 1.9 Hz, 1 H), 6.87 (dd, J = 8.2, 1.9 Hz, 1 H), 7.34 (d, J = 8.2 Hz, 1 H).

### Example 79 3-{4-chloro-3-[(1,3,4-trimethyl-1H-pyrazol-5-yl)oxy]phenyl}propionic acid

The title compound was obtained in the same manner as in Example 49 and from ethyl 3-{4-chloro-3-[(1,3,4-trimethyl-1H-pyrazol-5-yl)oxy]phenyl}propionate obtained in Example 78.
¹H-NMR (300 MHz, CDCl₃)δ:1.74 (s, 3 H), 2.18 (s, 3 H), 2.57 (t, J = 7.4 Hz, 2 H), 2.85 (t, J = 7.4 Hz, 2 H), 3.57 (s, 3 H), 6.53 (d, J = 1.9 Hz, 1 H), 6.89 (dd, J = 8.1, 1.9 Hz, 1 H), 7.35 (d, J = 8.1 Hz, 1 H).

### Example 80 3-{4-chloro-3-[(1,3,4-trimethyl-1H-pyrazol-5-yl)oxy]phenyl}-N-(methylsulfonyl)propanamide

The title compound was obtained in the same manner as in Example 63 and from 3-{4-chloro-3-[(1,3,4-trimethyl-1H-pyrazol-5-yl)oxy]phenyl}propionic acid obtained in Example 79 and methanesulfonamide.
¹H-NMR (300 MHz, CDCl₃)δ:1.75 (s, 3 H), 2.18 (s, 3 H), 2.57 (t, J = 7.5 Hz, 2 H), 2.89 (t, J = 7.5 Hz, 2 H), 3.25 (s, 3 H), 3.56 (s, 3 H), 6.49 (d, J = 1.9 Hz, 1 H), 6.88 (dd, J = 8.1, 1.9 Hz, 1 H), 7.36 (d, J = 8.1 Hz, 1 H).

### Example 81 3-{4-chloro-3-[(1,3,4-trimethyl-1H-pyrazol-5-yl)oxy]phenyl}-N-(pentylsulfonyl)propanamide

The title compound was obtained in the same manner as in Example 63 and from 3-{4-chloro-3-[(1,3,4-trimethyl-1H-pyrazol-5-yl)oxy]phenyl}propionic acid obtained in Example 79 and pentanesulfonamide.
¹H-NMR (300 MHz, CDCl₃)δ:0.84 - 0.96 (m, 3 H), 1.26 - 1.46 (m, 4 H), 1.67 - 1.82 (m, 5 H), 2.19 (s, 3 H), 2.56 (t, J = 7.5 Hz, 2 H), 2.88 (t, J = 7.5 Hz, 2 H), 3.31 - 3.43 (m, 2 H), 3.57 (s, 3 H), 6.50 (d, J = 1.9 Hz, 1 H), 6.88 (dd, J = 8.1, 1.9 Hz, 1 H), 7.36 (d, J = 8.1 Hz, 1 H).

### Example 82 ethyl (2E)-3-{4-chloro-3-[(1,3,4-trimethyl-1H-pyrazol-5-yl)oxy]phenyl}-2-methylacrylate

To a solution of ethyl 2-(diethoxyphosphoryl)propionate (0.472 mL) in tetrahydrofuran (20 mL) was added 60% sodium hydride (oil, 88 mg), and the mixture was stirred at room temperature for 20 min. 4-Chloro-3-[(1,3,4-trimethyl-1H-pyrazol-5-yl)oxy]benzaldehyde (529 mg) obtained in Reference Example 64 was added to the reaction mixture, and the mixture was stirred for 1 hr. Saturated aqueous ammonium chloride solution was added to the reaction mixture and the mixture was extracted with ethyl acetate. The organic layer was washed with water and saturated brine, dried over anhydrous magnesium sulfate and filtered. The filtrate was concentrated, and the residue was subjected to silica gel column chromatography (hexane-ethyl acetate 9:1 - 2:1, v/v) to give the title compound as a colorless oil (590 mg, yield 85%).
¹H-NMR (300 MHz, CDCl₃)δ:1.33 (t, J = 7.2 Hz, 3 H), 1.76 (s, 3 H), 1.99 (d, J= 1.5 Hz, 3 H), 2.19 (s, 3 H), 3.59 (s, 3 H), 4.25 (q, J = 7.2 Hz, 2 H), 6.65 (d, J = 1.9 Hz, 1 H), 7.07 (dd, J = 8.2, 1.9 Hz, 1 H), 7.43 - 7.50 (m, 2 H).

### Example 83 ethyl 3-{4-chloro-3-[(1,3,4-trimethyl-1H-pyrazol-5-yl)oxy]phenyl}-2-methylpropionate

The title compound was obtained in the same manner as in Example 52 and from ethyl (2E)-3-{4-chloro-3-[(1,3,4-trimethyl-1H-pyrazol-5-yl)oxy]phenyl}-2-methylacrylate obtained in Example 82.
¹H-NMR (300 MHz, CDCl₃)δ:1.10 (d, J = 6.6 Hz, 3 H), 1.17 (t, J = 7.2 Hz, 3 H), 1.73 (s, 3 H), 2.19 (s, 3 H), 2.51 - 2.66 (m, 2 H), 2.81 - 2.94 (m, 1 H), 3.58 (s, 3 H), 3.98 - 4.08 (m, 2 H), 6.48 (d, J = 1.9 Hz, 1 H), 6.83 (dd, J = 8.1, 1.9 Hz, 1 H), 7.33 (d, J = 8.1 Hz, 1 H).

### Example 84 3-{4-chloro-3-[(1,3,4-trimethyl-1H-pyrazol-5-yl)oxy]phenyl}-2-methylpropionic acid

The title compound was obtained in the same manner as in Example 49 and from ethyl 3-{4-chloro-3-[(1,3,4-trimethyl-1H-pyrazol-5-yl)oxy]phenyl}-2-methylpropionate obtained in Example 83.
¹H-NMR (300 MHz, DMSO-d₆)δ:1.00 (d, J = 6.8 Hz, 3 H), 1.63 (s, 3 H), 2.08 (s, 3 H), 2.48 - 2.64 (m, 2 H), 2.71 - 2.84 (m, 1 H), 3.49 (s, 3 H), 6.62 (d, J = 1.9 Hz, 1 H), 6.99 (dd, J = 8.1, 1.9 Hz, 1 H), 7.47 (d, J = 8.1 Hz, 1 H), 12.16 (br s, 1 H).

### Example 85 (2S)-2-{5-[(4-acetyl-1,3-dimethyl-1H-pyrazol-5-yl)oxy]-2,4-dichlorophenoxy}propionic acid

The title compound was obtained in the same manner as in Example 1 and from 1-[5-(2,4-dichloro-5-hydroxyphenoxy)-1,3-dimethyl-1H-pyrazol-4-yl]ethanone obtained in Reference Example 66.
¹H-NMR (300 MHz, CDCl₃)δ:1.65 (d, J = 6.8 Hz, 3 H), 2.25 (s, 3 H), 2.46 (s, 3 H), 3.57 (s, 3 H), 4.49 (q, J = 6.8 Hz, 1 H), 6.09 (s, 1 H), 7.53 (s, 1 H).

### Example 86 methyl (2S)-2-(2,4-dichloro-5-{[1,3-dimethyl-4-(2-methyl-1-propen-1-yl)-1H-pyrazol-5-yl]oxy}phenoxy)propionate

A solution of 2,4-dichloro-5-{[1,3-dimethyl-4-(2-methyl-1-propen-1-yl)-1H-pyrazol-5-yl]oxy}phenol (0.50 g) obtained in Reference Example 67, methyl (R)-(+)-lactate (0.39 g), and triphenylphosphine (0.99 g) in tetrahydrofuran (15 mL) was purged with nitrogen with stirring, and cooled to 0°C. Diethyl azodicarboxylate (40% toluene solution, 1.71 mL) was added dropwise to the reaction mixture, and the mixture was stirred for 12 hr while heating to room temperature. The reaction mixture was concentrated, and the residue was subjected to silica gel column chromatography (hexane-ethyl acetate 1:0 - 6:4, v/v) to give the title compound (0.49 g, yield 79%) as a colorless oil.
¹H-NMR (300 MHz, CDCl₃)δ:1.56 (d, J = 1.1 Hz, 3 H), 1.63 (d, J = 6.8 Hz, 3 H), 1.69 (d, J = 1.3 Hz, 3 H), 2.19 (s, 3 H), 3.71 (s, 3 H), 3.72 (s, 3 H), 4.56 (q, J = 6.8 Hz, 1 H), 5.39 - 5.44 (m, 1 H), 6.30 (s, 1 H), 7.44 (s, 1 H).

### Example 87 (2S)-2-(2,4-dichloro-5-{[1,3-dimethyl-4-(2-methyl-1-propen-1-yl-1H-pyrazol-5-yl]oxy}phenoxy)propionic acid

While stirring a solution of methyl (2S)-2-(2,4-dichloro-5-{[1,3-dimethyl-4-(2-methyl-1-propen-1-yl)-1H-pyrazol-5-yl]oxy}phenoxy)propionate (0.38 g) obtained in Example 86 in tetrahydrofuran (3 mL), water (3 mL) was added, and the mixture was cooled to 0°C. Lithium hydroxide monohydrate (0.16 g) was added, and the mixture was stirred at the same temperature for 2 hr. The reaction solution was neutralized with 1N hydrochloric acid (4 mL), and extracted with ethyl acetate. The organic layer was dried over anhydrous magnesium sulfate, and filtered, and the filtrate was concentrated. The residue was crystallized from hexane-ethyl acetate to give the title compound as colorless crystals (0.19 g, yield 50%).
¹H-NMR (300 MHz, CDCl₃) δ:1.61 - 1.68 (m, 6 H), 1.78 (d, J = 1.3 Hz, 3 H), 2.15 (s, 3 H), 3.52 (s, 3 H), 4.40 (q, J = 6.9 Hz, 1 H), 5.63 - 5.70 (m, 1 H), 6.07 (s, 1 H), 7.43 (s, 1 H).

### Example 88 methyl (2S)-2-[2,4-dichloro-5-({1,3-dimethyl-4-[(1E)-1-penten-1-yl]-1H-pyrazol-5-yl}oxy)phenoxy]propionate

The title compound was obtained in the same manner as in Example 86 and from 2,4-dichloro-5-({1,3-dimethyl-4-[(1E)-1-penten-1-yl]-1H-pyrazol-5-yl}oxy)phenol obtained in Reference Example 68.
¹H-NMR (300 MHz, CDCl₃)δ:0.82 (t, J = 7.3 Hz, 3 H), 1.24 - 1.40 (m, 2 H), 1.59 (d, J = 6.8 Hz, 3 H), 1.97 - 2.07 (m, 2 H), 2.32 (s, 3 H), 3.62 (s, 3 H), 3.65 (s, 3 H), 4.51 (q, J = 6.8 Hz, 1 H), 5.74 (dt, J = 6.8, 16.2 Hz, 1 H), 5.84 - 5.92 (m, 1 H), 6.18 (s, 1 H), 7.48 (s, 1 H).

### Example 89 (2S)-2-[2,4-dichloro-5-({1,3-dimethyl-4-[(1E)-1-penten-1-yl]-1H-pyrazol-5-yl}oxy)phenoxy]propionic acid

The title compound was obtained in the same manner as in Example 87 and from methyl (2S)-2-[2,4-dichloro-5-({1,3-dimethyl-4-[(lE)-1-penten-1-yl]-1H-pyrazol-5-yl}oxy)phenoxy]propionate obtained in Example 88.
¹H-NMR (300 MHz, CDCl₃)δ:0.84 (t, J = 7.3 Hz, 3 H), 1.36 (q, 2 H), 1.64 (d, J = 7.0 Hz, 3 H), 2.04 (m, 2 H), 2.27 (s, 3 H), 3.50 (s, 3 H), 4.42 (q, J = 7.0 Hz, 1 H), 5.82 (dt, J = 6.6, 16.2 Hz, 1 H), 5.97 (d, J = 16.2 Hz, 1 H), 6.09 (s, 1 H), 7.47 (s, 1 H).

### Example 90 methyl (2S)-2-{2,4-dichloro-5-[(4-isobutyl-1,3-dimethyl-1H-pyrazol-5-yl)oxy]phenoxy}propionate

The title compound was obtained in the same manner as in Example 86 and from 2,4-dichloro-5-[(4-isobutyl-1,3-dimethyl-1H-pyrazol-5-yl)oxy]phenol obtained in Reference Example 70.
¹H-NMR (300 MHz, CDCl₃)δ:0.82 (d, J = 6.6 Hz, 6 H), 1.56 - 1.70 (m, 4 H), 2.01 (d, J= 7.2 Hz, 2 H), 2.25 (s, 3 H), 3.65 (s, 3 H), 3.68 (s, 3 H), 4.54 (q, J = 6.8 Hz, 1 H), 6.20 (s, 1 H), 7.48 (s, 1 H).

### Example 91 (2S)-2-{2,4-dichloro-5-[(4-isobutyl-1,3-dimethyl-1H-pyrazol-5-yl)oxy]phenoxy}propionic acid

The title compound was obtained in the same manner as in Example 87 and from methyl (2S)-2-{2,4-dichloro-5-[(4-isobutyl-1,3-dimethyl-1H-pyrazol-5-yl)oxy]phenoxy}propionate obtained in Example 90.
¹H-NMR (300 MHz, CDCl₃)δ:0.87 (dd, J = 6.6, 0.9 Hz, 6 H), 1.58 - 1.76 (m, 4 H), 2.05 (dd, J = 6.6, 14.4 Hz, 1 H), 2.12 - 2.25 (m, 4 H), 3.48 (s, 3 H), 4.37 (q, J = 6.7 Hz, 1 H), 6.06 (s, 1 H), 7.46 (s, 1 H).

### Example 92 methyl (2S)-2-(2,4-dichloro-5-{[4-(cyclohexylmethyl)-1,3-dimethyl-1H-pyrazol-5-yl]oxy}phenoxy)propionate

The title compound was obtained in the same manner as in Example 86 and from 2,4-dichloro-5-{[4-(cyclohexylmethyl)-1,3-dimethyl-1H-pyrazol-5-yl]oxy}phenol obtained in Reference Example 72.
¹H-NMR (300 MHz, CDCl₃)δ:0.71 - 0.93 (m, 2 H), 1.00 - 1.41 (m, 5 H), 1.50 - 1.71 (m, 7 H), 2.02 (d, J = 7.2 Hz, 2 H), 2.19 (s, 3 H), 3.54 (s, 3 H), 3.66 (s, 3 H), 4.48 (q, J = 6.8 Hz, 1 H), 6.13 (s, 1 H), 7.46 (s, 1 H).

### Example 93 (2S)-2-(2,4-dichloro-5-{[4-(cyclohexylmethyl)-1,3-dimethyl-1H-pyrazol-5-yl]oxy}phenoxy)propionic acid

The title compound was obtained in the same manner as in Example 87 and from methyl (2S)-2-(2,4-dichloro-5-{[4-(cyclohexylmethyl)-1,3-dimethyl-1H-pyrazol-5-yl]oxy}phenoxy)propionate obtained in Example 92.
¹H-NMR (300 MHz, CDCl₃)δ:0.74 - 0.99 (m, 2 H), 1.01 - 1.21 (m, 3 H), 1.21 - 1.44 (m, 1 H), 1.52 - 1.76 (m, 8 H), 2.00 - 2.10 (m, 1 H), 2.12 - 2.24 (m, 4 H), 3.48 (s, 3 H), 4.36 (q, J = 6.8 Hz, 1 H), 6.06 (s, 1 H), 7.46 (s, 1 H).

### Example 94 methyl (2S)-2-{2,4-dichloro-5-[(1,3-dimethyl-4-pentyl-1H-pyrazol-5-yl)oxy]phenoxy}propionate

The title compound was obtained in the same manner as in Example 86 and from 2,4-dichloro-5-[(1,3-dimethyl-4-pentyl-1H-pyrazol-5-yl)oxy]phenol obtained in Reference Example 74.
¹H-NMR (300 MHz, CDCl₃)δ:0.78 - 0.88 (m, 3 H), 1.09 - 1.46 (m, 6 H), 1.61 (d, J = 6.8 Hz, 3 H), 2.07 - 2.19 (m, 2 H), 2.22 (s, 3 H), 3.58 (s, 3 H), 3.67 (s, 3 H), 4.52 (q, J = 6.8 Hz, 1 H), 6.18 (s, 1 H), 7.47 (s, 1 H).

### Example 95 (2S)-2-{2,4-dichloro-5-[(1,3-dimethyl-4-pentyl-1H-pyrazol-5-yl)oxy]phenoxy}propionic acid

The title compound was obtained in the same manner as in Example 87 and from methyl (2S)-2-{2,4-dichloro-5-[(1,3-dimethyl-4-pentyl-1H-pyrazol-5-yl)oxy]phenoxy}propionate obtained in Example 94.
¹H-NMR (300 MHz, CDCl₃)δ:0.85 (t, J = 6.9 Hz, 3 H), 1.13 - 1.34 (m, 4 H), 1.34 - 1.50 (m, 2 H), 1.65 (d, J = 6.8 Hz, 3 H), 2.12 - 2.36 (m, 5 H), 3.48 (s, 3 H), 4.38 (q, J = 6.7 Hz, 1 H), 6.07 (s, 1 H), 7.46 (s, 1 H).

### Example 96 methyl (2S)-2-(2,4-dichloro-5-{[4-(ethoxymethyl)-1,3-dimethyl-1H-pyrazol-5-yl]oxy}phenoxy)propionate

The title compound was obtained in the same manner as in Example 86 and from 2,4-dichloro-5-{[4-(ethoxymethyl)-1,3-dimethyl-1H-pyrazol-5-yl]oxy}phenol obtained in Reference Example 77.
¹H-NMR (300 MHz, CDCl₃)δ:1.10 (t, J = 7.1 Hz, 3 H), 1.60 (d, J = 6.8 Hz, 3 H), 2.26 (s, 3 H), 3.28 - 3.44 (m, 2 H), 3.57 (s, 3 H), 3.67 (s, 3 H), 4.08 (d, J = 1.7 Hz, 2 H), 4.58 (q, J = 6.8 Hz, 1 H), 6.38 (s, 1 H), 7.45 (s, 1 H).

### Example 97 (2S)-2-(2,4-dichloro-5-{[4-(ethoxymethyl)-1,3-dimethyl-1H-pyrazol-5-yl]oxy}phenoxy)propionic acid

The title compound was obtained in the same manner as in Example 87 and from methyl (2S)-2-(2,4-dichloro-5-{[4-(ethoxymethyl)-1,3-dimethyl-1H-pyrazol-5-yl]oxy}phenoxy)propionate obtained in Example 96.
¹H-NMR (300 MHz, CDCl₃)δ:1.11 - 1.19 (m, 3 H), 1.65 (d, J = 7.0 Hz, 3 H), 2.25 (s, 3 H), 3.35 - 3.52 (m, 2 H), 3.51 - 3.58 (m, 3 H), 4.07 - 4.25 (m, 2 H), 4.52 (q, J = 6.8 Hz, 1 H), 6.31 (s, 1 H), 7.46 (s, 1 H).

### Example 98 methyl (2S)-2-(2,4-dichloro-5-{[4-(methoxymethyl)-1,3-dimethyl-1H-pyrazol-5-yl]oxy}phenoxy)propionate

The title compound was obtained in the same manner as in Example 86 and from 2,4-dichloro-5-{[4-(methoxymethyl)-1,3-dimethyl-1H-pyrazol-5-yl]oxy}phenol obtained in Reference Example 79.
¹H-NMR (300 MHz, CDCl₃)δ:1.61 (d, J = 6.8 Hz, 3 H), 2.26 (s, 3 H), 3.21 (s, 3 H), 3.57 (s, 3 H), 3.67 (s, 3 H), 4.04 (d, J = 0.8 Hz, 2 H), 4.58 (q, J = 6.8 Hz, 1 H), 6.42 (s, 1 H), 7.45 (s, 1 H).

### Example 99 (2S)-2-(2,4-dichloro-5-{[4-(methoxymethyl)-1,3-dimethyl-1H-pyrazol-5-yl]oxy}phenoxy)propionic acid

The title compound was obtained in the same manner as in Example 87 and from methyl (2S)-2-(2,4-dichloro-5-{[4-(methoxymethyl)-1,3-dimethyl-1H-pyrazol-5-yl]oxy}phenoxy)propionate obtained in Example 98.
¹H-NMR (300 MHz, CDCl₃)δ:1.65 (d, J = 6.8 Hz, 3 H), 2.25 (s, 3 H), 3.28 (s, 3 H), 3.55 (s, 3 H), 4.05 - 4.22 (m, 2 H), 4.50 (q, J = 6.8 Hz, 1 H), 6.31 (s, 1 H), 7.46 (s, 1 H).

### Example 100 methyl (2S)-2-{2,4-dichloro-5-[(1,3-dimethyl-1H-pyrazol-5-yl)(methyl)amino]phenoxy}propionate

The title compound was obtained in the same manner as in Example 86 and from 2,4-dichloro-5-[(1,3-dimethyl-1H-pyrazol-5-yl)(methyl)amino]phenol obtained in Reference Example 83.
¹H-NMR (300 MHz, CDCl₃)δ:1.61 (d, J = 6.8 Hz, 3 H), 2.23 (s, 3 H), 3.09 (s, 3 H), 3.27 (s, 3 H), 3.67 (s, 3 H), 4.56 (q, J = 6.8 Hz, 1 H), 5.75 (s, 1 H), 6.26 (s, 1 H), 7.42 (s, 1 H).

### Example 101 (2S)-2-{2,4-dichloro-5-[(1,3-dimethyl-1H-pyrazol-5-yl)(methyl)amino]phenoxy}propionic acid

The title compound was obtained in the same manner as in Example 87 and from methyl (2S)-2-{2,4-dichloro-5-[(1,3-dimethyl-1H-pyrazol-5-yl)(methyl)amino]phenoxy}propionate obtained in Example 100.
¹H-NMR (300 MHz, CDCl₃)δ:1.64 (d, J = 6.8 Hz, 3 H), 2.30 (s, 3 H), 3.16 (s, 3 H), 3.18 (s, 3 H), 4.51 (q, J = 6.9 Hz, 1 H), 5.90 (s, 1 H), 6.17 (s, 1 H), 7.46 (s, 1 H).

### Example 102 methyl (2S)-2-{2,4-dichloro-5-[(4-formyl-1,3-dimethyl-1H-pyrazol-5-yl)oxy]phenoxy}propionate

The title compound was obtained in the same manner as in Example 86 and from 5-(2,4-dichloro-5-hydroxyphenoxy)-1,3-dimethyl-1H-pyrazole-4-carbaldehyde obtained in Reference Example 52.
¹H-NMR (300 MHz, CDCl₃)δ:1.64 (d, J = 6.8 Hz, 3 H), 2.46 (s, 3 H), 3.67 (s, 3 H), 3.69 (s, 3 H), 4.59 (q, J = 6.8 Hz, 1 H), 6.45 (s, 1 H), 7.50 (s, 1 H), 9.48 (s, 1 H).

### Example 103 methyl (2S)-2-[2,4-dichloro-5-({4-[(E)-(hydroxyimino)methyl]-1,3-dimethyl-1H-pyrazol-5-yl}oxy)phenoxy]propionate

To a solution of methyl (2S)-2-{2,4-dichloro-5-[(4-formyl-1,3-dimethyl-1H-pyrazol-5-yl)oxy]phenoxy}propionate (0.80 g) obtained in Example 102 in methanol (5 mL) was added hydroxylamine monohydrate (0.52 g), and the mixture was stirred at 50°C for 12 hr. The reaction mixture was concentrated, water was added to the residue, and the mixture was extracted with ethyl acetate. The organic layer was dried over anhydrous magnesium sulfate, and filtered, and the filtrate was concentrated. The residue was subjected to silica gel column chromatography (hexane-ethyl acetate 1:0 - 3:7, v/v), and crystallized from hexane-ethyl acetate to give the title compound (0.65 g, yield 78%) as colorless crystals.
¹H-NMR (300 MHz, CDCl₃)δ:1.62 (d, J = 6.8 Hz, 3 H), 2.35 (s, 3 H), 3.61 (s, 3 H), 3.69 (s, 3 H), 4.56 (q, J = 6.8 Hz, 1 H), 6.28 (s, 1 H), 7.21 (s, 1 H), 7.45 (s, 1 H), 7.79 (s, 1 H).

### Example 104 (2S)-2-[2,4-dichloro-5-({4-[(E)-(hydroxyimino)methyl]-1,3-dimethyl-1H-pyrazol-5-yl}oxy)phenoxy]propionic acid

The title compound was obtained in the same manner as in Example 87 and from methyl (2S)-2-[2,4-dichloro-5-({4-[(E)-(hydroxyimino)methyl]-1,3-dimethyl-1H-pyrazol-5-yl}oxy)phenoxy]propionate obtained in Example 103.
¹H-NMR (300 MHz, CDCl₃)δ:1.66 (d, J = 6.8 Hz, 3 H), 2.32 (s, 3 H), 3.56 (s, 3 H), 4.47 (q, J = 6.9 Hz, 1 H), 6.15 (s, 1 H), 7.49 (s, 1 H), 7.86 (s, 1 H).

### Example 105 methyl (2S)-2-{2,4-dichloro-5-[(4-cyano-1,3-dimethyl-1H-pyrazol-5-yl)oxy]phenoxy}propionate

A suspension of methyl (2S)-2-[2,4-dichloro-5-({4-[(E)-(hydroxyimino)methyl]-1,3-dimethyl-1H-pyrazol-5-yl}oxy)phenoxy]propionate (0.43 g) obtained in Example 103 in tetrahydrofuran (10 mL) was cooled to 0°C with stirring, triethylamine (0.30 mL) and trichloroacetyl chloride (0.24 mL) were added, and the mixture was stirred at 60°C for 3 hr. Water was added to the reaction mixture, and the mixture was extracted with ethyl acetate. The organic layer was dried over anhydrous magnesium sulfate, and filtered, and the filtrate was concentrated. The residue was subjected to silica gel column chromatography (hexane-ethyl acetate 1:0 - 4:6, v/v) and crystallized from hexane-ethyl acetate to give the title compound (0.34 g, yield 84%) as colorless crystals.
¹H-NMR (300 MHz, CDCl₃)δ:1.69 (d, J = 6.8 Hz, 3 H), 2.27 (s, 3 H), 3.74 (s, 3 H), 3.76 (s, 3 H), 4.73 (q, J = 6.8 Hz, 1 H), 6.81 (s, 1 H), 7.52 (s, 1 H).

### Example 106 (2S)-2-{2,4-dichloro-5-[(4-cyano-1,3-dimethyl-1H-pyrazol-5-yl)oxy]phenoxy}propionic acid

The title compound was obtained in the same manner as in Example 87 and from methyl (2S)-2-{2,4-dichloro-5-[(4-cyano-1,3-dimethyl-1H-pyrazol-5-yl)oxy]phenoxy}propionate obtained in Example 105.
¹H-NMR (300 MHz, CDCl₃)δ:1.73 (d, J = 6.8 Hz, 3 H), 2.27 (s, 3 H), 3.74 (s, 3 H), 4.78 (q, J = 6.8 Hz, 1 H), 6.87 (s, 1 H), 7.53 (s, 1 H).

### Example 107 (2S)-2-{5-[(4-benzyl-l,3-dimethyl-1H-pyrazol-5-yl)oxy]-2,4-dichlorophenoxy}propionic acid

The title compound was obtained in the same manner as in Example 1 and from 5-[(4-benzyl-1,3-dimethyl-1H-pyrazol-5-yl)oxy]-2,4-dichlorophenol obtained in Reference Example 85.
¹H-NMR (300 MHz, CDCl₃)δ:1.60 (d, J = 6.8 Hz, 3 H), 2.18 (s, 3 H), 3.53 (s, 3 H), 3.64 (s, 2 H), 4.13 (q, J = 6.9 Hz, 1 H), 5.89 (s, 1 H), 7.04 - 7.25 (m, 5 H), 7.40 (s, 1 H).

### Example 108 methyl (2S)-2-[2,4-dichloro-5-({1,3-dimethyl-4-[(E)-2-pyridin-2-ylvinyl]-1H-pyrazol-5-yl}oxy)phenoxy]propionate hydrochloride

A mixture containing a free form of the title compound was obtained in the same manner as in Example 86 and from 2,4-dichloro-5-({1,3-dimethyl-4-[(E)-2-pyridin-2-ylvinyl]-1H-pyrazol-5-yl}oxy)phenol (0.78 g) obtained in Reference Example 87. 10% Hydrochloric acid-methanol solution (5 mL) was added, and the mixture was stirred at room temperature for 30 min. The reaction mixture was concentrated, and the residue was crystallized from methanol-ethyl acetate to give the title compound (0.87 g, yield 84%) as pale-yellow crystals.
¹H-NMR (300 MHz, DMSO-d₆)δ:1.44 (d, J = 6.8 Hz, 3 H), 2.41 (s, 3 H), 3.48 (s, 3 H), 3.52 (s, 3 H), 4.94 (q, J = 6.7 Hz, 1 H), 6.29 (s, 1 H), 6.84 (d, J = 16.6 Hz, 1 H), 7.59 - 7.74 (m, 2 H), 7.88 (s, 1 H), 8.07 - 8.24 (m, 1 H), 8.34 (s, 1 H), 8.60 - 8.70 (m, 1 H).

### Example 109 methyl (2S)-2-(2,4-dichloro-5-{[4-(cyanomethyl)-1,3-dimethyl-1H-pyrazol-5-yl]oxy}phenoxy)propionate

The title compound was obtained in the same manner as in Example 86 and from [5-(2,4-dichloro-5-hydroxyphenoxy)-1,3-dimethyl-1H-pyrazol-4-yl]acetonitrile obtained in Reference Example 89.
¹H-NMR (300 MHz, CDCl₃)δ:1.64 (d, J = 6.4 Hz, 3 H), 2.33 (s, 3 H), 3.16 - 3.33 (m, 2 H), 3.66 (s, 3 H), 3.70 (s, 3 H), 4.68 (q, J = 6.6 Hz, 1 H), 6.34 (d, J = 2.3 Hz, 1 H), 7.51 (s, 1 H).

### Example 110 (2S)-2-(2,4-dichloro-5-{[4-(cyanomethyl)-1,3-dimethyl-1H-pyrazol-5-yl]oxy}phenoxy)propionic acid

The title compound was obtained in the same manner as in Example 87 and from methyl (2S)-2-(2,4-dichloro-5-{[4-(cyanomethyl)-1,3-dimethyl-1H-pyrazol-5-yl]oxy}phenoxy)propionate obtained in Example 109.
¹H-NMR (300 MHz, CDCl₃)δ:1.66 (d, J = 7.0 Hz, 3 H), 2.30 (s, 3 H), 3.36 (d, J = 1.1 Hz, 2 H), 3.55 (s, 3 H), 4.58 (q, J = 6.7 Hz, 1 H), 6.16 (s, 1 H), 7.50 (s, 1 H)

### Example 111 methyl (2S)-2-{3-[(1,3-dimethyl-4-vinyl-1H-pyrazol-5-yl)thio]phenoxy}propionate

A solution of 3-[(1,3-dimethyl-4-vinyl-1H-pyrazol-5-yl)thio]phenol (0.82 g) obtained in Reference Example 91, methyl (R)-(+)-lactate (0.937 g), and triphenylphosphine (2.36 g) in dichloromethane (30 mL) was purged with nitrogen, and cooled to 0°C. Diethyl azodicarboxylate (40% toluene solution, 4.21 mL) was added dropwise to the reaction mixture, and the mixture was stirred at 0°C for 15 min, and at room temperature for 15 hr. The reaction mixture was concentrated, and the residue was subjected to silica gel column chromatography (hexane-ethyl acetate 90:10 - 80:20, v/v). The solvent was evaporated to give a colorless oil (0.83 g, yield 75%).
¹H-NMR (300 MHz, CDC₃)δ:1.56 (d, J = 6.9 Hz, 3 H), 2.40 (s, 3 H), 3.72 (s, 3 H), 3.77 (s, 3 H), 4.63 (q, J = 6.9 Hz, 1 H), 5.20 (dd, J = 10.2, 1.5 Hz, 1 H), 5.57 (dd, J = 18.0, 1.5 Hz, 1 H), 6.43 - 6.44 (m, 1 H), 6.58 - 6.68 (m, 3 H), 7.10 - 7.15 (m, 1 H).

### Example 112 (2S)-2-{3-[(1,3-dimethyl-4-vinyl-1H-pyrazol-5-yl)thio]phenoxy}propionic acid

Methyl (2S)-2-{3-[(1,3-dimethyl-4-vinyl-1H-pyrazol-5-yl)thio]phenoxy}propionate (0.42 g) obtained in Example 111 was dissolved in a mixed solvent of tetrahydrofuran (15 mL) and water (5 mL), and the solution was cooled to 0°C. Lithium hydroxide monohydrate (0.42 g) was added, and the mixture was stirred at 0°C for 2 hr. To the reaction mixture were added water (20 mL) and ethyl acetate (50 mL), and the mixture was vigorously stirred for 5 min. Two layers were separated, and the aqueous layer was neutralized with 1N hydrochloric acid (2 mL), and extracted with ethyl acetate. The organic layer was washed with saturated brine, dried over anhydrous magnesium sulfate and filtered. The filtrate was concentrated, and the residue was crystallized from hexane-ethyl acetate to give the title compound as colorless crystals (0.552 g, yield 70%).
¹H-NMR (300 MHz, CDCl₃)δ:1.60 (d, J = 6.9 Hz, 3 H), 2.39 (s, 3 H), 3.70 (s, 3 H), 4.56 (q, J = 6.9 Hz, 1 H), 5.23 (dd, J = 11.7, 1.2 Hz, 1 H), 5.59 (dd, J = 18.3, 1.2 Hz, 1 H), 6.24 - 6.26 (m, 1 H), 6.58 - 6.73 (m, 3 H), 7.15 (t, J = 7.8 Hz, 1 H), 7.97 (br, 1 H).

### Example 113 3-(4-chloro-3-{[1-methyl-3-(trifluoromethyl)-1H-pyrazol-5-yl]oxy}phenyl)-1,2,4-oxadiazol-5(4H)-one

While stirring a solution of 1-methyl-3-(trifluoromethyl)-1H-pyrazol-5-ol (4.00 g, 24.1 mmol) cooled to 0°C in an ice bath and 4-chloro-3-fluorobenzonitrile (3.73 g, 24.0 mmol) in 1-methylpyrrolidin-2-one (15 mL), sodium hydride (0.96 g, 24.0 mmol) was added by small portions. The ice bath was removed, and the reaction mixture was stirred at 170°C for 6 hr. Water was added to the reaction mixture, and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 95: 5 - 88:12, v/v) to give a colorless oil. The obtained colorless oil was added to a solution of hydroxylamine hydrochloride (2.80 g, 40.0 mmol) and sodium hydrogen carbonate (3.36 g, 40.0 mmol) in dimethyl sulfoxide (15 mL) at 40°C and the mixture was stirred for 30 min and at 90°C for 6 hr. Water was added to the reaction mixture, and the mixture was extracted with ethyl acetate. The organic layer was washed with saturated brine, dried over anhydrous magnesium sulfate and filtered, and the filtrate was concentrated. The obtained residue was added to a solution of 1,1'-carbonylbis-1H-imidazole (1.08.g, 6.63 mmol) and 1,8-diazabicyclo[5.4.0]undec-7-ene (0.99 mL, 6.63 mmol) in dichloromethane (20 mL), and the mixture was stirred at room temperature for 2 hr. Water was added to the reaction mixture, and the mixture was extracted with ethyl acetate. The organic layer was washed with saturated brine, dried over anhydrous magnesium sulfate and filtered. The filtrate was concentrated, and the residue was recrystallized from ethyl acetate-hexane to give the title compound (0.433 g, yield 6%) as a colorless solid.
¹H-NMR (300 MHz, CDCl₃)δ:3.88 (s, 3 H), 5.93 (s, 1 H), 7.36 (t, J = 8.1 Hz, 1 H), 7.61 (d, J = 8.7 Hz, 1 H), 7.75 (d, J = 10.2 Hz, 1 H), 11.53 (br, 1 H).

### Example 114 methyl (2S)-2-{2,4-dichloro-5-[(1,3,4-trimethyl-1H-pyrazol-5-yl)thio]phenoxy}propionate

The title compound was obtained in the same manner as in Example 111 and from 2,4-dichloro-5-[(1,3,4-trimethyl-1H-pyrazol-5-yl)thio]phenol obtained in Reference Example 94. ¹H-NMR (300 MHz, CDCl₃)δ:1.57 (d, J = 6.9 Hz, 3 H), 1.99 (s, 3 H), 2.28 (s, 3 H), 3.68 (s, 3 H), 3.74 (s, 3 H), 4.33 (q, J = 6.9 Hz, 1 H), 5.76 (s, 1 H), 7.36 (s, 1 H).

### Example 115 (2S)-2-{2,4-dichloro-5-[(1,3,4-trimethyl-1H-pyrazol-5-yl)thio]phenoxy}propionic acid

The title compound was obtained in the same manner as in Example 112 and from methyl (2S)-2-{2,4-dichloro-5-[(1,3,4-trimethyl-1H-pyrazol-5-yl)thio]phenoxy}propionate obtained in Example 114.
¹H-NMR (300 MHz, CDCl₃)δ:1.62 (d, J = 6.9 Hz, 3 H), 2.04 (s, 3 H), 2.28 (s, 3 H), 3.79 (s, 3 H), 4.27 (q, J = 6.9 Hz, 1 H), 5.72 (s, 1 H), 7.36 (s, 1 H), 7.52 (br, 1 H).

### Example 116 methyl (2S)-2-{2,4-dichloro-5-[(4-formyl-1,3-dimethyl-1H-pyrazol-5-yl)(methyl)amino]phenoxy}propionate

5-[(2,4-Dichloro-5-hydroxyphenyl)(methyl)amino]-1,3-dimethyl-1H-pyrazole-4-carbaldehyde (0.8 g) obtained in Reference Example 95 and potassium carbonate (2.9 g) were charged, dimethyl sulfoxide (5 mL) was added and the mixture was stirred for 10 min. The reaction system was deaerated, and purged with nitrogen, and a solution of methyl (2R)-2-{[(4-methylphenyl)sulfonyl]oxy}propionate (0.8 g) obtained in Reference Example 96 in dimethyl sulfoxide (5 mL) was added carefully so that the temperature of the reaction system would not exceed 30°C. The mixture was stirred at room temperature for 4 hr, ice-cooled water was added to the reaction mixture, and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 90:10 - 60:40, v/v). The solvent was evaporated to give a colorless oil (0.92 g, yield 92%).
¹H-NMR (300 MHz, CDCl₃)δ:1.66 (d, J = 6.9 Hz, 3 H), 2.44 (s, 3 H), 3.32 (s, 3 H), 3.53 (s, 3 H), 3.72 (s, 3 H), 4.68 (q, J = 6.9 Hz, 1 H), 6.61 (s, 1 H), 7.34 (s, 1 H), 9.69 (s, 1 H).

### Example 117 (2S)-2-{2,4-dichloro-5-[(4-formyl-1,3-dimethyl-1H-pyrazol-5-yl)(methyl)amino]phenoxy}propionic acid

The title compound was obtained in the same manner as in, Example 112 and from methyl (2S)-2-{2,4-dichloro-5-[(4-formyl-1,3-dimethyl₋1H-pyrazol-5-yl)(methyl)amino]phenoxy}propionate obtained in Example 116.
¹H-NMR (300 MHz, CDCl₃)δ:1.68 (d, J = 6.9 Hz, 3 H), 2.43 (s, 3 H), 3.35 (s, 3 H), 3.44 (s, 3 H), 4.65 (q, J = 6.9 Hz, 1 H), 6.52 (s, 1 H), 7.36 (s, 1 H), 9.27 (br, 1 H), 9.74 (s, 1 H).

### Example 118 methyl (2S)-2-{2,4-dichloro-5-[(2,6-dimethyl-7-oxo-6,7-dihydro-2H-pyrazolo[3,4-d]pyridazin-3-yl)oxy]phenoxy}propionate

The title compound was obtained in the same manner as in Example 111 and from 3-(2,4-dichloro-5-hydroxyphenoxy)-2,6-dimethyl-2,6-dihydro-7H-pyrazolo[3,4-d]pyridazin-7-one obtained in Reference Example 99 and methyl (R)-(+)-lactate. ¹H-NMR (300 MHz, DMSO-d₆)δ:1.51 (d, J = 6.8 Hz, 3 H), 3.57 (s, 3 H), 3.60 (s, 3 H), 4.00 (s, 3 H), 5.01 - 5.25 (m, 1 H), 7.19 (s, 1 H), 7.32 (s, 1 H), 7.92 (s, 1 H).

### Example 119 (2S)-2-{2,4-dichloro-5-[(2,6-dimethyl-7-oxo-6,7-dihydro-2H-pyrazolo[3,4-d]pyridazin-3-yl)oxy]phenoxy}propionic acid

The title compound was obtained in the same manner as in Example 112 and from methyl (2S)-2-{2,4-dichloro-5-[(2,6-dimethyl-7-oxo-6,7-dihydro-2H-pyrazolo[3,4-d]pyridazin-3-yl)oxy]phenoxy}propionate obtained in Example 118.
¹H-NMR (300 MHz, DMSO-d₆)δ:1.50 (d, J = 6.8 Hz, 3 H), 3.59 (s, 3 H), 4.00 (s, 3 H), 4.73 - 5.22 (m, 1 H), 7.11 (s, 1 H), 7.31 (s, 1 H), 7.90 (s, 1 H), 13.32 (br s, 1 H).

### Example 120 (2S)-2-[2,4-dichloro-5-({3-[(ethylamino)carbonyl]-1,4-dimethyl-1H-pyrazol-5-yl}oxy)phenoxy]propionic acid

The title compound was obtained in the same manner as in Example 1 and from 5-(2,4-dichloro-5-hydroxyphenoxy)-N-ethyl-1,4-dimethyl-1H-pyrazole-3-carboxamide obtained in Reference Example 106 and methyl (R)-(+)-lactate.
¹H-NMR (300 MHz, DMSO-d₆) δ: 1.08 (t, J = 7.2 Hz, 3 H), 1.47 (d, J = 6.8 Hz, 3 H), 1.93 (s, 3 H), 3.23 (m, 2 H), 3.64 (s, 3 H), 4.87 (q, J = 6.8 Hz, 1 H), 6.47 (s, 1 H), 7.79 (s, 1 H), 8.02 (t, J = 5.9 Hz, 1 H), 13.28 (br s, 1 H).

### Example 121 methyl (2S)-2-(2,4-dichloro-5-{[3-(ethoxycarbonyl)-1,4-dimethyl-1H-pyrazol-5-yl]oxy}phenoxy)propionate

The title compound was obtained in the same manner as in Example 111 and from ethyl 5-(2,4-dichloro-5-hydroxyphenoxy)-1,4-dimethyl-1H-pyrazole-3-carboxylate obtained in Reference Example 104 and methyl (R)-(+)-lactate.
¹H-NMR (300 MHz, DMSO-d₆)δ:1.23 - 1.35 (m, 3 H), 1.47 (d, J = 6.8 Hz, 3 H), 1.88 (s, 3 H), 3.50 (s, 3 H), 3.69 (s, 3 H), 4.28 (q, J = 7.1 Hz, 2 H), 4.82 - 5.24 (m, 1 H), 6.38 (s, 1 H), 7.81 (s, 1 H).

### Example 122 methyl (2S)-2-[2,4-dichloro-5-({4-[(methoxyimino)methyl]-1,3-dimethyl-1H-pyrazol-5-yl}oxy)phenoxy]propionate

The title compound was obtained in the same manner as in Example 103 and using methyl (2S)-2-{2,4-dichloro-5-[(4-formyl-1,3-dimethyl-1H-pyrazol-5-yl)oxy]phenoxy}propionate obtained in Example 102 and O-methylhydroxylamine hydrochloride.
¹H-NMR (300 MHz, DMSO-d₆)δ:1.47 (d, J = 6.6 Hz, 3 H), 2.24 (s, 3 H), 3.58 (s, 6 H), 3.60 (s, 3 H), 5.00 (q, J = 6.6 Hz, 1 H), 6.45 (s, 1 H), 7.67 (s, 1 H), 7.78 (s, 1 H).

### Example 123 (2S)-2-[2,4-dichloro-5-({4-[(methoxyimino)methyl]-1,3-dimethyl-1H-pyrazol-5-yl}oxy)phenoxy]propionic acid

The title compound was obtained in the same manner as in Example 112 and using methyl (2S)-2-[2,4-dichloro-5-({4-[(methoxyimino)methyl]-1,3-dimethyl-1H-pyrazol-5-yl}oxy)phenoxy]propionate obtained in Example 122.
¹H-NMR (300 MHz, DMSO-d₆)δ:1.45 (d, J = 6.6 Hz, 3 H), 2.21 (s, 3 H), 3.55 (s, 3 H), 3.60 (s, 3 H), 4.84 (q, J = 6.7 Hz, 1 H), 6.51 (s, 1 H), 7.66 (s, 1 H), 7.76 (s, 1 H), 13.21 (br s, 1 H).

### Example 124 methyl (2S)-2-{5-[(4-{[(benzyloxy)imino]methyl}-1,3-dimethyl-1H-pyrazol-5-yl)oxy]-2,4-dichlorophenoxy}propionate

The title compound was obtained in the same manner as in Example 103 and using methyl (2S)-2-{2,4-dichloro-5-[(4-formyl-1,3-dimethyl-1H-pyrazol-5-yl)oxy]phenoxy}propionate obtained in Example 102 and O-benzylhydroxylamine hydrochloride.
¹H-NMR (300 MHz, DMSO-d₆)δ:1.45 (d, J = 6.8 Hz, 3 H), 2.22 (s, 3 H), 3.50 (s, 3 H), 3.56 (s, 3 H), 4.85 (d, J = 2.5 Hz, 2 H), 4.86 - 4.96 (m, 1 H), 6.37 (s, 1 H), 7.00 - 7.42 (m, 5 H), 7.57 - 7.89 (m, 2 H).

### Example 125 (2S)-2-{5-[(4-{[(benzyloxy)imino]methyl}-1,3-dimethyl-1H-pyrazol-5-yl)oxy]-2,4-dichlorophenoxy}propionic acid

The title compound was obtained in the same manner as in Example 112 and using methyl (2S)-2-{5-[(4-{[(benzyloxy)imino]methyl}-1,3-dimethyl-1H-pyrazol-5-yl)oxy]-2,4-dichlorophenoxy}propionate obtained in Example 124.
¹H-NMR (300 MHz, DMSO-d₆)δ:1.43 (d, J = 6.8 Hz, 3 H), 2.22 (s, 3 H), 3.53 (s, 3 H), 4. 64 - 4.79 (m, 1 H), 4.87 (s, 2 H), 6.44 (s, 1 H), 6.96 - 7.39 (m, 5 H), 7.65 - 7.88 (m, 2 H), 13.21 (br s, 1 H).

### Example 126 methyl (2S)-2-{2,4-dichloro-5-[{4-[(hydroxyimino)methyl]-1,3-dimethyl-1H-pyrazol-5-yl}(methyl)amino]phenoxy}propionate

The title compound was obtained in the same manner as in Example 103 and using methyl (2S)-2-{2,4-dichloro-5-[(4-formyl-1,3-dimethyl-1H-pyrazol-5-yl)(methyl)amino]phenoxy}propionate obtained in Example 116 and hydroxyamine monohydrate.
¹H-NMR (300 MHz, DMSO-d₆)δ:1.41 - 1.66 (m, 3 H), 2.03 - 2.28 (m, 3 H), 3.08 - 3.21 (m, 3 H), 3.41 - 3.62 (m, 3 H), 3.64 - 3.76 (m, 3 H), 5.17 - 5.33 (m, 1 H), 6.74 - 7.01 (m, 1 H), 7.37 - 7.58 (m, 2 H), 10.05 - 10.32 (m, 1 H).

### Example 127 (2S)-2-{2,4-dichloro-5-[{4-[(hydroxyimino)methyl]-1,3-dimethyl-1H-pyrazol-5-yl}(methyl)amino]phenoxy}propionic acid

The title compound was obtained in the same manner as in Example 112 and using methyl (2S)-2-{2,4-dichloro-5-[{4-[(hydroxylmino)methyl]-1,3-dimethyl-1H-pyrazol-5-yl}(methyl)amino]phenoxy}propionate obtained in Example 126.
¹H-NMR (300 MHz, DMSO-d₆)δ:1.47 - 1.63 (m, 3 H), 2. 07 - 2.34 (m, 3 H), 3.12 - 3.30 (m, 3 H), 3.49 - 3.63 (m, 3 H), 5.03 - 5.23 (m, 1 H), 6. 92 - 7.14 (m, 1 H), 7.33 - 7.54 (m, 2 H), 9.28 - 9.57 (m, 1 H).

### Example 128 3'-chloro-4'-[(1,3,4-trimethyl-1H-pyrazol-5-yl)oxy]biphenyl-2-carboxylic acid

A solution of 5-(4-bromo-2-chlorophenoxy)-1,3,4-trimethyl-1H-pyrazole (450 mg) obtained in Reference Example 101, 2-(carboxyphenyl)boric acid (355 mg), tetrakis(triphenylphosphine)palladium(0) (82 mg) and aqueous sodium carbonate solution (2 mol/L, 2.1 mL) in 1,2-dimethoxyethane (40 mL) was stirred under an argon atmosphere at 120°C for 19 hr. The reaction mixture was cooled to room temperature, dil. hydrochloric acid was added to the reaction mixture, and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 50:50 - 0:100, v/v) to give the title compound as a colorless amorphous compound (49 mg, yield 10%).
¹H-NMR (300 MHz, DMSO-d₆)δ:1.69 (s, 3 H), 2.08 (s, 3 H), 3.53 (s, 3 H), 6.79 (d, J = 8.3 Hz, 1 H), 7.11 - 7.30 (m, 1 H), 7.31 - 7.62 (m, 4 H), 7.73 (d, J = 8.0 Hz, 1 H).

### Example 129 (2S)-2-{4-chloro-2-methyl-5-[(1,3,4-trimethyl-1H-pyrazol-5-yl)oxy]phenoxy}propionic acid

The title compound was obtained in the same manner as in Example 116 and then Example 112, and from 4-chloro-2-methyl-5-[(1,3,4-trimethyl-1H-pyrazol-5-yl)oxy]phenol obtained in Reference Example 113 and methyl (2R)-2-{[(4-methylphenyl)sulfonyl]oxy}propionate obtained in Reference Example 96.
¹H-NMR (300 MHz, CDCl₃)δ:1.62 (d, J = 6.8 Hz, 3 H), 1.83 (s, 3 H), 2.17 (s, 3 H), 2.20 (s, 3 H), 3.52 (s, 3 H), 4.36 (q, J = 6.8 Hz, 1 H), 6.00 (s, 1 H), 7.19 (s, 1 H).

### Example 130 methyl (2S)-2-{4-chloro-2-formyl-5-[(1,3,4-trimethyl-1H-pyrazol-5-yl)oxy]phenoxy}propionate

The title compound was obtained in the same manner as in Example 116 and from 5-chloro-2-hydroxy-4-[(1,3,4-trimethyl-1H-pyrazol-5-yl)oxy]benzaldehyde obtained in Reference Example 119 and methyl (2R)-2-{[(4-methylphenyl)sulfonyl]oxy}propionate obtained in Reference Example 96.
¹H-NMR (300 MHz, CDCl₃)δ:1.63 (d, J = 6.8 Hz, 3 H), 1.77 (s, 3 H), 2.21 (s, 3 H), 3.56 (s, 3 H), 3.68 (s, 3 H), 4.59 (q, J = 6.8 Hz, 1 H), 6.08 (s, 1 H), 7.96 (s, 1 H), 10.37 (s, 1 H).

### Example 131 methyl (2S)-2-{4-chloro-2-[(E)-(hydroxyimino)methyl]-5-[(1,3,4-trimethyl-1H-pyrazol-5-yl)oxy]phenoxy}propionate

A mixture of methyl (2S)-2-{4-chloro-2-formyl-5-[(1,3,4-trimethyl-1H-pyrazol-5-yl)oxy]phenoxy}propionate (610 mg) obtained in Example 130, hydroxylamine hydrochloride (139 mg), sodium acetate (204 mg), and methanol (15 mL) was heated under reflux for 13 hr, and concentrated under reduced pressure. Water and ethyl acetate were added to the residue and the mixture was stirred until complete dissolution. Two layers were separated, and the organic layer was washed with saturated brine, and dried over anhydrous magnesium sulfate. After filtration, the filtrate was concentrated and the residue was crystallized from toluene to give the title compound (518 mg, yield 82%) as colorless crystals.
¹H-NMR (300 MHz, CDCl₃)δ:1.58 (d, J = 6.8 Hz, 3 H), 1.76 (s, 3 H), 2.20 (s, 3 H), 3.57 (s, 3 H), 3.66 (s, 3 H), 4.51 (q, J = 6.8 Hz, 1 H), 6.06 (s, 1 H), 7.64 (br s, 1 H), 7.88 (s, 1 H), 8.44 (s, 1 H).

### Example 132 (2S)-2-{4-chloro-2-[(E)-(methoxyimino)methyl]-5-[(1,3,4-trimethyl-1H-pyrazol-5-yl)oxy]phenoxy}propionic acid

The title compound was obtained in the same manner as in Example 131 and then Example 112, and from methyl (2S)-2-{4-chloro-2-formyl-5-[(1,3,4-trimethyl-1H-pyrazol-5-yl)oxy]phenoxy}propionate obtained in Example 130 and O-methylhydroxylamine hydrochloride.
¹H-NMR (300 MHz, CDCl₃)δ:1.61 (d, J = 6.8 Hz, 3 H), 1.84 (s, 3 H), 2.18 (s, 3 H), 3.54 (s, 3 H), 3.97 (s, 3 H), 4.42 (q, J = 6.7 Hz, 1 H), 6.05 (s, 1 H), 7.83 (s, 1 H), 8.31 (s, 1 H).

### Example 133 (2S)-2-{4-chloro-2-cyano-5-[(1,3,4-trimethyl-1H-pyrazol-5-yl)oxy]phenoxy}propionic acid

While stirring a solution, which was cooled to 0°C in an ice bath, of methyl (2S)-2-{4-chloro-2-[(E)-(hydroxyimino)methyl]-5-[(1,3,4-trimethyl-1H-pyrazol-5-yl)oxy]phenoxy}propionate (250 mg) obtained in Example 131 and triethylamine (0.190 mL) in tetrahydrofuran (5 mL), trichloroacetyl chloride (0.114 mL) was added dropwise. The mixture was stirred at room temperature for 24 hr, water was added, and the mixture was extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 80:20 - 40:60, v/v). The obtained colorless oil was dissolved in a mixed solvent of tetrahydrofuran (7 mL) and water (3 mL), and cooled to 0°C. Lithium hydroxide monohydrate (57 mg) was added, and the mixture was stirred at 0°C for 90 min. The reaction mixture was neutralized with 0.1N hydrochloric acid (14 mL), and extracted with ethyl acetate. The organic layer was washed with saturated brine, dried over anhydrous magnesium sulfate and filtered. The filtrate was concentrated, and the residue was crystallized from ethyl acetate to give the title compound as colorless crystals (91 mg, yield 38%).
¹H-NMR (300 MHz, CDCl₃)δ:1.70 (d, J = 6.8 Hz, 3 H), 1.86 (s, 3 H), 2.19 (s, 3 H), 3.53 (s, 3 H), 4.46 (q, J = 6.8 Hz, 1 H), 6.04 (s, 1 H), 7.66 (s, 1 H).

### Example 134 (2S)-2-{4-chloro-2-[(E)-(hydroxyimino)methyl]-5-[(1,3,4-trimethyl-1H-pyrazol-5-yl)oxy]phenoxy}propionic acid

The title compound was obtained in the same manner as in Example 112 and from methyl (2S)-2-{4-chloro-2-[(E)-(hydroxyimino)methyl]-5-[(1,3,4-trimethyl-1H-pyrazol-5-yl)oxy]phenoxy}propionate obtained in Example 131.
¹H-NMR (300 MHz, DMSO-d₆)δ:1.46 (d, J = 6.8 Hz, 3 H), 1.64 (s, 3 H), 2.07 (s, 3 H), 3.49 (s, 3 H), 4.76 (q, J = 6.7 Hz, 1 H), 6.32 (s, 1 H), 7.75 (s, 1 H), 8.22 (s, 1 H), 11.40 (s, 1 H), 13.20 (s, 1 H).

### Example 135 (2S)-2-{2-(carbamoyl)-4-chloro-5-[(1,3,4-trimethyl-1H-pyrazol-5-yl)oxy]phenoxy}propionic acid

The title compound was obtained in the same manner as in Example 116 and then Example 112, and from 5-chloro-2-hydroxy-4-[(1,3,4-trimethyl-1H-pyrazol-5-yl)oxy]benzamide obtained in Reference Example 121 and methyl (2R)-2-{[(4-methylphenyl)sulfonyl]oxy}propionate obtained in Reference Example 96.
¹H-NMR (300 MHz, CDCl₃)δ:1.60 (d, J = 6.8 Hz, 3 H), 1.81 (s, 3 H), 2.24 (s, 3 H), 3.61 (s, 3 H), 4.74 (q, J = 6.8 Hz, 1 H), 6.20 (s, 1 H), 8.30 (s, 1 H), 8.38 (s, 1 H), 9.21 (s, 1 H).

### Example 136 (2S)-2-{4-chloro-2-[dimethylcarbamoyl]-5-[(1,3,4-trimethyl-1H-pyrazol-5-yl)oxy]phenoxy}propionic acid

The title compound was obtained in the same manner as in Example 116 and then Example 112, and from 5-chloro-2-hydroxy-N,N-dimethyl-4-[(1,3,4-trimethyl-1H-pyrazol-5-yl)oxy]benzamide obtained in Reference Example 122 and methyl (2R)-2-{[(4-methylphenyl)sulfonyl]oxy}propionate obtained in Reference Example 96.
¹H-NMR (300 MHz, CDCl₃)δ:1.59 (d, J = 6.8 Hz, 3 H), 1.78 (s, 3 H), 2.19 (s, 3 H), 3.05 (s, 3 H), 3.14 (s, 3 H), 3.57 (s, 3 H), 4.58 (q, J = 6.6 Hz, 1 H), 6.25 (s, 1 H), 7.37 (s, 1 H).

### Example 137 (2S)-2-{4-chloro-2-(1-hydroxy-1-methylethyl)-5-[(1,3,4-trimethyl-1H-pyrazol-5-yl)oxy]phenoxy}propionic acid

To a solution of 5-chloro-2-hydroxy-4-[(1,3,4-trimethyl-1H-pyrazol-5-yl)oxy]benzoic acid (400 mg) obtained in Reference Example 120 in tetrahydrofuran (10 mL) was added methylmagnesium bromide (8.1 mL), and the mixture was stirred at room temperature for 48 hr. To the reaction mixture was added methylmagnesium bromide (8.1 mL), and the mixture was stirred at 60°C for 72 hr, and cooled to 0°C, after which acidified by gradually adding dil. hydrochloric acid. The mixture was extracted with ethyl acetate, and the organic layer was washed with saturated brine, and dried over anhydrous magnesium sulfate. After filtration, the filtrate was concentrated and the residue was subjected to silica gel column chromatography (hexane-ethyl acetate 80:20 - 40:60, v/v). The obtained pale-brown oil was dissolved in dimethyl sulfoxide (5 mL), and potassium carbonate (490 mg) and methyl (2R)-2-{[(4-methylphenyl)sulfonyl]oxy}propionate (458 mg) obtained in Reference Example 96 were added. This mixture was stirred at room temperature for 15 hr, ice-cooled water was added, and the mixture was extracted with ethyl acetate. The organic layer was washed with saturated brine, dried over anhydrous magnesium sulfate and filtered. The filtrate was concentrated, the residue was subjected to silica gel column chromatography (hexane-ethyl acetate 82:18 - 60:40, v/v), and the solvent was evaporated to give a colorless oil. The obtained oil was dissolved in a mixed solvent of tetrahydrofuran (7 mL) and water (3 mL), and the solution was cooled to 0°C. Lithium hydroxide monohydrate (74 mg) was added, and the mixture was stirred at 0°C for 90 min. The reaction mixture was neutralized with 0.1N hydrochloric acid (18 mL), and extracted with ethyl acetate. The organic layer was washed with saturated brine, dried over anhydrous magnesium sulfate and filtered. The filtrate was concentrated, and the residue was crystallized from hexane-ethyl acetate to give the title compound as colorless crystals (157 mg, yield 46%).
¹H-NMR (300 MHz, CDCl₃)δ:1.63 (s, 3 H), 1.64 (s, 6 H), 1.82 (s, 3 H), 2.18 (s, 3 H), 3.52 (s, 3 H), 4.51 (q, J = 6.8 Hz, 1 H), 6.06 (s, 1 H), 7.44 (s, 1 H).

### Example 138 (2S)-2-{2,4-dichloro-5-[(2-methyl-4,5,6,7-tetrahydro-2H-indazol-3-yl)methyllphenoxy}propionic acid

The title compound was obtained in the same manner as in Example 116 and then Example 112, and from 2,4-dichloro-5-[(2-methyl-4,5,6,7-tetrahydro-2H-indazol-3-yl)methyl]phenol obtained in Reference Example 129 and methyl (2R)-2-{[(4-methylphenyl)sulfonyl]oxy}propionate obtained in Reference Example 96.
¹H-NMR (300 MHz, DMSO-d₆)δ:1.50 (d, J = 6.8 Hz, 3 H), 1.54 - 1.72 (m, 4 H), 2.10 (t, J = 5.8 Hz, 2 H), 2.41 - 2.49 (m, 2 H), 3.59 (s, 3 H), 3.94 (s, 2 H), 4.76 (q, J = 6.8 Hz, 1 H), 6.69 (s, 1 H), 7.60 (s, 1 H), 13.15 (s, 1 H).

### Example 139 methyl (2R)-2-{2,4-dichloro-5-[(4-formyl-1,3-dimethyl-1H-pyrazol-5-yl)thio]phenoxy}propionate

The title compound was obtained in the same manner as in Example 40 and from 5-[(2,4-dichloro-5-hydroxyphenyl)thio]-1,3-dimethyl-1H-pyrazole-4-carbaldehyde obtained in Reference Example 130 and methyl (S)-(-)-lactate.
¹H-NMR (300 MHz, CDCl₃) δ:1.59 (d, J = 6.8 Hz, 3 H), 2.56 (s, 3 H), 3.67 (s, 3 H), 3.80 (s, 3 H), 4.38 (q, J = 6.8 Hz, 1 H), 5.95 (s, 1 H), 7.43 (s, 1 H), 9.94 (s, 1 H).

### Example 140 methyl (2R)-2-[2,4-dichloro-5-({4-[(E)-(hydroxyimino)methyl]-1,3-dimethyl-1H-pyrazol-5-yl}thio)phenoxy]propionate

A mixture of methyl (2R)-2-{2,4-dichloro-5-[(4-formyl-1,3-dimethyl-1H-pyrazol-5-yl)thio]phenoxy}propionate (225 mg) obtained in Example 139, hydroxylamine hydrochloride (47 mg), sodium acetate (69 mg), water (1 mL), and methanol (9 mL) was stirred at 50°C for 90 min and concentrated under reduced pressure. Water and diisopropyl ether were added to the residue, and the mixture was stirred until complete dissolution. Two layers were separated, and the organic layer was washed with saturated brine, and dried over anhydrous magnesium sulfate. After filtration, the filtrate was concentrated and the residue was crystallized from hexane-diisopropyl ether to give the title compound (190 mg, yield 82%) as colorless crystals.
¹H-NMR (300 MHz, CDCl₃)δ:1.58 (d, J = 6.8 Hz, 3 H), 2.47 (s, 3 H), 3.68 (s, 3 H), 3.79 (s, 3 H), 4.37 (q, J = 6.8 Hz, 1 H), 5.86 (s, 1 H), 7.32 (s, 1 H), 7.39 (s, 1 H), 8.10 (s, 1 H).

### Example 141 (2R)-2-{2,4-dichloro-5-[(4-cyano-1,3-dimethyl-1H-pyrazol-5-yl)thio]phenoxy}propionic acid

While stirring a solution, which was cooled to 0°C in an ice bath, of methyl (2R)-2-[2,4-dichloro-5-({4-[(E)-(hydroxyimino)methyl]-1,3-dimethyl-1H-pyrazol-5-yl}thio)phenoxy]propionate (100 mg) obtained in Example 140 and triethylamine (0.067 mL) in tetrahydrofuran (5 mL), trichloroacetyl chloride (0.040 mL) was added dropwise. The mixture was stirred at 60°C for 3 hr, water was added, and the mixture was extracted with diisopropyl ether. The organic layer was washed with saturated brine, and dried over anhydrous magnesium sulfate. After filtration, the filtrate was concentrated and the residue was subjected to silica gel column chromatography (hexane-ethyl acetate 90:10 - 75:25, v/v). The obtained colorless oil was dissolved in a mixed solvent of tetrahydrofuran (3.5 mL) and water (1.5 mL), and the solution was cooled to 0°C. Lithium hydroxide monohydrate (23 mg) was added, and the mixture was stirred at 0°C for 2 hr. The reaction mixture was neutralized with 0.1N hydrochloric acid (6 mL), and extracted with ethyl acetate. The organic layer was washed with saturated brine, dried over anhydrous magnesium sulfate and filtered. The filtrate was concentrated, and the residue was crystallized from hexane-ethyl acetate to give the title compound as colorless crystals (25 mg, yield 27% .
¹H-NMR (300 MHz, CDCl₃)δ:1.68 (d, J = 6.8 Hz, 3 H), 2.39 (s, 3 H), 3.84 (s, 3 H), 4.60 (q, J = 6. 5 Hz, 1 H), 6.39 (s, 1 H), 7.46 (s, 1 H).

### Example 142 5-{4-chloro-3-[(1,3,4-trimethyl-1H-pyrazol-5-yl)oxy]phenyl}-1H-tetrazole

A solution (5 mL) of 4-chloro-3-[(1,3,4-trimethyl-1H-pyrazol-5-yl)oxy]benzonitrile (504 mg) obtained in Reference Example 133, sodium azide (313 mg) and ammonium chloride (258 mg) in N,N-dimethylformamide was stirred at 130°C for 4 hr. 1N Hydrochloric acid was added to the reaction mixture, and the mixture was extracted with ethyl acetate. The organic layer was washed with saturated brine, dried over anhydrous magnesium sulfate and filtered. The filtrate was concentrated, and the residue was recrystallized from ethyl acetate to give the title compound as pale-yellow crystals (514 mg, yield 88%).
¹H-NMR (300 MHz, DMSO-d₆)δ:1.70 (s, 3 H), 2.12 (s, 3 H), 3.55 (s, 3 H), 7.41 (d, J = 1.5 Hz, 1 H), 7.80 - 7.90 (m, 2 H).

### Example 143 5-{3-chloro-4-[(1,3,4-trimethyl-1H-pyrazol-5-yl)oxy]phenyl}-1H-tetrazole

The title compound was obtained in the same manner as in Example 142 and from 3-chloro-4-[(1,3,4-trimethyl-1H-pyrazol-5-yl)oxy]benzonitrile obtained in Reference Example 139.
¹H-NMR (300 MHz, DMSO-d₆)δ:1.71 (s, 3 H), 2.10 (s, 3 H), 3.53 (s, 3 H), 7.00 (d, J = 8.7 Hz, 1 H), 7.99 (dd, J = 8.7, 2.1 Hz, 1 H), 8.24 (d, J = 2.1 Hz, 1 H).

### Example 144 (2S)-2-{2,4-dichloro-5-[(2-methyl-2,4,5,6-tetrahydrocyclopenta[c]pyrazol-3-yl)methyl]phenoxy}propionic acid

The title compound was obtained in the same manner as in Example 116 and then Example 112, and from 2,4-dichloro-5-[(2-methyl-2,4,5,6-tetrahydrocyclopenta[c]pyrazol-3-yl)methyl]phenol obtained in Reference Example 199 and methyl (2R)-2-{[(4-methylphenyl)sulfonyl]oxy}propionate obtained in Reference Example 96.
¹H-NMR (300 MHz, DMSO-d₆)δ:1.53 (d, J = 6.8 Hz, 3 H), 1.86 - 1.98 (m, 2 H), 2.08 - 2.19 (m, 2 H), 2.41 - 2.48 (m, 2 H), 3.68 (s, 3 H), 3.95 (s, 2 H), 4.89 (q, J = 6.8 Hz, 1 H), 6.99 (s, 1 H), 7.60 (s, 1 H), 13.16 (s, 1 H).

### Example 145 methyl 2-bromo-3-12,4-dichloro-5-[(1,3,4-trimethyl-1H-pyrazol-5-yl)oxy]phenyl}propionate

To a solution of 2,4-dichloro-5-[(1,3,4-trimethyl-1H-pyrazol-5-yl)oxy]aniline (1.0 g) obtained in Reference Example 143 in methanol (9.6 mL)-acetone (2.4 mL) was added an aqueous solution (1 mL) of sodium nitrite (266 mg) at 0°C. 47% Hydrobromic acid (1.6 mL) was added to the reaction mixture and the mixture was stirred for 20 min. Methyl acrylate (1.8 mL) was added, and the mixture was heated to 40°C. Copper oxide (80 mg) was added and the mixture was vigorously stirred for 20 min. The reaction mixture was concentrated, water was added and the mixture was alkalified with aqueous ammonia solution and extracted with ethyl acetate. The organic layer was washed with water and saturated brine, dried over anhydrous magnesium sulfate and filtered. The filtrate was concentrated, and the residue was subjected to silica gel column chromatography (hexane-ethyl acetate 9:1 - 4:1, v/v) to give the title compound as a colorless oil (419 mg, yield 27%).
¹H-NMR (300 MHz, CDCl₃)δ:1.75 (s, 3 H), 2.20 (s, 3 H), 3.16 - 3.49 (m, 2 H), 3.57 (s, 3 H), 3.72 (s, 3 H), 4.47 (t, J = 7.6 Hz, 1H), 6.60 (s, 1 H), 7.48 (s, 1 H).

### Example 146 5-{2,4-dichloro-5-[(1,3,4-trimethyl-1H-pyrazol-5-yl)oxy]benzyl}-2-imino-1,3-thiazolidin-4-one

A solution (4 mL) of methyl 2-bromo-3-{2,4-dichloro-5-[(1,3,4-trimethyl-1H-pyrazol-5-yl)oxy]phenyl}propionate (304 mg) obtained in Example 145, thiourea (86 mg) and sodium acetate (90 mg) in ethanol was heated under reflux for 3 hr. The reaction mixture was filtered, and the obtained crystals were washed with ethanol to give the title compound as orange crystals (224 mg, yield 80%).
¹H-NMR (300 MHz, DMSO-d₆)δ:1.66 (s, 3 H), 2.08 (s, 3 H), 2.98 (dd, J = 14.0, 9.0 Hz, 1 H), 3.43 (dd, J = 14.0, 4.6 Hz, 1 H), 3.50 (s, 3 H), 4.52 (dd, J = 9.0, 4.6 Hz, 1 H), 6.77 (s, 1 H), 7.78 (s, 1 H), 8.79 (br s 1 H), 9.02 (br s, 1 H).

### Example 147 5-{2,4-dichloro-5-[(1,3,4-trimethyl-1H-pyrazol-5-yl)oxy]benzyll-1,3-thiazolidine-2,4-dione

A solution (8 mL) of 5-{2,4-dichloro-5-[(1,3,4-trimethyl-1H-pyrazol-5-yl)oxy]benzyl}-2-imino-1,3-thiazolidin-4-one (500 mg) obtained in Example 146 in 2N hydrochloric acid was heated under reflux for 6 hr. The reaction mixture was neutralized with saturated sodium hydrogen carbonate, and the mixture was extracted with ethyl acetate. The organic layer was washed with saturated brine, dried over anhydrous magnesium sulfate and filtered. The filtrate was concentrated, and the residue was recrystallized from ethyl acetate to give the title compound as colorless crystals (472 mg, yield 94%).
¹H-NMR (300 MHz, DMSO-d₆)δ:1.66 (s, 3 H), 2.08 (s, 3 H), 3.20 (dd, J = 14.0, 9.1 Hz, 1 H), 3.41 - 3.55 (m, 2 H), 4.81 (dd, J = 9.1, 5.3 Hz, 1 H), 6.81 (s, 1 H), 7.82 (s, 1 H), 12.13 (br s, 1 H).

### Example 148 5-{4-chloro-3-[(1,3,4-trimethyl-1H-pyrazol-5-yl)oxylbenzyll-1,3-thiazolidine-2,4-dione

To a solution of thiazolidinedione (351 mg) in tetrahydrofuran (10 mL) was added lithium diisopropylamide (1.8M tetrahydrofuran solution, 3.3 mL) under an argon stream at -20°C, and the mixture was stirred for 30 min. To the reaction mixture was added dropwise a solution of 5-[5-(bromomethyl)-2-chlorophenoxy]-1,3,4-trimethyl-1H-pyrazole (659 mg) obtained in Reference Example 145 in tetrahydrofuran (2 mL), and the mixture was stirred at -20°C for 2 hr, and at 0°C for 1.5 hr. Water was added to the reaction mixture, and the mixture was extracted with ethyl acetate. The organic layer was washed with water and saturated brine, dried over anhydrous magnesium sulfate and filtered. The filtrate was concentrated, and the residue was recrystallized from hexane-ethyl acetate to give the title compound as colorless crystals (511 mg, yield 70%).
¹H-NMR (300 MHz, DMSO-d₆)δ:1.64 (s, 3 H), 2.08 (s, 3 H), 3.04 - 3.39 (m, 2 H), 3.49 (s, 3 H), 4.86 (dd, J = 8.3, 4.9 Hz, 1 H), 6.68 (d, J = 1.9 Hz, 1 H), 7.03 (dd, J = 8.0, 1.9 Hz, 1 H), 7.53 (d, J = 8.0 Hz, 1 H), 12.01 (br s, 1 H).

### Example 149 3-{4-chloro-3-[(1,3,4-trimethyl-1H-pyrazol-5-yl)oxy]benzyl}-1,2,4-oxadiazol-5(4H)-one

To a solution (5 mL) of 2-{4-chloro-3-[(l,3,4-trimethyl-1H-pyrazol-5-yl)oxy]phenyl}-N'-hydroxyacetimidamide (380 mg) obtained in Reference Example 147 in tetrahydrofuran were added 1,1'-carbonylbis-1H-imidazole (399 mg) and 1,8-diazabicyclo[5.4.0]undec-7-ene (0.448 mL), and the mixture was stirred at room temperature for 2 hr. 1N Hydrochloric acid (3 mL) was added to the reaction mixture, and the mixture was extracted with ethyl acetate. The organic layer was washed with water and saturated brine, dried over anhydrous magnesium sulfate and filtered. The filtrate was concentrated, and the residue was purified by HPLC to give the title compound as a pale-yellow oil. Recrystallization from hexane-ethyl acetate gave pale-yellow crystals (217 mg, yield 57%).
¹H-NMR (300 MHz, CDCl₃)δ:1.73 (s, 3 H), 2.11 (s, 3 H), 3.51 (s, 3 H), 3.78 (s, 2 H), 6.63 (d, J = 1.9 Hz, 1 H), 6.99 (dd, J = 8.2, 1.9 Hz, 1 H), 7.45 (d, J = 8.2 Hz, 1 H).

### Example 150 3-((E)-2-{4-chloro-3-[(1,3,4-trimethyl-1H-pyrazol-5-yl)oxy]phenyl}vinyl)-1,2,4-oxadiazol-5(4H)-one

The title compound was obtained in the same manners as in Reference Example 147 and Example 149 and from (2E)-3-{4-chloro-3-[(1,3,4-trimethyl-1H-pyrazol-5-yl)oxylphenyllacrylonitrile obtained in Reference Example 148.
¹H-NMR (300 MHz, DMSO-d₆)δ:1.66 (s, 3 H), 2.09 (s, 3 H), 3.53 (s, 3 H), 6.77 (d, J = 16.3 Hz, 1 H), 6.91 (d, J = 1.9 Hz, 1H), 7.17 (d, J = 16.3 Hz, 1 H), 7.46 (dd, J = 8.3, 1.9 Hz, 1 H), 7.60 (d, J = 8.3 Hz, 1 H).

### Example 151 methyl (2S)-2-{4-chloro-2-(hydroxymethyl)-5-[(1,3,4-trimethyl-1H-pyrazol-5-yl)oxy]phenoxy}propionate

The title compound was obtained in the same manner as in Reference Example 100 and from methyl (2S)-2-{4-chloro-2-formyl-5-[(1,3,4-trimethyl-1H-pyrazol-5-yl)oxy]phenoxy}propionate obtained in Example 130.
¹H-NMR (300 MHz, CDCl₃)δ:1.58 (d, J = 6.8 Hz, 3 H), 1.73 (s, 3 H), 2.19 (s, 3 H), 2.80 (dd, J = 8.3, 4.7 Hz, 1 H), 3.57 (s, 3 H), 3.68 (s, 3 H), 4.50 (dd, J = 12.6, 8.3 Hz, 1 H), 4.60 (q, J = 6.8Hz, 1 H), 4.80 (dd, J = 12.6, 4.7 Hz, 1 H), 6.12 (s, 1 H), 7.41 (s, 1 H).

### Example 152 methyl (2S)-2-{2-{(E)-[(benzyloxy)imino]methyl}-4-chloro-5-[(1,3,4-trimethyl-1H-pyrazol-5-yl)oxy]phenoxy}propionate

A solution (5 mL) of methyl (2S)-2-{4-chloro-2-formyl-5-[(1,3,4-trimethyl-1H-pyrazol-5-yl)oxy]phenoxy}propionate (366 mg) obtained in Example 130 and O-benzylhydroxylamine hydrochloride (192 mg) in methanol was stirred at 50°C for 2 hr. The reaction mixture was concentrated, water was added to the residue, and the mixture was extracted with ethyl acetate. The organic layer was washed with water and saturated brine, dried over anhydrous magnesium sulfate and filtered. The filtrate was concentrated, and the residue was subjected to silica gel column chromatography (hexane-ethyl acetate 2:1, v/v) to give the title compound as a colorless oil (444 mg, yield 94%).
¹H-NMR (300 MHz, CDCl₃)δ:1.54 (d, J = 6.7 Hz, 3 H), 1.74 (s, 3 H), 2.19 (s, 3 H), 3.55 (s, 3 H), 3.64 (s, 3 H), 4.48 (q, J = 6.7 Hz, 1 H), 5.21 (s, 2 H), 6.03 (s, 1 H), 7.29 - 7.51 (m, 5 H), 7.92 (s, 1 H).

### Example 153 (2S)-2-{2-{(E)-[(benzyloxy)imino]methyl}-4-chloro-5-[(1,3,4-trimethyl-1H-pyrazol-5-yl)oxy]phenoxy}propionic acid

The title compound was obtained in the same manner as in Example 112 and from methyl (2S)-2-{2-{(E)-[(benzyloxy)imino]methyl}-4-chloro-5-[(1,3,4-trimethyl-1H-pyrazol-5-yl)oxy]phenoxy}propionate obtained in Example 152.
¹H-NMR (300 MHz, CDCl₃)δ:1.59 (d, J = 6.8 Hz, 3 H), 1.84 (s, 3 H), 2.17 (s, 3 H), 3.52 (s, 3 H), 4.38 (q, J = 6.8 Hz, 1 H), 5.20 (s, 2 H), 6.01 (s, 1 H), 7.27 - 7.46 (m, 5 H), 7.88 (s, 1 H), 8.43 (s, 1 H).

### Example 154 (2S)-2-{4-chloro-2-(hydroxymethyl)-5-[(1,3,4-trimethyl-1H-pyrazol-5-yl)oxy]phenoxy}propionic acid

The title compound was obtained in the same manner as in Example 112 and from methyl (2S)-2-{4-chloro-2-(hydroxymethyl)-5-[(1,3,4-trimethyl-1H-pyrazol-5-yl)oxy]phenoxy}propionate obtained in Example 151.
¹H-NMR (300 MHz, CDCl₃)δ:1.62 (d, J = 6.8 Hz, 3 H), 1.80 (s, 3 H), 2.16 (s, 3 H), 3.49 (s, 3 H), 4.44 - 4.81 (m, 3 H), 6.09 (s, 1 H), 7.39 (s, 1 H).

### Example 155 methyl (2S)-2-{4-chloro-2-[(E)-(ethoxyimino)methyl]-5-[(1,3,4-trimethyl-1H-pyrazol-5-yl)oxy]phenoxy}propionate

The title compound was obtained in the same manner as in Example 152 and from methyl (2S)-2-{4-chloro-2-formyl-5-[(1,3,4-trimethyl-1H-pyrazol-5-yl)oxy]phenoxy}propionate obtained in Example 130 and O-ethylhydroxylamine hydrochloride.
LC-MS; m/z 409 [M⁺]

### Example 156 (2S)-2-{4-chloro-2-[(E)-(ethoxyimino)methyl]-5-[(1,3,4-trimethyl-1H-pyrazol-5-yl)oxy]phenoxy}propionic acid

The title compound was obtained in the same manner as in Example 112 and from methyl (2S)-2-{4-chloro-2-[(E)-(ethoxyimino)methyl]-5-[(1,3,4-trimethyl-1H-pyrazol-5-yl)oxy]phenoxy}propionate obtained in Example 155.
¹H-NMR (300 MHz, CDCl₃)δ:1.32 (d, J = 7.2 Hz, 3 H), 1.61 (d, J = 6.8 Hz, 3 H), 1.84 (s, 3 H), 3.53 (s, 3 H), 4.22 (q, J = 7.2 Hz, 2 H), 4.41 (q, J = 6.8 Hz, 1 H), 6.04 (s, 1 H), 7.86 (s, 1 H), 8.34 (s, 1 H).

### Example 157 methyl (2S)-2-{4-chloro-2-(cyanomethyl)-5-[(1,3,4-trimethyl-1H-pyrazol-5-yl)oxy]phenoxy}propionate

The title compound was obtained in the same manner as in Reference Example 88 and from methyl (2S)-2-{4-chloro-2-(hydroxymethyl)-5-[(1,3,4-trimethyl-1H-pyrazol-5-yl)oxy]phenoxy}propionate obtained in Example 151.
¹H-NMR (300 MHz, CDCl₃)δ:1.60 (d, J = 6.8 Hz, 3 H), 1.74 (s, 3 H), 2.19 (s, 3 H), 3.56 (s, 3 H), 3.62 - 3.82 (m, 5 H), 4.54 (q, J = 6.8 Hz, 1 H), 6.07 (s, 1 H), 7.46 (s, 1 H).

### Example 158 (2S)-2-{4-chloro-2-(cyanomethyl)-5-[(1,3,4-trimethyl-1H-pyrazol-5-yl)oxy]phenoxy}propionic acid

The title compound was obtained in the same manner as in Example 112 and from methyl (2S)-2-{4-chloro-2-(cyanomethyl)-5-[(1,3,4-trimethyl-1H-pyrazol-5-yl)oxy]phenoxy}propionate obtained in Example 157.
¹H-NMR (300 MHz, CDCl₃)δ:1.65 (d, J = 6.8 Hz, 3 H), 1.85 (s, 3 H), 2.18 (s, 3 H), 3.51 (s, 3 H), 3.56 - 3.84 (m, 2 H), 4.42 (q, J = 6.8 Hz, 1 H), 6.02 (s, 1 H), 7.46 (s, 1 H).

### Example 159 methyl (2S)-2-{2-acetyl-4-chloro-5-[(1,3,4-trimethyl-1H-pyrazol-5-yl)oxy]phenoxy}propionate

The title compound was obtained in the same manner as in Example 116 and from 1-{5-chloro-2-hydroxy-4-[(1,3,4-trimethyl-1H-pyrazol-5-yl)oxylphenyl}ethanone obtained in Reference Example 150 and methyl (2R)-2-{[(4-methylphenyl)sulfonylloxy}propionate obtained in Reference Example 96.
¹H-NMR (300 MHz, CDCl₃)δ:1.63 (d, J = 6.8 Hz, 3 H), 1.77 (s, 3 H), 2.21 (s, 3 H), 2.65 (s, 3 H), 3.55 (s, 3 H), 3.68 (s, 3 H), 4.59 (q, J = 6.8 Hz, 1 H), 6.04 (s, 1 H), 7.94 (s, 1 H).

### Example 160 (2S)-2-{2-acetyl-4-chloro-5-[(1,3,4-trimethyl-1H-pyrazol-5-yl)oxy]phenoxy}propionic acid

The title compound was obtained in the same manner as in Example 112 and from methyl (2S)-2-{2-acetyl-4-chloro-5-[(1,3,4-trimethyl-1H-pyrazol-5-yl)oxy]phenoxy}propionate obtained in Example 159.
¹H-NMR (300 MHz, CDCl₃)δ:1.69 (d, J = 6.8 Hz, 3 H), 1.88 (s, 3 H), 2.20 (s, 3 H), 2.65 (s, 3 H), 3.54 (s, 3 H), 4.49 (q, J = 6.8 Hz, 1 H), 6.04 (s, 1 H), 7.97 (s, 1 H).

### Example 161 methyl (2S)-2-{4-chloro-2-[(1E)-N-methoxyacetoimidyl]-5-[(1,3,4-trimethyl-1H-pyrazol-5-yl)oxy]phenoxy}propionate

The title compound was obtained in the same manner as in Example 152 and from methyl (2S)-2-{2-acetyl-4-chloro-5-[(1,3,4-trimethyl-1H-pyrazol-5-yl)oxy]phenoxy}propionate obtained in Example 159 and O-methylhydroxylamine hydrochloride.
¹H-NMR (300 MHz, CDCl₃)δ:1.54 (d, J = 6.8 Hz, 3 H), 1.75 (s, 3 H), 2.19 (s, 3 H), 2.20 (s, 3 H), 3.54 (s, 3 H), 3.65 (s, 3 H), 3.98 (s, 3 H), 4.50 (q, J = 6.8 Hz, 1 H), 6.04 (s, 1 H), 7.45 (s, 1 H).

### Example 162 (2S)-2-{4-chloro-2-[(1E)-N-methoxyacetoimidyl]-5-[(1,3,4-trimethyl-1H-pyrazol-5-yl)oxy]phenoxy}propionic acid

The title compound was obtained in the same manner as in Example 112 and from methyl (2S)-2-{4-chloro-2-[(1E)-N-methoxyacetoimidyl]-5-[(1,3,4-trimethyl-1H-pyrazol-5-yl)oxy]phenoxy}propionate obtained in Example 161.
¹H-NMR (300 MHz, CDCl₃)δ:1.60 (d, J = 6.8 Hz, 3 H), 1.82 (s, 3 H), 2.18 (s, 3 H), 2.23 (s, 3 H), 3.54 (s, 3 H), 3.98 (s, 3 H), 4.47 (q, J = 6.8 Hz, 1 H), 6.11 (s, 1 H), 7.45 (s, 1 H).

### Example 163 (2S)-2-{4-chloro-2-[(E)-(phenoxyimino)methyl]-5-[(1,3,4-trimethyl-1H-pyrazol-5-yl)oxy]phenoxy}propionic acid

The title compound was obtained in the same manner as in Example 131 and then Example 112, and from methyl (2S)-2-{4-chloro-2-formyl-5-[(1,3,4-trimethyl-1H-pyrazol-5-yl)oxy]phenoxy}propionate obtained in Example 130 and O-phenylhydroxylamine hydrochloride.
¹H-NMR (300 MHz, CDCl₃)δ:1.66 (d, J = 6.8 Hz, 3 H), 1.87 (s, 3 H), 2.20 (s, 3 H), 3.54 (s, 3 H), 4.44 (q, J = 6.8 Hz, 1 H), 6.05 (s, 1 H), 6.99 - 7.10 (m, 1 H), 7.19 - 7.41 (m, 4 H), 8.06 (s, 1 H), 8.73 (s, 1 H).

### Example 164 (2S)-2-{2-[(E)-(tert-butoxyimino)methyl]-4-chloro-5-[(1,3,4-trimethyl-1H-pyrazol-5-yl)oxy]phenoxy}propionic acid

The title compound was obtained in the same manner as in Example 131 and then Example 112, and from methyl (2S)-2-{4-chloro-2-formyl-5-[(1,3,4-trimethyl-1H-pyrazol-5-yl)oxy]phenoxy}propionate obtained in Example 130 and O-tert-butylhydroxylamine hydrochloride.
¹H-NMR (300 MHz, CDCl₃)δ:1.35 (s, 9 H), 1.61 (d, J = 6.8 Hz, 3 H), 1.84 (s, 3 H), 2.18 (s, 3 H), 3.51 (s, 3 H), 4.40 (q, J = 7.0 Hz, 1 H), 6.01 (s, 1 H), 7.90 (s, 1 H), 8.33 (s, 1 H).

### Example 165 methyl (2S)-2-{5-[(4-acetyl-1,3-dimethyl-1H-pyrazol-5-yl)oxy]-2,4-dichlorophenoxy}propionate

The title compound was obtained in the same manner as in Example 116 and from 1-[5-(2,4-dichloro-5-hydroxyphenoxy)-1,3-dimethyl-1H-pyrazol-4-yl]ethanone obtained in Reference Example 66 and methyl (2R)-2-{[(4-methylphenyl)sulfonyl]oxy}propionate obtained in Reference Example 96.
¹H-NMR (300 MHz, CDCl₃)δ:1.61 (d, J = 6.8 Hz, 3 H), 2.21 (s, 3 H), 2.47 (s, 3 H), 3.56 (s, 3 H), 3.66 (s, 3 H), 4.36 - 4.64 (m, 1 H), 6.06 (s, 1 H), 7.52 (s, 1 H).

### Example 166 methyl (2S)-2-(2,4-dichloro-5-{[4-(N-hydroxyacetoimidyl)-1,3-dimethyl-1H-pyrazol-5-yl]oxy}phenoxy)propionate

To a solution of methyl (2S)-2-{5-[(4-acetyl-1,3-dimethyl-1H-pyrazol-5-yl)oxy]-2,4-dichlorophenoxy}propionate (0.44 g) obtained in Example 165 in 95% ethanol (10 mL) were added hydroxylammonium chloride (0.092 g) and sodium carbonate (0.14 g), and the mixture was stirred at 80°C for 1 hr. Water was added to the reaction mixture, and the mixture was extracted with ethyl acetate. The organic layer was dried over anhydrous magnesium sulfate, and filtered, and the filtrate was concentrated. The residue was subjected to silica gel column chromatography (hexane-ethyl acetate 7:3 - 1:1, v/v), and crystallized from hexane-ethyl acetate to give the title compound (E/Z=1:6, 0.31 g, yield 78%) as colorless crystals.
¹H-NMR (300 MHz, CDCl₃)δ:1.61 (d, J = 6.8 Hz, 3 H), 2.00 (s, 3 H), 2.34 (s, 3 H), 3.57 (s, 3 H), 3.69 (s, 3 H), 4.48 - 4.60 (m, 1 H), 6.28 (s, 1 H), 7.31 (s, 1 H), 7.45 (s, 1 H).

### Example 167 (2S)-2-(2,4-dichloro-5-{[4-(N-hydroxyacetoimidyl)-1,3-dimethyl-1H-pyrazol-5-yl]oxy}phenoxy)propionic acid

The title compound (E/Z=1:25) was obtained in the same manner as in Example 112 and from methyl (2S)-2-(2,4-dichloro-5-{[4-(N-hydroxyacetoimidyl)-1,3-dimethyl-1H-pyrazol-5-yl]oxy}phenoxy)propionate obtained in Example 166.
¹H-NMR (300 MHz, DMSO-d₆)δ:1.34 - 1.52 (m, 3 H), 1.84 (s, 3 H), 2.26 (s, 3 H), 3.46 (s, 3 H), 4.74 (q, J = 6.8 Hz, 1 H), 6.27 (s, 1 H), 7.77 (s, 1 H), 10.86 (br s, 1 H), 13.26 (br s, 1 H).

### Example 168 methyl (2S)-2-(2,4-dichloro-5-{[4-formyl-1-methyl-3-(trifluoromethyl)-1H-pyrazol-5-yl]oxy}phenoxy)propionate

The title compound was obtained in the same manner as in Example 116 and from 5-(2,4-dichloro-5-hydroxyphenoxy)-1-methyl-3-(trifluoromethyl)-1H-pyrazole-4-carbaldehyde obtained in Reference Example 58 and methyl (2R)-2-{[(4-methylphenyl)sulfonyl]oxy}propionate obtained in Reference Example 96.
¹H-NMR (300 MHz, CDCl₃)δ:1.65 (d, J = 6.8 Hz, 3 H), 3.69 (s, 3 H), 3.84 (s, 3 H), 4.61 (q, J = 6.8 Hz, 1 H), 6.50 (s, 1 H), 7.49 (s, 1 H), 9.67 (s, 1 H).

### Example 169 methyl (2S)-2-(2,4-dichloro-5-{[4-[(E)-(hydroxyimino)methyl]-1-methyl-3-(trifluoromethyl)-1H-pyrazol-5-yl]oxy}phenoxy)propionate

The title compound was obtained in the same manner as in Example 103 and from methyl (2S)-2-(2,4-dichloro-5-{[4-formyl-1-methyl-3-(trifluoromethyl)-1H-pyrazol-5-yl]oxy}phenoxy)propionate obtained in Example 168 and hydroxylammonium chloride.
¹H-NMR (300 MHz, DMSO-d₆)δ:1.47 (d, J = 6.6 Hz, 3 H), 3.57 (s, 3 H), 3.72 (s, 3 H), 4.98 - 5.10 (m, 1 H), 6.47 (s, 1 H), 7.70 (d, J = 0.8 Hz, 1 H), 7.80 (s, 1 H), 11.39 (s, 1 H).

### Example 170 (2S)-2-(2,4-dichloro-5-{[4-[(E)-(hydroxyimino)methyl]-1-methyl-3-(trifluoromethyl)-1H-pyrazol-5-yl]oxy}phenoxy)propionic acid

The title compound was obtained in the same manner as in Example 112 and from methyl (2S)-2-(2,4-dichloro-5-{[4-[(E)-(hydroxyimino)methyl]-1-methyl-3-(trifluoromethyl)-1H-pyrazol-5-yl]oxy}phenoxy)propionate obtained in Example 169.
¹H-NMR (300 MHz, DMSO-d₆)δ:1.45 (d, J = 6.8 Hz, 3 H), 3.72 (s, 3 H), 4.89 (q, J = 6.7 Hz, 1 H), 6.55 (s, 1 H), 7.71 (s, 1 H), 7.78 (s, 1 H), 11.35 (br s, 1 H), 13.21 (br s, 1 H).

### Example 171 methyl (2S)-2-[2,4-dichloro-5-({4-[(1E)-3-hydroxyprop-1-en-1-yl]-1,3-dimethyl-1H-pyrazol-5-yl}oxy)phenoxy]propionate

The title compound was obtained in the same manner as in Example 116 and from 2,4-dichloro-5-({4-[(1E)-3-hydroxyprop-1-en-1-yl]-1,3-dimethyl-1H-pyrazol-5-yl}oxy)phenol obtained in Reference Example 152 and methyl (2R)-2-{[(4-methylphenyl)sulfonyl]oxy}propionate obtained in Reference Example 96.
¹H-NMR (300 MHz, CDCl₃)δ:1.60 (d, J = 6.8 Hz, 3 H), 1.74 (t, J = 6.0 Hz, 1 H), 2.31 (s, 3 H), 3.56 (s, 3 H), 3.68 (s, 3 H), 4.10 - 4.20 (m, 2 H), 4.51 (q, J = 6.8 Hz, 1 H), 5.78 - 6.02 (m, 1 H), 6.12 - 6.28 (m, 2 H), 7.48 (s, 1 H).

### Example 172 (2S)-2-[2,4-dichloro-5-({4-[(1E)-3-hydroxyprop-1-en-1-yl]-1,3-dimethyl-1H-pyrazol-5-yl}oxy)phenoxy]propionic acid

The title compound was obtained in the same manner as in Example 112 and from methyl (2S)-2-[2,4-dichloro-5-({4-[(1E)-3-hydroxyprop-1-en-1-yl]-1,3-dimethyl-1H-pyrazol-5-yl}oxy)phenoxy]propionate obtained in Example 171.
¹H-NMR (300 MHz, CDCl₃)δ:1.64 (d, J = 6.8 Hz, 3 H), 2.33 (s, 3 H), 3.59 (s, 3 H), 4.01 - 4.34 (m, 2 H), 4.41 - 4.60 (m, 1 H), 5.73 - 6.01 (m, 1 H), 6.15 - 6.28 (m, 1 H), 6.30 (s, 1 H), 7.46 (s, 1 H).

### Example 173 methyl (2S)-2-(2,4-dichloro-5-{[4-((1E)-3-{[ethylcarbamoyl]oxy}prop-1-en-1-yl)-1,3-dimethyl-1H-pyrazol-5-yl]oxy}phenoxy)propionate

Methyl (2S)-2-[2,4-dichloro-5-({4-[(1E)-3-hydroxyprop-1-en-1-yl]-1,3-dimethyl-1H-pyrazol-5-yl}oxy)phenoxy]propionate (301 mg) obtained in Example 171, and a solution (5 mL) of ethyl isocyanate (55 mg) in pyridine was stirred at 60°C for 1.5 hr. Ethyl isocyanate (165 mg) was further added, and the mixture was stirred at 60°C for 12 hr. Ethyl isocyanate (165 mg) was further added, and the mixture was stirred at 60°C for 3 hr. The solvent was concentrated, and the residue was subjected to silica gel column chromatography (hexane-ethyl acetate 5:5 - 3:1, v/v) to give the title compound (335 mg, yield 100%) as a colorless oil.
¹H-NMR (300 MHz, CDCl₃)δ:1.04 - 1.23 (m, 5 H), 1.54 - 1.60 (m, 3 H), 2.29 (s, 3 H), 3.10 - 3.26 (m, 2 H), 3.26 - 3.38 (m, 1 H), 3.55 (s, 3 H), 3.64 (s, 3 H), 4.45 - 4.53 (m, 1 H), 5.71 - 5.92 (m, 1 H), 6.14 (s, 1 H), 6.18 - 6.31 (m, 1 H), 7.48 (s, 1 H).

### Example 174 (2S)-2-(2,4-dichloro-5-{[4-((1E)-3-{[ethylcarbamoyl]oxy}prop-1-en-1-yl)-1,3-dimethyl-1H-pyrazol-5-yl]oxy}phenoxy)propionic acid

The title compound was obtained in the same manner as in Example 112 and from methyl (2S)-2-(2,4-dichloro-5-{[4-((1E)-3-{[ethylcarbamoyl]oxy}prop-1-en-1-yl)-1,3-dimethyl-1H-pyrazol-5-yl]oxy}phenoxy)propionate obtained in Example 173.
¹H-NMR (300 MHz, DMSO-d₆)δ:0.72 - 1.09 (m, 3 H), 1.43 (d, J = 6.8 Hz, 3 H), 2.13 (s, 3 H), 2.74 - 3.08 (m, 2 H), 3.52 (s, 3 H), 4.23 - 4.48 (m, 2 H), 4.59 - 4.73 (m, 1 H), 5.49 - 5.84 (m, 1 H), 5. 99 - 6.48 (m, 2 H), 6.78 - 7.19 (m, 1 H), 7.80 (s, 1 H), 13.36 (br s, 1 H).

### Example 175 methyl (2S)-2-{2,4-dichloro-5-[(3-formyl-1,4-dimethyl-1H-pyrazol-5-yl)oxy]phenoxy}propionate

The title compound was obtained in the same manner as in Example 116 and from 5-(2,4-dichloro-5-hydroxyphenoxy)-1,4-dimethyl-1H-pyrazole-3-carbaldehyde obtained in Reference Example 154.
¹H-NMR (300 MHz, CDC1₃)δ:1.62 (d, J = 6.4 Hz, 3 H), 2.05 (s, 3 H), 3.65 (s, 3 H), 3.76 (s, 3 H), 4.52 (q, J = 6.8 Hz, 1 H), 6.19 (s, 1 H), 7.49 (s, 1 H), 9.92 (s, 1 H).

### Example 176 methyl (2S)-2-[2,4-dichloro-5-({3-[(E)-(hydroxyimino)methyl]-1,4-dimethyl-1H-pyrazol-5-yl}oxy)phenoxy]propionate

The title compound was obtained in the same manner as in Example 103 and from methyl (2S)-2-{2,4-dichloro-5-[(3-formyl-1,4-dimethyl-1H-pyrazol-5-yl)oxy]phenoxy}propionate obtained in Example 175.
¹H-NMR (300 MHz, CDCl₃)δ:1.61 (d, J = 6.8 Hz, 3 H), 1.96 (s, 3 H), 3.69 (s, 6 H), 4.51 (q, J = 6.8 Hz, 1 H), 6.13 - 6.23 (m, 1 H), 7.33 (s, 1 H), 7.43 (s, 1 H), 8.15 (s, 1 H).

### Example 177 (2S)-2-[2,4-dichloro-5-({3-[(E)-(hydroxyimino)methyl]-1,4-dimethyl-1H-pyrazol-5-yl}oxy)phenoxy]propionic acid

The title compound was obtained in the same manner as in Example 112 and from methyl (2S)-2-[2,4-dichloro-5-({3-[(E)-(hydroxyimino)methyl]-1,4-dimethyl-1H-pyrazol-5-yl}oxy)phenoxy]propionate obtained in Example 176.
¹H-NMR (300 MHz, DMSO-d₆)δ:1.41 (d, J = 6.6 Hz, 3 H), 1.82 (s, 3 H), 3.59 (s, 3 H), 4.37 - 4.68 (m, 1 H), 6.48 (s, 1 H), 7.72 (s, 1 H), 7.96 (s, 1 H), 11.22 (br s, 1 H).

### Example 178 methyl (2S)-2-{2,4-dichloro-5-[(3-cyano-1,4-dimethyl-1H-pyrazol-5-yl)oxy]phenoxy}propionate

The title compound was obtained in the same manner as in Example 105 and from methyl (2S)-2-[2,4-dichloro-5-({3-[(E)-(hydroxyimino)methyl]-1,4-dimethyl-1H-pyrazol-5-yl}oxy)phenoxy]propionate obtained in Example 176.
¹H-NMR (300 MHz, CDCl₃)δ:1.64 (d, J = 6.8 Hz, 3 H), 1.90 (s, 3 H), 3.70 (s, 3 H), 3.73 (s, 3 H), 4.55 (q, J = 6.8 Hz, 1 H), 6.22 (s, 1 H), 7.50 (s, 1 H).

### Example 179 (2S)-2-{2,4-dichloro-5-[(3-cyano-1,4-dimethyl-1H-pyrazol-5-yl)oxy]phenoxy}propionic acid

The title compound was obtained in the same manner as in Example 112 and from methyl (2S)-2-{2,4-dichloro-5-[(3-cyano-1,4-dimethyl-1H-pyrazol-5-yl)oxy]phenoxy}propionate obtained in Example 178.
¹H-NMR (300 MHz, CDCl₃)δ:1.13 (d, J = 6.1 Hz, 3 H), 1.86 (s, 3 H), 3.73 (s, 3 H), 4.32 - 4.65 (m, 1 H), 6.40 (s, 1 H), 7.45 (s, 1 H).

### Example 180 methyl (2S)-2-(2,4-dichloro-5-{[4-cyano-1-methyl-3-(trifluoromethyl)-1H-pyrazol-5-yl]oxy}phenoxy)propionate

The title compound was obtained in the same manner as in Example 105 and from methyl (2S)-2-(2,4-dichloro-5-{[4-[(E)-(hydroxyimino)methyl]-1-methyl-3-(trifluoromethyl)-1H-pyrazol-5-yl]oxy}phenoxy)propionate obtained in Example 169.
¹H-NMR (300 MHz, CDCl₃)δ:1.70 (d, J = 6.8 Hz, 3 H), 3.77 (s, 3 H), 3.90 (s, 3 H), 4.74 (q, J = 6.8 Hz, 1 H), 6.91 (s, 1 H), 7.55 (s, 1 H).

### Example 181 (2S)-2-(2,4-dichloro-5-{[4-cyano-1-methyl-3-(trifluoromethyl)-1H-pyrazol-5-yl]oxy}phenoxy)propionic acid

The title compound was obtained in the same manner as in Example 112 and from methyl (2S)-2-(2,4-dichloro-5-{[4-cyano-1-methyl-3-(trifluoromethyl)-1H-pyrazol-5-yl]oxy}phenoxy)propionate obtained in Example 180.
¹H-NMR (300 MHz, DMSO-d₆)δ:1.44 (d, J = 6.8 Hz, 3 H), 3.84 (s, 3 H), 4.62 (q, J = 6.7 Hz, 1 H), 7.32 (s, 1 H), 7.83 (s, 1 H).

### Example 182 methyl (2S)-2-[2,4-dichloro-5-({4-[(E)-hydrazonomethyl]-1,3-dimethyl-1H-pyrazol-5-yl}oxy)phenoxy]propionate

Methyl (2S)-2-{2,4-dichloro-5-[(4-formyl-1,3-dimethyl-1H-pyrazol-5-yl)oxy]phenoxy}propionate (300 mg) obtained in Example 102 and a solution (8 mL) of hydrazine monohydrate (41 mg) in methanol were stirred at 50°C for 40 min. The solvent was concentrated, and the residue was subjected to silica gel column chromatography (hexane-ethyl acetate 7:3 - 1:1, v/v) to give the title compound (239 mg, yield 79%) as colorless crystals.
¹H-NMR (300 MHz, CDCl₃)δ:1.57 - 1.65 (m, 3 H), 2.38 (s, 3 H), 3.58 (s, 3 H), 3.68 (s, 3 H), 4.53 (q, J = 6.8 Hz, 1 H), 5.27 (br s, 2 H), 6.21 (s, 1 H), 7.40 (s, 1 H), 7.47 (s, 1 H).

### Example 183 methyl (2S)-2-{2,4-dichloro-5-[(1,3-dimethyl-4-{(E)-[(2-oxopropoxy)imino]methyl}-1H-pyrazol-5-yl)oxy]phenoxy}propionate

A solution of methyl (2S)-2-[2,4-dichloro-5-({4-[(E)-(hydroxyimino)methyl]-1,3-dimethyl-1H-pyrazol-5-yl}oxy)phenoxy]propionate (302 mg) obtained in Example 103 in N,N-dimethylformamide (6 mL) was cooled in an ice bath, and sodium hydride (33 mg) was added by small portions with stirring. Chloroacetone (77 mg) was added, and the mixture was stirred at 0°C for 30 min, at room temperature for 1 hr, and at 50°C for 2.5 hr. Chloroacetone (77 mg) was added and the mixture was stirred at 50°C for 19 hr. N,N-Dimethylformamide (6 mL), sodium hydride (33 mg) and chloroacetone (154 mg) were added, and the mixture was stirred at 50°C for 4 hr. Further, chloroacetone (154 mg) was added, and the mixture was stirred at 50°C for 3 hr. Water was added to the reaction mixture, and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 4:1 - 6:4, v/v) to give the title compound (18 mg, yield 5%) as a colorless oil.
¹H-NMR (300 MHz, CDCl₃)δ:1.56 - 1.65 (m, 3 H), 2.10 (s, 3 H), 2.34 (s, 3 H), 3.62 (s, 3 H), 3.68 (s, 3 H), 4.42 (s, 2 H), 4.54 (q, J = 6.8 Hz, 1 H), 6.19 (s, 1 H), 7.26 (s, 1 H), 7.86 (s, 1 H).

### Example 184 methyl (2S)-2-{2,4-dichloro-5-[(4-{(E)-[(cyanomethyl)imino]methyl}-1,3-dimethyl-1H-pyrazol-5-yl)oxy]phenoxy}propionate

To a solution (5 mL) of methyl (2S)-2-{2,4-dichloro-5-[(4-formyl-1,3-dimethyl-1H-pyrazol-5-yl)oxy]phenoxy}propionate (387 mg) obtained in Example 102 in methanol was added aminoacetonitrile hydrochloride (102 mg) and the mixture was stirred at 50°C for 2 hr. Sodium carbonate (117 mg) was added and the mixture was stirred at 50°C for 2.5 hr. Aminoacetonitrile hydrochloride (51 mg) and sodium carbonate (59 mg) were added and the mixture was stirred at 70°C for 1 hr. The reaction mixture was concentrated, water was added, and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 4:1 - 1:1, v/v), and then again silica gel column chromatography (NH silica, hexane-ethyl acetate 4:1, v/v) to give the title compound (64 mg, yield 15%) as a colorless oil.
¹H-NMR (300 MHz, CDCl₃)δ:1.62 (d, J = 6.8 Hz, 3 H), 2.42 (s, 3 H), 3.63 (s, 3 H), 3.68 (s, 3 H), 4.36 - 4.44 (m, 2 H), 4.55 (q, J = 6.7 Hz, 1 H), 6.27 (s, 1 H), 7.49 (s, 1 H), 8.07 (t, J = 1.7 Hz, 1 H).

### Example 185 methyl (2S)-2-{2,4-dichloro-5-[(4-{(E)-[(cyanomethoxy)imino]methyl}-1,3-dimethyl-1H-pyrazol-5-yl)oxy]phenoxy}propionate

A solution of methyl (2S)-2-[2,4-dichloro-5-({4-[(E)-(hydroxyimino)methyl]-1,3-dimethyl-1H-pyrazol-5-yl}oxy)phenoxy]propionate (308 mg) obtained in Example 103 in N,N-dimethylformamide (5 mL) was cooled in an ice bath and sodium hydride (46 mg) was added by small portions with stirring. Bromoacetonitrile (138 mg) was added, and the mixture was stirred at room temperature for 2.5 hr. Bromoacetonitrile (138 mg) was added and the mixture was stirred at 50°C for 1.5 hr. Sodium hydride (60 mg) was added, and the mixture was stirred at 80°C for 3 hr. Bromoacetonitrile (138 mg) was added, and the mixture was stirred at 50°C for 13 hr. Water was added to the reaction mixture, and the mixture was extracted with ethyl acetate. The organic layer was washed with saturated brine, dried over anhydrous magnesium sulfate and filtered. The filtrate was concentrated, and the residue was subjected to silica gel column chromatography (NH silica, hexane-ethyl acetate 4:1 - 1:1, v/v) to give the title compound (236 mg, yield 69%) as a colorless oil.
¹H-NMR (300 MHz, CDCl₃)δ:1.61 (d, J = 6.8 Hz, 3 H), 2.38 (s, 3 H), 3.62 (s, 3 H), 3.68 (s, 3 H), 4.49 - 4.58 (m, 1 H), 4.58 - 4.63 (m, 2 H), 6.22 (s, 1 H), 7.49 (s, 1 H), 7.78 (s, 1 H).

### Example 186 (2S)-2-{2,4-dichloro-5-[(4-{(E)-[(cyanomethoxy)imino]methyl}-1,3-dimethyl-1H-pyrazol-5-yl)oxy]phenoxy}propionic acid

The title compound was obtained in the same manner as in Example 112 and from methyl (2S)-2-{2,4-dichloro-5-[(4-{(E)-[(cyanomethoxy)imino]methyl}-1,3-dimethyl-1H-pyrazol-5-yl)oxy]phenoxy}propionate obtained in Example 185.
¹H-NMR (300 MHz, DMSO-d₆)δ:1.35 (d, J = 6.8 Hz, 3 H), 2.24 (s, 3 H), 3.59 (s, 3 H), 4.13 - 4.30 (m, 1 H), 4.81 (s, 2 H), 6.54 (s, 1 H), 7.64 (s, 1 H), 7.76 (s, 1 H).

### Example 187 methyl (2S)-2-(2,4-dichloro-5-{[4-[(E)-2-cyanovinyl]-1-methyl-3-(trifluoromethyl)-1H-pyrazol-5-yl]oxy}phenoxy)propionate

The title compound was obtained in the same manner as in Example 116 and from (2E)-3-[5-(2,4-dichloro-5-hydroxyphenoxy)-1-methyl-3-(trifluoromethyl)-1H-pyrazol-4-yl]acrylonitrile obtained in Reference Example 155 and methyl (2R)-2-{[(4-methylphenyl)sulfonyl]oxy}propionate obtained in Reference Example 96.
¹H-NMR (300 MHz, CDCl₃)δ:1.63 (d, J = 6.8 Hz, 3 H), 3.67 (s, 3 H), 3.70 (s, 3 H), 4.36 - 4.69 (m, 1 H), 5.63 (d, J = 16.8 Hz, 1 H), 6.08 (s, 1 H), 7.15 (d, J = 16.8 Hz, 1 H), 7.56 (s, 1 H).

### Example 188 (2S)-2-(2,4-dichloro-5-{[4-[(E)-2-cyanovinyl]-1-methyl-3-(trifluoromethyl)-1H-pyrazol-5-yl]oxy}phenoxy)propionic acid

The title compound was obtained in the same manner as in Example 112 and from methyl (2S)-2-(2,4-dichloro-5-{[4-[(E)-2-cyanovinyl]-1-methyl-3-(trifluoromethyl)-1H-pyrazol-5-yl]oxy}phenoxy)propionate obtained in Example 187.
¹H-NMR (300 MHz, CDCl₃)δ:1.15 - 1.36 (m, 3 H), 3.72 (s, 3 H), 4.36 - 4.66 (m, 1 H), 5.54 (d, J = 17.0 Hz, 1 H), 6.32 (s, 1 H), 7.10 (d, J = 17.0 Hz, 1 H), 7.52 (s, 1 H).

### Example 189 methyl (2S)-2-{2-chloro-4-formyl-5-[(1,3,4-trimethyl-1H-pyrazol-5-yl)oxy]phenoxy}propionate

The title compound was obtained in the same manner as in Example 116 and from 5-chloro-4-hydroxy-2-[(1,3,4-trimethyl-1H-pyrazol-5-yl)oxy]benzaldehyde obtained in Reference Example 160 and methyl (2R)-2-{[(4-methylphenyl)sulfonyl]oxy}propionate obtained in Reference Example 96.
¹H-NMR (300 MHz, CDCl₃)δ:1.65 (d, J = 6.8 Hz, 3 H), 1.75 (s, 3 H), 2.20 (s, 3 H), 3.55 (s, 3 H), 3.67 (s, 3 H), 4.12 (q, J = 7.2 Hz, 1 H), 6.01 (s, 1 H), 7.96 (s, 1 H), 10.45 (s, 1 H).

### Example 190 (2S)-2-{2-chloro-4-formyl-5-[(1,3,4-trimethyl-1H-pyrazol-5-yl)oxy]phenoxy}propionic acid

The title compound was obtained in the same manner as in Example 112 and from methyl (2S)-2-{2-chloro-4-formyl-5-[(1,3,4-trimethyl-1H-pyrazol-5-yl)oxy]phenoxy}propionate obtained in Example 189.
¹H-NMR (300 MHz, DMSO-d₆)δ:1.51 (d, J = 6.8 Hz, 3 H), 1.67 (s, 3 H), 2.07 (s, 3 H), 3.52 (s, 3 H), 4.91 (q, J = 6.7 Hz, 1 H), 6.23 (s, 1 H), 7.84 (s, 1 H), 10.31 (s, 1 H), 13.39 (s, 1 H).

### Example 191 methyl (2S)-2-{2-chloro-4-[(E)-(hydroxyimino)methyl]-5-[(1,3,4-trimethyl-1H-pyrazol-5-yl)oxy]phenoxy}propionate

The title compound was obtained in the same manner as in Example 103 and from methyl (2S)-2-{2-chloro-4-formyl-5-[(1,3,4-trimethyl-1H-pyrazol-5-yl)oxy]phenoxy}propionate obtained in Example 189 and hydroxylammonium chloride.
¹H-NMR (300 MHz, DMSO-d₆)δ:1.49 (d, J = 6.8 Hz, 3 H), 1.63 (s, 3 H), 2.08 (s, 3 H), 3.47 (s, 3 H), 3.59 (s, 3 H), 4.92 (q, J = 6.8 Hz, 1 H), 6.11 (s, 1 H), 7.76 (s, 1 H), 8.32 (s, 1 H), 11.48 (s, 1 H).

### Example 192 (2S)-2-{2-chloro-4-[(E)-(hydroxyimino)methyl]-5-[(1,3,4-trimethyl-1H-pyrazol-5-yl)oxy]phenoxy}propionic acid

The title compound was obtained in the same manner as in Example 112 and from methyl (2S)-2-{2-chloro-4-[(E)-(hydroxyimino)methyl]-5-[(1,3,4-trimethyl-1H-pyrazol-5-yl)oxy]phenoxy}propionate obtained in Example 191.
¹H-NMR (300 MHz, DMSO-d₆)δ:1.48 (d, J = 6.6 Hz, 3 H), 1.64 (s, 3 H), 2.06 (s, 3 H), 3.47 (s, 3 H), 4.75 (q, J = 6. 7 Hz, 1 H), 6.20 (s, 1 H), 7.75 (s, 1 H), 8.32 (s, 1 H), 11.46 (s, 1 H), 13.25 (br s, 1 H).

### Example 193 methyl (2S)-2-{2-chloro-4-cyano-5-[(1,3,4-trimethyl-1H-pyrazol-5-yl)oxy]phenoxy}propionate

The title compound was obtained in the same manner as in Example 105 and from methyl (2S)-2-{2-chloro-4-[(E)-(hydroxyimino)methyl]-5-[(1,3,4-trimethyl-1H-pyrazol-5-yl)oxy]phenoxy}propionate obtained in Example 191.
¹H-NMR (300 MHz, CDCl₃)δ:1.65 (d, J = 6.8 Hz, 3 H), 1.76 (s, 3 H), 2.19 (s, 3 H), 3.57 (s, 3 H), 3.68 (s, 3 H), 4.57 (q, J = 6.8 Hz, 1 H), 6.06 (s, 1 H), 7.66 (s, 1 H).

### Example 194 (2S)-2-{2-chloro-4-cyano-5-[(1,3,4-trimethyl-1H-pyrazol-5-yl)oxy]phenoxy}propionic acid

The title compound was obtained in the same manner as in Example 112 and from methyl (2S)-2-{2-chloro-4-cyano-5-[(1,3,4-trimethyl-1H-pyrazol-5-yl)oxy]phenoxy}propionate obtained in Example 193.
¹H-NMR (300 MHz, DMSO-d₆)δ:1.50 (d, J = 6.8 Hz, 3 H), 1.66 (s, 3 H), 2.03 (s, 3 H), 3.50 (s, 3 H), 4.97 (q, J = 6.8 Hz, 1 H), 6.32 (s, 1 H), 8.12 (s, 1 H), 13.42 (br s, 1 H).

### Example 195 methyl (2S)-2-{2-chloro-4-[(E)-(methoxyimino)methyl]-5-[(1,3,4-trimethyl-1H-pyrazol-5-yl)oxy]phenoxy}propionate

The title compound was obtained in the same manner as in Example 103 and using methyl (2S)-2-{2-chloro-4-formyl-5-[(1,3,4-trimethyl-1H-pyrazol-5-yl)oxy]phenoxy}propionate obtained in Example 189 and O-methylhydroxylamine hydrochloride.
¹H-NMR (300 MHz, CDCl₃)δ:1.61 (d, J = 6.8 Hz, 3 H), 1.72 (s, 3 H), 2.19 (s, 3 H), 3.54 (s, 3 H), 3.69 (s, 3 H), 4.01 (s, 3 H), 4.53 (q, J = 6.8 Hz, 1 H), 6.02 (s, 1 H), 7.92 (s, 1 H), 8.43 (s, 1 H).

### Example 196 (2S)-2-{2-chloro-4-[(E)-(methoxyimino)methyl]-5-[(1,3,4-trimethyl-1H-pyrazol-5-yl)oxy]phenoxy}propionic acid

The title compound was obtained in the same manner as in Example 112 and from methyl (2S)-2-{2-chloro-4-[(E)-(methoxyimino)methyl]-5-[(1,3,4-trimethyl-1H-pyrazol-5-yl)oxy]phenoxy}propionate obtained in Example 195.
¹H-NMR (300 MHz, DMSO-d₆)δ:1.48 (d, J = 6.8 Hz, 3 H), 1.63 (s, 3 H), 2.06 (s, 3 H), 3.47 (s, 3 H), 3.93 (s, 3 H), 4.56 - 4.94 (m, 1 H), 6.20 (s, 1 H), 7.75 (s, 1 H), 8.40 (s, 1 H), 13.28 (br s, 1 H).

### Example 197 methyl (2S)-2-{4-{(E)-[(benzyloxy)imino]methyl}-2-chloro-5-[(1,3,4-trimethyl-1H-pyrazol-5-yl)oxy]phenoxy}propionate

The title compound was obtained in the same manner as in Example 103 and using methyl (2S)-2-{2-chloro-4-formyl-5-[(1,3,4-trimethyl-1H-pyrazol-5-yl)oxy]phenoxy}propionate obtained in Example 189 and O-benzylhydroxylamine hydrochloride.
¹H-NMR (300 MHz, CDCl₃)δ:1.64 (d, J = 6.8 Hz, 3 H), 1.74 (s, 3 H), 2.20 (s, 3 H), 3.56 (s, 3 H), 3.67 (s, 3 H), 4.43 - 4.69 (m, 1 H), 6.05 (s, 1 H), 6.96 - 7.13 (m, 1 H), 7.28 - 7.46 (m, 6 H), 8.07 (s, 1 H), 8.81 (s, 1 H).

### Example 198 (2S)-2-{4-{(E)-[(benzyloxy)imino]methyl}-2-chloro-5-[(1,3,4-trimethyl-1H-pyrazol-5-yl)oxy]phenoxy}propionic acid

The title compound was obtained in the same manner as in Example 112 and from methyl (2S)-2-{4-{(E)-[(benzyloxy)imino]methyl}-2-chloro-5-[(1,3,4-trimethyl-1H-pyrazol-5-yl)oxy]phenoxy}propionate obtained in Example 197.
¹H-NMR (300 MHz, DMSO-d₆)δ:1.50 (d, J = 6.8 Hz, 3 H), 1.68 (s, 3 H), 2.03 (s, 3 H), 3.50 (s, 3 H), 4.69 - 4.95 (m, 1 H), 6.26 (s, 1 H), 6.83 - 7.46 (m, 7 H), 8.01 (s, 1 H), 8.85 (s, 1 H), 13.39 (br s, 1 H).

### Example 199 methyl (2S)-2-{2-chloro-4-(hydroxymethyl)-5-[(1,3,4-trimethyl-1H-pyrazol-5-yl)oxy]phenoxy}propionate

A solution (5 mL) of methyl (2S)-2-{2-chloro-4-formyl-5-[(1,3,4-trimethyl-1H-pyrazol-5-yl)oxy]phenoxy}propionate (219 mg) obtained in Example 189 in THF was cooled to °C in an ice bath. Sodium borohydride (23 mg) was added, then MeOH (0.2 mL) was added, and the mixture was stirred at 0°C for 15 min. Water was added to the reaction mixture, and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 4:1 - 0:1, v/v) to give the title compound (99 mg, yield 45%) as a colorless oil.
¹H-NMR (300 MHz, DMSO-d₆)δ:1.47 (d, J = 6.6 Hz, 3 H), 1.63 (s, 3 H), 2.07 (s, 3 H), 3.44 (s, 3 H), 3.57 (s, 3 H), 4.58 (d, J = 5.7 Hz, 2 H), 4.80 (q, J = 6.8 Hz, 1 H), 5.28 (t, J = 5.7 Hz, 1 H), 6.04 (s, 1 H), 7.49 (s, 1 H).

### Example 200 (2S)-2-{2-chloro-4-(hydroxymethyl)-5-[(1,3,4-trimethyl-1H-pyrazol-5-yl)oxy]phenoxy}propionic acid

The title compound was obtained in the same manner as in Example 112 and from methyl (2S)-2-{2-chloro-4-(hydroxymethyl)-5-[(1,3,4-trimethyl-1H-pyrazol-5-yl)oxy]phenoxy}propionate obtained in Example 199.
¹H-NMR (300 MHz, DMSO-d₆)δ:1.46 (d, J = 6.8 Hz, 3 H), 1.64 (s, 3 H), 2.02 (s, 3 H), 3.45 (s, 3 H), 4.46 - 4.78 (m, 3 H), 5.27 (t, J = 5.6 Hz, 1 H), 6.14 (s, 1 H), 7.47 (s, 1 H), 13.14 (br s, 1 H).

### Example 201 methyl (2S)-2-[2,4-dichloro-5-({4-[(E)-2-cyanovinyl]-1,3-dimethyl-1H-pyrazol-5-yl}oxy)phenoxy]propionate

To a solution of diethyl (cyanomethyl)phosphonate (709 mg) in tetrahydrofuran (20 mL) was added sodium hydride (60% in oil, 160 mg) at room temperature and the mixture was stirred for 30 min. To the reaction mixture was added 5-(2,4-dichloro-5-hydroxyphenoxy)-1,3-dimethyl-1H-pyrazole-4-carbaldehyde (500 mg) obtained in Reference Example 52 and the mixture was stirred for 30 min. Water was added to the reaction mixture, and the mixture was extracted with ethyl acetate. The organic layer was washed with water and saturated brine, dried over anhydrous magnesium sulfate and filtered. The filtrate was concentrated and potassium carbonate (967 mg) and dimethyl sulfoxide (5 mL) were added to the residue, and the mixture was stirred for 10 min. The reaction system was deaerated, and purged with nitrogen, and a solution of methyl (2R)-2-{[(4-methylphenyl)sulfonyl]oxy}propionate (532 mg) obtained in Reference Example 96 in dimethyl sulfoxide (5 mL) was added carefully so that the temperature of the reaction system would not exceed 30°C. The mixture was stirred at room temperature for 4 hr, ice-cooled water was added, and the mixture was extracted with ethyl acetate. The organic layer was washed with saturated brine, dried over anhydrous magnesium sulfate and filtered. The filtrate was concentrated, the residue was subjected to silica gel column chromatography (hexane-ethyl acetate 80:20 - 50:50, v/v), and the solvent was evaporated to give a colorless oil (388 mg, yield 57%).
¹H-NMR (300 MHz, CDCl₃)δ:1.61 (d, J = 6.9 Hz, 3 H), 2.35 (s, 3 H), 3.52 (s, 3 H), 3.67 (s, 3 H), 4.51 (q, J = 6.9 Hz, 1 H), 5.39 (d, J = 16.5 Hz, 1 H), 6.11 (s, 1 H), 7.03 (d, J = 16.5 Hz, 1 H), 7.52 (s, 1 H).

### Example 202 (2S)-2-[2,4-dichloro-5-({4-[(E)-2-cyanovinyl]-1,3-dimethyl-1H-pyrazol-5-yl}oxy)phenoxy]propionic acid

The title compound was obtained in the same manner as in Example 112 and from methyl (2S)-2-[2,4-dichloro-5-({4-[(E)-2-cyanovinyl]-1,3-dimethyl-1H-pyrazol-5-yl}oxy)phenoxy]propionate obtained in Example 201.
¹H-NMR (300 MHz, DMSO-d₆)δ:1.46 (d, J = 6.9 Hz, 3 H), 2.26 (s, 3 H), 3.52 (s, 3 H), 4.84 (q, J = 6.9 Hz, 1 H), 5.44 (dd, J = 16.5, 1.8 Hz, 1H), 6.34 (s, 1 H), 7.16 (dd, J = 16.5, 1.8 Hz, 1 H), 7.83 (s, 1 H), 13.25 (s, 1 H).

### Example 203 (2S)-2-{4-chloro-3-[(4-ethyl-1,3-dimethyl-1H-pyrazol-5-yl)thio]phenoxy}propionic acid

The title compound was obtained in the same manner as in Example 116 and then Example 112, and from 4-chloro-3-[(4-ethyl-1,3-dimethyl-1H-pyrazol-5-yl)thio]phenol obtained in Reference Example 161 and methyl (2R)-2-{[(4-methylphenyl)sulfonyl]oxy}propionate obtained in Reference Example 96.
¹H-NMR (300 MHz, CDCl₃)δ:1-08 (t, J = 7.5 Hz, 3 H), 1.56 (d, J = 6.9 Hz, 3H), 2.30 (s, 3 H), 2.50 (q, J = 7.5 Hz, 2 H), 3.73 (s, 3 H), 4.29 (q, J = 6.9 Hz, 1 H), 5.69 (d, J = 2.7 Hz, 1 H), 6.69 (dd, J = 8.7, 2.7 Hz, 1 H), 7.21 (d, J = 8.7 Hz, 1 H), 9.64 (br, 1 H).

### Example 204 (2S)-2-{2,4-dichloro-3-[(1,3,4-trimethyl-1H-pyrazol-5-yl)thio]phenoxy}propionic acid

The title compound was obtained in the same manner as in Example 116 and then Example 112, and from 2,4-dichloro-3-[(1,3,4-trimethyl-1H-pyrazol-5-yl)thio]phenol obtained in Reference Example 162 and methyl (2R)-2-{[(4-methylphenyl)sulfonyl]oxy}propionate obtained in Reference Example 96.
¹H-NMR (300 MHz, DMSO-d₆)δ:1.71 (d, J = 6.8 Hz, 3 H), 1.91 (s, 3 H), 2.14 (s, 3 H), 3.66 (s, 3 H), 4.76 (q, J = 6.8 Hz, 1 H), 6.83 (d, J = 9.1 Hz, 1 H), 7.19 - 7.28 (m, 1 H), 10.03 (br, 1 H).

### Example 205 (2S)-2-{2,4-dichloro-5-[(1,3-dimethyl-4-vinyl-1H-pyrazol-5-yl)thio]phenoxy}propionic acid

The title compound was obtained in the same manner as in Example 116 and then Example 112, and from 2,4-dichloro-5-[(1,3-dimethyl-4-vinyl-1H-pyrazol-5-yl)thio]phenol obtained in Reference Example 163 and methyl (2R)-2-{[(4-methylphenyl)sulfonyl]oxy}propionate obtained in Reference Example 96.
¹H-NMR (300 MHz, DMSO-d₆)δ:1.43 (d, J = 6.6 Hz, 3 H), 2.31 (s, 3 H), 3.71 (s, 3 H), 4.42 (q, J = 6.6 Hz, 1 H), 5.20 (dd, J = 11.4, 1.2 Hz, 1 H), 5.53 (dd, J = 18.0, 1.5 Hz, 1 H), 5.85 (s, 1 H), 6.44 - 6.53 (m, 1 H), 7.70 (s, 1 H), 13.13 (br, 1 H).

### Example 206 (2S)-2-{2,4-dichloro-5-[(4-ethyl-1,3-dimethyl-1H-pyrazol-5-yl)thio]phenoxy}propionic acid

The title compound was obtained in the same manner as in Example 116 and then Example 112, and from 2,4-dichloro-5-[(4-ethyl-1,3-dimethyl-1H-pyrazol-5-yl)thio]phenol obtained in Reference Example 164 and methyl (2R)-2-{[(4-methylphenyl)sulfonyl]oxy}propionate obtained in Reference Example 96.
¹H-NMR (300 MHz, CDCl₃)δ:0.96 (t, J = 7.5 Hz, 3 H), 1.45 (d, J = 6.9 Hz, 3 H), 2.19 (s, 3 H), 2.65 - 2.42 (m, 2 H), 3.66 (s, 3 H), 4.38 (q, J = 6.9 Hz, 1 H), 5.73 (s, 1 H), 7.68 (s, 1 H), 13.13 (s, 1 H).

### Example 207 methyl (2S)-2-{2,4-dichloro-5-[(4-formyl-1,3-dimethyl-1H-pyrazol-5-yl)thio]phenoxy}propionate

A mixture of 5-[(2,4-dichloro-5-hydroxyphenyl)thio]-1,3-dimethyl-1H-pyrazole-4-carbaldehyde (2.5 g) obtained in Reference Example 130, potassium carbonate (4.56 g) and dimethyl sulfoxide (20 mL) was stirred at room temperature for 10 min. The reaction system was deaerated, purged with argon, and methyl (2R)-2-{[(4-methylphenyl)sulfonyl]oxy}propionate (2.53 g) obtained in Reference Example 96 was added. The mixture was stirred at room temperature for 1 hr, water was added, and the mixture was extracted with ethyl acetate. The organic layer was washed with saturated brine, dried over anhydrous magnesium sulfate and filtered. The filtrate was concentrated, the residue was subjected to silica gel column chromatography (hexane-ethyl acetate 90:10 - 50:50, v/v), and the solvent was evaporated to give the title compound as crystals (3.52 g).
¹H-NMR (300 MHz, CDCl₃)δ:1.59 (d, J = 6.9 Hz, 3 H), 2.56 (s, 3 H), 3.67 (s, 3 H), 3.80 (s, 3 H), 4.38 (q, J = 6.9 Hz, 1 H), 5.93 (s, 1 H), 7.42 (s, 1 H), 9.92 (s, 1 H).

### Example 208 methyl (2S)-2-[2,4-dichloro-5-({4-[(E)-(hydroxyimino)methyl]-1,3-dimethyl-1H-pyrazol-5-yl}thio)phenoxy]propionate

A mixture of methyl (2S)-2-{2,4-dichloro-5-[(4-formyl-1,3-dimethyl-1H-pyrazol-5-yl)thio]phenoxy}propionate (2.0 g) obtained in Example 207, hydroxylamine hydrochloride (1.39 g) and methanol (200 mL) was stirred at 60°C for 3 hr, and concentrated under reduced pressure. Water was added to the residue, and the mixture was extracted with ethyl acetate. The organic layer was washed with saturated brine, and dried over anhydrous magnesium sulfate. After filtration, the filtrate was concentrated, the residue was subjected to silica gel column chromatography (hexane-ethyl acetate 90:10 - 60:40, v/v), and the solvent was evaporated to give the title compound as crystals (1.53 g, yield 74%).
¹H-NMR (300 MHz, DMSO-d₆)δ:1.46 (d, J = 6.6 Hz, 3 H), 2.38 (s, 3 H), 3.59 (s, 3 H), 3.71 (s, 3 H), 4.65 (q, J = 6.6 Hz, 1 H), 5.75 (s, 1 H), 7.74 (s, 1 H), 7.89 (s, 1 H), 11.16 (s, 1 H).

### Example 209 (2S)-2-[2,4-dichloro-5-({4-[(E)-(hydroxyimino)methyl]-1,3-dimethyl-1H-pyrazol-5-yl}thio)phenoxy]propionic acid

The title compound was obtained in the same manner as in Example 112 and from methyl (2S)-2-[2,4-dichloro-5-(4-[(E)-(hydroxyimino)methyl]-1,3-dimethyl-1H-pyrazol-5-yl}thio)phenoxy]propionate obtained in Example 208.
¹H-NMR (300 MHz, DMSO-d₆)δ:1.43 (d, J = 6.9 Hz, 3 H), 2.35 (s, 3 H), 3.71 (s, 3 H), 4.49 (q, J = 6.9 Hz, 1 H), 5.90 (s, 1 H), 7.72 (s, 1 H), 7.91 (s, 1 H), 11.12 (s, 1 H), 13.20 (br, 1 H).

### Example 210 (2S)-2-{2,4-dichloro-5-[(4-cyano-1,3-dimethyl-1H-pyrazol-5-yl)thio]phenoxy}propionic acid

A mixture of methyl (2S)-2-[2,4-dichloro-5-({4-[(E)-(hydroxyimino)methyl]-1,3-dimethyl-1H-pyrazol-5-yl}thio)phenoxy]propionate (1.0 g) obtained in Example 208, triethylamine (0.669 mL), trichloroacetyl chloride (0.527 mg) and tetrahydrofuran (50 mL) was stirred at 60°C for 3 hr, water was added, and the mixture was extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 90:10 - 67:33, v/v). The obtained oil was dissolved in a mixed solvent of tetrahydrofuran (7 mL) and water (2 mL). Lithium hydroxide monohydrate (184 mg) was added, and the mixture was stirred at room temperature for 3 hr. The reaction mixture was neutralized with dil. hydrochloric acid, and extracted with ethyl acetate. The organic layer was washed with saturated brine, dried over anhydrous magnesium sulfate and filtered, and the filtrate was concentrated to give the title compound as crystals (747 mg, yield 88%).
¹H-NMR (300 MHz, DMSO-d₆)δ:1.48 (d, J = 6.9 Hz, 3 H), 2.33 (s, 3 H), 3.80 (s, 3 H), 4.71 (q, J = 6.9 Hz, 1 H), 6.22 (s, 1 H), 7.78 (s, 1 H), 13.24 (s, 1 H).

### Example 211 (2S)-2-{2,4-dichloro-5-[(1,3,4-trimethyl-1H-pyrazol-5-yl)thio]phenoxy}-N-(pentylsulfonyl)propanamide

The title compound was obtained in the same manner as in Example 54 and from (2S)-2-{2,4-dichloro-5-[(1,3,4-trimethyl-1H-pyrazol-5-yl)thio]phenoxy}propionic acid obtained in Example 115 and pentanesulfonamide.
¹H-NMR (300 MHz, DMSO-d₆)δ:0.84 (d, J = 6.6 Hz, 3 H), 1.25 - 1.31 (m,4 H), 1.44 (d, J = 6.3 Hz, 3 H), 1.58 - 1.62 (m, 2 H), 1.92 (s, 3 H), 2.20 (s, 3 H), 3.28 - 3.33 (m, 2 H), 3.68 (s, 3 H), 4.51 (q, J = 6.3 Hz, 1 H), 5.89 (s, 1 H), 7.72 (s, 1 H), 11.93 (s, 1 H).

### Example 212 methyl (2S)-2-{2,4-dichloro-5-[(4-formyl-1-methyl-3-propyl-1H-pyrazol-5-yl)oxy]phenoxy}propionate

The title compound was obtained in the same manner as in Reference Example 50 and then Example 116, and from 5-{2,4-dichloro-5-[(4-methoxybenzyl)oxy]phenoxy}-1-methyl-3-propyl-1H-pyrazole-4-carbaldehyde obtained in Reference Example 45.
¹H-NMR (300 MHz, CDCl₃)δ:0.97 - 1.03 (m, 3 H), 1.63 (d, J = 6.9 Hz, 3H), 1.66 - 1.76 (m, 2 H), 2.78 - 2.84 (m, 2 H), 3.67 (s, 3 H), 3.68 (s, 3 H), 4.56 (q, J = 6.9 Hz, 1 H), 6.41 (s, 1 H), 7.48 (s, 1 H), 9.50 (s, 1 H).

### Example 213 methyl (2S)-2-[2,4-dichloro-5-({4-[(E)-(hydroxyimino)methyl]-1-methyl-3-propyl-1H-pyrazol-5-yl}oxy)phenoxy]propionate

The title compound was obtained in the same manner as in Example 140 and from methyl (2S)-2-{2,4-dichloro-5-[(4-formyl-1-methyl-3-propyl-1H-pyrazol-5-yl)oxy]phenoxy}propionate obtained in Example 212.
¹H-NMR (300 MHz, CDCl₃)δ:0.97 (t, J = 7.5 Hz, 3H), 1.61 (d, J = 6.9 Hz, 3 H), 1.62 - 1.73 (m, 2H), 2.67 (t, J = 7.5 Hz, 2 H), 3.60 (s, 3 H), 3.68 (s, 3 H), 4.53 (q, J = 6.9 Hz, 1 H), 6.24 (s, 1 H), 7.44 (s, 1 H), 7.80 (s, 1 H), 7.86 (s, 1 H).

### Example 214 (2S)-2-[2,4-dichloro-5-({4-[(E)-(hydroxyimino)methyl]-1-methyl-3-propyl-1H-pyrazol-5-yl}oxy)phenoxy]propionic acid

The title compound was obtained in the same manner as in Example 112 and from methyl (2S)-2-[2,4-dichloro-5-({4-[(E)-(hydroxyimino)methyl]-1-methyl-3-propyl-1H-pyrazol-5-yl}oxy)phenoxy]propionate obtained in Example 213.
¹H-NMR (300 MHz, CDCl₃)δ:0.95 (t, J = 7.2 Hz, 3 H), 1.59 - 1.72 (m, 2 H), 1.64 (d, J = 6.9 Hz, 3 H), 2.62 (t, J = 7.2 Hz, 2 H), 3.50 (s, 3 H), 4.37 (q, J = 6.9 Hz, 1 H), 6.07 (s, 1 H), 7.47 (s, 1 H), 7.93 (s, 1 H), 9.37 (br, 2 H).

### Example 215 methyl (2S)-2-{2,4-dichloro-5-[(3-cyclopropyl-4-formyl-1-methyl-1H-pyrazol-5-yl)oxy]phenoxy}propionate

The title compound was obtained in the same manner as in Example 116 and from 3-cyclopropyl-5-(2,4-dichloro-5-hydroxyphenoxy)-1-methyl-1H-pyrazole-4-carbaldehyde obtained in Reference Example 168 and methyl (2R)-2-{[(4-methylphenyl)sulfonyl]oxy}propionate obtained in Reference Example 96.
¹H-NMR (300 MHz, CDCl₃)δ:0.96 - 0.97 (m, 4 H), 1.63 (d, J = 6.9 Hz, 3H), 2.36 - 2.38 (m, 1 H), 3.61 (s, 3 H), 3.69 (s, 3 H), 4.58 (q, J = 6.9 Hz, 1 H), 6.39 (s, 1 H), 7.48 (s, 1 H), 9.59 (s, 1 H).

### Example 216 methyl (2S)-2-[2,4-dichloro-5-({3-cyclopropyl-4-[(E)-(hydroxyimino)methyl]-1-methyl-1H-pyrazol-5-yl}oxy)phenoxy]propionate

The title compound was obtained in the same manner as in Example 140 and from methyl (2S)-2-{2,4-dichloro-5-[(3-cyclopropyl-4-formyl-1-methyl-1H-pyrazol-5-yl)oxy]phenoxy}propionate obtained in Example 215.
¹H-NMR (300 MHz, CDCl₃)δ:0.89 - 0.92 (m, 4 H), 1.60 (d, J = 6.9 Hz, 3 H), 2.02 - 2.06 (m, 1 H), 3.53 (s, 3 H), 3.67 (s, 3 H), 4.53 (q, J = 6.9 Hz, 1 H), 6.20 (s, 1 H), 7.43 (s, 1 H), 7.91 (s, 1 H), 8.97 (s, 1 H).

### Example 217 (2S)-2-[2,4-dichloro-5-({3-cyclopropyl-4-[(E)-(hydroxyimino)methyl]-1-methyl-1H-pyrazol-5-yl}oxy)phenoxy]propionic acid

The title compound was obtained in the same manner as in Example 112 and from methyl (2S)-2-[2,4-dichloro-5-({3-cyclopropyl-4-[(E)-(hydroxyimino)methyl]-1-methyl-1H-pyrazol-5-yl}oxy)phenoxy]propionate obtained in Example 216.
¹H-NMR (300 MHz, CDCl₃)δ:0-85 - 0.92 (m, 4 H), 1.63 (d, J = 6.9 Hz, 3 H), 1.84 - 1.90 (m, 1 H), 3.45 (s, 3 H), 4.46 (q, J = 6.9 Hz, 1 H), 6.12 (s, 1 H), 7.47 (s, 1 H), 8.05 (s, 1 H), 9.49 (br, 2 H).

### Example 218 (2S)-2-{2,4-dichloro-5-[(4-cyano-3-cyclopropyl-1-methyl-1H-pyrazol-5-yl)oxy]phenoxy}propionic acid

The title compound was obtained in the same manner as in Example 141 and from methyl (2S)-2-[2,4-dichloro-5-({3-cyclopropyl-4-[(E)-(hydroxyimino)methyl]-1-methyl-1H-pyrazol-5-yl}oxy)phenoxy]propionate obtained in Example 216.
¹H-NMR (300 MHz, CDCl₃)δ:0.94 (d, J = 6.9 Hz, 4 H), 1.73 (d, J = 6.9 Hz, 3 H), 1.85 (quint, J = 6.9 Hz, 1 H), 3.69 (s, 3 H), 4.76 (q, J = 6.9 Hz, 1 H), 6.84 (s, 1 H), 7.51 (s, 1 H), 8.43 (br, 1 H).

### Example 219 (2S)-2-{2,4-dichloro-5-[(3-cyclopropyl-4-ethyl-1-methyl-1H-pyrazol-5-yl)oxy]phenoxy}propionic acid

The title compound was obtained in the same manner as in Example 116 and then Example 112, and from 2,4-dichloro-5-[(3-cyclopropyl-4-ethyl-1-methyl-1H-pyrazol-5-yl)oxy]phenol obtained in Reference Example 169 and methyl (2R)-2-{[(4-methylphenyl)sulfonyl]oxy}propionate obtained in Reference Example 96.
¹H-NMR (300 MHz, CDCl₃)δ:0.75 - 0.91 (m, 4 H), 1.08 (t, J = 7.5 Hz, 3 H), 1.67 (d, J = 6.9 Hz, 3 H), 1.71 -1.80 (m, 1 H), 2.26 - 2.46 (m, 2 H), 3.49 (s, 3 H), 4.43 (q, J = 6.9 Hz, 1 H), 6.08 (s, 1 H), 7.45 (s, 1 H), 9.85 (br, 1 H).

### Example 220 (2S)-2-{2,4-dichloro-5-[(3-cyclopropyl-1,4-dimethyl-1H-pyrazol-5-yl)oxy]phenoxy}propionic acid

The title compound was obtained in the same manner as in Example 116 and then Example 112, and from 2,4-dichloro-5-[(3-cyclopropyl-1,4-dimethyl-1H-pyrazol-5-yl)oxy]phenol obtained in Reference Example 170 and methyl (2R)-2-{[(4-methylphenyl)sulfonyl]oxy}propionate obtained in Reference Example 96.
¹H-NMR (300 MHz, CDCl₃)δ:0.75 - 0.89 (m, 4 H), 1.67 (d, J = 6.9 Hz, 3 H), 1.71 - 1.80 (m, 1 H), 1.87 (s, 3 H), 3.50 (s, 3 H), 4.46 (q, J = 6.9 Hz, 1 H), 6.11 (s, 1 H), 7.45 (s, 1 H), 10.47 (br, 1 H).

### Example 221 (2S)-2-{2,4-dichloro-5-[(3-cyclopropyl-1-methyl-4-vinyl-1H-pyrazol-5-yl)oxy]phenoxy}propionic acid

The title compound was obtained in the same manner as in Example 116 and then Example 112, and from 2,4-dichloro-5-[(3-cyclopropyl-1-methyl-4-vinyl-1H-pyrazol-5-yl)oxy]phenol obtained in Reference Example 171 and methyl (2R)-2-{[(4-methylphenyl)sulfonyl]oxy}propionate obtained in Reference Example 96.
¹H-NMR (300 MHz, CDCl₃)δ:0.79 - 0.93 (m, 4 H), 1.65 (d, J = 6.9 Hz, 3 H), 1.81 - 1.90 (m, 1 H), 3.52 (s, 3 H), 4.47 (q, J = 6.9 Hz, 1 H), 5.12 (dd, J = 11.4, 1.5 Hz, 1 H), 5.43 (dd, J = 17.7, 1.5 Hz, 1 H), 6.09 (s, 1 H), 6.38 - 6.48 (m, 1 H), 7.46 (s, 1 H), 9.02 (br, 1 H).

### Example 222 (2S)-2-{2,4-dichloro-5-[(4-cyano-1-methyl-3-propyl-1H-pyrazol-5-yl)oxy]phenoxy}propionic acid

The title compound was obtained in the same manner as in Example 141 and from methyl (2S)-2-[2,4-dichloro-5-({4-[(E)-(hydroxyimino)methyl]-1-methyl-3-propyl-1H-pyrazol-5-yl}oxy)phenoxy]propionate obtained in Example 213.
¹H-NMR (300 MHz, CDCl₃)δ:0.94 (t, J = 7.2 Hz, 3 H), 1.62 - 1,69 (m, 2H), 1.72 (d, J = 6.9 Hz, 3 H), 2.57 (t, J = 7.2 Hz, 2 H), 4.76 (q, J = 6.9 Hz, 1 H), 6.84 (s, 1 H), 7.41 (br, 1 H), 7.51 (s, 1 H).

### Example 223 (2S)-2-[2,4-dichloro-5-({4-[(E)-2-(4-cyanophenyl)vinyl]-1,3-dimethyl-1H-pyrazol-5-yl}oxy)phenoxy]propionic acid

The title compound was obtained in the same manner as in Example 116 and then Example 112, and from 4-{(E)-2-[5-(2,4-dichloro-5-hydroxyphenoxy)-1,3-dimethyl-1H-pyrazol-4-yl]vinyl}benzonitrile obtained in Reference Example 172 and methyl (2R)-2-{[(4-methylphenyl)sulfonyl]oxy}propionate obtained in Reference Example 96.
¹H-NMR (300 MHz, CDCl₃)δ:1.64 (d, J = 6.9 Hz, 3 H), 2.39 (s, 3 H), 3.60 (s, 3 H), 4.41 (q, J = 6.9 Hz, 1 H), 6.13 (s, 1 H), 6.72 - 6.87 (m, 2 H), 7.42 (d, J = 8.1 Hz, 2 H), 7.52 (s, 1 H), 7.57 (d, J = 8.1 Hz, 2 H), 11.39 (br, 1 H).

### Example 224 (2S)-2-(2,4-dichloro-5-{[3-(difluoromethyl)-1-methyl-4-vinyl-1H-pyrazol-5-yl]oxy}phenoxy)propionic acid

The title compound was obtained in the same manner as in Example 116 and then Example 112, and from 2,4-dichloro-5-{[3-(difluoromethyl)-1-methyl-4-vinyl-1H-pyrazol-5-yl]oxy}phenol obtained in Reference Example 177 and methyl (2R)-2-{[(4-methylphenyl)sulfonyl]oxy}propionate obtained in Reference Example 96.
¹H-NMR (300 MHz, CDCl₃)δ:1.65 (d, J = 6.8 Hz, 3 H), 3.64 (s, 3 H), 4.54 (q, J = 6.8 Hz, 1 H), 5.19 (dd, J = 11.7, 0.9 Hz, 1 H), 5.44 (d, J = 17.9 Hz, 1 H), 6.19 (s, 1 H), 6.37 - 6.85 (m, 2 H), 7.51 (s, 1 H).

### Example 225 methyl (2S)-2-(2,4-dichloro-5-{[3-(difluoromethyl)-4-formyl-1-methyl-1H-pyrazol-5-yl]oxy}phenoxy)propionate

The title compound was obtained in the same manner as in Example 116 and from 5-(2,4-dichloro-5-hydroxyphenoxy)-3-(difluoromethyl)-1-methyl-1H-pyrazole-4-carbaldehyde obtained in Reference Example 176 and methyl (2R)-2-{[(4-methylphenyl)sulfonyl]oxy}propionate obtained in Reference Example 96.
¹H-NMR (300 MHz, CDCl₃)δ:1.64 (d, J = 6.9 Hz, 3 H), 3.69 (s, 3 H), 3.80 (s, 3 H), 4.63 (d, J = 6.9 Hz, 1 H), 6.53 (s, 1 H), 6.83 (s, 1 H), 7.49 (s, 1 H), 9.56 (s, 1 H).

### Example 226 methyl (2S)-2-[2,4-dichloro-5-({3-(difluoromethyl)-4-[(E)-(hydroxyimino)methyl]-1-methyl-1H-pyrazol-5-yl}oxy)phenoxy]propionate

The title compound was obtained in the same manner as in Example 140 and from methyl (2S)-2-(2,4-dichloro-5-{[3-(difluoromethyl)-4-formyl-1-methyl-1H-pyrazol-5-yl]oxy}phenoxy)propionate obtained in Example 225.
¹H-NMR (300 MHz, CDCl₃)δ:1.63 (d, J = 6.9 Hz, 3 H), 3.69 (s, 3 H), 3.71 (s, 3 H), 4.57 (q, J = 6.9 Hz, 1 H), 6.31 (s, 1 H), 6.70 (s, 1 H), 7.44 (s, 1 H), 7.92 (s, 1 H), 8.20 (br, 1 H).

### Example 227 (2S)-2-[2,4-dichloro-5-({3-(difluoromethyl)-4-[(E)-(hydroxyimino)methyl]-1-methyl-1H-pyrazol-5-yl}oxy)phenoxy]propionic acid

The title compound was obtained in the same manner as in Example 112 and from methyl (2S)-2-[2,4-dichloro-5-({3-(difluoromethyl)-4-[(E)-(hydroxyimino)methyl]-1-methyl-1H-pyrazol-5-yl}oxy)phenoxy]propionate obtained in Example 226.
¹H-NMR (300 MHz, CDCl₃)δ:1.64 (d, J = 6.9 Hz, 3 H), 3.59 (s, 3 H), 4.43 (q, J = 6.9 Hz, 1 H), 6.19 (s, 1 H), 6.64 (s, 1 H), 7.50 (s, 1 H), 8.11 (s, 1 H), 9.08 (br, 2 H).

### Example 228 (2S)-2-(2,4-dichloro-5-{[3-(difluoromethyl)-1,4-dimethyl-1H-pyrazol-5-yl]oxy}phenoxy)propionic acid

The title compound was obtained in the same manner as in Example 116 and then Example 112, and from 2,4-dichloro-5-{[3-(difluoromethyl)-1,4-dimethyl-1H-pyrazol-5-yl]oxy}phenol obtained in Reference Example 178 and methyl (2R)-2-{[(4-methylphenyl)sulfonyl]oxy}propionate obtained in Reference Example 96.
¹H-NMR (300 MHz, CDCl₃)δ:1.67 (d, J = 6.9 Hz, 3 H), 1.93 (s, 3 H), 3.62 (s, 3 H), 4.54 (q, J = 6.9 Hz, 1 H), 6.20 (s, 1 H), 6.57 (s, 1 H), 7.49 (s, 1 H), 8.92 (br, 1 H).

### Example 229 (2S)-2-(2,4-dichloro-5-{[3-(difluoromethyl)-4-ethyl-1-methyl-1H-pyrazol-5-yl]oxy}phenoxy)propionic acid

The title compound was obtained in the same manner as in Example 116 and then Example 112, and from 2,4-dichloro-5-{[3-(difluoromethyl)-4-ethyl-1-methyl-1H-pyrazol-5-yl]oxy}phenol obtained in Reference Example 179 and methyl (2R)-2-{[(4-methylphenyl)sulfonyl]oxy}propionate obtained in Reference Example 96.
¹H-NMR (300 MHz, CDCl₃)δ:1.07 (t, J = 7.2 Hz, 3 H), 1.66 (d, J = 6.9 Hz, 3H), 2.42 (q, J = 7.2 Hz, 2 H), 3.59 (s, 3 H), 4.50 (q, J = 6.9 Hz, 1 H), 6.14 (s, 1 H), 6.59 (s, 1 H), 7.19 (br, 1 H), 7.49 (s, 1 H).

### Example 230 (2S)-2-(2,4-dichloro-5-{[4-cyano-3-(difluoromethyl)-1-methyl-1H-pyrazol-5-yl]oxy}phenoxy)propionic acid

The title compound was obtained in the same manner as in Example 141 and from methyl (2S)-2-[2,4-dichloro-5-({3-(difluoromethyl)-4-[(E)-(hydroxyimino)methyl]-1-methyl-1H-pyrazol-5-yl}oxy)phenoxy]propionate obtained in Example 226.
¹H-NMR (300 MHz, CDCl₃)δ:1.73 (d, J = 6.9 Hz, 3 H), 3.85 (s, 3 H), 4.78 (q, J = 6.9 Hz, 1 H), 6.02 (br, 1 H), 6.53 (s, 1 H), 6.95 (s, 1 H), 7.54 (s, 1 H).

### Example 231 methyl (2S)-2-{2,4-dichloro-5-[(3-cyclopropyl-4-formyl-1-methyl-1H-pyrazol-5-yl)thio]phenoxy}propionate

The title compound was obtained in the same manner as in Example 116 and from 3-cyclopropyl-5-[(2,4-dichloro-5-hydroxyphenyl)thio]-1-methyl-1H-pyrazole-4-carbaldehyde obtained in Reference Example 181 and methyl (2R)-2-{[(4-methylphenyl)sulfonyl]oxy}propionate obtained in Reference Example 96.
¹H-NMR (300 MHz, CDCl₃)δ:1-00 - 1.05 (m, 2 H), 1.58 - 1.60 (m, 2 H), 2.59 - 2.64 (m, 1 H), 3.69 (s, 3 H), 3.75 (s, 3 H), 4.39 (q, J = 6.9 Hz, 1 H), 5.94 (s, 1 H), 7.42 (s, 1 H), 9.97 (s, 1 H).

### Example 232 methyl (2S)-2-[2,4-dichloro-5-({3-cyclopropyl-4-[(E)-(hydroxyimino)methyl]-1-methyl-1H-pyrazol-5-yl}thio)phenoxy]propionate

The title compound was obtained in the same manner as in Example 140 and from methyl (2S)-2-{2,4-dichloro-5-[(3-cyclopropyl-4-formyl-1-methyl-1H-pyrazol-5-yl)thio]phenoxy}propionate obtained in Example 231.
¹H-NMR (300 MHz, CDCl₃)δ:0.97 (d, J = 6.9 Hz, 4 H), 1.57 (d, J = 6.9 Hz, 3H), 2.28 (quint, J = 6.9 Hz, 1 H), 3.69 (s, 3 H), 3.74 (s, 3 H), 4.37 (q, J = 6.9 Hz, 1 H), 5.87 (s, 1 H), 7.38 (s, 1 H), 8.09 (br, 1 H), 8.16 (s, 1 H).

### Example 233 (2S)-2-{2,4-dichloro-5-[(4-cyano-3-cyclopropyl-1-methyl-1H-pyrazol-5-yl)thio]phenoxy}propionic acid

The title compound was obtained in the same manner as in Example 141 and from methyl (2S)-2-[2,4-dichloro-5-({3-cyclopropyl-4-[(E)-(hydroxyimino)methyl]-1-methyl-1H-pyrazol-5-yl}thio)phenoxy]propionate obtained in Example 232.
¹H-NMR (300 MHz, CDCl₃)δ:0.99 - 1.07 (m, 4 H), 1.69 (d, J = 6.9 Hz, 3H), 1.94 - 2.03 (m, 1 H), 3.79 (s, 3 H), 4.61 (q, J = 6.9 Hz, 1 H), 6.39 (s, 1 H), 7.00 (br, 1 H), 7.44 (s, 1 H).

### Example 234 methyl (2S)-2-{2-chloro-4-methyl-5-[(1,3,4-trimethyl-1H-pyrazol-5-yl)oxy]phenoxy}propionate

The title compound was obtained in the same manner as in Example 116 and from 2-chloro-4-methyl-5-[(1,3,4-trimethyl-1H-pyrazol-5-yl)oxy]phenol obtained in Reference Example 184 and methyl (2R)-2-{[(4-methylphenyl)sulfonyl]oxy}propionate obtained in Reference Example 96.
¹H-NMR (300 MHz, CDCl₃)δ:1.61 (d, J = 6.8 Hz, 3 H), 2.25 (s, 3 H), 2.28 (s, 3 H), 3.66 (s, 3 H), 3.67 (s, 3 H), 4.56 (q, J = 6.8 Hz, 1 H), 6.16 (s, 1 H), 7.24 (s, 1 H).

### Example 235 (2S)-2-{2-chloro-4-methyl-5-[(1,3,4-trimethyl-1H-pyrazol-5-yl)oxy]phenoxy}propionic acid

The title compound was obtained in the same manner as in Example 112 and from methyl (2S)-2-{2-chloro-4-methyl-5-[(1,3,4-trimethyl-1H-pyrazol-5-yl)oxy]phenoxy}propionate obtained in Example 234.
¹H-NMR (300 MHz, CDCl₃)δ:1.65 (d, J = 7.0 Hz, 3 H), 1.79 (s, 3 H), 2.17 (s, 3 H), 2.29 (s, 3 H), 3.51 (s, 3 H), 4.41 (q, J = 6.8 Hz, 1 H), 6.08 (s, 1 H), 7.22 (s, 1 H).

### Example 236 methyl (2S)-2-{4-chloro-2-ethyl-5-[(1,3,4-trimethyl-1H-pyrazol-5-yl)oxy]phenoxy}propionate

The title compound was obtained in the same manner as in Example 116 and from 4-chloro-2-ethyl-5-[(1,3,4-trimethyl-1H-pyrazol-5-yl)oxy]phenol obtained in Reference Example 190 and methyl (2R)-2-{[(4-methylphenyl)sulfonyl]oxy}propionate obtained in Reference Example 96.
¹H-NMR (300 MHz, CDCl₃)δ:1.20 (t, J = 7.5 Hz, 3 H), 1.56 (d, J = 6.7 Hz, 3 H), 1.73 (s, 3 H), 2.18 (s, 3 H), 2.62 (q, J = 7.5 Hz, 2 H), 3.56 (s, 3 H), 3.65 (s, 3 H), 4.48 (q, J = 6.7 Hz, 1 H), 6.03 (s, 1 H), 7.20 (s, 1 H).

### Example 237 (2S)-2-{4-chloro-2-ethyl-5-[(1,3,4-trimethyl-1H-pyrazol-5-yl)oxy]phenoxy}propionic acid

The title compound was obtained in the same manner as in Example 112 and from methyl (2S)-2-{4-chloro-2-ethyl-5-[(1,3,4-trimethyl-1H-pyrazol-5-yl)oxy]phenoxy}propionate obtained in Example 236.
¹H-NMR (300 MHz, CDCl₃)δ:1.20 (t, J = 7.5 Hz, 3 H), 1.61 (d, J = 6.8 Hz, 3 H), 1.82 (s, 3 H), 2.17 (s, 3 H), 2.54 - 2.72 (m, 2 H), 3.52 (s, 3 H), 4.32 - 4.43 (m, 1 H), 6.01 (s, 1 H), 7.19 (s, 1 H).

### Example 238 methyl (2S)-2-{2-chloro-4-ethyl-5-[(1,3,4-trimethyl-1H-pyrazol-5-yl)oxy]phenoxy}propionate

The title compound was obtained in the same manner as in Example 116 and from 2-chloro-4-ethyl-5-[(1,3,4-trimethyl-1H-pyrazol-5-yl)oxy]phenol obtained in Reference Example 193 and methyl (2R)-2-{[(4-methylphenyl)sulfonyl]oxy}propionate obtained in Reference Example 96.
¹H-NMR (300 MHz, CDCl₃)δ:1.25 (t, J = 7.5 Hz, 3 H), 1.59 (d, J = 6.8 Hz, 3 H), 1.70 (s, 3 H), 2.19 (s, 3 H), 2.71 (q, J = 7.5 Hz, 2 H), 3.54 (s, 3 H), 3.66 (s, 3 H), 4.51 (q, J = 6.9 Hz, 1 H), 6.08 (s, 1 H), 7.22 (s, 1 H).

### Example 239 (2S)-2-{2-chloro-4-ethyl-5-[(1,3,4-trimethyl-1H-pyrazol-5-yl)oxy]phenoxy}propionic acid

The title compound was obtained in the same manner as in Example 112 and from methyl (2S)-2-{2-chloro-4-ethyl-5-[(1,3,4-trimethyl-1H-pyrazol-5-yl)oxy]phenoxy}propionate obtained in Example 238.
¹H-NMR (300 MHz, CDCl₃)δ:1.25 (t, J = 7.5 Hz, 3 H), 1.65 (d, J = 6.8 Hz, 3 H), 1.79 (s, 3 H), 2.17 (s, 3 H), 2.71 (q, J = 7.5 Hz, 2 H), 3.52 (s, 3 H), 4.41 (q, J = 6.8 Hz, 1 H), 6.07 (s, 1 H), 7.23 (s, 1 H).

### Example 240 methyl (2S)-2-{4-chloro-5-[(4-cyano-1,3-dimethyl-1H-pyrazol-5-yl)oxy]-2-ethylphenoxy}propionate

The title compound was obtained in the same manner as in Example 116 and from 5-(2-chloro-4-ethyl-5-hydroxyphenoxy)-1,3-dimethyl-1H-pyrazole-4-carbonitrile obtained in Reference Example 195 and methyl (2R)-2-{[(4-methylphenyl)sulfonyl]oxy}propionate obtained in Reference Example 96.
¹H-NMR (300 MHz, CDCl₃)δ:1.22 (t, J = 7.4 Hz, 3 H), 1.55 (s, 3 H), 1.64 (d, J = 6.8 Hz, 3 H), 2.26 (s, 3 H), 2.68 (q, J = 7.4 Hz, 2 H), 3.74 (s, 3 H), 4.71 (q, J = 6.8 Hz, 1 H), 6.60 (s, 1 H), 7.24 (s, 1 H).

### Example 241 (2S)-2-{4-chloro-5-[(4-cyano-1,3-dimethyl-1H-pyrazol-5-yl)oxy]-2-ethylphenoxy}propionic acid

The title compound was obtained in the same manner as in Example 112 and from methyl (2S)-2-{4-chloro-5-[(4-cyano-1,3-dimethyl-1H-pyrazol-5-yl)oxy]-2-ethylphenoxy}propionate obtained in Example 240.
¹H-NMR (300 MHz, CDCl₃)δ:1.22 (t, J = 7.5 Hz, 3 H), 1.69 (d, J = 6.8 Hz, 3 H), 2.25 (s, 3 H), 2.69 (q, J = 7.3 Hz, 2 H), 3.74 (s, 3 H), 4.77 (q, J = 6.9 Hz, 1 H), 6.69 (s, 1 H), 7.26 (s, 1 H).

### Example 242 methyl (2S)-2-[2,4-dichloro-5-({1,3-dimethyl-4-[5-(trimethylsilyl)isoxazol-3-yl]-1H-pyrazol-5-yl}oxy)phenoxy]propionate

To a solution of trimethylsilylacetylene (0.04 mL) and 5% aqueous sodium hypochlorite solution (0.60 mL) in tetrahydrofuran (1 mL) was added a solution of methyl (2S)-2-[2,4-dichloro-5-({4-[(E)-(hydroxyimino)methyl]-1,3-dimethyl-1H-pyrazol-5-yl}oxy)phenoxy]propionate (300 mg) obtained in Example 103 in tetrahydrofuran (1 mL) at 0°C, and the mixture was stirred for 3 hr. Saturated aqueous sodium chloride solution was added to the reaction mixture, and the mixture was extracted with ethyl acetate. The organic layer was dried over anhydrous magnesium sulfate. After filtration, the filtrate was concentrated and the residue was subjected to silica gel column chromatography (hexane-ethyl acetate 100:0 - 60:40, v/v), the solvent was evaporated to give the title compound (110 mg, yield 88%) as a colorless oil.
¹H-NMR (300 MHz, CDCl₃)δ:0.30 (s, 9 H), 1.54 (d, J = 6.8 Hz, 3 H), 2.53 (s, 3 H), 3.58 (s, 3 H), 3.63 (s, 3 H), 4.43 (q, J = 6.8 Hz, 1 H), 6.11 (s, 1 H), 6.41 (s, 1 H), 7.48 (s, 1 H).

### Example 243 methyl (2S)-2-(2,4-dichloro-5-{[4-(isoxazol-3-yl)-1,3-dimethyl-1H-pyrazol-5-yl]oxy}phenoxy)propionate

To a solution of methyl (2S)-2-[2,4-dichloro-5-({1,3-dimethyl-4-[5-(trimethylsilyl)isoxazol-3-yl]-1H-pyrazol-5-yl}oxy)phenoxy]propionate (108 mg) obtained in Example 242 in a mixed solvent of acetonitrile (1.1 mL) - ethanol (0.4 mL) was added cesium fluoride (67 mg), and the mixture was stirred at room temperature for 15 min. The reaction mixture was concentrated, the residue was subjected to silica gel column chromatography (hexane-ethyl acetate 100:0 - 50:50, v/v), and the solvent was evaporated to give the title compound (75 mg, yield 80%) as a pale-yellow oil.
¹H-NMR (300 MHz, CDCl₃)δ:1.55 (d, J = 6.9 Hz, 3 H), 2.55 (s, 3 H), 3.58 (s, 3 H), 3.62 (s, 3 H), 4.42 (q, J = 6.8 Hz, 1 H), 6.09 (s, 1 H), 6.34 (d, J = 1.7 Hz, 1 H), 7.49 (s, 1 H), 8.32 (d, J = 1.7 Hz, 1 H).

### Example 244 (2S)-2-(2,4-dichloro-5-{[4-(isoxazol-3-yl)-1,3-dimethyl-1H-pyrazol-5-yl]oxy}phenoxy)propionic acid

The title compound was obtained in the same manner as in Example 112 and from methyl (2S)-2-(2,4-dichloro-5-{[4-(isoxazol-3-yl)-1,3-dimethyl-1H-pyrazol-5-yl]oxy}phenoxy)propionate obtained in Example 243.
¹H-NMR (300 MHz, CDCl₃)δ:1.62 (d, J = 6.8 Hz, 3 H), 2.53 (s, 3 H), 3.65 (s, 3 H), 4.40 (q, J = 6.7 Hz, 1 H), 6.09 (s, 1 H), 6.39 (d, J = 1.7 Hz, 1 H), 7.49 (s, 1 H), 8.35 (d, J = 1.7 Hz, 1 H).

### Example 245 methyl (2S)-2-[2,4-dichloro-5-({4-[5-(hydroxymethyl)isoxazol-3-yl]-1,3-dimethyl-1H-pyrazol-5-yl}oxy)phenoxy]propionate

The title compound was obtained in the same manner as in Example 242 and using methyl (2S)-2-[2,4-dichloro-5-({4-[(E)-(hydroxyimino)methyl]-1,3-dimethyl-1H-pyrazol-5-yl}oxy)phenoxy]propionate obtained in Example 103 and 3-trimethylsilyloxy-1-propyne.
¹H-NMR (300 MHz, CDCl₃)δ:1.56 (d, J = 6.6 Hz, 3 H), 2.20 (t, J = 6.6 Hz, 1 H), 2.52 (s, 3 H), 3.60 (s, 3 H), 3.62 (s, 3 H), 4.44 (q, J = 6.8 Hz, 1 H), 4.71 (d, J = 6.0 Hz, 2 H), 6.11 (s, 1 H), 6.20 (s, 1 H), 7.49 (s, 1 H).

### Example 246 (2S)-2-[2,4-dichloro-5-({4-[5-(hydroxymethyl)isoxazol-3-yl]-1,3-dimethyl-1H-pyrazol-5-yl}oxy)phenoxy]propionic acid

The title compound was obtained in the same manner as in Example 112 and from methyl (2S)-2-[2,4-dichloro-5-({4-[5-(hydroxymethyl)isoxazol-3-yl]-1,3-dimethyl-1H-pyrazol-5-yl}oxy)phenoxy]propionate obtained in Example 245.
¹H-NMR (300 MHz, CDCl₃)δ:1.25 (s, 1 H), 1.61 (d, J = 6.8 Hz, 3 H), 2.51 (s, 3 H), 3.66 (s, 3 H), 4.48 (q, J = 7.1 Hz, 1 H), 4.75 (s, 2 H), 6.18 (s, 1 H), 6.25 (s, 1 H), 7.50 (s, 1 H).

### Example 247 methyl (2S)-2-{2,4-dichloro-5-[(1,3-dimethyl-4-{5-[(trimethylsilyl)methyl]isoxazol-3-yl}-1H-pyrazol-5-yl)oxy]phenoxy}propionate

The title compound was obtained in the same manner as in Example 242 and using methyl (2S)-2-[2,4-dichloro-5-({4-[(E)-(hydroxyimino)methyl]-1,3-dimethyl-1H-pyrazol-5-yl}oxy)phenoxy]propionate obtained in Example 103 and propargyltrimethylsilane.
¹H-NMR (300 MHz, CDCl₃)δ:0.00 (s, 9 H), 1.54 (d, J = 6.8 Hz, 3 H), 2.15 (s, 2 H), 2.52 (s, 3 H), 3.60 (s, 3 H), 3.63 (s, 3 H), 4.42 (q, J = 6.8 Hz, 1 H), 5.74 (s, 1 H), 6.10 (s, 1 H), 7.47 (s, 1 H).

### Example 248 methyl (2S)-2-(2,4-dichloro-5-{[1,3-dimethyl-4-(5-methylisoxazol-3-yl)-1H-pyrazol-5-yl]oxy}phenoxy)propionate

The title compound was obtained in the same manner as in Example 243 and from methyl (2S)-2-{2,4-dichloro-5-[(1,3-dimethyl-4-{5-[(trimethylsilyl)methyl]isoxazol-3-yl}-1H-pyrazol-5-yl)oxy]phenoxy}propionate obtained in Example 247.
¹H-NMR (300 MHz, CDCl₃)δ:1.56 (d, J = 6.8 Hz, 3 H), 2.37 (s, 3 H), 2.53 (s, 3 H), 3.59 (s, 3 H), 3.62 (s, 3 H), 4.45 (q, J = 6.8 Hz, 1 H), 5.96 (d, J = 0.9 Hz, 1 H), 6.10 (s, 1 H), 7.49 (s, 1 H).

### Example 249 (2S)-2-(2,4-dichloro-5-{[1,3-dimethyl-4-(5-methylisoxazol-3-yl)-1H-pyrazol-5-yl]oxy}phenoxy)propionic acid

The title compound was obtained in the same manner as in Example 112 and from methyl (2S)-2-(2,4-dichloro-5-{[1,3-dimethyl-4-(5-methylisoxazol-3-yl)-1H-pyrazol-5-yl]oxy}phenoxy)propionate obtained in Example 248.
¹H-NMR (300 MHz, CDCl₃)δ:1.62 (d, J = 6.8 Hz, 3 H), 2.39 (s, 3 H), 2.50 (s, 3 H), 3.62 (s, 3 H), 4.40 (q, J = 6.8 Hz, 1 H), 6.01 (d, J = 0.8 Hz, 1 H), 6.07 (s, 1 H), 7.49 (s, 1 H).

### Example 250 methyl (2S)-2-[2,4-dichloro-5-({4-[5-(1-hydroxy-1-methylethyl)isoxazol-3-yl]-1,3-dimethyl-1H-pyrazol-5-yl}oxy)phenoxy]propionate

The title compound was obtained in the same manner as in Example 242 and then Example 243, and from methyl (2S)-2-[2,4-dichloro-5-({4-[(E)-(hydroxyimino)methyl]-1,3-dimethyl-1H-pyrazol-5-yl}oxy)phenoxy]propionate obtained in Example 103 and [(1,1-dimethyl-2-propynyl)oxy]trimethylsilane.
¹H-NMR (300 MHz, CDCl₃)δ:1.49 - 1.61 (m, 9 H), 2.29 (s, 1 H), 2.51 (s, 3 H), 3.60 (s, 3 H), 3.62 (s, 3 H), 4.46 (q, J = 6.8 Hz, 1 H), 6.10 (s, 1 H), 6.13 (s, 1 H), 7.49 (s, 1 H).

### Example 251 (2S)-2-[2,4-dichloro-5-({4-[5-(1-hydroxy-1-methylethyl)isoxazol-3-yl]-1,3-dimethyl-1H-pyrazol-5-yl}oxy)phenoxy]propionic acid

The title compound was obtained in the same manner as in Example 112 and from methyl (2S)-2-[2,4-dichloro-5-({4-[5-(1-hydroxy-1-methylethyl)isoxazol-3-yl]-1,3-dimethyl-1H-pyrazol-5-yl}oxy)phenoxy]propionate obtained in Example 250.
¹H-NMR (300 MHz, CDCl₃)δ:1.56 - 1.69 (m, 9 H), 2.52 (s, 3 H), 3.67 (s, 3 H), 4.48 (q, J = 6.6 Hz, 1 H), 6.13 (s, 1 H), 6.19 (s, 1 H), 7.49 (s, 1 H).

### Example 252 (2S)-2-{2-bromo-5-[(4-cyano-1,3-dimethyl-1H-pyrazol-5-yl)oxy]-4-methylphenoxy}propionic acid

The title compound was obtained in the same manner as in Example 116 and then Example 112, and from 5-(4-bromo-5-hydroxy-2-methylphenoxy)-1,3-dimethyl-1H-pyrazole-4-carbonitrile obtained in Reference Example 206 and methyl (2R)-2-{[(4-methylphenyl)sulfonyl]oxy}propionate obtained in Reference Example 96.
¹H-NMR (300 MHz, CDCl₃)δ:1.70 (d, J = 6.8 Hz, 3 H), 2.22 (s, 3 H), 2.27 (s, 3 H), 3.70 (s, 3 H), 4.72 (q, J = 6.8 Hz, 1 H), 6.59 (s, 1 H), 7.49 (s, 1 H).

### Example 253 (2S)-2-{2-bromo-5-[(4-ethyl-1,3-dimethyl-1H-pyrazol-5-yl)oxy]-4-methylphenoxy}propionic acid

The title compound was obtained in the same manner as in Example 116 and then Example 112, and from 2-bromo-5-[(4-ethyl-1,3-dimethyl-1H-pyrazol-5-yl)oxy]-4-methylphenol obtained in Reference Example 207 and methyl (2R)-2-{[(4-methylphenyl)sulfonyl]oxy}propionate obtained in Reference Example 96.
¹H-NMR (300 MHz, CDCl₃)δ:1.03 (t, J = 7.6 Hz, 3 H), 1.65 (d, J = 6.8 Hz, 3 H), 2.19 (s, 3 H), 2.21 - 2.34 (m, 5 H), 3.51 (s, 3 H), 4.38 (q, J = 6.8 Hz, 1 H), 6.02 (s, 1 H), 7.39 (s, 1 H).

### Example 254 (2S)-2-{2-bromo-5-[(3-cyclopropyl-4-ethyl-1-methyl-1H-pyrazol-5-yl)oxy]-4-methylphenoxy}propionic acid

The title compound was obtained in the same manner as in Example 116 and then Example 112, and from 2-bromo-5-[(3-cyclopropyl-4-ethyl-1-methyl-1H-pyrazol-5-yl)oxy]-4-methylphenol obtained in Reference Example 209 and methyl (2R)-2-{[(4-methylphenyl)sulfonyl]oxy}propionate obtained in Reference Example 96.
¹H-NMR (300 MHz, CDCl₃)δ:0.76 - 0.90 (m, 4 H), 1.06 (t, J = 7.6 Hz, 3 H), 1.64 (d, J = 6.8 Hz, 3 H), 1.70 - 1.82 (m, 1 H), 2.25 - 2.39 (m, 5 H), 3.46 (s, 3 H), 4.42 (q, J = 6.8 Hz, 1 H), 5.98 (s, 1 H), 7.39 (s, 1 H).

### Example 255 (2S)-2-{2-bromo-5-[(3-cyclopropyl-1,4-dimethyl-1H-pyrazol-5-yl)oxy]-4-methylphenoxy}propionic acid

The title compound was obtained in the same manner as in Example 116 and then Example 112, and from 2-bromo-5-[(3-cyclopropyl-1,4-dimethyl-1H-pyrazol-5-yl)oxy]-4-methylphenol obtained in Reference Example 211 and methyl (2R)-2-{[(4-methylphenyl)sulfonyl]oxy}propionate obtained in Reference Example 96.
¹H-NMR (300 MHz, CDCl₃)δ:0.76 - 0.89 (m, 4 H), 1.65 (d, J = 7.0 Hz, 3 H), 1.69 - 1.80 (m, 1 H), 1.84 (s, 3 H), 2.29 (s, 3 H), 3.48 (s, 3 H), 4.44 (q, J = 6.8 Hz, 1 H), 6.01 (s, 1 H), 7.39 (s, 1 H).

### Example 256 (2S)-2-{2-bromo-5-[(4-cyano-3-cyclopropyl-1-methyl-1H-pyrazol-5-yl)oxy]-4-methylphenoxy}propionic acid

To 5-(4-bromo-5-hydroxy-2-methylphenoxy)-3-cyclopropyl-1-methyl-1H-pyrazole-4-carbonitrile (3.38 g) obtained in Reference Example 213 and potassium carbonate (3.35 g) was added dimethyl sulfoxide (20 mL), and the mixture was stirred for 10 min. The reaction system was deaerated, and purged with nitrogen, and a solution of methyl (2R)-2-{[(4-methylphenyl)sulfonyl]oxy}propionate (3.01 g) obtained in Reference Example 96 in dimethyl sulfoxide (10 mL) was added carefully so that the temperature of the reaction system would not exceed 30°C. The mixture was stirred at room temperature for 15 hr, diisopropyl ether was added to the reaction mixture, and the mixture was washed with water and saturated brine. The organic layer was dried over anhydrous magnesium sulfate and filtered. The filtrate was concentrated, and the residue was subjected to silica gel column chromatography (hexane-ethyl acetate 85:15 - 75:25, v/v), and the solvent was evaporated. The obtained colorless oil was dissolved in a mixed solvent of tetrahydrofuran (30 mL) and water (9 mL), and cooled to 0°C. Lithium hydroxide monohydrate (0.773 g) was added, and the mixture was stirred at 0°C for 2 hr. The reaction mixture was neutralized with dil. hydrochloric acid, and extracted with ethyl acetate. The organic layer was washed with saturated brine, dried over anhydrous magnesium sulfate and filtered. The filtrate was concentrated, and the residue was crystallized from hexane-ethyl acetate to give the title compound as a colorless amorphous compound (3.63 g, yield 89%).
¹H-NMR (300 MHz, CDCl₃)δ:0.92 - 0.99 (m, 4 H), 1.70 (d, J = 6.8 Hz, 3 H), 1.81 - 1.93 (m, 1 H), 2.22 (s, 3 H), 3.65 (s, 3 H), 4.72 (q, J = 6.9 Hz, 1 H), 6.58 (s, 1 H), 7.48 (s, 1 H).

### Example 257 2-(2,4-dichloro-5-{[4-(difluoromethyl)-1,3-dimethyl-1H-pyrazol-5-yl]oxy}phenoxy)propionic acid

To a solution of ethyl 2-{2,4-dichloro-5-[(4-formyl-1,3-dimethyl-1H-pyrazol-5-yl)oxy]phenoxy}propionate (100 mg) obtained in Example 2 in toluene (3 mL) was added (diethylamino)sulfur trifluoride (0.046 mL), and the mixture was stirred at 80°C for 12 hr. Further, (diethylamino)sulfur trifluoride (0.046 mL) was added, and the mixture was stirred at 80°C for 12 hr. The reaction mixture was cooled to room temperature, aqueous sodium hydrogen carbonate solution was added, and the mixture was extracted with ethyl acetate. The organic layer was washed with saturated brine, and dried over anhydrous magnesium sulfate. After filtration, the filtrate was concentrated and the residue was purified by preparative HPLC (apparatus and preparative conditions were similar to those in Reference Example 94). The purification product was dissolved in a mixed solvent of tetrahydrofuran (3.5 mL) and water (1.5 mL), and cooled to 0°C. Lithium hydroxide monohydrate (0.042 g) was added, and the mixture was stirred at room temperature for 5 hr. The reaction mixture was neutralized with 0.1N hydrochloric acid, and extracted with ethyl acetate. The organic layer was washed with saturated brine, dried over anhydrous magnesium sulfate and filtered. The filtrate was concentrated, and the residue was crystallized from hexane-ethyl acetate to give the title compound as colorless crystals (0.052 g, yield 53%).
¹H-NMR (300 MHz, CDCl₃)δ:1.67 (d, J = 6.8 Hz, 3 H), 2.34 (s, 3 H), 3.54 (s, 3 H), 4.52 (q, J = 6.8 Hz, 1 H), 6.21 (s, 1 H), 6.46 (t, J = 54.5 Hz, 1 H), 7.49 (s, 1 H).

### Example 258 ethyl (2E)-3-{5-chloro-2-(methoxymethoxy)-4-[(1,3,4-trimethyl-1H-pyrazol-5-yl)oxy]phenyl}acrylate

To a solution of diethylphosphonoethyl acetate (1.66 g) cooled to 0°C in an ice bath in tetrahydrofuran (15 mL) was added 60% sodium hydride (296 mg), and the mixture was stirred at 0°C for 1 hr. To the reaction mixture was added dropwise at 0°C a solution of 5-chloro-2-(methoxymethoxy)-4-[(1,3,4-trimethyl-1H-pyrazol-5-yl)oxy]benzaldehyde (2.00 g) obtained in Reference Example 214 in tetrahydrofuran (25 mL). The reaction mixture was stirred at 0°C for 3 hr, poured into aqueous ammonium chloride solution, and the mixture was extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 80:20 - 40:60, v/v) to give the title compound (2.40 g, yield 99%) as crystals. Crystallization from hexane-diisopropyl ether gave the title compound as colorless crystals.
¹H-NMR (300 MHz, CDCl₃)δ:1.34 (t, J = 7.2 Hz, 3 H), 1.76 (s, 3 H), 2.19 (s, 3 H), 3.41 (s, 3 H), 3.60 (s, 3 H), 4.26 (q, J = 7.1 Hz, 2 H), 5.08 (s, 2 H), 6.43 (d, J = 16.2 Hz, 1 H), 6.56 (s, 1 H), 7.60 (s, 1 H), 7.88 (d, J = 16.2 Hz, 1 H).

### Example 259 methyl (2S)-2-{4-chloro-2-(3-hydroxypropyl)-5-[(1,3,4-trimethyl-1H-pyrazol-5-yl)oxy]phenoxy}propionate

The title compound was obtained in the same manner as in Example 116 and from 4-chloro-2-(3-hydroxypropyl)-5-[(1,3,4-trimethyl-1H-pyrazol-5-yl)oxy]phenol obtained in Reference Example 216 and methyl (2R)-2-{[(4-methylphenyl)sulfonyl]oxy}propionate obtained in Reference Example 96.
¹H-NMR (300 MHz, CDCl₃)δ:1.55 (d, J = 6.8 Hz, 3 H), 1.73 (s, 3 H), 1.77 - 1.97 (m, 2 H), 2.18 (s, 3 H), 2.60 - 2.84 (m, 2 H), 3.56 (s, 3 H), 3.63 (s, 2 H), 3.67 (s, 3 H), 4.52 (q, J = 6.8 Hz, 1 H), 6.04 (s, 1 H), 7.22 (s, 1 H).

### Example 260 (2S)-2-{4-chloro-2-(3-hydroxypropyl)-5-[(1,3,4-trimethyl-1H-pyrazol-5-yl)oxy]phenoxy}propionic acid

The title compound was obtained in the same manner as in Example 112 and from methyl (2S)-2-{4-chloro-2-(3-hydroxypropyl)-5-[(1,3,4-trimethyl-1H-pyrazol-5-yl)oxy]phenoxy}propionate obtained in Example 259.
¹H-NMR (300 MHz, CDCl₃)δ:1.58 (d, J = 6.8 Hz, 3 H), 1.81 (s, 3 H), 1.83 - 1.98 (m, 2 H), 2.17 (s, 3 H), 2.57 - 2.69 (m, 1 H), 2.76 - 2.88 (m, 1 H), 3.51 (s, 3 H), 3.53 - 3.69 (m, 2 H), 4.45 (q, J = 6.9 Hz, 1 H), 6.03 (s, 1 H), 7.21 (s, 1 H).

### Example 261 (2S)-2-{4-chloro-2-(3-phenoxypropyl)-5-[(1,3,4-trimethyl-1H-pyrazol-5-yl)oxy]phenoxy}propionic acid

To a solution, which was cooled to 0°C in an ice bath, of methyl (2S)-2-{4-chloro-2-(3-hydroxypropyl)-5-[(1,3,4-trimethyl-1H-pyrazol-5-yl)oxy]phenoxy}propionate (86 mg) obtained in Example 259 phenol (25 mg) and tributylphosphine (0.157 mL) in tetrahydrofuran (3 mL) was added 1,1'-(azodicarbonyl)dipiperidine (164 mg). This mixture was stirred at room temperature for 15 hr. To the reaction mixture was added hexane, and the insoluble material was removed by filtration. The filtrate was concentrated, and the residue was subjected to silica gel column chromatography (hexane-ethyl acetate 75:25 - 50:50, v/v). The purification product was dissolved in a mixed solvent of tetrahydrofuran (3.5 mL) and water (1.5 mL), and cooled to 0°C. Lithium hydroxide monohydrate (0.027 g) was added, and the mixture was stirred at 0°C for 2 hr. The reaction mixture was neutralized with 0.1N hydrochloric acid, and extracted with ethyl acetate. The organic layer was washed with saturated brine, dried over anhydrous magnesium sulfate and filtered. The filtrate was concentrated, and the residue was crystallized from hexane-ethyl acetate to give the title compound as colorless crystals (0.025 g, yield 25%).
¹H-NMR (300 MHz, CDCl₃)δ:1.57 (d, J = 6.8 Hz, 3 H), 1.83 (s, 3 H), 2.01 - 2.24 (m, 5 H), 2.67 - 2.89 (m, 2 H), 3.49 (s, 3 H), 3.99 (t, J = 6.4 Hz, 2 H), 4.36 (q, J = 7.0 Hz, 1 H), 5.99 (s, 1 H), 6.84 - 6.97 (m, 3 H), 7.18 - 7.30 (m, 3 H).

### Example 262 (2S)-2-{4-chloro-2-[3-(methylsulfonyl)propyl]-5-[(1,3,4-trimethyl-1H-pyrazol-5-yl)oxy]phenoxy}propionic acid

To a solution, which was cooled to 0°C in an ice bath, of 3-{5-chloro-2-(methoxymethoxy)-4-[(1,3,4-trimethyl-1H-pyrazol-5-yl)oxy]phenyl}propan-1-ol (140 mg) obtained in Reference Example 215 and triethylamine (0.167 mL) in ethyl acetate (10 mL) was added methanesulfonyl chloride (0.061 mL). This mixture was stirred at room temperature for 15 hr. Water was added to the reaction mixture, and the mixture was extracted with ethyl acetate. The organic layer was washed with saturated brine, dried over anhydrous magnesium sulfate and filtered. The filtrate was concentrated, and the residue was dissolved in N,N-dimethylformamide (5 mL). Sodium methanesulfinate (405 mg) was added, and the mixture was stirred at 110°C for 4 hr. The reaction mixture was cooled to room temperature, water was added, and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 67:33 - 33:67, v/v). The purification product was dissolved in methanol (5 mL), concentrated hydrochloric acid (0.2 mL) was added, and the mixture was stirred at 60°C for 5 hr. The reaction mixture was cooled to room temperature, water was added, and the mixture was extracted with ethyl acetate. The organic layer was washed successively with aqueous sodium hydrogen carbonate solution and saturated brine. The organic layer was dried over anhydrous magnesium sulfate and filtered. The filtrate was concentrated, and the residue was dissolved in dimethyl sulfoxide (4 mL). Methyl (2R)-2-{[(4-methylphenyl)sulfonyl]oxy}propionate (154 mg) obtained in Reference Example 96 and potassium carbonate (164 mg) were added, and the mixture was stirred at room temperature for 15 hr. Water was added to the reaction mixture, and the mixture was extracted with ethyl acetate. The organic layer was washed with saturated brine, dried over anhydrous magnesium sulfate and filtered. The filtrate was concentrated, and the residue was purified by preparative HPLC (apparatus and preparative conditions were similar to those in Reference Example 94). The purification product was dissolved in a mixed solvent of tetrahydrofuran (3.5 mL) and water (1.5 mL), and cooled to 0°C. Lithium hydroxide monohydrate (0.015 g) was added, and the mixture was stirred at 0°C for 2 hr. The reaction mixture was neutralized with 0.1N hydrochloric acid, and extracted with ethyl acetate. The organic layer was washed with saturated brine, dried over anhydrous magnesium sulfate and filtered. The filtrate was concentrated, and the residue was crystallized from hexane-ethyl acetate to give the title compound as colorless crystals (0.034 g, yield 19%).
¹H-NMR (300 MHz, CDCl₃)δ:1.61 (d, J = 6.8 Hz, 3 H), 1.84 (s, 3 H), 2.05 - 2.32 (m, 5 H), 2.69 - 2.85 (m, 2 H), 2.87 (s, 3 H), 2.98 - 3.08 (m, 2 H), 3.49 (s, 3 H), 4.41 (q, J = 7.0 Hz, 1 H), 5.99 (s, 1 H), 7.19 (s, 1 H).

### Example 263 (2S)-2-{4-chloro-2-(phenoxymethyl)-5-[(1,3,4-trimethyl-1H-pyrazol-5-yl)oxy]phenoxy}propionic acid

The title compound was obtained in the same manner as in Example 116 and then Example 112, and from 4-chloro-2-(phenoxymethyl)-5-[(1,3,4-trimethyl-1H-pyrazol-5-yl)oxy]phenol obtained in Reference Example 219 and methyl (2R)-2-{[(4-methylphenyl)sulfonyl]oxy}propionate obtained in Reference Example 96.
¹H-NMR (300 MHz, CDCl₃)δ:1.61 (d, J = 7.0 Hz, 3 H), 1.85 (s, 3 H), 2.18 (s, 3 H), 3.53 (s, 3 H), 4.45 (q, J = 6.8 Hz, 1 H), 5.00 - 5.17 (m, 2 H), 6.07 (s, 1 H), 6.93 - 7.04 (m, 3 H), 7.27 - 7.34 (m, 2 H), 7.57 (s, 1 H).

### Example 264 methyl (2S)-2-{2-bromo-4-methyl-5-[(1,3,4-trimethyl-1H-pyrazol-5-yl)oxy]phenoxy}propionate

The title compound was obtained in the same manner as in Example 116 and from 2-bromo-4-methyl-5-[(1,3,4-trimethyl-1H-pyrazol-5-yl)oxy]phenol obtained in Reference Example 221 and methyl (2R)-2-{[(4-methylphenyl)sulfonyl]oxy}propionate obtained in Reference Example 96.
¹H-NMR (300 MHz, CDCl₃)δ:1.62 (s, 3 H), 1.71 (s, 3 H), 2.18 (s, 3 H), 2.29 (s, 3 H), 3.53 (s, 3 H), 3.66 (s, 3 H), 4.50 (q, J = 6.8 Hz, 1 H), 6.06 (s, 1 H), 7.38 (s, 1 H).

### Example 265 (2S)-2-{2-cyclopropyl-4-methyl-5-[(1,3,4-trimethyl-1H-pyrazol-5-yl)oxy]phenoxy}propionic acid

A mixture of methyl (2S)-2-{2-bromo-4-methyl-5-[(1,3,4-trimethyl-1H-pyrazol-5-yl)oxy]phenoxy}propionate (150 mg) obtained in Example 264, cyclopropylboric acid (65 mg), potassium carbonate (313 mg) and toluene (10 mL) was stirred under an argon atmosphere at room temperature for 0.5 hr. To the reaction mixture were added tris(dibenzylideneacetone)dipalladium(0) (9 mg) and 2-dicyclohexylphosphino-2',6'-dimethoxybiphenyl (16 mg), and the mixture was stirred under an argon atmosphere at 100°C for 18 hr. The reaction mixture was cooled to room temperature, water was added, and the mixture was extracted with ethyl acetate. The organic layer was washed with saturated brine, dried over anhydrous magnesium sulfate and filtered. The filtrate was concentrated, and the residue was purified by preparative HPLC (apparatus and preparative conditions were similar to those in Reference Example 94). The purification product was dissolved in a mixed solvent of tetrahydrofuran (7 mL) and water (3 mL), and cooled to 0°C. Lithium hydroxide monohydrate (0.032 g) was added, and the mixture was stirred at 0°C for 2 hr. The reaction mixture was neutralized with 0.1N hydrochloric acid, and extracted with ethyl acetate. The organic layer was washed with saturated brine, dried over anhydrous magnesium sulfate and filtered. The filtrate was concentrated, and the residue was crystallized from hexane-ethyl acetate to give the title compound as colorless crystals (0.100 g, yield 77%).
¹H-NMR (300 MHz, CDCl₃)δ:0.52 - 0.75 (m, 2 H), 0.84 - 0.96 (m, 2 H), 1.61 (d, J = 6.8 Hz, 3 H), 1.77 (s, 3 H), 2.03 - 2.19 (m, 4 H), 2.26 (s, 3 H), 3.52 (s, 3 H), 4.41 (q, J = 6.8 Hz, 1 H), 5.99 (s, 1 H), 6.67 (s, 1 H).

### Example 266 (2S)-2-({4'-cyano-5-methyl-4-[(1,3,4-trimethyl-1H-pyrazol-5-yl)oxy]biphenyl-2-yl}oxy)propionic acid

A mixture of methyl (2S)-2-{2-bromo-4-methyl-5-[(1,3,4-trimethyl-1H-pyrazol-5-yl)oxy]phenoxy}propionate (150 mg) obtained in Example 264, 4-cyanophenylboric acid (111 mg), potassium fluoride (88 mg), acetonitrile (7 mL) and water (3 mL) was stirred under an argon atmosphere at room temperature for 0.5 hr. To the reaction mixture was added tetrakis(triphenylphosphine)palladium(0) (26 mg) and the mixture was heated under reflux under an argon atmosphere for 18 hr. The reaction mixture was cooled to room temperature, water was added, and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 75:25 - 40:60, v/v). The purification product was dissolved in a mixed solvent of tetrahydrofuran (7 mL) and water (3 mL), and cooled to 0°C. Lithium hydroxide monohydrate (0.032 g) was added, and the mixture was stirred at 0°C for 1.5 hr. The reaction mixture was neutralized with 0.1N hydrochloric acid, and extracted with ethyl acetate. The organic layer was washed with saturated brine, dried over anhydrous magnesium sulfate and filtered. The filtrate was concentrated, and the residue was crystallized from hexane-ethyl acetate to give the title compound as colorless crystals (0.077 g, yield 51%).
¹H-NMR (300 MHz, CDCl₃)δ:1.50 (d, J = 6.8 Hz, 3 H), 1.86 (s, 3 H), 2.20 (s, 3 H), 2.36 (s, 3 H), 3.54 (s, 3 H), 4.33 - 4.43 (m, 1 H), 6.04 (s, 1 H), 7.19 (s, 1 H), 7. 61 - 7.75 (m, 4 H).

### Example 267 (2S)-2-({4'-fluoro-5-methyl-4-[(1,3,4-trimethyl-1H-pyrazol-5-yl)oxy]biphenyl-2-yl}oxy)propionic acid

The title compound was obtained in the same manner as in Example 266 and from methyl (2S)-2-{2-bromo-4-methyl-5-[(1,3,4-trimethyl-1H-pyrazol-5-yl)oxy]phenoxy}propionate obtained in Example 264 and 4-fluorophenylboric acid.
¹H-NMR (300 MHz, DMSO-d₆)δ:1.34 (d, J = 6.6 Hz, 3 H), 1.67 (s, 3 H), 2.07 (s, 3 H), 2.29 (s, 3 H), 3.48 (s, 3 H), 4.50 (q, J = 6.8 Hz, 1 H), 6.12 (s, 1 H), 7.17 - 7.26 (m, 3 H), 7.55 - 7.65 (m, 2 H), 13.04 (s, 1 H).

### Example 268 (2S)-2-{2-bromo-4-methyl-5-[(1,3,4-trimethyl-1H-pyrazol-5-yl)oxy]phenoxy}propionic acid

The title compound was obtained in the same manner as in Example 112 and from methyl (2S)-2-{2-bromo-4-methyl-5-[(1,3,4-trimethyl-1H-pyrazol-5-yl)oxy]phenoxy}propionate obtained in Example 264.
¹H-NMR (300 MHz, CDCl₃)δ:1.65 (d, J = 7.0 Hz, 3 H), 1.79 (s, 3 H), 2.17 (s, 3 H), 2.30 (s, 3 H), 3.51 (s, 3 H), 4.40 (q, J = 6.9 Hz, 1 H), 6.04 (s, 1 H), 7.39 (s, 1 H).

### Example 269 (2S)-2-({5-methyl-4-[(1,3,4-trimethyl-1H-pyrazol-5-yl)oxy]biphenyl-2-yl}oxy)propionic acid

The title compound was obtained in the same manner as in Example 266 and from methyl (2S)-2-{2-bromo-4-methyl-5-[(1,3,4-trimethyl-1H-pyrazol-5-yl)oxy]phenoxy}propionate obtained in Example 264 and phenylboric acid.
¹H-NMR (300 MHz, CDCl₃)δ:1.46 (d, J = 6.8 Hz, 3 H), 1.84 (s, 3 H), 2.19 (s, 3 H), 2.35 (s, 3 H), 3.55 (s, 3 H), 4.32 (q, J = 6.7 Hz, 1 H), 6.11 (s, 1 H), 7.20 (s, 1 H), 7.25 - 7.33 (m, 1 H), 7.39 (t, J = 7.3 Hz, 2 H), 7.57 (d, J = 7.2 Hz, 2 H).

### Example 270 methyl (2S)-2-{4-methyl-2-(4,4,5,5-tetramethyl-1,3,2-dioxaborolan-2-yl)-5-[(1,3,4-trimethyl-1H-pyrazol-5-yl)oxy]phenoxy}propionate

A mixture of methyl (2S)-2-{2-bromo-4-methyl-5-[(1,3,4-trimethyl-1H-pyrazol-5-yl)oxy]phenoxy}propionate (500 mg) obtained in Example 264, [1,1'-bis(diphenylphosphino)ferrocene]palladium (II) dichloride dichloromethane complex (51 mg), bis(pinacolato)diboron (352 mg), potassium acetate (371 mg) and dimethyl sulfoxide (5 mL) was stirred under an argon atmosphere at 90°C for 48 hr. The reaction mixture was cooled to room temperature, and insoluble material was removed by filtration. Water was added to the filtrate, and the mixture was extracted with ethyl acetate. The organic layer was washed with saturated brine, dried over anhydrous magnesium sulfate and filtered, and the filtrate was concentrated. The residue was purified by preparative HPLC (apparatus and preparative conditions were similar to those in Reference Example 94) to give the title compound as a colorless oil (0.122 g, yield 22%).
¹H-NMR (300 MHz, CDCl₃)δ:1.35 (s, 12 H), 1.55 - 1.64 (m, 6 H), 2.26 (s, 3 H), 2.32 (s, 3 H), 3.67 (s, 3 H), 3.82 (s, 3 H), 4.57 (q, J = 6.8 Hz, 1 H), 6.25 (s, 1 H), 7.58 (s, 1 H).

### Example 271 (2S)-2-{4-methyl-2-(1,3-thiazol-2-yl)-5-[(1,3,4-trimethyl-1H-pyrazol-5-yl)oxy]phenoxy}propionic acid

The title compound was obtained in the same manner as in Example 266 and from methyl (2S)-2-{4-methyl-2-(4,4,5,5-tetramethyl-1,3,2-dioxaborolan-2-yl)-5-[(1,3,4-trimethyl-1H-pyrazol-5-yl)oxy]phenoxy}propionate obtained in Example 270 and 2-bromothiazole.
¹H-NMR (300 MHz, CDCl₃)δ:1.67 (d, J = 6.8 Hz, 3 H), 1.79 (s, 3 H), 2.22 (s, 3 H), 2.39 (s, 3 H), 3.58 (s, 3 H), 4.67 (q, J = 7.0 Hz, 1 H), 6.22 (s, 1 H), 7.37 (d, J = 3.4 Hz, 1 H), 7.76 (s, 1 H), 7.88 (d, J = 3.4 Hz, 1 H).

### Example 276 {2,4-dichloro-5-[(4-cyano-1,3-dimethyl-1H-pyrazol-5-yl)thio]phenoxy}acetic acid

The title compound was obtained in the same manner as in Example 274 and from 5-[(2,4-dichloro-5-hydroxyphenyl)thio]-1,3-dimethyl-1H-pyrazole-4-carbonitrile obtained in Reference Example 237 and ethyl bromoacetate.
¹H-NMR (300 MHz, DMSO-d₆)δ:2.32 (s, 3 H), 3.80 (s, 3 H), 4.71 (s, 2 H), 6.38 (s, 1 H), 7.80 (s, 1 H), 13.24 (br s, 1 H).

### Example 277 2-{2,4-dichloro-5-[(4-cyano-1,3-dimethyl-1H-pyrazol-5-yl)thio]phenoxy}-2-methylpropionic acid

To a suspension of 5-[(2,4-dichloro-5-hydroxyphenyl)thio]-1,3-dimethyl-1H-pyrazole-4-carbonitrile (1.00 g) obtained in Reference Example 237 and 2-bromo-2-methylpropionic acid (800 mg) in 2-butanone (10 mL) was added potassium hydroxide (981 mg) at room temperature with stirring, and the mixture was stirred at 50°C for 14 hr. The reaction mixture was cooled to room temperature, neutralized with 1N hydrochloric acid, and the mixture was extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 9:1 - 3:7, v/v), and crystallized from heptane-ethyl acetate to give the title compound as white crystals (1.05 g, yield 82%).
¹H-NMR (300 MHz, DMSO-d₆)δ:1.43 (s, 6 H), 2.34 (s, 3 H), 3.81 (s, 3 H), 6.20 (s, 1 H), 7.81 (s, 1 H), 13.27 (s, 1 H).

### Example 278 ethyl {2,4-dichloro-5-[(4-cyano-1,3-dimethyl-1H-pyrazol-5-yl)thio]phenoxy}(fluoro)acetate

The title compound was obtained in the same manner as in Example 16 and from 5-[(2,4-dichloro-5-hydroxyphenyl)thio]-1,3-dimethyl-1H-pyrazole-4-carbonitrile obtained in Reference Example 237 and ethyl bromofluoroacetate.
¹H-NMR (300 MHz, CDCl₃)δ:1.35 (t, J = 7.2 Hz, 3 H), 2.40 (s, 3 H), 3.89 (s, 3 H), 4.35 (q, J = 7.2 Hz, 2 H), 5.81 (d, J = 58.4 Hz, 1 H), 6.86 (d, J = 0.8 Hz, 1 H), 7.52 (s, 1 H).

### Example 279 {2,4-dichloro-5-[(4-cyano-1,3-dimethyl-1H-pyrazol-5-yl)thio]phenoxy}(fluoro)acetic acid

The title compound was obtained in the same manner as in Example 37 and from ethyl {2,4-dichloro-5-[(4-cyano-1,3-dimethyl-1H-pyrazol-5-yl)thio]phenoxy}(fluoro)acetate obtained in Example 278.
¹H-NMR (300 MHz, DMSO-d₆)δ:2.32 (s, 3 H), 3.84 (s, 3 H), 6.32 (d, J = 57.3 Hz, 1 H), 6.84 (s, 1 H), 7.94 (s, 1 H), 14.39 (br s, 1 H).

### Example 280 (2S)-2-{2,4-dichloro-5-[(4-formyl-1,3-dimethyl-1H-pyrazol-5-yl)oxy]phenoxy}propionic acid

The title compound was obtained in the same manner as in Example 37 and from methyl (2S)-2-{2,4-dichloro-5-[(4-formyl-1,3-dimethyl-1H-pyrazol-5-yl)oxy]phenoxy}propionate obtained in Example 102.
¹H-NMR (300 MHz, DMSO-d₆)δ:1.47 (d, J = 6.8 Hz, 3 H), 2.34 (s, 3 H), 3.61 (s, 3 H), 4.94 (q, J = 6.7 Hz, 1 H), 6.80 (s, 1 H), 7.81 (s, 1 H), 9.35 (s, 1 H), 13.20 (br s, 1 H).

### Example 281 (2S)-2-[2,4-dichloro-5-({4-[(Z)-(2,4-dioxo-1,3-thiazolidin-5-ylidene)methyl]-1,3-dimethyl-1H-pyrazol-5-yl}oxy)phenoxy]propionic acid

To a solution of (2S)-2-{2,4-dichloro-5-[(4-formyl-1,3-dimethyl-1H-pyrazol-5-yl)oxy]phenoxy}propionic acid (0.92 g) obtained in Example 280 in ethanol (10 mL) were added piperidine (1.25 mL) and 1,3-thiazolidine-2,4-dione (1.16 g) and the mixture was heated under reflux with stirring for 3 hr. The reaction mixture was cooled to room temperature, neutralized with 1N hydrochloric acid, and the mixture was extracted with ethyl acetate. The organic layer was washed with saturated brine, and dried over anhydrous magnesium sulfate. After filtration, the filtrate was concentrated and the residue was subjected to silica gel column chromatography (ethyl acetate alone - ethyl acetate:methanol 4:1, v/v), subjected to silica gel column chromatography (hexane-ethyl acetate 4:1 - ethyl acetate alone, v/v), and crystallized from heptane-ethyl acetate to give the title compound as 0.40 heptane solvate in the form of white crystals (0.60 g, yield 47%).
¹H-NMR (300 MHz, DMSO-d₆)δ:1.44 (d, J = 6.6 Hz, 3 H), 2.27 (s, 3 H), 3.46 (s, 3 H), 4.77 (q, J = 6.8 Hz, 1 H), 6.17 (s, 1 H), 7.40 (s, 1 H), 7.84 (s, 1 H), 12.34 (br s, 1 H), 13.22 (br s, 1 H).

### Example 282 (2S)-2-[2,4-dichloro-5-({1,3-dimethyl-4-[(phenoxyimino)methyl]-1H-pyrazol-5-yl}oxy)phenoxy]propionic acid

The title compound was obtained in the same manner as in Example 103 and then Example 37, and from methyl (2S)-2-{2,4-dichloro-5-[(4-formyl-1,3-dimethyl-1H-pyrazol-5-yl)oxy]phenoxy}propionate obtained in Example 102 and O-phenylhydroxylamine hydrochloride, as a mixture of E:Z=5:1.
¹H-NMR (300 MHz, DMSO-d₆)δ:1.42 (d, J = 6.8 Hz, 3/2 H), 1.52 (d, J = 6.8 Hz, 3/2 H), 2.17 (s, 3/2 H), 2.33 (s, 3/2 H), 3.62 (s, 3/2 H), 3.70 (s, 3/2 H), 4.83 (q, J = 6.8 Hz, 1/2 H), 5.02 (q, J = 6.8 Hz, 1/2 H), 6.52 (s, 1 H), 6.79 (d, J = 7.7 Hz, 2 H), 6.96 (t, J = 7.3 Hz, 1 H), 7.14 - 7.27 (m, 2 H), 7.81 (s, 1/2 H), 7.86 (s, 1/2 H), 8.21 (s, 1 H), 13.19 (br s, 1 H).

### Example 283 methyl (2S)-2-(5-{[4-((E)-2-{[(tert-butoxycarbonyl)amino]sulfonyl}vinyl)-1,3-dimethyl-1H-pyrazol-5-yl]oxy}-2,4-dichlorophenoxy)propionate and

### Example 284 methyl (2S)-2-[5-({4-[(E)-2-(aminosulfonyl)vinyl]-1,3-dimethyl-1H-pyrazol-5-yl}oxy)-2,4-dichlorophenoxy]propionate

To a solution of tert-butyl {[(diphenylphosphoryl)methyl]sulfonyl}carbamate (5.00 g) in N,N-dimethylformamide (25 mL) was added sodium hydride (60% in oil, 2.52 g) at 0°C, and the mixture was stirred for 1 hr. 5-(2,4-Dichloro-5-hydroxyphenoxy)-1,3-dimethyl-1H-pyrazole-4-carbaldehyde (3.60 g) obtained in Reference Example 52 was added to the reaction mixture, and the mixture was stirred for 13 hr while allowing to warm to room temperature. Saturated aqueous ammonium chloride solution was added to the reaction mixture, and the mixture was diluted with water and extracted with ethyl acetate. The organic layer was washed with water and saturated brine, and dried over anhydrous magnesium sulfate. After filtration, the filtrate was concentrated and the residue was subjected to silica gel column chromatography (hexane-ethyl acetate 4:1 - ethyl acetate alone, v/v). The residue was dissolved in ethyl acetate, and the organic layer was washed with water and saturated brine, and dried over anhydrous magnesium sulfate. After filtration, the filtrate was concentrated and crystallized from hexane to give a white solid. The obtained white solid and potassium carbonate (5.36 g) were suspended in dimethyl sulfoxide (25 mL) and placed in an ice bath. A solution of methyl (2R)-2-{[(4-methylphenyl)sulfonyl]oxy}propionate (2.40 g) obtained in Reference Example 96 in dimethyl sulfoxide (25 mL) was added carefully so that the temperature of the reaction system would not exceed 30°C. The mixture was stirred at room temperature for 4 hr, saturated aqueous ammonium chloride solution was added, and the mixture was diluted with water, and extracted with ethyl acetate. The organic layer was washed with water and saturated brine, dried over anhydrous magnesium sulfate and filtered. The filtrate was concentrated, and the residue was subjected to silica gel column chromatography (hexane-ethyl acetate 9:1 - ethyl acetate alone, v/v) to give the title compound (Example 283) as a white solid (3.09 g, yield 46%) from a fraction with a short retention time. Subsequently, the title compound (Example 284) was obtained as a yellow oil (0.34 g, yield 6%) from a fraction with a long retention time. Example 283 (small retention time)
¹H-NMR (300 MHz, CDCl₃)δ:1.43 (s, 9 H), 1.61 (d, J = 6.8 Hz, 3 H), 2.37 (s, 3 H), 3.57 (s, 3 H), 3.67 (s, 3 H), 4.53 (q, J = 6.7 Hz, 1 H), 6.15 (s, 1 H), 6.55 (d, J = 15.4 Hz, 1 H), 7.08 (br s, 1 H), 7.35 (d, J= 15.4 Hz, 1 H), 7.51 (s, 1 H). Example 284 (long retention time)
¹H-NMR (300 MHz, CDCl₃)δ:1.62 (d, J = 6.8 Hz, 3 H), 2.35 (s, 3 H), 3.62 (s, 3 H), 3.73 (s, 3 H), 4.54 (q, J = 6.7 Hz, 1 H), 4.79 (s, 2 H), 6.13 (s, 1 H), 6.33 (d, J = 15.6 Hz, 1 H), 7.18 (d, J = 15.4 Hz, 1 H), 7.53 (s, 1 H).

### Example 285 (2S)-2-(5-{[4-((E)-2-{[(tert-butoxycarbonyl)amino]sulfonyl}vinyl)-1,3-dimethyl-1H-pyrazol-5-yl]oxy}-2,4-dichlorophenoxy)propionic acid

The title compound was obtained in the same manner as in Example 37 and from methyl (2S)-2-(5-{[4-((E)-2-{[(tert-butoxycarbonyl)amino]sulfonyl}vinyl)-1,3-dimethyl-1H-pyrazol-5-yl]oxy}-2,4-dichlorophenoxy)propionate obtained in Example 283.
¹H-NMR (300 MHz, DMSO-d₆)δ:1.32 (s, 9 H), 1.45 (d, J = 6.8 Hz, 3 H), 2.28 (s, 3 H), 3.53 (s, 3 H), 4.81 (q, J = 6.5 Hz, 1 H), 6.37 (s, 1 H), 6.49 (d, J = 15.6 Hz, 1 H), 7.09 (d, J = 15.4 Hz, 1 H), 7.84 (s, 1 H), 11.18 (br s, 1 H), 13.26 (br s, 1 H).

### Example 286 (2S)-2-[5-({4-[(E)-2-(aminosulfonyl)vinyl]-1,3-dimethyl-1H-pyrazol-5-yl}oxy)-2,4-dichlorophenoxy]propionic acid

The title compound was obtained in the same manner as in Example 37 and from methyl (2S)-2-[5-({4-[(E)-2-(aminosulfonyl)vinyl]-1,3-dimethyl-1H-pyrazol-5-yl}oxy)-2,4-dichlorophenoxy]propionate obtained in Example 284.
¹H-NMR (300 MHz, DMSO-d₆)δ:1.45 (d, J = 6.8 Hz, 3 H), 2.27 (s, 3 H), 3.52 (s, 3 H), 4.80 (q, J = 6.7 Hz, 1 H), 6.32 (s, 1 H), 6.40 (d, J = 15.8 Hz, 1 H), 6.91 (d, J =1 5.6 Hz, 1 H), 6.91 (s, 2 H), 7.85 (s, 1 H), 13.24 (br s, 1 H).

### Example 287 methyl (2S)-2-[5-({4-[2-(aminosulfonyl)ethyl]-1,3-dimethyl-1H-pyrazol-5-yl}oxy)-2,4-dichlorophenoxy]propionate

Methyl (2S)-2-[5-({4-[(E)-2-(aminosulfonyl)vinyl]-1,3-dimethyl-1H-pyrazol-5-yl}oxy)-2,4-dichlorophenoxy]propionate (0.86 g) obtained in Example 284 was dissolved in a mixed solvent of tetrahydrofuran (5 mL) and ethanol (5 mL), palladium carbon ethylenediamine complex (0.83 g) was added, and the mixture was stirred under a hydrogen atmosphere (1 atm) at room temperature for 8 hr. The catalyst was removed by filtration, and the filtrate was concentrated. The residue was crystallized from hexane-ethyl acetate to give the title compound as 0.25 hydrate in the form of white crystals (655 mg, 75%).
¹H-NMR (300 MHz, CDCl₃)δ:1.64 (d, J = 6.8 Hz, 3 H), 2.23 (s, 3 H), 2.68 - 2.78 (m, 2 H), 3.04 - 3.15 (m, 2 H), 3.57 (s, 3 H), 3.71 (s, 3 H), 4.54 - 4.68 (m, 1 H), 4.62 (s, 2 H), 6.28 (s, 1 H), 7.49 (s, 1 H).

### Example 288 (2S)-2-[5-({4-[2-(aminosulfonyl)ethyl]-1,3-dimethyl-1H-pyrazol-5-yl}oxy)-2,4-dichlorophenoxy]propionic acid

The title compound was obtained in the same manner as in Example 37 and from methyl (2S)-2-[5-({4-[2-(aminosulfonyl)ethyl]-1,3-dimethyl-1H-pyrazol-5-yl}oxy)-2,4-dichlorophenoxy]propionate obtained in Example 287.
¹H-NMR (300 MHz, DMSO-d₆)δ:1.47 (d, J = 6.8 Hz, 3 H), 2.12 (s, 3 H), 2.57 (t, J = 8.4 Hz, 2 H), 2.93 (t, J = 8.4 Hz, 2 H), 3.45 (s, 3 H), 4.79 (q, J = 6.8 Hz, 1 H), 6.29 (s, 1 H), 6.80 (s, 2 H), 7.77 (s, 1 H), 13.25 (s, 1 H).

### Example 289 methyl (2S)-2-(2,4-dichloro-5-{[4-(hydroxymethyl)-1,3-dimethyl-1H-pyrazol-5-yl]oxy}phenoxy)propionate

The title compound was obtained in the same manner as in Reference Example 59 and from methyl (2S)-2-{2,4-dichloro-5-[(4-formyl-1,3-dimethyl-1H-pyrazol-5-yl)oxy]phenoxy}propionate obtained in Example 102.
¹H-NMR (300 MHz, CDCl₃)δ:1.62 (d, J = 6.8 Hz, 3 H), 2.30 (s, 3 H), 3.58 (s, 3 H), 3.67 (s, 3 H), 4.30 (d, J = 5.5 Hz, 2 H), 4.61 (q, J= 6.7 Hz, 1 H), 6.46 (s, 1 H), 7.46 (s, 1 H).

### Example 290 (2S)-2-(2,4-dichloro-5-{[1,3-dimethyl-4-(phenoxymethyl)-1H-pyrazol-5-yl]oxy}phenoxy)propionic acid

A solution of methyl (2S)-2-(2,4-dichloro-5-{[4-(hydroxymethyl)-1,3-dimethyl-1H-pyrazol-5-yl]oxy}phenoxy)propionate (0.66 g) obtained in Example 289, phenol (191 mg) and triphenylphosphine (669 mg) in tetrahydrofuran (15 mL) was cooled to 0°C. Diethyl azodicarboxylate (40% toluene solution, 1.16 mL) was added dropwise to the reaction mixture, and the mixture was stirred for 2 hr while allowing to gradually warm to room temperature. The reaction mixture was concentrated, and the residue was subjected to silica gel column chromatography (hexane-ethyl acetate 19:1 - 3:2, v/v). The obtained yellow oil was dissolved in a mixed solvent of tetrahydrofuran (5 mL) and water (2.5 mL), and cooled to 0°C. Lithium hydroxide monohydrate (173 mg) was added, and the mixture was stirred at 0°C for 30 min. The reaction mixture was neutralized with 1N hydrochloric acid, and extracted with ethyl acetate. The organic layer was washed with saturated brine, dried over anhydrous magnesium sulfate and filtered. The filtrate was concentrated, and the residue was crystallized from heptane-ethyl acetate to give the title compound as white crystals (373 mg, yield 49%).
¹H-NMR (300 MHz, DMSO-d₆)δ:1.44 (d, J = 6.8 Hz, 3 H), 2.16 (s, 3 H), 3.54 (s, 3 H), 4.45 - 4.54 (m, 1 H), 4.59 - 4.71 (m, 2 H), 6.50 (s, 1 H), 6.75 - 6.83 (m, 2 H), 6.89 (t, J = 7.3 Hz, 1 H), 7.22 (t, J = 7.9 Hz, 2 H), 7.68 (s, 1 H), 13.26 (s, 1 H).

### Example 291 methyl (2S)-2-(2,4-dichloro-5-{[4-({[(ethylamino)carbonyl]oxy}methyl)-1,3-dimethyl-1H-pyrazol-5-yl]oxy}phenoxy)propionate

To a solution of methyl (2S)-2-(2,4-dichloro-5-{[4-(hydroxymethyl)-1,3-dimethyl-1H-pyrazol-5-yl]oxy}phenoxy)propionate (0.50 g) obtained in Example 289 in N,N-dimethylformamide (6 mL) was added 1,1'-carbonylbis-1H-imidazole (231 mg) at 0°C with stirring, and the mixture was stirred for 30 min. Ethylamine (2M tetrahydrofuran solution, 5 mL) was added to the reaction mixture, and the mixture was stirred for 3 hr while allowing to gradually warm to room temperature. 1N Hydrochloric acid was added to the reaction mixture, and the mixture was extracted with ethyl acetate. The organic layer was washed with water and saturated brine, and dried over anhydrous magnesium sulfate. After filtration, the filtrate was concentrated and the residue was subjected to silica gel column chromatography (hexane-ethyl acetate 9:1 - 65:35, v/v) to give the title compound as a white solid (241 mg, yield 41%). Crystallization from hexane-ethyl acetate gave white crystals.
¹H-NMR (300 MHz, CDCl₃)δ:1.09 (t, J = 7.2 Hz, 3 H), 1.62 (d, J = 7.0 Hz, 3 H), 2.25 (s, 3 H), 3.14 (dq, J = 6.7, 6.7 Hz, 2 H), 3.58 (s, 3 H), 3.67 (s, 3 H), 4.55 (q, J = 6.8 Hz, 1 H), 4.73 (s, 2 H), 5.05 (br s, 1 H), 6.26 (s, 1 H), 7.46 (s, 1 H).

### Example 292 (2S)-2-(2,4-dichloro-5-{[4-({[(ethylamino)carbonyl]oxy}methyl)-1,3-dimethyl-1H-pyrazol-5-yl]oxy}phenoxy)propionic acid

The title compound was obtained in the same manner as in Example 37 and from methyl (2S)-2-(2,4-dichloro-5-{[4-({[(ethylamino)carbonyl]oxy}methyl)-1,3-dimethyl-1H-pyrazol-5-yl]oxy}phenoxy)propionate obtained in Example 291.
¹H-NMR (300 MHz, DMSO-d₆)δ:0.95 (t, J = 7.2 Hz, 3 H), 1.47 (d, J = 6.8 Hz, 3 H), 2.14 (s, 3 H), 2.80 - 2.98 (m, 2 H), 3.49 (s, 3 H), 4. 47 - 4.64 (m, 2 H), 4.77 (q, J = 6.2 Hz, 1 H), 6.40 (s, 1 H), 6.92 (br s, 1 H), 7.75 (s, 1 H), 13.26 (s, 1 H).

### Example 293 methyl (2S)-2-(2,4-dichloro-5-{[4-(2-hydroxyethyl)-1,3-dimethyl-1H-pyrazol-5-yl]oxy}phenoxy)propionate

The title compound was obtained in the same manner as in Example 116 and from 2,4-dichloro-5-{[4-(2-hydroxyethyl)-1,3-dimethyl-1H-pyrazol-5-yl]oxy}phenol obtained in Reference Example 224 and methyl (2R)-2-{[(4-methylphenyl)sulfonyl]oxy}propionate obtained in Reference Example 96.
¹H-NMR (300 MHz, CDCl₃)δ:1.58 (br s, 1 H), 1.61 (d, J = 6.8 Hz, 3 H), 2.24 (s, 3 H), 2.47 (t, J = 6.6 Hz, 2 H), 3.54 (s, 3 H), 3.60 (dt, J = 6.5, 6.5 Hz, 2 H), 3.67 (s, 3 H), 4.54 (q, J = 6.8 Hz, 1 H), 6.23 (s, 1 H), 7.46 (s, 1 H).

### Example 294 (2S)-2-(2,4-dichloro-5-{[4-(2-{[(ethylamino)carbonyl]oxy}ethyl)-1,3-dimethyl-1H-pyrazol-5-yl]oxy}phenoxy)propionic acid

The title compound was obtained in the same manner as in Example 291 and then Example 37, and from methyl (2S)-2-(2,4-dichloro-5-{[4-(2-hydroxyethyl)-1,3-dimethyl-1H-pyrazol-5-yl]oxy}phenoxy)propionate obtained in Example 293 and ethylamine (2M tetrahydrofuran solution).
¹H-NMR (300 MHz, DMSO-d₆)δ:0.96 (t, J = 7.2 Hz, 3 H), 1.47 (d, J = 6.8 Hz, 3 H), 2.10 (s, 3 H), 2.25 - 2.47 (m, 2 H), 2.88 - 3.01 (m, 2 H), 3.46 (s, 3 H), 3.82 (t, J = 7.3 Hz, 2 H), 4.80 (q, J = 6.8 Hz, 1 H), 6.34 (s, 1 H), 6.97 (t, J = 5.3 Hz, 1 H), 7.76 (s, 1 H), 13.24 (br s, 1 H).

### Example 295 (2S)-2-(2,4-dichloro-5-{[4-((1E)-3-{[(cyclopropylamino)carbonyl]oxy}prop-1-en-1-yl)-1,3-dimethyl-1H-pyrazol-5-yl]oxy}phenoxy)propionic acid

The title compound was obtained in the same manner as in Example 294 and from methyl (2S)-2-[2,4-dichloro-5-({4-[(1E)-3-hydroxyprop-1-en-1-yl]-1,3-dimethyl-1H-pyrazol-5-yl}oxy)phenoxy]propionate obtained in Example 171 and cyclopropylamine.
¹H-NMR (300 MHz, DMSO-d₆)δ:0.34 (d, J = 3.0 Hz, 2 H), 0.53 (d, J = 5.3 Hz, 2 H), 1.45 (d, J = 6.8 Hz, 3 H), 2.20 (s, 3 H), 2.31 - 2.45 (m, 1 H), 3.48 (s, 3 H), 4.32 - 4.56 (m, 2 H), 4.71 (q, J = 6.4 Hz, 1 H), 5.69 (ddd, J = 16.1, 6.2, 6.1 Hz, 1 H), 6.22 (d, J = 16.7 Hz, 1 H), 6.26 (s, 1 H), 7.26 (br s, 1 H), 7.78 (s, 1 H), 13.20 (br s, 1 H).

### Example 296 (2S)-2-[2,4-dichloro-5-({4-[(1E)-3-({[(cyclopropylmethyl)amino]carbonyl}oxy)prop-1-en-1-yl]-1,3-dimethyl-1H-pyrazol-5-yl}oxy)phenoxy]propionic acid

The title compound was obtained in the same manner as in Example 294 and from methyl (2S)-2-[2,4-dichloro-5-({4-[(1E)-3-hydroxyprop-1-en-1-yl]-1,3-dimethyl-1H-pyrazol-5-yl}oxy)phenoxy]propionate obtained in Example 171 and cyclopropylmethylamine.
¹H-NMR (300 MHz, DMSO-d₆)δ:0.11 (q, J = 4.9 Hz, 2 H), 0.28 - 0.39 (m, 2 H), 0.77 - 0.92 (m, 1 H), 1.45 (d, J = 6.8 Hz, 3 H), 2.20 (s, 3 H), 2.83 (t, J = 6.2 Hz, 2 H), 3.48 (s, 3 H), 4.33 - 4.58 (m, 2 H), 4.70 (q, J = 6.4 Hz, 1 H), 5.70 (ddd, J = 16.2, 6.1, 6.1 Hz, 1 H), 6.23 (d, J = 16.7 Hz, 1 H), 6.26 (s, 1 H), 7.17 (t, J = 5.5 Hz, 1 H), 7.78 (s, 1 H), 13.20 (br s, 1 H).

### Example 297 methyl (2S)-2-(2,4-dichloro-5-{[4-(3-hydroxypropyl)-1,3-dimethyl-1H-pyrazol-5-yl]oxy}phenoxy)propionate

To 2,4-dichloro-5-{[4-(3-hydroxypropyl)-1,3-dimethyl-1H-pyrazol-5-yl]oxy}phenol (4.30 g) obtained in Reference Example 226 and potassium carbonate (5.38 g) was added dimethyl sulfoxide (20 mL), and the mixture was stirred for 10 min. The reaction system was deaerated, and purged with nitrogen, and a solution of methyl (2R)-2-{[(4-methylphenyl)sulfonyl]oxy}propionate obtained (3.69 g) in Reference Example 96 in dimethyl sulfoxide (20 mL) was added carefully so that the temperature of the reaction system would not exceed 30°C. The mixture was stirred at room temperature for 3 hr, aqueous ammonium chloride solution was added, and the mixture was extracted with ethyl acetate. The organic layer was washed with saturated brine, dried over anhydrous magnesium sulfate and filtered. The filtrate was concentrated, and the residue was subjected to silica gel column chromatography (hexane-ethyl acetate 90:10 - 20:80, v/v) to give the title compound as a colorless oil (3.68 g, yield 68%).
¹H-NMR (300 MHz, CDCl₃)δ:1.43 (t, J = 5.1 Hz, 1 H), 1.58 - 1.70 (m, 5 H), 2.21 (s, 3 H), 2.28 (t, J = 7.4 Hz, 2 H), 3.54 (s, 3 H), 3.56 - 3.61 (m, 2 H), 3.67 (s, 3 H), 4.53 (q, J = 6.8 Hz, 1 H), 6.16 (s, 1 H), 7.46 (s, 1 H).

### Example 298 (2S)-2-(2,4-dichloro-5-{[4-(3-{[(ethylamino)carbonyl]oxy}propyl)-1,3-dimethyl-1H-pyrazol-5-yl]oxy}phenoxy)propionic acid

To a solution of methyl (2S)-2-(2,4-dichloro-5-{[4-(3-hydroxypropyl)-1,3-dimethyl-1H-pyrazol-5-yl]oxy}phenoxy)propionate (3.68 g) obtained in Example 297 in pyridine (30 mL) was added ethyl isocyanate (2.10 mL), and the mixture was stirred at room temperature for 20 hr, and at 50°C for 3 hr. The reaction mixture was cooled to room temperature, water was added, and the mixture was extracted with ethyl acetate. The organic layer was washed with 1N hydrochloric acid and saturated brine, and dried over anhydrous magnesium sulfate. After filtration, the filtrate was concentrated and the residue was subjected to silica gel column chromatography (hexane-ethyl acetate 85:15 - 20:80, v/v). The obtained oil was dissolved in a mixed solvent of tetrahydrofuran (30 mL) and water (15 mL), and cooled to 0°C. Lithium hydroxide monohydrate (1.34 g) was added, and the mixture was stirred at 0°C for 30 min. The reaction mixture was neutralized with dil. hydrochloric acid, and extracted with ethyl acetate. The organic layer was washed with saturated brine, dried over anhydrous magnesium sulfate and filtered. The filtrate was concentrated, and the residue was crystallized from heptane-ethyl acetate to give the title compound as colorless crystals (2.67 g, yield 74%).
¹H-NMR (300 MHz, DMSO-d₆)δ:0.97 (t, J = 7.1 Hz, 3 H), 1.45 (d, J = 6.8 Hz, 3 H), 1.54 (tt, J = 6.8, 6.8 Hz, 2 H), 2.08 (s, 3 H), 2.11 - 2.25 (m, 2 H), 2.87 - 3.04 (m, 2 H), 3.46 (s, 3 H), 3.78 (t, J = 6.2 Hz, 2 H), 4. 30 - 4.94 (m, 1 H), 6.30 (s, 1 H), 7.00 (br s, 1 H), 7.74 (s, 1 H), 13.31 (br s, 1 H).

### Example 299 (2S)-2-[2,4-dichloro-5-({4-[3-({[ethyl(methyl)amino]carbonyl}oxy)propyl]-1,3-dimethyl-1H-pyrazol-5-yl}oxy)phenoxy]propionic acid

The title compound was obtained in the same manner as in Example 294 and from methyl (2S)-2-(2,4-dichloro-5-{[4-(3-hydroxypropyl)-1,3-dimethyl-1H-pyrazol-5-yl]oxy}phenoxy)propionate obtained in Example 297 and ethylmethylamine.
¹H-NMR (300 MHz, DMSO-d₆)δ:0.90 - 1.02 (m, 3 H), 1.47 (d, J = 6.8 Hz, 3 H), 1.57 (tt, J = 7.1, 6.8 Hz, 2 H), 2.09 (s, 3 H), 2.11 - 2.25 (m, 2 H), 2.65 - 2.79 (m, 3 H), 3.02 - 3.25 (m, 2 H), 3.46 (s, 3 H), 3.83 (t, J = 5.7 Hz, 2 H), 4.74 (q, J = 6.7 Hz, 1 H), 6.29 (s, 1 H), 7.75 (s, 1 H), 13.22 (br s, 1 H).

### Example 311 5-({2,4-dichloro-5-[(1S)-2-methoxy-1-methyl-2-oxoethoxy]phenyl}thio)-1,3-dimethyl-1H-pyrazole-4-carboxylic acid

To a solution of methyl (2S)-2-{2,4-dichloro-5-[(4-formyl-1,3-dimethyl-1H-pyrazol-5-yl)thio]phenoxy}propionate (4.11 g) obtained in Example 207 in a mixed solvent of tetrahydrofuran (60 mL), tert-butanol (30 mL) and water (15 mL) were added successively 2-methyl-2-butene (5.50 mL), sodium dihydrogen phosphate (6.11 g) and sodium chlorite (1.84 g) at 0°C, and the mixture was stirred for 2 hr while allowing to gradually warm to room temperature. To the reaction mixture were added sodium bisulfite (3.18 g) and ethyl acetate, and then 1N hydrochloric acid, and the organic layer was washed with saturated brine, and dried over anhydrous magnesium sulfate. After filtration, the filtrate was concentrated and the residue was subjected to silica gel column chromatography (hexane-ethyl acetate 9:1 - 1:4, v/v), and crystallized from hexane-ethyl acetate to give the title compound as white crystals (2.57 g, yield 60%).
¹H-NMR (300 MHz, DMSO-d₆)δ:1.45 (d, J = 6.8 Hz, 3 H), 2.41 (s, 3 H), 3.59 (s, 3 H), 3.73 (s, 3 H), 4.64 (q, J = 6.7 Hz, 1 H), 5.88 (s, 1 H), 7.72 (s, 1 H), 12.55 (br s, 1 H).

### Example 312 5-({5-[(1S)-1-carboxyethoxy]-2,4-dichlorophenyl}thio)-1,3-dimethyl-1H-pyrazole-4-carboxylic acid

The title compound was obtained in the same manner as in Example 37 and from 5-({2,4-dichloro-5-[(1S)-2-methoxy-1-methyl-2-oxoethoxy]phenyl}thio)-1,3-dimethyl-1H-pyrazole-4-carboxylic acid obtained in Example 311.
¹H-NMR (300 MHz, DMSO-d₆)δ:1.44 (d, J = 6.6 Hz, 3 H), 2.39 (s, 3 H), 3.73 (s, 3 H), 4.48 (q, J = 6.7 Hz, 1 H), 6.01 (s, 1 H), 7.70 (s, 1 H), 12.47 (br s, 1 H), 13.08 (br s, 1 H).

### Example 313 methyl (2S)-2-(5-{[4-(aminocarbonyl)-1,3-dimethyl-1H-pyrazol-5-yl]thio}-2,4-dichlorophenoxy)propionate

To a solution of 5-({2,4-dichloro-5-[(1S)-2-methoxy-1-methyl-2-oxoethoxy]phenyl}thio)-1,3-dimethyl-1H-pyrazole-4-carboxylic acid (500 mg) obtained in Example 311 in acetonitrile (5 mL) were added 1-hydroxybenzotriazole ammonia complex (271 mg), triethylamine (0.25 mL) and N-ethyl-N'-(3-dimethylaminopropyl)carbodiimide hydrochloride (351 mg), and the mixture was stirred at room temperature for 1 hr. 1N Hydrochloric acid was added to the reaction mixture, and the mixture was extracted with ethyl acetate. The organic layer was washed with 1N hydrochloric acid and saturated brine, and dried over anhydrous magnesium sulfate. After filtration, the filtrate was concentrated and the residue was subjected to silica gel column chromatography (hexane-ethyl acetate 9:1 - 1:9, v/v) to give the title compound as a white solid (426 mg, yield 86%). Crystallization from hexane-ethyl acetate gave white crystals.
¹H-NMR (300 MHz, CDCl₃)δ:1.56 - 1.63 (m, 3 H), 2.60 (s, 3 H), 3.69 (s, 3 H), 3.82 (s, 3 H), 4.37 (q, J = 6.8 Hz, 1 H), 5.37 (br s, 1 H), 5.82 (s, 1 H), 6.91 (br s, 1 H), 7.43 (s, 1 H).

### Example 314 (2S)-2-(5-{[4-(aminocarbonyl)-1,3-dimethyl-1H-pyrazol-5-yl]thio}-2,4-dichlorophenoxy)propionic acid

The title compound was obtained in the same manner as in Example 37 and from methyl (2S)-2-(5-{[4-(aminocarbonyl)-1,3-dimethyl-1H-pyrazol-5-yl]thio}-2,4-dichlorophenoxy)propionate obtained in Example 313.
¹H-NMR (300 MHz, DMSO-d₆)δ:1.45 (d, J = 6.8 Hz, 3 H), 2.33 (s, 3 H), 3.69 (s, 3 H), 4.50 (q, J = 6.8 Hz, 1 H), 6.00 (s, 1 H), 7.16 (br s, 1 H), 7.32 (br s, 1 H), 7.71 (s, 1 H), 13.17 (br s, 1 H).

### Example 315 methyl (2S)-2-(2,4-dichloro-5-{[1,3-dimethyl-4-(pyrrolidin-1-ylcarbonyl)-1H-pyrazol-5-yl]thio}phenoxy)propionate

To a solution of 5-({2,4-dichloro-5-[(1S)-2-methoxy-1-methyl-2-oxoethoxy]phenyl}thio)-1,3-dimethyl-1H-pyrazole-4-carboxylic acid (500 mg) obtained in Example 311 in acetonitrile (5 mL) were added 1-hydroxybenzotriazole (161 mg), triethylamine (0.25 mL), pyrrolidine (0.15 mL) and N-ethyl-N'-(3-dimethylaminopropyl)carbodiimide hydrochloride (351 mg), and the mixture was stirred at room temperature for 1 hr. 1N Hydrochloric acid was added to the reaction mixture, and the mixture was extracted with ethyl acetate. The organic layer was washed with 1N hydrochloric acid and saturated brine, and dried over anhydrous magnesium sulfate. After filtration, the filtrate was concentrated and the residue was subjected to silica gel column chromatography (hexane-ethyl acetate 9:1 - 1:9, v/v) to give the title compound as a colorless oil (530 mg, yield 94%).
¹H-NMR (300 MHz, CDCl₃)δ:1.51 - 1.62 (m, 3 H), 1.77 - 2.10 (m, 4 H), 2.32 (s, 3 H), 3.21 - 3.38 (m, 2 H), 3.49 - 3.60 (m, 2 H), 3.69 (s, 3 H), 3.76 (s, 3 H), 4.67 (q, J = 6.8 Hz, 1 H), 6.26 (s, 1 H), 7.35 (s, 1 H).

### Example 316 (2S)-2-(2,4-dichloro-5-{[1,3-dimethyl-4-(pyrrolidin-1-ylcarbonyl)-1H-pyrazol-5-yl]thio}phenoxy)propionic acid

The title compound was obtained in the same manner as in Example 37 and from methyl (2S)-2-(2,4-dichloro-5-{[1,3-dimethyl-4-(pyrrolidin-1-ylcarbonyl)-1H-pyrazol-5-yl]thio}phenoxy)propionate obtained in Example 315.
¹H-NMR (300 MHz, DMSO-d₆)δ:1.47 (d, J = 6.8 Hz, 3 H), 1.70 - 1.87 (m, 4 H), 2.17 (s, 3 H), 3.15 (t, J = 6.4 Hz, 2 H), 3.33 - 3.42 (m, 2 H), 3.70 (s, 3 H), 4.61 (q, J = 6.9 Hz, 1 H), 6.27 (s, 1 H), 7.71 (s, 1 H), 13.16 (br s, 1 H).

### Example 317 (2S)-2-{2,4-dichloro-5-[(4-{[(3-methoxypropyl)amino]carbonyl}-1,3-dimethyl-1H-pyrazol-5-yl)thio]phenoxy}propionic acid

The title compound was obtained in the same manner as in Example 315 and then Example 37, and from 5-({2,4-dichloro-5-[(1S)-2-methoxy-1-methyl-2-oxoethoxy]phenyl}thio)-1,3-dimethyl-1H-pyrazole-4-carboxylic acid obtained in Example 311 and 3-methoxypropylamine.
¹H-NMR (300 MHz, DMSO-d₆)δ:1.45 (d, J = 6.8 Hz, 3 H), 1.62 (tt, J = 6.5, 6.5 Hz, 2 H), 2.30 (s, 3 H), 3.15 (s, 3 H), 3.16 - 3.24 (m, 2 H), 3.26 (t, J = 6.3 Hz, 2 H), 3.70 (s, 3 H), 4.50 (q, J = 6.7 Hz, 1 H), 6.01 (s, 1 H), 7.71 (s, 1 H), 7.77 (br s, 1 H), 13.17 (s, 1 H).

### Example 331 methyl (2S)-2-(5-{[4-(aminocarbonyl)-1,3-dimethyl-1H-pyrazol-5-yl]oxy}-2,4-dichlorophenoxy)propionate

The title compound was obtained in the same manner as in Example 116 and from 5-(2,4-dichloro-5-hydroxyphenoxy)-1,3-dimethyl-1H-pyrazole-4-carboxamide obtained in Reference Example 239 and methyl (2R)-2-{[(4-methylphenyl)sulfonyl]oxy}propionate obtained in Reference Example 96.
¹H-NMR (300 MHz, CDCl₃)δ:1.61 (d, J = 6.8 Hz, 3 H), 2.52 (s, 3 H), 3.54 (s, 3 H), 3.68 (s, 3 H), 4.52 (q, J = 6.8 Hz, 1 H), 5.20 (br s, 1 H), 5.87 (br s, 1 H), 6.10 (s, 1 H), 7.51 (s, 1 H).

### Example 332 (2S)-2-(5-{[4-(aminocarbonyl)-1,3-dimethyl-1H-pyrazol-5-yl]oxy}-2,4-dichlorophenoxy)propionic acid

The title compound was obtained in the same manner as in Example 112 and from methyl (2S)-2-(5-{[4-(aminocarbonyl)-1,3-dimethyl-1H-pyrazol-5-yl]oxy}-2,4-dichlorophenoxy)propionate obtained in Example 331.
¹H-NMR (300 MHz, CDCl₃)δ:1.65 (d, J = 6.8 Hz, 3 H), 2.52 (s, 3 H), 3.55 (s, 3 H), 4.45 (q, J = 6.4 Hz, 1 H), 5.84 (br s, 1 H), 6.06 (s, 1 H), 7.49 (s, 1 H), 7.57 (br s, 1 H).

### Example 333 methyl (2S)-2-(2,4-dichloro-5-{[1,3-dimethyl-4-(pyrrolidin-1-ylcarbonyl)-1H-pyrazol-5-yl]oxy}phenoxy)propionate

The title compound was obtained in the same manner as in Example 116 and from 2,4-dichloro-5-{[1,3-dimethyl-4-(pyrrolidin-1-ylcarbonyl)-1H-pyrazol-5-yl]oxy}phenol obtained in Reference Example 241 and methyl (2R)-2-{[(4-methylphenyl)sulfonyl]oxy}propionate obtained in Reference Example 96.
¹H-NMR (300 MHz, CDCl₃)δ:1.62 (d, J = 6.8 Hz, 3 H), 1.73 - 1.86 (m, 4 H), 2.29 (s, 3 H), 3.22 - 3.47 (m, 4 H), 3.62 (s, 3 H), 3.73 (s, 3 H), 4.73 (q, J = 6.8 Hz, 1 H), 6.48 (s, 1 H), 7.42 (s, 1 H).

### Example 334 (2S)-2-(2,4-dichloro-5-{[1,3-dimethyl-4-(pyrrolidin-1-ylcarbonyl)-1H-pyrazol-5-yl]oxy}phenoxy)propionic acid

The title compound was obtained in the same manner as in Example 112 and from methyl (2S)-2-(2,4-dichloro-5-{[1,3-dimethyl-4-(pyrrolidin-1-ylcarbonyl)-1H-pyrazol-5-yl]oxy}phenoxy)propionate obtained in Example 333.
¹H-NMR (300 MHz, CDCl₃)δ:1.61 (d, J = 6.8 Hz, 3 H), 1.75 - 1.93 (m, 4 H), 2.33 (s, 3 H), 3.29 - 3.57 (m, 4 H), 3.65 (s, 3 H), 4.53 (q, J = 6.8 Hz, 1 H), 6.32 (s, 1 H), 7.43 (s, 1 H).

### Example 335 methyl (2S)-2-[2,4-dichloro-5-({4-[2-(1-hydroxycyclopropyl)ethyl]-1,3-dimethyl-1H-pyrazol-5-yl}oxy)phenoxy]propionate

The title compound was obtained in the same manner as in Example 116 and from 2,4-dichloro-5-({4-[2-(1-hydroxycyclopropyl)ethyl]-1,3-dimethyl-1H-pyrazol-5-yl}oxy)phenol obtained in Reference Example 243 and methyl (2R)-2-{[(4-methylphenyl)sulfonyl]oxy}propionate obtained in Reference Example 96.
¹H-NMR (300 MHz, CDCl₃)δ:0.92 - 1.05 (m, 3 H), 1.61 (d, J = 6.8 Hz, 3 H), 2.21 (s, 3 H), 2.35 (q, J = 7.3 Hz, 2 H), 2.40 - 2.59 (m, 4 H), 3.52 (s, 3 H), 3.67 (s, 3 H), 4.54 (q, J = 6.8 Hz, 1 H), 6.14 (s, 1 H), 7.46 (s, 1 H).

### Example 336 (2S)-2-[2,4-dichloro-5-({4-[2-(1-hydroxycyclopropyl)ethyl]-1,3-dimethyl-1H-pyrazol-5-yl}oxy)phenoxy]propionic acid

The title compound was obtained in the same manner as in Example 112 and from methyl (2S)-2-[2,4-dichloro-5-({4-[2-(1-hydroxycyclopropyl)ethyl]-1,3-dimethyl-1H-pyrazol-5-yl}oxy)phenoxy]propionate obtained in Example 335.
¹H-NMR (300 MHz, CDCl₃)δ:0.93 - 1.06 (m, 3 H), 1.67 (d, J = 6.8 Hz, 3 H), 2.20 (s, 3 H), 2.38 (q, J = 7.4 Hz, 2 H), 2.44 - 2.65 (m, 4 H), 3.52 (s, 3 H), 4.46 (q, J = 6.8 Hz, 1 H), 6.12 (s, 1 H), 7.46 (s, 1 H).

### Example 337 (2S)-2-[2,4-dichloro-5-({1,3-dimethyl-4-[(1E)-3-methylbut-1,3-dien-1-yl]-1H-pyrazol-5-yl}oxy)phenoxy]propionic acid

Methyl (2S)-2-[2,4-dichloro-5-({1,3-dimethyl-4-[(1E)-3-methylbut-1,3-dien-1-yl]-1H-pyrazol-5-yl}oxy)phenoxy]propionate was obtained in the same manner as in Example 116 and from 2,4-dichloro-5-({4-[(1E)-3-hydroxy-3-methylbut-1-en-1-yl]-1,3-dimethyl-1H-pyrazol-5-yl}oxy)phenol obtained in Reference Example 244 and methyl (2R)-2-{[(4-methylphenyl)sulfonyl]oxy}propionate obtained in Reference Example 96. The title compound was obtained in the same manner as in Example 112 from this compound.
¹H-NMR (300 MHz, CDCl₃)δ:1.63 (d, J = 6.6 Hz, 3 H), 1.86 (s, 3 H), 2.32 (s, 3 H), 3.56 (s, 3 H), 4.43 (q, J = 6.9 Hz, 1 H), 4.83 - 5.03 (m, 2 H), 6.11 (d, J = 16.6 Hz, 1 H) 6.13 (s, 1 H), 6.54 (d, J = 16.6 Hz, 1 H), 7.49 (s, 1 H).

### Example 338 (2S)-2-[2,4-dichloro-5-({4-[(E)-2-(1-hydroxycyclopropyl)vinyl]-1,3-dimethyl-1H-pyrazol-5-yl}oxy)phenoxy]propionic acid

Methyl (2S)-2-[2,4-dichloro-5-({4-[(E)-2-(1-hydroxycyclopropyl)vinyl]-1,3-dimethyl-1H-pyrazol-5-yl}oxy)phenoxy]propionate was obtained in the same manner as in Example 116 and from 2,4-dichloro-5-({4-[(E)-2-(1-hydroxycyclopropyl)vinyl]-1,3-dimethyl-1H-pyrazol-5-yl}oxy)phenol obtained in Reference Example 245 and methyl (2R)-2-{[(4-methylphenyl)sulfonyl]oxy}propionate obtained in Reference Example 96. The title compound was obtained in the same manner as in Example 112 from this compound.
¹H-NMR (300 MHz, CDCl₃)δ:1.08 (t, J = 7.3 Hz, 3 H), 1.65 (d, J = 6.8 Hz, 3 H), 2.37 (s, 3 H), 2.55 (q, J = 7.3 Hz, 2 H), 3.65 (s, 3 H), 4.54 (q, J = 6.5 Hz, 1 H), 6.30 (s, 1 H), 6.35 (d, J = 16.2 Hz, 1 H), 7.21 (d, J = 16.2 Hz, 1 H), 7.49 (s, 1 H).

### Example 339 (2S)-2-(2,4-dichloro-5-{[1,3-dimethyl-4-(3-methyl-1,2,4-oxadiazol-5-yl)-1H-pyrazol-5-yl]oxy}phenoxy)propionic acid

Methyl (2S)-2-(2,4-dichloro-5-{[1,3-dimethyl-4-(3-methyl-1,2,4-oxadiazol-5-yl)-1H-pyrazol-5-yl]oxy}phenoxy)propionate was obtained in the same manner as in Example 116 and from 2,4-dichloro-5-{[1,3-dimethyl-4-(3-methyl-1,2,4-oxadiazol-5-yl)-1H-pyrazol-5-yl]oxy}phenol obtained in Reference Example 247 and methyl (2R)-2-{[(4-methylphenyl)sulfonyl]oxy}propionate obtained in Reference Example 96. The title compound was obtained in the same manner as in Example 112 from this compound.
¹H-NMR (300 MHz, CDCl₃)δ:1.62 (d, J = 6.8 Hz, 3 H), 2.34 (s, 3 H), 2.54 (s, 3 H), 3.68 (s, 3 H), 4.50 (q, J = 6.8 Hz, 1 H), 6.31 (s, 1 H), 7.49 (s, 1 H).

### Example 340 tert-butyl (2E)-3-(5-{2,4-dichloro-5-[(1S)-2-methoxy-1-methyl-2-oxoethoxy]phenoxy}-1,3-dimethyl-1H-pyrazol-4-yl)acrylate

The title compound was obtained in the same manner as in Example 116 and from tert-butyl (2E)-3-[5-(2,4-dichloro-5-hydroxyphenoxy)-1,3-dimethyl-1H-pyrazol-4-yl]acrylate obtained in Reference Example 248 and methyl (2R)-2-{[(4-methylphenyl)sulfonyl]oxy}propionate obtained in Reference Example 96.
¹H-NMR (300 MHz, CDCl₃)δ:1.47 (s, 9 H), 1.59 (d, J = 6.8 Hz, 3 H), 2.37 (s, 3 H), 3.55 (s, 3 H), 3.64 (s, 3 H), 4.48 (q, J = 6.8 Hz, 1 H), 5.94 (d, J = 15.9 Hz, 1 H), 6.11 (s, 1 H), 7.27 (d, J = 15.9 Hz, 1 H), 7.50 (s, 1 H).

### Example 341 (2E)-3-(5-{2,4-dichloro-5-[(1S)-2-methoxy-1-methyl-2-oxoethoxy]phenoxy}-1,3-dimethyl-1H-pyrazol-4-yl)acrylic acid

A mixture of tert-butyl (2E)-3-(5-{2,4-dichloro-5-[(1S)-2-methoxy-1-methyl-2-oxoethoxy]phenoxy}-1,3-dimethyl-1H-pyrazol-4-yl)acrylate (4.70 g) obtained in Example 340 and trifluoroacetic acid (1.44 mL) was stirred at 50°C for 3 hr. The reaction mixture was concentrated, water was added to the residue, and the mixture was extracted with ethyl acetate. The residue was subjected to silica gel column chromatography (hexane-ethyl acetate 80:20 - 20:80, v/v) to give the title compound (3.92 g, yield 94%) as a white solid.
¹H-NMR (300 MHz, CDCl₃)δ:1.59 (d, J = 6.8 Hz, 3 H), 2.39 (s, 3 H), 3.57 (s, 3 H), 3.63 (s, 3 H), 4.49 (q, J = 6.8 Hz, 1 H), 5.99 (d, J = 16.2 Hz, 1 H), 6.12 (s, 1 H), 7.45 (d, J = 16.2 Hz, 1 H), 7.51 (s, 1 H).

### Example 346 methyl (2S)-2-(2,4-dichloro-5-{[4-(4-cyanobenzyl)-1,3-dimethyl-1H-pyrazol-5-yl]oxy}phenoxy)propionate

The title compound was obtained in the same manner as in Example 116 and from 4-{[5-(2,4-dichloro-5-hydroxyphenoxy)-1,3-dimethyl-1H-pyrazol-4-yl]methyl}benzonitrile obtained in Reference Example 250 and methyl (2R)-2-{[(4-methylphenyl)sulfonyl]oxy}propionate obtained in Reference Example 96.
¹H-NMR (300 MHz, CDCl₃) δ:1.61 (d, J = 6.8 Hz, 3 H), 2.15 (s, 3 H), 3.47 - 3.65 (m, 5 H), 3.65 - 3.72 (m, 3 H), 4.38 (q, J = 7.0 Hz, 1 H), 6.07 (s, 1 H), 7.11 (d, J = 7.9 Hz, 2 H), 7.36 (s, 1 H), 7.48 (d, J = 7.9 Hz, 2 H).

### Example 347 (2S)-2-(2,4-dichloro-5-{[4-(4-cyanobenzyl)-1,3-dimethyl-1H-pyrazol-5-yl]oxy}phenoxy)propionic acid

The title compound was obtained in the same manner as in Example 112 and from methyl (2S)-2-(2,4-dichloro-5-{[4-(4-cyanobenzyl)-1,3-dimethyl-1H-pyrazol-5-yl]oxy}phenoxy)propionate obtained in Example 346.
¹H-NMR (300 MHz, CDCl₃)δ:1.66 (d, J = 6.8 Hz, 3 H), 2.14 (s, 3 H), 3.55 (s, 3 H), 3.67 (s, 2 H), 4.16 - 4.33 (m, 1 H), 6.00 (s, 1 H), 7.18 (d, J = 7.7 Hz, 2 H), 7.41 (s, 1 H), 7.51 (d, J = 7.9 Hz, 2 H).

### Example 367 (2S)-2-(2,4-dichloro-5-{[1,3-dimethyl-4-(5-methyl-1,3,4-thiadiazol-2-yl)-1H-pyrazol-5-yl]oxy}phenoxy)propionic acid

Methyl (2S)-2-(2,4-dichloro-5-{[1,3-dimethyl-4-(5-methyl-1,3,4-thiadiazol-2-yl)-1H-pyrazol-5-yl]oxy}phenoxy)propionate was obtained in the same manner as in Example 116 and from 2,4-dichloro-5-{[1,3-dimethyl-4-(5-methyl-1,3,4-thiadiazol-2-yl)-1H-pyrazol-5-yl]oxy}phenol obtained in Reference Example 258 and methyl (2R)-2-{[(4-methylphenyl)sulfonyl]oxy}propionate obtained in Reference Example 96. The title compound was obtained in the same manner as in Example 112 from this compound..
¹H-NMR (300 MHz, CDCl₃)δ:1.62 (d, J = 7.0 Hz, 3 H), 2.59 (s, 3 H), 2.72 (s, 3 H), 3.66 (s, 3 H), 4.47 (q, J = 6.8 Hz, 1 H), 6.19 (s, 1 H), 7.50 (s, 1 H).

### Example 368 methyl (2S)-2-(2,4-dichloro-5-{[4-(hydrazinocarbonyl)-1,3-dimethyl-1H-pyrazol-5-yl]oxy}phenoxy)propionate

The title compound was obtained in the same manner as in Example 116 and from 5-(2,4-dichloro-5-hydroxyphenoxy)-1,3-dimethyl-1H-pyrazole-4-carbohydrazide obtained in Reference Example 260 and methyl (2R)-2-{[(4-methylphenyl)sulfonyl]oxy}propionate obtained in Reference Example 96.
¹H-NMR (300 MHz, CDCl₃)δ:δ 1.61 (d, J = 6.8 Hz, 3 H), 2.51 (s, 3 H), 3.53 (s, 3 H), 3.68 (s, 3 H), 3.92 (br s, 2 H), 4.52 (q, J = 6.8 Hz, 1 H), 6.11 (s, 1 H), 7.13 (br s, 1 H), 7.51 (s, 1 H).

### Example 369 tert-butyl 2-[(5-{2,4-dichloro-5-[(1S)-2-methoxy-1-methyl-2-oxoethoxy]phenoxy}-1,3-dimethyl-1H-pyrazol-4-yl)carbonyl]hydrazinecarboxylate

To a solution of methyl (2S)-2-(2,4-dichloro-5-{[4-(hydrazinocarbonyl)-1,3-dimethyl-1H-pyrazol-5-yl]oxy}phenoxy)propionate (335 mg) obtained in Example 368 in tetrahydrofuran (5 mL) were added di-tert-butyl dicarbonate (192 mg) and triethylamine (0.33 mL), and the mixture was stirred at room temperature for 3 hr. Water was added to the reaction mixture, and the mixture was extracted with ethyl acetate. The organic layer was dried over anhydrous magnesium sulfate, and filtered, and the filtrate was concentrated. The residue was subjected to silica gel column chromatography (hexane-ethyl acetate 100:0 - 50:50, v/v) to give the title compound (409 mg, yield 100%) as a colorless amorphous solid. ¹H-NMR (300 MHz, CDCl₃)δ:1.44 (s, 9 H), 1.61 (d, J = 6.8 Hz, 3 H), 2.50 (s, 3 H), 3.55 (s, 3 H), 3.67 (s, 3 H), 4.59 - 4.79 (m, 1 H), 6.29 (s, 1 H), 6.39 (br s, 1 H), 7.49 (s, 1 H), 7.51 (d, J = 3.0 Hz, 1 H).

### Example 370 (2S)-2-{5-[(4-{[2-(tert-butoxycarbonyl)hydrazino]carbonyl}-1,3-dimethyl-1H-pyrazol-5-yl)oxy]-2,4-dichlorophenoxy}propionic acid

The title compound was obtained in the same manner as in Example 112 and from tert-butyl 2-[(5-{2,4-dichloro-5-[(1S)-2-methoxy-1-methyl-2-oxoethoxy]phenoxy}-1,3-dimethyl-1H-pyrazol-4-yl)carbonyl]hydrazinecarboxylate obtained in Example 369.
¹H-NMR (300 MHz, CDCl₃)δ:1,47 (s, 9 H), 1.65 (d, J = 6.8 Hz, 3 H), 2.50 (s, 3 H), 3.56 (s, 3 H), 4.56 (q, J = 6.7 Hz, 1 H), 6.36 (s, 1 H), 6.53 (br s, 1 H), 7.37 (br s, 1 H), 7.50 (s, 1 H).

### Example 371 (2S)-2-(2,4-dichloro-5-{[4-(hydrazinocarbonyl)-1,3-dimethyl-1H-pyrazol-5-yl]oxy}phenoxy)propionic acid hydrochloride

To a solution of (2S)-2-{5-[(4-{[2-(tert-butoxycarbonyl)hydrazino]carbonyl}-1,3-dimethyl-1H-pyrazol-5-yl)oxy]-2,4-dichlorophenoxy}propionic acid (329 mg) obtained in Example 370 in ethyl acetate (1.2 mL) was added 4N hydrochloric acid-ethyl acetate solution (1.2 mL), and the mixture was stirred at room temperature for 1 hr. The reaction mixture was concentrated and the resulting solid was recrystallized from tetrahydrofuran-diisopropyl ether to give the title compound (225 mg, yield 79%) as a white powder. ¹H-NMR (300 MHz, DMSO-d₆)δ:1.46 (d, J = 6.8 Hz, 3 H), 2.35 (s, 3 H), 3.54 (s, 3 H), 4.84 (q, J = 6.6 Hz, 1 H), 6.46 (s, 1 H), 7.77 (s, 1 H), 10.35 (br s, 1 H).

### Example 376 ethyl (2S)-2-{2,4-dichloro-5-[(1,3,4-trimethyl-1H-pyrazol-5-yl)oxy]phenoxy}propionate

(2S)-2-{2,4-Dichloro-5-[(1,3,4-trimethyl-1H-pyrazol-5-yl)oxy]phenoxy}propionic acid (2.50 g) obtained in Example 1, ethyl iodide (5.56 mL), potassium carbonate (9.62 g) and N,N'-dimethylformamide (100 mL) were mixed, and the mixture was stirred at room temperature for 18 hr. Ice-cooled water was added to the reaction mixture, and the mixture was extracted with ethyl acetate. The organic layer was washed with saturated brine, and dried over anhydrous magnesium sulfate. After filtration, the filtrate was concentrated and the residue was subjected to silica gel column chromatography (hexane-ethyl acetate 100:0 - 70:30, v/v), and crystallized from water - ethanol to give the title compound (2.21 g, yield 82%) as white crystals.
¹H-NMR (300 MHz, CDCl₃)δ:1.21 (t, J = 7.2 Hz, 3 H), 1.61 (d, J = 6.8 Hz, 3 H), 1.74 (s, 3 H), 2.18 (s, 3 H), 3.56 (s, 3 H), 3. 99 - 4.23 (m, 2 H), 4.51 (q, J = 6.8 Hz, 1 H), 6.21 (s, 1 H), 7.46 (s, 1 H).

### Example 377 tert-butyl (2S)-2-{2,4-dichloro-5-[(1,3,4-trimethyl-1H-pyrazol-5-yl)oxy]phenoxy}propionate

The title compound was obtained in the same manner as in Example 116 and from 2,4-dichloro-5-[(1,3,4-trimethyl-1H-pyrazol-5-yl)oxy]phenol obtained in Reference Example 3 and tert-butyl (2R)-2-{[(4-methylphenyl)sulfonyl]oxy}propionate obtained in Reference Example 261.
¹H-NMR (300 MHz, CDCl₃)δ:1.36 (s, 9 H), 1.58 (d, J = 6.8 Hz, 3 H), 1.75 (s, 3 H), 2.17 (s, 3 H), 3.56 (s, 3 H), 4.40 (q, J = 6.8 Hz, 1 H), 6.24 (s, 1 H), 7.46 (s, 1 H).

### Example 378 (2S)-2-{2,4-dichloro-5-[(4-cyano-1,3-dimethyl-1H-pyrazol-5-yl)thio]phenoxy}-N-methoxypropanamide

The title compound was obtained in the same manner as in Reference Example 22 and from (2S)-2-{2,4-dichloro-5-[(4-cyano-1,3-dimethyl-1H-pyrazol-5-yl)thio]phenoxy}propionic acid obtained in Example 210 and O-methylhydroxylammonium chloride.
¹H-NMR (300 MHz, CDCl₃)δ:1.59 (d, J = 6.8 Hz, 3 H), 2.39 (s, 3 H), 3.80 (s, 3 H), 3.91 (s, 3 H), 4.52 - 4.71 (m, 1 H), 6.69 (s, 1 H), 7.49 (s, 1 H), 9.08 (s, 1 H).

### Example 379 methyl (2S)-2-(2-bromo-5-{[3-cyclopropyl-4-(3-hydroxypropyl)-1-methyl-1H-pyrazol-5-yl]oxy}-4-methylphenoxy)propionate

The title compound was obtained in the same manner as in Example 116 and from 2-bromo-5-{[3-cyclopropyl-4-(3-hydroxypropyl)-1-methyl-1H-pyrazol-5-yl]oxy}-4-methylphenol obtained in Reference Example 265 and methyl (2R)-2-{[(4-methylphenyl)sulfonyl]oxy}propionate obtained in Reference Example 96.
¹H-NMR (300 MHz, CDCl₃)δ:0.81 - 0.92 (m, 4 H), 1.36 (t, J = 5.6 Hz, 1 H), 1.59 (d, J = 6.8 Hz, 3 H), 1.64 - 1.83 (m, 3 H), 2.29 (s, 3 H), 2.36 (t, J = 7.5 Hz, 2 H), 3.47 (s, 3 H), 3.54 - 3.63 (m, 2 H), 3.67 (s, 3 H), 4.49 (q, J = 6.8 Hz, 1 H), 6.01 (s, 1 H), 7.39 (s, 1 H).

### Example 380 (2S)-2-(2-bromo-5-{[3-cyclopropyl-4-(3-{[(ethylamino)carbonyl]oxy}propyl)-1-methyl-1H-pyrazol-5-yl]oxy}-4-methylphenoxy)propionic acid

The title compound was obtained in the same manner as in Example 173 and then Example 37, and from methyl (2S)-2-(2-bromo-5-{[3-cyclopropyl-4-(3-hydroxypropyl)-1-methyl-1H-pyrazol-5-yl]oxy}-4-methylphenoxy)propionate obtained in Example 379 and ethyl isocyanate.
¹H-NMR (300 MHz, DMSO-d₆)δ:0.68 - 0.83 (m, 4 H), 0.97 (t, J = 7.3 Hz, 3 H), 1.45 (d, J = 6.8 Hz, 3 H), 1.62 (tt, J = 6.9, 6.9 Hz, 2 H), 1.69 - 1.85 (m, 1 H), 2.17 - 2.34 (m, 2 H), 2.24 (s, 3 H), 2.87 - 3.06 (m, 2 H), 3.39 (s, 3 H), 3.82 (t, J = 6.4 Hz, 2 H), 4.55 (q, J = 6.7 Hz, 1 H), 6.02 (s, 1 H), 6.98 (t, J = 5.3 Hz, 1 H), 7.51 (s, 1 H), 13.13 (br. s., 1 H).

### Experimental Example 1 (PPARγ-RXRα heterodimer ligand activity)

PPARγ:RXRα:4ERPP/CHO-K1 cells described in WO03/099793 were cultured in hamF12 medium [manufactured by Life Technologies, Inc., USA] supplemented with 10% fetal bovine serum [manufactured by Life Technologies, Inc., USA], plated on a 96 well white half area plate [manufactured by Corning Coster Corporation, USA] at 1×10⁴ cells/well and cultured overnight in a carbon dioxide gas incubator at 37°C.
The medium was removed from the 96 well white half area plate, 45 µl of hamF12 medium containing 0.1% fatty acid-free bovine serum albumin (BSA) and 5 µl of a test compound were added, and the cells were cultured in a carbon dioxide gas incubator at 37°C for 1 day. The medium was removed, and 20 µl of PicaGene 7.5 (manufactured by Wako Pure Chemical Industries, Ltd.) diluted 2-fold with HBSS (HANKS' BALANCED SALT SOLUTION) [manufactured by BIO WHITTAKER, USA] was added. After stirring, the luciferase activity was measured using a 1420 ARVO Multilabel Counter [manufactured by Perkin Elmer Inc., USA].
The induction ratio was calculated from the luciferase activity of each test compound relative to the luciferase activity of the test compound non-administration group as 1. The concentration of the test compound and the induction ratio were analyzed using PRISM [manufactured by GraphPad Software, Inc., USA] and EC₅₀ value (concentration of compound showing 50% of the maximum induction ratio) of the test compound was calculated. The results are shown in Table 1.

**Table 1**

| Test Compound | EC₅₀ |
|---|---|
| (Example No.) | (nM) |
| 1 | 24 |
| 14 | 5.0 |
| 20 | 3.7 |
| 30 | 5.8 |
| 31 | 9.3 |
| 41 | 2.4 |
| 43 | 4.6 |
| 74 | 13 |
| 76 | 9.9 |
| 89 | 2.0 |
| 104 | 25 |
| 106 | 120 |
| 115 | 10 |
| 123 | 4.1 |
| 125 | 3.1 |
| 210 | 9.9 |
| 256 | 22 |
| 298 | 17 |

As shown above, the compound of the present invention has been shown to have a superior PPARγ-RXRα heterodimer ligand activity.

**Formulation Example 1 (production of capsule)**

| | |
|---|---|
| 1) compound of Example 1 | 30 mg |
| 2) finely divided powder cellulose | 10 mg |
| 3) lactose | 19 mg |
| 4) magnesium stearate | 1 mg |
| total | 60 mg |

| | |
|---|---|
| 1), 2), 3) and 4) are mixed and filled in a gelatin capsule. | |

**Formulation Example 2 (production of tablet)**

| | |
|---|---|
| 1) compound of Example 1 | 30 g |
| 2) lactose | 50 g |
| 3) cornstarch | 15 g |
| 4) calcium carboxymethylcellulose | 44 g |
| 5) magnesium stearate | 1 g |
| 1000 tablets total | 140 g |

The total amount of 1), 2), 3) and 30 g of 4) are kneaded with water, vacuum dried and sized. The sized powder is mixed with 14 g of 4) and 1 g of 5) and the mixture is punched out with a tableting machine. In this way, 1000 tablets containing 30 mg of the compound of Example 1 per tablet are obtained.

### Industrial Applicability

The compound of the present invention is useful for an agent for the prophylaxis or treatment of diabetes, which has a superior hypoglycemic action, and is associated with a fewer side effects such as body weight gain and the like.

This application is based on patent application Nos. 17656/2007 and 207273/2007 filed in Japan, the contents of which are hereby incorporated by reference.

## Claims

1. A compound represented by the formula (I): 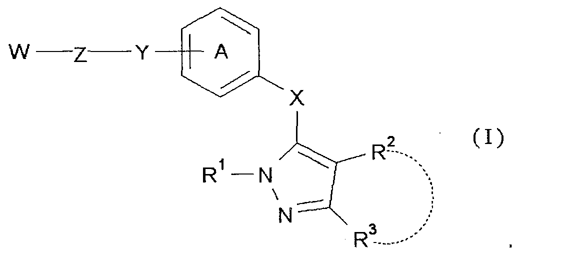 wherein
A is an optionally further substituted benzene ring;
R¹ is a hydrogen atom, an optionally substituted hydrocarbon group or an optionally substituted heterocyclic group;
R² is a hydrogen atom or a substituent (excluding an optionally substituted diazenyl group),
R³ is a hydrogen atom or a substituent, or
R² and R³ are optionally bonded to each other to form an optionally substituted monocyclic ring;
X is O, NR⁴ wherein R⁴ is a hydrogen atom or a C₁₋₆ alkyl group, S, SO, S(O)₂ or CH₂;
Y is a bond, O, NR⁵ wherein R⁵ is a hydrogen atom or a C₁₋₆ alkyl group, S, SO or S(O)₂;
Z is a bond, or a divalent hydrocarbon group having 1 to 9 atoms in the main chain, which is optionally substituted;
W is -C(=O)Q wherein Q is an optionally substituted hydroxy group or an optionally substituted amino group, or a 5- or 6-membered heterocyclic group containing NH and optionally having substituent(s),
provided that
1) when Y is a bond, and Z is a bond, then W should be a 5- or 6-membered heterocyclic group containing NH and optionally having substituent(s) (excluding a 5-phenylimino-4,5-dihydro-1,3,4-thiadiazolyl group), and the heterocyclic group should not be uracil, thioxooxadiazolinyl and thioxotriazolinyl, each of which is optionally substituted;
2) when -Z-Y- is 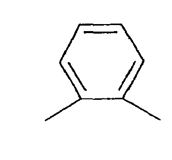 or 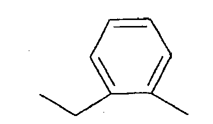 then X should not be CH₂;
3) -Z-Y- is not 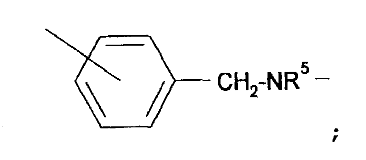 and
4) when Y is NR⁵, and Z is a bond, then W should be a 5- or 6-membered heterocyclic group containing NH and optionally having substituent(s),
or a salt thereof.

2. The compound of claim 1, wherein R¹ is an optionally substituted hydrocarbon group.

3. The compound of claim 1, wherein R² is an optionally substituted hydrocarbon group.

4. The compound of claim 1, wherein R³ is an optionally substituted hydrocarbon group.

5. The compound of claim 1, wherein X is O or S.

6. The compound of claim 1, wherein Y is a bond or O.

7. The compound of claim 1, wherein Z is a C₁₋₆ alkylene group or a C₂₋₆ alkenylene group.

8. The compound of claim 1, wherein W is -C(=O)Q wherein Q is as defined in claim 1.

9. (2S)-2-{2,4-dichloro-5-[(1,3,4-trimethyl-1H-pyrazol-5-yl)oxy]phenoxy}propionic acid,
(2S)-2-{2,4-dichloro-5-[(1,3-dimethyl-4-vinyl-1H-pyrazol-5-yl)oxy]phenoxy}propionic acid,
(2S)-2-{2,4-dichloro-5-[(2-methyl-2H-indazol-3-yl)oxy]phenoxy}propionic acid,
(2S)-2-{2,4-dichloro-5-[(2-methyl-4,5,6,7-tetrahydro-2H-indazol-3-yl)oxy]phenoxy}propionic acid,
(2S)-2-{2,4-dichloro-5-[(4-cyano-1,3-dimethyl-1H-pyrazol-5-yl)thio]phenoxy}propionic acid,
(2S)-2-{2-bromo-5-[(4-cyano-3-cyclopropyl-1-methyl-1H-pyrazol-5-yl)oxy]-4-methylphenoxy}propionic acid, or
(2S)-2-(2,4-dichloro-5-{[4-(3-{[(ethylamino)carbonyl]oxy}propyl)-1,3-dimethyl-1H-pyrazol-5-yl]oxy}phenoxy)propionic acid,
or a salt thereof.

10. A prodrug of the compound of claim 1.

11. A pharmaceutical agent comprising the compound of claim 1 or a prodrug thereof.

12. The pharmaceutical agent of claim 11, which is an insulin sensitizer.

13. The pharmaceutical agent of claim 11, which is an agent for the prophylaxis or treatment of diabetes.

14. A method of improving insulin resistance in a mammal, which comprises administering the compound of claim 1 or a prodrug thereof to the mammal.

15. A method for the prophylaxis or treatment of diabetes in a mammal, which comprises administering the compound of claim 1 or a prodrug thereof to the mammal.

16. Use of the compound of claim 1 or a prodrug thereof for the production of an insulin sensitizer.

17. Use of the compound of claim 1 or a prodrug thereof for the production of an agent for the prophylaxis or treatment of diabetes.
